# EUROPEAN PATENT APPLICATION

(11) **EP 1 475 389 A1**
(43) Date of publication of application: **10.11.2004**
(21) Application number: 04007416.3
(22) Date of filing: 08.11.2000
(51) Int. Cl.: C07K 14/515, C12N 15/12, G01N 33/68

(54) **Human secreted protein that shares sequence homology with the murine Sperizin protein**

(30) Priority: 12.11.1999 US 164834 P; 04.08.2000 US 224007 P
(62) Divisional of application: 00979145.0
(71) Applicant: Human Genome Sciences, Inc., Rockville, MD 20850 (US)
(72) Inventor: Ruben, Steven, M., Brookeville, MD 20833 (US); Duan, Roxanne, D., Bethesda, MD 20878 (US); Komatsoulis, George, A., Silver Spring, MD 20901 (US); Ebner, Reinhard, Gaithersburg, MD 20878 (US); Olsen, Henrik, S., Gaithersburg, MD 20878 (US)
(74) Representative: VOSSIUS & PARTNER

(57) **Abstract**

The present invention relates to a human secreted protein that displays some sequence homology with the murine sperizin protein. The invention also relates to isolated nucleic acids containing the coding regions of the gene encoding said protein. Also provided are vectors, host cells, antibodies, and recombinant methods for producing said human secreted protein. The invention further relates to diagnostic and therapeutic methods useful for diagnosing and treating disease, disorders, and/or conditions related to this novel human secreted protein, in particular those of the reproductive system.

<110> Human Genome Sciences, Inc.

<120> 15 Human Secreted Proteins

<130> PS715PCT

<140> Unassigned
<141> 2000-11-08

<150> 60/164,834
<151> 1999-11-12

<150> 60/224,007
<151> 2000-08-04

<160> 134

<170> PatentIn Ver. 2.0


<210> 1
<211> 733
<212> DNA
<213> Homo sapiens

<400> 1
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat 733


<210> 2
<211> 5
<212> PRT
<213> Homo sapiens

<220>
<221> Site
<222> (3)
<223> Xaa equals any of the twenty naturally ocurring L-amino acids

<400> 2
Trp Ser Xaa Trp Ser
  1               5

<210> 3
<211> 86
<212> DNA
<213> Homo sapiens

<400> 3
gcgcctcgag atttccccga aatctagatt tccccgaaat gatttccccg aaatgatttc 60
cccgaaatat ctgccatctc aattag 86


<210> 4
<211> 27
<212> DNA
<213> Homo sapiens

<400> 4
   gcggcaagct ttttgcaaag cctaggc 27

<210> 5
<211> 271
<212> DNA
<213> Homo sapiens

<400> 5
ctcgagattt ccccgaaatc tagatttccc cgaaatgatt tccccgaaat gatttccccg 60
aaatatctgc catctcaatt agtcagcaac catagtcccg cccctaactc cgcccatccc 120
gcccctaact ccgcccagtt ccgcccattc tccgccccat ggctgactaa ttttttttat 180
ttatgcagag gccgaggccg cctcggcctc tgagctattc cagaagtagt gaggaggctt 240
ttttggaggc ctaggctttt gcaaaaagct t 271

<210> 6
<211> 32
<212> DNA
<213> Homo sapiens

<400> 6
gcgctcgagg gatgacagcg atagaacccc gg 32

<210> 7
<211> 31
<212> DNA
<213> Homo sapiens

<400> 7
gcgaagcttc gcgactcccc ggatccgcct c 31

<210> 8
<211> 12
<212> DNA
<213> Homo sapiens

<400> 8
ggggactttc cc 12

<210> 9
<211> 73
<212> DNA
<213> Homo sapiens

<400> 9
gcggcctcga ggggactttc ccggggactt tccggggact ttccgggact ttccatcctg 60
ccatctcaat tag 73

<210> 10
<211> 256
<212> DNA
<213> Homo sapiens

<400> 10
ctcgagggga ctttcccggg gactttccgg ggactttccg ggactttcca tctgccatct 60
caattagtca gcaaccatag tcccgcccct aactccgccc atcccgcccc taactccgcc 120
cagttccgcc cattctccgc cccatggctg actaattttt tttatttatg cagaggccga 180
ggccgcctcg gcctctgagc tattccagaa gtagtgagga ggcttttttg gaggcctagg 240
cttttgcaaa aagctt 256

<210> 11
<211> 2339
<212> DNA
<213> Homo sapiens

<400> 11
cccacgcgtc cggggatgtt tgcgagcggc tggaaccaga cggtgccgat agaggaagcg 60
ggctccatgg ctgccctcct gctgctgccc ctgctgctgt tgctaccgct gctgctgctg 120
aagctacacc tctggccgca gttgcgctgg cttccggcgg acttggcctt tgcggtgcga 180
gctctgtgct gcaaaagggc tcttcgagcc cgcgccctgg ccgcggctgc cgccgacccg 240
gaaggtcccg aggggccctg catcctggcc tggcgcctcg cggaactggc ccagcagcgc 300
gcgcgaaact ttctattacg gtcgcgcgct ttagctactc agaggcggag cgcgagagta 360
acagggctga cgcgccttcc tacgtgcgct aggctgggac tggggacccg acggcggcga 420
cagcggcgag gggagcgctg gagaaggcga gcgggcagcg ccggaagccg gagatgcagc 480
ggccggaagc ggcgcggagt ttgccggagg ggacggtgcc gccagaggtg gaggagccgc 540
gcccctctgt cacctggagc aactgtggcg ctgctcctcc ccgctggccc agagtttctg 600
tggctctgga tcgggctggc caaggccggc ctgcgcactg cctttgtgcc caccgccctg 660
cgccggggcc ccctgctgca ctgcctccgc agctgcggcg cgcgcgcgct ggtgctggcg 720
ccagagtttc tggagtccct ggagccggac ctgcccgccc tgagagccat ggggctccac 780
ctgtgggctg caggcccagg aacccaccct gctggaatta gcgatttgct ggctgaagtg 840
tccgctgaag tggatggccc agtgccagga tacctctctt ccccccagag cataacagac 900
acgtgcctgt acatcttcac ctctggcacc acgggcctcc ccaaggctgc tcggatcagt 960
catctgaaga tcctgcaatg ccagggcttc tatcagctgt gtggtgtcca ccaggaagat 1020
gtgatctacc tcgccctccc actctaccac atgtccggtt ccctgctggg catcgtgggc 1080
tgcatgggca ttggggccac agtggtgctg aaatccaagt tctcggctgg tcagttctgg 1140
gaagattgcc agcagcacag ggtgacggtg ttccagtaca ttggggagct gtgccgatac 1200
cttgtcaacc agcccccgag caaggcagaa cgtggccata aggtccggct ggcagtgggc 1260
agcgggctgc gcccagatac ctgggagcgt tttgtgcggc gcttcgggcc cctgcaggtg 1320
ctggagacat atggactgac agagggcaac gtgccaccat caactacaca ggacagcggg 1380
gcgctgtggg gcgtgcttcc tggctttaca agcatatctt ccccttctcc ttgattcgct 1440
atgatgtcac cacaggagag ccaattcggg acccaggggc actgtatggc cacatctcca 1500
gggagcaggg ctgctggtgg ccccggtaag ccagcagtcc ccattcctgg gctatgctgg 1560
cgggccagag ctggcccagg ggaagttgct aaaggatgtc ttccggcctg ggatgttttt 1620
cttcaacact gggacctgct ggtctgcatg accaaggttt tctccgcttc catgatcgta 1680
ctggagacac cttcaggtgg aagggggaga atgtggccac aaccgaggtg gcagaggtct 1740
tcgaggccct agattttctt caggaggtga acgtctatgg agtcatgtgc cagggcatga 1800
aggcagggct ggaatggcag ccctagttct gcgtcccccc cacgctttgg accttatgca 1860
gctctacacc cacgtgtctg agaacttgcc accttatgcc cggccccgat tcctcaggct 1920
ccggagtctt tggccccaca gagaccttca aacagcagaa aagttcggat ggcaaatgag 1980
ggcttcgacc ccagcaccct gtctgaccca ctgtacgttc tggaccaggc tgtaggtgcc 2040
tacctgcccc tcacaactgc ccggtacagt gccctcctgg caggaaacct tcgaatctga 2100
gaacttccac acctgaggca cctgagagag gaactctgtg gggtgggggc cgttgcaggt 2160
gtactgggct gtcagggatc ttttctatac cagaactgcg gtcactattt tgtaataaat 2220
gtggctggag ctgatccagc tgtctctgac tacaaaaaaa aaaaaaaaaa aaaaaaaaaa 2280
aaaaaaaaaa aaaaaaaaaa aaaaaaaaaa aaaaaaaaaa aaaaaaaaaa aaaaaaaaa 2339

<210> 12
<211> 1574
<212> DNA
<213> Homo sapiens

<400> 12
ccacgcgtcc gggaggaccc gggagccggc ggcccggagc tgggagcgcg gggaaggcgg 60
ttggggttct gacagctgcg cgcgatcctg ctctctctca gccgcctgtg gacatgcgca 120
aagggccctc tcctgagtcc agatgatgct cataccaatg gcttcagtga tggcggtgac 180
tgaaccgaaa tgggtctcgg tctggagccg cttcctctgg gtgacgctgc tgagcatggt 240
gctggggtcc ctgctggccc tgctgctgcc gctgggggct gtggaggagc agtgcttggc 300
tgtgctcaaa ggcctctacc tgctcaggag caaaccggac agggcgcagc atgccgccac 360
caagtgcacc agcccgtcca cggagctcag catcacctcc aggggcgcga cgctgctggt 420
ggccaagacc aaggcctctc cagcgggtaa gttggaagcc agagctgccc tgaaccaggc 480
cctggagatg aagcgccagg gcaagcggga aaaagcccaa aagctcttca tgcacgccct 540
caagatggac ccggacttcg tggacgcgct caccgagttt ggcatcttct cggaagaaga 600
caaggacatc atccaggcgg actacttgta caccagagca ttgaccatct caccctacca 660
tgagaaagca ctggtcaacc gcgatcggac actgcctctt gtggaagaga tcgaccagag 720
gtatttcagc atcatcgaca gcaaagtgaa gaaggtcatg tccatcccca aggggaactc 780
agctctgcgc agggtcatgg aggagaccta ctaccatcac atctaccaca cagtggccat 840
cgagggcaac accctcaccc tctcggaaat caggcacatc ctggagaccc gctacgccgt 900
gcccgggaag agcctggagg agcagaacga ggtcataggc atgcatgcag ccatgaagta 960
catcaacacg actctggttt cgcgcatcgg ctccgtcacc atcagcgacg tgctggagat 1020
ccacaggcgg gtgctgggct acgtggaccc cgtggaagcc ggcaggtttc ggacaacaca 1080
ggtcctggtc ggacaccaca tccctcccca tccgcaggat gtggaaaagc agatgcagga 1140
gtttgtacag tggctcaact ccgaggaagc catgaacctg cacccagtgg agtttgcagc 1200
cttagcccat tataaactcg tttacatcca ccctttcatt gatggcaacg ggaggacctc 1260
ccgtctgctc atgaacctca tcctcatgca ggcgggctac ccgcccatca ccatccgcaa 1320

ggagcagcgg tccgactact accacgtgtt ggaagctgcc aacgagggcg acgtgaggcc 1380
tttcattcgc ttcatcgcca agtgtactga gaccaccctg gacaccctgc tttttgccac 1440
aactgagtac tcggtggcac tgccagaagc ccaacccaac cactctgggt tcaaggagac 1500
gcttcctgtg aagccctaac cctagaaatc ctcagtgaca aaggctgtcc tgaggtagga 1560
aaaaaaaaaa aaaa 1574

<210> 13
<211> 1431
<212> DNA
<213> Homo sapiens

<400> 13
ggcacgaggt catcggcact gggagtgcca acagctacgg cctctacacc atccgtgata 60
gggagaaccg cacctctctg gagctatgct ggttccgtga agggacaacc atgtacgccc 120
tctatatcac cgtccacggc tacttcctca tcaccttcct ctttggcatg gtggtcctgg 180
ccctggtggt ctggaagatc ttcaccctgt cccgtgctac agcggtcaag gagcggggga 240
agaaccggaa gaaggtgctc accctgctgg gcctctcgag cctggtgggt gtgacatggg 300
ggttggccat cttcaccccg ttgggcctct ccaccgtcta catctttgca cttttcaact 360
ccttgcaagg tgtcttcatc tgctgctggt tcaccatcct ttacctccca agtcagagca 420
ccacagtctc ctcctctact gcaagattgg accaggccca ctccgcatct caagaatagg 480
aaggcacggc cctgcaatat ggactcagct ctggctctct gtgtgacctt gggcagctcc 540
gtgcctctct ctgtactccc tcagtttcct tctctgtaca atgtggctgg ggagggagag 600
gatgggacca ggttggacca cgtggcatca gaggtcccat ccagatccaa ctataggtcc 660
aagagtccac gtaagcaggt ttgcaaggct ctaaagttcc tatagtcctg agaccccctg 720
ccagcaaaga gtgacagtca cctccatgcc ctgccctcat tgcaaagccc tcactcacct 780
tctggtctca gcaagggagg agagtctgtt gctggcatag ccctggaagg agcccccagc 840
ctctcccctc ctcctccttg tcactggcct cccacaactc cccttctggc tgcctgtaac 900
cttgaggggc attcaggagg ccagcgttcc ctcaggcact gggggtttgt tttggggggt 960
gggagttgat cctcccaccc agtctgcccc tggtctctgc ccatccaatc agagcccacc 1020
ctcctggaag agacccccgt gttcagagtg ctggcagccc tgcacgtgtc cagggacact 1080
gcatttcaaa gaaccactga gtgggtgagc taccttgggc aaacccccca ctcctgactc 1140
tgactgccac gtgggtggcc cgacctctga cctgctgtca tcgtagaggt agaaagcaaa 1200
caatctgtgg ctcagcacac ctgggggtgc tcccactcat tcagtgtgtg gggcccctga 1260
gcagaggctg ggcattgcca ctaggacctg agctcctaga gaacaaggac ctgggtggcc 1320
tcgcttactg ttccagccca ggccaagcac agggtctggc tcgtggcaaa ccttgaataa 1380
atatttgttg gctgaaaaaa aaaaaaaaaa aaaaaaaaaa aaaaaaaaaa a 1431

<210> 14
<211> 1145
<212> DNA
<213> Homo sapiens

<400> 14
ggcacgagcc tgagccccca gaatggcacc tgggcagaca ccttctctct gctcttggct 60
cttgccgttg ccctctactt gggctactac tgggcatgtg tgcttcagtg acatcctcca 120
aacaccagat ggaggccagc tcctgctaga ctgggccaag cagcctgaca gcagccaaga 180
ccctgatcct actacccagc ccattgtgct gctgcttcct ggcatcactg gcagtagcca 240
ggagacatac gtcttgcacc tagttaacca agctctgagg gatggctacc aggctgtcgt 300
gtttaacaac cggggctgcc gtggggagga actgcggacc cacagggctt tttgtgccag 360
caatactgaa gatctagaga cagtcgtgaa ccacataaag catcgttatc cccaagctcc 420
actgctggcc gtgggcatct cttttggagg gatactggtg ctgaatcacc tggcacaggc 480
caggcaggct gcagggctgg tggcagcact gactctgtct gcatgctggg attcctttga 540
gaccactcgc tccctggaaa ccccactcaa ctcactgctc ttcaatcagc ccctcactgc 600
tgggctctgc caacttgtgg aacgcctatc ctatggtaag acctgcaggc ccgtacaatc 660
cgccagtttg atgagcgcta cacatctgtg gcctttggat atcaagactg tgttacctac 720
tacaaagcag caagccctag aaccaagata gatgccatcc ggatccctgt gctctatctc 780
agtgcagcag atgacccctt ctcccccgtc tgtgcccttc ccatacaggc cgcccaacac 840
tccccctacg ttgcgctgct catcacagcc cggggtggcc acatcggctt cctggaaggg 900
ctgctcccgt ggcagcactg gtacatgagc cgcctcttgc atcagtacgc caaagccatc 960
ttccaggacc cagaggggct gcctgacctc agggctctct taccttctga ggacagaaac 1020
agctgacaag agtaccattt ggggtctcag ttcactcttt ccttgtttat taaatatcaa 1080
cttttcctgc ctaaaaaaaa aaaaaaaaaa aaaaaaaaaa aaaaaaaaaa aaaaaaaaaa 1140
aaaaa 1145

<210> 15
<211> 1214
<212> DNA
<213> Homo sapiens

<400> 15
   gcctagcagc acacagacag cgaagcgggt gaccatgggg tggccacggc cgggccgagc 60
   cctcgtggca gtcaaagcct tgctggtctt gtcgctgctc caggtgcccg cgcaggcagt 120
   ggtacgggcc gtgctggaag acaactcgag ctcggtggac tttgcggatc tgccggcgct 180
   gttcggcgtc cccctggccc ccgagggcat acggggctac ctgatggagg tcaagccagc 240
   caacgcgtgc catcccatcg aggccccgcg actgggcaac cgctctctgg gcgccatcgt 300
   gctgatccgc cgctacgact gcaccttcga cctcaaggtg ctgaacgccc agcgcgccgg 360
   cttcgaggcg gccatcgtgc acaacgtcca ctccgacgac ctcgtgagca tgacccacgt 420
   ctacgaggac ttgaggggcc agatcgccat cccctcagtg ttcgtgagcg aggccgcctc 480
   gcaggacctg cgggtcatcc tgggctgcaa caagtcggcc cacgcgctgc tcctgcccga 540
   cgacccaccg tgccacgacc tgggctgtca ccccgtgctg accgtgtcct gggtgctggg 600
   ctgtaccctg gccctggtcg tatcagcctt ctttgtcctg aaccacctgt ggctctgggc 660
   ccaggcctgc tgcagccaca gacggccggt gaagacgtct acctgccaga aggcccaggt 720
   ccgcaccttc acgtggcaca acgacctgtg tgccatctgc ctggatgagt atgaggaggg 780
   cgaccaactc aagatcctgc cctgctccca cacctaccac tgcaaatgca ttgacccctg 840
   gttctcccaa gccccccggc gctcctgccc cgtgtgcaaa cagtcggtgg ccgccacaga 900
   agacagcttt gactccacca cctacagctt cagggacgag gacccctccc taccgggcca 960
   ccggcccccc atctgggcca ttcaagtcca gctacgctcc cggaggctgg agctgctggg 1020
   ccgcgccagt ccccactgcc actgcagcac cacgtccctg gaggcagagt ataccactgt 1080
   ctcctcagcc cctcctgagg cccctggtca gtaaagatct agggcaggga ggggggtgca 1140
   atgaggaatg tttctggtct gaaaagaata aagtgggttt gaaagcggaa aaaaaaaaaa 1200
   aaaaaaaaac tcga 1214

<210> 16
<211> 2005
<212> DNA
<213> Homo sapiens

<400> 16
ccacgcgtcc gcgcccagcc atgactgcgc cggtccccgc gccgcggatc ctgttgccgt 60
tgctgttgct gctgctgcta acgccgcctc cgggtgcacg tggtgaggtg tgtatggctt 120
cccgtggact cagcctcttc cccgagtcct gtccagattt ctgctgtggt acctgtgatg 180
accaatactg ctgctctgac gtgctgaaga aatttgtgtg gagcgaggaa aggtgtgctg 240
tgcctgaggc cagcgtgcct gccagtgtag agccggtgga gcagctgggc tcggcgctga 300
ggtttcgccc tggctacaac gaccccatgt cagggttcgg agcgaccttg gccgttggcc 360
tgaccatctt tgtgctgtct gtcgtcacta tcatcatctg cttcacctgc tcctgctgct 420
gcctttacaa gacgtgccgc cgaccacgtc cggttgtcac caccaccaca tccaccactg 480
tggtgcatgc cccttatcct cagcctccaa gtgtgccgcc cagctaccct ggaccaagct 540
accagggcta ccacaccatg ccgcctcagc cagggatgcc agcagcaccc tacccaatgc 600
agtacccacc accttaccca gcccagccca tgggcccacc ggcctaccac gagaccctgg 660
ctggaggagc agccgcgccc taccccgcca gccagcctcc ttacaacccg gcctacatgg 720
atgccccgaa ggcggccctc tgagcattcc ctggcctctc tggctgccac ttggttatgt 780
tgtgtgtgtg cgtgagtggt gtgcaggcgc ggttccttac gccccatgtg tgctgtgtgt 840
gtccaggcac ggttccttac gccccatgtg tgctgtgtgt gtcctgcctg tatatgtggc 900
ttcctctgat gctgacaagg tggggaacaa tccttgccag agtgggctgg gaccagactt 960
tgttctcttc ctcacctgaa attatgcttc ctaaaatctc aagccaaact caaagaatgg 1020
ggtggtgggg ggcaccctgt gaggtggccc ctgagaggtg gggcctctcc cagggcacat 1080
ctggagttct tctccagctt accctagggt gaccaagtag ggcctgtcac accagggtgg 1140
cgcagctttc tgtgtgatgc agatgtgtcc tggtttcggc agcgtagcca gctgctgctt 1200
gaggccatgg ctcgtccccg gagttggggg tacccgttgc agagccaggg acatgatgca 1260
ggcgaagctt gggatctggc caagttggac tttgatcctt tgggcagatg tcccattgct 1320
ccctggagcc tgtcatgcct gttggggatc aggcagcctc ctgatgccag aacacctcag 1380
gcagagccct actcagctgt acctgtctgc ctggactgtc ccctgtcccc gcatctcccc 1440
tgggaccagc tggagggcca catgcacaca cagcctagct gcccccaggg agctctgctg 1500
cccttgctgg ccctgccctt cccacaggtg agcagggctc ctgtccacca gcacactcag 1560
ttctcttccc tgcagtgttt tcattttatt ttagccaaac attttgcctg ttttctgttt 1620
caaacatgat agttgatatg agactgaaac ccctgggttg tggagggaaa ttggctcaga 1680
gatggacaac ctggcaactg tgagtccctg cttcccgaca ccagcctcat ggaatatgca 1740
acaactcctg taccccagtc cacggtgttc tggcagcagg gacacctggg ccaatgggcc 1800
atctggacca aaggtggggt gtggggccct ggatggcagc tctggcccag acatgaatac 1860
ctcgtgttcc tcctccctct attactgttt caccagagct gtcttagctc aaatctgttg 1920
tgtttctgag tctagggtct gtacacttgt ttataataaa tgcaatcgtt tggaaaaaaa 1980
aaaaaaaaaa aaaaaaaaaa aaaaa 2005

<210> 17
<211> 2329
<212> DNA
<213> Homo sapiens

<400> 17
gcgtccgcaa gatgtccctg tggactccca aactctactc cagatgggga ggtgccctta 60
acaccaagat tttaaaagct ccaatttcag agcaagagtc gaaaactcac agataaagtt 120
atagttattt cagggttctg aaaagacgca gaacatgaag ggactcagaa gtctggcagc 180
aacaaccttg gctcttttcc tggtgtttgt tttcctggga aactccagct gcgctccgca 240
gagactgttg gagagaagga actggactcc tcaagctatg ctctacctga aaggggcaca 300
gggtcgccgc ttcatctccg accagagccg gagaaaggac ctctccgacc ggccactgcc 360
ggaaagacga agcccaaatc cccaactact aactattccg gaggcagcaa ccatcttact 420
ggcgtccctt cagaaatcac cagaagatga agaaaaaaac tttgatcaaa ccagattcct 480
ggaagacagt ctgcttaact ggtgaaaata tactggatta tgtttaatta tggttctatt 540
ctctttgaaa acatgaacca tgtgaataaa acctttggac ccttttattc catttgtaat 600
cttaagaaca cacacagata gttttattct ttcagaaaca aaatatatat aggatgctta 660
gctgagaaca tcatcttctt tcattgcttc aggtcctgtt tagatgacca aaaatgtttt 720
cagatcacct tgtgtcttac tcttgagttt cttagaatat ttataattat aaggctgaag 780
actaaagtgt tctttccttt taactatagc cagtacctgt cttgatctta gttgtgtttt 840
tttttttcat tttgttaccc acttgcattt tgttttcact cagcagaaat tctccttctc 900
tgttttcctt ttatcccatc cccaagaatg tggaaggaag gtgagaaaca tggcaggatg 960
ggaaatagga gagtatgact ctctatagct catccaggag taatcaatta agaagataaa 1020
ttggatgact gtggagaagc tctgtgatag gaacacttca gtgtggttgc tgagaggaga 1080
cagtcattga ggtagaaggt ttgccaaaga tccagagctc agagctccct ttgtgctctt 1140
tgggaattac cttgcattca gtttagaaac atggatctaa aagttactgg gaaataagca 1200
gatggagaca cactctgttg tttacgtatt ggaagaaggg aacaagccag ttttgttaga 1260
ggtaactcat tttccatgac caaacagact caacagattc aagtactctg cttactctaa 1320
ttgactagac tctaggtttt atttgacatc atagcattac ataaatcact ctgataacat 1380
aagtgcacag taatatgcct gatctcttcc tttttaaaag ccaacttgag ttcagtacca 1440
tctgaataca cacacatgca catataccca cacacgcata cacacatact cctgtggcaa 1500
acataataat gtatttattt agaattataa tatgaccatc atgttaatta tttttttacct 1560
aatcagagtt gttattgaca aatgtcataa gtggaaagta ttaattctta ttgtcatcag 1620
tatttagcca ttatttagta gctcaagaat atctttatgt gaatgtctct gtaacttgga 1680
attgcaattt cactgtgtta agtaatcaga actctgctta taagatttat ctgtatcttg 1740
tttcataatt taataatgaa actaaattca agttaatgta atgttgatct ccgtcgaaaa 1800
ataacttgtg agcattaaaa tatctgtatg gcattataat aggcacctgt cattaattat 1860
gatttgattt gaaagtagac ttactaggta gtgcttacat ctgatttatt gaaaattttt 1920
tccaaaagca ttgtctttta tttatacagt cctaaccact tcaccattag gaggggtggg 1980
acattaaaaa ttatttaccc atcagacata aaattgtaca aattgactat tcaaaatagt 2040
atgtgtattc aaagaacact tttaacatct atcttatgaa ataattcttc ctacaactaa 2100
ccagaacaat ctcaaaaatg tcatactaac cattgtttta aaagctgtct caatgatcaa 2160
tagctgttaa aatatagctc caagtattct aagtactcaa attccttgaa tgttcttatg 2220
tacaaatatg ctgattaatt aaaaggtccc tcttcccaaa aaaaaaaaaa aaaaaaaaaa 2280
aaaaaaaaaa aaaaaaaaaa aaaaaaaaaa aaaaaaaaaa aaaaaaaaa 2329

<210> 18
<211> 1452
<212> DNA
<213> Homo sapiens

<400> 18
ccacgcgtcc gggaagaagc cgagatggcg gcagccagcg ctggggcaac ccggctgctc 60
ctgctcttgc tgatggcggt agcagcgccc agtcgagccc ggggcagcgg ctgccgggcc 120
gggactggtg cgcgaggggc tggggcggaa ggtcgagagg gcgaggcctg tggcacggtg 180
gggctgctgc tggagcactc atttgagatc gatgacagtg ccaacttccg gaagcggggc 240
tcactgctct ggaaccagca ggatggtacc ttgtccctgt cacagcggca gctcagcgag 300
gaggagcggg gccgactccg ggatgtggca gccctgaatg gcctgtaccg ggtccggatc 360
ccaaggcgac ccggggccct ggatggcctg gaagctggtg gctatgtctc ctcctttgtc 420
cctgcgtgct ccctggtgga gtcgcacctg tcggaccagc tgaccctgca cgtggatgtg 480
gccggcaacg tggtgggcgt gtcggtggtg acgcacccca tggccccatg cagccccagg 540
ggcttccccc ctgcccatgg agtagagccc gagatcctgg ccactatgcc agttctgacc 600
tcgcatcccc ctaccccgag cccatgcagt ctgggaacat gccgccttct ctccagcctc 660
tgtgcctttg ttccaggtgg tctcaccctc ctgtccctgg ctgggctagg tggtcctgtc 720
caggctcctg cagcgccccc ctcactttga cactggacta ggatgcagcc tcccttctgt 780
gtccccttga gggtaccctg ggtcccctca tcaggggcag aggcatgaaa gagtcggggc 840
tgtatggcca ggggcttctg ggcccgacgc ctagtgcagc ccctggggtc gtggtttgac 900
atttgtctgc ctggtgcaaa caaggaatcc ttgcctttaa ggtgacaggc cctccacagg 960
cttccagact tgaaggaaaa ggtttaagaa agaaaacaaa accaacagtt agtggagtca 1020
aagcccagac actgtaaata gaaccccctc caccaccccc cgccgcccag catcctacct 1080
ggactgcggt gctacgaggg cctgcgggcc tttgctgtgt gccaccctcc ctgtaagtct 1140
atttaaaaac atcgacgata cattgaaatg tgtgaacgtt ttgaaaagct acagcttcca 1200
gcagccaaaa gcaactgttg ttttggcaag acggtcctga tgtacaagct tgattgaaat 1260
tcactgctca cttgatacgt tattcagaaa cccaaggaat ggctgtcccc atcctcatgt 1320

ggctgtgtgg agctcagctg tgttgtgtgg cattttatta aactgtcccc cagatcgaaa 1380
aaaaaaaaaa aaaaaaaaaa aaaaaaaaaa aaaaaaaaaa aaaaaaaaaa aaaaaaaaaa 1440
aaaaaaaaaa aa 1452

<210> 19
<211> 2778
<212> DNA
<213> Homo sapiens

<400> 19
ccacgcgtcc ggccgacctg ccgagatgga ggcgaccggc acctgggcgc tgctgctggc 60
gctggcgctg ctcctgctgc tgacgctggc gctgtccggg accagggccc gaggccacct 120
gcccccgggg cccacgccgc taccactgct gggaaacctc ctgcagctac ggcccggggc 180
gctgtattca gggctcatgc ggctgagtaa gaagtacgga ccggtgttca ccatctacct 240
gggaccctgg cggcctgtgg tggtcctggt tgggcaggag gctgtgcggg aggccctggg 300
aggtcaggct gaggagttca gcggccgggg aaccgtagcg atgctggaag ggacttttga 360
tggccatggg gttttcttct ccaacgggga gcggtggagg cagctgagga agtttaccat 420
gcttgctctg cgggacctgg gcatggggaa gcgagaaggc gaggagctga tccaggcgga 480
ggcccggtgt ctggtggaga cattccaggg gacagaagga cgcccattcg atccctccct 540
gctgctggcc caggccacct ccaacgtagt ctgctccctc ctctttggcc tccgcttctc 600
ctatgaggat aaggagttcc aggccgtggt ccgggcagct ggtggtaccc tgctgggagt 660
cagctcccag gggggtcagg tgagtgggtg ggacccctct ccaactacct tccctgaagg 720
ttcctgccaa ggtcccatga gaactagctg cccttctccc cacagaccta cgagatgttc 780
tcctggttcc tgcggcccct gccaggcccc cacaagcagc tcctccacca cgtcagcacc 840
ttggctgcct tcacagtccg gcaggtgcag cagcaccagg ggaacctgga tgcttcgggc 900
cccgcacgtg accttgtcga tgccttcctg ctgaagatgg cacaggagga acaaaaccca 960
ggcacagaat tcaccaacaa gaacatgctg atgacagtca tttatttgct gtttgctggg 1020
acgatgacgg tcagcaccac ggtcggctat accctcctgc tcctgatgaa ataccctcat 1080
gtccaaaagt gggtacgtga ggagctgaat cgggagctgg gggctggcca ggcaccaagc 1140
ctaggggacc gtacccgcct cccttacacc gacgcggttc tgcatgaggc gcagcggctg 1200
ctggcgctgg tgcccatggg aataccccgc accctcatgc ggaccacccg cttccgaggg 1260
tacaccctgc cccagggcac ggaggtcttc cccctccttg gctccatcct gcatgacccc 1320
aacatcttca agcacccaga agagttcaac ccagaccgtt tcctggatgc agatggacgg 1380
ttcaggaagc atgaggcgtt cctgcccttc tccttaggga agcgtgtctg ccttggagag 1440
ggcctggcaa aagcggagct cttcctcttc ttcaccacca tcctacaagc cttctccctg 1500
gagagcccgt gcccgccgga caccctgagc ctcaagccca ccgtcagtgg ccttttcaac 1560
attcccccag ccttccagct gcaagtccgt cccactgacc ttcactccac cacgcagacc 1620
agatgaagga aggcaacttg gaagtggtgg gtgcccggga cggtgcctcc agcctcaaca 1680
gtgggcatgg acagggttaa tgtctccaga gtgtacactg caggcagcca catttacacg 1740
cctgcagttg ttttccggag tctgtcccac ggcccacacg ctcacttgac tcatgctgct 1800
aagatgcaca accgcacacc catacacaac tacaagggcc acaaagcaac tgctgggtta 1860
gctttccaca gacataaata tagtccatct gcaatcacaa gcacatagcc aggtaaccca 1920
ccaactcccc tggatctgca gcccacacgt gggagtctgg ctgtcacctt cacaagccac 1980
agaaacggcc acacatgttc acagctcaca cgccctctcc attcatcgaa cttctcagtg 2040
tccctgtccc tggtgcctgg cacagggaac agcatgcccc ctccggggtc atgccaccca 2100
gagactgtcg ctgtctatgg ccccaactca tgctccctct cttggctaca ccactctccc 2160
agcctgtgac cacagatgtc cacacacccc caaccacttg tccacacagc tacccacgta 2220
cgacatcgtc ctggctcccc agagtatctt cccactgaga cacgccgccc ccacagaggc 2280
acagtcccca gccacctctg caactgcagc cctcagtcac ccctttttaa gcaccctgat 2340
tctaccaaat gcaaacacat ctgggtctgc gattatgcac agagactttg gacatacgag 2400
gaccctcaga ccggaggaac acctgcccaa ccccaacacg tgcttatgta accacgtgga 2460
aagcggcccc tgctgcccct ccacacacac atacacactc actgatctac agcccctgtt 2520
cggcgtcaga gtccccacta gacccagtgg aaggggttag agaccaagta ggggccagtt 2580
tccaattcac cctgtcaggg agtgagccgg atctgacgtt ccttgtgact taagggtccg 2640
gcttgggaat taaagtttgt ttctggcctt tagcctaaaa aaaaaaaaaa aaaaaaaaaa 2700
aaaaaaaaaa aaaaaaaaaa aaaaaaaaaa aaaaaaaaaa aaaaaaaaaa aaaaaaaaaa 2760
aaaaaaaaaa aaaaaagg 2778

<210> 20
<211> 2865
<212> DNA
<213> Homo sapiens

<220>
<221> SITE
<222> (677)
<223> n equals a,t,g, or c

<400> 20

aattcccggg tcgacccacg cgtccgaaga tggctacggc agggggtggc tctggggctg 60
acccgggaag tcggggtctc cttcgccttc tgtctttctg cgtcctacta gcaggtttgt 120
gcaggggaaa ctcagtggag aggaagatat atatcccctt aaataaaaca gctccctgtg 180
ttcgcctgct caacgccact catcagattg gctgccagtc ttcaattagt ggagacacag 240
gggttatcca cgtagtagag aaagaggagg acctacagtg ggtattgact gatggcccca 300
acccccctta catggttctg ctggagagca agcattttac cagggattta atggagaagc 360
tgaaagggag aaccagccga attgctggtc ttgcagtgtc cttgaccaag cccagtcctg 420
cctcaggctt ctctcctagt gtacagtgcc caaatgatgg gtttggtgtt tactccaatt 480
cctatgggcc agagtttgct cactgcagag aaatacagtg gaattcgctg ggcaatggtt 540
tggcttatga agactttagt ttccccatct ttcttcttga agatgaaaat gaaaccaaag 600
tcatcaagca gtgctatcaa gatcacaacc tgagtcagaa tggctcagca ccatccttcc 660
cactatgtgc catgcanctc ttttcacaca tgcatgctgt catcagcact gccacctgca 720
tgcggcgcag ctccatccaa agcaccttca gcatcaaccc agaaatcgtc tgtgaccccc 780
tgtctgatta caatgtgtgg agcatgctaa agcctataaa tacaactggg acattaaagc 840
ctgacgacag ggttgtggtt gctgccaccc ggctggatag tcgttccttt ttctggaatg 900
tggccccagg ggctgaaagc gcagtggctt cctttgtcac ccagctggct gctgctgaag 960
ctttgcaaaa ggcacctgat gtgaccaccc tgccccgcaa tgtcatgttt gtcttctttc 1020
aaggggaaac ttttgactac attggcagct cgaggatggt ctacgatatg gagaagggca 1080
agtttcccgt gcagttagag aatgttgact catttgtgga gctgggacag gtggccttaa 1140
gaacttcatt agagctttgg atgcacacag atcctgtttc tcagaaaaat gagtctgtac 1200
ggaaccaggt ggaggatctc ctggccacat tggagaagag tggtgctggt gtccctgctg 1260
tcatcctcag gaggccaaat cagtcccagc ctctcccacc atcttccctg cagcgatttc 1320
ttcgagctcg aaacatctct ggcgttgttc tggctgacca ctctggtgcc ttccataaca 1380
aatattacca gagtatttac gacactgctg agaacattaa tgtgagctat cccgaatggc 1440
tgagccctga agaggacctg aactttgtaa cagacactgc caaggccctg gcagatgtgg 1500
ccacggtgct gggacgtgct ctgtatgagc ttgcaggagg aaccaacttc agcgacacag 1560
ttcaggctga tccccaaacg gttacccgcc tgctctatgg gttcctgatt aaagccaaca 1620
actcatggtt ccagtctatc ctcaggcagg acctaaggtc ctacttgggt gacgggcctc 1680
ttcaacatta catcgctgtc tccagcccca ccaacaccac ttatgttgta cagtatgcct 1740
tggcaaattt gactggcaca gtggtcaacc tcacccgaga gcagtgccag gatccaagta 1800
aagtcccaag tgaaaacaag gatctgtatg agtactcatg ggtccagggc cctttgcatt 1860
ctaatgagac ggaccgactc cccggtgtgg tgcgttctac tgcacgatta gccagggcct 1920
tgtctcctgc ctttgaactg agtcagtgga gctctactga atactctaca tggactgaga 1980
gccgctggaa agatatccgt gcccggatat ttctcatcgc cagcaaagag cttgagttga 2040
tcaccctgac agtgggcttc ggcatcctca tcttctccct catcgtcacc tactgcatca 2100
atgccaaagc tgatgtcctt ttcattgctc cccgggagcc aggagctgtg tcatactgag 2160
gaggacccca gcttttcttg ccagctcagc agttcacttc ctagagcatc tgtcccactg 2220
ggacacaacc actaatttgt cactggaacc tccctgggcc tgtctcagat tgggattaac 2280
ataaaagagt ggaactatcc aaaagagaca gggagaaata aataaattgc ctcccttcct 2340
ccgctcccct ttcccatcac cccttcccca tttcctcttc cttctctact catgccagat 2400
tttgggatta caaatagaag cttcttgctc ctgtttaact ccctagttac ccaccctaat 2460
ttgcccttca ggacccttct acttttcct tcctgccctg tacctctctc tgctcctcac 2520
ccccacccct gtacccagcc accttcctga ctgggaagga cataaaaggt ttaatgtcag 2580
ggtcaaacta cattgagccc ctgaggacag gggcatctct gggctgagcc tactgtctcc 2640
ttcccactgt cctttctcca ggccctcaga tggcacatta gggtgggcgt gctgcgggtg 2700
ggtatcccac ctccagccca cagtgctcag ttgtactttt tattaagctg taatatctat 2760
ttttgttttt gtctttttcc tttattcttt ttgtaaatat atatataatg agtttcatta 2820
aaatagatta tcccaaaaaa aaaaaaaaaa aaaaaaaaaa aaaaa 2865

<210> 21
<211> 2513
<212> DNA
<213> Homo sapiens

<400> 21
gggtcgaccc acgcgtccgg ggtgtccgag ggccacaaga gtatgacggg gctgtacgag 60
ctggtgtggc gggtgctgca cgcgctgctc tgtctgcacc gcacgctcac ctcctggctc 120
cgcgttcggt tcggcacctg gaactggatc tggcggcgct gctgccgcgc cgcctctgcc 180
gcggtcctag cgccgctcgg cttcacgctc cgcaagcccc cggcagtcgg caggaaccgc 240
cgtcaccacc ggcacccgcg cggggggtcg tgcctggcag ccgcacacca ccggatgcgc 300
tggcgcgcgg acggtcgttc cttggagaag ctgcctgtgc atatgggcct ggtgatcacc 360
gaggtggagc aggaacccag cttctcggac atcgcgagcc tcgtggtgtg gtgtatggcc 420
gtgggcatct cctacattag cgtctacgac caccaaggta ttttcaaaag aaataattcc 480
agattgatgg atgaaatttt aaaacaacag caagaacttc tgggcctaga ttgttcaaaa 540
tactcaccag aatttgcaaa tagtaatgac aaagatgatc aagttttaaa ttgccatttg 600
gcagtgaagg tgctgtctcc ggaagatgga aaagcagata ttgtaagagc tgctcaggac 660
ttttgccagt tagtagccca gaagcaaaag agacccacag atttggatgt agatacgtta 720
gccagtttac ttagttcaaa tggttgtcct gatcctgatt tagtattgaa gttcggtcct 780
gtggacagca cattaggctt tcttccctgg cacatcagat tgactgagat tgtctctttg 840
ccttcccacc taaacatcag ttatgaggac tttttctctg cccttcgtca atatgcagcc 900

tgtgaacagc gtctgggaaa gtagtggtca ttggttgcat aatttgattt gaggcttgtg 960
gaggaaagga accaagtgac tctgatgttt acaaagcacc tatgaaaccc tgtacacacc 1020
tagttcataa tcctcataat ttatcaacaa acacaaaaaa gtgtcttact tgagagtgag 1080
tgtgtgtgtg tgcgtgtgca cgtgcacaca tgtgcacgtt tgtatgtatg gaaataaact 1140
tataaatggg gacgtattgg agaaggaaat acatagacct acaactttga gcaaatagca 1200
gtgatgtttt aggaactgaa atgtcacact taaagtcttc agcccagcta cttccctatt 1260
tttgtgggga gaagagggcc tgattagaac tgttctggtt gtgtttggcg ggaggggaat 1320
aatttttgtt cagtccttct tagtgaccaa actttaattt ttaagaataa tatattgact 1380
tactgaactg aagcattctg agttgaaagg agctccagag gagtggagtt ctgtgttgct 1440
cacatgttaa aatcttgctc accttcagag cagagggaat acctatcttc agatatccgt 1500
ccattttcat ctcttaattg tagtcaaaag tatgacttga gagtgttgct ctggtattct 1560
gggttctgaa gtctggtatt ctggtattct gggttcaaaa gtatgacttg agagtgttgc 1620
tctggtattc tgagagttgc tctgtattct gggttctgaa gattatttga aaaataactc 1680
ctactacatt gaaatgcaga cttaaaaatt taaacattgg attaggcagt caaaaaaacc 1740
aagcaagcat aaaaggtcaa taagttgtaa tcttgatagt aaaggtggaa aacttattat 1800
aaatggaaag aaagttttat ttcctttttt gtttgatggg cagtatgcca tattataccc 1860
aaagttcttt taaaaaatat ttccatcaac catttttatt taaaataaac atttgaggga 1920
agttaccaag gcagcttttt tcctcaaaag taacctgttc ctctttggaa tagcacattt 1980
taggggcatg gttaatacct gagattttta ctcagtaaat cctgatggtt actgtgtgta 2040
aaatatcttt aagtaggatt gaaggcctct gtgggggaat aaaatattac caaagtctat 2100
aaaaataaat tttacatgtt ctcttttatg acagagagca gcactggttc tgttattttt 2160
aaaatgaata attgatttct tgataggtgt ttaatatttc ttccctcact gctgattctt 2220
agatagaaac cattctttat atttgataga ctgctttcag aaaaccctta tcaacaagtg 2280
tacaatactt atctaaaact atacatttag aatggagcag tttaatacta gatctcagaa 2340
gttttgaaaa atagcaaaga agactggatt tggaaagcat ggtctacaat tggttgttaa 2400
attctgaagc tatgaagaat aaatgtttca actttggatt atgaaacccc atttatgatt 2460
ttttaaatac acttgaaata aaaatgatta aactaaaaaa aaaaaaaaaa aaa 2513

<210> 22
<211> 2185
<212> DNA
<213> Homo sapiens

<400> 22
ccacgcgtcc ggactcactg ctcttcatgg tggaggagcc tgggaggttc ctgcctctct 60
ggctgcacat tctcctaatt acggtgctgc tggtgctgtc gggcatattt tctggcctca 120
acctcgggct tatggccctg gaccccatgg agctgcgcat cgtgcagaac tgtggcaccg 180
agaaggagag gcgctatgcc cgcaagattg agcccatccg gcgcaagggc aactaccttc 240
tctgctcgtt gctcctaggg aacgtgctgg tcaacacctc cctcacaatc cttctagaca 300
acctcatcgg gtccggcctc atggcggtgg cctcctccac cattggcatt gtcatctttg 360
gggagatcct acctcaggcc ctgtgctccc gacatgggct ggctgtgggt gccaacacca 420
tccttctcac caaattcttt atgctactca ccttccccct cagttttccc attagcaagc 480
tcctggactt ttttctgggc caggagattc gcactgttta caaccgggag aagctgatgg 540
agatgttgaa ggtgacggag ccctataatg acctcgtgaa agaggagctc aatatgatcc 600
agggtgccct ggaactacgg accaaaactg tagaggatat catgacccag ctccaggact 660
gcttcatgat ccgcagcgat gccatcctgg acttcaacac catgtcggag ataatggaaa 720
gcggctatac tcgcatcccg gtgttcgaag acgagcagtc caatattgta gatattctct 780
acgtcaaaga cttggccttt gtggaccccg atgactgcac ccccctcaag actatcactc 840
gcttctataa ccacccggtg cactttgtct tccatgacac caagttggat gccatgctgg 900
aggagttcaa gaaggggaag tcccacctgg ccatcgtgca gaaggtaaac aacgagggtg 960
agggtgaccc cttctacgag gtcctgggcc tggtcaccct ggaggacgtg atcgaggaga 1020
tcatcaagtc ggagatcctg gacgagtccg acatgtacac tgacaaccga agccggaagc 1080
gggtgtctga gaagaacaag cgtgacttct ctgccttcaa ggatgcggac aatgagctca 1140
aagtgaaaat ctccccgcag ctcctcctgg ccgctcatcg cttcctagcc acagaggtct 1200
ctcagtttag cccctccctg atatcagaga agatcctgct gcggctactc aagtacccag 1260
atgtcattca ggaactcaag tttgacgagc acaataagta ctacgcccgc cattacctgt 1320
acacccgaaa taagccggcc gactacttca tcctcatcct gcaggggaag gtggaggtgg 1380
aggcagggaa ggagaacatg aagtttgaga cgggcgcctt ctcctactat gggactatgg 1440
ccctgacctc ggtcccctcc gaccgttccc cagcacaccc caccccactc agccgctcag 1500
cctccctcag ttacccagac cgcacagacg tctcaactgc agcaaccttg gcaggcagca 1560
gcaaccagtt tggcagctct gtcctgggcc agtacatctc tgacttcagc gtccgggcac 1620
tcgtggactt gcagtacatc aagatcactc ggcagcagta ccagaacggg ctgctggctt 1680
ctcgcatgga gaacagccct cagtttccca tagacgggtg caccacccac atggagaact 1740
tggccgagaa gtctgagctg cctgtggtgg acgagaccac aactcttctc aacgagcgta 1800
actccttgct gcacaaagcc tcccacgaga atgccatctg acaggagggc ccgggggccc 1860
ctgcccacct gcgggggcct ccccagtggg cccacatgaa gagagggaac ctgttagtcc 1920
agaaaggata cggatagata gcctgtctga ctgaacagcc agatggcccc cagcctatgg 1980
gggatctggc ctctgccagg gacctctgag tagctctgag gtggcactgt ccagccctgg 2040
ataggggggg cagtgggcca gctaccgtaa gcaaaggctg ttttttactg agagaatttc 2100
taaagtaggc tcatcacttt tttttaaata tcattttggg aagggaagac agggttaagg 2160

aactttattt aaaaaaaaaa aaaaa 2185

<210> 23
<211> 2512
<212> DNA
<213> Homo sapiens

<400> 23
ggcacgaggg ggatcaggga atgagggtct ccaccccagg ggtctccttg cagtgagtct 60
atatgcaggc ctgcgttctg ctcctggggc tggttctgag tgcccagctt cagtctcctg 120
agaacatgag gatgggaggg ggcagagtct tgctgagggc acacccagtt cccgctggag 180
gaggacagtg ccagtcttct gcaaagggac cttgggtggg aacgggcccg gagcgggagg 240
aacgtgactc cccagaggga agatgggcat catactgggc ccagagctgg gaaggagttg 300
ctgccagcac agggtgggcc tggactcccc tcgcccctac ccccagtggt tgtggctgta 360
gccctaagcc tggagagcag gaccggcccg gggtgtctgg gaggctgcca ggtgcctccc 420
agagctccca agggccccca cctgcaagtg ccagcctcag ggcagtgccc aaatgaggcc 480
ctctcagctg cagccagcga tgccttggga tgctcaccgg gagggaggcg gctttgggct 540
cctaagtcct tgggagaggc tgggagcagt cactgcgcgg cttgcgcaag cccattgtcg 600
ggttgggtgg cttcctcagc cagggctggg agggactcca ggatcaggtc ctccctgtct 660
cgagtctcag tggggtgatg gggaggagac ctggccaccc atggctcagg ggcagctgag 720
aacaaggacc tgctggagct ggaagtgctg tggtgttgag gggtggggtg ggcagcttct 780
cacacctgcc tcctgcctcc ttctgtccac ctttccacca ccctgacctg tcccagcccc 840
acacatggtt ctgcctggct ggcctgccct tggcacctgg cgtagagcac acagaaggca 900
ctcagctaat gctgggcagg cccactcatg gggagtgcgt ggctgtgcag caccagggaa 960
ccggcacagc agcgccggca gaaatcacag cagtaaactt gtccgggttg tatgcatcaa 1020
ggtggcgatg gacgtgggtc cccccactgc actgtggccc tgagcactgt atagcagccc 1080
ggcaatggga gccattatct tgcccctttg acagaggagg acacagaggc acagggaggt 1140
gaagtagctg ccccacacta gtgcctcctc gctcactcac caccccctgc accacagtgc 1200
agccgcttct cccaccagct ggggttcctt ggacccccaa gcctgggaag ggggaggtga 1260
gtttacaaaa tggaaagctt aaaaggagaa aagtggaacc agaggtttga gaagccctga 1320
gtggtagagt aaggcctcca gcgctgcctc tgggtgcagg gcagagtggc agaggagagg 1380
gggagaggca ctgggcacca tgggggccca gttcccactt cggggatctc tctcgcagaa 1440
ccgagggtcc ccttcatggg ggtagatgcc cagggctagc tgttgccact gtctgtgtgg 1500
acctgagtcc tggacatgcc cgagtgactc aggagtggct gcttgggcgg gctctgtcac 1560
cctaggatgt tatacattct gggaactgga caggagtggc tgcttgggcg ggctctggca 1620
ccctgggatg ttatacattc tgggaactgg acaggagtgg ctgcttgggt gggctctggc 1680
accctgggat gttatacatt ctgggaactg caatcagcca ctagagaagt cggagctaca 1740
ggaagtgacc ctggggtggg acctggggac atggccaggt cagcatgggg acacccggct 1800
ccagcaggag ctctggtctg tcctggggtc tttgggggca gggctgcggc cctgggcagg 1860
cttcctccag gcggaggtcc tggggaagtg ggggagccag gccagctgcc gcctccccca 1920
ctatgtagca tctgattcgt catctctcat gaaggcgatt tggttcataa ctctgaaact 1980
ctgaaaaagg tcaaaagaag cagagaggcc ctcggtggat atgccagctt ttctgccggt 2040
gctttctccc actactctgg gtggtctgct ctcctcttca aacctcagct cgcagggagg 2100
gcctgaatct gccagcccct caggatctcc ttccctctgg gccctcccca gccttaagga 2160
gcctcccaga cagaagggtg gacagagcca cctgggcagc ccgagagaca cacgggggtc 2220
ctccctgtgg acagccctgc cagcttccgc ccagccctga gcttcatttg catcttgagg 2280
agtaaggggt ggtgaaatgg gaatgctggt ctggctcagc tggtcgtggg cataagtgcc 2340
cgctgaatgg atggcatctc tccctcctgt cttatgttct ggggtccagg tgcttcccag 2400
ggccatgccc ctgctgctaa tgcttgccct aacccttacc ctaaccagcg tccagcgtcg 2460
tctcaccgag ccgtaaataa atcaacagat tcgcaaaaaa aaaaaaaaaa aa 2512

<210> 24
<211> 1996
<212> DNA
<213> Homo sapiens

<220>
<221> SITE
<222> (3)
<223> n equals a,t,g, or c

<220>
<221> SITE
<222> (5)
<223> n equals a,t,g, or c

<220>
<221> SITE
<222> (6)

<223> n equals a,t,g, or c

<220>
<221> SITE
<222> (16)
<223> n equals a,t,g, or c

<220>
<221> SITE
<222> (28)
<223> n equals a,t,g, or c

<220>
<221> SITE
<222> (967)
<223> n equals a,t,g, or c

<220>
<221> SITE
<222> (1874)
<223> n equals a,t,g, or c

<400> 24
tcnannggga aatgcnccct cactaaangg aacaaaagct ggagctccac cgcggtggcg 60
gccgctctag aactagtgga tcccccgggc tgcaggtttc caaacagaag ctgtattcca 120
aattctttcc taatgtggca tacaaagcct ttaggatctg gctcctgtgt gcctctcctg 180
ccgctgctgc tgctgctgct gctcctgttt ccgcttctgc catggccacc gccactgccg 240
ccgcctcctc cttcttccct gcaccccttc gcgcctgcct tccccgcgac cggcagcctg 300
agctctaaca attctcaact actagcacct cttagactcc aaaatgcttt gcacctattt 360
aaatgttttc ccgtgctctt ccctctacat aaaatcattt ctttccatcc tgagtaccca 420
tggcaagctc ctatcttcca atatttttac ttaagcatcc cctcttcttc tctgcacccg 480
gagcatcttg gacatagctt tgtttccacc ttgcattcac ccactagaca gtaagctttt 540
tcaagacaga tactgtctct tcttcatctc tatatctgta gmmcagggtt tctcagcgta 600
agtactgaag gtattttgga ctggacaatt ctttgtgtgc agctgtttta tgcatcatat 660
aggatgttta gcagaacccc tggcctctac ccgttagata gcagcacgcc cccttccctc 720
agatgcctcc aaatattgcc aaatgtcccc ttgtggacaa aattgtccca tttatttatt 780
gagagacgga ggctggatgt tgtcgcccag gctggagtgc agtagcgcga tctcagctca 840
ctgcaacctc tgcctcccag gttcaagcaa ttcttatacc tcagcctcct gagtagctgg 900
gattacaggt gtgtgccacc acacgcagct aattttgcat ttttagtaga gatggggttt 960
tgccatnttg cccaggctgg tctcaaattc ctggcctcaa gtgatccacc cacctcggcc 1020
tcccaaagtc ctgagattac aggtgtgagc cactgtgctc agccaattgt ccccatttaa 1080
gaaccactac cctggaaacc agcagtaacc agaccctggt gggtgctgag aaaatgttga 1140
attattcaga tagttgattc acattgaatt gtatacatca aatgtaagaa ttatgaatta 1200
catttcaata aaactttttt aaaaaaagta ttggtttact attagttatt attagtggca 1260
ttattatgag gtggtttcca tttgttttcc tcagtggcca ccagtgcaac tgattttgtc 1320
atatactgta ctcattgatg ccaaatatat atatcttatt ttatgctgac ttcaaacctg 1380
ggaagttgat ttgtttatct cttcttaact tttwggaaat gcagacacaa aaagattcag 1440
caaattgctt aaaatcgagg taactagcaa gcatatatca agggatacat gactcggctt 1500
ctgtctagtt tcaaagcctg tactcattcc atctgaccat gttgcttcca taagccacat 1560
gccagccaca taatgaattt cttgattaga atacaatgca tatacctacc aaagatagta 1620
tgtgcatata gcaaatatga gcaatttcta aataatggga gcattatttt tgttcaaaat 1680
gctaaaaact ggggccaggc gtggtggcac acgcctgtaa tcccagcact ttgggaggcc 1740
aaggtgggmr gatcacctga ggtcaggagt ttgagaccgg cctggccagc atggtgaagc 1800
cctgtctcta ctaaaaattc aaaagttagc tgggcgtggt ggcacacgcc tgtaatctta 1860
gctactctgg aggntgaggc aggaaaatca cttgaacccg gaaggcagag attgcagagc 1920
caagattgca ccagtgcatt ccagcctggg tgacagagca agactccatc tcaaaaaaaa 1980
aaaaaaaaaa ctcgag 1996

<210> 25
<211> 2616
<212> DNA
<213> Homo sapiens

<400> 25
aattcccggg tcgacccacg cgtccgcgga cagcgtcagc cccccaccct ctgtcatcac 60
ccaggaaaac ctaggcaggg tgaagcgtct cggatgcttt gaccatgctc agcggcagct 120
tggggagcgc tgtctgtatg tcttcccagc caaggtagag ccagccaaga ttgcctggga 180
atgtccagaa aaaaactgcg cccaggggga gggctctatg gccaagaagg tgaagcgcct 240
gttgaagaag caggttgtgc ggatcatgtc atgctcccca gacacccagt gttcccggga 300
ccattccatg gaagacccag acaagaaggg ggaatccaaa accaagagcg aagcggagtc 360

tgccagccct gaggagacgc ccgatggctc tgccagtcca gtggagatgc aggacgaggg 420
tgcagaggag ccccacgagg caggagagca gctgccccca ttcctgctaa aagaaggcag 480
agatgacagg ctgcactcgg cagagcagga cgcagatgat gaggctgctg atgacacgga 540
cgacaccagt tcggtgacct cctctgccag ctccaccact tcctcccaga gcggcagcgg 600
cacgagtcgc aaaaagagca tccccttgtc catcaagaac ttaaagcgca aacacaagag 660
gaagaagaat aaaatcactc gagacttcaa gccaggggac agggtggcag tggaggtggt 720
gaccacgatg acctcagccg acgtgatgtg gcaggatggc tccgtggaat gcaacatccg 780
ctccaacgac ctcttccctg tgcaccacct ggacaacaac gagttctgcc ctggagactt 840
cgtggtagat aagcgagtcc agagctgtcc agaccctgct gtctacggtg tggtacagtc 900
tggggaccac atcggccgta cctgcatggt gaagtggttc aagctgaggc cgagtgggga 960
cgacgtggag ctgattggag aagaggaaga tgtgagtgtt tacgacattg ctgaccaccc 1020
tgactttagg ttccgtacaa ctgacatcgt catccgcatc ggcaatactg aggatggggc 1080
tcctcacaag gaggatgagc catcggtggg ccaggtggcc cgtgtggacg tcagcagcaa 1140
ggtggaggtg gtgtgggctg acaactcaaa gaccatcatc ctgccccagc acttgtacaa 1200
catagagtct gagattgagg agtcagacta cgattcggta gaaggcagca ccagcggggc 1260
atcctcggat gaatgggaag atgatagtga cagctgggag acggacaatg ggctggtgga 1320
ggacgagcac cccaagatag aggagccccc catcccaccc ctggagcagc cggtggcccc 1380
tgaggacaag ggagtggtga tcagtgaaga ggcagccaca gctgccgtcc agggggctgt 1440
ggccatggct gcccccatgg ccgggctgat ggagaaggct ggcaaggacg ggcccaccaa 1500
gagcttccgg gagttgaaag aggccatcaa gatcctggag agcctcaaga acatgactgt 1560
ggagcagctg ctgacgggct cgcccacctc tccgactgtg gagcctgaga agccaactcg 1620
ggagaagaag tttctggatg acatcaagaa gctacaggaa aacctcaaga agaccctgga 1680
caatgtggcc attgtagagg aggagaagat ggaagcagtg cccgacgtag agcgcaagga 1740
ggacaagccc gaggggcagt cacctgtgaa ggctgagtgg cccagcgaaa ccccggtgct 1800
gtgccagcag tgtggcggca agcctggcgt caccttcacc agcgccaagg gcgaggtctt 1860
ctccgtactg gagtttgcac cctcaaatca ttcttttaag aaaattgagt tccagcctcc 1920
agaagccaag aagttcttca gcacagtgcg gaaggagatg gcgctgctgg ctacctcact 1980
gcctgagggc atcatggtca agacttttga agatagaatg gcaagtaggc ttggcatggg 2040
caggtgcaga tgtgctgtgt gggcacccag tgcccaagag gccaccaacc ttgggtcccc 2100
aaacctccgg ggctgactgg cacctgcgag gacactcacc cacccacctt ttacagtgac 2160
ggctcagggc tgtagtccca gctactctgg aggctgaggc aggagaatgg cgtgaacctg 2220
ggaggtggag cttgcagtga gccgagatca cgccactgca ctccagcctg ggtgacagag 2280
cgagattctg tctcaaaaaa aaaaattact ttgaaatagg ccgggtgcgg tggctcacga 2340
ctgtaatccc agcacttcgg gaagccgagg caggtggatc acctgaggtc agaagtttga 2400
gaccagcctg accaacccgg tgaaaccctg tctctactta aaaatacaaa aattagccgg 2460
atgtggtggc gggtgccagt aatcccagct actcgggaga ctgaggcagg agaatcactt 2520
gaacctggga ggcagaggtt gtagtgagcc aagatcgcac cattgcactc cagcctgggc 2580
aacagagcga gacttcatct caaaaaaaaa aaaaaa 2616

<210> 26
<211> 397
<212> DNA
<213> Homo sapiens

<220>
<221> SITE
<222> (322)
<223> n equals a,t,g, or c

<220>
<221> SITE
<222> (394)
<223> n equals a,t,g, or c

<220>
<221> SITE
<222> (397)
<223> n equals a,t,g, or c

<400> 26
gtttgcgagc ggctggaacc agacggtgcc gatagaggaa gcgggctcca tggctgccct 60
cctgctgctg cccctgctgc tgttgctacc gctgctgctg ctgaagctac acctctggcc 120
gcagttgcgc tggcttccgg cggacttggc ctttgcggtg cgagctctgt gctgcaaaag 180
ggctcttcga gctcgcgccc tggccgcggc tgccgccgac ccggaaggtc ccgagggggg 240
ctgcagcctg gcctggcgcc tcgcggaact ggcccagcag cgcgcggaac ttttattacg 300
gtcgcgcgct ttagctactc anaggcggag cgcgagagta acaggctgac gcgccttcct 360
acgtgcgcta ggctgggact ggggacccga cggnggn 397

<210> 27

<211> 635
<212> DNA
<213> Homo sapiens

<220>
<221> SITE
<222> (37)
<223> n equals a,t,g, or c

<220>
<221> SITE
<222> (539)
<223> n equals a,t,g, or c

<220>
<221> SITE
<222> (547)
<223> n equals a,t,g, or c

<220>
<221> SITE
<222> (628)
<223> n equals a,t,g, or c

<400> 27
cccgactctg attaccgccc gaattcccgg tcgaccncgc gtccgggagg acccgggagc 60
cggcggccgg agctgggagc gcggggaagg cggttggggt tctgacagct gcgcgcgatc 120
ctgctctctc tcagccgcct gtggacatgc gcaaagggcc ctctcctgag tccagatgat 180
gctcatacca atggcttcag tgatggcggt gactgaaccg aaatgggtct cggtctggag 240
ccgcttcctc tgggtgacgc tgctgagcat ggtgctgggg tccctgctgg ccctgctgct 300
gccgctgggg gctgtggagg agcagtgctt ggctgtgctc aaaggcctct acctgctcag 360
gagcaaaccg gacagggcgc aacatgccgc accaagtgca ccarcccgtc cacggagcty 420
arcatcacct ycaggggcgc gacgctkctt ggtggccaag accaaggcct tytccagcgg 480
ggtaaagttt gggaaagccc aagagcttgc ccttgaacca aggccctgga agattaaanc 540
cgcccanggg caaagccggg gaaaaaaagc ccaaaaaagc tctttcaatg caccgccctt 600
taaggaatgg gaccccggga actttccngg ggacc 635

<210> 28
<211> 2543
<212> DNA
<213> Homo sapiens

<220>
<221> SITE
<222> (524)
<223> n equals a,t,g, or c

<220>
<221> SITE
<222> (2539)
<223> n equals a,t,g, or c

<400> 28
ggcacgagcg ttccagatgt cattgtgctc atccagtaat gttgcggcag gtgtctcagg 60
aggactgaaa ggccttcact cctagaaaca ttagtggtaa tcccttcatg tcatcaactg 120
tccactcaaa tttccacctt cctacaaata tcagctctcc tccctgtaaa ttattggttg 180
gtaagcctct taagtcttaa aaggtaggtt cccctccatc tcttttttat acaaaaccaa 240
ctttttacta ggctttactt ctgaattaaa ttcaagcggc ggctattgac agcagcaaga 300
acctttgctg tcccacgcgt tcctcctgca gtgttctctg tgatgctgct tggaaggctg 360
atggactgtt cagctggtgt gatgatttgg gtagctcaga gccctcatgg ttggggacct 420
gacacatgaa gagccctgaa tatcactatg ggtgagtgtg gccttacttt tgctgcttca 480
cattaacggt ttggctttgc tgcagccagt attctgacct taangtcaca cttgcagatg 540
tcagcccagg cttggtacag gggcaaacct tttcaggctt gagcagaacc cagttaggtt 600
ccgggtgttg actgttctag aaatggattt ccaggactac atgagtgggt tcgtgtgttt 660
tccccagaaa atccagaact gatgataaat agaaatgtga aaaactactt cagttacttg 720
actgtttgtt agtaaaaaaa aatcagtaat tcatccttgc ccacctaggt aggcggttta 780
tccaacttct gggtctcttg cccatggaaa tcaccatctt ggatctgttg ctctcccaga 840
acagtattat gaatagggct aaaggtcaga atacccccac tgcccttatg acagatggga 900
acatctccca aataagacag tctcaagcat tcatgtaata aaataacttt attagaatca 960
ggaatttgtt aaaataatat ccttcacata tagctaaagt gcaaattctc ttgctctgta 1020

gtagaagcaa gcacatctgt cagacctccg tggtgtgctt ataaaaccaa gacgagaaga 1080
gttttaagaa gtacctctgg cagcataaga gtacgaaaac acattttata aaggtttgaa 1140
caattatcat ttggagccat ttcatatatc gtgtatgtct tttcacttat ttttcttaac 1200
ctttcaaaaa ctactctgtg ctatttatac aactgaagaa ctgtataaac tactttagag 1260
acaaggctta acttagtttt tttttttttt taaaactaaa acatttggag gtaaagaatg 1320
tttctccttt tgctttagga agtgactagg tttctagaaa tgctgtttct ttttttttcc 1380
tacctcagga cagcctttgt cactgaggat ttctagggtt agggcttcac aggaagcgtc 1440
tccttgaacc cagagtggtt gggttgggct tctggcagtg ccaccgagta ctcagttgtg 1500
gcaaaaagca gggtgtccag ggtggtctca gtacacttgg cgatgaagcg aatgaaaggc 1560
ctcacgtcgc cctcgttggc agcttccaac acgtggtagt agtcggaccg ctgctccttg 1620
cggatggtga tgggcgggta gcccgcctgc atgaggatga ggttcatgag cagacgggag 1680
gtcctcccgt tgccatcaat gaaagggtgg atgtaaacga gtttataatg ggctaaggct 1740
gcaaactcca ctgggtgcag gttcatggct tcctcggagt tgagccactg tacaaactcc 1800
tgcatctgct tttccacatc ctgcggatgg ggagggatgt ggtgtccgac caggacctgt 1860
gttgtccgaa acctgccggc ttccacgggg tccacgtagc ccagcacccg cctgtggatc 1920
tccagcacgt cgctgatggt gacggagccg atgcgcgaaa ccagagtcgt gttgatgtac 1980
ttcatggctg catgcatgcc tatgacctcg ttctgctcct ccaggctctt cccgggcacg 2040
gcgtagcggg tctccaggat gtgcctgatt tccgagaggg tgagggtgtt gccctcgatg 2100
gccactgtgt ggtagatgtg atggtagtag gtctcctcca tgaccctgcg cagagctgag 2160
ttccccttgg ggatggacat gaccttcttc actttgctgt cgatgatgct gaaatacctc 2220
tggtcgatct cttccacaag aggcagtgtc cgatcgcggt tgaccagtgc tttctcatgg 2280
tagggtgaga tggtcaatgc tctggtgtac aagtagtccg cctggatgat gtccttgtct 2340
tcttccgaga agatgccaaa ctcggtgagc gcgtccacga agtccgggtc catcttgagg 2400
gcgtgcatga agagcttttg ggctttttcc cgcttgccct ggcgcttcaw ctccagggcc 2460
tggttcaggg cagctctggc ttccaactta cccgsgggag wggcctaawc aagccagaga 2520
gtgtcctcta ttctggggnc ttt 2543

<210> 29
<211> 2502
<212> DNA
<213> Homo sapiens

<400> 29
ggcacgagag agcctggggc cagagggcca gacagccaca gagctcctgg cgtgggcaag 60
gctggccaag gatggcgacg cccaggggcc tgggggccct gctcctgctc ctcctgctcc 120
cgacctcagg tcaggaaaag cccaccgaag ggccaagaaa cacctgcctg gggagcaaca 180
acatgtacga catcttcaac ttgaatgaca aggctttgtg cttcaccaag tgcaggcagt 240
cgggcagcga ctcctgcaat gtggaaaact tgcagaggtt ccgaggcagg tgatgaagga 300
cgaggacaag ccccctgaca gagtgcgact tcccaagagc ctttttcgat ccctgccagg 360
caacaggtct gtggtccgct tggccgtcac cattctggac attggtccag ggactctctt 420
caagggcccc cggctcggcc tgggagatgg cagcggcgtg ttgaacaatc gcctggtggg 480
tttgagtgtg ggacaaatgc atgtcaccaa gctggctgag cctctggaga tcgtcttctc 540
tcaccagcga ccgcccccta acatgaccct cacctgtgta ttctgggatg tgactaaagg 600
gaccgtgtgc tcctgagacc caccttggac cagtccacgg tgcatatcct cacacgcatc 660
tcccaggcgg gctgtggggt ctccatgatc ttcctggcct tcaccattat tctttatgcc 720
tttctgaggc ctgcccgccc tgatggtcat cggcactggg agtgccaaca gctacggcct 780
ctacaccatc cgtgataggg agaaccgcac ctctctggag ctatgctggt tccgtgaagg 840
gacaaccatg tacgccctct atatcaccgt ccacggctac ttcctcatca ccttcctctt 900
tggcatggtg gtcctggccc tggtggtctg gaagatcttc accctgtccc gtgctacagc 960
ggtcaaggag cgggggaaga accggaagaa ggtgctcacc ctgctgggcc tctcgagcct 1020
ggtgggtgtg acatgggggt tggccatctt caccccgttg ggcctctcca ccgtctacat 1080
ctttgcactt ttcaactcct tgcaagccca gagaggcatc acagtctgac tgtgagagaa 1140
acagccaaga caggagtgac gagactcaac ctgttcaggg aagtcactag aaacccaggc 1200
gtcctagatg cagcgggtat aagcccccaa gaccaggact ggctcccagc gccccatgag 1260
agatgtgtgg cttagtggct agggccaggc agccctgggt ccaaatccta gccccatccc 1320
tgacccagca agtcactcag ctccttccgt cctcattcat cgcgaaattg ggataatccc 1380
gtacttatct caccagcttt tttttttttt ttaattgaga tggagtcttg ctctgtcacc 1440
caggctggag tgcagtggca cgatctcagc tcactgcgac ctccacctcc tgggttcaag 1500
taattcttct ggtttcagcc tcctgagtag ctggaactac aggcacatgc caccatgccc 1560
agctaatttt tgaattttta gtagaaatgg ggtttcacca tactggtcag gctggtctca 1620
aactcctgac ctcagatgat ctacctgcct cagcctccga aagtgctggg attacaggcg 1680
tgagccactg tgcctggcct gccagatttc acaatgaaca atgacaatgc atatgtggga 1740
actactaggt cttcaatatg tgggaactat attaataatc atagaaatta tgactgtaag 1800
gccatctgag gctgtctcca ggtggagaat catgagtcca tgcctggaga atcccagggg 1860
ttgatggttg gggagaaatg aactttgaaa ctatagctga catcgttatt ctttcagagg 1920
ttaccttaat atgtaagcct gcacacttca ctctactaag tttccactgg gtctgagtta 1980
ttctgtattt ctctcttcct ctccaataca acagagccct tggtatactt gaattgccca 2040
ttgaactctt ctcaatattt gtcttgcata tagaggttta gttacagatt tccttgaaac 2100
atagagtttt tctaagtcat gttctgtact tactataatt tctttcaccc cacactttcc 2160
cttttcctct tgctggagtg cctcccataa taatccttta tagagggagt gatggttgtt 2220

ttgtatgtct gaaaattcct ctattttgct ctttcttttc tgtaataggc ataggtagat 2280
attttatttg aaggtctctg tttttcattt ccaagatttc gatttggttc ttttttattt 2340
catttttgaa atggttgtga aatggttgca tttttgcctt tcccagcagt tcttgagtgg 2400
ttacggtggg tctcctccct ctcagcagcc accagcccag gctgccccac tctctgccca 2460
cccctttcca ggctcaccga ggcctctcct tcctaacagg tg 2502

<210> 30
<211> 838
<212> DNA
<213> Homo sapiens

<400> 30
tgccccatcg tttttytyts accagcgtac gcccctaaca tgaccctcac ctgtgtattc 60
tgggatgtga ctaaagggac cgtgtgctcc tgagacccac cttggaccag tccacggtgc 120
atatcctcac acgcatctcc caggcgggct gtggggtctc catgatcttc ctggccttca 180
ccattattct ttatgccttt ctgaggcctg cccgccctga tggtcatcgg cactgggagt 240
gccaacagct acggcctcta caccatccgt gatagggaga accgcacctc tctggagcta 300
tgctggttcc gtgaagggac aaccatgtac gccctctata tcaccgtcca cggctacttc 360
ctcatcacct tcctctttgg catggtggtc ctggccctgg tggtctggaa gatcttcacc 420
ctgtmccgtg ctacagcggt caaggagcgg gggaagaacc ggaagaaggt gctcaccctg 480
ctgggcctct cgagcctggt gggtgtgaca tgggggttgg ccatcttcac cccgttgggc 540
ctytccaccg kctacatctt tgcacttttc aactccttgc aagcccagag aggcatcaca 600
gtctgactgt gagagaaaca gccaagacag gagtgacgag actcaacctg ttcagggaag 660
tcactagaaa cccaggcgtc ctagatgcag cgggtataag cccccaagac caggactggc 720
tcccagcgcc ccatgagaga tgtgtggctt agtggctagg gccaggcagc cctgggtcca 780
aatcctagcc ccatccctga cccagcaagt cactcagctc cttccgtcct cattcatc 838

<210> 31
<211> 1091
<212> DNA
<213> Homo sapiens

<400> 31
gcctgakccc ccagaatggg cacctgggca gacaccttct ctctgctctt ggctcttgcc 60
gttgccctct acttgggcta ctactgggca tgtgtgcttc agtgacatcc tccaaacacc 120
agatggaggc cagctcctgc tagactgggc caagcagcct gacagcagcc aagaccctga 180
tcctactacc cagcccattg tgctgctgct tcctggcatc actggcagta gccaggagac 240
atacgtcttg cacctagtta accaagctct gagggatggc taccaggctg tcgtgtttaa 300
caaccggggc tgccgtgggg aggaactgcg gacccacagg gctttttgtg ccagcaatac 360
tgaagatcta gagacagtcg tgaaccacat aaagcatcgt tatccccaag ctccactgct 420
ggccgtgggc atctcttttg gagggatact ggtgctgaat cacctggcac aggccaggca 480
ggctgcaggg ctggtggcag cactgactct gtctgcatgc tgggattcct ttgagaccac 540
tcgctccctg gaaaccccac tcaactcact gctcttcaat cagcccctca ctgctgggct 600
ctgccaactt gtggaacgcc tatcctatgg taagacctgc aggcccgtac aatccgccag 660
tttgatgagc gctacacatc tgtggccttt ggatatcaag actgtgttac ctactacaaa 720
gcagcaagcc ctagaaccaa gatagatgcc atccggatcc ctgtgctcta tctcagtgca 780
gcagatgacc ccttctcccc cgtctgtgcc cttcccatac aggccgccca acactccccc 840
tacgttgcgc tgctcatcac agcccggggt ggccacatcg gcttcctgga agggctgctc 900
ccgtggcagc actggtacat gagccgcctc ttgcatcagt acgccaaagc catcttccag 960
gacccagagg ggctgcctga cctcagggct ctcttacctt ctgaggacag aaacagctga 1020
caagagtacc atttggggtc tcagttcact ctttccttgt ttattaaata tcaacttttc 1080
ctgcctaaaa a 1091

<210> 32
<211> 819
<212> DNA
<213> Homo sapiens

<220>
<221> SITE
<222> (712)
<223> n equals a,t,g, or c

<220>
<221> SITE
<222> (758)
<223> n equals a,t,g, or c

<220>
<221> SITE
<222> (761)
<223> n equals a,t,g, or c

<220>
<221> SITE
<222> (807)
<223> n equals a,t,g, or c

<400> 32
gcctagcagc acacagacag cgaagcgggt gaccatgggg tggccacggc cgggccgagc 60
cctcgtggca gtcaaagcct tgctggtctt gtcgctgctc caggtgcccg cgcaggcagt 120
ggtacgggcc gtgctggaag acaactcgag ctcggtggac tttgcggatc tgccggcgct 180
gttcggcgtc cccctggccc ccgagggcat acggggctac ctgatggagg tcaagccagc 240
caacgcgtgc catcccatcg aggccccgcg actgggcaac cgctctctgg gcgccatcgt 300
gctgatccgc cgctacgact gcaccttcga cctcaaggtg ctgaacgccc agcgcgccgg 360
cttcgaggcg gccatcgtgc acaacgtcca ctccgacgac ctcgtgagca tgacccacgt 420
ctacgaggac ttgaggggcc agatcgccat cccctcagtg ktcgtgagcg aggccgctcg 480
caggacctgc gggtcatcct gggctgcaac aagtcggccc acgcgctgtc ctgccgacga 540
cccaccgtgc cacgacctgg ctgtcacccc gtgctgaccg tgtctgggtg ctgggctgta 600
ccctggcctg gtcgtatagc cttctttgtc tgaaccacct gtggctctgg gcccaggcct 660
gttcagcaca gacggccggt gagacgtcta ctgcagaagg ccagtccgac cntacgtgga 720
caacgactgg tgcattgctg atagatgaga gggacaanta ntctgctgtc aactacatga 780
atgatgacct gtttcagccc cgggttntgc gggaaaagg 819

<210> 33
<211> 677
<212> DNA
<213> Homo sapiens

<220>
<221> SITE
<222> (614)
<223> n equals a,t,g, or c

<220>
<221> SITE
<222> (676)
<223> n equals a,t,g, or c

<220>
<221> SITE
<222> (677)
<223> n equals a,t,g, or c

<400> 33
tcgagttttt tttttttttt ttttttccgc tttcaaaccc actttattct tttcagacca 60
gaaacattcc tcattgcacc cccctccctg ccctagatct ttactgacca ggggcctcag 120
gaggggctga ggagacagtg gtatactctg cctccaggga cgtggtgctg cagtggcagt 180
ggggactggc gcggcccagc agctccagcc tccgggagcg tactggactt gaatggccca 240
gatggggggc cggtggcccg gtagggaggg gtcctcgtcc ctgaagctgt aggtggtgga 300
gtcaaagctg tcttctgtgg cggccaccga ctgtttgcac acggggcagg agcgccgggg 360
ggcttgggag aaccaggggt caatgcattt gcagtggtag gtgtgggagc agggcaggat 420
cttgagttgg tcgccctcct catactcatc caggcagatg gcacacaggt cgttgtgcca 480
cgtgaaggtg cggacctggg ccttctggca ggtagacgtc ttcaccggcc gtctgtggct 540
kcagcaggcc tgggcccaga gccacaggtg gttcaggaca aagaaggctg atacgaccag 600
ggccagggta cagnccagca cccaggacac ggtcagcacg ggggtgacaa tcccaggtcg 660
ttggcacggt tgggtnn 677

<210> 34
<211> 2073
<212> DNA
<213> Homo sapiens

<220>
<221> SITE
<222> (14)
<223> n equals a,t,g, or c

<220>
<221> SITE
<222> (2037)
<223> n equals a,t,g, or c

<220>
<221> SITE
<222> (2061)
<223> n equals a,t,g, or c

<220>
<221> SITE
<222> (2066)
<223> n equals a,t,g, or c

<400> 34
ggaggctgtg gcgncactgc gcccggcgct cgcgtccgtc cgcccgtccg cccgcccagc 60
catgactgcg ccggtccccg cgccgcggat cctgttgccg ttgctgttgc tgctgctgct 120
aacgccgcct ccgggtgcac gtggtgaggt gtgtatggct tcccgtggac tcagcctctt 180
ccccgagtcc tgtccagatt tctgctgtgg tacctgtgat gaccaatact gctgctctga 240
cgtgctgaag aaatttgtgt ggagcgagga aaggtgtgct gtgcctgagg ccagcgtgcc 300
tgccagtgta gagccggtgg agcagctggg ctcggcgctg aggtttcgcc ctggctacaa 360
cgaccccatg tcagggttcg gagcgacctt ggccgttggc ctgaccatct ttgtgctgtc 420
tgtcgtcact atcatcatct gcttcacctg ctcctgctgc tgcctttaca agacgtgccg 480
ccgaccacgt ccggttgtca ccaccaccac atccaccact gtggtgcatg ccccttatcc 540
tcagcctcca agtgtgccgc ccagctaccc tggaccaagc taccagggct accacaccat 600
gccgcctcag ccagggatgc cagcagcacc ctacccaatg cagtacccac caccttaccc 660
agcccagccc atgggcccac cggcctacca cgagaccctg gctggaggag cagccgcgcc 720
ctaccccgcc agccagcctc cttacaaccc ggcctacatg gatgccccga aggcggccct 780
ctgagcattc cctggcctct ctggctgcca cttggttatg ttgtgtgtgt gcgtgagtgg 840
tgtgcaggcg cggttcctta cgccccatgt gtgctgtgtg tgtccaggca cggttcctta 900
cgccccatgt gtgctgtgtg tgtcctgcct gtatatgtgg cttcctctga tgctgacaag 960
gtggggaaca atccttgcca gagtgggctg ggaccagact ttgttctctt cctcacctga 1020
aattatgctt cctaaaatct caagccaaac tcaaagaatg gggtggtggg gggcaccctg 1080
tgaggtggcc cctgagaggt gggggcctct ccagggcaca tctggagttc ttctccagct 1140
taccctaggg tgaccaagta gggcctgtca caccagggtg gcgcagcttt ctgtgtgatg 1200
cagatgtgtc ctggtttcgg cagcgtagcc agctgctgct tgaggccatg gctcgtcccc 1260
ggagttgggg gtacccgttg cagagccagg gacatgatgc aggcgaagct tgggatctgg 1320
ccaagttgga ctttgatcct ttgggcagat gtcccattgc tccctggagc ctgtcatgcc 1380
tgttggggat caggcagcct cctgatgcca gaacacctca ggcagagccc tactcagctg 1440
tacctgtctg cctggactgt cccctgtccc cgcatctccc ctgggaccag ctggagggcc 1500
acatgcacac acagcctagc tgcccccagg gagctctgct gcccttgctg gccctgccct 1560
tcccacaggt gagcagggct cctgtccacc agcacactca gttctcttcc ctgcagtgtt 1620
ttcattttat tttagccaaa cattttgcct gttttctgtt tcaaacatga tagttgatat 1680
gagactgaaa cccctgggtt gtggagggaa attggctcag agatggacaa cctggcaact 1740
gtgagtccct gcttcccgac accagcctca tggaatatgc aacaactcct gtaccccagt 1800
ccacggtgtt ctggcagcag ggacacctgg gccaatgggc catctggacc aaaggtgggg 1860
tgtggggccc tggatggcag ctctggccca gacatgaata cctcgtgttc ctcctccctc 1920
tattactgtt tcaccagagc tgtcttagct caaatctgtt gtgtttctga gtctagggtc 1980
tgtacacttg tttataataa atgcaatcgt ttggaaaaaa aaaaaaaaaa aactcgnagg 2040
gggggccgct ctaaaaggat nccccnaagg ggg 2073

<210> 35
<211> 1929
<212> DNA
<213> Homo sapiens

<220>
<221> SITE
<222> (2)
<223> n equals a,t,g, or c

<220>
<221> SITE
<222> (792)
<223> n equals a,t,g, or c

<220>
<221> SITE

<222> (1927)
<223> n equals a,t,g, or c

<220>
<221> SITE
<222> (1928)
<223> n equals a,t,g, or c

<220>
<221> SITE
<222> (1929)
<223> n equals a,t,g, or c

<400> 35
anaattttgc aaaaagctat ttaggtgaca ctatagaagg tacgcctgca ggyaccggtc 60
cggaattccc gggtcgaccc acgcgtccgc aagatgtccc tgtggactcc caaactctac 120
tccagatggg gaggtgccct taacaccaag attttaaaag ctccaatttc agagcaagag 180
tcgaaaactc acagataaag ttatagttat ttcagggttc tgaaaagacg cagaacatga 240
agggactcag aagtctggca gcaacaacct tggctctttt cctggtgttt gttttcctgg 300
gaaactccag ctgcgctccg cagagactgt tggagagaag gaactggact cctcaagcta 360
tgctctacct gaaaggggca cagggtcgcc gcttcatctc cgaccagagc cggagaaagg 420
acctctccga ccggccactg ccggaaagac gaagcccaaa tccccaacta ctaactattc 480
cggaggcagc aaccatctta ctggcgtccc ttcagaaatc accagaagat gaagaaaaaa 540
actttgatca aaccagattc ctggaagaca gtctgcttaa ctggtgaaaa tatactggat 600
tatgtttaat tatggttcta ttctctttga aaacatgaac catgtgaata aaacctttgg 660
acccttttat tccatttgka atcttaagaa cacacacaga tagktttatt ctttcagaaa 720
caaaatatat ataggatgct tarctgagaa catcatcttc tttcattgct tcargtcctg 780
gttagatgac cnaaaaatgk tttcagatca ccttgkgkct tactcttgag tttcttasaa 840
tatttataat tataaggctg aagactaaag tgttctttcc ttttaactat agccagtacc 900
tgtcttgatc ttagttgtgt tttttttttc attttgttac ccacttgcat tttgttttca 960
ctcagcagaa attctccttc tctgttttcc ttttatccca tccccaagaa tgtggaagga 1020
aggtgagaaa catggcagga tgggaaatag gagagtatga ctctctatag ctcatccagg 1080
agtaatcaat taagaagata aattggatga ctgtggagaa gctctgtgat aggaacactt 1140
cagtgtggtt gctgagagga gacagtcatt gaggtagaag gtttgccara gatccagagc 1200
tcagagctcc ctttgtgctc tttgggaatt accttgcatt cagtttagaa acatggatct 1260
aaaagttact gggaaataag cagatggaga cacactctgt tgtttacgta ttggaagaag 1320
ggaacaagcc agttttgtta gaggtaacyc attttccatg accmaacaga ctcmacagat 1380
tcmagtactc tgcttactct aattgactag actctaggtt ttatttgaca tcatagcatt 1440
acataaatca ctctgataac ataagtgcac agtaatatgc ctgatctctt cctttttaaa 1500
agccaacttg agttcagtac catctgaata cacacacatg cacatatacc cacacacgca 1560
tacacacmta ctcctgtggc aaacataata atgtatttat ttagaattat aatatgacca 1620
tcatgttaat tattttttac ctaatcagag ttgttattga caaatgtcat aagtggaaag 1680
tattaattct tattgtcatc agtatttagc cattatttag tagctcaaga atatctttat 1740
gtgaatgtct ctgtaacttg gaattgcaat ttcactgtgt taagtaatca gaactctgct 1800
tataagattt atctgtatct tgtttcataa tttaataatg aaactaaatt caagttaatg 1860
taatgttgat ctccgtcgaa aaataacttg tgagcattaa aatatctgta tggcattaaa 1920
aaaaaannn 1929

<210> 36
<211> 790
<212> DNA
<213> Homo sapiens

<220>
<221> SITE
<222> (553)
<223> n equals a,t,g, or c

<220>
<221> SITE
<222> (649)
<223> n equals a,t,g, or c

<220>
<221> SITE
<222> (660)
<223> n equals a,t,g, or c

<220>
<221> SITE

<222> (668)
<223> n equals a,t,g, or c

<220>
<221> SITE
<222> (723)
<223> n equals a,t,g, or c

<400> 36
gggaagaagc cgagatggcg gcagccagcg ctggggcaac ccggctgctc ctgctcttgc 60
tgatggcggt agcagcgccc agtcgagccc ggggcagcgg ctgccgggcc gggactggtg 120
cgcgaggggc tggggcggaa ggtcgagagg gcgaggcctg tggcacggtg gggctgctgc 180
tggagcactc atttgagatc gatgacagtg ccaacttccg gaagcggggc tcactgctct 240
ggaaccagca ggatggtacc ttgtccctgt cacagcggca gctcagcgag gaggagcggg 300
gccgactccg ggatgtggca gccctgaatg gcctgtaccg ggtccggatc ccaaggcgac 360
ccggggccct ggatggcctg gaagctggtg gctatgtctc ctcctttgtc cctgcgtgct 420
ccctggtgga gtcgcacctg tcggaccagc tgaccctgca cgtggatgtg gccggcaacg 480
tggtgggcgt gtcggtggtg acgcacccca tggccccatk cagccccarg ggcttccccc 540
tgccatggag tanagccgag atcctggcca ctatccagtt ctgacctcgc atccccctac 600
cccgagccca tgcagtctgg gaacatgccg cctcttcttc agcctcttng ccttttgttn 660
caagtggnct taccccttctg gtccctggct gggctaagtg gtcctggtcc cggcttctgg 720
aanggccccc cttaactttt gacacttgga ctaaggatgc aaccctccct ttttggggcc 780
ccctttaagg 790

<210> 37
<211> 855
<212> DNA
<213> Homo sapiens

<400> 37
cgagatggyt ggcagccagc gctggggcaa cccggctgct cctgctcttg ctgatggcgg 60
tagcagcgcc cagtcgagcc cggggcagcg gctgccgggc cgggactggt gcgcgagggg 120
ctggggcgga aggtcgagag ggcgaggcct gtggcacggt ggggctgctg ctggagcact 180
catttgagat cctggccact atgccagttc tgacctcgca tccccctacc ccgagcccat 240
gcagtctggg aacatgccgc cttctctcca gcctctgtgc ctttgttcca ggtggtctca 300
ccctcctgtc cctggctggg ctaggtggtc ctgtccaggc tcctgcagcg cccccctcac 360
tttgacactg gactaggatg cagcctccct tctgtgtccc cttgagggta ccctgggtcc 420
cctcatcagg ggcagaggca tgaaagagtc ggggctggat ggccaggggc ttctgggccc 480
gacgcctagt gcagcccctg gggtcgtggt ttgacatttg tctgcctggt gcaaacaagg 540
aatccttgcc tttaaggtga caggccctcc acaggcttcc agacttgaag gaaaagggtt 600
taagaaagaa aacaaaacca acagttagtg gccggtgagg gcccaggctg gtcagcgtcc 660
cgtcttgcac acccaggggc ctccctttct gctggagtcc cctgtgtcct ccaccacccc 720
ccgccgccca gcatcctacc tggactgcgg tgctacgagg gcctgcgggc ctttgctgtg 780
tgccaccctc cctgtaagtc tatttaaaaa catcgacgat acattgaaaa aaaaaaaaaa 840
aaaaagggcg gccac 855

<210> 38
<211> 3030
<212> DNA
<213> Homo sapiens

<220>
<221> SITE
<222> (51)
<223> n equals a,t,g, or c

<220>
<221> SITE
<222> (3023)
<223> n equals a,t,g, or c

<220>
<221> SITE
<222> (3026)
<223> n equals a,t,g, or c

<220>
<221> SITE
<222> (3027)

<223> n equals a,t,g, or c

<400> 38
tgggaggtct atataagcag agctcgttta gtgaaccgtc agatcgcctg nagacgccat 60
ccacgctgtt ttgacctcca tagaagcacc gggaccgatc cagcctccgg actytagcct 120
aggccgcggg acggataaca atttcacaca ggaaacagct atgaccacta ggcttttgca 180
aaaagctatt taggtgacac tatagaaggt acgcctgcag gtaccggtcc ggaattcccg 240
ggtcgaccca cgcgtccggc cgacctgccg agatggaggc gaccggcacc tgggcgctgc 300
tgctggcgct ggcgctgctc ctgctgctga cgctggcgct gtccgggacc agggcccgag 360
gccacctgcc ccccgggccc acgccgctac cactgctggg aaacctcctg cagctacggc 420
ccggggcgct gtattcaggg ctcatgcggc tgagtaagaa gtacggaccg gtgttcacca 480
tctacctggg accctggcgg cctgtggtgg tcctggttgg gcaggaggct gtgcgggagg 540
ccctgggagg tcaggctgag gagttcagcg gccggggaac cgtagcgatg ctggaaggga 600
cttttgatgg ccatggggtt ttcttctcca acggggagcg gtggaggcag ctgaggaagt 660
ttaccatgct tgctctgcgg gacctgggca tggggaagcg agaaggcgag gagctgatcc 720
aggcggaggc ccggtgtctg gtggagacat tccaggggac agaaggacgc ccattcgatc 780
cctccctgct gctggcccag gccacctcca acgtagtctg ctccctcctc tttggcctcc 840
gcttctccta tgaggataag gagttccagg ccgtggtccg ggcagctggt ggtaccctgc 900
tgggagtcag ctcccagggg ggtcaggtga gtgggtggga cccctctcca actaccttcc 960
ctgaaggttc ctgccaaggt cccatgagaa ctagctgccc ttctccccac agacctacga 1020
gatgttctcc tggttcctgc ggcccctgcc aggcccccac aagcagctcc tccaccacgt 1080
cagcaccttg gctgccttca cagtccggca ggtgcagcag caccagggga acctggatgc 1140
ttcgggcccc gcacgtgacc ttgtcgatgc cttcctgctg aagatggcac aggaggaaca 1200
aaacccaggc acagaattca ccaacaagaa catgctgatg acagtcattt atttgctgtt 1260
tgctgggacg atgacggtca gcaccacggt cggctatacc ctcctgctcc tgatgaaata 1320
ccctcatgtc caaaagtggg tacgtgagga gctgaatcgg gagctggggg ctggccaggc 1380
accaagccta ggggaccgta cccgcctccc ttacaccgac gcggttctgc atgaggcgca 1440
gcggctgctg gcgctggtgc ccatgggaat accccgcacc ctcatgcgga ccacccgctt 1500
ccgagggtac accctgcccc agggcacgga ggtcttcccc ctccttggct ccatcctgca 1560
tgaccccaac atcttcaagc acccagaaga gttcaaccca gaccgtttcc tggatgcaga 1620
tggacggttc aggaagcatg aggcgttcct gcccttctcc ttagggaagc gtgtctgcct 1680
tggagagggc ctggcaaaag cggagctctt cctcttcttc accaccatcc tacaagcctt 1740
ctccctggag agcccgtgcc cgccggacac cctgagcctc aagcccaccg tcagtggcct 1800
tttcaacatt cccccagcct tccagctgca agtccgtccc actgaccttc actccaccac 1860
gcagaccaga tgaaggaagg caacttggaa gtggtgggtg cccgggacgg tgcctccagc 1920
ctcaacagtg ggcatggaca gggttaatgt ctccagagtg tacactgcag gcagccacat 1980
ttacacgcct gcagttgttt tccggagtct gtcccacggc ccacacgctc acttgactca 2040
tgctgctaag atgcacaacc gcacacccat acacaactac aagggccaca aagcaactgc 2100
tgggttagct ttccacagac ataaatatag tccatctgca atcacaagca catagccagg 2160
taacccacca actcccctgg atctgcagcc cacacgtggg agtctggctg tcaccttcac 2220
aagccacaga aacggccaca catgttcaca gctcacacgc cctctccatt catcgaactt 2280
ctcagtgtcc ctgtccctgg tgcctggcac agggaacagc atgcccctc cggggtcatg 2340
ccacccagag actgtcgctg tctatggccc caactcatgc tccctctctt ggctacacca 2400
ctctcccagc ctgtgaccac mgatgtccac acacccccaa ccacttgtcc acacagctac 2460
ccacgtacga catcgtcctg gctccccaga gtatcttccc actgagacac gccgccccca 2520
cagaggcaca gtccccagcc acctctgcaa ctgcagccct cagtcacccc tttttaagca 2580
ccctgattct accaaatgca aacacatctg ggtctgcgat tatgcacaga gactttggac 2640
atacgaggac cctcagaccg gaggaacacc tgcccaaccc caacacgtgc ttatgtaacc 2700
acgtggaaag cggcccctgc tgcccctcca cacacacata cacactcact gatctacagc 2760
ccctgttcgg cgtcagagtc cccactagac ccagtggaag ggttagaga ccaagtaggg 2820
gccagtttcc aattcaccct gtcagggagt gagccggatc tgacgttcct tgtgacttaa 2880
gggtccggct tgggaattaa agtttgtttc tggcctttag cctamaaaaa aaaaaaaaaa 2940
aaaaaaaaaa aaaaaaaaaa aaaaaaaaaa aaaaaaaaaa aaaaaaaaaa aaaaaaaaaa 3000
aaaaaaaaaa aaaaaaaaaa aanggnngcc 3030

<210> 39
<211> 788
<212> DNA
<213> Homo sapiens

<400> 39
   ccccctgcag gttgagatgt gtataagaga cacgcctcca ccacgtcagc accttggctg 60
   ccttcacagt ccggcaggtg cagcagcacc aggggaacct ggatgcttcg ggccccgcac 120
   gtgaccttgt cgatgccttc ctgctgaaga tggcacagga ggaacaaaac ccaggcacag 180
   aattcaccaa caagaacatg ctgatgacag tcatttattt gctgtttgct gggacgatga 240
   cggtcagcac cacggtcggc tataccctcc tgctcctgat gaaataccct catgtccaaa 300
   agtgggtacg tgaggagctg aatcgggagc tgggggctgg gcaggcacca agcctagggg 360
   accgtacccg ctcccttaca ccgacgcggt tctgcatgag gcgcagcggc tgctggcgct 420
   ggtgcccatg ggaatacccc gcaccctcat gcggaccacc cgcttccgag ggtacaccct 480
   gccccagggc acggaggtct tccccctcct tggctccatc ctgcatgacc ccaacatctt 540
   caagcaccca gaagagttca acccagaccg tytcctggat gcagatggac ggttcaggaa 600
   gcatgaggcg ttcctgcctt ctccttaagg aagcgtgtct gccttggaaa gggcctggca 660
   aaagcggagc tcttcctytt cttcaccacc atcctacaag ccttctccyt ggagagcccg 720
   tgcccgccgg acaccctgag cctyaagccc accgtcagtg gccttttcaa cattccccca 780
   gccttcca 788

<210> 40
<211> 1308
<212> DNA
<213> Homo sapiens

<400> 40
ttggtacgcc tgcaggtacc ggtccggaat tcccgggtcg acccacgmgk ccgaagatgg 60
ctacggcagg gggtggctct ggggctgacc cgggaagtcg ggtctccttt cgccttctgt 120
ctttctgcgt cctactagca ggtttgtgca ggggaaactc agtggagagg aagatatata 180
tccccttaaa taaaacagct ccctgtgttc gcctgctcaa cgccactcat cagattggct 240
gccagtcttc aattagtgga gacacagggg ttatccacgt agtagagaaa gaggaggacc 300
tacagtgggt attgactgat ggccccaacc ccccttacat ggttctgctg gagagcaagc 360
attttaccag ggatttaatg gagaagctga aagggagaac cagccgaatt gctggtcttg 420
cagtgtcctt gaccaagccc agtcctgcct caggcttctc tcctagtgta cagtgcccaa 480
atgatgggtt tggtgtttac tccaattcct atgggccaga gtttgctcac tgcagagaaa 540
tacagtggaa ttcgctgggc aatggtttgg cttatgaaga ctttagtttc cccatctttc 600
ttcttgaaga tgaaaatgaa accaaagtca tcaagcagtg ctatcaagat cacaacctga 660
gtcagaatgg ctcagcacca accttcccac tatgtgccat gcagctcttt tcacacatgc 720
atgctgtcat cagcactgcc acctgcatgc ggcgcagctc catccaaagc accttcagca 780
tcaacccaga aatcgtctgt gaccccctgt ctgattacaa tgtgtggagc atgctaaagc 840
ctataaatac aactgggaca ttaaagcctg acgacagggt tgtggttgct gccacccggc 900
tggatagtcg ttcctttttc tggaatgtgg ccccaggggc tgaaagcgca gtggcttcct 960
ttgtcaccca gctggctgct gctgaagctt tgcaaaaggc acctgatgtg accaccctgc 1020
cccgcaatgt catgtttgtc ttctttcaag gggaaacttt tgactacatt ggcagctcga 1080
ggatggtcta cgatatggag aagggcaagt ttcccgtgca gttagagaat gttgactcat 1140
ttgtggagct gggacaggtg gccttaagaa cttcattaga gctttggatg cacacagatc 1200
ctgtttctca gaaaaatgag tctgtacgga accaggtgga ggatctcctg gccacattgg 1260
agactgtctc ttatgcacat ctcaacctgc agggggggga agtgcttc 1308

<210> 41
<211> 2040
<212> DNA
<213> Homo sapiens

<220>
<221> SITE
<222> (2027)
<223> n equals a,t,g, or c

<400> 41
cggctgataa gtcgttcctt tttctggaat gttgccccca ggggctgaaa aggcagtggc 60
ttcctttgtc acccagctgg ctgctgctga agctttgcaa aaggcacctg atgtgaccac 120
cctgccccgc aatgtcatgt ttgtcttctt tcaaggggaa acttttgact acattggcag 180
ctcgaggatg gtctacgata tggagaaggg caagtttccc gtgcagttag agaatgttga 240
ctcatttgtg gagctgggac aggtggcctt aagaacttca ttagagcttt ggatgcacac 300
agatcctgtt tctcagaaaa atgagtctgt acggaaccag gtggaggatc tcctggccac 360
attggagaag agtggtgctg gtgtccctgc tgtcatcctc aggaggccaa atcagtccca 420
gcctctccca ccatcttccc tgcagcgatt tcttcgagct cgaaacatct ctggcgttgt 480
tctggctgac cactctggtg ccttccataa caaatattac cagagtattt acgacactgc 540
tgagaacatt aatgtgagct atcccgaatg gctgagccct gaagaggacc tgaactttgt 600
aacagacact gccaaggccc tggcagatgt ggccacggtg ctgggacgtg ctctgtatga 660
gcttgcagga ggaaccaact tcagcgacac agttcaggct gatccccaaa cggttacccg 720
cctgctctat gggttcctga ttaaagccaa caactcatgg ttccagtcta tcctcaggca 780
ggacctaagg tcytacttgg gtgacgggcc tcttcaacat tacatcgctg tctccagccc 840
caccaacacc acttatgttg tacagtatgc cttggcaaat ttgactggca cagtggtcaa 900
cctcacccga gagcagtgcc aggatccaag taaagtccca agtgaaaaca aggatctgta 960
tgagtactca tgggtccagg gccctttgca ttctaatgag acggaccgac tcccccggtg 1020
tgtgcgttct actgcacgat tagccagggc cttgtctcct gcctttgaac tgagtcagtg 1080
gagctctact gaatactcta catggactga gagccgctgg aaagatatcc gtgcccggat 1140
atttctcatc gccagcaaag agcttgagtt gatcaccctg acagtgggct tcggcatcct 1200
catcttctcc ctcatcgtca cctactgcat caatgccaaa gctgatgtcc ttttcattgc 1260
tccccgggag ccaggagctg tgtcatactg aggaggaccc cagcttttct tgccagctca 1320
gcagttcact tcctagagca tctgtcccac tgggacacaa ccactaattt gtcactggaa 1380

cctccctggg cctgtctcag attgggatta acataaaaga gtggaactat ccaaaagaga 1440
cagggagaaa taaataaatt gcctcccttc ctccgctccc ctttcccatc accccttccc 1500
catttcctct tccttctcta ctcatgccag attttgggat tacaaataga agcttcttgc 1560
tcctgtttaa ctccctagtt acccacccta atttgccctt caggaccctt ctactttttc 1620
cttcctgccc tgtacctctc tctgctcctc acccccaccc ctgtacccag ccaccttcct 1680
gactgggaag gacataaaag gtttaatgtc agggtcaaac tacattgagc ccctgaggac 1740
aggggcatct ctgggctgag cctactgtct ccttcccact gtcctttctc caggccctca 1800
gatggcacat tagggtgggc gtgctgcggg tgggtatccc acctccagcc cacagtgctc 1860
agttgtactt tttattaagc tgtaatatct attttgtttt ttgtcttttt cctttattct 1920
ttttgtaaat atatatataa tgagtttcat taaaatagat tatcccacaa aaaaaaaaaa 1980
aaaaaaaaaa aaaaaaaaaa aaaaaaggaa aaaagggggg aaaaaanaaa aaaaaagggg 2040

<210> 42
<211> 2620
<212> DNA
<213> Homo sapiens

<220>
<221> SITE
<222> (20)
<223> n equals a,t,g, or c

<220>
<221> SITE
<222> (26)
<223> n equals a,t,g, or c

<220>
<221> SITE
<222> (27)
<223> n equals a,t,g, or c

<220>
<221> SITE
<222> (2599)
<223> n equals a,t,g, or c

<220>
<221> SITE
<222> (2609)
<223> n equals a,t,g, or c

<400> 42
cggggggggg ggggaagatn aagcannaat ttacgtgaca ctatagaagg tacgcctgca 60
ggtaccggtc cggaattccc gggtcgaccc acgcgtccgg ggtgtccgag ggccacaaga 120
gtatgacggg gctgtacgag ctggtgtggc gggtgctgca cgcgctgctc tgtctgcacc 180
gcacgctcac ctcctggctc cgcgttcggt tcggcacctg gaactggatc tggcggcgct 240
gctgccgcgc cgcctctgcc gcggtcctag cgccgctcgg cttcacgctc cgcaagcccc 300
cggcagtcgg caggaaccgc cgtcaccacc ggcacccgcg cggggggtcg tgcctggcag 360
ccgcacacca ccggatgcgc tggcgcgcgg acggtcgttc cttggagaag ctgcctgtgc 420
atatgggcct ggtgatcacc gaggtggagc aggaacccag cttctcggac atcgcgagcc 480
tcgtggtgtg gtgtatggcc gtgggcatct cctacattag cgtctacgac caccaaggta 540
ttttcaaaag aaataattcc agattgatgg atgaaatttt aaaacaacag caagaacttc 600
tgggcctaga ttgttcaaaa tactcaccag aatttgcaaa tagtaatgac aaagatgatc 660
aagttttaaa ttgccatttg gcagtgaagg tgctgtctgc cggaagatgg aaaagcagat 720
attgtaagag ctgctcagga cttttgccag tkagtagccc agaagcaaar gagacccaca 780
gatttggatg tagatacgtt agccagttta cttagttcaa atggttgtcc tgatcctgat 840
ttagtaytga agttcggtcc tgtggacagc acaykaggct ttcttccctg gcacatcaga 900
ttgactgaga ttgtctcttt gccttcccay ctaaacatca gttatgagga ctttttctct 960
gcccttcgtc aatatgcagc ctgtgaacag cgtctgggaa agtagtggtc attggttgca 1020
taatttgatt tgaggcttgt ggaggaaagg aaccaagtga ctctgatgtt tacaaagcac 1080
ctatgaaacc ctgtacacac ctagttcata atcctcataa tttatcaaca aacacaaaaa 1140
agtgtcttac ttgagagtga gtgtgtgtgt gtgcgtgtgc acgtgcacac atgtgcacgt 1200
ttgtatgtat ggaaataaac ttataaatgg ggacgtattg gagaaggaaa tacatagacc 1260
tacaactttg agcaaatagc agtgatgttt taggaactga aatgtcacac ttaaagtctt 1320
cagcccagct acttccctat ttttgtgggg agaagagggc ctgattagaa ctgttctggt 1380
tgtgtttggc gggaggggaa taatttttgt tcagtccttc ttagtgacca aactttaatt 1440
tttaagaata atatattgac ttactgaact gaagcattct gagttgaaag gagctycaga 1500
ggagtggagt tctgtgttgc tcacatgtta aaatcttgct caccttcaga gcagagggaa 1560
tacctatctt cagatatccg tccattttca tctcttaatt gtagtcaaaa gtatgacttg 1620

agagtgttgc tctggtattc tgggttctga agtctggtat tctggtattc tgggttcaaa 1680
agtatgactt gagagtgttg ctctggtatt ctgagagttg ctctgtattc tgggttctga 1740
agattatttg aaaaataact cctactacat tgaaatgcag acttaaaaat ttaaacattg 1800
gattaggcag tcaaaaaaac caagcaagca taaaaggtca ataagttgta atcttgatag 1860
taaaggtgga aaactyatta taaatggaaa gaaagtttta tttccttttt tgtttgatgg 1920
gcagtatgcc atattatacc caaagttskt ttaaaaaata yttccatcaa cyatttttat 1980
ttaaaataaa catttgaggg aagttaccaa ggcagctttt ttcctcaaaa gtaacctgtt 2040
cctctttgga ayagcacatt ttaggggcat ggttaatacc tgagattttt actcagtaaa 2100
tcctgatggt tacygtgtgt aaaatatctt taagtaggat tgaaggcctc tgtgggggaa 2160
taaaatatta ccaaagtcta taaaaataaa ttttacatgt tctcttttat gacagagagc 2220
agcactggtt ctgttatttt taaaatgaat aattgatttc ttgataggtg tttaatattt 2280
cttccctcac tgctgattct tagatagaaa ccattcttta tatttgatag actgctttca 2340
gaaaaccctt atcaacaagt gtacaatact tatctaaaac tatacattta gaatggagca 2400
gtttaatact agatctcaga agttttgaaa aatagcaaag aagactggat ttggaaagca 2460
tggtctacaa ttggttgtta aattctgaag ctatgaagaa taaatgtttc aactttggat 2520
tatgaaaccc catttatgat tttttaaata cacttgaaat aaaaatgatt aaactaaaaa 2580
aaaaaaaaaa aaaaaattnc tgcggccgnc aagggaattc 2620

<210> 43
<211> 537
<212> DNA
<213> Homo sapiens

<220>
<221> SITE
<222> (399)
<223> n equals a,t,g, or c

<220>
<221> SITE
<222> (453)
<223> n equals a,t,g, or c

<220>
<221> SITE
<222> (496)
<223> n equals a,t,g, or c

<220>
<221> SITE
<222> (518)
<223> n equals a,t,g, or c

<220>
<221> SITE
<222> (532)
<223> n equals a,t,g, or c

<400> 43
ggactcactg ctcttcatgg tggaggagcc tgggaggttc ctgcctctct ggctgcacat 60
tctcctaatt acggtgctgc tggtgctgtc gggcatattt tctggcctca acctcgggct 120
tatggccctg gaccccatgg agctgcgcat cgtgcagaac tgtggcaccg agaaggagag 180
gcgctatgcc cgcaagattg agcccatccg gcgcaagggc aactaccttc tctgctcgtt 240
gctcctaggg aacgtgctgg tcaacaccty cctcacaatc cttctagaca acctcatcgg 300
gtccggcctc atggcggtgg cctcctccac cattggcatt gtcatctttg gggagatcct 360
acctcaggcc ctgtgctccc gacatgggct ggctgtggnt gccaacacca tccttctcac 420
caaattcttt atgctactca ccttccccct canttttccc attagcaagc tcctggactt 480
ttttctgggc cagganattc gcactgttta caaccggnag aagctgatgg anatgtt 537

<210> 44
<211> 4224
<212> DNA
<213> Homo sapiens

<220>
<221> SITE
<222> (3690)
<223> n equals a,t,g, or c

<220>
<221> SITE
<222> (3697)
<223> n equals a,t,g, or c

<220>
<221> SITE
<222> (4183)
<223> n equals a,t,g, or c

<400> 44
ctaactttct gggagtaaag cttcaggtgg aagaacaatt ggatcaaact tggaaaacat 60
gcccccagtt tgtctactcc attgaacttt gcatgcttgg agcccctggc ctcagcacac 120
tcaatcgcac aagccagcct gcagcacctg tacagcacca tgggagtccc ctgcagagct 180
gcaactttga gagtgggtat agtgacgtaa ctcaggttct tcaagggatg aacactccaa 240
ctttgaaacc mctagcctgt aggtgtggac gaaaaggcag ctgcatgtta taaaacaata 300
ttactcatac ttttggatca agcttctttc agtcccatgg gtagggagga ggggcaattt 360
gctgaagccc actgcccttc cagtactcac aagccaaggg csctatggga tctgtttcac 420
aggactcatg cttatggcag ctgagcacat ctgtcctgta tgtctgccag cagccacgtc 480
cctctcactc ctgtgacgac agccttgact atatttagaa attccatttc tgattgcatt 540
tcactgctga atggtcctag aatcctttat tgcccttgtc ccatcacaag agacagccag 600
ccacagccac ttttatctca acaacatact gaatactgac agaacaaaca ggcaagttgt 660
agattcacca ggatttatac atatttgatt ttttattacc aatcaaaaat aaattccata 720
tatcgtttag caaatatcat tgttttgtga caaaagacac aagagtcata acaacaaaac 780
tccccgagag tcaaactcat aacgccaaaa taaatcacaa aaatatacaa attaaaatat 840
tatgcaaaat aaatacggcg gctgtcacct gcctacccat ttggatgccc tttgcaaagg 900
tctcccttac gtggaagaca cagtgggtgg gccagttcca gggtatggct catcccagga 960
accagaggtt gaaataggaa gggaaaaatt gcactgggaa gaggaagtca tcagacaaac 1020
aatatttgga aataatgatg accctctgtg agaagggatg atcaatgggc cagggaagag 1080
gaggagggcc agccagttgg tggtaaccgt gtgcacagag gtcactgtgg aggtgtgtgc 1140
acctgcccct tttgcttcac atacccccac caatgtcttt tgctctacct ggcagtcagg 1200
gctgtcagga atatagctct gccagcttcc aaaaggactt gggagcaagc tgctcctgta 1260
caagactaag ggctttcccc cactagggaa caaaagtctg gtgtcttttt tccttacagt 1320
tgagaaccca tgggtgtcac caccttctct ccaggcttcc aggagtcagt tctatggcta 1380
ggagacctca gactggccag gggtaggcat acttggtgca agacaatccc tggtcctaag 1440
agttaggata ctctaggcac ctggagagca gggcacttgg ggtgaggaaa ggagtgaata 1500
aataataatc aggcggaagg cctgcagagt ttcacctgcc aggctctcgg gacccagctc 1560
ctgctgcacc cagtggaagg accaacatgg acyttggtcc cacagcctcc ccaaccctgg 1620
gaagcctgga gttttgcagc agggcctgat tcagccccag ggaagaggcg ccagggcagg 1680
aattcacatg agggcagagc tcttagtaca gtastactaa cttcagcaag ggcaggatga 1740
cctctcacgc taggatcaca cgtgtgagaa agaggggctg caagctgcat gcctttagga 1800
ggagccctcc tctcctgagg ttccataagt gggtggataa ggccaggtga gccttgccag 1860
ccacagagga gaggacataa agaacctgct tccgtggctt cccacatgtc ctcttgtctc 1920
cacccccaag agggactgaa gcttggggat tcgaaataag gggtctggga aaaaggcttc 1980
agtcaacaag tcagccttga ctgtcattgt ggggcggggg tggtactgac tgctaacaac 2040
gcagatgctg atgactgaca gttccttctg gaaccaaaag gaagaaccca gacaaatcac 2100
ctccaacaca gatgccctcc cgccgaggga gaacctttgg taaaagtgag ggcagggcct 2160
ggggagcagg ggtgagcaat caaaggcctg agaccctgcc taacacttga gtcagccctg 2220
tcacaaaggg ccagttgtcc aaagggccag atgggaggca gggtggggat gtgtcctcag 2280
ctgagtcccg actcaccagg agaggctgtc ggcagagttc tagatttctg ggtacagcag 2340
ttgacaacag atgtgctcct ctgcatagct cagattatca tgtccctgat cacagctacc 2400
caggagctcg tggcctcagc gttgtcagat aagctacagc gcaggtgctc aggcagctga 2460
tctccatgcc attttgttct ttgcttctgt gaagagctgt cttcctccca acagataagc 2520
ctccactggc aggctctggg atcccacctc cggaggagga ggaggaggag gaagggagag 2580
ctccctgaat cagggacaca agctggaagg ccatggctgg gaacagatta tgtccgttgc 2640
ttcccgggac aagaaaccct tcctctttta tagtttttag gaaaaaaata tttttttttt 2700
aaataaagtt ccttaaccct gtcttccctt cccaaaatga tatttaaaaa aaagtgatga 2760
gcctacttta gaaattctct cagtaaaaaa cagcctttgc ttacggtagc tggcccactg 2820
ccccccctat ccagggctgg acagtgccac ctcagagcta ctcagaggtc cctggcagag 2880
gccagatccc ccataggctg ggggccatct ggctgttcag tcagacaggc tatctatccg 2940
tatcctttct ggactaacag gttccctctc ttcatgtggg cccactgggg aggcccccgc 3000
aggtgggcag gggcccccgg gccctcctgt cagatggcat tctcgtggga ggctttgtgc 3060
agcaaggagt tacgctcgtt gagaagagtt gtggtctcgt ccaccacagg cagctcagac 3120
ttctcggcca agttctccat gtgggtggtg cacccgtcta tgggaaactg agggctgttc 3180
tccatgcgag aagccagcag cccgttctgg tactgctgcc gagtgatctt gatgtactgc 3240
aagtccacga gtgcccggac gctgaagtca gagatgtact ggcccaggac agagctgcca 3300
aactggttgc tgctgcctgc caaggttgct gcagttgaga cgtctgtgcg gtctgggtaa 3360
ctgagggagg ctgagcggct gagtggggtg gggtgtgctg gggaacggtc ggaggggacc 3420
gaggtcaggg ccatagtccc atagtaggag aaggcgcccg tctcaaactt catgttctcc 3480
ttccctgcct ccacctccac cttcccctgc aggatgagga tgaagtagtc ggccggctta 3540
tttcgggtgt acaggtaatg gcgggcgtag tacttattgt gctcgtcaaa cttgagttcc 3600

tgaatgacat ctgggtactt gagtagccgc agcaggatct tctctgatat cagggagggg 3660
ctaaactgag agacctctgt ggctaggaan cgatgancgg ccaggaggag ctgcggggag 3720
attttcactt tgagctcatt gtccgcatcc ttgaaggcag agaagtcacg cttgttcttc 3780
tcagacaccc gcttccggct tcggttgtca gtgtacatgt cggactcgtc caggatctcc 3840
gacttgatga tctcctcgat cacgtcctcc agggtgacca ggcccaggac ctcgtagaag 3900
gggtcaccct caccctcgtt gtttaccttc tgcacgatgg ccaggtggga cttcccagct 3960
ccagctgctt tttctcatgg ggaaaagcca ggggccccag agcagaccag ggactcccac 4020
cacccaagaa ggagtcctct gcatcttact caatgagtct cccaggaagg tccagcctct 4080
gagatcccag gggcccagga tgttcccgcg ctytcaggcc ctcccatggg agaggtgcgg 4140
gctggactga ctggaagcca caggcttggg ccctgggtcc acncctgcag atagctgagc 4200
accctgattt cttctcgtgg ccgg 4224

<210> 45
<211> 624
<212> DNA
<213> Homo sapiens

<220>
<221> SITE
<222> (6)
<223> n equals a,t,g, or c

<220>
<221> SITE
<222> (12)
<223> n equals a,t,g, or c

<220>
<221> SITE
<222> (13)
<223> n equals a,t,g, or c

<220>
<221> SITE
<222> (19)
<223> n equals a,t,g, or c

<220>
<221> SITE
<222> (54)
<223> n equals a,t,g, or c

<220>
<221> SITE
<222> (540)
<223> n equals a,t,g, or c

<220>
<221> SITE
<222> (583)
<223> n equals a,t,g, or c

<220>
<221> SITE
<222> (605)
<223> n equals a,t,g, or c

<220>
<221> SITE
<222> (619)
<223> n equals a,t,g, or c

<400> 45
accccnaacg gnnaacccnt cactataggt acagctggta cgcctgcagg tacncggtcc 60
ggaattcccg ggtcgaccca cgcgtccgga ctcactgctc ttcatggtgg aggagcctgg 120
gaggttcctg cctctctggc tgcacattct cctaattacg gtgctgctgg tgctgtcggg 180
catatttctt ggcctcaacc tcgggcttat ggccctggac cccatggagc tgcgcatcgt 240
gcagaactgt ggcaccgaga aggagaggcg ctatgcccgc aagattgagc ccatccggcg 300
caagggcaac taccttctct gctcgttgct cctagggaac gtgctggtca acacctccct 360
cacaatcctt ctagacaacc tcatcgggtc cggcctcatg gcggtggcct cctccaccat 420

tggcattgtc atctttgggg agatcctacc tcaggccctg tgctcccgac atgggctggc 480
tgtgggtgcc aacaccatcc ttctcaccaa attctttatg ctactcacct tccccctcan 540
ttttcccatt agcaagctcc tggacttttt tctgggccag ganattcgca ctgtttacaa 600
ccggnagaag ctgatggana tgtt 624

<210> 46
<211> 1543
<212> DNA
<213> Homo sapiens

<220>
<221> SITE
<222> (47)
<223> n equals a,t,g, or c

<400> 46
gagctccacc gcggtggcgg ccgctctaga actagtggat cccccgntct gcaggaattc 60
ggcacgaggg ggatcaggga atgagggtct ccaccccagg ggtctccttg cagtgagtct 120
atatgcaggc ctgcgttctg ctcctggggc tggttctgag tgcccagctt cagtctcctg 180
agaacatgag gatgggaggg ggcagagtct tgctgagggc acacccagtt cccgctggag 240
gaggacagtg ccagtcttct gcaaagggac cttgggtggg aacgggcccg gagcgggagg 300
aacgtgactc cccagaggga agatgggcat catactgggc ccagagctgg gaaggagttg 360
ctgccagcac agggtgggcc tggactcccc tcgcccctac ccccagtggt tgtggctgta 420
gcctaagcct ggagagcagg accggcccgg ggtgtctggg aggctgccag gtgcctccag 480
agctcccaag ggcccccacc tgcaagtgcc agcctcaggg cagtgcccaa atgaggccct 540
ctcagctgca gccagcgatg ccttgggatg ctcaccggga gggaggcggc tttgggctcc 600
taagtccttg ggagaggctg ggagcagtca ctgcgcggct tgcgcaagcc cattgtcggg 660
ttgggtggct tcctcagcca gggctgggag ggactccagg atcaggtcct ccctgtctcg 720
agtctcagtg gggtgatggg gaggagacct ggccacccat ggctcakggg cagctgagaa 780
caaggacctg ctggagctgg aagtgctgtg gtgttgaggg gtggggtggg cagcttctca 840
cacctgcctc ctgcctcctt ctgtccacct ttccascasc ctgacctgtc ccagccccac 900
acatggttct gcctggctgg cctgcccttg gcacctggcg tagagcacac agaaggcact 960
cagctaatgc tgggcaggcc cactcatggg gagtgcgtgg ctgtgcagca ccagggaacc 1020
ggcacagcag cgccggcaga aatcacagca gtaaacttgt ccgggttgta tgcatcaagg 1080
tkgcgatkga cgtgggtccc cccactgcac tgtggccctg agcactgtat agcagcccgg 1140
caatkggagc cattatcttg cccctttgac agaggaggac acagaggcac agggaggtga 1200
agtagctgcc ccacactagt gcctmctcgc tcactcacca cccsctgcac cacagtgcag 1260
ccgcttctcc caccagctkg ggttccttgg acccccaagc ctgggaaggg ggaggtragt 1320
ttacaaaatg gaaagcttaa aaggagaaaa gtggaaccag aggtttgaga agccctgagt 1380
ggtagagtaa ggcctccagc gctgcctctg ggtgcagggc agagtggcag aggagagggg 1440
gagaggcact gggcaccatg gggccagtk tcccacttcg ggatctctc tcgcagaacc 1500
gagggtcccc ttcatggggg ttagatgccc agggctagct gtt 1543

<210> 47
<211> 810
<212> DNA
<213> Homo sapiens

<220>
<221> SITE
<222> (681)
<223> n equals a,t,g, or c

<220>
<221> SITE
<222> (709)
<223> n equals a,t,g, or c

<220>
<221> SITE
<222> (753)
<223> n equals a,t,g, or c

<220>
<221> SITE
<222> (767)
<223> n equals a,t,g, or c

<220>

<221> SITE
<222> (781)
<223> n equals a,t,g, or c

<220>
<221> SITE
<222> (790)
<223> n equals a,t,g, or c

<400> 47
tcgagttttt tttttttttt tttgcgaatc tgttgattta tttacggctc ggtgagacga 60
cgctggacgc tggttagggt aagggttagg gcaagcatta gcagcagggg catggccctg 120
ggaagcacct ggaccccaga acataagaca ggagggagag atgccatcca ttcagcgggc 180
acttatgccc acgaccagct gagccagacc agcattccca tttcaccacc ccttactcct 240
caagatgcaa atgaagctca gggctgggcg gaagctggca gggctgtcca cagggaggac 300
ccccgtgtgt ctctcgggct gcccaggtgg ctctgtccac ccttctgtct gggaggctcc 360
ttaaggctgg ggagggccca gagggaagga gatcctgagg ggctggcaga ttcaggccct 420
ccctgcgagc tgaggtttga agaggagagc agaccaccca gagtagtggg agaaagcacc 480
ggcagaaaag ctggcatatc caccgagggc ctctctgctt cttttgacct ttttcagagt 540
ttcagagtta tgaaccaaat cgccttcatg agagatgacg aatcagatgc kacatagtgg 600
gggakgcggc actggcctgg sttcccccac ttccccasga cctccgcctg gaggaagcct 660
tgcccaaggc cgcagccctg ncccccaaag accccaggac agaccaganc tcctgcttgg 720
aacccggggt gttccccaat gcttgacctg ggncaatggt ccccaangtt cccaaacccc 780
nagggtcacn ttcctggtag cttccgaact 810

<210> 48
<211> 746
<212> DNA
<213> Homo sapiens

<220>
<221> SITE
<222> (3)
<223> n equals a,t,g, or c

<220>
<221> SITE
<222> (5)
<223> n equals a,t,g,. or c

<220>
<221> SITE
<222> (6)
<223> n equals a,t,g, or c

<220>
<221> SITE
<222> (16)
<223> n equals a,t,g, or c

<220>
<221> SITE
<222> (28)
<223> n equals a,t,g, or c

<220>
<221> SITE
<222> (668)
<223> n equals a,t,g, or c

<220>
<221> SITE
<222> (696)
<223> n equals a,t,g, or c

<220>
<221> SITE
<222> (697)
<223> n equals a,t,g, or c

<220>
<221> SITE
<222> (706)
<223> n equals a,t,g, or c

<220>
<221> SITE
<222> (742)
<223> n equals a,t,g, or c

<400> 48
tcnannggga aatgcnccct cactaaangg aacaaaagct ggagctccac cgcggtggcg 60
gccgctctag aactagtgga tcccccgggc tgcaggtttc caaacagaag ctgtattcca 120
aattctttcc taatgtggca tacaaagcct ttaggatctg gctcctgtgt gcctctcctg 180
ccgctgctgc tgctgctgct gctcctgttt ccgcttctgc catggccacc gccactgccg 240
ccgcctcctc cttcttccct gcaccccttc gcgcctgcct tccccgcgac cggcagcctg 300
agctctaaca attctcaact actagcacct cttagactcc aaaatgcttt gcacctattt 360
aaatgttttc ccgtgctctt ccctctacat aaaatcattt ctttccatcc tgagtaccca 420
tggcaagctc ctatcttcca atatttttac ttaagcatcc cctcttcttc tctgcacccg 480
gagcatcttg gacatagctt tgtttccacc ttgcattcac ccactagaca gtaagctttt 540
tcaagacaga tactgtctct tcttcatctc tatatctgta gaacagggtt tctcagcgta 600
agtactgaag gtattttgga ctggacaatt ctttgtgtgc aagctggttt atgcatcata 660
taggatgntt aagcagaacc cctggcctct acccgnnaga tagcancacg cccccttcct 720
caaatgcctc caaatattgc cnaatg 746

<210> 49
<211> 846
<212> DNA
<213> Homo sapiens

<220>
<221> SITE
<222> (123)
<223> n equals a,t,g, or c

<400> 49
ctcgagtttt tttttttttt ttttgagatg gagtcttgct ctgtcaccca ggctggaatg 60
cactggtgca atcttggctc tgcaatctct gccttccggg ttcaagtgat tttcctgcct 120
cancctccag agtagctaag attacaggcg tgtgccacca cgcccagcta acttttgaat 180
ttttagtaga gacagggctt caccatgctg gccaggccgg tctcaaactc ctgacctcag 240
gtgatyygcc caccttggcc tcccaaagtg ctgggattac aggcgtgtgc caccacgcct 300
ggccccagtt tttagcattt tgaacaaaaa taatgctccc attatttaga aattgctcat 360
atttgctata tgcacatact atctttggta ggtatatgca ttgtattcta atcaagaaat 420
tcattatgtg gctggcatgt ggcttatgga agcaacatgg tcagatggaa tgagtacagg 480
ctttgaaact agacagaagc cgagtcatgt atcccttgat atatgcttgc tagttacctc 540
gattttaagc aatttgctga atctttttgt gtctgcattt ccaaaaagtt aagaagagat 600
aaacaaatca acttcccagg tttgaagtca gcataaaata agatatatat atttgggcat 660
caatgagtac agtatatgac aaaatcagtt gcactggtgg ccactgaggr aaacaaatgg 720
raaccacctc ataataatgc cactaataat actaatagtw aaccaatact tttttaaaaa 780
agttttattg aaatgtaatc ataatyctac atttgaggat acattcaagt gaatcactac 840
cggata 846

<210> 50
<211> 804
<212> DNA
<213> Homo sapiens

<220>
<221> SITE
<222> (798)
<223> n equals a,t,g, or c

<400> 50
ccactaggct tttgcaaaaa gctatttagg tgacactatm gaakgtacgc ctgcaggtac 60
cggtccggaa ttcccgggtc gacccacgcg tccgcggaca gcgtcagccc cccaccctct 120
gtcatcaccc aggaaaacct aggcagggtg aagcgtctcg gatgctttga ccatgctcag 180
cggcagcttg gggagcgctg tctgtatgtc ttcccagcca aggtagagcc agccaagatt 240
gcctgggaat gtccagaaaa aaactgcgcc cagggggagg gctctatggc caagaaggtg 300
aagcgcctgt tgaagaagca ggttgtgcgg atcatgtcat gctccccaga cacccagtgt 360

tcccgggacc attccatgga agacccagac aagaaggggg aatccraaac caagagcgaa 420
gcggagtctg ccagccctga ggagacgccc gatggctctg ccagtccagt ggagatgcag 480
gacgagggtg cagaggagcc ccacgaggca ggagagcagc tgcccccatt cctgctaaaa 540
gaaggcagag atgacaggct gcactcggca gagcaggacg cagatgatga ggctgctgat 600
gacacggacg acaccagttc ggtgacctcc tctgccagct ccaccacttc ctcccagagc 660
ggcagcggca cgagtcgcaa aaagagcatc cccttgtcca tcaagaactt aaagcgcaaa 720
cacaagagga agaagaataa aatcactcga gacttcaagc caggggacag ggtggcagtg 780
gaggtggtga ccacgatnac ctca 804

<210> 51
<211> 846
<212> DNA
<213> Homo sapiens

<220>
<221> SITE
<222> (657)
<223> n equals a,t,g, or c

<400> 51
gcggccgccc tttttttttt tttttgagat gaagtctcgc tctgttgccc aggctggagt 60
gcaatggtgc gatcttggct cactacaacc tctgcctccc aggttcaagt gattctcctg 120
cctcagtctc ccgagtagct gggattactg gcacccgcca ccacatccgg ctaatttttg 180
tatttttaag tagagacagg gtttcaccgg gttggtcagg ctggtctcaa acttctgacc 240
tcaggtgatc cacctgcctc ggcttcccga agtgctggga ttacagtcgt gagccaccgc 300
acccggccta tttcaaagta attttttttt ttgagacaga atctcgctct gtcacccagg 360
ctggagtgca gtggcgtgat ctcggctcac tgcaagctcc acctcccagg ttcacgccat 420
tctcctgcct cagcctccag agtagctggg actacagccc tgagccgtca ctgtaaaagg 480
tgggtgggtg agtgtcctcg caggtgccag tcagccccgg aggtttgggg acccaaggtt 540
ggtggcctct tgggcactgg gtgcccacac agcacatctg cacctgccca tgccaagcct 600
acttgccatt ctatcttcaa aagtcttgac catgatgccc tcaggcagtg aggtaancca 660
gcagcggcat tccttccgca ctgtgctgaa gaacttcttg gcttytggar gctggaactc 720
aattttctta aaagaatgat ttgagggtgc aaactccagt acggagaaga cctccccttg 780
gcgctggtga aggtgacgcc agcttgccgc cacactgctg gcacagcacc ggggtttcgc 840
tgggcc 846

<210> 52
<211> 455
<212> PRT
<213> Homo sapiens

<400> 52
Met Ala Ala Leu Leu Leu Leu Pro Leu Leu Leu Leu Leu Pro Leu Leu
1               5                   10                  15

Leu Leu Lys Leu His Leu Trp Pro Gln Leu Arg Trp Leu Pro Ala Asp
            20                  25                  30

Leu Ala Phe Ala Val Arg Ala Leu Cys Cys Lys Arg Ala Leu Arg Ala
        35                  40                  45

Arg Ala Leu Ala Ala Ala Ala Ala Asp Pro Glu Gly Pro Glu Gly Pro
    50                  55                  60

Cys Ile Leu Ala Trp Arg Leu Ala Glu Leu Ala Gln Gln Arg Ala Arg
65                  70                  75                  80

Asn Phe Leu Leu Arg Ser Arg Ala Leu Ala Thr Gln Arg Arg Ser Ala
                85                  90                  95

Arg Val Thr Gly Leu Thr Arg Leu Pro Thr Cys Ala Arg Leu Gly Leu
            100                 105                 110

Gly Thr Arg Arg Arg Arg Gln Arg Arg Gly Glu Arg Trp Arg Arg Arg
        115                 120                 125

Ala Gly Ser Ala Gly Ser Arg Arg Cys Ser Gly Arg Lys Arg Arg Gly
    130                 135                 140

Val Cys Arg Arg Gly Arg Cys Arg Gln Arg Trp Arg Ser Arg Ala Pro
145                 150                 155                 160

Leu Ser Pro Gly Ala Thr Val Ala Leu Leu Leu Pro Ala Gly Pro Glu
                165                 170                 175

Phe Leu Trp Leu Trp Ile Gly Leu Ala Lys Ala Gly Leu Arg Thr Ala
            180                 185                 190

Phe Val Pro Thr Ala Leu Arg Arg Gly Pro Leu Leu His Cys Leu Arg
        195                 200                 205

Ser Cys Gly Ala Arg Ala Leu Val Leu Ala Pro Glu Phe Leu Glu Ser
    210                 215                 220

Leu Glu Pro Asp Leu Pro Ala Leu Arg Ala Met Gly Leu His Leu Trp
225                 230                 235                 240

Ala Ala Gly Pro Gly Thr His Pro Ala Gly Ile Ser Asp Leu Leu Ala
                245                 250                 255

Glu Val Ser Ala Glu Val Asp Gly Pro Val Pro Gly Tyr Leu Ser Ser
            260                 265                 270

Pro Gln Ser Ile Thr Asp Thr Cys Leu Tyr Ile Phe Thr Ser Gly Thr
        275                 280                 285

Thr Gly Leu Pro Lys Ala Ala Arg Ile Ser His Leu Lys Ile Leu Gln
    290                 295                 300

Cys Gln Gly Phe Tyr Gln Leu Cys Gly Val His Gln Glu Asp Val Ile
305                 310                 315                 320

Tyr Leu Ala Leu Pro Leu Tyr His Met Ser Gly Ser Leu Leu Gly Ile
                325                 330                 335

Val Gly Cys Met Gly Ile Gly Ala Thr Val Val Leu Lys Ser Lys Phe
            340                 345                 350

Ser Ala Gly Gln Phe Trp Glu Asp Cys Gln Gln His Arg Val Thr Val
        355                 360                 365

Phe Gln Tyr Ile Gly Glu Leu Cys Arg Tyr Leu Val Asn Gln Pro Pro
    370                 375                 380

Ser Lys Ala Glu Arg Gly His Lys Val Arg Leu Ala Val Gly Ser Gly
385                 390                 395                 400

Leu Arg Pro Asp Thr Trp Glu Arg Phe Val Arg Arg Phe Gly Pro Leu
                405                 410                 415

Gln Val Leu Glu Thr Tyr Gly Leu Thr Glu Gly Asn Val Pro Pro Ser
            420                 425                 430

Thr Thr Gln Asp Ser Gly Ala Leu Trp Gly Val Leu Pro Gly Phe Thr
        435                 440                 445

Ser Ile Ser Ser Pro Ser Pro
    450                 455


<210> 53
<211> 458
<212> PRT
<213> Homo sapiens

<400> 53
Met Met Leu Ile Pro Met Ala Ser Val Met Ala Val Thr Glu Pro Lys
  1               5                  10                  15

Trp Val Ser Val Trp Ser Arg Phe Leu Trp Val Thr Leu Leu Ser Met

            20                  25                  30

Val Leu Gly Ser Leu Leu Ala Leu Leu Leu Pro Leu Gly Ala Val Glu
        35                  40                  45

Glu Gln Cys Leu Ala Val Leu Lys Gly Leu Tyr Leu Leu Arg Ser Lys
    50                  55                  60

Pro Asp Arg Ala Gln His Ala Ala Thr Lys Cys Thr Ser Pro Ser Thr
65                  70                  75                  80

Glu Leu Ser Ile Thr Ser Arg Gly Ala Thr Leu Leu Val Ala Lys Thr
                85                  90                  95

Lys Ala Ser Pro Ala Gly Lys Leu Glu Ala Arg Ala Ala Leu Asn Gln
            100                 105                 110

Ala Leu Glu Met Lys Arg Gln Gly Lys Arg Glu Lys Ala Gln Lys Leu
        115                 120                 125

Phe Met His Ala Leu Lys Met Asp Pro Asp Phe Val Asp Ala Leu Thr
    130                 135                 140

Glu Phe Gly Ile Phe Ser Glu Glu Asp Lys Asp Ile Ile Gln Ala Asp
145                 150                 155                 160

Tyr Leu Tyr Thr Arg Ala Leu Thr Ile Ser Pro Tyr His Glu Lys Ala
                165                 170                 175

Leu Val Asn Arg Asp Arg Thr Leu Pro Leu Val Glu Glu Ile Asp Gln
            180                 185                 190

Arg Tyr Phe Ser Ile Ile Asp Ser Lys Val Lys Lys Val Met Ser Ile
        195                 200                 205

Pro Lys Gly Asn Ser Ala Leu Arg Arg Val Met Glu Glu Thr Tyr Tyr
    210                 215                 220

His His Ile Tyr His Thr Val Ala Ile Glu Gly Asn Thr Leu Thr Leu
225                 230                 235                 240

Ser Glu Ile Arg His Ile Leu Glu Thr Arg Tyr Ala Val Pro Gly Lys
                245                 250                 255

Ser Leu Glu Glu Gln Asn Glu Val Ile Gly Met His Ala Ala Met Lys
            260                 265                 270

Tyr Ile Asn Thr Thr Leu Val Ser Arg Ile Gly Ser Val Thr Ile Ser
        275                 280                 285

Asp Val Leu Glu Ile His Arg Arg Val Leu Gly Tyr Val Asp Pro Val
    290                 295                 300

Glu Ala Gly Arg Phe Arg Thr Thr Gln Val Leu Val Gly His His Ile
305                 310                 315                 320

Pro Pro His Pro Gln Asp Val Glu Lys Gln Met Gln Glu Phe Val Gln
                325                 330                 335

Trp Leu Asn Ser Glu Glu Ala Met Asn Leu His Pro Val Glu Phe Ala
            340                 345                 350

Ala Leu Ala His Tyr Lys Leu Val Tyr Ile His Pro Phe Ile Asp Gly
        355                 360                 365

Asn Gly Arg Thr Ser Arg Leu Leu Met Asn Leu Ile Leu Met Gln Ala
    370                 375                 380

Gly Tyr Pro Pro Ile Thr Ile Arg Lys Glu Gln Arg Ser Asp Tyr Tyr
385                 390                 395                 400

His Val Leu Glu Ala Ala Asn Glu Gly Asp Val Arg Pro Phe Ile Arg
                405                 410                 415

Phe Ile Ala Lys Cys Thr Glu Thr Thr Leu Asp Thr Leu Leu Phe Ala
            420                 425                 430

Thr Thr Glu Tyr Ser Val Ala Leu Pro Glu Ala Gln Pro Asn His Ser
        435                 440                 445

Gly Phe Lys Glu Thr Leu Pro Val Lys Pro
    450                 455


<210> 54
<211> 122
<212> PRT
<213> Homo sapiens

<400> 54


<210> 55
<211> 242
<212> PRT
<213> Homo sapiens

<400> 55
Met Ala Pro Gly Gln Thr Pro Ser Leu Cys Ser Trp Leu Leu Pro Leu
1               5                   10                  15

Pro Ser Thr Trp Ala Thr Thr Gly His Val Cys Phe Ser Asp Ile Leu
            20                  25                  30

Gln Thr Pro Asp Gly Gly Gln Leu Leu Leu Asp Trp Ala Lys Gln Pro
        35                  40                  45

Asp Ser Ser Gln Asp Pro Asp Pro Thr Thr Gln Pro Ile Val Leu Leu
    50                  55                  60

Leu Pro Gly Ile Thr Gly Ser Ser Gln Glu Thr Tyr Val Leu His Leu
65                  70                  75                  80

Val Asn Gln Ala Leu Arg Asp Gly Tyr Gln Ala Val Val Phe Asn Asn
                85                  90                  95

Arg Gly Cys Arg Gly Glu Glu Leu Arg Thr His Arg Ala Phe Cys Ala
            100                 105                 110

Ser Asn Thr Glu Asp Leu Glu Thr Val Val Asn His Ile Lys His Arg
        115                 120                 125

Tyr Pro Gln Ala Pro Leu Leu Ala Val Gly Ile Ser Phe Gly Gly Ile
    130                 135                 140

Leu Val Leu Asn His Leu Ala Gln Ala Arg Gln Ala Ala Gly Leu Val
145                 150                 155                 160

Ala Ala Leu Thr Leu Ser Ala Cys Trp Asp Ser Phe Glu Thr Thr Arg
                165                 170                 175

Ser Leu Glu Thr Pro Leu Asn Ser Leu Leu Phe Asn Gln Pro Leu Thr
            180                 185                 190

Ala Gly Leu Cys Gln Leu Val Glu Arg Leu Ser Tyr Gly Lys Thr Cys
        195                 200                 205

Arg Pro Val Gln Ser Ala Ser Leu Met Ser Ala Thr His Leu Trp Pro
    210                 215                 220

Leu Asp Ile Lys Thr Val Leu Pro Thr Thr Lys Gln Gln Ala Leu Glu
225                 230                 235                 240

Pro Arg

<210> 56
<211> 359
<212> PRT
<213> Homo sapiens

<400> 56

<210> 57
<211> 240
<212> PRT
<213> Homo sapiens

<400> 57
Met Thr Ala Pro Val Pro Ala Pro Arg Ile Leu Leu Pro Leu Leu Leu
1               5                   10                  15

Leu Leu Leu Leu Thr Pro Pro Pro Gly Ala Arg Gly Glu Val Cys Met
            20                  25                  30

Ala Ser Arg Gly Leu Ser Leu Phe Pro Glu Ser Cys Pro Asp Phe Cys
        35                  40                  45

Cys Gly Thr Cys Asp Asp Gln Tyr Cys Cys Ser Asp Val Leu Lys Lys
    50                  55                  60

Phe Val Trp Ser Glu Glu Arg Cys Ala Val Pro Glu Ala Ser Val Pro
65                  70                  75                  80

Ala Ser Val Glu Pro Val Glu Gln Leu Gly Ser Ala Leu Arg Phe Arg
                85                  90                  95

Pro Gly Tyr Asn Asp Pro Met Ser Gly Phe Gly Ala Thr Leu Ala Val
            100                 105                 110

Gly Leu Thr Ile Phe Val Leu Ser Val Val Thr Ile Ile Ile Cys Phe
        115                 120                 125

Thr Cys Ser Cys Cys Cys Leu Tyr Lys Thr Cys Arg Arg Pro Arg Pro
    130                 135                 140

Val Val Thr Thr Thr Thr Ser Thr Thr Val Val His Ala Pro Tyr Pro
145                 150                 155                 160

Gln Pro Pro Ser Val Pro Pro Ser Tyr Pro Gly Pro Ser Tyr Gln Gly
                165                 170                 175

Tyr His Thr Met Pro Pro Gln Pro Gly Met Pro Ala Ala Pro Tyr Pro
            180                 185                 190

Met Gln Tyr Pro Pro Pro Tyr Pro Ala Gln Pro Met Gly Pro Pro Ala
        195                 200                 205

Tyr His Glu Thr Leu Ala Gly Gly Ala Ala Ala Pro Tyr Pro Ala Ser
    210                 215                 220

Gln Pro Pro Tyr Asn Pro Ala Tyr Met Asp Ala Pro Lys Ala Ala Leu
225                 230                 235                 240

<210> 58
<211> 116
<212> PRT
<213> Homo sapiens

<400> 58
Met Lys Gly Leu Arg Ser Leu Ala Ala Thr Thr Leu Ala Leu Phe Leu
  1               5                  10                  15

Val Phe Val Phe Leu Gly Asn Ser Ser Cys Ala Pro Gln Arg Leu Leu
            20                  25                  30

Glu Arg Arg Asn Trp Thr Pro Gln Ala Met Leu Tyr Leu Lys Gly Ala
        35                  40                  45

Gln Gly Arg Arg Phe Ile Ser Asp Gln Ser Arg Arg Lys Asp Leu Ser
    50                  55                  60

Asp Arg Pro Leu Pro Glu Arg Arg Ser Pro Asn Pro Gln Leu Leu Thr
 65                  70                  75                  80

Ile Pro Glu Ala Ala Thr Ile Leu Leu Ala Ser Leu Gln Lys Ser Pro
                85                  90                  95

Glu Asp Glu Glu Lys Asn Phe Asp Gln Thr Arg Phe Leu Glu Asp Ser
            100                 105                 110

Leu Leu Asn Trp
        115

<210> 59
<211> 241
<212> PRT
<213> Homo sapiens

<400> 59
Met Ala Ala Ala Ser Ala Gly Ala Thr Arg Leu Leu Leu Leu Leu Leu
  1               5                  10                  15

Met Ala Val Ala Ala Pro Ser Arg Ala Arg Gly Ser Gly Cys Arg Ala
            20                  25                  30

Gly Thr Gly Ala Arg Gly Ala Gly Ala Glu Gly Arg Glu Gly Glu Ala
        35                  40                  45

Cys Gly Thr Val Gly Leu Leu Leu Glu His Ser Phe Glu Ile Asp Asp
    50                  55                  60

Ser Ala Asn Phe Arg Lys Arg Gly Ser Leu Leu Trp Asn Gln Gln Asp
 65                  70                  75                  80

Gly Thr Leu Ser Leu Ser Gln Arg Gln Leu Ser Glu Glu Glu Arg Gly
                85                  90                  95

Arg Leu Arg Asp Val Ala Ala Leu Asn Gly Leu Tyr Arg Val Arg Ile
            100                 105                 110

Pro Arg Arg Pro Gly Ala Leu Asp Gly Leu Glu Ala Gly Gly Tyr Val
        115                 120                 125

Ser Ser Phe Val Pro Ala Cys Ser Leu Val Glu Ser His Leu Ser Asp
    130                 135                 140

Gln Leu Thr Leu His Val Asp Val Ala Gly Asn Val Val Gly Val Ser
145                 150                 155                 160

Val Val Thr His Pro Met Ala Pro Cys Ser Pro Arg Gly Phe Pro Pro
                165                 170                 175

Ala His Gly Val Glu Pro Glu Ile Leu Ala Thr Met Pro Val Leu Thr
            180                 185                 190

Ser His Pro Pro Thr Pro Ser Pro Cys Ser Leu Gly Thr Cys Arg Leu
        195                 200                 205

Leu Ser Ser Leu Cys Ala Phe Val Pro Gly Gly Leu Thr Leu Leu Ser
    210                 215                 220

Leu Ala Gly Leu Gly Gly Pro Val Gln Ala Pro Ala Ala Pro Pro Ser
225                 230                 235                 240

Leu

<210> 60
<211> 302
<212> PRT
<213> Homo sapiens

<400> 60
Met Glu Ala Thr Gly Thr Trp Ala Leu Leu Leu Ala Leu Ala Leu Leu
 1               5                  10                  15

Leu Leu Leu Thr Leu Ala Leu Ser Gly Thr Arg Ala Arg Gly His Leu
            20                  25                  30

Pro Pro Gly Pro Thr Pro Leu Pro Leu Leu Gly Asn Leu Leu Gln Leu
        35                  40                  45

Arg Pro Gly Ala Leu Tyr Ser Gly Leu Met Arg Leu Ser Lys Lys Tyr
    50                  55                  60

Gly Pro Val Phe Thr Ile Tyr Leu Gly Pro Trp Arg Pro Val Val Val
65                  70                  75                  80

Leu Val Gly Gln Glu Ala Val Arg Glu Ala Leu Gly Gly Gln Ala Glu
                85                  90                  95

Glu Phe Ser Gly Arg Gly Thr Val Ala Met Leu Glu Gly Thr Phe Asp
            100                 105                 110

Gly His Gly Val Phe Phe Ser Asn Gly Glu Arg Trp Arg Gln Leu Arg
        115                 120                 125

Lys Phe Thr Met Leu Ala Leu Arg Asp Leu Gly Met Gly Lys Arg Glu
    130                 135                 140

Gly Glu Glu Leu Ile Gln Ala Glu Ala Arg Cys Leu Val Glu Thr Phe
145                 150                 155                 160

Gln Gly Thr Glu Gly Arg Pro Phe Asp Pro Ser Leu Leu Leu Ala Gln
                165                 170                 175

Ala Thr Ser Asn Val Val Cys Ser Leu Leu Phe Gly Leu Arg Phe Ser
            180                 185                 190

Tyr Glu Asp Lys Glu Phe Gln Ala Val Val Arg Ala Ala Gly Gly Thr
        195                 200                 205

Leu Leu Gly Val Ser Ser Gln Gly Gly Gln Val Ser Gly Trp Asp Pro
    210                 215                 220

Ser Pro Thr Thr Phe Pro Glu Gly Ser Cys Gln Gly Pro Met Arg Thr
225                 230                 235                 240

Ser Cys Pro Ser Pro His Arg Pro Thr Arg Cys Ser Pro Gly Ser Cys
                245                 250                 255

Gly Pro Cys Gln Ala Pro Thr Ser Ser Ser Ser Thr Thr Ser Ala Pro
            260                 265                 270

Trp Leu Pro Ser Gln Ser Gly Arg Cys Ser Ser Thr Arg Gly Thr Trp
        275                 280                 285

Met Leu Arg Ala Pro His Val Thr Leu Ser Met Pro Ser Cys
    290                 295                 300

<210> 61
<211> 709
<212> PRT
<213> Homo sapiens

<220>
<221> SITE
<222> (216)
<223> Xaa equals any of the naturally occurring L-amino acids

<400> 61
Met Ala Thr Ala Gly Gly Gly Ser Gly Ala Asp Pro Gly Ser Arg Gly
  1               5                  10                  15

Leu Leu Arg Leu Leu Ser Phe Cys Val Leu Leu Ala Gly Leu Cys Arg
             20                  25                  30

Gly Asn Ser Val Glu Arg Lys Ile Tyr Ile Pro Leu Asn Lys Thr Ala
         35                  40                  45

Pro Cys Val Arg Leu Leu Asn Ala Thr His Gln Ile Gly Cys Gln Ser
     50                  55                  60

Ser Ile Ser Gly Asp Thr Gly Val Ile His Val Val Glu Lys Glu Glu
 65                  70                  75                  80

Asp Leu Gln Trp Val Leu Thr Asp Gly Pro Asn Pro Pro Tyr Met Val
                 85                  90                  95

Leu Leu Glu Ser Lys His Phe Thr Arg Asp Leu Met Glu Lys Leu Lys
            100                 105                 110

Gly Arg Thr Ser Arg Ile Ala Gly Leu Ala Val Ser Leu Thr Lys Pro
        115                 120                 125

Ser Pro Ala Ser Gly Phe Ser Pro Ser Val Gln Cys Pro Asn Asp Gly
    130                 135                 140

Phe Gly Val Tyr Ser Asn Ser Tyr Gly Pro Glu Phe Ala His Cys Arg
145                 150                 155                 160

Glu Ile Gln Trp Asn Ser Leu Gly Asn Gly Leu Ala Tyr Glu Asp Phe
                165                 170                 175

Ser Phe Pro Ile Phe Leu Leu Glu Asp Glu Asn Glu Thr Lys Val Ile
            180                 185                 190

Lys Gln Cys Tyr Gln Asp His Asn Leu Ser Gln Asn Gly Ser Ala Pro
        195                 200                 205

Ser Phe Pro Leu Cys Ala Met Xaa Leu Phe Ser His Met His Ala Val
    210                 215                 220

Ile Ser Thr Ala Thr Cys Met Arg Arg Ser Ser Ile Gln Ser Thr Phe
225                 230                 235                 240

Ser Ile Asn Pro Glu Ile Val Cys Asp Pro Leu Ser Asp Tyr Asn Val
                245                 250                 255

Trp Ser Met Leu Lys Pro Ile Asn Thr Thr Gly Thr Leu Lys Pro Asp
            260                 265                 270

Asp Arg Val Val Val Ala Ala Thr Arg Leu Asp Ser Arg Ser Phe Phe
        275                 280                 285

Trp Asn Val Ala Pro Gly Ala Glu Ser Ala Val Ala Ser Phe Val Thr
    290                 295                 300

Gln Leu Ala Ala Ala Glu Ala Leu Gln Lys Ala Pro Asp Val Thr Thr
305                 310                 315                 320

Leu Pro Arg Asn Val Met Phe Val Phe Phe Gln Gly Glu Thr Phe Asp
                325                 330                 335

Tyr Ile Gly Ser Ser Arg Met Val Tyr Asp Met Glu Lys Gly Lys Phe
            340                 345                 350

Pro Val Gln Leu Glu Asn Val Asp Ser Phe Val Glu Leu Gly Gln Val
        355                 360                 365

Ala Leu Arg Thr Ser Leu Glu Leu Trp Met His Thr Asp Pro Val Ser
    370                 375                 380

Gln Lys Asn Glu Ser Val Arg Asn Gln Val Glu Asp Leu Leu Ala Thr
385                 390                 395                 400

Leu Glu Lys Ser Gly Ala Gly Val Pro Ala Val Ile Leu Arg Arg Pro
                405                 410                 415

Asn Gln Ser Gln Pro Leu Pro Pro Ser Ser Leu Gln Arg Phe Leu Arg
            420                 425                 430

Ala Arg Asn Ile Ser Gly Val Val Leu Ala Asp His Ser Gly Ala Phe
        435                 440                 445

His Asn Lys Tyr Tyr Gln Ser Ile Tyr Asp Thr Ala Glu Asn Ile Asn
    450                 455                 460

Val Ser Tyr Pro Glu Trp Leu Ser Pro Glu Glu Asp Leu Asn Phe Val
465                 470                 475                 480

Thr Asp Thr Ala Lys Ala Leu Ala Asp Val Ala Thr Val Leu Gly Arg
                485                 490                 495

Ala Leu Tyr Glu Leu Ala Gly Gly Thr Asn Phe Ser Asp Thr Val Gln
            500                 505                 510

Ala Asp Pro Gln Thr Val Thr Arg Leu Leu Tyr Gly Phe Leu Ile Lys
        515                 520                 525

Ala Asn Asn Ser Trp Phe Gln Ser Ile Leu Arg Gln Asp Leu Arg Ser
    530                 535                 540

Tyr Leu Gly Asp Gly Pro Leu Gln His Tyr Ile Ala Val Ser Ser Pro
545                 550                 555                 560

Thr Asn Thr Thr Tyr Val Val Gln Tyr Ala Leu Ala Asn Leu Thr Gly

565                 570                 575

Thr Val Val Asn Leu Thr Arg Glu Gln Cys Gln Asp Pro Ser Lys Val
            580                 585                 590

Pro Ser Glu Asn Lys Asp Leu Tyr Glu Tyr Ser Trp Val Gln Gly Pro
        595                 600                 605

Leu His Ser Asn Glu Thr Asp Arg Leu Pro Arg Cys Val Arg Ser Thr
    610                 615                 620

Ala Arg Leu Ala Arg Ala Leu Ser Pro Ala Phe Glu Leu Ser Gln Trp
625                 630                 635                 640

Ser Ser Thr Glu Tyr Ser Thr Trp Thr Glu Ser Arg Trp Lys Asp Ile
                645                 650                 655

Arg Ala Arg Ile Phe Leu Ile Ala Ser Lys Glu Leu Glu Leu Ile Thr
            660                 665                 670

Leu Thr Val Gly Phe Gly Ile Leu Ile Phe Ser Leu Ile Val Thr Tyr
        675                 680                 685

Cys Ile Asn Ala Lys Ala Asp Val Leu Phe Ile Ala Pro Arg Glu Pro
    690                 695                 700

Gly Ala Val Ser Tyr
705

<210> 62
<211> 293
<212> PRT
<213> Homo sapiens

<400> 62


<210> 63
<211> 604
<212> PRT
<213> Homo sapiens

<400> 63
Met Val Glu Glu Pro Gly Arg Phe Leu Pro Leu Trp Leu His Ile Leu
1               5                   10                  15

Leu Ile Thr Val Leu Leu Val Leu Ser Gly Ile Phe Ser Gly Leu Asn
            20                  25                  30

Leu Gly Leu Met Ala Leu Asp Pro Met Glu Leu Arg Ile Val Gln Asn
        35                  40                  45

Cys Gly Thr Glu Lys Glu Arg Arg Tyr Ala Arg Lys Ile Glu Pro Ile
    50                  55                  60

Arg Arg Lys Gly Asn Tyr Leu Leu Cys Ser Leu Leu Leu Gly Asn Val
65                  70                  75                  80

Leu Val Asn Thr Ser Leu Thr Ile Leu Leu Asp Asn Leu Ile Gly Ser
                85                  90                  95

Gly Leu Met Ala Val Ala Ser Ser Thr Ile Gly Ile Val Ile Phe Gly
            100                 105                 110

Glu Ile Leu Pro Gln Ala Leu Cys Ser Arg His Gly Leu Ala Val Gly
        115                 120                 125

Ala Asn Thr Ile Leu Leu Thr Lys Phe Phe Met Leu Leu Thr Phe Pro
    130                 135                 140

Leu Ser Phe Pro Ile Ser Lys Leu Leu Asp Phe Phe Leu Gly Gln Glu
145                 150                 155                 160

Ile Arg Thr Val Tyr Asn Arg Glu Lys Leu Met Glu Met Leu Lys Val
                165                 170                 175

Thr Glu Pro Tyr Asn Asp Leu Val Lys Glu Glu Leu Asn Met Ile Gln
            180                 185                 190

Gly Ala Leu Glu Leu Arg Thr Lys Thr Val Glu Asp Ile Met Thr Gln
        195                 200                 205

Leu Gln Asp Cys Phe Met Ile Arg Ser Asp Ala Ile Leu Asp Phe Asn
    210                 215                 220

Thr Met Ser Glu Ile Met Glu Ser Gly Tyr Thr Arg Ile Pro Val Phe
225                 230                 235                 240

Glu Asp Glu Gln Ser Asn Ile Val Asp Ile Leu Tyr Val Lys Asp Leu
                245                 250                 255

Ala Phe Val Asp Pro Asp Asp Cys Thr Pro Leu Lys Thr Ile Thr Arg
            260                 265                 270

Phe Tyr Asn His Pro Val His Phe Val Phe His Asp Thr Lys Leu Asp
        275                 280                 285

Ala Met Leu Glu Glu Phe Lys Lys Gly Lys Ser His Leu Ala Ile Val
    290                 295                 300

Gln Lys Val Asn Asn Glu Gly Glu Gly Asp Pro Phe Tyr Glu Val Leu
305                 310                 315                 320

Gly Leu Val Thr Leu Glu Asp Val Ile Glu Glu Ile Ile Lys Ser Glu
                325                 330                 335

Ile Leu Asp Glu Ser Asp Met Tyr Thr Asp Asn Arg Ser Arg Lys Arg
            340                 345                 350

Val Ser Glu Lys Asn Lys Arg Asp Phe Ser Ala Phe Lys Asp Ala Asp
        355                 360                 365

Asn Glu Leu Lys Val Lys Ile Ser Pro Gln Leu Leu Leu Ala Ala His
    370                 375                 380

Arg Phe Leu Ala Thr Glu Val Ser Gln Phe Ser Pro Ser Leu Ile Ser
385                 390                 395                 400

Glu Lys Ile Leu Leu Arg Leu Leu Lys Tyr Pro Asp Val Ile Gln Glu
                405                 410                 415

Leu Lys Phe Asp Glu His Asn Lys Tyr Tyr Ala Arg His Tyr Leu Tyr
            420                 425                 430

Thr Arg Asn Lys Pro Ala Asp Tyr Phe Ile Leu Ile Leu Gln Gly Lys
        435                 440                 445

Val Glu Val Glu Ala Gly Lys Glu Asn Met Lys Phe Glu Thr Gly Ala
    450                 455                 460

Phe Ser Tyr Tyr Gly Thr Met Ala Leu Thr Ser Val Pro Ser Asp Arg
465                 470                 475                 480

Ser Pro Ala His Pro Thr Pro Leu Ser Arg Ser Ala Ser Leu Ser Tyr
                485                 490                 495

Pro Asp Arg Thr Asp Val Ser Thr Ala Ala Thr Leu Ala Gly Ser Ser
            500                 505                 510

Asn Gln Phe Gly Ser Ser Val Leu Gly Gln Tyr Ile Ser Asp Phe Ser
        515                 520                 525

Val Arg Ala Leu Val Asp Leu Gln Tyr Ile Lys Ile Thr Arg Gln Gln
    530                 535                 540

Tyr Gln Asn Gly Leu Leu Ala Ser Arg Met Glu Asn Ser Pro Gln Phe
545                 550                 555                 560

Pro Ile Asp Gly Cys Thr Thr His Met Glu Asn Leu Ala Glu Lys Ser
                565                 570                 575

Glu Leu Pro Val Val Asp Glu Thr Thr Thr Leu Leu Asn Glu Arg Asn
            580                 585                 590

Ser Leu Leu His Lys Ala Ser His Glu Asn Ala Ile
        595                 600

<210> 64
<211> 137
<212> PRT
<213> Homo sapiens

<400> 64
Met Gln Ala Cys Val Leu Leu Leu Gly Leu Val Leu Ser Ala Gln Leu
1               5                   10                  15

Gln Ser Pro Glu Asn Met Arg Met Gly Gly Gly Arg Val Leu Leu Arg
            20                  25                  30

Ala His Pro Val Pro Ala Gly Gly Gly Gln Cys Gln Ser Ser Ala Lys
        35                  40                  45

Gly Pro Trp Val Gly Thr Gly Pro Glu Arg Glu Glu Arg Asp Ser Pro
    50                  55                  60

Glu Gly Arg Trp Ala Ser Tyr Trp Ala Gln Ser Trp Glu Gly Val Ala
65                  70                  75                  80

Ala Ser Thr Gly Trp Ala Trp Thr Pro Leu Ala Pro Thr Pro Ser Gly
                85                  90                  95

Cys Gly Cys Ser Pro Lys Pro Gly Glu Gln Asp Arg Pro Gly Val Ser
            100                 105                 110

Gly Arg Leu Pro Gly Ala Ser Gln Ser Ser Gln Gly Pro Pro Pro Ala
        115                 120                 125

Ser Ala Ser Leu Arg Ala Val Pro Lys
    130                 135


<210> 65
<211> 133
<212> PRT
<213> Homo sapiens

<400> 65
Met Trp His Thr Lys Pro Leu Gly Ser Gly Ser Cys Val Pro Leu Leu
1               5                   10                  15

Pro Leu Leu Leu Leu Leu Leu Leu Leu Phe Pro Leu Leu Pro Trp Pro
            20                  25                  30

Pro Pro Leu Pro Pro Pro Pro Pro Ser Ser Leu His Pro Phe Ala Pro
        35                  40                  45

Ala Phe Pro Ala Thr Gly Ser Leu Ser Ser Asn Asn Ser Gln Leu Leu
    50                  55                  60

Ala Pro Leu Arg Leu Gln Asn Ala Leu His Leu Phe Lys Cys Phe Pro
65                  70                  75                  80

Val Leu Phe Pro Leu His Lys Ile Ile Ser Phe His Pro Glu Tyr Pro
                85                  90                  95

Trp Gln Ala Pro Ile Phe Gln Tyr Phe Tyr Leu Ser Ile Pro Ser Ser
            100                 105                 110

Ser Leu His Pro Glu His Leu Gly His Ser Phe Val Ser Thr Leu His
        115                 120                 125

Ser Pro Thr Arg Gln
    130


<210> 66

<211> 17
<212> PRT
<213> Homo sapiens

<400> 66
Met Leu Ser Gly Ser Leu Gly Ser Ala Val Cys Met Ser Ser Gln Pro
1               5                   10                  15

Arg

<210> 67
<211> 99
<212> PRT
<213> Homo sapiens

<220>
<221> SITE
<222> (91)
<223> Xaa equals any of the naturally occurring L-amino acids

<400> 67
Met Ala Ala Leu Leu Leu Leu Pro Leu Leu Leu Leu Leu Pro Leu Leu
1               5                   10                  15

Leu Leu Lys Leu His Leu Trp Pro Gln Leu Arg Trp Leu Pro Ala Asp
            20                  25                  30

Leu Ala Phe Ala Val Arg Ala Leu Cys Cys Lys Arg Ala Leu Arg Ala
        35                  40                  45

Arg Ala Leu Ala Ala Ala Ala Ala Asp Pro Glu Gly Pro Glu Gly Gly
    50                  55                  60

Cys Ser Leu Ala Trp Arg Leu Ala Glu Leu Ala Gln Gln Arg Ala Glu
65                  70                  75                  80

Leu Leu Leu Arg Ser Arg Ala Leu Ala Thr Xaa Arg Arg Ser Ala Arg
                85                  90                  95

Val Thr Gly

<210> 68
<211> 153
<212> PRT
<213> Homo sapiens

<220>
<221> SITE
<222> (77)
<223> Xaa equals any of the naturally occurring L-amino acids

<220>
<221> SITE
<222> (82)
<223> Xaa equals any of the naturally occurring L-amino acids

<220>
<221> SITE
<222> (83)
<223> Xaa equals any of the naturally occurring L-amino acids

<220>
<221> SITE
<222> (86)
<223> Xaa equals any of the naturally occurring L-amino acids

<220>

<221> SITE
<222> (91)
<223> Xaa equals any of the naturally occurring L-amino acids

<220>
<221> SITE
<222> (122)
<223> Xaa equals any of the naturally occurring L-amino acids

<220>
<221> SITE
<222> (124)
<223> Xaa equals any of the naturally occurring L-amino acids

<400> 68
Met Met Leu Ile Pro Met Ala Ser Val Met Ala Val Thr Glu Pro Lys
1               5                   10                  15

Trp Val Ser Val Trp Ser Arg Phe Leu Trp Val Thr Leu Leu Ser Met
            20                  25                  30

Val Leu Gly Ser Leu Leu Ala Leu Leu Leu Pro Leu Gly Ala Val Glu
        35                  40                  45

Glu Gln Cys Leu Ala Val Leu Lys Gly Leu Tyr Leu Leu Arg Ser Lys
    50                  55                  60

Pro Asp Arg Ala Gln His Ala Ala Pro Ser Ala Pro Xaa Arg Pro Arg
65                  70                  75                  80

Ser Xaa Xaa Ser Pro Xaa Gly Ala Arg Arg Xaa Leu Val Ala Lys Thr
                85                  90                  95

Lys Ala Phe Ser Ser Gly Val Lys Phe Gly Lys Ala Gln Glu Leu Ala
            100                 105                 110

Leu Glu Pro Arg Pro Trp Lys Ile Lys Xaa Ala Xaa Gly Gln Ser Arg
        115                 120                 125

Gly Lys Lys Ala Gln Lys Ser Ser Phe Asn Ala Pro Pro Phe Lys Glu
    130                 135                 140

Trp Asp Pro Gly Asn Phe Pro Gly Asp
145                 150


<210> 69
<211> 361
<212> PRT
<213> Homo sapiens

<220>
<221> SITE
<222> (2)
<223> Xaa equals any of the naturally occurring L-amino acids

<220>
<221> SITE
<222> (4)
<223> Xaa equals any of the naturally occurring L-amino acids

<220>
<221> SITE
<222> (19)
<223> Xaa equals any of the naturally occurring L-amino acids

<400> 69
Ala Xaa Pro Xaa Gly Lys Leu Glu Ala Arg Ala Ala Leu Asn Gln Ala
1               5                   10                  15

Leu Glu Xaa Lys Arg Gln Gly Lys Arg Glu Lys Ala Gln Lys Leu Phe

20                  25                  30

Met His Ala Leu Lys Met Asp Pro Asp Phe Val Asp Ala Leu Thr Glu
        35                  40                  45

Phe Gly Ile Phe Ser Glu Glu Asp Lys Asp Ile Ile Gln Ala Asp Tyr
    50                  55                  60

Leu Tyr Thr Arg Ala Leu Thr Ile Ser Pro Tyr His Glu Lys Ala Leu
65                  70                  75                  80

Val Asn Arg Asp Arg Thr Leu Pro Leu Val Glu Glu Ile Asp Gln Arg
                85                  90                  95

Tyr Phe Ser Ile Ile Asp Ser Lys Val Lys Lys Val Met Ser Ile Pro
            100                 105                 110

Lys Gly Asn Ser Ala Leu Arg Arg Val Met Glu Glu Thr Tyr Tyr His
        115                 120                 125

His Ile Tyr His Thr Val Ala Ile Glu Gly Asn Thr Leu Thr Leu Ser
    130                 135                 140

Glu Ile Arg His Ile Leu Glu Thr Arg Tyr Ala Val Pro Gly Lys Ser
145                 150                 155                 160

Leu Glu Glu Gln Asn Glu Val Ile Gly Met His Ala Ala Met Lys Tyr
                165                 170                 175

Ile Asn Thr Thr Leu Val Ser Arg Ile Gly Ser Val Thr Ile Ser Asp
            180                 185                 190

Val Leu Glu Ile His Arg Arg Val Leu Gly Tyr Val Asp Pro Val Glu
        195                 200                 205

Ala Gly Arg Phe Arg Thr Thr Gln Val Leu Val Gly His His Ile Pro
    210                 215                 220

Pro His Pro Gln Asp Val Glu Lys Gln Met Gln Glu Phe Val Gln Trp
225                 230                 235                 240

Leu Asn Ser Glu Glu Ala Met Asn Leu His Pro Val Glu Phe Ala Ala
                245                 250                 255

Leu Ala His Tyr Lys Leu Val Tyr Ile His Pro Phe Ile Asp Gly Asn
            260                 265                 270

Gly Arg Thr Ser Arg Leu Leu Met Asn Leu Ile Leu Met Gln Ala Gly
        275                 280                 285

Tyr Pro Pro Ile Thr Ile Arg Lys Glu Gln Arg Ser Asp Tyr Tyr His
    290                 295                 300

Val Leu Glu Ala Ala Asn Glu Gly Asp Val Arg Pro Phe Ile Arg Phe
305                 310                 315                 320

Ile Ala Lys Cys Thr Glu Thr Thr Leu Asp Thr Leu Leu Phe Ala Thr
                325                 330                 335

Thr Glu Tyr Ser Val Ala Leu Pro Glu Ala Gln Pro Asn His Ser Gly
            340                 345                 350

Phe Lys Glu Thr Leu Pro Val Lys Pro
        355                 360

<210> 70
<211> 93
<212> PRT
<213> Homo sapiens

<400> 70
Met Tyr Ala Leu Tyr Ile Thr Val His Gly Tyr Phe Leu Ile Thr Phe
1               5                   10                  15

Leu Phe Gly Met Val Val Leu Ala Leu Val Val Trp Lys Ile Phe Thr
            20                  25                  30

Leu Ser Arg Ala Thr Ala Val Lys Glu Arg Gly Lys Asn Arg Lys Lys
        35                  40                  45

Val Leu Thr Leu Leu Gly Leu Ser Ser Leu Val Gly Val Thr Trp Gly
    50                  55                  60

Leu Ala Ile Phe Thr Pro Leu Gly Leu Ser Thr Val Tyr Ile Phe Ala
65                  70                  75                  80

Leu Phe Asn Ser Leu Gln Ala Gln Arg Gly Ile Thr Val
                85                  90


<210> 71
<211> 93
<212> PRT
<213> Homo sapiens

<220>
<221> SITE
<222> (34)
<223> Xaa equals any of the naturally occurring L-amino acids

<220>
<221> SITE
<222> (76)
<223> Xaa equals any of the naturally occurring L-amino acids

<400> 71
Met Tyr Ala Leu Tyr Ile Thr Val His Gly Tyr Phe Leu Ile Thr Phe
1               5                   10                  15

Leu Phe Gly Met Val Val Leu Ala Leu Val Val Trp Lys Ile Phe Thr
            20                  25                  30

Leu Xaa Arg Ala Thr Ala Val Lys Glu Arg Gly Lys Asn Arg Lys Lys
        35                  40                  45

Val Leu Thr Leu Leu Gly Leu Ser Ser Leu Val Gly Val Thr Trp Gly
    50                  55                  60

Leu Ala Ile Phe Thr Pro Leu Gly Leu Ser Thr Xaa Tyr Ile Phe Ala
65                  70                  75                  80

Leu Phe Asn Ser Leu Gln Ala Gln Arg Gly Ile Thr Val
                85                  90


<210> 72
<211> 241
<212> PRT
<213> Homo sapiens

<400> 72
Ala Pro Gly Gln Thr Pro Ser Leu Cys Ser Trp Leu Leu Pro Leu Pro
1               5                   10                  15

Ser Thr Trp Ala Thr Thr Gly His Val Cys Phe Ser Asp Ile Leu Gln
            20                  25                  30

Thr Pro Asp Gly Gly Gln Leu Leu Leu Asp Trp Ala Lys Gln Pro Asp
        35                  40                  45

Ser Ser Gln Asp Pro Asp Pro Thr Thr Gln Pro Ile Val Leu Leu Leu

50                  55                  60

Pro Gly Ile Thr Gly Ser Ser Gln Glu Thr Tyr Val Leu His Leu Val
65                  70                  75                  80

Asn Gln Ala Leu Arg Asp Gly Tyr Gln Ala Val Val Phe Asn Asn Arg
                85                  90                  95

Gly Cys Arg Gly Glu Glu Leu Arg Thr His Arg Ala Phe Cys Ala Ser
            100                 105                 110

Asn Thr Glu Asp Leu Glu Thr Val Val Asn His Ile Lys His Arg Tyr
        115                 120                 125

Pro Gln Ala Pro Leu Leu Ala Val Gly Ile Ser Phe Gly Gly Ile Leu
    130                 135                 140

Val Leu Asn His Leu Ala Gln Ala Arg Gln Ala Ala Gly Leu Val Ala
145                 150                 155                 160

Ala Leu Thr Leu Ser Ala Cys Trp Asp Ser Phe Glu Thr Thr Arg Ser
                165                 170                 175

Leu Glu Thr Pro Leu Asn Ser Leu Leu Phe Asn Gln Pro Leu Thr Ala
            180                 185                 190

Gly Leu Cys Gln Leu Val Glu Arg Leu Ser Tyr Gly Lys Thr Cys Arg
        195                 200                 205

Pro Val Gln Ser Ala Ser Leu Met Ser Ala Thr His Leu Trp Pro Leu
    210                 215                 220

Asp Ile Lys Thr Val Leu Pro Thr Thr Lys Gln Gln Ala Leu Glu Pro
225                 230                 235                 240

Arg

<210> 73
<211> 180
<212> PRT
<213> Homo sapiens

<220>
<221> SITE
<222> (143)
<223> Xaa equals any of the naturally occurring L-amino acids

<400> 73
Met Gly Trp Pro Arg Pro Gly Arg Ala Leu Val Ala Val Lys Ala Leu
  1               5                  10                  15

Leu Val Leu Ser Leu Leu Gln Val Pro Ala Gln Ala Val Val Arg Ala
             20                  25                  30

Val Leu Glu Asp Asn Ser Ser Ser Val Asp Phe Ala Asp Leu Pro Ala
         35                  40                  45

Leu Phe Gly Val Pro Leu Ala Pro Glu Gly Ile Arg Gly Tyr Leu Met
     50                  55                  60

Glu Val Lys Pro Ala Asn Ala Cys His Pro Ile Glu Ala Pro Arg Leu
 65                  70                  75                  80

Gly Asn Arg Ser Leu Gly Ala Ile Val Leu Ile Arg Arg Tyr Asp Cys
                 85                  90                  95

Thr Phe Asp Leu Lys Val Leu Asn Ala Gln Arg Ala Gly Phe Glu Ala
            100                 105                 110

Ala Ile Val His Asn Val His Ser Asp Asp Leu Val Ser Met Thr His
        115                 120                 125

Val Tyr Glu Asp Leu Arg Gly Gln Ile Ala Ile Pro Ser Val Xaa Val
    130                 135                 140

Ser Glu Ala Ala Arg Arg Thr Cys Gly Ser Ser Trp Ala Ala Thr Ser
145                 150                 155                 160

Arg Pro Thr Arg Cys Pro Ala Asp Asp Pro Pro Cys His Asp Leu Ala
                165                 170                 175

Val Thr Pro Cys
            180

<210> 74
<211> 225
<212> PRT
<213> Homo sapiens

<220>
<221> SITE
<222> (21)
<223> Xaa equals any of the naturally occurring L-amino acids

<220>
<221> SITE
<222> (45)
<223> Xaa equals any of the naturally occurring L-amino acids

<400> 74
Thr Gln Pro Cys Gln Arg Pro Gly Ile Val Thr Pro Val Leu Thr Val
1               5                   10                  15

Ser Trp Val Leu Xaa Cys Thr Leu Ala Leu Val Val Ser Ala Phe Phe
            20                  25                  30

Val Leu Asn His Leu Trp Leu Trp Ala Gln Ala Cys Xaa Ser His Arg
        35                  40                  45

Arg Pro Val Lys Thr Ser Thr Cys Gln Lys Ala Gln Val Arg Thr Phe
    50                  55                  60

Thr Trp His Asn Asp Leu Cys Ala Ile Cys Leu Asp Glu Tyr Glu Glu
65                  70                  75                  80

Gly Asp Gln Leu Lys Ile Leu Pro Cys Ser His Thr Tyr His Cys Lys
                85                  90                  95

Cys Ile Asp Pro Trp Phe Ser Gln Ala Pro Arg Arg Ser Cys Pro Val
            100                 105                 110

Cys Lys Gln Ser Val Ala Ala Thr Glu Asp Ser Phe Asp Ser Thr Thr
        115                 120                 125

Tyr Ser Phe Arg Asp Glu Asp Pro Ser Leu Pro Gly His Arg Pro Pro
    130                 135                 140

Ile Trp Ala Ile Gln Val Gln Tyr Ala Pro Gly Gly Trp Ser Cys Trp
145                 150                 155                 160

Ala Ala Pro Val Pro Thr Ala Thr Ala Ala Pro Arg Pro Trp Arg Gln
                165                 170                 175

Ser Ile Pro Leu Ser Pro Gln Pro Leu Leu Arg Pro Leu Val Ser Lys
            180                 185                 190

Asp Leu Gly Gln Gly Gly Gly Cys Asn Glu Glu Cys Phe Trp Ser Glu
        195                 200                 205

Lys Asn Lys Val Gly Leu Lys Ala Glu Lys Lys Lys Lys Lys Lys Thr
    210                 215                 220

Arg
225


<210> 75
<211> 240
<212> PRT
<213> Homo sapiens

<400> 75
Met Thr Ala Pro Val Pro Ala Pro Arg Ile Leu Leu Pro Leu Leu Leu
  1               5                  10                  15

Leu Leu Leu Leu Thr Pro Pro Pro Gly Ala Arg Gly Glu Val Cys Met
             20                  25                  30

Ala Ser Arg Gly Leu Ser Leu Phe Pro Glu Ser Cys Pro Asp Phe Cys
         35                  40                  45

Cys Gly Thr Cys Asp Asp Gln Tyr Cys Cys Ser Asp Val Leu Lys Lys
     50                  55                  60

Phe Val Trp Ser Glu Glu Arg Cys Ala Val Pro Glu Ala Ser Val Pro
 65                  70                  75                  80

Ala Ser Val Glu Pro Val Glu Gln Leu Gly Ser Ala Leu Arg Phe Arg
                 85                  90                  95

Pro Gly Tyr Asn Asp Pro Met Ser Gly Phe Gly Ala Thr Leu Ala Val
            100                 105                 110

Gly Leu Thr Ile Phe Val Leu Ser Val Val Thr Ile Ile Ile Cys Phe
        115                 120                 125

Thr Cys Ser Cys Cys Cys Leu Tyr Lys Thr Cys Arg Arg Pro Arg Pro
    130                 135                 140

Val Val Thr Thr Thr Thr Ser Thr Thr Val Val His Ala Pro Tyr Pro
145                 150                 155                 160

Gln Pro Pro Ser Val Pro Pro Ser Tyr Pro Gly Pro Ser Tyr Gln Gly
                165                 170                 175

Tyr His Thr Met Pro Pro Gln Pro Gly Met Pro Ala Ala Pro Tyr Pro
            180                 185                 190

Met Gln Tyr Pro Pro Pro Tyr Pro Ala Gln Pro Met Gly Pro Pro Ala
        195                 200                 205

Tyr His Glu Thr Leu Ala Gly Gly Ala Ala Ala Pro Tyr Pro Ala Ser
    210                 215                 220

Gln Pro Pro Tyr Asn Pro Ala Tyr Met Asp Ala Pro Lys Ala Ala Leu
225                 230                 235                 240




<210> 76
<211> 116
<212> PRT
<213> Homo sapiens

<400> 76
Met Lys Gly Leu Arg Ser Leu Ala Ala Thr Thr Leu Ala Leu Phe Leu
  1               5                  10                  15

Val Phe Val Phe Leu Gly Asn Ser Ser Cys Ala Pro Gln Arg Leu Leu
            20                  25                  30

Glu Arg Arg Asn Trp Thr Pro Gln Ala Met Leu Tyr Leu Lys Gly Ala
        35                  40                  45

Gln Gly Arg Arg Phe Ile Ser Asp Gln Ser Arg Arg Lys Asp Leu Ser
    50                  55                  60

Asp Arg Pro Leu Pro Glu Arg Arg Ser Pro Asn Pro Gln Leu Leu Thr
65                  70                  75                  80

Ile Pro Glu Ala Ala Thr Ile Leu Leu Ala Ser Leu Gln Lys Ser Pro
                85                  90                  95

Glu Asp Glu Glu Lys Asn Phe Asp Gln Thr Arg Phe Leu Glu Asp Ser
            100                 105                 110

Leu Leu Asn Trp
        115


<210> 77
<211> 189
<212> PRT
<213> Homo sapiens

<220>
<221> SITE
<222> (169)
<223> Xaa equals any of the naturally occurring L-amino acids

<220>
<221> SITE
<222> (172)
<223> Xaa equals any of the naturally occurring L-amino acids

<220>
<221> SITE
<222> (180)
<223> Xaa equals any of the naturally occurring L-amino acids

<400> 77
Met Ala Ala Ala Ser Ala Gly Ala Thr Arg Leu Leu Leu Leu Leu Leu
  1               5                  10                  15

Met Ala Val Ala Ala Pro Ser Arg Ala Arg Gly Ser Gly Cys Arg Ala
            20                  25                  30

Gly Thr Gly Ala Arg Gly Ala Gly Ala Glu Gly Arg Glu Gly Glu Ala
        35                  40                  45

Cys Gly Thr Val Gly Leu Leu Leu Glu His Ser Phe Glu Ile Asp Asp
    50                  55                  60

Ser Ala Asn Phe Arg Lys Arg Gly Ser Leu Leu Trp Asn Gln Gln Asp
65                  70                  75                  80

Gly Thr Leu Ser Leu Ser Gln Arg Gln Leu Ser Glu Glu Glu Arg Gly
                85                  90                  95

Arg Leu Arg Asp Val Ala Ala Leu Asn Gly Leu Tyr Arg Val Arg Ile
            100                 105                 110

Pro Arg Arg Pro Gly Ala Leu Asp Gly Leu Glu Ala Gly Gly Tyr Val
        115                 120                 125

Ser Ser Phe Val Pro Ala Cys Ser Leu Val Glu Ser His Leu Ser Asp
    130                 135                 140

Gln Leu Thr Leu His Val Asp Val Ala Gly Asn Val Val Gly Val Ser

145                 150                 155                 160

Val Val Thr His Pro Met Ala Pro Xaa Ser Pro Xaa Gly Phe Pro Leu
                165                 170                 175

Pro Trp Ser Xaa Ala Glu Ile Leu Ala Thr Ile Gln Phe
            180                 185


<210> 78
<211> 117
<212> PRT
<213> Homo sapiens

<400> 78
Ala Ala Ser Ala Gly Ala Thr Arg Leu Leu Leu Leu Leu Leu Met Ala
1               5                   10                  15

Val Ala Ala Pro Ser Arg Ala Arg Gly Ser Gly Cys Arg Ala Gly Thr
            20                  25                  30

Gly Ala Arg Gly Ala Gly Ala Glu Gly Arg Glu Gly Glu Ala Cys Gly
        35                  40                  45

Thr Val Gly Leu Leu Leu Glu His Ser Phe Glu Ile Leu Ala Thr Met
    50                  55                  60

Pro Val Leu Thr Ser His Pro Pro Thr Pro Ser Pro Cys Ser Leu Gly
65                  70                  75                  80

Thr Cys Arg Leu Leu Ser Ser Leu Cys Ala Phe Val Pro Gly Gly Leu
                85                  90                  95

Thr Leu Leu Ser Leu Ala Gly Leu Gly Gly Pro Val Gln Ala Pro Ala
            100                 105                 110

Ala Pro Pro Ser Leu
        115


<210> 79
<211> 302
<212> PRT
<213> Homo sapiens

<400> 79
Met Glu Ala Thr Gly Thr Trp Ala Leu Leu Leu Ala Leu Ala Leu Leu
1               5                   10                  15

Leu Leu Leu Thr Leu Ala Leu Ser Gly Thr Arg Ala Arg Gly His Leu
            20                  25                  30

Pro Pro Gly Pro Thr Pro Leu Pro Leu Leu Gly Asn Leu Leu Gln Leu
        35                  40                  45

Arg Pro Gly Ala Leu Tyr Ser Gly Leu Met Arg Leu Ser Lys Lys Tyr
    50                  55                  60

Gly Pro Val Phe Thr Ile Tyr Leu Gly Pro Trp Arg Pro Val Val Val
65                  70                  75                  80

Leu Val Gly Gln Glu Ala Val Arg Glu Ala Leu Gly Gly Gln Ala Glu
                85                  90                  95

Glu Phe Ser Gly Arg Gly Thr Val Ala Met Leu Glu Gly Thr Phe Asp
            100                 105                 110

Gly His Gly Val Phe Phe Ser Asn Gly Glu Arg Trp Arg Gln Leu Arg
        115                 120                 125

Lys Phe Thr Met Leu Ala Leu Arg Asp Leu Gly Met Gly Lys Arg Glu

130                 135                 140

Gly Glu Glu Leu Ile Gln Ala Glu Ala Arg Cys Leu Val Glu Thr Phe
145                 150                 155                 160

Gln Gly Thr Glu Gly Arg Pro Phe Asp Pro Ser Leu Leu Leu Ala Gln
                165                 170                 175

Ala Thr Ser Asn Val Val Cys Ser Leu Leu Phe Gly Leu Arg Phe Ser
            180                 185                 190

Tyr Glu Asp Lys Glu Phe Gln Ala Val Val Arg Ala Ala Gly Gly Thr
        195                 200                 205

Leu Leu Gly Val Ser Ser Gln Gly Gly Gln Val Ser Gly Trp Asp Pro
    210                 215                 220

Ser Pro Thr Thr Phe Pro Glu Gly Ser Cys Gln Gly Pro Met Arg Thr
225                 230                 235                 240

Ser Cys Pro Ser Pro His Arg Pro Thr Arg Cys Ser Pro Gly Ser Cys
                245                 250                 255

Gly Pro Cys Gln Ala Pro Thr Ser Ser Ser Ser Thr Thr Ser Ala Pro
            260                 265                 270

Trp Leu Pro Ser Gln Ser Gly Arg Cys Ser Ser Thr Arg Gly Thr Trp
        275                 280                 285

Met Leu Arg Ala Pro His Val Thr Leu Ser Met Pro Ser Cys
    290                 295                 300

<210> 80
<211> 254
<212> PRT
<213> Homo sapiens

<220>
<221> SITE
<222> (218)
<223> Xaa equals any of the naturally occurring L-amino acids

<220>
<221> SITE
<222> (228)
<223> Xaa equals any of the naturally occurring L-amino acids

<220>
<221> SITE
<222> (240)
<223> Xaa equals any of the naturally occurring L-amino acids

<400> 80
Glu Thr Arg Leu His His Val Ser Thr Leu Ala Ala Phe Thr Val Arg
  1               5                  10                  15

Gln Val Gln Gln His Gln Gly Asn Leu Asp Ala Ser Gly Pro Ala Arg
             20                  25                  30

Asp Leu Val Asp Ala Phe Leu Leu Lys Met Ala Gln Glu Glu Gln Asn
         35                  40                  45

Pro Gly Thr Glu Phe Thr Asn Lys Asn Met Leu Met Thr Val Ile Tyr
     50                  55                  60

Leu Leu Phe Ala Gly Thr Met Thr Val Ser Thr Thr Val Gly Tyr Thr
 65                  70                  75                  80

Leu Leu Leu Leu Met Lys Tyr Pro His Val Gln Lys Trp Val Arg Glu
                 85                  90                  95

Glu Leu Asn Arg Glu Leu Gly Ala Gly Gln Ala Pro Ser Leu Gly Asp
            100                 105                 110

Arg Thr Arg Ser Leu Thr Pro Thr Arg Phe Cys Met Arg Arg Ser Gly
        115                 120                 125

Cys Trp Arg Trp Cys Pro Trp Glu Tyr Pro Ala Pro Ser Cys Gly Pro
    130                 135                 140

Pro Ala Ser Glu Gly Thr Pro Cys Pro Arg Ala Arg Arg Ser Ser Pro
145                 150                 155                 160

Ser Leu Ala Pro Ser Cys Met Thr Pro Thr Ser Ser Ser Thr Gln Lys
                165                 170                 175

Ser Ser Thr Gln Thr Val Ser Trp Met Gln Met Asp Gly Ser Gly Ser
            180                 185                 190

Met Arg Arg Ser Cys Leu Leu Leu Lys Glu Ala Cys Leu Pro Trp Lys
        195                 200                 205

Gly Pro Gly Lys Ser Gly Ala Leu Pro Xaa Leu His His His Pro Thr
    210                 215                 220

Ser Leu Leu Xaa Gly Glu Pro Val Pro Ala Gly His Pro Glu Pro Xaa
225                 230                 235                 240

Ala His Arg Gln Trp Pro Phe Gln His Ser Pro Ser Leu Pro
                245                 250


<210> 81
<211> 417
<212> PRT
<213> Homo sapiens

<400> 81
Met Ala Thr Ala Gly Gly Gly Ser Gly Ala Asp Pro Gly Ser Arg Gly
1               5                   10                  15

Leu Leu Arg Leu Leu Ser Phe Cys Val Leu Leu Ala Gly Leu Cys Arg
            20                  25                  30

Gly Asn Ser Val Glu Arg Lys Ile Tyr Ile Pro Leu Asn Lys Thr Ala
        35                  40                  45

Pro Cys Val Arg Leu Leu Asn Ala Thr His Gln Ile Gly Cys Gln Ser
    50                  55                  60

Ser Ile Ser Gly Asp Thr Gly Val Ile His Val Val Glu Lys Glu Glu
65                  70                  75                  80

Asp Leu Gln Trp Val Leu Thr Asp Gly Pro Asn Pro Pro Tyr Met Val
                85                  90                  95

Leu Leu Glu Ser Lys His Phe Thr Arg Asp Leu Met Glu Lys Leu Lys
            100                 105                 110

Gly Arg Thr Ser Arg Ile Ala Gly Leu Ala Val Ser Leu Thr Lys Pro
        115                 120                 125

Ser Pro Ala Ser Gly Phe Ser Pro Ser Val Gln Cys Pro Asn Asp Gly
    130                 135                 140

Phe Gly Val Tyr Ser Asn Ser Tyr Gly Pro Glu Phe Ala His Cys Arg
145                 150                 155                 160

Glu Ile Gln Trp Asn Ser Leu Gly Asn Gly Leu Ala Tyr Glu Asp Phe
                165                 170                 175

Ser Phe Pro Ile Phe Leu Leu Glu Asp Glu Asn Glu Thr Lys Val Ile
            180                 185                 190

Lys Gln Cys Tyr Gln Asp His Asn Leu Ser Gln Asn Gly Ser Ala Pro
        195                 200                 205

Thr Phe Pro Leu Cys Ala Met Gln Leu Phe Ser His Met His Ala Val
    210                 215                 220

Ile Ser Thr Ala Thr Cys Met Arg Arg Ser Ser Ile Gln Ser Thr Phe
225                 230                 235                 240

Ser Ile Asn Pro Glu Ile Val Cys Asp Pro Leu Ser Asp Tyr Asn Val
                245                 250                 255

Trp Ser Met Leu Lys Pro Ile Asn Thr Thr Gly Thr Leu Lys Pro Asp
            260                 265                 270

Asp Arg Val Val Val Ala Ala Thr Arg Leu Asp Ser Arg Ser Phe Phe
        275                 280                 285

Trp Asn Val Ala Pro Gly Ala Glu Ser Ala Val Ala Ser Phe Val Thr
    290                 295                 300

Gln Leu Ala Ala Ala Glu Ala Leu Gln Lys Ala Pro Asp Val Thr Thr
305                 310                 315                 320

Leu Pro Arg Asn Val Met Phe Val Phe Phe Gln Gly Glu Thr Phe Asp
                325                 330                 335

Tyr Ile Gly Ser Ser Arg Met Val Tyr Asp Met Glu Lys Gly Lys Phe
            340                 345                 350

Pro Val Gln Leu Glu Asn Val Asp Ser Phe Val Glu Leu Gly Gln Val
        355                 360                 365

Ala Leu Arg Thr Ser Leu Glu Leu Trp Met His Thr Asp Pro Val Ser
    370                 375                 380

Gln Lys Asn Glu Ser Val Arg Asn Gln Val Glu Asp Leu Leu Ala Thr
385                 390                 395                 400

Leu Glu Thr Val Ser Tyr Ala His Leu Asn Leu Gln Gly Gly Glu Val
                405                 410                 415

Leu

<210> 82
<211> 426
<212> PRT
<213> Homo sapiens

<400> 82
Val Val Pro Phe Ser Gly Met Leu Pro Pro Gly Ala Glu Lys Ala Val
  1               5                  10                  15

Ala Ser Phe Val Thr Gln Leu Ala Ala Ala Glu Ala Leu Gln Lys Ala
             20                  25                  30

Pro Asp Val Thr Thr Leu Pro Arg Asn Val Met Phe Val Phe Phe Gln
         35                  40                  45

Gly Glu Thr Phe Asp Tyr Ile Gly Ser Ser Arg Met Val Tyr Asp Met
     50                  55                  60

Glu Lys Gly Lys Phe Pro Val Gln Leu Glu Asn Val Asp Ser Phe Val
 65                  70                  75                  80

Glu Leu Gly Gln Val Ala Leu Arg Thr Ser Leu Glu Leu Trp Met His

85                  90                  95

Thr Asp Pro Val Ser Gln Lys Asn Glu Ser Val Arg Asn Gln Val Glu
            100                 105                 110

Asp Leu Leu Ala Thr Leu Glu Lys Ser Gly Ala Gly Val Pro Ala Val
        115                 120                 125

Ile Leu Arg Arg Pro Asn Gln Ser Gln Pro Leu Pro Pro Ser Ser Leu
    130                 135                 140

Gln Arg Phe Leu Arg Ala Arg Asn Ile Ser Gly Val Val Leu Ala Asp
145                 150                 155                 160

His Ser Gly Ala Phe His Asn Lys Tyr Tyr Gln Ser Ile Tyr Asp Thr
                165                 170                 175

Ala Glu Asn Ile Asn Val Ser Tyr Pro Glu Trp Leu Ser Pro Glu Glu
            180                 185                 190

Asp Leu Asn Phe Val Thr Asp Thr Ala Lys Ala Leu Ala Asp Val Ala
        195                 200                 205

Thr Val Leu Gly Arg Ala Leu Tyr Glu Leu Ala Gly Gly Thr Asn Phe
    210                 215                 220

Ser Asp Thr Val Gln Ala Asp Pro Gln Thr Val Thr Arg Leu Leu Tyr
225                 230                 235                 240

Gly Phe Leu Ile Lys Ala Asn Asn Ser Trp Phe Gln Ser Ile Leu Arg
                245                 250                 255

Gln Asp Leu Arg Ser Tyr Leu Gly Asp Gly Pro Leu Gln His Tyr Ile
            260                 265                 270

Ala Val Ser Ser Pro Thr Asn Thr Thr Tyr Val Val Gln Tyr Ala Leu
        275                 280                 285

Ala Asn Leu Thr Gly Thr Val Val Asn Leu Thr Arg Glu Gln Cys Gln
    290                 295                 300

Asp Pro Ser Lys Val Pro Ser Glu Asn Lys Asp Leu Tyr Glu Tyr Ser
305                 310                 315                 320

Trp Val Gln Gly Pro Leu His Ser Asn Glu Thr Asp Arg Leu Pro Arg
                325                 330                 335

Cys Val Arg Ser Thr Ala Arg Leu Ala Arg Ala Leu Ser Pro Ala Phe
            340                 345                 350

Glu Leu Ser Gln Trp Ser Ser Thr Glu Tyr Ser Thr Trp Thr Glu Ser
        355                 360                 365

Arg Trp Lys Asp Ile Arg Ala Arg Ile Phe Leu Ile Ala Ser Lys Glu
    370                 375                 380

Leu Glu Leu Ile Thr Leu Thr Val Gly Phe Gly Ile Leu Ile Phe Ser
385                 390                 395                 400

Leu Ile Val Thr Tyr Cys Ile Asn Ala Lys Ala Asp Val Leu Phe Ile
                405                 410                 415

Ala Pro Arg Glu Pro Gly Ala Val Ser Tyr
            420                 425

<210> 83
<211> 230
<212> PRT
<213> Homo sapiens

<400> 83
Met Thr Gly Leu Tyr Glu Leu Val Trp Arg Val Leu His Ala Leu Leu
1               5                   10                  15

Cys Leu His Arg Thr Leu Thr Ser Trp Leu Arg Val Arg Phe Gly Thr
            20                  25                  30

Trp Asn Trp Ile Trp Arg Arg Cys Cys Arg Ala Ala Ser Ala Ala Val
        35                  40                  45

Leu Ala Pro Leu Gly Phe Thr Leu Arg Lys Pro Pro Ala Val Gly Arg
    50                  55                  60

Asn Arg Arg His His Arg His Pro Arg Gly Gly Ser Cys Leu Ala Ala
65                  70                  75                  80

Ala His His Arg Met Arg Trp Arg Ala Asp Gly Arg Ser Leu Glu Lys
                85                  90                  95

Leu Pro Val His Met Gly Leu Val Ile Thr Glu Val Glu Gln Glu Pro
            100                 105                 110

Ser Phe Ser Asp Ile Ala Ser Leu Val Val Trp Cys Met Ala Val Gly
        115                 120                 125

Ile Ser Tyr Ile Ser Val Tyr Asp His Gln Gly Ile Phe Lys Arg Asn
    130                 135                 140

Asn Ser Arg Leu Met Asp Glu Ile Leu Lys Gln Gln Gln Glu Leu Leu
145                 150                 155                 160

Gly Leu Asp Cys Ser Lys Tyr Ser Pro Glu Phe Ala Asn Ser Asn Asp
                165                 170                 175

Lys Asp Asp Gln Val Leu Asn Cys His Leu Ala Val Lys Val Leu Ser
            180                 185                 190

Ala Gly Arg Trp Lys Ser Arg Tyr Cys Lys Ser Cys Ser Gly Leu Leu
        195                 200                 205

Pro Val Ser Ser Pro Glu Ala Lys Glu Thr His Arg Phe Gly Cys Arg
    210                 215                 220

Tyr Val Ser Gln Phe Thr
225                 230


<210> 84
<211> 173
<212> PRT
<213> Homo sapiens

<220>
<221> SITE
<222> (85)
<223> Xaa equals any of the naturally occurring L-amino acids

<220>
<221> SITE
<222> (128)
<223> Xaa equals any of the naturally occurring L-amino acids

<220>
<221> SITE
<222> (146)
<223> Xaa equals any of the naturally occurring L-amino acids

<220>
<221> SITE
<222> (160)
<223> Xaa equals any of the naturally occurring L-amino acids

<220>
<221> SITE
<222> (168)
<223> Xaa equals any of the naturally occurring L-amino acids

<220>
<221> SITE
<222> (172)
<223> Xaa equals any of the naturally occurring L-amino acids

<400> 84
Met Val Glu Glu Pro Gly Arg Phe Leu Pro Leu Trp Leu His Ile Leu
1               5                   10                  15

Leu Ile Thr Val Leu Leu Val Leu Ser Gly Ile Phe Ser Gly Leu Asn
            20                  25                  30

Leu Gly Leu Met Ala Leu Asp Pro Met Glu Leu Arg Ile Val Gln Asn
        35                  40                  45

Cys Gly Thr Glu Lys Glu Arg Arg Tyr Ala Arg Lys Ile Glu Pro Ile
    50                  55                  60

Arg Arg Lys Gly Asn Tyr Leu Leu Cys Ser Leu Leu Leu Gly Asn Val
65                  70                  75                  80

Leu Val Asn Thr Xaa Leu Thr Ile Leu Leu Asp Asn Leu Ile Gly Ser
                85                  90                  95

Gly Leu Met Ala Val Ala Ser Ser Thr Ile Gly Ile Val Ile Phe Gly
            100                 105                 110

Glu Ile Leu Pro Gln Ala Leu Cys Ser Arg His Gly Leu Ala Val Xaa
        115                 120                 125

Ala Asn Thr Ile Leu Leu Thr Lys Phe Phe Met Leu Leu Thr Phe Pro
    130                 135                 140

Leu Xaa Phe Pro Ile Ser Lys Leu Leu Asp Phe Phe Leu Gly Gln Xaa
145                 150                 155                 160

Ile Arg Thr Val Tyr Asn Arg Xaa Lys Leu Met Xaa Met
                165                 170


<210> 85
<211> 314
<212> PRT
<213> Homo sapiens

<220>
<221> SITE
<222> (93)
<223> Xaa equals any of the naturally occurring L-amino acids

<400> 85
Glu Lys Ala Ala Gly Ala Gly Lys Ser His Leu Ala Ile Val Gln Lys
1               5                   10                  15

Val Asn Asn Glu Gly Glu Gly Asp Pro Phe Tyr Glu Val Leu Gly Leu
            20                  25                  30

Val Thr Leu Glu Asp Val Ile Glu Glu Ile Ile Lys Ser Glu Ile Leu
        35                  40                  45

Asp Glu Ser Asp Met Tyr Thr Asp Asn Arg Ser Arg Lys Arg Val Ser
    50                  55                  60

Glu Lys Asn Lys Arg Asp Phe Ser Ala Phe Lys Asp Ala Asp Asn Glu
65                  70                  75                  80

Leu Lys Val Lys Ile Ser Pro Gln Leu Leu Leu Ala Xaa His Arg Phe
                85                  90                  95

Leu Ala Thr Glu Val Ser Gln Phe Ser Pro Ser Leu Ile Ser Glu Lys
            100                 105                 110

Ile Leu Leu Arg Leu Leu Lys Tyr Pro Asp Val Ile Gln Glu Leu Lys
        115                 120                 125

Phe Asp Glu His Asn Lys Tyr Tyr Ala Arg His Tyr Leu Tyr Thr Arg
    130                 135                 140

Asn Lys Pro Ala Asp Tyr Phe Ile Leu Ile Leu Gln Gly Lys Val Glu
145                 150                 155                 160

Val Glu Ala Gly Lys Glu Asn Met Lys Phe Glu Thr Gly Ala Phe Ser
                165                 170                 175

Tyr Tyr Gly Thr Met Ala Leu Thr Ser Val Pro Ser Asp Arg Ser Pro
            180                 185                 190

Ala His Pro Thr Pro Leu Ser Arg Ser Ala Ser Leu Ser Tyr Pro Asp
        195                 200                 205

Arg Thr Asp Val Ser Thr Ala Ala Thr Leu Ala Gly Ser Ser Asn Gln
    210                 215                 220

Phe Gly Ser Ser Val Leu Gly Gln Tyr Ile Ser Asp Phe Ser Val Arg
225                 230                 235                 240

Ala Leu Val Asp Leu Gln Tyr Ile Lys Ile Thr Arg Gln Gln Tyr Gln
                245                 250                 255

Asn Gly Leu Leu Ala Ser Arg Met Glu Asn Ser Pro Gln Phe Pro Ile
            260                 265                 270

Asp Gly Cys Thr Thr His Met Glu Asn Leu Ala Glu Lys Ser Glu Leu
        275                 280                 285

Pro Val Val Asp Glu Thr Thr Thr Leu Leu Asn Glu Arg Asn Ser Leu
    290                 295                 300

Leu His Lys Ala Ser His Glu Asn Ala Ile
305                 310

<210> 86
<211> 173
<212> PRT
<213> Homo sapiens

<220>
<221> SITE
<222> (146)
<223> Xaa equals any of the naturally occurring L-amino acids

<220>
<221> SITE
<222> (160)
<223> Xaa equals any of the naturally occurring L-amino acids

<220>
<221> SITE
<222> (168)
<223> Xaa equals any of the naturally occurring L-amino acids

<220>
<221> SITE
<222> (172)
<223> Xaa equals any of the naturally occurring L-amino acids

<400> 86
Met Val Glu Glu Pro Gly Arg Phe Leu Pro Leu Trp Leu His Ile Leu
1               5                   10                  15

Leu Ile Thr Val Leu Leu Val Leu Ser Gly Ile Phe Ser Gly Leu Asn
            20                  25                  30

Leu Gly Leu Met Ala Leu Asp Pro Met Glu Leu Arg Ile Val Gln Asn
        35                  40                  45

Cys Gly Thr Glu Lys Glu Arg Arg Tyr Ala Arg Lys Ile Glu Pro Ile
    50                  55                  60

Arg Arg Lys Gly Asn Tyr Leu Leu Cys Ser Leu Leu Leu Gly Asn Val
65                  70                  75                  80

Leu Val Asn Thr Ser Leu Thr Ile Leu Leu Asp Asn Leu Ile Gly Ser
                85                  90                  95

Gly Leu Met Ala Val Ala Ser Ser Thr Ile Gly Ile Val Ile Phe Gly
            100                 105                 110

Glu Ile Leu Pro Gln Ala Leu Cys Ser Arg His Gly Leu Ala Val Gly
        115                 120                 125

Ala Asn Thr Ile Leu Leu Thr Lys Phe Phe Met Leu Leu Thr Phe Pro
    130                 135                 140

Leu Xaa Phe Pro Ile Ser Lys Leu Leu Asp Phe Phe Leu Gly Gln Xaa
145                 150                 155                 160

Ile Arg Thr Val Tyr Asn Arg Xaa Lys Leu Met Xaa Met
                165                 170

<210> 87
<211> 253
<212> PRT
<213> Homo sapiens

<220>
<221> SITE
<222> (215)
<223> Xaa equals any of the naturally occurring L-amino acids

<220>
<221> SITE
<222> (252)
<223> Xaa equals any of the naturally occurring L-amino acids

<220>
<221> SITE
<222> (253)
<223> Xaa equals any of the naturally occurring L-amino acids

<400> 87
Met Gln Ala Cys Val Leu Leu Leu Gly Leu Val Leu Ser Ala Gln Leu
1               5                   10                  15

Gln Ser Pro Glu Asn Met Arg Met Gly Gly Gly Arg Val Leu Leu Arg
            20                  25                  30

Ala His Pro Val Pro Ala Gly Gly Gly Gln Cys Gln Ser Ser Ala Lys
        35                  40                  45

Gly Pro Trp Val Gly Thr Gly Pro Glu Arg Glu Glu Arg Asp Ser Pro
    50                  55                  60

Glu Gly Arg Trp Ala Ser Tyr Trp Ala Gln Ser Trp Glu Gly Val Ala
65                  70                  75                  80

Ala Ser Thr Gly Trp Ala Trp Thr Pro Leu Ala Pro Thr Pro Ser Gly
                85                  90                  95

Cys Gly Cys Ser Leu Ser Leu Glu Ser Arg Thr Gly Pro Gly Cys Leu
            100                 105                 110

Gly Gly Cys Gln Val Pro Pro Glu Leu Pro Arg Ala Pro Thr Cys Lys
        115                 120                 125

Cys Gln Pro Gln Gly Ser Ala Gln Met Arg Pro Ser Gln Leu Gln Pro
    130                 135                 140

Ala Met Pro Trp Asp Ala His Arg Glu Gly Gly Gly Phe Gly Leu Leu
145                 150                 155                 160

Ser Pro Trp Glu Arg Leu Gly Ala Val Thr Ala Arg Leu Ala Gln Ala
                165                 170                 175

His Cys Arg Val Gly Trp Leu Pro Gln Pro Gly Leu Gly Gly Thr Pro
            180                 185                 190

Gly Ser Gly Pro Pro Cys Leu Glu Ser Gln Trp Gly Asp Gly Glu Glu
        195                 200                 205

Thr Trp Pro Pro Met Ala Xaa Gly Gln Leu Arg Thr Arg Thr Cys Trp
    210                 215                 220

Ser Trp Lys Cys Cys Gly Val Glu Gly Trp Gly Gly Gln Leu Leu Thr
225                 230                 235                 240

Pro Ala Ser Cys Leu Leu Leu Ser Thr Phe Pro Xaa Xaa
                245                 250


<210> 88
<211> 102
<212> PRT
<213> Homo sapiens

<400> 88
Asn Ser Glu Lys Gly Gln Lys Lys Gln Arg Gly Pro Arg Trp Ile Cys
1               5                   10                  15

Gln Leu Phe Cys Arg Cys Phe Leu Pro Leu Leu Trp Val Val Cys Ser
            20                  25                  30

Pro Leu Gln Thr Ser Ala Arg Arg Glu Gly Leu Asn Leu Pro Ala Pro
        35                  40                  45

Gln Asp Leu Leu Pro Ser Gly Pro Ser Pro Ala Leu Arg Ser Leu Pro
    50                  55                  60

Asp Arg Arg Val Asp Arg Ala Thr Trp Ala Ala Arg Glu Thr His Gly
65                  70                  75                  80

Gly Pro Pro Cys Gly Gln Pro Cys Gln Leu Pro Pro Ser Pro Glu Leu
                85                  90                  95

His Leu His Leu Glu Glu
            100


<210> 89
<211> 133
<212> PRT
<213> Homo sapiens

<400> 89
Met Trp His Thr Lys Pro Leu Gly Ser Gly Ser Cys Val Pro Leu Leu
1               5                   10                  15

Pro Leu Leu Leu Leu Leu Leu Leu Leu Phe Pro Leu Leu Pro Trp Pro
            20                  25                  30

Pro Pro Leu Pro Pro Pro Pro Pro Ser Ser Leu His Pro Phe Ala Pro
        35                  40                  45

Ala Phe Pro Ala Thr Gly Ser Leu Ser Ser Asn Asn Ser Gln Leu Leu
    50                  55                  60

Ala Pro Leu Arg Leu Gln Asn Ala Leu His Leu Phe Lys Cys Phe Pro
65                  70                  75                  80

Val Leu Phe Pro Leu His Lys Ile Ile Ser Phe His Pro Glu Tyr Pro
                85                  90                  95

Trp Gln Ala Pro Ile Phe Gln Tyr Phe Tyr Leu Ser Ile Pro Ser Ser
            100                 105                 110

Ser Leu His Pro Glu His Leu Gly His Ser Phe Val Ser Thr Leu His
        115                 120                 125

Ser Pro Thr Arg Gln
    130

<210> 90
<211> 86
<212> PRT
<213> Homo sapiens

<220>
<221> SITE
<222> (72)
<223> Xaa equals any of the naturally occurring L-amino acids

<400> 90
Pro Cys Cys Phe His Lys Pro His Ala Ser His Ile Met Asn Phe Leu
1               5                   10                  15

Ile Arg Ile Gln Cys Ile Tyr Leu Pro Lys Ile Val Cys Ala Tyr Ser
            20                  25                  30

Lys Tyr Glu Gln Phe Leu Asn Asn Gly Ser Ile Ile Phe Val Gln Asn
        35                  40                  45

Ala Lys Asn Trp Gly Gln Ala Trp Trp His Thr Pro Val Ile Pro Ala
    50                  55                  60

Leu Trp Glu Ala Lys Val Gly Xaa Ser Pro Glu Val Arg Ser Leu Arg
65                  70                  75                  80

Pro Ala Trp Pro Ala Trp
                85

<210> 91
<211> 17
<212> PRT
<213> Homo sapiens

<400> 91
Met Leu Ser Gly Ser Leu Gly Ser Ala Val Cys Met Ser Ser Gln Pro
1               5                   10                  15

Arg

<210> 92
<211> 125

<212> PRT
<213> Homo sapiens

<220>
<221> SITE
<222> (46)
<223> Xaa equals any of the naturally occurring L-amino acids

<400> 92
Ala Gln Arg Asn Pro Gly Ala Val Pro Ala Val Trp Arg Gln Ala Gly
1               5                   10                  15

Val Thr Phe Thr Ser Ala Lys Gly Arg Ser Ser Pro Tyr Trp Ser Leu
            20                  25                  30

His Pro Gln Ile Ile Leu Leu Arg Lys Leu Ser Ser Ser Xaa Gln Lys
        35                  40                  45

Pro Arg Ser Ser Ser Ala Gln Cys Gly Arg Asn Ala Ala Ala Gly Leu
    50                  55                  60

Pro His Cys Leu Arg Ala Ser Trp Ser Arg Leu Leu Lys Ile Glu Trp
65                  70                  75                  80

Gln Val Gly Leu Ala Trp Ala Gly Ala Asp Val Leu Cys Gly His Pro
                85                  90                  95

Val Pro Lys Arg Pro Pro Thr Leu Gly Pro Gln Thr Ser Gly Ala Asp
            100                 105                 110

Trp His Leu Arg Gly His Ser Pro Thr His Leu Leu Gln
        115                 120                 125


<210> 93
<211> 16
<212> PRT
<213> Homo sapiens

<400> 93
Leu Leu Gly Leu Ser Ser Leu Val Gly Val Thr Trp Gly Leu Ala Ile
1               5                   10                  15




<210> 94
<211> 158
<212> PRT
<213> Homo sapiens

<400> 94
His Glu Val Ile Gly Thr Gly Ser Ala Asn Ser Tyr Gly Leu Tyr Thr
1               5                   10                  15

Ile Arg Asp Arg Glu Asn Arg Thr Ser Leu Glu Leu Cys Trp Phe Arg
            20                  25                  30

Glu Gly Thr Thr Met Tyr Ala Leu Tyr Ile Thr Val His Gly Tyr Phe
        35                  40                  45

Leu Ile Thr Phe Leu Phe Gly Met Val Val Leu Ala Leu Val Val Trp
    50                  55                  60

Lys Ile Phe Thr Leu Ser Arg Ala Thr Ala Val Lys Glu Arg Gly Lys
65                  70                  75                  80

Asn Arg Lys Lys Val Leu Thr Leu Leu Gly Leu Ser Ser Leu Val Gly
                85                  90                  95

Val Thr Trp Gly Leu Ala Ile Phe Thr Pro Leu Gly Leu Ser Thr Val
            100                 105                 110

Tyr Ile Phe Ala Leu Phe Asn Ser Leu Gln Gly Val Phe Ile Cys Cys
        115                 120                 125

Trp Phe Thr Ile Leu Tyr Leu Pro Ser Gln Ser Thr Thr Val Ser Ser
    130                 135                 140

Ser Thr Ala Arg Leu Asp Gln Ala His Ser Ala Ser Gln Glu
145                 150                 155

<210> 95
<211> 157
<212> PRT
<213> Homo sapiens

<400> 95

<210> 96
<211> 128
<212> PRT
<213> Homo sapiens

<220>
<221> SITE
<222> (69)
<223> Xaa equals any of the naturally occurring L-amino acids

<220>
<221> SITE
<222> (111)
<223> Xaa equals any of the naturally occurring L-amino acids

<400> 96
Met Val Ile Gly Thr Gly Ser Ala Asn Ser Tyr Gly Leu Tyr Thr Ile
  1               5                  10                  15

Arg Asp Arg Glu Asn Arg Thr Ser Leu Glu Leu Cys Trp Phe Arg Glu
             20                  25                  30

Gly Thr Thr Met Tyr Ala Leu Tyr Ile Thr Val His Gly Tyr Phe Leu
        35                  40                  45

Ile Thr Phe Leu Phe Gly Met Val Val Leu Ala Leu Val Val Trp Lys
    50                  55                  60

Ile Phe Thr Leu Xaa Arg Ala Thr Ala Val Lys Glu Arg Gly Lys Asn
65                  70                  75                  80

Arg Lys Lys Val Leu Thr Leu Leu Gly Leu Ser Ser Leu Val Gly Val
                85                  90                  95

Thr Trp Gly Leu Ala Ile Phe Thr Pro Leu Gly Leu Ser Thr Xaa Tyr
            100                 105                 110

Ile Phe Ala Leu Phe Asn Ser Leu Gln Ala Gln Arg Gly Ile Thr Val
        115                 120                 125

<210> 97
<211> 27
<212> PRT
<213> Homo sapiens

<400> 97
Pro Leu Gly Leu Ser Thr Val Tyr Ile Phe Ala Leu Phe Asn Ser Leu
1               5                   10                  15

Gln Gly Val Phe Ile Cys Cys Trp Phe Thr Ile
            20                  25

<210> 98
<211> 31
<212> PRT
<213> Homo sapiens

<400> 98
Tyr Ile Thr Val His Gly Tyr Phe Leu Ile Thr Phe Leu Phe Gly Met
1               5                   10                  15

Val Val Leu Ala Leu Val Val Trp Lys Ile Phe Thr Leu Ser Arg
            20                  25                  30

<210> 99
<211> 36
<212> PRT
<213> Homo sapiens

<400> 99
Val Ile Gly Thr Gly Ser Ala Asn Ser Tyr Gly Leu Tyr Thr Ile Arg
1               5                   10                  15

Asp Arg Glu Asn Arg Thr Ser Leu Glu Leu Cys Trp Phe Arg Glu Gly
            20                  25                  30

Thr Thr Met Tyr
        35

<210> 100
<211> 173
<212> PRT
<213> Homo sapiens

<400> 100

Ser Glu Val Leu Gln Thr Pro Asp Gly Gly Gln Phe Leu Leu Asp Trp
1               5                   10                  15

Ala Glu Gln Pro Asn Ser Thr His Tyr Pro Asp Pro Thr Thr Gln Pro
            20                  25                  30

Ile Val Leu Leu Leu Pro Gly Ile Ser Gly Ser Ser Gln Glu Pro Tyr
        35                  40                  45

Ile Leu His Leu Val Asn Gln Ala Leu Lys Asp Gly Tyr Arg Ala Val
    50                  55                  60

Val Phe Asn Asn Arg Gly Cys Arg Gly Glu Glu Leu Leu Thr His Arg
65                  70                  75                  80

Ala Tyr Cys Ala Ser Asn Thr Glu Asp Leu Glu Thr Val Val Lys His
                85                  90                  95

Ile Lys Arg Arg Tyr Ser Gln Ala Pro Leu Leu Ala Val Gly Ile Ser
            100                 105                 110

Phe Gly Gly Ile Leu Val Leu Asn Tyr Leu Ala Gln Thr Gly Lys Ala
        115                 120                 125

Gly Gly Leu Val Ala Gly Leu Thr Met Ser Ala Cys Trp Asp Ser Phe
    130                 135                 140

Glu Thr Val Asp Ser Leu Glu Thr Pro Leu Asn Ser Leu Leu Phe Asn
145                 150                 155                 160

Gln Pro Leu Thr Ala Gly Leu Cys Arg Leu Val Ala Arg
                165                 170

<210> 101
<211> 173
<212> PRT
<213> Homo sapiens

<400> 101
Ser Asp Ile Leu Gln Thr Pro Asp Gly Gly Gln Leu Leu Leu Asp Trp
1               5                   10                  15

Ala Lys Gln Pro Asp Ser Ser Gln Asp Pro Asp Pro Thr Thr Gln Pro
            20                  25                  30

Ile Val Leu Leu Leu Pro Gly Ile Thr Gly Ser Ser Gln Glu Thr Tyr
        35                  40                  45

Val Leu His Leu Val Asn Gln Ala Leu Arg Asp Gly Tyr Gln Ala Val
    50                  55                  60

Val Phe Asn Asn Arg Gly Cys Arg Gly Glu Glu Leu Arg Thr His Arg
65                  70                  75                  80

Ala Phe Cys Ala Ser Asn Thr Glu Asp Leu Glu Thr Val Val Asn His
                85                  90                  95

Ile Lys His Arg Tyr Pro Gln Ala Pro Leu Leu Ala Val Gly Ile Ser
            100                 105                 110

Phe Gly Gly Ile Leu Val Leu Asn His Leu Ala Gln Ala Arg Gln Ala
        115                 120                 125

Ala Gly Leu Val Ala Ala Leu Thr Leu Ser Ala Cys Trp Asp Ser Phe
    130                 135                 140

Glu Thr Thr Arg Ser Leu Glu Thr Pro Leu Asn Ser Leu Leu Phe Asn
145                 150                 155                 160

Gln Pro Leu Thr Ala Gly Leu Cys Gln Leu Val Glu Arg

165                 170

<210> 102
<211> 189
<212> PRT
<213> Homo sapiens

<400> 102
Thr Gly Lys Ala Gly Gly Leu Val Ala Gly Leu Thr Met Ser Ala Cys
1               5                   10                  15

Trp Asp Ser Phe Glu Thr Val Asp Ser Leu Glu Thr Pro Leu Asn Ser
            20                  25                  30

Leu Leu Phe Asn Gln Pro Leu Thr Ala Gly Leu Cys Arg Leu Val Ala
        35                  40                  45

Arg Asn Arg Lys Pro Ile Glu Lys Val Leu Asp Val Asp Phe Ala Ile
    50                  55                  60

Lys Ala Arg Thr Ile Arg Gln Leu Asp Glu Arg Tyr Thr Ser Val Ala
65                  70                  75                  80

Phe Gly Tyr Lys Asp Cys Ala Ala Tyr Tyr Gln Ala Ala Ser Pro Arg
                85                  90                  95

Thr Lys Val Asp Ala Ile His Thr Pro Val Leu Cys Leu Asn Ala Ala
            100                 105                 110

Asp Asp Pro Phe Ser Pro Val His Ala Phe Pro Leu Gln Ala Ala Gln
        115                 120                 125

Lys Ser Pro Tyr Val Ala Leu Leu Ile Thr Ala Arg Gly Gly His Ile
    130                 135                 140

Gly Phe Leu Glu Gly Leu Met Pro Trp Gln His Cys Tyr Met Asn Arg
145                 150                 155                 160

Val Leu His Gln Tyr Ala Arg Ala Ile Phe Gln His Ser Val Gly Leu
                165                 170                 175

Pro Asp Leu Gly Val Leu Thr Pro Glu Asp Gly Lys Ser
            180                 185

<210> 103
<211> 126
<212> PRT
<213> Homo sapiens

<400> 103
Leu Gln Ala Arg Thr Ile Arg Gln Phe Asp Glu Arg Tyr Thr Ser Val
1               5                   10                  15

Ala Phe Gly Tyr Gln Asp Cys Val Thr Tyr Tyr Lys Ala Ala Ser Pro
            20                  25                  30

Arg Thr Lys Ile Asp Ala Ile Arg Ile Pro Val Leu Tyr Leu Ser Ala
        35                  40                  45

Ala Asp Asp Pro Phe Ser Pro Val Cys Ala Leu Pro Ile Gln Ala Ala
    50                  55                  60

Gln His Ser Pro Tyr Val Ala Leu Leu Ile Thr Ala Arg Gly Gly His
65                  70                  75                  80

Ile Gly Phe Leu Glu Gly Leu Leu Pro Trp Gln His Trp Tyr Met Ser
                85                  90                  95

Arg Leu Leu His Gln Tyr Ala Lys Ala Ile Phe Gln Asp Pro Glu Gly

            100                 105                 110

Leu Pro Asp Leu Arg Ala Leu Leu Pro Ser Glu Asp Arg Asn
        115                 120                 125

<210> 104
<211> 33
<212> PRT
<213> Homo sapiens

<400> 104
Thr Ser Leu Ser Pro Gln Asn Gly Thr Trp Ala Asp Thr Phe Ser Leu
1               5                   10                  15

Leu Leu Ala Leu Ala Val Ala Leu Tyr Leu Gly Tyr Tyr Trp Ala Cys
            20                  25                  30

Val

<210> 105
<211> 133
<212> PRT
<213> Homo sapiens

<400> 105
Met Ala Pro Gly Gln Thr Pro Ser Leu Cys Ser Trp Leu Leu Pro Leu
1               5                   10                  15

Pro Ser Thr Trp Ala Thr Thr Gly His Val Cys Phe Ser Asp Ile Leu
            20                  25                  30

Gln Thr Pro Asp Gly Gly Gln Leu Leu Leu Asp Trp Ala Lys Gln Pro
        35                  40                  45

Asp Ser Ser Gln Asp Pro Asp Pro Thr Thr Gln Pro Ile Val Leu Leu
    50                  55                  60

Leu Pro Gly Ile Thr Gly Ser Ser Gln Glu Thr Tyr Val Leu His Leu
65                  70                  75                  80

Val Asn Gln Ala Leu Arg Asp Gly Tyr Gln Ala Val Val Phe Asn Asn
                85                  90                  95

Arg Gly Cys Arg Gly Glu Glu Leu Arg Thr His Arg Ala Phe Cys Ala
            100                 105                 110

Ser Asn Thr Glu Asp Leu Glu Thr Val Val Asn His Ile Lys His Arg
        115                 120                 125

Tyr Pro Gln Ala Pro
    130

<210> 106
<211> 111
<212> PRT
<213> Homo sapiens

<400> 106
Thr Gly His Val Cys Phe Ser Asp Ile Leu Gln Thr Pro Asp Gly Gly
1               5                   10                  15

Gln Leu Leu Leu Asp Trp Ala Lys Gln Pro Asp Ser Ser Gln Asp Pro
            20                  25                  30

Asp Pro Thr Thr Gln Pro Ile Val Leu Leu Leu Pro Gly Ile Thr Gly
        35                  40                  45

Ser Ser Gln Glu Thr Tyr Val Leu His Leu Val Asn Gln Ala Leu Arg
    50                  55                  60

Asp Gly Tyr Gln Ala Val Val Phe Asn Asn Arg Gly Cys Arg Gly Glu
65                  70                  75                  80

Glu Leu Arg Thr His Arg Ala Phe Cys Ala Ser Asn Thr Glu Asp Leu
                85                  90                  95

Glu Thr Val Val Asn His Ile Lys His Arg Tyr Pro Gln Ala Pro
            100                 105                 110

<210> 107
<211> 190
<212> PRT
<213> Homo sapiens

<220>
<221> SITE
<222> (154)
<223> Xaa equals any of the naturally occurring L-amino acids

<400> 107
Pro Ser Ser Thr Gln Thr Ala Lys Arg Val Thr Met Gly Trp Pro Arg
  1               5                  10                  15

Pro Gly Arg Ala Leu Val Ala Val Lys Ala Leu Leu Val Leu Ser Leu
             20                  25                  30

Leu Gln Val Pro Ala Gln Ala Val Val Arg Ala Val Leu Glu Asp Asn
         35                  40                  45

Ser Ser Ser Val Asp Phe Ala Asp Leu Pro Ala Leu Phe Gly Val Pro
    50                  55                  60

Leu Ala Pro Glu Gly Ile Arg Gly Tyr Leu Met Glu Val Lys Pro Ala
 65                  70                  75                  80

Asn Ala Cys His Pro Ile Glu Ala Pro Arg Leu Gly Asn Arg Ser Leu
                 85                  90                  95

Gly Ala Ile Val Leu Ile Arg Arg Tyr Asp Cys Thr Phe Asp Leu Lys
            100                 105                 110

Val Leu Asn Ala Gln Arg Ala Gly Phe Glu Ala Ala Ile Val His Asn
        115                 120                 125

Val His Ser Asp Asp Leu Val Ser Met Thr His Val Tyr Glu Asp Leu
    130                 135                 140

Arg Gly Gln Ile Ala Ile Pro Ser Val Xaa Val Ser Glu Ala Ala Arg
145                 150                 155                 160

Arg Thr Cys Gly Ser Ser Trp Ala Ala Thr Ser Arg Pro Thr Arg Cys
                165                 170                 175

Pro Ala Asp Asp Pro Pro Cys His Asp Leu Ala Val Thr Pro
            180                 185                 190

<210> 108
<211> 185
<212> PRT
<213> Homo sapiens

<400> 108
Met Gly Trp Pro Arg Pro Gly Arg Ala Leu Val Ala Val Lys Ala Leu
  1               5                  10                  15

Leu Val Leu Ser Leu Leu Gln Val Pro Ala Gln Ala Val Val Arg Ala

20                  25                  30

Val Leu Glu Asp Asn Ser Ser Ser Val Asp Phe Ala Asp Leu Pro Ala
        35                  40                  45

Leu Phe Gly Val Pro Leu Ala Pro Glu Gly Ile Arg Gly Tyr Leu Met
    50                  55                  60

Glu Val Lys Pro Ala Asn Ala Cys His Pro Ile Glu Ala Pro Arg Leu
65                  70                  75                  80

Gly Asn Arg Ser Leu Gly Ala Ile Val Leu Ile Arg Arg Tyr Asp Cys
                85                  90                  95

Thr Phe Asp Leu Lys Val Leu Asn Ala Gln Arg Ala Gly Phe Glu Ala
            100                 105                 110

Ala Ile Val His Asn Val His Ser Asp Asp Leu Val Ser Met Thr His
        115                 120                 125

Val Tyr Glu Asp Leu Arg Gly Gln Ile Ala Ile Pro Ser Val Phe Val
    130                 135                 140

Ser Glu Ala Ala Ser Gln Asp Leu Arg Val Ile Leu Gly Cys Asn Lys
145                 150                 155                 160

Ser Ala His Ala Leu Leu Leu Pro Asp Asp Pro Pro Cys His Asp Leu
                165                 170                 175

Gly Cys His Pro Val Leu Thr Val Ser
            180                 185


<210> 109
<211> 157
<212> PRT
<213> Homo sapiens

<400> 109
Val Val Arg Ala Val Leu Glu Asp Asn Ser Ser Ser Val Asp Phe Ala
1               5                   10                  15

Asp Leu Pro Ala Leu Phe Gly Val Pro Leu Ala Pro Glu Gly Ile Arg
            20                  25                  30

Gly Tyr Leu Met Glu Val Lys Pro Ala Asn Ala Cys His Pro Ile Glu
        35                  40                  45

Ala Pro Arg Leu Gly Asn Arg Ser Leu Gly Ala Ile Val Leu Ile Arg
    50                  55                  60

Arg Tyr Asp Cys Thr Phe Asp Leu Lys Val Leu Asn Ala Gln Arg Ala
65                  70                  75                  80

Gly Phe Glu Ala Ala Ile Val His Asn Val His Ser Asp Asp Leu Val
                85                  90                  95

Ser Met Thr His Val Tyr Glu Asp Leu Arg Gly Gln Ile Ala Ile Pro
            100                 105                 110

Ser Val Phe Val Ser Glu Ala Ala Ser Gln Asp Leu Arg Val Ile Leu
        115                 120                 125

Gly Cys Asn Lys Ser Ala His Ala Leu Leu Leu Pro Asp Asp Pro Pro
    130                 135                 140

Cys His Asp Leu Gly Cys His Pro Val Leu Thr Val Ser
145                 150                 155


<210> 110

<211> 16
<212> PRT
<213> Homo sapiens

<400> 110
Cys Lys Cys Ile Asp Pro Trp Phe Ser Gln Ala Pro Arg Arg Ser Cys
1               5                   10                  15

<210> 111
<211> 109
<212> PRT
<213> Homo sapiens

<400> 111
Met Thr Ala Pro Val Pro Ala Pro Arg Ile Leu Leu Pro Leu Leu Leu
1               5                   10                  15

Leu Leu Leu Leu Thr Pro Pro Pro Gly Ala Arg Gly Glu Val Cys Met
            20                  25                  30

Ala Ser Arg Gly Leu Ser Leu Phe Pro Glu Ser Cys Pro Asp Phe Cys
        35                  40                  45

Cys Gly Thr Cys Asp Asp Gln Tyr Cys Cys Ser Asp Val Leu Lys Lys
    50                  55                  60

Phe Val Trp Ser Glu Glu Arg Cys Ala Val Pro Glu Ala Ser Val Pro
65                  70                  75                  80

Ala Ser Val Glu Pro Val Glu Gln Leu Gly Ser Ala Leu Arg Phe Arg
                85                  90                  95

Pro Gly Tyr Asn Asp Pro Met Ser Gly Phe Gly Ala Thr
            100                 105

<210> 112
<211> 81
<212> PRT
<213> Homo sapiens

<400> 112
Glu Val Cys Met Ala Ser Arg Gly Leu Ser Leu Phe Pro Glu Ser Cys
1               5                   10                  15

Pro Asp Phe Cys Cys Gly Thr Cys Asp Asp Gln Tyr Cys Cys Ser Asp
            20                  25                  30

Val Leu Lys Lys Phe Val Trp Ser Glu Glu Arg Cys Ala Val Pro Glu
        35                  40                  45

Ala Ser Val Pro Ala Ser Val Glu Pro Val Glu Gln Leu Gly Ser Ala
    50                  55                  60

Leu Arg Phe Arg Pro Gly Tyr Asn Asp Pro Met Ser Gly Phe Gly Ala
65                  70                  75                  80

Thr

<210> 113
<211> 114
<212> PRT
<213> Homo sapiens

<400> 113

Cys Phe Thr Cys Ser Cys Cys Cys Leu Tyr Lys Thr Cys Arg Arg Pro
1               5                   10                  15

Arg Pro Val Val Thr Thr Thr Thr Ser Thr Thr Val Val His Ala Pro
            20                  25                  30

Tyr Pro Gln Pro Pro Ser Val Pro Pro Ser Tyr Pro Gly Pro Ser Tyr
        35                  40                  45

Gln Gly Tyr His Thr Met Pro Pro Gln Pro Gly Met Pro Ala Ala Pro
    50                  55                  60

Tyr Pro Met Gln Tyr Pro Pro Pro Tyr Pro Ala Gln Pro Met Gly Pro
65                  70                  75                  80

Pro Ala Tyr His Glu Thr Leu Ala Gly Gly Ala Ala Ala Pro Tyr Pro
                85                  90                  95

Ala Ser Gln Pro Pro Tyr Asn Pro Ala Tyr Met Asp Ala Pro Lys Ala
            100                 105                 110

Ala Leu

<210> 114
<211> 12
<212> PRT
<213> Homo sapiens

<400> 114
Gly Pro Val Gln Ala Pro Ala Ala Pro Pro Ser Leu
1               5                   10

<210> 115
<211> 160
<212> PRT
<213> Homo sapiens

<400> 115
Ser Arg Ala Arg Gly Ser Gly Cys Arg Ala Gly Thr Gly Ala Arg Gly
1               5                   10                  15

Ala Gly Ala Glu Gly Arg Glu Gly Glu Ala Cys Gly Thr Val Gly Leu
            20                  25                  30

Leu Leu Glu His Ser Phe Glu Ile Asp Asp Ser Ala Asn Phe Arg Lys
        35                  40                  45

Arg Gly Ser Leu Leu Trp Asn Gln Gln Asp Gly Thr Leu Ser Leu Ser
    50                  55                  60

Gln Arg Gln Leu Ser Glu Glu Glu Arg Gly Arg Leu Arg Asp Val Ala
65                  70                  75                  80

Ala Leu Asn Gly Leu Tyr Arg Val Arg Ile Pro Arg Arg Pro Gly Ala
                85                  90                  95

Leu Asp Gly Leu Glu Ala Gly Gly Tyr Val Ser Ser Phe Val Pro Ala
            100                 105                 110

Cys Ser Leu Val Glu Ser His Leu Ser Asp Gln Leu Thr Leu His Val
        115                 120                 125

Asp Val Ala Gly Asn Val Val Gly Val Ser Val Val Thr His Pro Met
    130                 135                 140

Ala Pro Cys Ser Pro Arg Gly Phe Pro Pro Ala His Gly Val Glu Pro
145                 150                 155                 160

<210> 116
<211> 276
<212> PRT
<213> Homo sapiens

<400> 116
Phe Ser Ser Gln Val Phe Glu Leu Phe Ser Gly Phe Leu Lys His Phe
1               5                   10                  15

Pro Gly Thr His Arg Gln Ile Tyr Arg Asn Leu Gln Glu Ile Asn Thr
            20                  25                  30

Phe Ile Thr Gln Ser Val Glu Lys His Arg Ala Thr Leu Asp Pro Ser
        35                  40                  45

Asn Pro Arg Asp Phe Ile Asp Val Tyr Leu Leu Arg Met Glu Lys Asp
    50                  55                  60

Lys Ser Asp Pro Ser Ser Glu Phe His His Gln Asn Leu Ile Leu Thr
65                  70                  75                  80

Val Leu Ser Leu Phe Phe Ala Gly Thr Glu Thr Thr Ser Thr Thr Leu
                85                  90                  95

Arg Tyr Gly Phe Leu Leu Met Leu Lys Tyr Pro His Val Thr Glu Arg
            100                 105                 110

Val Gln Lys Glu Ile Glu Gln Val Ile Gly Ser His Arg Pro Pro Ala
        115                 120                 125

Leu Asp Asp Arg Ala Lys Met Pro Tyr Thr Asp Ala Val Ile His Glu
    130                 135                 140

Ile Gln Arg Leu Gly Asp Leu Ile Pro Phe Gly Val Pro His Thr Val
145                 150                 155                 160

Thr Lys Asp Thr Gln Phe Arg Gly Tyr Val Ile Pro Lys Asn Thr Glu
                165                 170                 175

Val Phe Pro Val Leu Ser Ser Ala Leu His Asp Pro Arg Tyr Phe Lys
            180                 185                 190

Thr Pro Asn Thr Phe Asn Pro Gly His Phe Leu Asp Ala Asn Gly Ala
        195                 200                 205

Leu Lys Arg Asn Glu Gly Phe Met Pro Phe Ser Leu Gly Lys Arg Val
    210                 215                 220

Cys Leu Gly Glu Gly Ile Ala Arg Thr Glu Leu Phe Leu Phe Phe Thr
225                 230                 235                 240

Thr Ile Leu Gln Asn Phe Ser Ile Ala Ser Pro Val Pro Pro Glu Asp
                245                 250                 255

Ile Asp Leu Thr Pro Arg Glu Ser Gly Val Gly Asn Val Pro Pro Ser
            260                 265                 270

Tyr Gln Ile Arg
        275


<210> 117
<211> 277
<212> PRT
<213> Homo sapiens

<400> 117

Phe Ser Pro Gln Thr Tyr Glu Met Phe Ser Trp Phe Leu Arg Pro Leu
1               5                   10                  15

Pro Gly Pro His Lys Gln Leu Leu His His Val Ser Thr Leu Ala Ala
            20                  25                  30

Phe Thr Val Arg Gln Val Gln Gln His Gln Gly Asn Leu Asp Ala Ser
        35                  40                  45

Gly Pro Ala Arg Asp Leu Val Asp Ala Phe Leu Leu Lys Met Ala Gln
    50                  55                  60

Glu Glu Gln Asn Pro Gly Thr Glu Phe Thr Asn Lys Asn Met Leu Met
65                  70                  75                  80

Thr Val Ile Tyr Leu Leu Phe Ala Gly Thr Met Thr Val Ser Thr Thr
                85                  90                  95

Val Gly Tyr Thr Leu Leu Leu Leu Met Lys Tyr Pro His Val Gln Lys
            100                 105                 110

Trp Val Arg Glu Glu Leu Asn Arg Glu Leu Gly Ala Gly Gln Ala Pro
        115                 120                 125

Ser Leu Gly Asp Arg Thr Arg Leu Pro Tyr Thr Asp Ala Val Leu His
    130                 135                 140

Glu Ala Gln Arg Leu Leu Ala Leu Val Pro Met Gly Ile Pro Arg Thr
145                 150                 155                 160

Leu Met Arg Thr Thr Arg Phe Arg Gly Tyr Thr Leu Pro Gln Gly Thr
                165                 170                 175

Glu Val Phe Pro Leu Leu Gly Ser Ile Leu His Asp Pro Asn Ile Phe
            180                 185                 190

Lys His Pro Glu Glu Phe Asn Pro Asp Arg Phe Leu Asp Ala Asp Gly
        195                 200                 205

Arg Phe Arg Lys His Glu Ala Phe Leu Pro Phe Ser Leu Gly Lys Arg
    210                 215                 220

Val Cys Leu Gly Glu Gly Leu Ala Lys Ala Glu Leu Phe Leu Phe Phe
225                 230                 235                 240

Thr Thr Ile Leu Gln Ala Phe Ser Leu Glu Ser Pro Cys Pro Pro Asp
                245                 250                 255

Thr Leu Ser Leu Lys Pro Thr Val Ser Gly Leu Phe Asn Ile Pro Pro
            260                 265                 270

Ala Phe Gln Leu Gln
        275

<210> 118
<211> 310
<212> PRT
<213> Homo sapiens

<400> 118


<210> 119
<211> 670
<212> PRT
<213> Homo sapiens

<220>
<221> SITE
<222> (216)
<223> Xaa equals any of the naturally occurring L-amino acids

<400> 119
Met Ala Thr Ala Gly Gly Gly Ser Gly Ala Asp Pro Gly Ser Arg Gly
  1               5                  10                  15

Leu Leu Arg Leu Leu Ser Phe Cys Val Leu Leu Ala Gly Leu Cys Arg
             20                  25                  30

Gly Asn Ser Val Glu Arg Lys Ile Tyr Ile Pro Leu Asn Lys Thr Ala
         35                  40                  45

Pro Cys Val Arg Leu Leu Asn Ala Thr His Gln Ile Gly Cys Gln Ser
    50                  55                  60

Ser Ile Ser Gly Asp Thr Gly Val Ile His Val Val Glu Lys Glu Glu
65                  70                  75                  80

Asp Leu Gln Trp Val Leu Thr Asp Gly Pro Asn Pro Pro Tyr Met Val
                85                  90                  95

Leu Leu Glu Ser Lys His Phe Thr Arg Asp Leu Met Glu Lys Leu Lys
            100                 105                 110

Gly Arg Thr Ser Arg Ile Ala Gly Leu Ala Val Ser Leu Thr Lys Pro
        115                 120                 125

Ser Pro Ala Ser Gly Phe Ser Pro Ser Val Gln Cys Pro Asn Asp Gly
    130                 135                 140

Phe Gly Val Tyr Ser Asn Ser Tyr Gly Pro Glu Phe Ala His Cys Arg
145                 150                 155                 160

Glu Ile Gln Trp Asn Ser Leu Gly Asn Gly Leu Ala Tyr Glu Asp Phe
                165                 170                 175

Ser Phe Pro Ile Phe Leu Leu Glu Asp Glu Asn Glu Thr Lys Val Ile
            180                 185                 190

Lys Gln Cys Tyr Gln Asp His Asn Leu Ser Gln Asn Gly Ser Ala Pro
        195                 200                 205

Ser Phe Pro Leu Cys Ala Met Xaa Leu Phe Ser His Met His Ala Val
    210                 215                 220

Ile Ser Thr Ala Thr Cys Met Arg Arg Ser Ser Ile Gln Ser Thr Phe
225                 230                 235                 240

Ser Ile Asn Pro Glu Ile Val Cys Asp Pro Leu Ser Asp Tyr Asn Val
                245                 250                 255

Trp Ser Met Leu Lys Pro Ile Asn Thr Thr Gly Thr Leu Lys Pro Asp
            260                 265                 270

Asp Arg Val Val Val Ala Ala Thr Arg Leu Asp Ser Arg Ser Phe Phe
        275                 280                 285

Trp Asn Val Ala Pro Gly Ala Glu Ser Ala Val Ala Ser Phe Val Thr
    290                 295                 300

Gln Leu Ala Ala Ala Glu Ala Leu Gln Lys Ala Pro Asp Val Thr Thr
305                 310                 315                 320

Leu Pro Arg Asn Val Met Phe Val Phe Phe Gln Gly Glu Thr Phe Asp
                325                 330                 335

Tyr Ile Gly Ser Ser Arg Met Val Tyr Asp Met Glu Lys Gly Lys Phe
            340                 345                 350

Pro Val Gln Leu Glu Asn Val Asp Ser Phe Val Glu Leu Gly Gln Val
        355                 360                 365

Ala Leu Arg Thr Ser Leu Glu Leu Trp Met His Thr Asp Pro Val Ser
    370                 375                 380

Gln Lys Asn Glu Ser Val Arg Asn Gln Val Glu Asp Leu Leu Ala Thr
385                 390                 395                 400

Leu Glu Lys Ser Gly Ala Gly Val Pro Ala Val Ile Leu Arg Arg Pro
                405                 410                 415

Asn Gln Ser Gln Pro Leu Pro Pro Ser Ser Leu Gln Arg Phe Leu Arg
            420                 425                 430

Ala Arg Asn Ile Ser Gly Val Val Leu Ala Asp His Ser Gly Ala Phe
        435                 440                 445

His Asn Lys Tyr Tyr Gln Ser Ile Tyr Asp Thr Ala Glu Asn Ile Asn
    450                 455                 460

Val Ser Tyr Pro Glu Trp Leu Ser Pro Glu Glu Asp Leu Asn Phe Val
465                 470                 475                 480

Thr Asp Thr Ala Lys Ala Leu Ala Asp Val Ala Thr Val Leu Gly Arg
                485                 490                 495

Ala Leu Tyr Glu Leu Ala Gly Gly Thr Asn Phe Ser Asp Thr Val Gln
            500                 505                 510

Ala Asp Pro Gln Thr Val Thr Arg Leu Leu Tyr Gly Phe Leu Ile Lys
        515                 520                 525

Ala Asn Asn Ser Trp Phe Gln Ser Ile Leu Arg Gln Asp Leu Arg Ser
    530                 535                 540

Tyr Leu Gly Asp Gly Pro Leu Gln His Tyr Ile Ala Val Ser Ser Pro
545                 550                 555                 560

Thr Asn Thr Thr Tyr Val Val Gln Tyr Ala Leu Ala Asn Leu Thr Gly
                565                 570                 575

Thr Val Val Asn Leu Thr Arg Glu Gln Cys Gln Asp Pro Ser Lys Val
            580                 585                 590

Pro Ser Glu Asn Lys Asp Leu Tyr Glu Tyr Ser Trp Val Gln Gly Pro
        595                 600                 605

Leu His Ser Asn Glu Thr Asp Arg Leu Pro Arg Cys Val Arg Ser Thr
    610                 615                 620

Ala Arg Leu Ala Arg Ala Leu Ser Pro Ala Phe Glu Leu Ser Gln Trp
625                 630                 635                 640

Ser Ser Thr Glu Tyr Ser Thr Trp Thr Glu Ser Arg Trp Lys Asp Ile
                645                 650                 655

Arg Ala Arg Ile Phe Leu Ile Ala Ser Lys Glu Leu Glu Leu
            660                 665                 670


<210> 120
<211> 34
<212> PRT
<213> Homo sapiens

<220>
<221> SITE
<222> (18)
<223> Xaa equals any of the naturally occurring L-amino acids

<220>
<221> SITE
<222> (24)
<223> Xaa equals any of the naturally occurring L-amino acids

<400> 120
Pro Glu Asp Gly Lys Ala Asp Ile Val Arg Ala Ala Gln Asp Phe Cys
  1               5                  10                  15

Gln Xaa Val Ala Gln Lys Gln Xaa Arg Pro Thr Asp Leu Asp Val Asp
             20                  25                  30

Thr Leu

<210> 121
<211> 22
<212> PRT
<213> Homo sapiens

<400> 121
Phe Ala Asn Ser Asn Asp Lys Asp Asp Gln Val Leu Asn Cys His Leu
1               5                   10                  15

Ala Val Lys Val Leu Ser
            20


<210> 122
<211> 12
<212> PRT
<213> Homo sapiens

<400> 122
Val Tyr Leu Val Gln Met Val Val Leu Ile Leu Ile
1               5                   10


<210> 123
<211> 116
<212> PRT
<213> Homo sapiens

<400> 123
Met Thr Gly Leu Tyr Glu Leu Val Trp Arg Val Leu His Ala Leu Leu
1               5                   10                  15

Cys Leu His Arg Thr Leu Thr Ser Trp Leu Arg Val Arg Phe Gly Thr
            20                  25                  30

Trp Asn Trp Ile Trp Arg Arg Cys Cys Arg Ala Ala Ser Ala Ala Val
        35                  40                  45

Leu Ala Pro Leu Gly Phe Thr Leu Arg Lys Pro Pro Ala Val Gly Arg
    50                  55                  60

Asn Arg Arg His His Arg His Pro Arg Gly Gly Ser Cys Leu Ala Ala
65                  70                  75                  80

Ala His His Arg Met Arg Trp Arg Ala Asp Gly Arg Ser Leu Glu Lys
                85                  90                  95

Leu Pro Val His Met Gly Leu Val Ile Thr Glu Val Glu Gln Glu Pro
            100                 105                 110

Ser Phe Ser Asp
        115


<210> 124
<211> 93
<212> PRT
<213> Homo sapiens

<400> 124
Ser Trp Leu Arg Val Arg Phe Gly Thr Trp Asn Trp Ile Trp Arg Arg
1               5                   10                  15

Cys Cys Arg Ala Ala Ser Ala Ala Val Leu Ala Pro Leu Gly Phe Thr
            20                  25                  30

Leu Arg Lys Pro Pro Ala Val Gly Arg Asn Arg Arg His His Arg His
        35                  40                  45

Pro Arg Gly Gly Ser Cys Leu Ala Ala Ala His His Arg Met Arg Trp
50                  55                  60

Arg Ala Asp Gly Arg Ser Leu Glu Lys Leu Pro Val His Met Gly Leu
65                  70                  75                  80

Val Ile Thr Glu Val Glu Gln Glu Pro Ser Phe Ser Asp
                85                  90

<210> 125
<211> 160
<212> PRT
<213> Homo sapiens

<400> 125
Val Tyr Asp His Gln Gly Ile Phe Lys Arg Asn Asn Ser Arg Leu Met
1               5                   10                  15

Asp Glu Ile Leu Lys Gln Gln Gln Glu Leu Leu Gly Leu Asp Cys Ser
            20                  25                  30

Lys Tyr Ser Pro Glu Phe Ala Asn Ser Asn Asp Lys Asp Asp Gln Val
        35                  40                  45

Leu Asn Cys His Leu Ala Val Lys Val Leu Ser Pro Glu Asp Gly Lys
    50                  55                  60

Ala Asp Ile Val Arg Ala Ala Gln Asp Phe Cys Gln Leu Val Ala Gln
65                  70                  75                  80

Lys Gln Lys Arg Pro Thr Asp Leu Asp Val Asp Thr Leu Ala Ser Leu
                85                  90                  95

Leu Ser Ser Asn Gly Cys Pro Asp Pro Asp Leu Val Leu Lys Phe Gly
            100                 105                 110

Pro Val Asp Ser Thr Leu Gly Phe Leu Pro Trp His Ile Arg Leu Thr
        115                 120                 125

Glu Ile Val Ser Leu Pro Ser His Leu Asn Ile Ser Tyr Glu Asp Phe
    130                 135                 140

Phe Ser Ala Leu Arg Gln Tyr Ala Ala Cys Glu Gln Arg Leu Gly Lys
145                 150                 155                 160

<210> 126
<211> 149
<212> PRT
<213> Homo sapiens

<220>
<221> SITE
<222> (61)
<223> Xaa equals any of the naturally occurring L-amino acids

<220>
<221> SITE
<222> (104)
<223> Xaa equals any of the naturally occurring L-amino acids

<220>
<221> SITE
<222> (122)
<223> Xaa equals any of the naturally occurring L-amino acids

<220>

<221> SITE
<222> (136)
<223> Xaa equals any of the naturally occurring L-amino acids

<220>
<221> SITE
<222> (144)
<223> Xaa equals any of the naturally occurring L-amino acids

<220>
<221> SITE
<222> (148)
<223> Xaa equals any of the naturally occurring L-amino acids

<400> 126
Ser Gly Ile Phe Ser Gly Leu Asn Leu Gly Leu Met Ala Leu Asp Pro
1               5                   10                  15

Met Glu Leu Arg Ile Val Gln Asn Cys Gly Thr Glu Lys Glu Arg Arg
            20                  25                  30

Tyr Ala Arg Lys Ile Glu Pro Ile Arg Arg Lys Gly Asn Tyr Leu Leu
        35                  40                  45

Cys Ser Leu Leu Leu Gly Asn Val Leu Val Asn Thr Xaa Leu Thr Ile
    50                  55                  60

Leu Leu Asp Asn Leu Ile Gly Ser Gly Leu Met Ala Val Ala Ser Ser
65                  70                  75                  80

Thr Ile Gly Ile Val Ile Phe Gly Glu Ile Leu Pro Gln Ala Leu Cys
                85                  90                  95

Ser Arg His Gly Leu Ala Val Xaa Ala Asn Thr Ile Leu Leu Thr Lys
            100                 105                 110

Phe Phe Met Leu Leu Thr Phe Pro Leu Xaa Phe Pro Ile Ser Lys Leu
        115                 120                 125

Leu Asp Phe Phe Leu Gly Gln Xaa Ile Arg Thr Val Tyr Asn Arg Xaa
    130                 135                 140

Lys Leu Met Xaa Met
145


<210> 127
<211> 97
<212> PRT
<213> Homo sapiens

<400> 127
Met Val Glu Glu Pro Gly Arg Phe Leu Pro Leu Trp Leu His Ile Leu
1               5                   10                  15

Leu Ile Thr Val Leu Leu Val Leu Ser Gly Ile Phe Ser Gly Leu Asn
            20                  25                  30

Leu Gly Leu Met Ala Leu Asp Pro Met Glu Leu Arg Ile Val Gln Asn
        35                  40                  45

Cys Gly Thr Glu Lys Glu Arg Arg Tyr Ala Arg Lys Ile Glu Pro Ile
    50                  55                  60

Arg Arg Lys Gly Asn Tyr Leu Leu Cys Ser Leu Leu Leu Gly Asn Val
65                  70                  75                  80

Leu Val Asn Thr Ser Leu Thr Ile Leu Leu Asp Asn Leu Ile Gly Ser
                85                  90                  95

Gly

<210> 128
<211> 68
<212> PRT
<213> Homo sapiens

<400> 128
Gly Leu Asn Leu Gly Leu Met Ala Leu Asp Pro Met Glu Leu Arg Ile
1               5                   10                  15

Val Gln Asn Cys Gly Thr Glu Lys Glu Arg Arg Tyr Ala Arg Lys Ile
            20                  25                  30

Glu Pro Ile Arg Arg Lys Gly Asn Tyr Leu Leu Cys Ser Leu Leu Leu
        35                  40                  45

Gly Asn Val Leu Val Asn Thr Ser Leu Thr Ile Leu Leu Asp Asn Leu
    50                  55                  60

Ile Gly Ser Gly
65


<210> 129
<211> 490
<212> PRT
<213> Homo sapiens

<400> 129
Leu Pro Gln Ala Leu Cys Ser Arg His Gly Leu Ala Val Gly Ala Asn
1               5                   10                  15

Thr Ile Leu Leu Thr Lys Phe Phe Met Leu Leu Thr Phe Pro Leu Ser
            20                  25                  30

Phe Pro Ile Ser Lys Leu Leu Asp Phe Phe Leu Gly Gln Glu Ile Arg
        35                  40                  45

Thr Val Tyr Asn Arg Glu Lys Leu Met Glu Met Leu Lys Val Thr Glu
    50                  55                  60

Pro Tyr Asn Asp Leu Val Lys Glu Glu Leu Asn Met Ile Gln Gly Ala
65                  70                  75                  80

Leu Glu Leu Arg Thr Lys Thr Val Glu Asp Ile Met Thr Gln Leu Gln
                85                  90                  95

Asp Cys Phe Met Ile Arg Ser Asp Ala Ile Leu Asp Phe Asn Thr Met
            100                 105                 110

Ser Glu Ile Met Glu Ser Gly Tyr Thr Arg Ile Pro Val Phe Glu Asp
        115                 120                 125

Glu Gln Ser Asn Ile Val Asp Ile Leu Tyr Val Lys Asp Leu Ala Phe
    130                 135                 140

Val Asp Pro Asp Asp Cys Thr Pro Leu Lys Thr Ile Thr Arg Phe Tyr
145                 150                 155                 160

Asn His Pro Val His Phe Val Phe His Asp Thr Lys Leu Asp Ala Met
                165                 170                 175

Leu Glu Glu Phe Lys Lys Gly Lys Ser His Leu Ala Ile Val Gln Lys
            180                 185                 190

Val Asn Asn Glu Gly Glu Gly Asp Pro Phe Tyr Glu Val Leu Gly Leu
        195                 200                 205

Val Thr Leu Glu Asp Val Ile Glu Glu Ile Ile Lys Ser Glu Ile Leu

210                 215                 220

Asp Glu Ser Asp Met Tyr Thr Asp Asn Arg Ser Arg Lys Arg Val Ser
225                 230                 235                 240

Glu Lys Asn Lys Arg Asp Phe Ser Ala Phe Lys Asp Ala Asp Asn Glu
                245                 250                 255

Leu Lys Val Lys Ile Ser Pro Gln Leu Leu Leu Ala Ala His Arg Phe
            260                 265                 270

Leu Ala Thr Glu Val Ser Gln Phe Ser Pro Ser Leu Ile Ser Glu Lys
        275                 280                 285

Ile Leu Leu Arg Leu Leu Lys Tyr Pro Asp Val Ile Gln Glu Leu Lys
    290                 295                 300

Phe Asp Glu His Asn Lys Tyr Tyr Ala Arg His Tyr Leu Tyr Thr Arg
305                 310                 315                 320

Asn Lys Pro Ala Asp Tyr Phe Ile Leu Ile Leu Gln Gly Lys Val Glu
                325                 330                 335

Val Glu Ala Gly Lys Glu Asn Met Lys Phe Glu Thr Gly Ala Phe Ser
            340                 345                 350

Tyr Tyr Gly Thr Met Ala Leu Thr Ser Val Pro Ser Asp Arg Ser Pro
        355                 360                 365

Ala His Pro Thr Pro Leu Ser Arg Ser Ala Ser Leu Ser Tyr Pro Asp
    370                 375                 380

Arg Thr Asp Val Ser Thr Ala Ala Thr Leu Ala Gly Ser Ser Asn Gln
385                 390                 395                 400

Phe Gly Ser Ser Val Leu Gly Gln Tyr Ile Ser Asp Phe Ser Val Arg
                405                 410                 415

Ala Leu Val Asp Leu Gln Tyr Ile Lys Ile Thr Arg Gln Gln Tyr Gln
            420                 425                 430

Asn Gly Leu Leu Ala Ser Arg Met Glu Asn Ser Pro Gln Phe Pro Ile
        435                 440                 445

Asp Gly Cys Thr Thr His Met Glu Asn Leu Ala Glu Lys Ser Glu Leu
    450                 455                 460

Pro Val Val Asp Glu Thr Thr Thr Leu Leu Asn Glu Arg Asn Ser Leu
465                 470                 475                 480

Leu His Lys Ala Ser His Glu Asn Ala Ile
                485                 490


<210> 130
<211> 172
<212> PRT
<213> Homo sapiens

<400> 130
Met Pro Arg Ala Ser Cys Cys His Cys Leu Cys Gly Pro Glu Ser Trp
  1               5                  10                  15

Thr Cys Pro Ser Asp Ser Gly Val Ala Ala Trp Ala Gly Ser Val Thr
             20                  25                  30

Leu Gly Cys Tyr Thr Phe Trp Glu Leu Asp Arg Ser Gly Cys Leu Gly
         35                  40                  45

Gly Leu Trp His Pro Gly Met Leu Tyr Ile Leu Gly Thr Gly Gln Glu
     50                  55                  60

Trp Leu Leu Gly Trp Ala Leu Ala Pro Trp Asp Val Ile His Ser Gly
65                  70                  75                  80

Asn Cys Asn Gln Pro Leu Glu Lys Ser Glu Leu Gln Glu Val Thr Leu
                85                  90                  95

Gly Trp Asp Leu Gly Thr Trp Pro Gly Gln His Gly Asp Thr Arg Leu
            100                 105                 110

Gln Gln Glu Leu Trp Ser Val Leu Gly Ser Leu Gly Ala Gly Leu Arg
        115                 120                 125

Pro Trp Ala Gly Phe Leu Gln Ala Glu Val Leu Gly Lys Trp Gly Ser
    130                 135                 140

Gln Ala Ser Cys Arg Leu Pro His Tyr Val Ala Ser Asp Ser Ser Ser
145                 150                 155                 160

Leu Met Lys Ala Ile Trp Phe Ile Thr Leu Lys Leu
                165                 170


<210> 131
<211> 56
<212> PRT
<213> Homo sapiens

<400> 131
Trp Gly Thr Leu Ser Val Cys Leu Pro Ser Gln Gly Arg Ala Gly Gln
 1               5                  10                  15

Asp Cys Leu Gly Met Ser Arg Lys Thr Ala Pro Arg Gly Gly Leu Tyr
            20                  25                  30

Gly Gln Glu Gly Glu Ala Ser Val Glu Glu Ala Ser Cys Glu Asn His
        35                  40                  45

Val Leu His Pro Arg Tyr Pro Val
    50                  55


<210> 132
<211> 57
<212> PRT
<213> Homo sapiens

<400> 132
Trp Gly Ala Leu Ser Val Cys Leu Pro Ser Gln Gly Arg Ala Ser Gln
 1               5                  10                  15

Asp Cys Leu Gly Met Ser Arg Lys Lys Leu Arg Pro Gly Gly Gly Leu
            20                  25                  30

Tyr Gly Gln Glu Gly Glu Ala Pro Val Glu Glu Ala Gly Cys Ala Asp
        35                  40                  45

His Val Met Leu Pro Arg His Pro Val
    50                  55


<210> 133
<211> 704
<212> PRT
<213> Homo sapiens

<400> 133
Ile Pro Gly Ser Thr His Ala Ser Ala Asp Ser Val Ser Pro Pro Pro
 1               5                  10                  15

Ser Val Ile Thr Gln Glu Asn Leu Gly Arg Val Lys Arg Leu Gly Cys

            20                  25                  30

Phe Asp His Ala Gln Arg Gln Leu Gly Glu Arg Cys Leu Tyr Val Phe
        35                  40                  45

Pro Ala Lys Val Glu Pro Ala Lys Ile Ala Trp Glu Cys Pro Glu Lys
    50                  55                  60

Asn Cys Ala Gln Gly Glu Gly Ser Met Ala Lys Lys Val Lys Arg Leu
65                  70                  75                  80

Leu Lys Lys Gln Val Val Arg Ile Met Ser Cys Ser Pro Asp Thr Gln
                85                  90                  95

Cys Ser Arg Asp His Ser Met Glu Asp Pro Asp Lys Lys Gly Glu Ser
            100                 105                 110

Lys Thr Lys Ser Glu Ala Glu Ser Ala Ser Pro Glu Glu Thr Pro Asp
        115                 120                 125

Gly Ser Ala Ser Pro Val Glu Met Gln Asp Glu Gly Ala Glu Glu Pro
    130                 135                 140

His Glu Ala Gly Glu Gln Leu Pro Pro Phe Leu Leu Lys Glu Gly Arg
145                 150                 155                 160

Asp Asp Arg Leu His Ser Ala Glu Gln Asp Ala Asp Asp Glu Ala Ala
                165                 170                 175

Asp Asp Thr Asp Asp Thr Ser Ser Val Thr Ser Ser Ala Ser Ser Thr
            180                 185                 190

Thr Ser Ser Gln Ser Gly Ser Gly Thr Ser Arg Lys Lys Ser Ile Pro
        195                 200                 205

Leu Ser Ile Lys Asn Leu Lys Arg Lys His Lys Arg Lys Lys Asn Lys
    210                 215                 220

Ile Thr Arg Asp Phe Lys Pro Gly Asp Arg Val Ala Val Glu Val Val
225                 230                 235                 240

Thr Thr Met Thr Ser Ala Asp Val Met Trp Gln Asp Gly Ser Val Glu
                245                 250                 255

Cys Asn Ile Arg Ser Asn Asp Leu Phe Pro Val His His Leu Asp Asn
            260                 265                 270

Asn Glu Phe Cys Pro Gly Asp Phe Val Val Asp Lys Arg Val Gln Ser
        275                 280                 285

Cys Pro Asp Pro Ala Val Tyr Gly Val Val Gln Ser Gly Asp His Ile
    290                 295                 300

Gly Arg Thr Cys Met Val Lys Trp Phe Lys Leu Arg Pro Ser Gly Asp
305                 310                 315                 320

Asp Val Glu Leu Ile Gly Glu Glu Glu Asp Val Ser Val Tyr Asp Ile
                325                 330                 335

Ala Asp His Pro Asp Phe Arg Phe Arg Thr Thr Asp Ile Val Ile Arg
            340                 345                 350

Ile Gly Asn Thr Glu Asp Gly Ala Pro His Lys Glu Asp Glu Pro Ser
        355                 360                 365

Val Gly Gln Val Ala Arg Val Asp Val Ser Ser Lys Val Glu Val Val
    370                 375                 380

Trp Ala Asp Asn Ser Lys Thr Ile Ile Leu Pro Gln His Leu Tyr Asn
385                 390                 395                 400

Ile Glu Ser Glu Ile Glu Glu Ser Asp Tyr Asp Ser Val Glu Gly Ser
                405                 410                 415

Thr Ser Gly Ala Ser Ser Asp Glu Trp Glu Asp Asp Ser Asp Ser Trp
            420                 425                 430

Glu Thr Asp Asn Gly Leu Val Glu Asp Glu His Pro Lys Ile Glu Glu
        435                 440                 445

Pro Pro Ile Pro Pro Leu Glu Gln Pro Val Ala Pro Glu Asp Lys Gly
    450                 455                 460

Val Val Ile Ser Glu Glu Ala Ala Thr Ala Ala Val Gln Gly Ala Val
465                 470                 475                 480

Ala Met Ala Ala Pro Met Ala Gly Leu Met Glu Lys Ala Gly Lys Asp
                485                 490                 495

Gly Pro Pro Lys Ser Phe Arg Glu Leu Lys Glu Ala Ile Lys Ile Leu
            500                 505                 510

Glu Ser Leu Lys Asn Met Thr Val Glu Gln Leu Leu Thr Gly Ser Pro
        515                 520                 525

Thr Ser Pro Thr Val Glu Pro Glu Lys Pro Thr Arg Glu Lys Lys Phe
    530                 535                 540

Leu Asp Asp Ile Lys Lys Leu Gln Glu Asn Leu Lys Lys Thr Leu Asp
545                 550                 555                 560

Asn Val Ala Ile Val Glu Glu Glu Lys Met Glu Ala Val Pro Asp Val
                565                 570                 575

Glu Arg Lys Glu Asp Lys Pro Glu Gly Gln Ser Pro Val Lys Ala Glu
            580                 585                 590

Trp Pro Ser Glu Thr Pro Val Leu Cys Gln Gln Cys Gly Gly Lys Pro
        595                 600                 605

Gly Val Thr Phe Thr Ser Ala Lys Gly Glu Val Phe Ser Val Leu Glu
    610                 615                 620

Phe Ala Pro Ser Asn His Ser Phe Lys Lys Ile Glu Phe Gln Pro Pro
625                 630                 635                 640

Glu Ala Lys Lys Phe Phe Ser Thr Val Arg Lys Glu Met Ala Leu Leu
                645                 650                 655

Ala Thr Ser Leu Pro Glu Gly Ile Met Val Lys Thr Phe Glu Asp Arg
            660                 665                 670

Met Ala Ser Arg Leu Gly Met Gly Arg Cys Arg Cys Ala Val Trp Ala
        675                 680                 685

Pro Ser Ala Gln Glu Ala Thr Asn Leu Gly Ser Pro Asn Leu Arg Gly
    690                 695                 700

<210> 134
<211> 632
<212> PRT
<213> Homo sapiens

<400> 134
Met Ala Lys Lys Val Lys Arg Leu Leu Lys Lys Gln Val Val Arg Ile
1               5                   10                  15

Met Ser Cys Ser Pro Asp Thr Gln Cys Ser Arg Asp His Ser Met Glu

                20                  25                  30

Asp Pro Asp Lys Lys Gly Glu Ser Lys Thr Lys Ser Glu Ala Glu Ser
            35                  40                  45

Ala Ser Pro Glu Glu Thr Pro Asp Gly Ser Ala Ser Pro Val Glu Met
        50                  55                  60

Gln Asp Glu Gly Ala Glu Glu Pro His Glu Ala Gly Glu Gln Leu Pro
65                  70                  75                  80

Pro Phe Leu Leu Lys Glu Gly Arg Asp Asp Arg Leu His Ser Ala Glu
                85                  90                  95

Gln Asp Ala Asp Asp Glu Ala Ala Asp Asp Thr Asp Asp Thr Ser Ser
            100                 105                 110

Val Thr Ser Ser Ala Ser Ser Thr Thr Ser Ser Gln Ser Gly Ser Gly
        115                 120                 125

Thr Ser Arg Lys Lys Ser Ile Pro Leu Ser Ile Lys Asn Leu Lys Arg
    130                 135                 140

Lys His Lys Arg Lys Lys Asn Lys Ile Thr Arg Asp Phe Lys Pro Gly
145                 150                 155                 160

Asp Arg Val Ala Val Glu Val Val Thr Thr Met Thr Ser Ala Asp Val
                165                 170                 175

Met Trp Gln Asp Gly Ser Val Glu Cys Asn Ile Arg Ser Asn Asp Leu
            180                 185                 190

Phe Pro Val His His Leu Asp Asn Asn Glu Phe Cys Pro Gly Asp Phe
        195                 200                 205

Val Val Asp Lys Arg Val Gln Ser Cys Pro Asp Pro Ala Val Tyr Gly
    210                 215                 220

Val Val Gln Ser Gly Asp His Ile Gly Arg Thr Cys Met Val Lys Trp
225                 230                 235                 240

Phe Lys Leu Arg Pro Ser Gly Asp Asp Val Glu Leu Ile Gly Glu Glu
                245                 250                 255

Glu Asp Val Ser Val Tyr Asp Ile Ala Asp His Pro Asp Phe Arg Phe
            260                 265                 270

Arg Thr Thr Asp Ile Val Ile Arg Ile Gly Asn Thr Glu Asp Gly Ala
        275                 280                 285

Pro His Lys Glu Asp Glu Pro Ser Val Gly Gln Val Ala Arg Val Asp
    290                 295                 300

Val Ser Ser Lys Val Glu Val Val Trp Ala Asp Asn Ser Lys Thr Ile
305                 310                 315                 320

Ile Leu Pro Gln His Leu Tyr Asn Ile Glu Ser Glu Ile Glu Glu Ser
                325                 330                 335

Asp Tyr Asp Ser Val Glu Gly Ser Thr Ser Gly Ala Ser Ser Asp Glu
            340                 345                 350

Trp Glu Asp Asp Ser Asp Ser Trp Glu Thr Asp Asn Gly Leu Val Glu
        355                 360                 365

Asp Glu His Pro Lys Ile Glu Glu Pro Pro Ile Pro Pro Leu Glu Gln
    370                 375                 380

Pro Val Ala Pro Glu Asp Lys Gly Val Val Ile Ser Glu Glu Ala Ala
385                 390                 395                 400

Thr Ala Ala Val Gln Gly Ala Val Ala Met Ala Ala Pro Met Ala Gly
                405                 410                 415

Leu Met Glu Lys Ala Gly Lys Asp Gly Pro Pro Lys Ser Phe Arg Glu
            420                 425                 430

Leu Lys Glu Ala Ile Lys Ile Leu Glu Ser Leu Lys Asn Met Thr Val
        435                 440                 445

Glu Gln Leu Leu Thr Gly Ser Pro Thr Ser Pro Thr Val Glu Pro Glu
    450                 455                 460

Lys Pro Thr Arg Glu Lys Lys Phe Leu Asp Asp Ile Lys Lys Leu Gln
465                 470                 475                 480

Glu Asn Leu Lys Lys Thr Leu Asp Asn Val Ala Ile Val Glu Glu Glu
                485                 490                 495

Lys Met Glu Ala Val Pro Asp Val Glu Arg Lys Glu Asp Lys Pro Glu
            500                 505                 510

Gly Gln Ser Pro Val Lys Ala Glu Trp Pro Ser Glu Thr Pro Val Leu
        515                 520                 525

Cys Gln Gln Cys Gly Gly Lys Pro Gly Val Thr Phe Thr Ser Ala Lys
    530                 535                 540

Gly Glu Val Phe Ser Val Leu Glu Phe Ala Pro Ser Asn His Ser Phe
545                 550                 555                 560

Lys Lys Ile Glu Phe Gln Pro Pro Glu Ala Lys Lys Phe Phe Ser Thr
                565                 570                 575

Val Arg Lys Glu Met Ala Leu Leu Ala Thr Ser Leu Pro Glu Gly Ile
            580                 585                 590

Met Val Lys Thr Phe Glu Asp Arg Met Ala Ser Arg Leu Gly Met Gly
        595                 600                 605

Arg Cys Arg Cys Ala Val Trp Ala Pro Ser Ala Gln Glu Ala Thr Asn
    610                 615                 620

Leu Gly Ser Pro Asn Leu Arg Gly
625                 630

1

## Description

### Field of the Invention

This invention relates to newly identified polynucleotides, polypeptides encoded by these polynucleotides, antibodies that bind these polypeptides, uses of such polynucleotides, polypeptides, and antibodies, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space-a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical diseases, disorders, and/or conditions by using secreted proteins or the genes that encode them.

### Summary of the Invention

The present invention relates to novel polynucleotides and the encoded polypeptides. Moreover, the present invention relates to vectors, host cells, antibodies, and recombinant and synthetic methods for producing the polypeptides and polynucleotides. Also provided are diagnostic methods for detecting diseases, disorders, and/or conditions related to the polypeptides and polynucleotides, and therapeutic methods for treating such diseases, disorders, and/or conditions. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide. The term "isolated" does not refer to genomic or cDNA libraries, whole cell total or mRNA preparations, genomic DNA preparations (including those separated by electrophoresis and transferred onto blots), sheared whole cell genomic DNA preparations or other compositions where the art demonstrates no distinguishing features of the polynucleotide/sequences of the present invention.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

In specific embodiments, the polynucleotides of the invention are at least 15, at least 30, at least 50, at least 100, at least 125, at least 500, or at least 1000 continuous nucleotides but are less than or equal to 300 kb, 200 kb, 100 kb, 50 kb, 15 kb, 10 kb, 7.5 kb, 5 kb, 2.5 kb, 2.0 kb, or 1 kb, in length. In a further embodiment, polynucleotides of the invention comprise a portion of the coding sequences, as disclosed herein, but do not comprise all or a portion of any intron. In another embodiment, the polynucleotides comprising coding sequences do not contain coding sequences of a genomic flanking gene (i.e., 5' or 3' to the gene of interest in the genome). In other embodiments, the polynucleotides of the invention do not contain the coding sequence of more than 1000, 500, 250,100, 50, 25, 20, 15, 10, 5, 4, 3, 2, or 1 genomic flanking gene(s).

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA Within the clone deposited with the ATCC. "Stringent hybridization conditions" refers to an overnight incubation at 42 degree C in a solution comprising 50% formamide, 5x SSC (750 mM NaCl, 75 mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65 degree C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37 degree C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50 degree C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone generated using oligo dT as a primer).

The polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single and double-stranded regions, single- and double-stranded RNA, and RNA that is mixture of single- and double-stranded regions, hybrid molecules comprising DNA and RNA that may be single-stranded or, more typically, double-stranded or a mixture of single-and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

The translation product of this gene shares sequence homology with a family of fatty acid transporters (see, e.g., Genbank Accession No. gb|AAC40187.1| (AF072758); all references available through this accession are hereby incorporated by reference in their entirety herein). Long chain fatty acids (LCFAs) are an important source of energy for most organisms (see, e.g., Hirsch,D.et al. PNAS 95: 8625-8629 (1998) which is hereby incorporated by reference herein). They also function as blood hormones, regulating key metabolic functions such as hepatic glucose production. Although LCFAs can diffuse through the hydrophobic core of the plasma membrane into cells, this nonspecific transport cannot account for the high affinity and specific transport of LCFAs exhibited by cells such as cardiac muscle, hepatocytes, and adipocytes. Transport of LCFAs across the plasma membrane is facilitated by fatty acid transport protein (FATP), a plasma membrane protein that increases LCFA uptake when expressed in cultured mammalian cells (see, e.g., Schaffer, J. E. & Lodish, H. F. Cell 79:427-436 (1994) which is hereby incorporated by reference herein). Based on the sequence similarity, the translation product of this gene is expected to share at least some biological activities with fatty acid transporter proteins. Such activities are known in the art, some of which are described elsewhere herein.

This gene is expressed primarily in immune and hematopoietic tissues including Primary Dendritic Cells, lib 1.

Polynucleotides and polypeptides of the invention are useful as reagents for differential identification of the tissue(s) or cell type(s) present in a biological sample and for diagnosis of diseases and conditions which include but are not limited to: disorders of the immune system. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 2

The gene encoding the disclosed cDNA is believed to reside on chromosome 12, specifically at interval D12S78-D12S79. Accordingly, polynucleotides related to this invention would be useful as a marker in linkage analysis for chromosome 12.

The polypeptide encoded by this gene has been determined to have a transmembrane domain at about amino acid position 25 to about 41 of the amino acid sequence referenced in Table 1 for this gene. Moreover, a cytoplasmic tail encompassing amino acids 1 to about 24 of this protein has also been determined. Based upon these characteristics, it is believed that the protein product of this gene shares structural features to type II membrane proteins.

This gene is expressed primarily in the following tissues/cDNA libraries: NCI_CGAP_Thy1; Primary Dendritic Cells, lib 1 and to a lesser extent in NCI_CGAP_Col1; Human Bone Marrow, re-excision; Breast Cancer Cell line, angiogenic; Human Fetal Dura Mater; Human Activated Monocytes; Ulcerative Colitis; Macrophage (GM-CSF treated); Smooth muscle, serum induced,re-exc; NCI_CGAP_Kid11; Normal colon; NCI_CGAP_GC6; Human Microvascular Endothelial Cells, fract. A; Human Testes; Human Endometrial Tumor; Activated T-cell(12h)/Thiouridine-re-excision; T cell helper II; Soares_NhHMPu_S1 and NCI_CGAP_GCB1.

Polynucleotides and polypeptides of the invention are useful as reagents for differential identification of the tissue(s) or cell type(s) present in a biological sample and for diagnosis of diseases and conditions which include but are not limited to: disorders of the immune and/or hematopoietic systems. Similarly, polypeptides and antibodies directed to these polypeptides are useful in providing immunological probes for differential identification of the tissue(s) or cell type(s). For a number of disorders of the above tissues or cells, particularly of the immune and/or hematopoietic system, expression of this gene at significantly higher or lower levels may be routinely detected in certain tissues or cell types (e.g., immune, hematopoietic, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13,14,16,18,19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury: In addition, this gene product may have commercial utility in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 3

The polypeptide encoded by this gene has been determined to have transmembrane domains at about amino acid position 12 to about 28; at about 52 to about 68; and at about 76 to about 92 of the amino acid sequence referenced in Table 1 for this gene. Based upon these characteristics, it is believed that the protein product of this gene shares structural features to type IIIb membrane proteins.

Included in this invention as preferred domains are Phosphopantetheine attachment sites, which were identified using the ProSite analysis tool (Swiss Institute of Bioinformatics). Phosphopantetheine (or pantetheine 4' phosphate) is the prosthetic group of acyl carrier proteins (ACP) in some multienzyme complexes where it serves as a 'swinging arm' for the attachment of activated fatty acid and amino-acid groups. Phosphopantetheine is attached to a serine residue in these proteins. ACP proteins or domains have been found in various enzyme systems including Fatty acid synthetase (FAS). The sequence around the phosphopantetheine attachment site is conserved in all these proteins and can be used as a signature pattern. The consensus pattern is as follows: [DEQGSTALMKRH]-[LIVMFYSTAC]-[GNQ]-[LIVMFYAG]-[DNEKHS]-S-[LIVMST]-{PCFY}-[STAGCPQLIVMF]-[LIVMATN]-[DENQGTAKRHLM]-[LIVMWSTA]-[LIVGSTACR]-x(2)-[LIVMFA] [S is the pantetheine attachment site].

In specific embodiments, polypeptides of the invention comprise, or alternatively consist of, the following amino acid sequence: LLGLSSLVGVTWGLAI (SEQ ID NO: 93). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention. Further preferred are polypeptides comprising the phosphopantetheine attachment site above, and at least 5, 10, 15, 20, 25, 30, 50, or 75 additional contiguous amino acid residues of the amino acid sequence referenced in Table 1 for this gene.

The additional contiguous amino acid residues may be N-terminal or C-terminal to the phosphopantetheine attachment site. Alternatively, the additional contiguous amino acid residues may be both N-terminal and C-tenninal to the phosphopantetheine attachment site, wherein the total N- and C-terminal contiguous amino acid residues equal the specified number.

In specific embodiments, polypeptides of the invention comprise, or alternatively consist of, an amino acid sequence selected from the group: 
HEVIGTGSANSYGLYTIRDRENRTSLELCWFREGTTMYALYITVHGYFLITFLF
GMVVLALVVWKIFTLSRATAVKERGKNRKKVLTLLGLSSLVGVTWGLAIFTP
LGLSTVYIFALFNSLQGVFICCWFTILYLPSQSTTVSSSTARLDQAHSASQE
(SEQ ID NO: 94);
 



(SEQ ID NO: 95);
 
MVIGTGSANSYGLYTIRDRENRTSLELCWFREGTTMYALYITVHGYFLITFLF
GMVVLAL
VVWKIFTLXRATAVKERGKNRKKVLTLLGLSSLVGVTWGLAIFTPLGLSTXY
IFALFNSL QAQRGITV (SEQ ID NO: 96);
 PLGLSTVYIFALFNSLQGVFICCWFTI (SEQ ID NO: 97); YITVHGYFLITFLFGMVVLALVVWKIFTLSR (SEQ ID NO: 98); and VIGTGSANSYGLYTIRDRENRTSLELCWFREGTTMY (SEQ ID NO: 99). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in immune and hematopoietic tissues, as demonstrated by expression in the following tissues/cDNA libraries: Human Neutrophil, Activated and to a lesser extent in Neutrophils control, re-excision; Human Neutrophils, Activated, re-excision; Human Neutrophil; neutrophils control; Human Pancreatic Carcinoma; Human Fetal Bone; NCI_CGAP_Co12; Human Pancreas Tumor; Neutrophils IL-1 and LPS induced; Human Eosinophils; Endothelial cells-control and Neutrophils IL-1 and LPS induced.

Polynucleotides and polypeptides of the invention are useful as reagents for differential identification of the tissue(s) or cell type(s) present in a biological sample and for diagnosis of diseases and conditions which include but are not limited to: disorders of the immune and/or hematopoietic systems. Similarly, polypeptides and antibodies directed to these polypeptides are useful in providing immunological probes for differential identification of the tissue(s) or cell type(s). For a number of disorders of the above tissues or cells, particularly of the immune and/or hematopoietic systems, expression of this gene at significantly higher or lower levels may be routinely detected in certain tissues or cell types (e.g., immune, hematopoietic, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution in immune tissues indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types. Further the tissue distribution in hematopoietic tissues indicates that polynucleotides and/or polypeptides corresponding to this gene would be useful for the treatment, prevention, detection and/or diagnosis of hematopoietic related disorders such as anemia, pancytopenia, leukopenia, thrombocytopenia or leukemia since stromal cells are important in the production of cells of hematopoietic lineages. Briefly, the uses include bone marrow cell ex-vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. Furthermore, the protein may also be used to determine biological activity, raise antibodies, as tissue markers, to isolate cognate ligands or receptors, to identify agents that modulate their interactions,. in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 4

The computer algorithm BLASTX has been used to determine that the translation product of this gene shares sequence homology with the murine alpha/beta hydrolase-1 (see, e.g., Genbank Accession No. gb|AAF01068.1|AF189764_1 (portions of the sequence of which are shown as SEQ ID NO:100 and SEQ ID NO:102); all information available through the recited accession number is incorporated herein by reference). Based on the structural similarity these homologous polypeptides are expected to share at least some biological activities. Such activities are known in the art, some of which are described elsewhere herein. Assays for determining such activities are also known in the art, some of which have been described elsewhere herein.

In specific embodiments, polypeptides of the invention comprise, or alternatively consist of, an amino acid sequence selected from the group: 
SDILQTPDGGQLLLDWAKQPDSSQDPDPTTQPIVLLLPGITGSSQETYVLHLVN
QALRDGYQAVVFNNRGCRGEELRTHRAFCASNTEDLETVVNHIKHRYPQAP
LLAVGISFGGILVLNHLAQARQAAGLVAALTLSACWDSFETTRSLETPLNSLL
FNQPLTAGLCQLVER (SEQ ID NO: 101);
 
LQARTIRQFDERYTSVAFGYQDCVTYYKAASPRTKIDAIRIPVLYLSAADDPF
SPVCALPIQAAQHSPYVALLITARGGHIGFLEGLLPWQHWYMSRLLHQYAKA
IFQDPEGLPDLRALLPSEDRN (SEQ ID NO: 103); and/or
 TSLSPQNGTWADTFSLLLALAVALYLGYYWACV (SEQ ID NO: 104). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

The polypeptide encoded by this gene has been determined to have a transmembrane domain at about amino acid position 134 to about 150 of the amino acid sequence referenced in Table 1 for this gene. Moreover, a cytoplasmic tail encompassing amino acids 151 to about 242 of this protein has also been determined. Based upon these characteristics, it is believed that the protein product of this gene shares structural features to type Ia membrane proteins.

In specific embodiments polypeptide fragments comprise, or alternatively consist of, the soluble extracellular domain of the protein of the invention.

In a further embodiment polypeptide fragments comprise, or alternatively consist of, an amino acid sequence selected from the group:

MAPGQTPSLCSWLLPLPSTWATTGHVCFSDILQTPDGGQLLLDWAKQPDSSQ
DPDPTTQPIVLLLPGITGSSQETYVLHLVNQALRDGYQAVVFNNRGCRGEELR
THRAFCASNTEDLETVVNHIKHRYPQAP (SEQ ID NO: 105) and

TGHVCFSDILQTPDGGQLLLDWAKQPDSSQDPDPTTQPIVLLLPGITGSSQETY
VLHLVNQALRDGYQAVVFNNRGCRGEELRTHRAFCASNTEDLETVVNHIKH
RYPQAP (SEQ ID NO: 106).

This gene is expressed primarily in the following tissues/cDNA libraries: Soares_testis_NHT and to a lesser extent in Human Adult Testes, Large Inserts, Reexcision; Human Testes; Soares_NFL_T_GBC_S1; NCI_CGAP_Ut3; Human adult testis, large inserts; Human Liver, normal; NCI_CGAP_GC4 and Human Testes, Reexcision.

Polynucleotides and polypeptides of the invention are useful as reagents for differential identification of the tissue(s) or cell type(s) present in a biological sample and for diagnosis of diseases and conditions which include but are not limited to: disorders of the male reproductive system, particularly diseases of the testes. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g., reproductive, testicular, cancerous and wounded tissues) or bodily fluids (e.g., semen, lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution of this gene suggest that this clone or the protein product from this gene may be useful for treatment and diagnosis of disorders of the reproductive and metabolic systems.

The tissue distribution in testicular cancer tissue indicates that polynucleotides and polypeptides corresponding to this gene would be useful for the treatment and diagnosis of conditions concerning proper testicular function (e.g. endocrine function, sperm maturation), as well as cancer. Therefore, this gene product is useful in the treatment of male infertility and/or impotence. This gene product is also useful in assays designed to identify binding agents, as such agents (antagonists) would be useful as male contraceptive agents. Similarly, the protein is believed to be useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications. In addition, expression in the testis may implicate this gene product would be involved in normal testicular function. In addition, this gene product may be useful in the treatment of male infertility, and/or could be used as a male contraceptive. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Moreover, the membrane localization and testis specific expression indicates that this gene would be a good target for antagonists, particularly small molecules or antibodies, which block functional activity (e.g., binding of the receptor by its cognate ligand(s); binding of an antibody directed to a protein for the invention). Accordingly, preferred are antibodies and or small molecules which specifically bind an extracellular portion of the translation product of this gene.

The extracellular regions can be ascertained from the information regarding the transmembrane domains as set out above and as shown in SEQ ID NO: 1 05 and/or SEQ ID NO:106. Also provided is a kit for detecting testicular cancer. Such a kit comprises in one embodiment an antibody specific for the translation product of this gene bound to a solid support. Also provided is a method of detecting testicular cancer in an individual which comprises a step of contacting an antibody specific for the translation product of this gene to a bodily fluid from the individual, preferably serum and/or semen, and ascertaining whether antibody binds to an antigen found in the bodily fluid. Preferably the antibody is bound to a solid support and the bodily fluid is serum. The above embodiments, as well as other treatments and diagnostic tests (kits and methods), are more particularly described elsewhere herein.

### FEATURES OF PROTEIN ENCODED BY GENE NO: 5

The translation product of this gene shares sequence homology with the murine Sperizin protein (see, e.g., Genbank Accession No. dbj|BAA77407. 1| (AB016984); all references available through this accession are hereby incorporated in their entirety herein). Sperizin, whose expression is induced in mouse spermatogenic cells and specific to the round spermatid, is thought to be involved in spermatogenesis (see, e.g., Fujii et al. Genomics 57 (1), 94-101 (1999) which is hereby incorporated by reference herein). Based on the sequence similarity, the translation product of this gene is expected to share at least some biological activities with Sperizin. Such activities are known in the art, some of which are described elsewhere herein.

The polypeptide encoded by this gene has been determined to have a transmembrane domain at about amino acid position 186 to about 202 of the amino acid sequence referenced in Table 1 for this gene. Moreover, a cytoplasmic tail encompassing amino acids 203 to about 359 of this protein has also been determined. Based upon these characteristics, it is believed that the protein product of this gene shares structural features to type Ia membrane proteins.

In specific embodiments, polypeptide fragments comprise, or alternatively consist of, the soluble extracellular domain of the protein of the invention.

In a further embodiment, polypeptide fragments comprise or alternatively consist of, an amino acid sequence selected from the group:
MGWPRGRALVAVKALLVLSLLQVPAQAVVRAVLEDNSSSVDFADLPALFG VPLAPEGIRGYLMEVKPANACHPIEAPRLGNRSLGAIVLIRRYDCTFDLKVLN AQRAGFEAAIVHNVHSDDL VSMTHVYEDLRGQIAIPSVFVSEAASQDLRVILG CNKSAHALLLPDDPPCHDLGCHPVLTVS (SEQ ID NO: 108) and
VVRAVLEDNSSSVDFADLPALFGVPLAPEGIRGYLMEVKPANACHPIEAPRLG NRSLGAIVLIRRYDCTFDLKVLNAQRAGFEAAIVHNVHSDDLVSMTHVYEDL RGQIAIPSVFVSEAASQDLRVILGCNKSAHALLLPDDPPCECDLGCHPVLTVS (SEQ ID NO: 109). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

Included in this invention as preferred domains are EGF-like domain signatures, which were identified using the ProSite analysis tool (Swiss Institute of Bioinformatics). A sequence of about thirty to forty amino-acid residues long found in the sequence of epidermal growth factor (EGF) has been shown to be present, in a more or less conserved form, in a large number of other, mostly animal proteins. A common feature is that these repeats are found in the extracellular domain of membrane-bound proteins or in proteins known to be secreted (exception: prostaglandin G/H synthase). The EGF domain includes six cysteine residues which have been shown (in EGF) to be involved in disulfide bonds. The main structure is a two-stranded beta-sheet followed by a loop to a C-terminal short two-stranded sheet. The region between the 5th and 6th cysteine contains two conserved glycines of which at least one is present in most EGF-like domains. Patterns for this domain were created, each including one of these C-terminal conserved glycine residues. The consensus pattern is as follows:C-x-C-x(5)-G-x(2)-C [The 3 C's are involved in disulfide bonds].

In specific embodiments, preferred polypeptides of the invention comprise, or alternatively consist of, the following amino acid sequence: CKCIDPWFSQAPRRSC (SEQ ID NO: 110). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

Further preferred are polypeptides comprising the EGF-like domain signature above, and at least 5, 10, 15, 20, 25, 30, 50, or 75 additional contiguous amino acid residues of the amino acid sequence referenced in Table 1 for this gene. The additional contiguous amino acid residues may be N-terminal or C- terminal to the EGF-like domain signature.

Alternatively, the additional contiguous amino acid residues may be both N-terminal and C-terminal to the EGF-like domain signature, wherein the total N- and C-terminal contiguous amino acid residues equal the specified number.

In a further embodiment, polypeptides of the invention comprise, or alternatively consist of, the following amino acid sequence: 
PSSTQTAKRVTMGWPRPGRALVAVKALLVLSLLQVPAQAVVRAVLEDNSSS
VDFADLPALFGVPLAPEGIRGYLMEVKPANACHPIEAPRLGNRSLGAIVLIRR
YDCTFDLKVLNAQRAGFEAAIVHNVHSDDLVSMTHVYEDLRGQIAIPSVXVS
EAARRTCGSSWAATSRPTRCPADDPPCHDLAVTP (SEQ ID NO: 107).
 Moreover, fragments and variants of these polypeptides (such as, for example; fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Human Adult Testes, Large Inserts, Reexcision; Human adult testis, large inserts; Soares_NFL_T_GBC_S1

Polynucleotides and polypeptides of the invention are useful as reagents for differential identification of the tissue(s) or cell type(s) present in a biological sample and for diagnosis of diseases and conditions which include but are not limited to: disorders of the male reproductive system, particularly diseases of the testes. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g., reproductive, testicular, cancerous and wounded tissues) or bodily fluids (e.g., semen, lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution of this gene suggest that polynucleotides and polypeptides corresponding to this gene may be used for treatment and diagnosis of disorders of the reproductive system.

The tissue distribution in testicular cancer tissue indicates that polynucleotides. and polypeptides corresponding to this gene would be useful for the treatment and diagnosis of conditions concerning proper testicular function (e.g. endocrine function, sperm maturation), as well as cancer. Therefore, this gene product is useful in the treatment of male infertility and/or impotence. This gene product is also useful in assays designed to identify binding agents, as such agents (antagonists) would be useful as male contraceptive agents. Similarly, the protein is believed to be useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications. In addition, expression in the testis may implicate this gene product would be involved in normal testicular function. In addition, this gene product may be useful in the treatment of male infertility, and/or could be used as a male contraceptive. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

In addition, the membrane localization and testis specific expression indicates that this gene would be a good target for antagonists, particularly small molecules or antibodies, which block functional activity (e.g., binding of the receptor by its cognate ligand(s); and/or binding to an antibody directed to a protein of the invention). Accordingly, preferred are antibodies and or small molecules which specifically bind an extracellular portion of the translation product of this gene. The extracellular regions can be ascertained from the information regarding the transmembrane domains as set out above and/or as shown in SEQ ID NO:109 and/or SEQ ID NO: 110. Also provided is a kit for detecting testicular cancer. Such a kit comprises in one embodiment an antibody specific for the translation product of this gene bound to a solid support. Also provided is a method of detecting testicular cancer in an individual which comprises a step of contacting an antibody specific for the translation product of this gene to a bodily fluid from the individual, preferably serum and/or semen, and ascertaining whether antibody binds to an antigen found in the bodily fluid. Preferably the antibody is bound to a solid support and the bodily fluid is serum. The above embodiments, as well as other treatments and diagnostic tests (kits and methods), are more particularly described elsewhere herein.

### FEATURES OF PROTEIN ENCODED BY GENE NO: 6

The polypeptide encoded by this gene has been determined to have a transmembrane domain at about amino acid position 110 to about 126 of the amino acid sequence referenced in Table 1 for this gene. Moreover, a cytoplasmic tail encompassing amino acids 127 to about 240 of this protein has also been determined. Based upon these characteristics, it is believed that the protein product of this gene shares structural features to type Ia membrane proteins.

In specific embodiments, polypeptides of the invention comprise, or alternatively consist of, the soluble extracellular domain (at about amino acid 1 to about 109 or alternatively, at about amino acid 30 to about 109), the transmembrane domain, and/or the cytoplasmic domain (at about amino acid 127 to about 240) of the amino acid sequence referenced in Table 1 for this gene.

In specific embodiments, polypeptides of the invention comprise, or alternatively consist of, an amino acid sequence selected from the group:
MTAPVPAPRILLPLLLLLLLTPPPGARGEVCMASRGLSLFPESCPDFCCGTCDD QYCCSDVLKKFVWSEERCAVPEASVPASVEPVEQLGSALRFRPGYNDPMSGF GAT (SEQ ID NO: 111);
EVCMASRGLSLFPESCPDFCCGTCDDQYCCSDVLKKFVWSEERCAVPEASVP
ASVEPVEQLGSALRFRPGYNDPMSGFGAT (SEQ ID NO: 112); and
CFTCSCCCLYKTCRRPRPVVTTTTSTTWHAPYPQPPSVPPSYPGPSYQGYHT - MPPQPGMPAAPYPMQYPPPYPAQPMGPPAYHETLAGGAAAPYPASQPPYNP AYMDAPKAAL (SEQ ID NO: 113). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Soares ovary tumor NbHOT and to a lesser extent in Keratinocyte; Soares_fetal_heart NbHH19W; Human Primary Breast Cancer Reexcision; NCI_CGAP_Pr22; NTERA2, control; Soares_pregnant_uterus_NbHPU; Soares_fetal_liver_spleen_1NFLS_S1; NCI_CGAP_GC3; Clontech human aorta polyA+ mRNA (#6572); Stratagene lung (#937210); Activated T-cell(12h)/Thiouridine-re-excision; Colon Tumor II; Soares fetal liver spleen 1NFLS; Human Primary Breast Cancer,re-excision; NCI_CGAP_Pr25; Soares adult brain N2b4HB55Y; NCI_CGAP_Ut2; Human Thymus; Ovary, Normal: (9805C040R); Human Thymus; Bone Marrow Stromal Cell, untreated; NCI_CGAP_Pan1; CD34 depleted Buffy Coat (Cord Blood), re-excision; NCI_CGAP_Brn25; Soares_senescent_fibroblasts_NbHSF; H. Frontal cortex,epileptic,re-excision; Human Endometrial Tumor, Hodgkin's Lymphoma II; Osteoblasts; Soares_fetal_lung_NbHL19W; Colon Normal III; Soares placenta Nb2HP; Human Leukocytes,normalized control #4; Human Adult Liver; Human Spleen; Human Old Ovary; Adenocarcinoma; Thyroid Normal (SDCA2 No); Human Whole 6 Week Old Embryo (II), subt; LNCAP + 30nM R1881; Saos2 Cells, Vitamin D3 Treated; KG1-a Lambda Zap Express cDNA library; NCI_CGAP_Co2; Saos2 Cells, Untreated; Resting T-Cell; Breast, Cancer: (9806C012R); Human promyelocyte; NCI_CGAP_GC2; Human OB HOS treated (10 nM E2) fraction I; Jia bone marrow stroma; Human Adult Pulmonary; NCI_CGAP_Ov23; Amniotic Cells - TNF induced; NCI_CGAP_Co12; Human Soleus; Human Pineal Gland; Breast, Cancer: (4005522 A2); B Cell lymphoma; Human endometrial stromal cells-treated with estradiol; Smooth muscle, IL1b induced; Human pancreatic islet; Stratagene ovary (#937217); NCI_CGAP_Co10; Healing groin wound, 6.5 hours post incision; Ovary, Cancer: (4004332 A2); CD34 depleted Buffy Coat (Cord Blood); Stratagene neuroepithelium (#937231); NCI_CGAP_Pr2; Stratagene NT2 neuronal precursor 937230; NCI_CGAP_Ut1; NCI_CGAP_Kid6; Human Fetal Kidney; Soares_pineal_ gland_N3BPG; Human Osteoblasts II; Human Hypothalmus,Schizophrenia; Stratagene HeLa cell s3 937216; Liver, Hepatoma; NCI_CGAP_Br2; Human Rhabdomyosarcoma; NCI_CGAP_CLL1; Human Thymus Stromal Cells; CHME Cell Line,treated 5 hrs; Stratagene hNT neuron (#937233); Rejected Kidney, lib 4; Ovarian Tumor 10-3-95; Human Liver, normal; NCI_CGAP_Co3; Smooth muscle, serum induced,re-exc; CHME Cell Line, untreated; Fetal Liver, subtraction II; Smooth muscle, serum treated; breast lymph node CDNA library; H Macrophage (GM-CSF treated), re-excision; NCI_CGAP_GC4; Dendritic cells, pooled; 12 Week Early Stage Human II, Reexcision; Activated T-Cell (12hs)/Thiouridine labelledEco; NCI_CGAP_Kid5; NCI_CGAP_Brn23; Monocyte activated; Soares_placenta_8to9weeks_2NbHP8to9W; T Cell helper I; Human Testes; T cell helper II; Human Cerebellum and NCI_CGAP_GCB 1.

Polynucleotides and polypeptides of the invention are useful as reagents for differential identification of the tissue(s) or cell type(s) present in a biological sample and for diagnosis of diseases and conditions which include but are not limited to: disorders of the immune and/or reproductive system. Similarly, polypeptides and antibodies directed to these polypeptides are useful in providing immunological probes for differential identification of the tissue(s) or cell type(s). For a number of disorders of the above tissues or cells, particularly of the immune and/or reproductive systems, expression of this gene at significantly higher or lower levels may be routinely detected in certain tissues or cell types (e.g., immune, reproductive, cancerous and wounded tissues) or bodily fluids (e.g., vaginal pool, lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution of this sequence predicts that it is useful in the diagnosis and treatment of ovary tumor, psoriasis, and colon cancers. The tissue distribution also indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates. the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 7

This gene is expressed primarily in the following tissues/cDNA libraries: NTERA2, control; NCI_CGAP_Kid11; Human Ovarian Cancer Reexcision and to a lesser extent in NCI_CGAP_Kid8; Ovary, Cancer: (4004332 A2); Human Chronic Synovitis; NCI_CGAP_Br2; Hepatocellular Tumor, re-excision; Pancreas normal PCA4 No; NCI_CGAP_Kid3; Soares_multiple_sclerosis_2NbHMSP; Soares_NFL_T_GBC_S1; Soares_fetal_heart_NbHH19W and Soares_testis_NHT.

Polynucleotides and polypeptides of the invention are useful as reagents for differential identification of the tissue(s) or cell type(s) present in a biological sample and for diagnosis of diseases and conditions which include but are not limited to: disorders of the genitourinary tract, including diseases of the kidney, particularly cancers. Similarly, polypeptides and antibodies directed to these polypeptides are useful in providing immunological probes for differential identification of the tissue(s) or cell type(s). For a number of disorders of the above tissues or cells, particularly of the urinary tract, and/or reproductive system, expression of this gene at significantly higher or lower levels may be routinely detected in certain tissues or cell types (e.g., urinary, genital, reproductive, kidney, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

Expression in teratocarcinoma, ovarian cancer and other predominantly neoplastic organs indicates that polynucleotides and polypeptides corresponding to this gene would be useful in the diagnosis, study and treatment of developmental disorders and cancers of reproductive, genitourinary tract, and other organs where expression is indicated.

Furthermore, the tissue distribution in kidney indicates that polynucleotides and/or polypeptides corresponding to this gene would be useful in the treatment, prevention, diagnosis and/or detection of kidney diseases including renal failure, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilm's Tumor Disease, and congenital kidney abnormalities such as horseshoe kidney, polycystic kidney, and Falconi's syndrome.

The tissue distribution in ovarian cancer tissue, indicates that polynucleotides and polypeptides corresponding to this gene would be useful for the treatment and diagnosis of tumors, especially ovarian cancer, as well as cancers of other tissues where expression has been indicated. In addition, the expression in ovarian cancer tissue may indicate the gene or its products can be used to treat and/or diagnose disorders of the ovary, including inflammatory disorders, such as oophoritis (e.g., caused by viral or bacterial infection), ovarian cysts, amenorrhea, infertility, hirsutism, and ovarian cancer (including, but not limited to, primary and secondary cancerous growth, endometrioid carcinoma of the ovary, ovarian papillary serous adenocarcinoma, ovarian mucinous adenocarcinoma, Ovarian Krukenberg tumor). Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 8

The polypeptide encoded by this gene has been determined to have two transmembrane domains at about amino acid position 3 to about 19; and at about amino acid position 212 to about 228 of the amino acid sequence referenced in Table 1 for this gene. Based upon these characteristics, it is believed that the protein product of this gene shares structural features to type IIIb membrane proteins. In specific embodiments, polypeptides of the invention comprise, or alternatively consist of, the soluble domains of the protein of the invention.

In further specific embodiments, polypeptides of the invention comprise, or alternatively consist of, an amino acid sequence selected from the group:
GPVQAPAAPPSL (SEQ ID NO: 114) and
SRARGSGCRAGTGARGAGAEGREGEACGTVGLLLEHSFEIDDSANFRKRGSL LWNQQDGTLSLSQRQLSEEERGRLRDVAALNGLYRVRIPRRPGALDGLEAGG YVSSFVPACSLVESHLSDQLTLHVDVAGNWGVSWTHPMAPCSPRGFPPAH GVEP (SEQ ID NO: 115). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Soares_fetal_heart_NbHH19W and to a lesser extent in NCI_CGAP_Lu5; Soares_NFL_ T_ GBC_ S1; Human Primary Breast Cancer Reexcision; NCI_CGAP_Kid3; Soares ovary tumor NbHOT; Human Pineal Gland; Colon Carcinoma; NCI_CGAP_Brn25; NCI_CGAP_Kid5; Nine Week Old Early Stage Human; Soares placenta Nb2HP; Human Placenta, subtracted; Human OB HOS treated (1 nM E2) fraction I; Human Prostate; Stratagene pancreas (#937208); NCI_CGAP_Pan 1; Soares breast 3NbHBst; Soares melanocyte 2NbHM; Soares fetal_lung_NbHL19W; Soares_testis_NHT; Soares fetal liver spleen 1NFLS; Human Thymus Tumor; Human adult lung 3' directed Mbol cDNA; Namalwa Cells; Crohn's Disease; Human placenta polyA+ (TFujiwara); LNCAP + o.3nM R1881; Barstead pancreas HPLRB 1; Human Uterus, normal; NCI_CGAP_Pr24; NCI_CGAP_Eso2; NCI_CGAP_Pr11; NCI_CGAP_GC2; NCI_CGAP_Kid8; Human Primary Breast Cancer,re-excision; Human placenta cDNA (TFujiwara); Healing Abdomen wound,70&90 min post incision; Resting T-Cell, re-excision; CD40 activated monocyte dendridic cells; NCI_CGAP_GC3; NCI_CGAP_Ut4; B Cell lymphoma; Human endometrial stromal cells-treated with estradiol; Human Hypothalamus, schizophrenia, re-excision; human corpus colosum; Human Osteosarcoma; NCI_CGAP_Co14; Human Osteoclastoma, re-excision; NCI_CGAP_Pr12; wilm's tumor; Prostate BPH; Breast, Normal: (4005522B2); NCI_CGAP_Ew1; Gessler Wilms tumor; KMH2; Human Brain, Striatum; NCI_CGAP_Ut1; NCI_CGAP_Pr22; Apoptotic T-cell; Human Fetal Kidney; human ovarian cancer; Human Adult Testes, Large Inserts, Reexcision; Stratagene HeLa cell s3 937216; Olfactory epithelium,nasalcavity; Ovary, Cancer: (4004576 A8); Human Thymus; Synovial Fibroblasts (control); Soares breast 2NbHBst; Human Adrenal Gland Tumor; Hepatocellular Tumor, re-excision; Human Ovary; Macrophage-oxLDL, re-excision; Pancreas Islet Cell Tumor; 12 Week Old Early Stage Human; NCI_CGAP_GC4; Human Synovial Sarcoma; Human Adult Pulmonary,re-excision; Endothelial-induced; Stratagene lung (#937210); NCI_CGAP_Bm23; Monocyte activated; Spleen, Chronic lymphocytic leukemia; Colon Normal III and Primary Dendritic Cells, lib 1.

Polynucleotides and polypeptides of the invention are useful as reagents for differential identification of the tissue(s) or cell type(s) present in a biological sample and for diagnosis of diseases and conditions which include but are not limited to: disorders of the reproductive, immune and/or hematopoietic systems. Similarly, polypeptides and antibodies directed to these polypeptides are useful in providing immunological probes for differential identification of the tissue(s) or cell type(s). For a number of disorders of the above tissues or cells, particularly of the reproductive, immune and/or hematopoietic systems, expression of this gene at significantly higher or lower levels may be routinely detected in certain tissues or cell types (e.g., reproductive, immune, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

Enriched expression in fetal heart and lung, and in breast cancer, and colon carcinoma suggests utility in the study and treatment of cardiopulmonary and vascular disorders, congenital and functional heart defects, gastrointestinal inflammatory conditions and cancer. Further, the tissue distribution in immune tissues indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 15, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types.

In addition, the expression in reproductive tissues indicates that polynucleotides and/or polypeptides of the invention would be useful in the detection, prevention and/or treatment of disorders of the reproductive organs, such as, for example, disorders of the ovary including inflammatory disorders, such as oophoritis (e.g., caused by viral or bacterial infection), ovarian cysts, amenorrhea, infertility, hirsutism, and ovarian cancer (including, but not limited to, primary and secondary cancerous growth, endometrioid carcinoma of the ovary, ovarian papillary serous adenocarcinoma, ovarian mucinous adenocarcinoma, Ovarian Krukenberg tumor); disorders of the breast including breast neoplasia and breast cancers, such as fibroadenoma, pipillary carcinoma, ductal carcinoma, Paget's disease, medullary carcinoma, mucinous carcinoma, tubular carcinoma, secretory carcinoma and apocrine carcinoma, as well as juvenile hypertrophy and gynecomastia, mastitis and abscess, duct ectasia, fat necrosis and fibrocystic diseases; disorders of the prostate including inflammatory disorders, such as chronic prostatitis, granulomatous prostatitis and malacoplakia, prostatic hyperplasia and prostate neoplastic disorders, including adenocarcinoma, transitional cell carcinomas, ductal carcinomas, squamous cell carcinomas, or as hormones or factors with systemic or reproductive functions. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 9

The computer algorithm BLASTX has been used to determine that the translation product of this gene shares sequence homology with several cytochrome P450 isoforms (see, e.g., Genbank Accession Nos. gb|AAB27705.1| (a portion of which are shown as SEQ ID NO:116) or gb|AAA31434.1|; all information available through the recited accession number is incorporated herein by reference). The cytochrome P450 2B4 isoform Bx metabolizes androsienedione, testosterone, and 7-ethoxycoumarin in a manner characterized by preference for 16 beta-hydroxylation of androstenedione, low testosterone 16-hydroxylation, and high ethoxycoumarin O-deethylation. Expression is induced by phenobarbital in rabbits. Based on the structural similarity these homologous polypeptides are expected to share at least some biological activities. Such activities are known in the art, some of which are described elsewhere herein. Assays for determining such activities are also known in the art (see, for example, Ryan et al., Arch. Biochem. Biophys. 304:454-463 (1993)) some of which have been described elsewhere herein.

In specific embodiments, polypeptides of the invention comprise, or alternatively consist of, an amino acid sequence selected from the group:
FSPQTYEMFSWFLRPLPGPHKQLLHHVSTLAAFTVRQVQQHQGNLDASGPAR DLVDAFLLKMAQEEQNPGTEFTNKNMLMTVIYLLFAGTMTVSTTVGYTLLL LMKYPHVQKWVREELNRELGAGQAPSLGDRTRLPYTDAVLHEAQRLLALVP MGIPRTLMRTTRFRGYTLPQGTEVFPLLGSILHDPNIFKHPEEFNPDRFLDADG RFRKHEAFLPFSLGKRVCLGEGLAKAELFLFFTTILQAFSLESPCPPDTLSLKPT
VSGLFNIPPAFQLQ (SEQ ID NO: 117); and
HASGRPAEMEATGTWALLLALALLLLLTLALSGTRARGHLPPGPTPLPLLGN LLQLRPGALYSGLMRLSKKYGPVFTIYLGPWRPVVVLVGQEAVREALGGQA EEFSGRGTVAMLEGTFDGHGVFFSNGERWRQLRKFTMLALRDLGMGKREGE ELIQAEARCLVETFQGTEGRPFDPSLLLAQATSNVVCSLLFGLRFSYEDKEFQ AVVRAAGGTLLGVSSQGGQVSGWDPSPTTFPEGSCQGPMRTSCPSPHRPTRC SPGSCGPCQAPTSSSSTTSAPWLPSQSGRCSSTRGTWMLRAPHVTLSMPSC (SEQ ID NO: 118). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Human (HCC) cell line liver (mouse) metastasis, remake; Olfactory epithelium, nasalcavity; Human Testes Tumor; Monocyte activated and to a lesser extent in H Macrophage (GM-CSF treated), re-excision; Soares ovary tumor NbHOT; Human colon mucosa; Healing groin wound - zero hr post-incision (control); NCI_CGAP_Co10; Human Adult Small Intestine; NCI_CGAP_Kid6; Human Testes Tumor, re-excision; NTERA2, control; NCI_CGAP_Co3; CHME Cell Line,untreated; Colon Normal II; Dendritic cells, pooled; Primary Dendritic cells,frac 2; Soares_NFL_T_GBC_S1; Soares_fetal_heart_NbHH19W and Scares fetal liver spleen INFLS.

Polynucleotides and polypeptides of the invention are useful as reagents for differential identification of the tissue(s) or cell type(s) present in a biological sample and for diagnosis of diseases and conditions which include but are not limited to: disorders of the immune system and/or disorders of the digestive system. Similarly, polypeptides and antibodies directed to these polypeptides are useful in providing immunological probes for differential identification of the tissue(s) or cell type(s). For a number of disorders of the above tissues or cells, particularly of the immune and/or digestive systems, expression of this gene at significantly higher or lower levels may be routinely detected in certain tissues or cell types (e.g., immune, digestive, liver, cancerous and wounded tissues) or bodily fluids (e.g., lymph, bile, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types. In addition, the tissue distribution indicates the polynucleotides and/or polypeptides corresponding to this' gene would be useful for the detection and treatment of liver disorders and cancers (e.g. hepatoblastoma, liver cancer, jaundice, hepatitis, liver metabolic diseases and conditions that are attributable to the differentiation of hepatocyte progenitor cells).Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 10

The gene encoding the disclosed cDNA is believed to reside on chromosome 1, specifically at interval D1S2635-D1S2844. Accordingly, polynucleotides related to this invention are useful as a marker in linkage analysis for chromosome 1.

The polypeptide encoded by this gene has been determined to have a transmembrane domain at about amino acid position 670 to about 686 of the amino acid sequence referenced in Table 1 for this gene. Moreover, a cytoplasmic tail encompassing amino acids 687 to about 709 of this protein has also been determined. Based upon these characteristics, it is believed that the protein product of this gene shares structural features to type Ib membrane proteins.

In specific embodiments, polypeptides which comprise the soluble extracellular domain, or fragments thereof, are included in the invention.

In specific embodiments, polypeptides comprising the soluble extracellular domain comprise, or alternatively consist of, the following amino acid sequence: 
MATAGGGSGADPGSRGLLRLLSFCVLLAGLCRGNSVERKIYIPLNKTAPCVRL
LNATHQIGCQSSISGDTGVIHVVEKEEDLQWVLTDGPNPPYMVLLESKHFTR
DLMEKLKGRTSRIAGLAVSLTKPSPASGFSPSVQCPNDGFGVYSNSYGPEFAH
CREIQWNSLGNGLAYEDFSFPIFLLEDENETKVIKQCYQDHNLSQNGSAPSFPL
CAMXLFSHMHAVISTATCMRRSSIQSTFSINPEIVCDPLSDYNVWSMLKPINTT
GTLKPDDRVVVAATRLDSRSFFWNVAPGAESAVASFVTQLAAAEALQKAPD
VTTLPRNVMFVFFQGETFDYIGSSRMVYDMEKGKFPVQLENVDSFVELGQV
ALRTSLELWMHTDPVSQKNESVRNQVEDLLATLEKSGAGVPAVILRRPNQSQ
PLPPSSLQRFLRARNISGVVLADHSGAFHNKYYQSIYDTAENINVSYPEWLSP
EEDLNFVTDTAKALADVATVLGRALYELAGGTNFSDTVQADPQTVTRLLYG
FLIKANNSWFQSILRQDLRSYLGDGPLQHYIAVSSPTNTTYVVQYALANLTGT
VVNLTREQCQDPSKVPSENKDLYEYSWVQGPLHSNETDRLPRCVRSTARLAR
ALSPAFELSQWSSTEYSTWTESRWKDIRARIFLIASKELEL (SEQ ID NO:119).
 Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Primary Dendritic Cells, lib 1 and to a lesser extent in Soares placenta Nb2HP; Soares infant brain 1NIB; Scares melanocyte 2NbHM; Soares_pregnant_uterus_NbHPU; Soares_testis_NHT; NCI_CGAP_Lu5; Soares fetal liver spleen 1NFLS; Smooth muscle, serum treated; H Macrophage (GM-CSF treated), re-excision; Soares_parathyroid_tumor_NbHPA; Smooth muscle,control; normalized infant brain cDNA; Bone Marrow Stromal Cell, untreated; Human Adrenal Gland Tumor; Fetal Heart; Activated T-Cell (12hs)/Thiouridine labelledEco; Soares_multiple_sclerosis_2NbHMSP; Monocyte activated; Soares_placenta_8to9weeks_2NbHP8to9W; Soares ovary tumor NbHOT; Human Endometrial Tumor; Soares_NFL_T_GBC_S1; NCI_CGAP_Lu1; Lung, Cancer (4005313 A3): Invasive Poorly Differentiated Lung Adenocarcinoma,; STROMAL - OSTEOCLASTOMA; Smooth muscle, IL1b induced; Human Osteoclastoma Stromal Cells - unamplified; Human endometrial stromal cells; Myoloid Progenitor Cell Line; Human Umbilical Vein, Reexcision; Stratagene HeLa cell s3 937216; Liver, Hepatoma; Human Hippocampus; Stratagene liver (#937224); NCI_CGAP_Co8; Primary Dendritic cells,frac 2; Human Adult Pulmonary,re-excision; NCI_CGAP_Bm25; Human Microvascular Endothelial Cells, fract. A; Human Cerebellum; Soares_fefal_heart_NbHH19W; Human normal gingiva; NCI_CGAP_Ov1; Human pancreatic cancer cell line Patu 8988t; NCI_CGAP_HN3; NCI_CGAP_Co4; Human colorectal cancer; Colon, Cancer: (9808C064R); Human OB HOS treated (1 nM E2) fraction I; NCI_CGAP_Bm35; NCI_CGAP_Lym5; Infant brain, Bento Soares; Human OB MG63 treated (10 nM E2) fraction I; Human Aortic Endothelium; Jia bone marrow stroma; Human Cerebellum, subtracted; Human Placenta; Fetal Heart, re-excision; Smooth Muscle Serum Treated, Norm; NCI_ CGAP_Co12; Human Quadriceps; NCI_CGAP _Ut4; Human retina CDNA randomly primed sublibrary; Healing groin wound - zero hr post-incision (control); B Cell lymphoma; Hepatocellular Tumor,re-excision; human corpus colosum; Human Umbilical Vein, Endo. remake; NTERA2 + retinoic acid, 14 days; Glioblastoma; Ovary, Cancer: (15799A1F) Poorly differentiated carcinoma; Human endometrial stromal cells-treated with progesterone; Jurkat T-cell G1 phase; Jurkat T-Cell, S phase; Synovial Fibroblasts (II1/TNF), subt; Breast, Normal: (4005522B2); Human Infant Brain; Human Chronic Synovitis; Ovary, Cancer (15395A1F): Grade II Papillary Carcinoma; Gessler Wilms tumor; NCI_CGAP_Ut1; Monocyte activated, re-excision; Apoptotic T-cell; HUMAN JURKAT MEMBRANE BOUND POLYSOMES; NCI_CGAP_Gas4; Ovary, Cancer (9809C332): Poorly differentiated adenocarcinoma; Ovary, Cancer: (4004576 A8); Human Rhabdomyosarcoma; Human Thymus; Hemangiopericytoma; NCI_CGAP_CLL1; Macrophage (GM-CSF treated); CHME Cell Line,treated 5 hrs; Ovarian Tumor 10-3-95; Hepatocellular Tumor, re-excision; NCI_CGAP_Pan1; Macrophage-oxLDL, re-excision; NCI_CGAP_Co3; Pancreas Islet Cell Tumor; PC3 Prostate cell line; CHME Cell Line,untreated; Fetal Liver, subtraction II; Colon Tumor; Stratagene colon (#937204); Colon Carcinoma; Human Eosinophils; Brain frontal cortex; Adipocytes; Normal colon; Dendritic cells, pooled; Human Fetal Kidney, Reexcision; Human Synovial Sarcoma; B-cells (stimulated); Human Placenta; Endothelial-induced; Endothelial cells-control; Anergic T-cell; Soares_senescent_fibroblasts_NbHSF; NCI_CGAP_Kid3; NCI_CGAP_Kid5; NCI_CGAP_Brn23; Pancreas Tumor PCA4 Tu; Neutrophils IL-1 and LPS induced; Human fetal heart, Lambda ZAP Express; Soares_total_fetus_Nb2HF8_9w and NCI_CGAP_GCB1.

Polynucleotides and polypeptides of the invention are useful as reagents for differential identification of the tissue(s) or cell type(s) present in a biological sample and for diagnosis of diseases and conditions which include but are not limited to: disorders of the immune and/or hematopoietic systems. Similarly, polypeptides and antibodies directed to these polypeptides are useful in providing immunological probes for differential identification of the tissue(s) or cell type(s). For a number of disorders of the above tissues or cells, particularly of the immune and/or hematopoietic systems, expression of this gene at significantly higher or lower levels may be routinely detected in certain tissues or cell types (e.g., immune, hematopoietic, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

Enriched expression in dendritic cells and placenta suggests utility in the study and treatment of hemopoietic, immune and inflammatory and metabolic and reproductive disorders. The tissue distribution indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as art agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 11

The polypeptide encoded by this gene has been determined to have a transmembrane domain at about amino acid position 117 to about 133 of the amino acid sequence referenced in Table 1 for this gene. Moreover, a cytoplasmic domain encompassing amino acids 134 to about 293 of this protein has also been determined. Based upon these characteristics, it is believed that the protein product of this gene shares structural features to type Ia membrane proteins.

In specific embodiments, polypeptides of the invention comprise, or alternatively consist of, the extracellular soluble domain (e.g., from about amino acid 1 to about 116; or alternatively, from about amino acid 24 to about 116 of the amino acid sequence referenced in Table 1 for this gene); the cytoplasmic domain; the transmembrane domain; and/or combinations of the above (e.g., the extracellular domain and the transmembrane domain without the cytoplasmic domain):

In further specific embodiments, polypeptides of the invention comprise, or alternatively consist of, an amino acid sequence selected from the group:
MTGLYELVWRVLHALLCLHRTLTSWLRVRFGTWNWIWRRCCRAASAAVLA PLGFTLRKPPAVGRNRRHHRHPRGGSCLAAAHHRMRWRADGRSLEKLPVH MGLVITEVEQEPSFSD (SEQ ID NO: 123);
SWLRVRFGTWNWIWRRCCRAASAAVLAPLGFTLRKPPAVGRNRRHHRHPR. GGSCLAAAHHRMRWRADGRSLEKLPVHMGLVITEVEQEPSFSD (SEQ ID NO: 124); and
VYDHQGIFKRNNSRLMDEILKQQQELLGLDCSKYSPEFANSNDKDDQVLNCH. LAVKVLSPEDGKADIVRAAQDFCQLVAQKQKRPTDLDVDTLASLLSSNGCPD PDLVLKFGPVDSTLGFLPWHIRLTEIVSLPSHLNISYEDFFSALRQYAACEQRL GK (SEQ ID NO: 125). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

In other specific embodiments, polypeptides of the invention comprise, or alternatively consist of, an amino acid sequence selected from the group:
PEDGKADIRAAQDFCQXVAQKQXRPTDLDVDTL (SEQ ID NO: 120), FANSNDKDDQVLNCHLAVKVLS (SEQ ID NO: 121) and VYLVQMVVLILI (SEQ ID NO: 122). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Human Endometrial Tumor and to a lesser extent in Human Osteoclastoma; Ovary, Cancer: (4004576 A8); Shares placenta Nb2HP; NCI_CGAP_GCB1; NCI_CGAP_Ut1; Soares breast 3NbHBst; Soares infant brain 1NIB; Ovarian Tumor 10-3-95; Stratagene lung (#937210); Human adult small intestine,re-excision;
Salivary Gland, Lib 2; Human Osteoclastoma, re-excision; Jurkat T-cell G1 phase; Monocyte activated, re-excision; Human Placenta (re-excision); Human Fetal Heart; human tonsils; NCI_CGAP_GC6; Endothelial-induced; NCI_CGAP_Kid5; Monocyte activated; Osteoblasts; Soares_parathyroid_tumor_NbHPA; Soares_total_fetus_Nb2HF8_9w; Soares_NhHMPu_S1; Human Infant Adrenal Gland; Healing Abdomen Wound, 15 days post incision; HPAS (human pancreas, subtracted); Human colorectal cancer; Ku 812F Basophils Line; Human (Caco-2) cell line, adenocarcinoma, colon, remake; Adipocytes,re-excision; HUMAN STOMACH; Fetal Heart, re-excision; NTERA2 teratocarcinoma cell line+retinoic acid (14 days); HEL cell line; Stomach cancer (human),re-excision; pBMC stimulated w/ poly I/C; Human adult (K.Okubo); human corpus colosum; Healing groin wound, 7.5 hours post incision; NCI_CGAP_Co14; Myoloid Progenitor Cell Line; Stratagene lung carcinoma 937218; L428; Human Heart; Stromal cell TF274; Macrophage-oxLDL; Human Hypothalmus,Schizophrenia; Stratagene endothelial cell 937223; Ulcerative Colitis; Epithelial-TNFa and INF induced; Macrophage (GM-CSF treated); Human Adult Heart,re-excision; Human Gall Bladder; PC3 Prostate cell line; Human Testes Tumor; Primary Dendritic cells,frac 2; Human Testes, Reexcision; 12 Week Early, Stage Human II, Reexcision; Anergic T-cell; NCI_CGAP_Brn25; NCI_CGAP_Brn23; HUMAN B CELL LYMPHOMA; Spleen, Chronic lymphocytic leukemia; H. Frontal cortex,epileptic,re-excision; NCI_CGAP_Lu5; normalized infant brain cDNA; Human 8 Week Whole Embryo; T cell helper II; Soares_pregnant_uterus_NbHPU; Soares_testis_NHT and Primary Dendritic Cells, lib 1.

Polynucleotides and polypeptides of the invention are useful as reagents for differential identification of the tissue(s) or cell type(s) present in a biological sample and for diagnosis of diseases and conditions which include but are not limited to: disorders of the immune and/or reproductive systems, including but not limited to ovarian cancer. Similarly, polypeptides and antibodies directed to these polypeptides are useful in providing immunological probes for differential identification of the tissue(s) or cell type(s). For a number of disorders of the above tissues or cells, particularly of the immune and/or reproductive systems, expression of this gene at significantly higher or lower levels may be routinely detected in certain tissues or cell types (e.g., immune, reproductive, cancerous and wounded tissues) or bodily fluids (e.g., lymph, vaginal pool, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

Enriched expression in endometrial and ovarian tumors and in osteoclastoma indicates that polynucleotides and polypeptides corresponding to this gene would be useful in the study, detection, prevention and treatment of endometrial bleeding; endometrial, ovarian and other neoplasms; vascular, circulatory, hemopoietic disorders, osteoporosis and tumor metastases. The tissue distribution in ovarian cancer tissue, indicates that polynucleotides and polypeptides corresponding to this gene would be useful for the treatment and diagnosis of tumors, especially ovarian cancer, as well as cancers of other tissues where expression has been indicated. The expression in ovarian cancer tissue may indicate the gene or its products can be used to treat and/or diagnose disorders of the ovary, including inflammatory disorders, such as oophoritis (e.g., caused by viral or bacterial infection), ovarian cysts, amenorrhea, infertility, hirsutism, and ovarian cancer (including, but not limited to, primary and secondary cancerous growth, endometrioid carcinoma of the ovary, ovarian papillary serous adenocarcinoma, ovarian mucinous adenocarcinoma, Ovarian Krukenberg tumor).

The tissue distribution in immune tissues indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial-utility in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 12

The polypeptide encoded by this gene has been determined to have a transmembrane domain at about amino acid position 98 to about 114 of the amino acid sequence referenced in Table 1 for this gene. Moreover, a cytoplasmic domain encompassing amino acids 115 to about 604 of this protein has also been determined. Based upon these characteristics, it is believed that the protein product of this gene shares structural features to type Ia membrane proteins. In specific embodiments, polypeptides of the invention comprise or alternatively consist of, the extracellular domain, the transmembrane domain, the cytoplasmic domain and/or combinations of one, two, or all of these domains.

In further embodiments, polypeptides of the invention comprise, or alternatively consist of, an amino acid sequence selected from the group: 
MVEEPGRFLPLWLHILLITVLLVLSGIFSGLNLGLMALDPMELRIVQNCGTEK
ERRYARKIEPIRRKGNYLLCSLLLGNVLVNTSLTILLDNLIGSG (SEQ ID NO:
127);
 
GLNLGLMALDPMELRIVQNCGTEKERRYARKIEPIRRKGNYLLCSLLLGNVL
VNTSLTILLDNLIGSG (SEQ ID NO: 128);
 
LPQALCSRHGLAVGANTILLTKFFMLLTFPLSFPISKLLDFFLGQEIRTVYNRE
KLMEMLKVTEPYNDLVKEELNMIQGALELRTKTVEDIMTQLQDCFMIRSDAI
LDFNTMSEIMESGYTRIPVFEDEQSNIVDILYVKDLAFVDPDDCTPLKTITRFY
NHPVHFVFHDTKLDAMLEEFKKGKSHLAIVQKVNNEGEGDPFYEVLGLVTL
EDVIEEIIKSEILDESDMYTDNRSRKRVSEKNKRDFSAFKDADNELKVKISPQL
LLAAHRFLATEVSQFSPSLISEKILLRLLKYPDVIQELKFDEHNKYYARHYLYT
RNKPADYFILILQGKVEVEAGKENMKFETGAFSYYGTMALTSVPSDRSPAHP
TPLSRSASLSYPDRTDVSTAATLAGSSNQFGSSVLGQYISDFSVRALVDLQYIK
 
ITRQQYQNGLLASRMENSPQFPIDGCTTHMENLAEKSELPVVDETTTLLNERN
SLLHKASHENAI (SEQ ID NO: 129);
 and 
SGIFSGLNLGLMALDPMELRIVQNCGTEKERRYARKIEPIRRKGNYLLCSLLL
GNVLVNTXLTILLDNLIGSGLMAVASSTIGIVIFGEILPQALCSRHGLAVXANTI
LLTKFFMLLTFPLXFPISKLLDFFLGQXIRTVYNRXKLMXM (SEQ ID NO: 126).
 Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

This gene is expressed primarily in the following tissues/cDNA libraries: Synovial hypoxia-RSF subtracted and to a lesser extent in Soares placenta Nb2HP; NCI_CGAP_GCB1; NCI_CGAP_Br2; Colon Normal II; Human Cerebellum; Soares_NFL_T_GBC_S1; Soares_fetal_heart_NbHH19W; Human Adult Testes, Large Inserts, Reexcision; Human Fetal Heart; NCI_CGAP_GC6; Human Adult Pulmonary,re-excision; Soares retina N2b4HR; Soares ovary tumor NbHOT; Soares_NhHMPu_S1; Soares_testis_NHT; Soares infant brain 1NIB; Human epidermal keratinocyte; Breast, Cancer: (4005385 A2); Human OB HOS treated (10 nM E2) fraction I; NTERA2 teratocarcinoma cell line+retinoic acid (14 days); Human retina cDNA randomly primed sublibrary; Human Colon Cancer,re-excision; Smooth muscle, IL1b induced; NTERA2 + retinoic acid, 14 days; Human endometrial stromal cells; Ovary, Cancer: (4004332 A2); Human Brain, Striatum; Soares_pineal_gland_N3HPG; Human Hypothalmus,Schizophrenia; Human Pancreas Tumor, Reexcision; NCI_CGAP_CLL1; NCI_CGAP_Pan1; 12 Week Old Early Stage Human; Human Placenta; Dendritic cells, pooled; Human Fetal Kidney, Reexcision; Human Osteoclastoma; NCI_CGAP_ Brn25; Soares_placenta-8to9weeks_2NbHP8to9W; Spleen, Chronic lymphocytic leukemia; Human Testes; Soares_parathyroid_tumor_NbHPA; Soares_total_fetus_Nb2HF8_9w; Soares_fetal_liver_spleen_1NPLS_S1 and Soares fetal liver spleen 1NFLS.

Polynucleotides and polypeptides of the invention are useful as reagents for differential identification of the tissue(s) or cell type(s) present in a biological sample and for diagnosis of diseases and conditions which include but are not limited to: disorders of the immune, musculoskeletal, nervous and/or reproductive systems. Similarly, polypeptides and antibodies directed to these polypeptides are useful in providing immunological probes for differential identification of the tissue(s) or cell type(s). For a number of disorders of the above tissues or cells, particularly of the immune, musculoskeletal, nervous and/or reproductive systems, expression of this gene at significantly higher or lower levels may be routinely detected in certain tissues or cell types (e.g., immune, nervous, musculoskeletal, reproductive, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

Preferential expression in hypoxic synoviocytes and homology to Mycoplasma and C. elegans hemolysins indicates utility for the diagnosis, study and treatment of arthritic and general musculoskeletal disorders as well as hemopoietic and immune disorders and impaired wound healing. The tissue distribution indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13; 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types.

Alternatively, the tissue distribution in cerebellum and brain tissues indicates that polynucleotides and/or polypeptides corresponding to this gene would be useful for the detection, diagnosis, treatment, and/or prevention of neurodegenerative disease states, behavioral disorders, or inflammatory conditions. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, toxic neuropathies induced by neurotoxins, peripheral neuropathies, multiple sclerosis, neoplasia of neuroectodermal origin, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception. In addition, elevated expression of this gene product in regions of the brain indicates it plays a role in normal neural function. Potentially, this gene product is involved in synapse formation, neurotransmission, learning, cognition, homeostasis, or neuronal differentiation or survival. Similarly, the expression in reproductive tissues indicates that polynucleotides and/or polypeptides corresponding to this gene would be useful in the treatment, prevention and/or detection of disorders of reproductive organs, particularly cancers including, but not limited to, ovarian, endometrial, testicular and of other tissues where expression has been indicated.

### FEATURES OF PROTEIN ENCODED BY GENE NO: 13

In specific embodiments, polypeptides of the invention comprise, or alternatively consist of, the following amino acid sequence:
MPRASCCHCLCGPESWTCPSDSGVAAWAGSVTLGCYTFWELDRSGCLGGL WHPGMLYILGTGQEWLLGWALAPWDVIHSGNCNQPLEKSELQEVTLGWDL GTWPGQHGDTRLQQELWSVLGSLGAGLRPWAGFLQAEVLGKW GSQASCRLPHYVASDSSSLMKAIWFITLKL (SEQ ID NO: 130). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

The gene encoding the disclosed cDNA is believed to reside on chromosome 8, more specifically at interval D8S272-qTEL. Accordingly, polynucleotides related to this invention would be useful as a marker in linkage analysis for chromosome 8.

This gene is expressed primarily in the following tissues/cDNA libraries: Human Cerebellum and to a lesser extent in Stratagene schizo brain S11; Human Whole Brain #2 - Oligo dT > 1.5Kb; Human OB MG63 control fraction I; Human Brain, Striatum; Human Substantia Nigra; Soares breast 3NbHBst; Human Fetal Kidney, Reexcision; normalized infant brain cDNA; Keratinocyte and Soares infant brain 1NIB.

Polynucleotides and polypeptides of the invention are useful as reagents for differential identification of the tissue(s) or cell type(s) present in a biological sample and for diagnosis of diseases and conditions which include but are not limited to: neurodegenerative disorders and/or disorders of the reproductive system. Similarly, polypeptides and antibodies directed to these polypeptides are useful in providing immunological probes for differential identification of the tissue(s) or cell type(s). For a number of disorders of the above tissues or cells, particularly of the nervous and/or reproductive system (s), expression of this gene at significantly higher or lower levels may be routinely detected in certain tissues or cell types (e.g., nervous, neural, neuronal, cancerous and wounded tissues) or bodily fluids (e.g., amniotic fluid, lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The expression of this gene in neurological tissues indicates that polynucleotides and polypeptides corresponding to this gene would be useful for the detection, diagnosis, treatment, and/or prevention of neurodegenerative disease states, behavioral disorders, or inflammatory conditions. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, toxic neuropathies induced by neurotoxins, peripheral neuropathies, multiple sclerosis; neoplasia of neuroectodermal origin, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception. In addition, elevated expression of this gene product in regions of the brain indicates it plays a role in normal neural function. Potentially, this gene product is involved in synapse formation, neurotransmission, learning, cognition, homeostasis, or neuronal differentiation or survival.

In addition, the expression of this gene in reproductive tissues indicates that polynucleotides and/or polypeptides corresponding to this gene would be useful in the treatment, prevention, detection and/or diagnosis of disorders of the reproductive system, including, but not limited to, breast cancer, testicular cancer and/or ovarian cancer and/or developmental disorders. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 14

This gene is expressed primarily in PC3 Prostate cell line.

Polynucleotides and polypeptides of the invention are useful as reagents for differential identification of the tissue(s) or cell type(s) present in a biological sample and for diagnosis of diseases and conditions which include but are not limited to: disorders of the male reproductive system, particularly diseases of the prostate. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g., reproductive, cancerous and wounded tissues) or bodily fluids (e.g., semen, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.

The protein product of this clone would be useful for the treatment, detection, and/or prevention of reproductive diseases and/or disorders, particularly prostate cancers. The expression in the prostate tissue may indicate polynucleotides and polypeptides corresponding to this gene would be useful for treating, preventing, detecting and/or diagnosing disorders of the prostate, including inflammatory disorders, such as chronic prostatitis, granulomatous prostatitis and malacoplakia, prostatic hyperplasia and prostate neoplastic disorders, including adenocarcinoma, transitional cell carcinomas, ductal carcinomas, squamous cell carcinomas, or as hormones or factors with systemic or reproductive functions. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

### FEATURES OF PROTEIN ENCODED BY GENE NO: 15

The computer algorithm BLASTX has been used to determine that the translation product of this gene shares sequence homology with the murine ubiquitinating enzyme E2-230 kDa (see, e.g., Genbank Accession No. gb|AAA69916.1| (a portion of which is shown as SEQ ID NO:131); all information available through the recited accession number is incorporated herein by reference). Based on the structural similarity these homologous polypeptides are expected to share at least some biological activities. Such activities are known in the art, some of which are described elsewhere herein. Assays for determining such activities are also known in the art, some of which have been described elsewhere herein.

In specific embodiments, polypeptides of the invention comprise, or alternatively consist of, an amino acid sequence selected from the group:
WGALSVCLPSQGRASQDCLGMSRKKLRPGGGLYGQEGEAPVEEAGCADHV MLPRHPV (SEQ ID NO: 132);
IPGSTHASADSVSPPPSVITQENLGRVKRLGCFDHAQRQLGERCLYVFPAKVE PAKIAWECPEKNCAQGEGSMAKKVKRLLKKQVVRIMSCSPDTQCSRDHSME DPDKKGESKTKSEAESASPEETPDGSASPVEMQDEGAEEPHEAGEQLPPFLLK EGRDDRLHSAEQDADDEAADDTDDTSSVTSSASSTTSSQSGSGTSRkKSIPLSI KNLKRKHKRKKNKITRDFKPGDRVAVEVVTTMTSADVMWQDGSVECNIRS NDLFPVHHLDNNEFCPGDFVVDKRVQSCPDPAVYGVVQSGDHIGRTCMVK WFKLRPSGDDVELIGEEEDVSVYDIADHPDFRFRTTDIVIRIGNTEDGAPHKED EPSVGQVARVDVSSKVEVVWADNSKTIILPQHLYNIESEIEESDYDSVEGSTSG ASSDEWEDDSDSWETDNGLVEDEHPKIEEPPIPPLEQPVAPEDKGVVISEEAA TAAVQGAVAMAAPMAGLMEKAGKDGPPKSFRELKEAIKILESLKNMTVEQL LTGSPTSPTVEPEKPTREKKFLDDIKKLQENLKKTLDNVAIVEEEKMEAVPDV ERKEDKPEGQSPVKAEWPSETPVLCQQCGGKPGVTFTSAKGEVFSVLEFAPS NHSFKKIEFQPPEAKKFFSTVRKEMALLATSLPEGIMVKTFEDRMASRLGMG RCRCAVWAPSAQEATNLGSPNLRG (SEQ ID NO: 133);
MAKKVKRLLKKQWRIMSCSPDTQCSRDHSMEDPDKKGESKTKSEAESASP EETPDGSASPVEMQDEGAEEPHEAGEQLPPFLLKEGRDDRLHSAEQDADDEA ADDTDDTSSVTSSASSTTSSQSGSGTSRKKSIPLSIKNLKRXHKRKKNKTTRDF KPGDRVAVEVVTTMTSADVMWQDGSVECNIRSNDLFPVHHLDNNEFCPGDF VVDKRVQSCPDPAVYGVVQSGDHIGRTCMVKWFKLRPSGDDVELIGEEEDV SVYDIADHPDFRFRTTDIVIRIGNTEDGAPHKEDEPSVGQVARVDVSSKVEVV WADNSKTIILPQHLYNIESEIEESDYDSVEGSTSGASSDEWEDDSDSWETDNG LVEDEHPKIEEPPIPPLEQPVAPEDKGWISEEAATAAVQGAVAMAAPMAGL MEKAGKDGPPKSFRELKEAIKILESLKNMTVEQLLTGSPTSPTVEPEKPTREK KFLDDIKKLQENLKKTLDNVAIVEEEKMEAVPDVERKEDKPEGQSPVKAEWP SETPVLCQQCGGKPGVTFTSAKGEVFSVLEFAPSNHSFKKIEFQPPEAKKFFST VRKEMALLATSLPEGIMVKTFEDRMASRLGMGRCRCAVWAPSAQEATNLGS PNLRG (SEQ ID NO:134). Moreover, fragments and variants of these polypeptides (such as, for example, fragments as described herein, polypeptides at least 80%, 85%, 90%, 95%, 96%, 97%, 98%, 99%, or 100% identical to these polypeptides, or polypeptides encoded by a polynucleotide which hybridizes, under stringent conditions, to the polynucleotide encoding these polypeptides) are encompassed by the invention. Antibodies that bind polypeptides of the invention and polynucleotides encoding these polypeptides are also encompassed by the invention.

In addition, the translation product of this gene shown as SEQ ID NO: 134 is predicted to have a nuclear localization and may represent a novel factor involved in DNA compaction, replication and/or transcription.

This gene is expressed primarily in the following tissues/cDNA libraries: Healing groin wound, 6.5 hours post incision; Dendritic cells, pooled; HUMAN B CELL LYMPHOMA; Bone Marrow Cell Line (RS4,11); normalized infant brain cDNA.

Polynucleotides and polypeptides of the invention are useful as reagents for differential identification of the tissue(s) or cell type(s) present in a biological sample and for diagnosis of diseases and conditions which include but are not limited to: disorders of the immune and/or hematopoietic systems. Similarly, polypeptides and antibodies directed to these polypeptides are useful in providing immunological probes for differential identification of the tissue(s) or cell type(s). For a number of disorders of the above tissues or cells, particularly of the immune and/or hematopoietic systems, expression of this gene at significantly higher or lower levels may be routinely detected in certain tissues or cell types (e.g., immune, hematopoietic, cancerous and wounded tissues) or bodily fluids (e.g., lymph, serum, plasma, urine, synovial fluid and spinal fluid) or another tissue or sample taken from an individual having such a disorder, relative to the standard gene expression level, i.e., the expression level in healthy tissue or bodily fluid from an individual not having the disorder.
The tissue distribution indicates that polynucleotides and polypeptides corresponding to this gene would be useful for the diagnosis, detection, prevention and/or treatment of a variety of immune system disorders. Representative uses are described in the "Immune Activity" and "Infectious Disease" sections below, in Example 11, 13, 14, 16, 18, 19, 20, and 27, and elsewhere herein. Briefly, the expression of this gene product indicates a role in regulating the proliferation; survival; differentiation; and/or activation of hematopoietic cell lineages, including blood stem cells. Involvement in the regulation of cytokine production, antigen presentation, or other processes indicates a usefulness in the treatment of cancer (e.g., by boosting immune responses). Expression in cells of lymphoid origin, indicates the natural gene product would be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease; sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, Hodgkin's rheumatoid arthritis, Sjogren's disease, scleroderma and tissues. Moreover, the protein may represent a secreted factor that influences the differentiation or behavior of other blood cells, or that recruits hematopoietic cells to sites of injury. In addition, this gene product may have commercial utility in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to.its use as a nutritional supplement. Protein, as well as, antibodies directed against the protein may show utility as a tumot marker and/or immunotherapy targets for the above listed tissues.

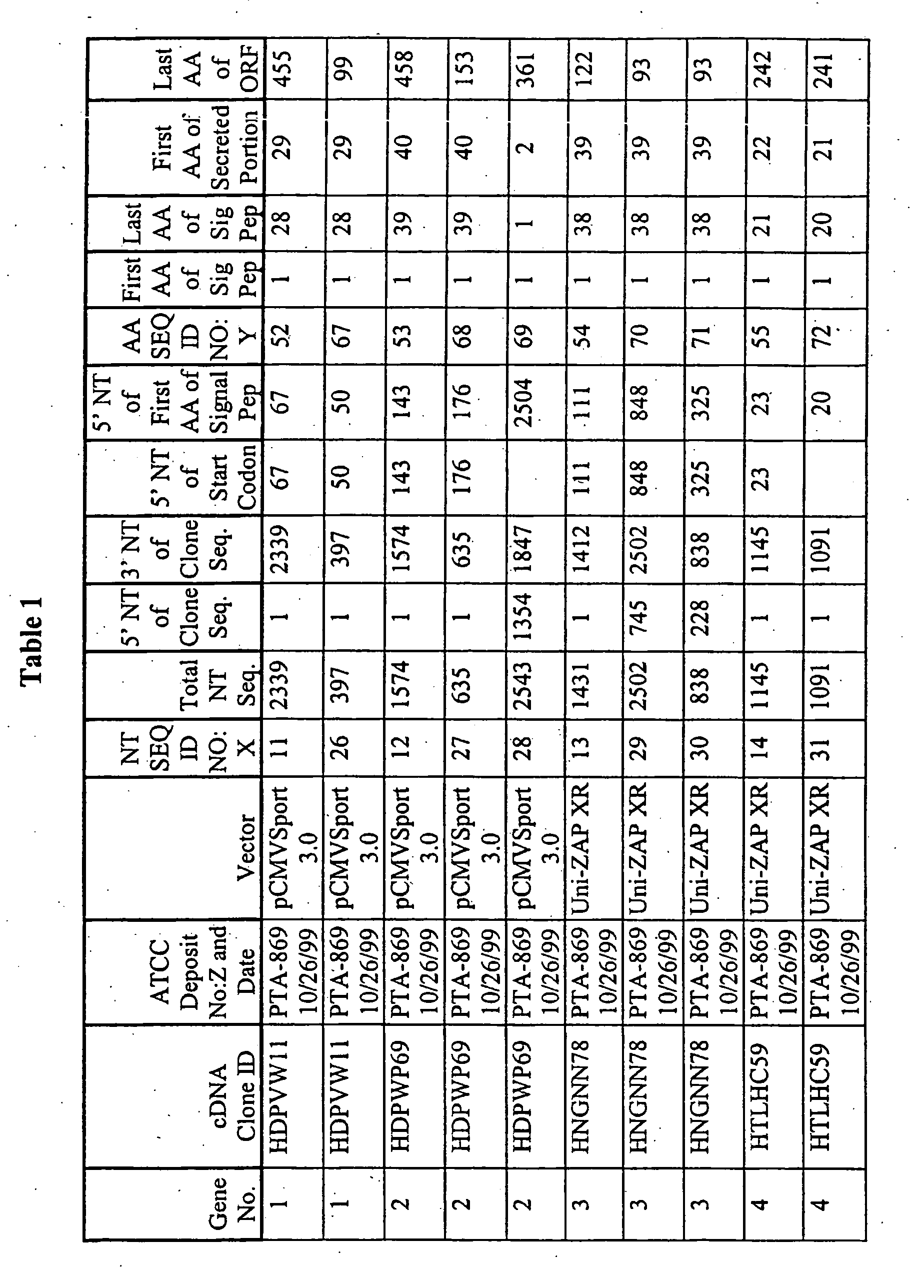

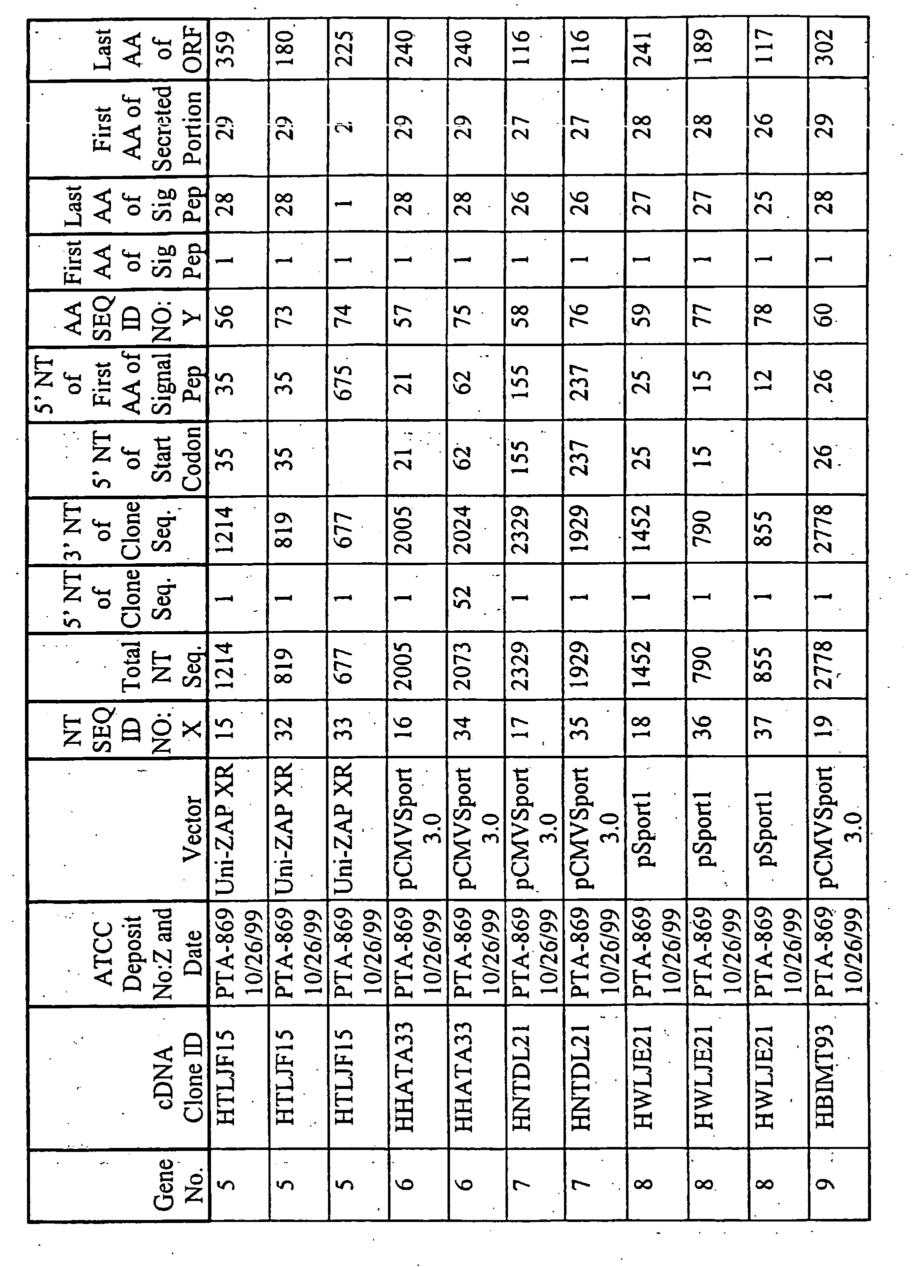

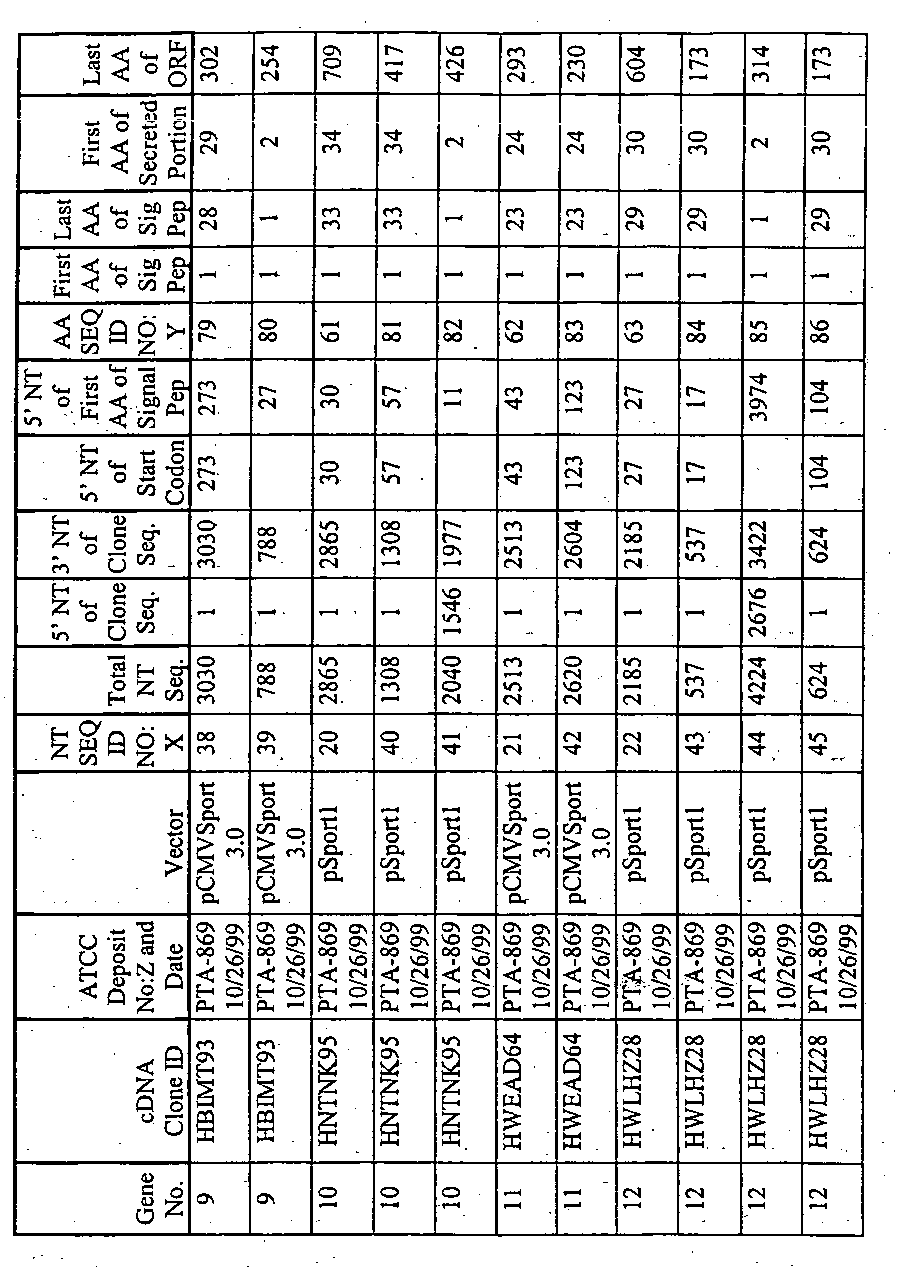

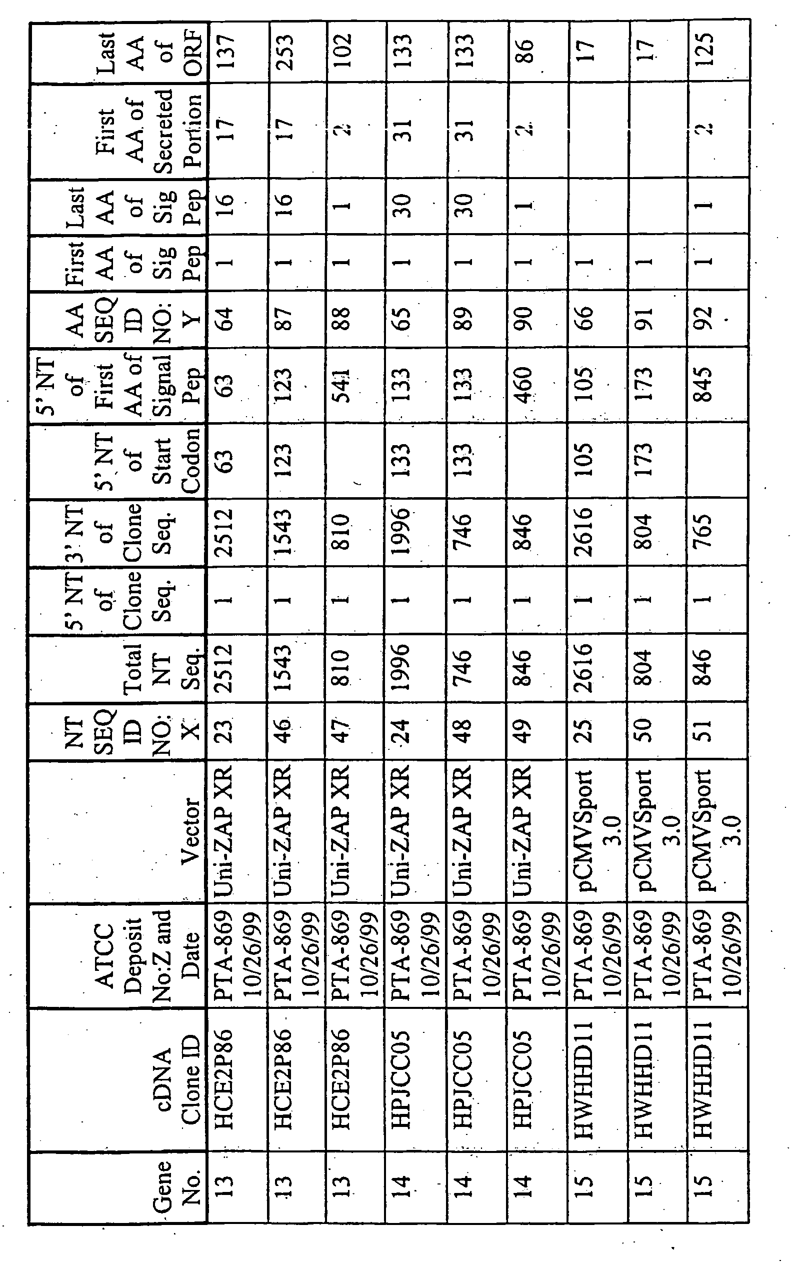

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly, the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X (where X may be any of the polynucleotide sequences disclosed in the sequence listing) and the translated SEQ ID NO:Y (where Y may be any of the polypeptide sequences disclosed in the sequence listing) are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used, for example, to generate antibodies which bind specifically to proteins containing the polypeptides and the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are allelic variants, orthologs, and/or species homologs. Procedures known in the art can be used to obtain full-length genes, allelic variants, splice variants, full-length coding portions, orthologs, and/or species homologs of genes corresponding to SEQ ID NO:X, SEQ ID NO:Y, or a deposited clone, using information from the sequences disclosed herein or the clones deposited with the ATCC. For example, allelic variants and/or species homologs may be isolated and identified by making suitable probes or primers from the sequences provided herein and screening a suitable nucleic acid source for allelic variants and/or the desired homologue.

Table 2 provides preferred epitopes contained in certain embodiments of the invention and polynucleotide sequences that may be disclaimed according to certain embodiments of the invention. The first column refers to each "Gene No." described above in Table 1. The second column provides the sequence identifier, "NT SEQ ID NO:X", for polynucleotide sequences disclosed in Table 1. The third column provides the sequence identifier, "AA SEQ ID NO:Y", for polypeptide sequences. disclosed in Table 1. The fourth column provides a unique integer "ntA" where "ntA" is any integer between 1 and the final nucleotide minus 15 of SEQ ID NO:X, and the fifth column provides a unique integer "ntB" where "ntB" is any integer between 15 and the final nucleotide of SEQ ID NO:X, where both ntA and ntB correspond to the positions of nucleotide residues shown in SEQ ID NO:X, and where ntB is greater than or equal to a + 14. For each of the polynucleotides shown as SEQ ID NO:X, the uniquely defined integers can be substituted into the general formula of a-b, and used to describe polynucleotides which may be preferably excluded from the invention. Column 6 lists residues comprising predicted epitopes contained in the polypeptides encoded by each of the preferred ORFs (SEQ ID NO:Y). Identification of potential immunogenic regions was performed according to the method of Jameson and Wolf ((1988) CABIOS, **4**,181-186); specifically, the Genetics Computer Group (GCG) implementation of this algorithm, embodied in the program PEPTIDESTRUCTUTRE (Wisconsin Package v10.0, Genetics Computer Group (GCG), Madison, Wisc.). This method returns a measure of the probability that a given residue is found on the surface of the protein. Regions where the antigenic index score is greater than 0.9 over at least 6 amino acids are indicated in Table 2 as "Preferred Epitopes". Polypeptides of the invention may possess one, two, three, four, five or more antigenic epitopes comprising residues described in Table 2. It will be appreciated that depending on the analytical criteria used to predict antigenic determinants, the exact address of the determinant may vary slightly.

Table 3 summarizes the expression profile of polynucleotides corresponding to the clones disclosed in Table 1. The first column provides a unique clone identifier, "Clone ID", for a cDNA clone related to each contig sequence disclosed in Table 1. Column 2, "Library Codes" shows the expression profile of tissue and/or cell line libraries which express the polynucleotides of the invention. Each Library Code in column 2 represents a tissue/cell source identifier code corresponding to the Library Code and Library description provided in Table 4. Expression of these polynucleotides was not observed in the other tissues and/or cell libraries tested. One of skill in the art could routinely use this information to identify tissues which show a predominant expression pattern of the corresponding polynucleotide of the invention or to identify polynucleotides which show predominant and/or specific tissue expression.

Table 4 provides a key to the Library Code disclosed in Table 3. Column 1 provides the Library Code disclosed in Table 3, column 2. Column 2 provides a description of the tissue or cell source from which the corresponding library was derived. Library codes corresponding to diseased Tissues are indicated in column 3 with the word "disease".

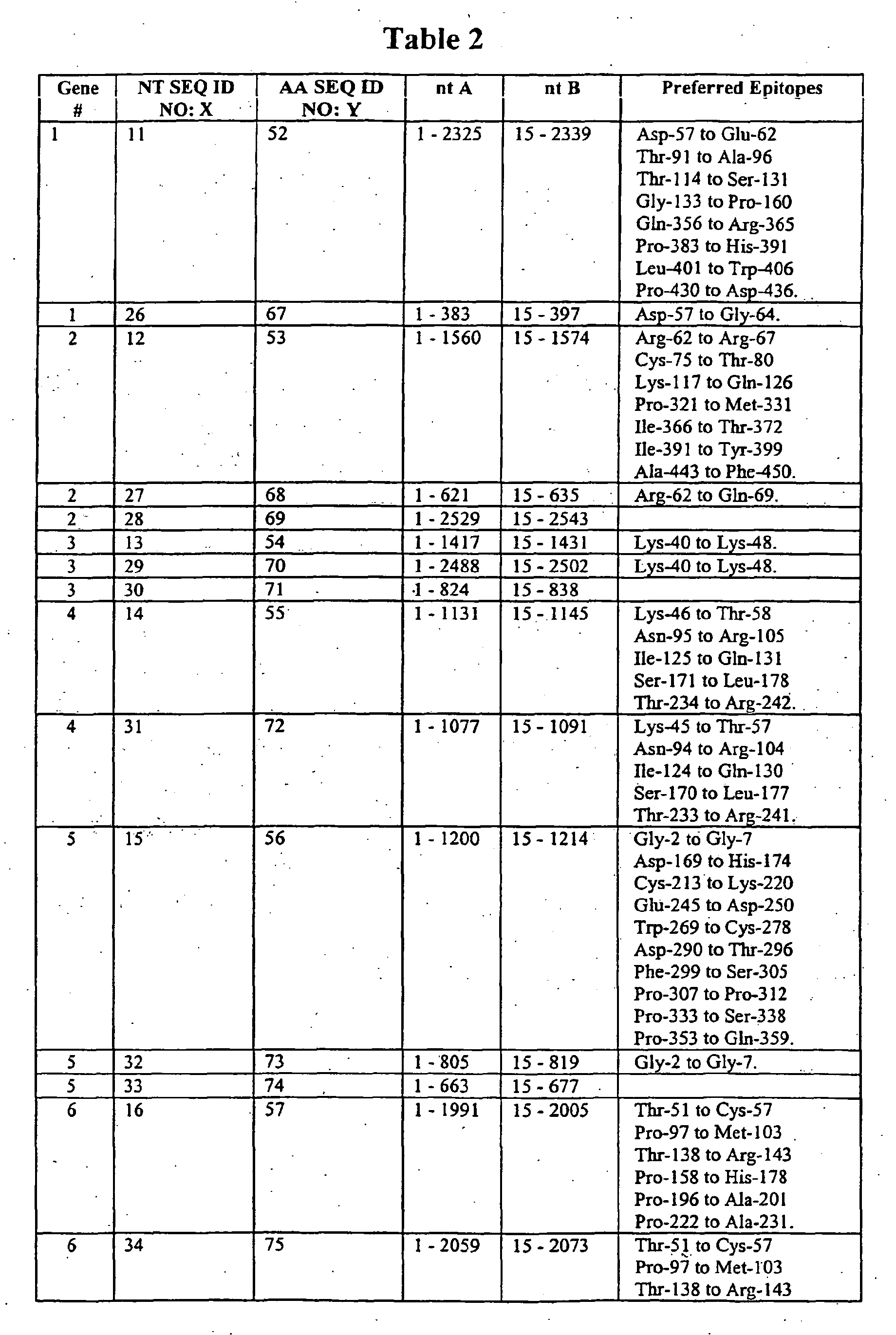

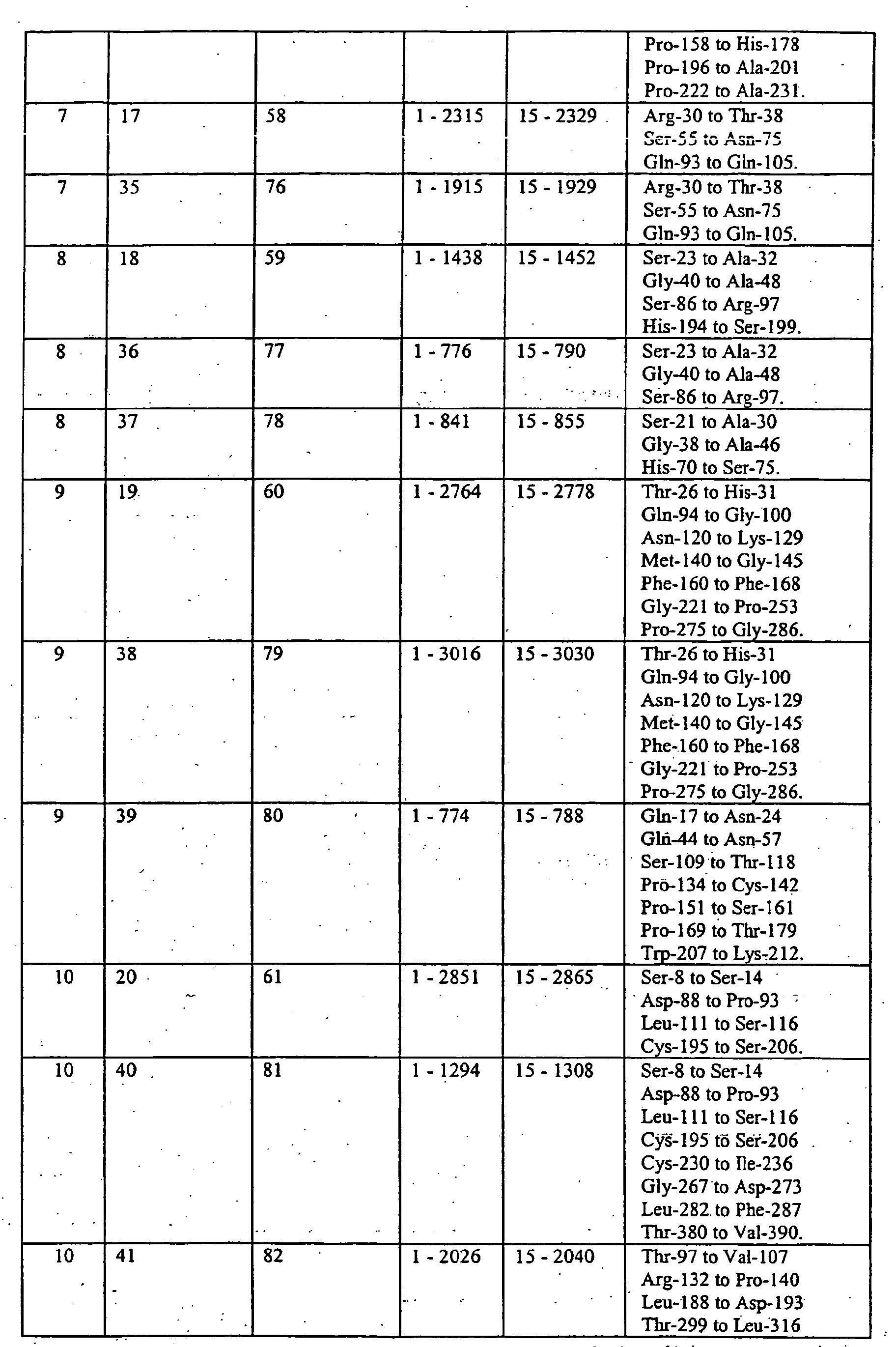

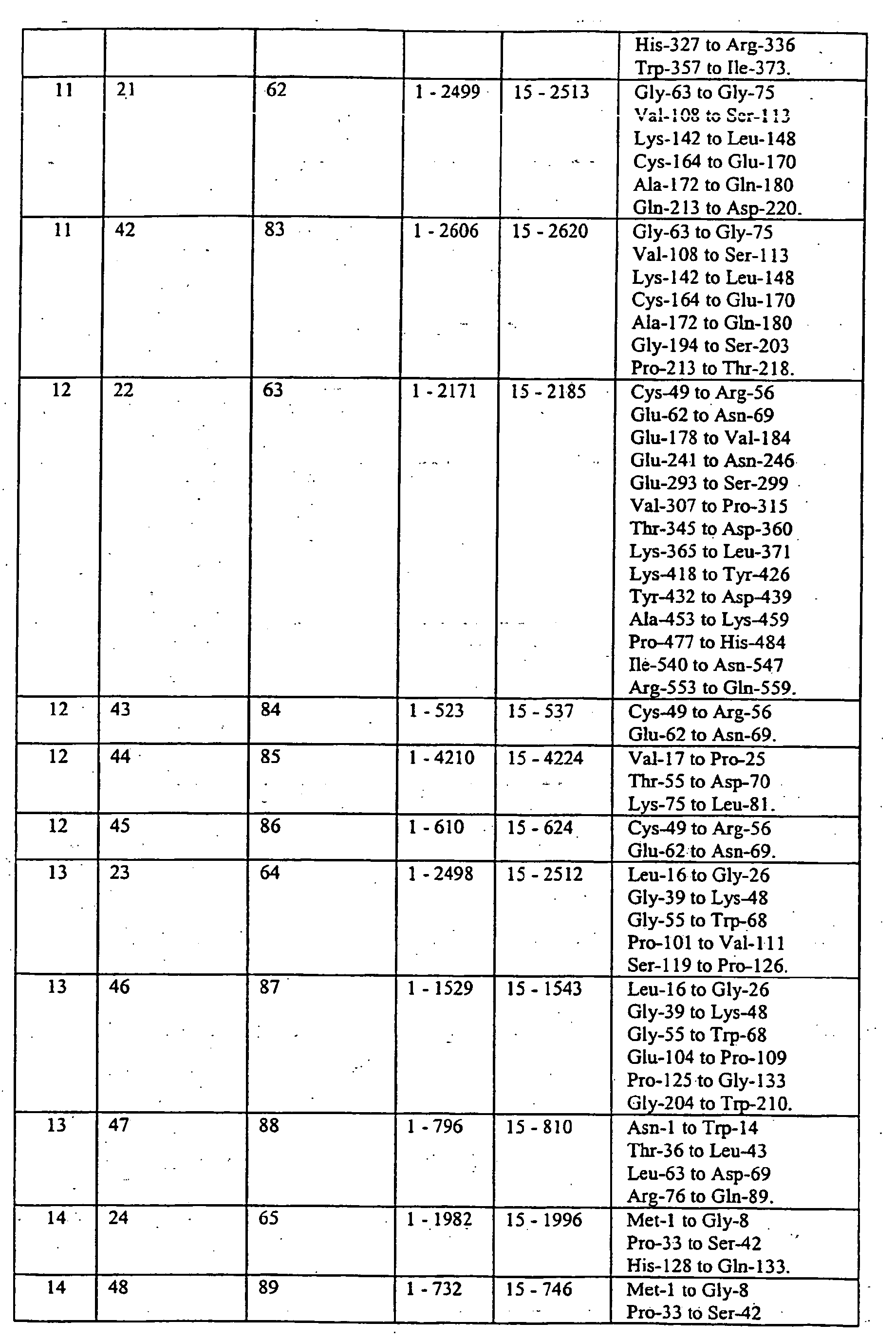

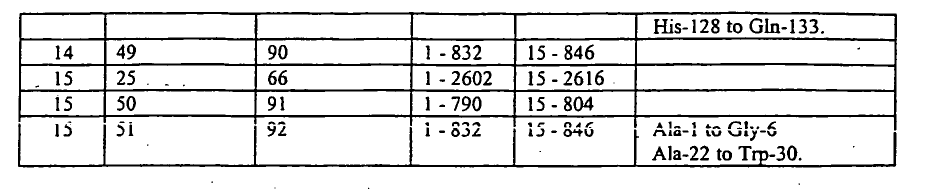

**Table 3**

| **Clone ID** | **Library Codes** |
|---|---|
| HDPVW11 | H0087 H0100 H0124 H0255 H0327 H0393 H0428 H0457 H0484 H0506 H0509 H0521 H0522 H0549 H0575 H0581 H0587 H0619 H0645 H0653 H0670 H0672 H0698 L1290 S0028 S0212 S0278 S0280 S0354 S0380 S0386 |
| HDPWP69 | H0038 H0046 H0123 H0250 H0266 H0484 H0506 H0543 H0672 L1290 S0144 S0374 S3014 |
| HNGNN78 | H0271 H0416 H0457 S0428 |
| HTLHC59 | H0038 H0253 H0510 H0616 H0618 L1290 |
| HTLJF15 | H0253 H0618 L1290 S0052 |
| HHATA33 | H0003 H0012 H0028 H0038 H0042 H0046 H0052 H0063 H0087 H0102 H0124 H0132 H0135 H0187 H0255 H0264 H0265 H0266 H0292 H0294 H0306 H0341 H0376 H0386 H0393 H0402 H0412 H0413 H0422 H0423 H0427 H0435 H0436 H0445 H0457 H0486 H0494 H0497 H0509 H0510 H0518 H0519 H0520 H0522 H0529 H0539 H0542 H0543 H0545 H0547 H0551 H0555 H0556 H0580 H0583 H0586 H0606 H0617 H0618 H0619 H0622 H0624 H0626 H0627 H0628 H0630 H0650 H0651 H0660 H0663 H0664 H0666 H0667 H0668 H0672 H0690 H0696 H0697 H0698 L1290 S0002 S0027 S0028 S0037 S0045 S0051 S0126 S0212 S0222 S0250 S0278 S0328 S0358 S0360 S0366 S0404 S0418 S0420 S0424 S0458 S0470 S3014 T0006 |
| HNTDL21 | H0309 H0519 H0529 H0574 H0615 H0666 L1290 S0378 S0474 |
| HWLJE21 | H0012 H0024 H0026 H0032 H0052 H0058 H0087 H0100 H0111 H0131 H0135 H0144 H0156 H0170 H0252 H0253 H0290 H0327 H0328 H0352 H0424 H0428 H0445 H0489 H0521 H0539 H0544 H0545 H0546 H0547 H0550 H0560 H0574 H0575 H0576 H0583 H0593 H0599 H0600 H0606 H0617 H0618 H0635 H0637 H0638 H0651 H0656 H0660 H0662 H0672 H0687 H0690 L0022 L1290 S0002 S0007 S0044 S0046 S0049 S0134 S0192 S0210 S0214 S0216 S0344 S0356 S0358 S0388 S0420 S0442 T0006 T0069 |
| HBIMT93 | H0036 H0040 H0519 H0522 H0580 H0592 H0593 H0634 H0656 L0022 S0002 S0278 S0354 S0420 S0434 T0109 |
| HNTNK95 | H0030 H0031 H0036 H0046 H0051 H0052 H0056 H0083 H0087 H0124 H0135 H0156 H0194 H0265 H0266 H0286 H0290 H0309 H0327 H0333 H0351 H0393 H0435 H0457 H0494 H0509 H0520 H0521 H0522 H0529 H0539 H0540 H0544 H0545 H0547 H0555 H0556 H0574 H0575 H0580 H0583 H0592 H0619 H0620 H0623 H0628 H0632 H0634 H0638 H0645 H0646 H0656 H0657 H0658 H0659 H0660 H0662 H0672 H0675 H0687 H0689 H0698 H0702 L0022 S0001 S0002 S0011 S0022 S0027 S0028 S0037 S0040 S0045 S0046 S0053 S0114 S0134 S0144 S0152 S0212 S0242 S0278 S0328 S0344 S0356 S0364 S0374 S0376 S0380 S0418 S0420 S0426 S3012 T0010 T0041 T0042 T0048 |
| HWEAD64 | H0013 H0014 H0021 H0040 H0046 H0050 H0100 H0264 H0270 H0316 H0318 H0327 H0373 H0422 H0435 H0436 H0445 H0478 H0497 H0506 H0518 H0519 H0521 H0522 H0529 H0543 H0547 H0555 H0556 H0561 H0575 H0587 H0590 H0595 H0599 H0601 H0616 H0624 H0625 H0641 H0644 H0645 H0648 H0658 H0659 H0670 H0672 H0684 H0686 L0022 S0002 S0003 S0026 S0046 S0051 S0114 S0126 S0132 S0142 S0144 S0152 S0214 S0222 S0328 S0342 S0426 T0041 T0114 |
| HWLHZ28 | H0050 H0052 H0170 H0251 H0271 H0292 H0445 H0483 H0520 H0544 H0575 H0618 H0620 H0622 H0666 H0669 L0022 S0037 S0049 S0354 |
| HCE2P86 | H0051 H0052 H0150 H0284 H0441 H0494 H0539 H0550 H0599 H0620 H0646 H0687 L0022 S0036 S0038 S0049 |
| HPJCC05 | S0152 |
| HWHHD11 | H0041 H0052 H0081 H0135 H0224 H0225 H0255 H0265 H0286 H0309 H0373 H0411 H0542 H0547 H0549 H0556 H0581 H0586 H0592 H0618 H0620 H0646 H0674 H0684 L0022 S0007 S0010 S0028 S0040 S0114 S0216 S0222 S0328 S0360 S0424 T0006 T0049 |

**Table 4**

| **Library Code** | **Library Description** | **Disease** |
|---|---|---|
| H0003 | Human Adult Liver | |
| H0012 | Human Fetal Kidney | |
| H0013 | Human 8 Week Whole Embryo | |
| H0014 | Human Gall Bladder | |
| H0021 | Human Infant Adrenal Gland | |
| H0024 | Human Fetal Lung III | |
| H0026 | Namalwa Cells | |
| H0028 | Human Old Ovary | |
| H0030 | Human Placenta | |
| H0031 | Human Placenta | |
| H0032 | Human Prostate | |
| H0036 | Human Adult Small Intestine | |
| H0038 | Human Testes | |
| H0040 | Human Testes Tumor | disease |
| H0041 | Human Fetal Bone | |
| H0042 | Human Adult Pulmonary | |
| H0046 | Human Endometrial Tumor | disease |
| H0050 | Human Fetal Heart | |
| H0051 | Human Hippocampus | |
| H0052 | Human Cerebellum | |
| H0056 | Human Umbilical Vein, Endo. remake | |
| H0058 | Human Thymus Tumor | disease |
| H0063 | Human Thymus | |
| H0081 | Human Fetal Epithelium (Skin) | |
| H0083 | HUMAN JURKAT MEMBRANE BOUND POLYSOMES | |
| H0087 | Human Thymus | |
| H0100 | Human Whole Six Week Old Embryo | |
| H0102 | Human Whole 6 Week Old Embryo (II), subt | |
| H0111 | Human Placenta, subtracted | |
| H0123 | Human Fetal Dura Mater | |
| H0124 | Human Rhabdomyosarcoma | disease |
| H0131 | LNCAP + o.3nM R1881 | |
| H0132 | LNCAP + 30nM R1881 | |
| H0135 | Human Synovial Sarcoma | |
| H0144 | Nine Week Old Early Stage Human | |
| H0150 | Human Epididymus | |
| H0156 | Human Adrenal Gland Tumor | disease |
| H0170 | 12 Week Old Early Stage Human | |
| H0187 | Resting T-Cell | |
| H0194 | Human Cerebellum, subtracted | |
| H0224 | Activated T-Cells, 12 hrs, subtracted | |
| H0225 | Activated T-Cells, 12hrs, differentially expressed | |
| H0250 | Human Activated Monocytes | |
| H0251 | Human Chondrosarcoma | disease |
| H0252 | Human Osteosarcoma | disease |
| H0253 | Human adult testis, large inserts | |
| H0255 | breast lymph node CDNA library | |
| H0264 | human tonsils | |
| H0265 | Activated T-Cell (12hs)/Thiouridine labelledEco | |
| H0266 | Human Microvascular Endothelial Cells, fract. A | |
| H0270 | HPAS (human pancreas, subtracted) | |
| H0271 | Human Neutrophil, Activated | |
| H0284 | Human OB MG63 control fraction I | |
| H0286 | Human OB MG63 treated (10 nM E2) fraction I | |
| H0290 | Human OB HOS treated (1 nM E2) fraction I | |
| H0292 | Human OB HOS treated (10 nM E2) fraction I | |
| H0294 | Amniotic Cells - TNF induced | |
| H0306 | CD34 depleted Buffy Coat (Cord Blood) | |
| H0309 | Human Chronic Synovitis | disease |
| H0316 | HUMAN STOMACH | |
| H0318 | HUMAN B CELL LYMPHOMA | disease |
| H0327 | human corpus colosum | |
| H0328 | human ovarian cancer | disease |
| H0333 | Hemangiopericytoma | disease |
| H0341 | Bone Marrow Cell Line-(RS4,11) | |
| H0351 | Glioblastoma | disease |
| H0352 | wilm's tumor | disease |
| H0373 | Human Heart | |
| H0376 | Human Spleen | |
| H0386 | Leukocyte and Lung, 4 screens | |
| H0393 | Fetal Liver, subtraction II | |
| H0402 | CD34 depleted Buffy Coat (Cord Blood), re-excision | |
| H0411 | H Female Bladder, Adult | |
| H0412 | Human umbilical vein endothelial cells, IL-4 induced | |
| H0413 | Human Umbilical Vein Endothelial Cells, uninduced | |
| H0416 | Human Neutrophils, Activated, re-excision | |
| H0422 | T-Cell PHA 16 hrs | |
| H0423 | T-Cell PHA 24 hrs | |
| H0424 | Human Pituitary, subt IX | |
| H0427 | Human Adipose | |
| H0428 | Human Ovary | |
| H0435 | Ovarian Tumor 10-3-95 | |
| H0436 | Resting T-Cell Library,II | |
| H0441 | H. Kidney Cortex, subtracted | |
| H0445 | Spleen, Chronic lymphocytic leukemia | disease |
| H0457 | Human Eosinophils | |
| H0478 | Salivary Gland, Lib 2 | |
| H0483 | Breast Cancer cell line, MDA 36 | |
| H0484 | Breast Cancer Cell line, angiogenic | |
| H0486 | Hodgkin's Lymphoma II | disease |
| H0489 | Crohn's Disease | disease |
| H0494 | Keratinocyte | |
| H0497 | HEL cell line | |
| H0506 | Ulcerative Colitis | |
| H0509 | Liver, Hepatoma | disease |
| H0510 | Human Liver, normal | |
| H0518 | pBMC stimulated w/ poly I/C | |
| H0519 | NTERA2, control | |
| H0520 | NTERA2 + retinoic acid, 14 days | |
| H0521 | Primary Dendritic Cells, lib 1 | |
| H0522 | Primary Dendritic cells,frac 2 | |
| H0529 | Myoloid Progenitor Cell Line | |
| H0539 | Pancreas Islet Cell Tumor | disease |
| H0540 | Skin, burned | |
| H0542 | T Cell helper I | |
| H0543 | T cell helper II | |
| H0544 | Human endometrial stromal cells | |
| H0545 | Human endometrial stromal cells-treated with progesterone | |
| H0546 | Human endometrial stromal cells-treated with estradiol | |
| H0547 | NTERA2 teratocarcinoma cell line+retinoic acid (14 days) | |
| H0549 | H. Epididiymus, caput & corpus | |
| H0550 | H. Epididiymus, cauda | |
| H0551 | Human Thymus Stromal Cells | |
| H0555 | Rejected Kidney, lib 4 | disease |
| H0556 | Activated T-cell(12h)/Thiouridine-re-excision | |
| H0560 | KMH2 | |
| H0561 | L428 | |
| H0574 | Hepatocellular Tumor, re-excision | disease |
| H0575 | Human Adult Pulmonary,re-excision | |
| H0576 | Resting T-Cell, re-excision | |
| H0580 | Dendritic cells, pooled | |
| H0581 | Human Bone Marrow, treated | |
| H0583 | B Cell lymphoma | disease |
| H0586 | Healing groin wound, 6.5 hours post incision | disease |
| H0587 | Healing groin wound, 7.5 hours post incision | disease |
| H0590 | Human adult small intestine,re-excision | |
| H0592 | Healing groin wound - zero hr post-incision (control) | disease |
| H0593 | Olfactory epithelium, nasalcavity | |
| H0595 | Stomach cancer (human),re-excision | disease |
| H0599 | Human Adult Heart,re-excision | |
| H0600 | Healing Abdomen wound,70&90 min post incision | disease |
| H0601 | Healing Abdomen Wound, 15 days post incision | disease |
| H0606 | Human Primary Breast Cancer,re-excision | disease |
| H0615 | Human Ovarian Cancer Reexcision | disease |
| H0616 | Human Testes, Reexcision | |
| H0617 | Human Primary Breast Cancer Reexcision | disease |
| H0618 | Human Adult Testes, Large Inserts, Reexcision | |
| H0619 | Fetal Heart | |
| H0620 | Human Fetal Kidney, Reexcision | |
| H0622 | Human Pancreas Tumor, Reexcision | disease |
| H0623 | Human Umbilical Vein, Reexcision | |
| H0624 | 12 Week Early Stage Human II, Reexcision | |
| H0625 | Ku 812F Basophils Line | |
| H0626 | Saos2 Cells, Untreated | |
| H0627 | Saos2 Cells, Vitamin D3 Treated | |
| H0628 | Human Pre-Differentiated Adipocytes | |
| H0630 | Human Leukocytes,normalized control #4 | |
| H0632 | Hepatocellular Tumor,re-excision | |
| H0634 | Human Testes Tumor, re-excision | disease |
| H0635 | Human Activated T-Cells, re-excision | |
| H0637 | Dendritic Cells From CD34 Cells | |
| H0638 | CD40 activated monocyte dendridic cells | |
| H0641 | LPS activated derived dendritic cells | |
| H0644 | Human Placenta (re-excision) | |
| H0645 | Fetal Heart, re-excision | |
| H0646 | Lung, Cancer (4005313 A3): Invasive Poorly Differentiated Lung Adenocarcinoma, | |
| H0648 | Ovary, Cancer: (4004562 B6) Papillary Serous Cystic Neoplasm, Low Malignant Pot | disease |
| H0650 | B-Celis | |
| H0651 | Ovary, Normal: (9805C040R) | |
| H0653 | Stromal Cells | |
| H0656 | B-cells (unstimulated) | |
| H0657 | B-cells (stimulated) | |
| H0658 | Ovary, Cancer (9809C332): Poorly differentiated adenocarcinoma | disease |
| H0659 | Ovary, Cancer (15395A1F): Grade II Papillary Carcinoma | disease |
| H0660 | Ovary, Cancer. (15799A1F) Poorly differentiated carcinoma | disease |
| H0662 | Breast, Normal: (4005522B2) | |
| H0663 | Breast, Cancer. (4005522 A2) | disease |
| H0664 | Breast, Cancer: (9806C012R) | disease |
| H0666 | Ovary, Cancer: (4004332 A2) | disease |
| H0667 | Stroma) cells(HBM3.18) | |
| H0668 | stromal cell clone 2.5 | |
| H0669 | Breast, Cancer: (4005385 A2) | |
| H0670 | Ovary, Cancer(4004650 A3): Well-Differentiated Micropapillary Serous Carcinoma | |
| H0672 | Ovary, Cancer: (4004576 A8) | |
| H0674 | Human Prostate Cancer, Stage C, re-excission | |
| H0675 | Colon, Cancer: (9808C064R) | |
| H0684 | Ovarian cancer, Serous Papillary Adenocarcinoma | |
| H0686 | Adenocarcinoma of Ovary, Human Cell Line | |
| H0687 | Human normal ovary(#9610G215) | |
| H0689 | Ovarian Cancer | |
| H0690 | Ovarian Cancer, # 9702G001 | |
| H0696 | Prostate Adenocarcinoma | |
| H0697 | NK Cells (NKYao20 Control) | |
| H0698 | NK CellsYao20 IL2 treated for 48 hrs | |
| H0702 | NK15(IL2 treated for 48 hours) | |
| L0022 | Stratagene schizo brain S11 | |
| L1290 | Human placenta polyA+ (TFujiwara) | |
| S0001 | Brain frontal cortex | |
| S0002 | Monocyte activated | |
| S0003 | Human Osteoclastoma | disease |
| S0007 | Early Stage Human Brain | |
| S0010 | Human Amygdala | |
| S0011 | STROMAL -OSTEOCLASTOMA | disease |
| S0022 | Human Osteoclastoma Stromal Cells - unamplified | |
| S0026 | Stromal cell TF274 | |
| S0027 | Smooth muscle, serum treated | |
| S0028 | Smooth muscle,control | |
| S0036 | Human Substantia Nigra | |
| S0037 | Smooth muscle, IL1b induced | |
| S0038 | Human Whole Brain #2 - Oligo dT > 1.5Kb | |
| S0040 | Adipocytes | |
| S0044 | Prostate BPH | disease |
| S0045 | Endothelial cells-control | |
| S0046 | Endothelial-induced | |
| S0049 | Human Brain, Striatum | |
| S0051 | Human Hypothalmus,Schizophrenia | disease |
| S0052 | neutrophils control | |
| S0053 | Neutrophils IL-1 and LPS induced | |
| S0114 | Anergic T-cell | |
| S0126 | Osteoblasts | |
| S0132 | Epithelial-TNFa and INF induced | |
| S0134 | Apoptotic T-cell | |
| S0142 | Macrophage-oxLDL | |
| S0144 | Macrophage (GM-CSF treated) | |
| S0152 | PC3 Prostate cell line | |
| S0192 | Synovial Fibroblasts (control) | |
| S0210 | Messangial cell, frac 2 | |
| S0212 | Bone Marrow Stromal Cell, untreated | |
| S0214 | Human Osteoclastoma, re-excision | disease |
| S0216 | Neutrophils IL-1 and LPS induced | |
| S0222 | H. Frontal cortex,epileptic,re-excision | disease |
| S0242 | Synovial Fibroblasts (Il1/TNF), subt | |
| S0250 | Human Osteoblasts II | disease |
| S0278 | H Macrophage (GM-CSF treated), re-excision | |
| S0280 | Human Adipose Tissue, re-excision | |
| S0328 | Palate carcinoma | disease |
| S0342 | Adipocytes,re-excision | |
| S0344 | Macrophage-oxLDL, re-excision | |
| S0354 | Colon Normal II | |
| S0356 | Colon Carcinoma | disease |
| S0358 | Colon Normal III | |
| S0360 | Colon Tumor II | disease |
| S0364 | Human Quadriceps | |
| S0366 | Human Soleus | |
| S0374 | Normal colon | |
| S0376 | Colon Tumor | disease |
| S0378 | Pancreas normal PCA4 No | |
| S0380 | Pancreas Tumor PCA4 Tu | disease |
| S0386 | Human Whole Brain, re-excision | |
| S0388 | Human Hypothalamus,schizophrenia, re-excision | disease |
| S0404 | Rectum normal | |
| S0418 | CHME Cell Line,treated 5 hrs | |
| S0420 | CHME Cell Line,untreated | |
| S0424 | TF-1 Cell Line GM-CSF Treated | |
| S0426 | Monocyte activated, re-excision | |
| S0428 | Neutrophils control, re-excision | |
| S0434 | Stomach Normal | disease |
| S0442 | Colon Normal | |
| S0458 | Thyroid Normal (SDCA2 No) | |
| S0470 | Adenocarcinoma | disease |
| S0474 | Human blood platelets | |
| S3012 | Smooth Muscle Serum Treated, Norm | |
| S3014 | Smooth muscle, serum induced,re-exc | |
| T0006 | Human Pineal Gland | |
| T0010 | Human Infant Brain | |
| T0041 | Jurkat T-cell G1 phase | |
| T0042 | Jurkat T-Cell, S phase | |
| T0048 | Human Aortic Endothelium | |
| T0049 | Aorta endothelial cells + TNF-a | |
| T0069 | Human Uterus, normal | |
| T0109 | Human (HCC) cell line liver (mouse) metastasis, remake | |
| T0114 | Human (Caco-2) cell line, adenocarcinoma, colon, remake | |

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification, such as multiple histidine residues, or an'additional sequence for stability during recombinant production.

The polypeptides of the present invention are preferably provided in an isolated form, and preferably are substantially purified. A recombinantly produced version of a polypeptide, including the secreted polypeptide, can be substantially purified using techniques described herein or otherwise known in the art, such as, for example, by the one-step method described in Smith and Johnson, Gene 67:31-40 (1988). Polypeptides of the invention also can be purified from natural, synthetic or recombinant sources using techniques described herein or otherwise known in the art, such as, for example, antibodies of the invention raised against the secreted protein.

The present invention provides a polynucleotide comprising, or alternatively consisting of, the nucleic acid sequence of SEQ ID NO:X, and/or a cDNA contained in ATCC deposit Z. The present invention also provides a polypeptide comprising, or alternatively, consisting of, the polypeptide sequence of SEQ ID NO:Y and/or a polypeptide encoded by the cDNA contained in ATCC deposit Z. Polynucleotides encoding a polypeptide comprising, or alternatively consisting of the polypeptide sequence of SEQ ID NO:Y and/or a polypeptide sequence encoded by the cDNA contained in ATCC deposit Z are also encompassed by the invention.

### Signal Sequences

The present invention also encompasses mature forms of the polypeptide having the polypeptide sequence of SEQ ID NO:Y and/or the polypeptide sequence encoded by the cDNA in a deposited clone. Polynucleotides encoding the mature forms (such as, for example, the polynucleotide sequence in SEQ ID NO:X and/or the polynucleotide sequence contained in the cDNA of a deposited clone) are also encompassed by the invention. According to the signal hypothesis, proteins secreted by mammalian cells have a signal or secretary leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species of the protein. Further, it has long been known that cleavage specificity of a secreted protein is ultimately determined by the primary structure of the complete protein, that is, it is inherent in the amino acid sequence of the polypeptide.

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. Nonetheless, the present invention provides the mature protein produced by expression of the polynucleotide sequence of SEQ ID NO:X and/or the polynucleotide sequence contained in the cDNA of a deposited clone, in a mammalian cell (e.g., COS cells, as desribed below). These polypeptides, and the polynucleotides encoding such polypeptides, are contemplated by the present invention.

### Polynucleotide and Polypeptide Variants

The present invention is directed to variants of the polynucleotide sequence disclosed in SEQ ID NO:X, the complementary strand thereto, and/or the cDNA sequence contained in a deposited clone.

The present invention also encompasses variants of the polypeptide sequence disclosed in SEQ ID NO:Y and/or encoded by a deposited clone.

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

The present invention is also directed to nucleic acid molecules which comprise, or alternatively consist of, a nucleotide sequence which is at least 80%, 85%, 90%, 95%, 96%, 97%, 98% or 99% identical to, for example, the nucleotide coding sequence in SEQ ID NO:X or the complementary strand thereto, the nucleotide coding sequence contained in a deposited cDNA clone or the complementary strand thereto, a nucleotide sequence encoding the polypeptide of SEQ ID NO:Y, a nucleotide sequence encoding the polypeptide encoded by the cDNA contained in a deposited clone, and/or polynucleotide fragments of any of these nucleic acid molecules (e.g., those fragments described herein). Polynucleotides which hybridize to these nucleic acid molecules under stringent hybridization conditions or lower stringency conditions are also encompassed by the invention, as are polypeptides encoded by these polynucleotides.

The present invention is also directed to polypeptides which comprise, or alternatively consist of, an amino acid sequence which is at least 80%, 85%, 90%, 95%, 96%, 97%, 98%, 99% identical to, for example, the polypeptide sequence shown in SEQ ID NO:Y, the polypeptide sequence encoded by the cDNA contained in a deposited clone, and/or polypeptide fragments of any of these polypeptides (e.g., those fragments described herein).

By a nucleic acid having a nucleotide sequence at least, for example, 95% "identical" to a reference nucleotide sequence of the present invention, it is intended that the nucleotide sequence of the nucleic acid is identical to the reference sequence except that the nucleotide sequence may include up to five point mutations per each 100 nucleotides of the reference nucleotide sequence encoding the polypeptide. In other words, to obtain a nucleic acid having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragment specified as described herein.

As a practical matter, whether any particular nucleic acid molecule or polypeptide is at least 80%, 85%, 90%, 95%, 96%, 97%, 98% or 99% identical to a nucleotide sequence of the presence invention can be determined conventionally using known computer programs. A preferred method for determining the best overall match between a query sequence (a sequence of the present invention) and a subject sequence, also referred to as a global sequence alignment, can be determined using the FASTDB computer program based on the algorithm ofBrutlag et al. (Comp. App. Biosci. 6:237-245(1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 95%, 96%, 97%, 98% or 99% identical to, for instance, an amino acid sequences shown in Table 1 (SEQ ID NO:Y) or to the amino acid sequence encoded by cDNA contained in a deposited clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245(1990)). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is because the FASTDB program does not account for N-and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are hot matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N-or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level and are included in the present invention. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions
where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989)) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i), substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as, for example, an IgG Fc fusion region peptide, or leader or secretory sequence, or a sequence facilitating purification or (v) fusion of the polypeptide with another compound, such as albumin (including, but not limited to, recombinant albumin (see, e.g., U.S. Patent No. 5,876,969, issued March 2, 1999, EP Patent 0 413 622, and U.S. Patent No. 5,766,883, issued June 16, 1998, herein incorporated by reference in their entirety)). Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

A further embodiment of the invention relates to a polypeptide which comprises the amino acid sequence of the present invention having an amino acid sequence which contains at least one amino acid substitution, but not more than 50 amino acid substitutions, even more preferably, not more than 40 amino acid substitutions, still more preferably, not more than 30 amino acid substitutions, and still even more preferably, not more than 20 amino acid substitutions. Of course, in order of ever-increasing preference, it is highly preferable for a peptide or polypeptide to have an amino acid sequence which comprises the amino acid sequence of the present invention, which contains at least one, but not more than 10, 9, 8, 7, 6, 5, 4, 3, 2 or I amino acid substitutions. In specific embodiments, the number of additions, substitutions, and/or deletions in the amino acid sequence of the present invention or fragments thereof (e.g., the mature form and/or other fragments described herein), is 1-5, 5-10, 5-25, 5-50, 10-50 or 50-150, conservative amino acid substitutions are preferable.

### Polynucleotide and Polypeptide Fragments

The present invention is also directed to polynucleotide fragments of the polynucleotides of the invention.

In the present invention, a "polynucleotide fragment" refers to a short polynucleotide having a nucleic acid sequence which: is a portion of that contained in a deposited clone, or encoding the polypeptide encoded by the cDNA in a deposited clone; is a portion of that shown in SEQ ID NO:X or the complementary strand thereto, or is a portion of a polynucleotide sequence 'encoding the polypeptide of SEQ ID NO:Y. The nucleotide fragments of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A fragment "at least 20 nt in length," for example, is intended to include 20 or more contiguous bases from the cDNA sequence contained in a deposited clone or the nucleotide sequence shown in SEQ ID NO:X. In this context "about" includes the particularly recited value, a value larger or smaller by several (5, 4, 3, 2, or 1) nucleotides, at either terminus or at both termini. These nucleotide fragments have uses that include, but are not limited to, as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments comprising, or alternatively consisting of, a sequence from about nucleotide number 1-50, 51-100, 101-150, 151-200, 201-250,. 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X, or the complementary strand thereto, or the cDNA contained in a deposited clone. In this context "about" includes the particularly recited ranges, and ranges larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein. Polynucleotides which hybridize to these nucleic acid molecules under stringent hybridization conditions or lower stringency conditions are also encompassed by the invention, as are polypeptides encoded by these polynucleotides.

In the present invention, a "polypeptide fragment" refers to an amino acid sequence which is a portion of that contained in SEQ ID NO:Y or encoded by the cDNA contained in a deposited clone. Protein (polypeptide) fragments may be "freestanding," or comprised within a larger polypeptide of which the fragment forms a part or region, most preferably as a single continuous region. Representative examples of polypeptide fragments of the invention, include, for example, fragments comprising, or alternatively consisting of, from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges or values, and ranges or values larger or smaller by several (5, 4, 3, 2, or 1) amino acids, at either extreme or at both extremes. Polynucleotides encoding these polypeptides are also encompassed by the invention.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. :Similarly, polynucleotides encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotides encoding these domains are also contemplated.

Other preferred polypeptide fragments are biologically active fragments. Biologically active fragments are those exhibiting activity similar, but not necessarily identical, to an activity of the polypeptide of the present invention. The biological activity of the fragments may include an improved desired activity, or a decreased undesirable activity. Polynucleotides encoding these polypeptide fragments are also encompassed by the invention.

Preferably, the polynucleotide fragments of the invention encode a polypeptide which demonstrates a functional activity. By a polypeptide demonstrating a "functional activity" is meant, a polypeptide capable of displaying one or more known functional activities associated with a full-length (complete) ,polypeptide of invention protein. Such functional activities include, but are not limited to, biological activity, antigenicity [ability to bind (or compete with a polypeptide of the invention for binding) to an antibody to the polypeptide of the invention], immunogenicity (ability to generate antibody which binds to a polypeptide of the invention), ability to form multimers with polypeptides of the invention, and ability to bind to a receptor or ligand for a polypeptide of the invention.

The functional activity of polypeptides of the invention, and fragments, variants derivatives, and analogs thereof, can be assayed by various methods.

For example, in one embodiment where one is assaying for the ability to bind or compete with full-length polypeptide of the invention for binding to an antibody of the polypeptide of the invention, various immunoassays known in the art can be used, including but not limited to, competitive and non-competitive assay systems using techniques such as radioimmunoassays, ELISA (enzyme linked immunosorbent assay), "sandwich" immunoassays, immunoradiometric assays, gel diffusion precipitation reactions, immunodiffusion assays, in situ immunoassays (using colloidal gold, enzyme or radioisotope labels, for example), western blots, precipitation reactions, agglutination assays (e.g., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

In another embodiment, where a ligand for a polypeptide of the invention identified, or the ability of a polypeptide fragment, variant or derivative of the invention to multimerize is being evaluated, binding can be assayed, e.g., by means well-known in the art, such as, for example, reducing and non-reducing gel chromatography, protein affinity chromatography, and affinity blotting. See generally, Phizicky, E., et al., 1995, Microbiol. Rev. 59:94-123. In another embodiment, physiological correlates of binding of a polypeptide of the invention to its substrates (signal transduction) can be assayed.

In addition, assays described herein (see Examples) and otherwise known in the art may routinely be applied to measure the ability of polypeptides of the invention and fragments, variants derivatives and analogs thereof to elicit related biological activity related to that of the polypeptide of the invention (either in vitro or in vivo). Other methods will be known to the skilled artisan and are within the scope of the invention.

### Epitopes and Antibodies

The present invention encompasses polypeptides comprising, or alternatively consisting of, an epitope of the polypeptide having an amino acid sequence of SEQ ID NO:Y, or an epitope of the polypeptide sequence encoded by a polynucleotide sequence contained in ATCC deposit No. Z or encoded by a polynucleotide that hybridizes to the complement of the sequence of SEQ ID NO:X or contained in ATCC deposit No. Z under stringent hybridization conditions or lower stringency hybridization conditions as defined supra. The present invention further encompasses polynucleotide sequences encoding an epitope of a polypeptide sequence of the invention (such as, for example, the sequence disclosed in SEQ ID NO:X), polynucleotide sequences of the complementary strand of a polynucleotide sequence encoding an epitope of the invention, and polynucleotide sequences which hybridize to the complementary strand under stringent hybridization conditions or lower stringency hybridization conditions defined supra.

The term "epitopes," as used herein, refers to portions of a polypeptide having antigenic or immunogenic activity in an animal, preferably a mammal, and most preferably in a human. In a preferred embodiment, the present invention encompasses a polypeptide comprising an epitope, as well as the polynucleotide encoding this polypeptide. An "immunogenic epitope," as used herein, is defined as a portion of a protein that elicits an antibody response in an animal, as determined by any method known in the art, for example, by the methods for generating antibodies described infra. (See, for example, Geysen et al., Proc. Natl. Acad. Sci. USA 81:3998- 4002 (1983)). The term "antigenic epitope," as used herein, is defined as a portion of a protein to which an antibody can immunospecifically bind its antigen as determined by any method well known in the art, for example, by the immunoassays described herein. Immunospecific binding excludes non-specific binding but does not necessarily exclude cross- reactivity with other antigens. Antigenic epitopes need not necessarily be immunogenic.

Fragments which function as epitopes may be produced by any conventional means. (See, e.g., Houghten, Proc. Natl. Acad. Sci. USA 82:5131-5135 (1985), further described in U.S. Patent No. 4,631,211).

In the present invention, antigenic epitopes preferably contain a sequence of at least 4, at least 5, at least 6, at least 7, more preferably at least 8, at least 9, at least 10, at least 11, at least 12, at least 13, at least 14, at least 15, at least 20, at least 25, at least.30, at least 40, at least 50, and, most preferably, between about 15 to about 30 amino acids. Preferred polypeptides comprising immunogenic or antigenic epitopes are at least 10, 15, 20, 25, 30, 35, 40, 45, 50*,* 55, 60, 65, 70, 75, 80, 85, 90, 95, or 100 amino acid residues in length. Additional non-exclusive preferred antigenic epitopes include the antigenic epitopes disclosed herein, as well as portions thereof. Antigenic epitopes are useful, for example, to raise antibodies, including monoclonal antibodies, that specifically bind the epitope. Preferred antigenic epitopes include the antigenic epitopes disclosed herein, as well as any combination of two, three, four, five or more of these antigenic epitopes. Antigenic epitopes can be used as the target molecules in immunoassays. (See, for instance, Wilson et al., Cell 37:767-778 (1984); Sutcliffe et al., Science 219:660-666 (1983)).

Similarly, immunogenic epitopes can be used, for example, to induce antibodies according to methods well known in the art. (See, for instance, Sutcliffe et al., supra; Wilson et al., supra; Chow et al., Proc. Natl. Acad. Sci. USA 82:910-914; and Bittle et al., J. Gen. Virol. 66:2347-2354 (1985). Preferred immunogenic epitopes include the immunogenic epitopes disclosed herein, as well as any combination of two, three, four, five or more of these immunogenic epitopes. The polypeptides comprising one or more immunogenic epitopes may be presented for eliciting an antibody response together with a carrier protein, such as an albumin, to an animal system (such as rabbit or mouse), or, if the polypeptide is of sufficient length (at least about 25 amino acids), the polypeptide may be presented without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting).

Epitope-bearing polypeptides of the present invention may be used to induce antibodies according to methods well known in the art including, but not limited to, in vivo immunization; in vitro immunization, and phage display methods. See, e.g., Sutcliffe et al., supra; Wilson et al., supra, and Bittle et al., J. Gen. Virol., 66:2347-2354 (1985). If in vivo immunization is used, animals may be immunized with free peptide; however, anti-peptide antibody titer may be boosted by coupling the peptide to a macromolecular carrier, such as keyhole limpet hemacyanin (KLH) or tetanus toxoid. For instance, peptides containing cysteine residues may be coupled to a carrier using a linker such as maleimidobenzoyl- N-hydroxysuccinimide ester (MBS), while other peptides may be coupled to carriers using a more general linking agent such as glutaraldehyde. Animals such as rabbits, rats and mice are immunized with either free or carrier- coupled peptides, for instance; by intraperitoneal and/or intradermal injection of emulsions containing about 100 µg of peptide or carrier protein and Freund's adjuvant or any other adjuvant known for stimulating an immune response. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

As one of skill in the art will appreciate, and as discussed above, the polypeptides of the present invention comprising an immunogenic or antigenic epitope can be fused to other polypeptide sequences. For example, the polypeptides of the present invention may be fused with the constant domain of immunoglobulins (IgA, IgE, IgG, IgM), or portions thereof (CH1, CH2, CH3, or any combination thereof and portions thereof), or albumin (including but not limited to recombinant albumin (see, e.g., U.S. Patent No. 5,876,969, issued March 2, 1999, EP Patent 0 413 622, and U.S. Patent No. 5,766,883, issued June 16, 1998, herein incorporated by reference in their entirety)), resulting in chimeric polypeptides. Such fusion proteins may facilitate purification and may increase half-life in vivo. This has been shown for chimeric proteins consisting of the first two domains of the human CD4-polypeptide and various domains of the constant regions of the heavy or light chains of mammalian immunoglobulins. See, e.g., EP 394,827; Traunecker et al., Nature, 331:84-86 (1988). Enhanced delivery of an antigen across the epithelial barrier to the immune system has been demonstrated for antigens (e.g., insulin) conjugated to an FcRn binding partner such as IgG or Fc fragments (see, e.g., PCT Publications WO 96/22024 and WO 99/04813). IgG Fusion proteins that have a disulfide-linked dimeric structure due to the IgG portion desulfide bonds have also been found to be more efficient in binding and neutralizing other molecules than monomeric polypeptides or fragments thereof alone. See, e.g., Fountoulakis et al., J. Biochem., 270:3958-3964 (1995). Nucleic acids encoding the above epitopes can also be recombined with a gene of interest as an epitope tag (e.g., the hemagglutinin ("HA") tag or flag tag) to aid in detection and purification of the expressed polypeptide. For example, a system described by Janknecht et al. allows for the ready purification of non-denatured fusion proteins expressed in human cell lines (Janknecht et al., 1991, Proc. Natl. Acad. Sci. USA 88:8972- 897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with the recombinant vaccinia virus are loaded onto Ni2+. nitriloacetic acid-agarose column and histidine-tagged proteins can be selectively eluted with imidazole-containing buffers.

Additional fusion proteins of the invention may be generated through the techniques of gene-shuffling, motif-shuffling, exon-shuffling, and/or codon-shuffling (collectively referred to as "DNA shuffling"). DNA shuffling may be employed to modulate the activities of polypeptides of the invention, such methods can be used to generate polypeptides with altered activity, as well as agonists and antagonists of the polypeptides. See, generally, U.S. Patent Nos. 5,605,793; 5,811,238; 5,830,721; 5,834,252; and 5,837,458, and Patten et al., Curr. Opinion Biotechnol. 8:724-33 (1997); Harayama, Trends Biotechnol. 16(2):76-82 (1998); Hansson, et al., J. Mol. Biol. 287:265-76 (1999); and Lorenzo and Blasco, Biotechniques 24(2):308- 13 (1998) (each of these patents and publications are hereby incorporated by reference in its entirety). In one embodiment, alteration of polynucleotides corresponding to SEQ ID NO:X and the polypeptides encoded by these polynucleotides may be achieved by DNA shuffling. DNA shuffling involves the assembly of two or more DNA segments by homologous or site-specific recombination to generate variation in the polynucleotide sequence. In another embodiment, polynucleotides of the invention, or the encoded polypeptides, may be altered by being subjected to random mutagenesis by error-prone PCR, random nucleotide insertion or other methods prior to recombination. In another embodiment, one or more components, motifs, sections, parts, domains, fragments, etc., of a polynucleotide encoding a polypeptide of the invention may be recombined with one or more components, motifs, sections, parts, domains, fragments, etc. of one or more heterologous molecules.

### Antibodies

Further polypeptides of the invention relate to antibodies and T-cell antigen receptors (TCR) which immunospecifically bind a polypeptide, polypeptide fragment, or variant of SEQ ID NO:Y, and/or an epitope, of the present invention (as determined by immunoassays well known in the art for assaying specific antibody-antigen binding). Antibodies of the invention include, but are not limited to, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules of the invention can be of any type (e.g., IgG, IgE, IgM, IgD, IgA and IgY), class (e.g., IgG1, IgG2, IgG3, IgG4, IgA1 and IgA2) or subclass of immunoglobulin molecule. In preferred embodiments, the immunoglobulin molecules of the invention are IgG1. In other preferred embodiments, the immunoglobulin molecules of the invention are IgG4.

Most preferably the antibodies are human antigen-binding antybody fragments of the present invention and include, but are not limited to, Fab, Fab' and F(ab')2, Fd, single-chain Fvs (scFv), single-chain antibodies, disulfide-linked Fvs (sdFv) and fragments comprising either a VL or VH domain. Antigen-binding antibody fragments, including single-chain antibodies, may comprise the variable region(s) alone or in combination with the entirety or a portion of the following: hinge region, CH1, CH2, and CH3 domains. Also included in the invention are antigen-binding fragments also comprising any combination of variable region(s) with a hinge region, CH1, CH2, and CH3 domains. The antibodies of the invention may be from any animal origin including birds and mammals. Preferably, the antibodies are human, murine (e.g., mouse and rat), donkey, ship rabbit, goat, guinea pig, camel, horse, or chicken. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example in, U.S. Patent No. 5,939,598 by Kucherlapati et al.

The antibodies of the present invention may be monospecific, bispecific, trispecific or of greater multi specificity. Multispecific antibodies may be specific for different epitopes of a polypeptide of the present invention or may be specific for both a polypeptide of the present invention as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Patent Nos. 4,474,893; 4,714,681; 4,925,648; 5,573,920; 5,601,819; Kostelny et al., J. Immunol. 148:1547-1553 (1992).

Antibodies of the present invention may be described or specified in terms of the epitope(s) or portion(s) of a polypeptide of the present invention which they recognize or specifically bind. The epitope(s) or polypeptide portion(s) may be specified as described herein, e.g., by N-terminal and C-terminal positions, by size in contiguous amino acid residues, or listed in the Tables and Figures. Antibodies which specifically bind any epitope or polypeptide of the present invention may also be excluded. Therefore, the present invention includes antibodies that specifically bind polypeptides of the present invention, and allows for the exclusion of the same.

Antibodies of the present invention may also be described or specified in terms of their cross-reactivity. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide of the present invention are also included in the present invention. In specific embodiments, antibodies of the present invention cross-react with murine, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide of the present invention are also included in the present invention. In a specific embodiment, the above-described cross-reactivity is with respect to any single specific antigenic or immunogenic polypeptide, or combination(s) of 2, 3, 4, 5, or more of the specific antigenic and/or immunogenic polypeptides disclosed herein. Further included in the present invention are antibodies which bind polypeptides encoded by polynucleotides which hybridize to a polynucleotide of the present invention under stringent hybridization conditions (as described herein). Antibodies of the present invention may also be described or specified in terms of their binding affinity to a polypeptide of the invention. Preferred binding affinities include those with a dissociation constant or Kd less than 5 X 10⁻² M, 10⁻² M, 5 X 10⁻³ M, 10⁻³ M, 5 X 10⁻⁴ M, 10⁻⁴ M, 5 X 10⁻⁵ M, 10⁻⁵ M, 5 X 10⁻⁶ M, 10⁻⁶M, 5 X 10⁻⁷ M, 10⁷ M, 5 X 10⁻⁸ M, 10⁻⁸ M, 5 X 10⁻⁹ M, 10⁻⁹ M, 5 X 10⁻¹⁰ M, 10⁻¹⁰ M, 5 X 10⁻¹¹ M, 10⁻¹¹ M, 5 X 10⁻¹²M, ¹⁰⁻¹² M, 5 X 10⁻¹¹ M, 10⁻¹³ M, 5 X 10⁻¹⁴ M, 10⁻¹⁴ M, 5 X 10⁻¹⁵ M, or 10⁻¹⁵M.

The invention also provides antibodies that competitively inhibit binding of an antibody to an epitope of the invention as determined by any method known in the art for determining competitive binding, for example, the immunoassays described herein. In preferred embodiments, the antibody competitively inhibits binding to the epitope by at least 95%, at least 90%, at least 85 %, at least 80%, at least 75%, at least 70%, at least 60%, or at least 50%.

Antibodies of the present invention may act as agonists or antagonists of the polypeptides of the present invention. For example, the present invention includes antibodies which disrupt the receptor/ligand interactions with the polypeptides of the invention either partially or fully. Preferrably, antibodies of the present invention bind an antigenic epitope disclosed herein, or a portion thereof. The invention features both receptor-specific antibodies and ligand-specific antibodies. The invention also features receptor-specific antibodies which do not prevent ligand binding but prevent receptor activation. Receptor activation (i.e., signaling) may be determined by techniques described herein or otherwise known in the art. For example, receptor activation can be determined by detecting the phosphorylation (e.g., tyrosine or serine/threonine) of the receptor or its substrate by immunoprecipitation followed by western blot analysis (for example, as described supra). In specific embodiments, antibodies are provided that inhibit ligand activity or receptor activity by at least 95%, at least 90%, at least 85%, at least 80%, at least 75%, at least 70%, at least 60%, or at least 50% of the activity in absence of the antibody.

The invention also features receptor-specific antibodies which both prevent ligand binding and receptor activation as well as antibodies that recognize the receptor-ligand complex, and, preferably, do not specifically recognize the unbound receptor or the unbound ligand. Likewise, included in the invention are neutralizing antibodies which bind the ligand and prevent binding of the ligand to the receptor, as well as antibodies which bind the ligand, thereby preventing receptor activation, but do not prevent the ligand from binding the receptor. Further included in the invention are antibodies which activate the receptor. These antibodies may act as receptor agonists, i.e., potentiate or activate either all or a subset of the biological activities of the ligand-mediated receptor activation, for example, by inducing dimerization of the receptor. The antibodies may be specified as agonists, antagonists or inverse agonists for biological activities comprising the specific biological activities of the peptides of the invention disclosed herein. The above antibody agonists can be made using methods known in the art. See, e.g., PCT publication WO 96/40281; U.S. Patent No. 5,811,097; Deng et al., Blood 92(6):1981-1988 (1998); Chen et al., Cancer Res. 58(16):3668-3678 (1998); Harrop et al., J. Immunol. 161(4):1786-1794 (1998); Zhu et al., Cancer Res. 58(15):3209-3214 (1998); Yoon et al., J. Immunol. 160(7):3170-3179 (1998); Prat et al., J. Cell. Sci. 11 1(Pt2):237-247 (1998); Pitard et al., J. Immunol. Methods 205(2):177-190 (1997); Liautard et al., Cytokine 9(4):233-241 (1997); Carlson et al., J. Biol. Chem. 272(17):11295-11301 (1997); Taryman et al., Neuron 14(4):755-762 (1995); Muller et al., Structure 6(9):1153-1167 (1998); Bartunek et al., Cytokine 8(1):14-20 (1996) (which are all incorporated by reference herein in their entireties).

Antibodies of the present invention may be used, for example, but not limited to, to purify, detect, and target the polypeptides of the present invention, including both in vitro and in vivo diagnostic and therapeutic methods. For example, the antibodies have use in immunoassays for qualitatively and quantitatively measuring levels of the polypeptides of the present invention in biological samples. See, e.g., Harlow et al., Antibodies: A Laboratory Manual, (Cold Spring Harbor Laboratory Press, 2nd ed. 1988) (incorporated by reference herein in its entirety).

As discussed in more detail below, the antibodies of the present invention may be used either alone or in combination with other compositions. The antibodies may further be recombinantly fused to a heterologous polypeptide at the N- or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs, radionuclides, or toxins. See, e.g., PCT publications WO 92/08495; WO 91/14438; WO 89/12624; U.S. Patent No. 5,314,995; and EP 396,387.

The antibodies of the invention include derivatives that are modified, i.e, by the covalent attachment of any type of molecule to the antibody such that covalent attachment does not prevent the antibody from generating an anti-idiotypic response. For example, but not by way of limitation, the antibody derivatives include antibodies that have been modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

The antibodies of the present invention may be generated by any suitable method known in the art. Polyclonal antibodies to an antigen-of- interest can be produced by various procedures well known in the art. For example, a polypeptide of the invention can be administered to various host animals including, but not limited to, rabbits, mice, rats, etc. to induce the production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art and are discussed in detail in the Examples (e.g., Example 16). In a non-limiting example, mice can be immunized with a polypeptide of the invention or a cell expressing such peptide. Once an immune response is detected, e.g., antibodies specific for the antigen are detected in the mouse serum, the mouse spleen is harvested and splenocytes isolated. The splenocytes are then fused by weii known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, the present invention provides methods of generating monoclonal antibodies as well as antibodies produced by the method comprising culturing a hybridoma cell secreting an antibody of the invention wherein, preferably, the hybridoma is generated by fusing splenocytes isolated from a mouse immunized with an antigen of the invention with myeloma cells and then screening the hybridomas resulting from the fusion for hybridoma clones that secrete an antibody able to bind a polypeptide of the invention.

Antibody fragments which recognize specific epitopes may be generated by known techniques. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain.

For example, the antibodies of the present invention can also be generated using various phage display methods known in the art. In phage display methods, functional antibody domains are displayed on the surface of phage particles which carry the polynucleotide sequences encoding them. In a particular embodiment, such phage can be utilized to display antigen binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M 13 binding domains expressed from phage with Fab, Fv or disulfide stabilized Fv antibody domains recombinantly fused to either the phage gene in or gene VIII protein. Examples of phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988) (said references incorporated by reference in their entireties).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988). For some uses, including in vivo use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. See e.g., Morrison, Science 229:1202 (1985); Oi et al., BioTechniques 4:214 (1986); Gillies et al., (1989) J. Immunol. Methods 125:191-202; U.S. Patent Nos. 5,807,715; 4,816,567; and 4,816397, which are incorporated herein by reference in their entirety. Humanized antibodies are antibody molecules from non-human species antibody that binds the desired antigen having one or more complementarity determining regions (CDRs) from the non-human species and a framework regions from a human immunoglobulin molecule. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. (See, e.g., Queen et al., U.S. Patent No. 5,585,089; Riechmann et al., Nature 332:323 (1988), which are incorporated herein by reference in their entireties.) Antibodies can be humanized using a variety of techniques known in the art including, for example, CDR-grafting (EP 239,400; PCT publication WO 91/09967; U.S. Patent Nos. 5,225,539; 5,530,101; and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Patent No. 5,565,332).

Completely human antibodies are particularly desirable for therapeutic treatment of human patients. Human antibodies can be made by a variety of methods known in the art including phage display methods described'above using antibody libraries derived from human immunoglobulin sequences. See also, U.S. Patent Nos. 4,444,887 and 4,716,111; and PCT publications WO 98/46645, WO 98/50433, WO 98/24893, WO 98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, e.g., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar, Int. Rev. Immunol. 13:65-93 (1995). For a detailed discussion of this technology for producing human antibodies and human monoclonal antibodies and protocols for producing such antibodies, see, e.g., PCT publications WO 98/24893; WO 92/01047; WO 96/34096; WO 96/33735; European Patent No. 0 598 877; U.S. Patent Nos. 5,413,923; 5,625,126; 5,633,425; 5,569,825; 5,661,016; 5,545,806; 5,814,318; 5,885,793; 5,916,771; and 5,939,598, which are incorporated by reference herein in their entirety. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology 12:899-903 (1988)).

Further, antibodies to the polypeptides of the invention can, in turn, be utilized to generate anti-idiotype antibodies that "mimic" polypeptides of the invention using techniques well known to those skilled in the art. (See, e.g., Greenspan & Bona, FASEB J. 7(5):437-444; (1989) and Nissinoff, J. Immunol. 147(8):2429-2438 (1991)). For example, antibodies which bind to and competitively inhibit polypeptide multimerization and/or binding of a polypeptide of the invention to a ligand can be used to generate anti-idiotypes that "mimic" the polypeptide multimerization and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide of the invention and/or to bind its ligands/receptors, and thereby block its biological activity.

### Polynucleotides Encoding Antibodies

The invention further provides polynucleotides comprising a nucleotide sequence encoding an antibody of the invention and fragments thereof. The invention also encompasses polynucleotides that hybridize under stringent or lower stringency hybridization conditions, e.g., as defined supra, to polynucleotides that encode an antibody, preferably, that specifically binds to a polypeptide of the invention, preferably, an antibody that binds to a polypeptide having the amino acid sequence of SEQ ID NO:Y.

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (e.g., an antibody cDNA library, or a cDNA library generated from, or nucleic acid, preferably poly A+ RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties ), to generate antibodies having a different amino acid sequence, for example to create amino acid substitutions, deletions, and/or insertions.

In a specific embodiment, the amino acid sequence of the heavy and/or light chain variable domains may be inspected to identify the sequences of the complementarity determining regions (CDRs) by methods that are well know in the art, e.g., by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody, as described supra. The framework regions may be naturally occurring or consensus framework regions, and preferably human framework regions (see, e.g., Chothia et al., J. Mol. Biol. 278: 457-479 (1998) for a listing of human framework regions). Preferably, the polynucleotide generated by the combination of the framework regions and CDRs encodes an antibody that specifically binds a polypeptide of the invention. Preferably, as discussed supra, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human immunoglobulin constant region, e.g., humanized antibodies.

Alternatively, techniques described for the production of single chain antibodies (U.S. Patent No. 4,946,778; Bird, Science 242:423- 42 (1988); Huston et al., Proc. Natl. Acad. Sci. USA 85:5879-5883 (1988); and Ward et al., Nature 334:544-54 (1989)) can be adapted to produce single chain antibodies. Single chain antibodies are formed by linking the heavy and light chain fragments of the Fv region via an amino acid bridge, resulting in a single chain polypeptide. Techniques for the assembly of functional Fv fragments in E. coli may also be used (Skerra et al,. Science 242:1038-1041 (1988)).

### Methods of Producing Antibodies

The antibodies of the invention can be produced by any method known in the art for the synthesis of antibodies, in particular, by chemical synthesis or preferably, by recombinant expression techniques.

Recombinant expression of an antibody of the invention, or fragment, derivative or analog thereof, (e.g., a heavy or light chain of an antibody of the invention or a single chain antibody of the invention), requires construction of an expression vector containing a polynucleotide that encodes the antibody. Once a polynucleotide encoding an antibody molecule or a heavy or light chain of an antibody, or portion thereof (preferably containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. The invention, thus, provides replicable vectors comprising a nucleotide sequence encoding an antibody molecule of the invention, or a heavy or light chain thereof, or a heavy or light chain variable domain, operably linked to a promoter. Such vectors may include the nucleotide sequence encoding the constant region of the antibody molecule (see, e.g., PCT Publication WO 86/05807; PCT Publication WO 89/01036; and U.S. Patent No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody of the invention. Thus, the invention includes host cells containing a polynucleotide encoding an antibody of the invention, or a heavy or light chain thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention. Such host-expression systems represent vehicles by which the coding sequences of interest may be produced and subsequently purified, but also represent cells which may, when transformed or transfected with the appropriate nucleotide coding sequences, express an antibody molecule of the invention in situ. These include but are not limited to microorganisms such as bacteria (e.g., E. coli, B. subtilis) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Preferably, bacterial cells such as Escherichia coli, and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., EMBO J. 2:1791 (1983)), in which the antibody coding sequence may be ligated individually into the vector in frame with the lac Z coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non- essential region of the viral genome (e.g., region E1 or E3) will result in a recombinant virus that is viable and capable of expressing the antibody molecule in infected hosts. (e.g., see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., Methods in Enzymol. 153:51-544 (1987)).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, WI38, and in particular, breast cancer cell lines such as, for example, BT483, Hs578T, HTB2, BT20 and T47D, and normal mammary gland cell line such as, for example, CRL7030 and Hs578Bst.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule: Such engineered cell lines may be particularly useful in screening and evaluation of compounds that interact directly or indirectly with the antibody molecule.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 (1980)) genes can be employed in tk-, hgprt- or aprt- cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, 1993, TIB TECH 11(5):155-215); and hygro, which confers resistance to hygromycin (Santerre et al., Gene 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981), which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol. 3:257 (1983)).

The host cell may be co-transfected with two expression vectors of the invention, the first vector encoding a heavy chain derived polypeptide and the second vector encoding a light chain derived polypeptide: The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

Once an antibody molecule of the invention has been produced by an animal, chemically synthesized, or recombinantly expressed, it may be purified by any method known in the art for purification of an immunoglobulin molecule, for example, by chromatography (e.g., ion exchange, affinity, particularly by affinity for the specific antigen after Protein A, and sizing column chromatography), centrifugation, differential solubility, or by any other standard technique for the purification of proteins. In addition, the antibodies of the present invention or fragments thereof can be fused to heterologous polypeptide sequences described herein or otherwise known in the art, to facilitate purification.

The present invention encompasses antibodies recombinantly fused or chemically conjugated (including both covalently and non-covalently conjugations) to a polypeptide (or portion thereof, preferably at least 10, 20, 30, 40, 50, 60, 70, 80, 90 or 100 amino acids of the polypeptide) of the present invention to generate fusion proteins. The fusion does not necessarily need to be direct, but may occur through linker sequences. The antibodies may be specific for antigens other than polypeptides (or portion thereof, preferably at least 10, 20, 30, 40, 50, 60, 70, 80, 90 or 100 amino acids of the polypeptide) of the present invention. For example, antibodies may be used to target the polypeptides of the present invention to particular cell types, either in vitro or in vivo, by fusing or conjugating the polypeptides of the present invention to antibodies specific for particular cell surface receptors. Antibodies fused or conjugated to the polypeptides of the present invention may also be used in in vitro immunoassays and purification methods using methods known in the art. See e.g., Harbor et al., supra, and PCT publication WO 93/21232; EP 439,095; Naramura et al., Immunol. Lett. 39:91-99 (1994); U.S: Patent 5,474,981; Gillies et al., PNAS 89:1428-1432 (1992); Fell et al., J. Immunol. 146:2446-2452(1991), which are incorporated by reference in their entireties.

The present invention further includes compositions comprising the polypeptides of the present invention fused or conjugated to antibody domains other than the variable regions. For example, the polypeptides of the present invention may be fused or conjugated to an antibody Fc region, or portion thereof. The antibody portion fused to a polypeptide of the present invention may comprise the constant region, hinge region, CH1 domain, CH2 domain, and CH3 domain or any combination of whole domains or portions thereof. The polypeptides may also be fused or conjugated to the above antibody portions to form multimers. For example, Fc portions fused to the polypeptides of the present invention can form dimers through disulfide bonding between the Fc portions. Higher multimeric forms can be made by fusing the polypeptides to portions of IgA and IgM. Methods for fusing or conjugating the polypeptides of the present invention to antibody portions are known in the art. See, e.g., U.S. Patent Nos. 5,336,603; 5,622,929; 5,359,046; 5,349,053; 5,447,851; 5,112,946; EP 307,434; EP 367,166; PCT publications WO 96/04388; WO 91/06570; Ashkenazi et al., Proc. Natl. Acad. Sci. USA 88:10535-10539 (1991); Zheng et al., J. Immunol. 154:5590-5600 (1995); and Vil et al., Proc. Natl. Acad. Sci. USA 89:11337- 11341(1992) (said references incorporated by reference in their entireties).

As discussed, supra, the polypeptides corresponding to a polypeptide, polypeptide fragment, or a variant of SEQ ID NO:Y may be fused or conjugated to the above antibody portions to increase the in vivo half life of the polypeptides or for use in immunoassays using methods known in the art. Further, the polypeptides corresponding to SEQ ID NO:Y may be fused or conjugated to the above antibody portions to facilitate purification. One reported example describes chimeric proteins consisting of the first two domains of the human CD4-polypeptide and various domains of the constant regions of the heavy or light chains of mammalian immunoglobulins. (EP 394,827; Traunecker et al., Nature 331:84-86 (1988). The polypeptides of the present invention fused or conjugated to an antibody having disulfide- linked dimeric structures (due to the IgG) may also be more efficient in binding and neutralizing other molecules, than the monomeric secreted protein or protein fragment alone. (Fountoulakis et al., J. Biochem. 270:3958-3964 (1995)). In many cases, the Fc part in a fusion protein is beneficial in therapy and diagnosis, and thus can result in, for example, improved pharmacokinetic properties. (EP A 232,262). Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, Bennett et al., J. Molecular Recognition 8:52-58 (1995); Johanson et al., J. Biol. Chem. 270:9459-9471 (1995).

Moreover, the antibodies or fragments thereof of the present invention can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37:767 (1984)) and the "flag" tag.

The present invention further encompasses antibodies or fragments thereof conjugated to a diagnostic or therapeutic agent. The antibodies can be used diagnostically to, for example, monitor the development or progression of a tumor as part of a clinical testing procedure to, e.g., determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody (or fragment thereof) or indirectly, through an intermediate (such as, for example, a linker known in the art) using techniques known in the art. See, for example, U.S. Patent No. 4,741,900 for metal ions which can be conjugated to antibodies for use as diagnostics according to the present invention. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include 125I, 131I, 111In or 99Tc.

Further, an antibody or fragment thereof may be conjugated to a therapeutic moiety such as a cytotoxin, e.g., a cytostatic or cytocidal agent, a therapeutic agent or a radioactive metal ion, e.g., alpha-emitters such as, for example, 213Bi. A cytotoxin or cytotoxic agent includes any agent that is detrimental to cells. Examples include paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The conjugates of the invention can be used for modifying a given biological response, the therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, a-interferon, ß-interferon, nerve growth factor, platelet derived growth factor, tissue plasminogen activator, an apoptotic agent, e.g., TNF-alpha, TNF-beta, AIM I (See, International Publication No. WO 97/33899), AIM II (See, International Publication No. WO 97/34911), Fas Ligand (Takahashi *et al., Int. Immunol.,* 6:1567-1574 (1994)), VEGI (See, International Publication No. WO 99/23105), a thrombotic agent or an anti- angiogenic agent, e.g., angiostatin or endostatin; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

Techniques for conjugating such therapeutic moiety to antibodies are well known, see, e.g., Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev. 62:119-58 (1982).

Alternatively, an antibody can be conjugated to a second antibody to form an antibody heteroconjugate as described by Segal in U.S. Patent No. 4,676,980, which is incorporated herein by reference in its entirety.

An antibody, with or without a therapeutic moiety conjugated to it, administered alone or in combination with cytotoxic factor(s) and/or cytokine(s) can be used as a therapeutic.

### Immunophenotyping

The antibodies of the invention may be utilized for immunophenotyping of cell lines and biological samples. The translation product of the gene of the present invention may be useful as a cell specific marker, or more specifically as a cellular marker that is differentially expressed at various stages of differentiation and/or maturation of particular cell types. Monoclonal antibodies directed against a specific epitope, or combination of epitopes, will allow for the screening of cellular populations expressing the marker. Various techniques can be utilized using monoclonal antibodies to screen for cellular populations expressing the marker(s), and include magnetic separation using antibody-coated magnetic beads, "panning" with antibody attached to a solid matrix (i.e., plate), and flow cytometry (See, e.g., U.S. Patent 5,985,660; and Morrison *et al., Cell,* 96:737-49 (1999)).

These techniques allow for the screening of particular populations of cells, such as might be found with hematological malignancies (i.e. minimal residual disease (MRD) in acute leukemic patients) and "non-self" cells in transplantations to prevent Graft-versus-Host Disease (GVHD). Alternatively, these techniques allow for the screening of hematopoietic stem and progenitor cells capable of undergoing proliferation and/or differentiation, as might be found in human umbilical cord blood.

### Assays For Antibody Binding

The antibodies of the invention may be assayed for immunospecific binding by any method known in the art. The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer such as RIPA buffer (1% NP-40 or Triton X- 100, 1% sodium deoxycholate, 0.1% SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4° C, adding protein A and/or protein G sepharose beads to the cell lysate, incubating for about an hour or more at 4° C, washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with sepharose beads). For further discussion regarding immunoprecipitation protocols see, e.g., Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (e.g., 8%- 20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction can be determined by competitive binding assays. One example of a competitive binding assay is a radioimmunoassay comprising the incubation of labeled antigen (e.g., 3H or 125I) with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound (e.g., 3H or 125I) in the presence of increasing amounts of an unlabeled second antibody.

### Therapeutic Uses

The present invention is further directed to antibody-based therapies which involve administering antibodies of the invention to an animal, preferably a mammal, and most preferably a human, patient for treating one or more of the disclosed diseases, disorders, or conditions. Therapeutic compounds of the invention include, but are not limited to, antibodies of the invention (including fragments, analogs and derivatives thereof as described herein) and nucleic acids encoding antibodies of the invention (including fragments, analogs and derivatives thereof and anti-idiotypic antibodies as described herein). The antibodies of the invention can be used to treat, inhibit or prevent diseases, disorders or conditions associated with aberrant expression and/or activity of a polypeptide of the invention, including, but not limited to, any one or more of the diseases, disorders, or conditions described herein. The treatment and/or prevention of diseases, disorders, or conditions associated with aberrant expression and/or activity of a polypeptide of the invention includes, but is not limited to, alleviating symptoms associated with those diseases, disorders or conditions. Antibodies of the invention may be provided in pharmaceutically acceptable compositions as known in the art or as described herein.

A summary of the ways in which the antibodies of the present invention may be used therapeutically includes binding polynucleotides or polypeptides of the present invention locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies of the present invention for diagnostic, monitoring or therapeutic purposes without undue experimentation.

The antibodies of this invention may be advantageously utilized in combination with other monoclonal or chimeric antibodies, or with lymphokines or hematopoietic growth factors (such as, e.g., IL-2, IL-3 and IL-7), for example, which serve to increase the number or activity of effector cells- which interact with the antibodies.

The antibodies of the invention may be administered alone or in combination with other types of treatments (e.g., radiation therapy, chemotherapy, hormonal therapy, immunotherapy and anti-tumor agents). Generally, administration of products of a species origin or species reactivity (in the case of antibodies) that is the same species as that of the patient is preferred. Thus, in a preferred embodiment, human antibodies, fragments derivatives, analogs, or nucleic acids, are administered to a human patient for therapy or prophylaxis.

It is preferred to use high affinity and/or potent in vivo inhibiting and/or neutralizing antibodies against polypeptides or polynucleotides of the present invention, fragments or regions thereof, for both immunoassays directed to and therapy of disorders related to polynucleotides or polypeptides, including fragments thereof, of the present invention. Such antibodies, fragments; or regions, will preferably have an affinity for polynucleotides or polypeptides of the invention, including fragments thereof. Preferred binding affinities include those with a dissociation constant or Kd less than 5 X 10⁻² M, 10⁻² M, 5 X 10⁻³ M, 10⁻³ M, 5 X 10⁻⁴ M, 10⁻⁴ M, 5 X 10⁻⁵ M, 10⁻⁵ M, 5 X 10⁻⁶ M, 10⁻⁶ M, 5 X 10⁻⁷ M, 10⁻⁷ M, 5 X 10⁻⁸ M, 10⁻⁸ M, 5 X 10⁻⁹ M, 10⁻⁹ M, 5 X 10⁻¹⁰ M, 10⁻¹⁰ M, 5 X 10⁻¹¹M, 10⁻¹¹M, 5 X 10⁻¹² M, 10⁻¹² M, 5 X 10⁻¹³ M, 10⁻¹³ M, 5 X 10⁻¹⁴ M, 10⁻¹⁴ M, 5 X 10⁻¹⁵ M, and 10⁻¹⁵ M.

### Gene Therapy

In a specific embodiment, nucleic acids comprising sequences encoding antibodies or functional derivatives thereof, are administered to treat, inhibit or prevent a disease or disorder associated with aberrant expression and/or activity of a polypeptide of the invention, by way of gene therapy. Gene therapy refers to therapy performed by the administration to a subject of an expressed or expressible nucleic acid. In this embodiment of the invention, the nucleic acids produce their encoded protein that mediates a therapeutic effect:

Any of the methods for gene therapy available in the art can be used according to the present invention. Exemplary methods are described below.

For general reviews of the methods of gene therapy, see Goldspiel et al., Clinical Pharmacy 12:488-505 (1993); Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, TIBTECH 11(5):155-215 (1993). Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In a preferred aspect, the compound comprises nucleic acid sequences encoding an antibody, said nucleic acid sequences being part of expression vectors that express the antibody or fragments or chimeric proteins or heavy or light chains thereof in a suitable host. In particular, such nucleic acid sequences have promoters operably linked to the antibody coding region, said promoter being inducible or constitutive, and, optionally, tissue- specific. In another particular embodiment, nucleic acid molecules are used in which the antibody coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA 86:8932-8935 (1989); Zijlstra et al., Nature 342:435-438 (1989). In specific embodiments, the expressed antibody molecule is a single chain antibody; alternatively, the nucleic acid sequences include sequences encoding both the heavy and light chains, or fragments thereof, of the antibody.

Delivery of the nucleic acids into a patient may be either direct, in which case the patient is directly exposed to the nucleic acid or nucleic acid- carrying vectors, or indirect, in which case, cells are first transformed with the nucleic acids in vitro, then transplanted into the patient. These two approaches are known, respectively, as in vivo or ex vivo gene therapy.

In a specific embodiment, the nucleic acid sequences are directly administered in vivo, where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retrovirals or other viral vectors (see U.S. Patent No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem. 262:4429-4432 (1987)) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180; WO 92/22635; WO92/20316; WO93/14188, WO 93/20221). Alternatively, the nucleic acid can be introduced intracellularly and incorporated within host cell DNA for expression, by homologous recombination (Koller and Smithies, Proc. Natl. Acad. Sci. USA 86:8932-8935 (1989); Zijlstra et al., Nature 342:435-438 (1989)).

In a specific embodiment, viral vectors that contains nucleic acid sequences encoding an antibody of the invention are used. For example, a retroviral vector can be used (see Miller et al., Meth. Enzymol. 217:581-599 (1993)). These retroviral vectors contain the components necessary for the correct packaging of the viral genome and integration into the host cell DNA. The nucleic acid sequences encoding the antibody to be used in gene therapy are cloned into one or more vectors, which facilitates delivery of the gene into a patient. More detail about retroviral vectors can be found in Boesen et al., Biotherapy 6:291-302 (1994), which describes the use of a retroviral vector to deliver the mdr1 gene to hematopoietic stem cells in order to make the stem cells more resistant to chemotherapy. Other references illustrating the use of retroviral vectors in gene therapy are: Clowes et al., J. Clin. Invest. 93:644-651 (1994); Kiem et al., Blood 83:1467-1473 (1994); Salmons and Gunzberg, Human Gene Therapy 4:129-141 (1993); and Grossman and Wilson, Curr. Opin. in Genetics and Devel. 3:110-114 (1993).

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, Current Opinion in Genetics and Development 3:499-503 (1993) present a review of adenovirus-based gene therapy. Bout et al., Human Gene Therapy 5:3-10 (1994) demonstrated the use of adenovirus vectors to transfer genes to the respiratory epithelia of rhesus monkeys. Other instances of the use of adenoviruses in gene therapy can be found in Rosenfeld et al., Science 252:431-434 (1991); Rosenfeld et al., Cell 68:143- 155 (1992); Mastrangeli et al., J. Clin. Invest. 91:225-234 (1993); PCT Publication WO94/12649; and Wang, et al., Gene Therapy 2:775-783 (1995). In a preferred embodiment, adenovirus vectors are used.

Adeno-associated virus (AAV) has also been proposed for use in gene therapy (Walsh et al., Proc. Soc. Exp. Biol. Med. 204:289-300 (1993); U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler and Behr, Meth. Enzymol. 217:599-618 (1993); Cohen et al., Meth. Enzymol. 217:618-644 (1993); Cline, Pharmac. Ther. 29:69-92m (1985) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a patient by various methods known in the art. Recombinant blood cells (e.g., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as Tlymphocytes, Blymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

In a preferred embodiment, the cell used for gene therapy is autologous to the patient.

In an embodiment in which recombinant cells are used in gene therapy, nucleic acid sequences encoding an antibody are introduced into the cells such that they are expressible by the cells or their progeny, and the recombinant cells are then administered in vivo for therapeutic effect. In a specific embodiment, stem or progenitor cells are used. Any stem and/or progenitor cells which can be isolated and maintained in vitro can potentially be used in accordance with this embodiment of the present invention (see e.g. PCT Publication WO 94/08598; Stemple and Anderson, Cell 71:973-985 (1992); Rheinwald, Meth. Cell Bio. 21A:229 (1980); and Pittelkow and Scott, Mayo Clinic Proc. 61:771 (1986)).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

### Demonstration of Therapeutic or Prophylactic Activity

The compounds or pharmaceutical compositions of the invention are preferably tested in vitro, and then in vivo for the desired therapeutic or prophylactic activity, prior to use in humans. For example, in vitro assays to demonstrate the therapeutic or prophylactic utility of a compound or pharmaceutical composition include, the effect of a compound on a cell line or a patient tissue sample. The effect of the compound or composition on the cell line and/or tissue sample can be determined utilizing techniques known to those of skill in the art including, but not limited to, rosette formation assays and cell lysis assays. In accordance with the invention, in vitro assays which can be used to determine whether administration of a specific compound is indicated, include in vitro cell culture assays in which a patient tissue sample is grown in culture, and exposed to or otherwise administered a compound, and the effect of such compound upon the tissue sample is observed.

### Therapeutic/Prophylactic Administration and Composition

The invention provides methods of treatment, inhibition and prophylaxis by administration to a subject of an effective amount of a compound or pharmaceutical composition of the invention, preferably an antibody of the invention. In a preferred aspect, the compound is substantially purified (e.g., substantially free from substances that limit its effect or produce undesired side-effects). The subject is preferably an animal, including but not limited to animals such as cows, pigs, horses, chickens, cats, dogs, etc., and is preferably a mammal, and most preferably human.

Formulations and methods of administration that can be employed when the compound comprises a nucleic acid or an immunoglobulin are described above; additional appropriate formulations and routes of administration can be selected from among those described herein below.

Various delivery systems are known and can be used to administer a compound of the invention, e.g., encapsulation in liposomes, microparticles, microcapsules, recombinant cells capable of expressing the compound, receptor-mediated endocytosis (see, e.g., Wu and Wu, J. Biol. Chem. 262:4429-4432 (1987)), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the invention, care must be taken to use materials to which the protein does not absorb.

In another embodiment, the compound or composition can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353- 365 (1989); Lopez-Berestein, ibid., pp. 317-327; see generally ibid.)

In yet another embodiment, the compound or composition can be delivered in a controlled release system. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., Macromol. Sci. Rev. Macromol. Chem. 23:61 (1983); see also Levy et al., Science 228:190 (1985); During et al., Ann. Neurol. 25:351 (1989); Howard et al., J.Neurosurg. 71:105 (1989)). In yet another embodiment, a controlled release system can be placed in proximity of the therapeutic target, i.e., the brain, thus requiring only a fraction of the systemic dose (see, e.g., Goodson, in Medical Applications of Controlled Release, supra, vol. 2, pp. 115-138 (1984)).

Other controlled release systems are discussed in the review by Langer (Science 249:1527-1533 (1990)).

In a specific embodiment where the compound of the invention is a nucleic acid encoding a protein, the nucleic acid can be administered in vivo to promote expression of its encoded protein, by constructing it as part of an appropriate nucleic acid expression vector and administering it so that it becomes intracellular, e.g., by use of a retroviral vector (see U.S. Patent No. 4,980,286), or by direct injection, or by use of microparticle bombardment (e.g., a gene gun; Biolistic, Dupont), or coating with lipids or cell-surface receptors or transfecting agents, or by administering it in linkage to a homeobox- like peptide which is known to enter the nucleus (see e.g., Joliot et al., Proc. Natl. Acad. Sci. USA 88:1864-1868 (1991)), etc. Alternatively, a nucleic acid can be introduced intracellularly and incorporated within host cell DNA for expression, by homologous recombination.

The present invention also provides pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The amount of the compound of the invention which will be effective in the treatment, inhibition and prevention of a disease or disorder associated with aberrant expression and/or activity of a polypeptide of the invention can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems.

For antibodies, the dosage administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention may be reduced by enhancing uptake and tissue penetration (e.g., into the brain) of the antibodies by modifications such as, for example, lipidation.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### Diagnosis and Imaging

Labeled antibodies, and derivatives and analogs thereof, which specifically bind to a polypeptide of interest can be used for diagnostic purposes to detect, diagnose, or monitor diseases, disorders, and/or conditions associated with the aberrant expression and/or activity of a polypeptide of the invention. The invention provides for the detection of aberrant expression of a polypeptide of interest, comprising (a) assaying the expression of the polypeptide of interest in cells or body fluid of an individual using one or more antibodies specific to the polypeptide interest and (b) comparing the level of gene expression with a standard gene expression level, whereby an increase or decrease in the assayed polypeptide gene expression level compared to the standard expression level is indicative of aberrant expression.

The invention provides a diagnostic assay for diagnosing a disorder, comprising (a) assaying the expression of the polypeptide of interest in cells or body fluid of an individual using one or more antibodies specific to the polypeptide interest and (b) comparing the level of gene expression with a standard gene expression level, whereby an increase or decrease in the assayed polypeptide gene expression level compared to the standard expression level is indicative of a particular disorder. With respect to cancer, the presence of a relatively high amount of transcript in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Antibodies of the invention can be used to assay protein levels in a biological sample using classical immunohistological methods known to those of skill in the art (e.g., see Jalkanen, et al., J. Cell. Biol. 101:976-985 (1985); Jalkanen, et al., J. Cell. Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase; radioisotopes, such as iodine (125I, 121I), carbon (14C), sulfur (35S), tritium (3H), indium (112In), and technetium (99Tc); luminescent labels, such as luminol; and fluorescent labels, such as fluorescein and rhodamine, and biotin.

One aspect of the invention is the detection and diagnosis of a disease or disorder associated with aberrant expression of a polypeptide of interest in an animal, preferably a mammal and most preferably a human. In one embodiment, diagnosis comprises: a) administering (for example, parenterally, subcutaneously, or intraperitoneally) to a subject an effective amount of a labeled molecule which specifically binds to the polypeptide of interest; b) waiting for a time interval following the administering for permitting the labeled molecule to preferentially concentrate at sites in the subject where the polypeptide is expressed (and for unbound labeled molecule to be cleared to background level); c) determining background level; and d) detecting the labeled molecule in the subject, such that detection of labeled molecule above the background level indicates that the subject has a particular disease or disorder associated with aberrant expression of the polypeptide of interest. Background level can be determined by various methods including, comparing the amount of labeled molecule detected to a standard value previously determined for a particular system.

It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).

Depending on several variables, including the type of label used and the mode of administration, the time interval following the administration for permitting the labeled molecule to preferentially concentrate at sites in the subject and for unbound labeled molecule to be cleared to background level is 6 to 48 hours or 6 to 24 hours or 6 to 12 hours. In another embodiment the time interval following administration is 5 to 20 days or 5 to 10 days.

In an embodiment, monitoring of the disease or disorder is carried out by repeating the method for diagnosing the disease or disease, for example, one month after initial diagnosis, six months after initial diagnosis, one year after initial diagnosis, etc.

Presence of the labeled molecule can be detected in the patient using methods known in the art for in vivo scanning. These methods depend upon the type of label used. Skilled artisans will be able to determine the appropriate method for detecting a particular label. Methods and devices that may be used in the diagnostic methods of the invention include, but are not limited to, computed tomography (CT), whole body scan such as position emission tomography (PET), magnetic resonance imaging (MRI), and sonography.

In a specific embodiment, the molecule is labeled with a radioisotope and is detected in the patient using a radiation responsive surgical instrument (Thurston et al., U.S. Patent No. 5,441,050). In another embodiment, the molecule is labeled with a fluorescent compound and is detected in the patient using a fluorescence responsive scanning instrument. In another embodiment, the molecule is labeled with a positron emitting metal and is detected in the patent using positron emission-tomography. In yet another embodiment, the molecule is labeled with a paramagnetic label and is detected in a patient using magnetic resonance imaging (MRI).

### Kits

The present invention provides kits that can be used in the above methods. In one embodiment, a kit comprises an antibody of the invention, preferably a purified antibody, in one or more containers. In a specific embodiment, the kits of the present invention contain a substantially isolated polypeptide comprising an epitope which is specifically immunoreactive with an antibody included in the kit. Preferably, the kits of the present invention further comprise a control antibody which does not react with the polypeptide of interest. In another specific embodiment, the kits of the present invention contain a means for detecting the binding of an antibody to a polypeptide of interest (e.g., the antibody may be conjugated to a detectable substrate such as a fluorescent compound, an enzymatic substrate, a radioactive compound or a luminescent compound, or a second antibody which recognizes the first antibody may be conjugated to a detectable substrate).

In another specific embodiment of the present invention, the kit is a diagnostic kit for use in screening serum containing antibodies specific against proliferative and/or cancerous polynucleotides and polypeptides. Such a kit may include a control antibody that does not react with the polypeptide of interest. Such a kit may include a substantially isolated polypeptide antigen comprising an epitope which is specifically immunoreactive with at least one anti-polypeptide antigen antibody. Further, such a kit includes means for detecting the binding of said antibody to the antigen (e.g., the antibody may be conjugated to a fluorescent compound such as fluorescein or rhodamine which can be detected by flow cytometry). In specific embodiments, the kit may include a recombinantly produced or chemically synthesized polypeptide antigen. The polypeptide antigen of the kit may also be attached to a solid support.

In a more specific embodiment the detecting means of the above-described kit includes a solid support to which said polypeptide antigen is attached. Such a kit may also include a non-attached reporter-labeled anti-human antibody. In this embodiment, binding of the antibody to the polypeptide antigen can be detected by binding of the said reporter-labeled antibody.

In an additional embodiment, the invention includes a diagnostic kit for use in screening serum containing antigens of the polypeptide of the invention. The diagnostic kit includes a substantially isolated antibody specifically immunoreactive with polypeptide or polynucleotide antigens, and means for detecting the binding of the polynucleotide or polypeptide antigen to the antibody. In one embodiment, the antibody is attached to a solid support. In a specific embodiment, the antibody may be a monoclonal antibody. The detecting means of the kit may include a second, labeled monoclonal antibody. Alternatively, or in addition, the detecting means may include a labeled, competing antigen.

In one diagnostic configuration, test serum is reacted with a solid phase reagent having a surface-bound antigen obtained by the methods of the present invention. After binding with specific antigen antibody to the reagent and removing unbound serum components by washing, the reagent is reacted with reporter-labeled anti-human antibody to bind reporter to the reagent in proportion to the amount of bound anti-antigen antibody on the solid support. The reagent is again washed to remove unbound labeled antibody, and the amount of reporter associated with the reagent is determined. Typically, the reporter is an enzyme which is detected by incubating the solid phase in the presence of a suitable fluorometric, luminescent or colorimetric substrate (Sigma, St. Louis, MO).

The solid surface reagent in the above assay is prepared by known techniques for attaching protein material to solid support material, such as polymeric beads, dip sticks, 96-well plate or filter material. These attachment methods generally include non-specific adsorption of the protein to the support or covalent attachment of the protein, typically through a free amine group, to a chemically reactive group on the solid support, such as an activated carboxyl, hydroxyl, or aldehyde group. Alternatively, streptavidin coated plates can be used in conjunction with biotinylated antigen(s).

Thus, the invention provides an assay system or kit for carrying out this diagnostic method. The kit generally includes a support with surface- bound recombinant antigens, and a reporter-labeled anti-human antibody for detecting surface-bound anti-antigen antibody.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgA, IgE, IgG, IgM) or portions thereof (CH1, CH2, CH3, and any combination thereof, including both entire domains and portions thereof), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian. immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).) Polynucleotides comprising or alternatively consisting of nucleic acids which encode these fusion proteins are also encompassed by the invention.

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells (e.g., Saccharomyces cerevisiae or Pichia pastoris (ATCC Accession No. 201178)); insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Preferred expression vectors for use in yeast systems include, but are not limited to pYES2, pYD1, pTEF1/Zeo, pYES2/GS, pPICZ,pGAPZ, pGAPZalph, pPIC9, pPIC3.5, pHIL-D2, pHIL-S1, pPIC3.5K, pPIC9K, and PAO815 (all available from Invitrogen, Carlbad, CA). Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

In one embodiment, the yeast *Pichia pastoris* is used to express the polypeptide of the present invention in a eukaryotic system. *Pichia pastoris* is a methylotrophic yeast which can metabolize methanol as its sole carbon source. A main step in the methanol metabolization pathway is the oxidation of methanol to formaldehyde using O₂. This reaction is catalyzed by the enzyme alcohol oxidase. In order to metabolize methanol as its sole carbon source, *Pichia pastoris* must generate high levels of alcohol oxidase due, in part, to the relatively low affinity of alcohol oxidase for O₂. Consequently, in a growth medium depending on methanol as a main carbon source, the promoter region of one of the two alcohol oxidase genes (*AOX1*) is highly active. In the presence of methanol, alcohol oxidase produced from the *AOX1* gene comprises up to approximately 30% of the total soluble protein in *Pichia pastoris. See,* Ellis, S.B., *et al., Mol. Cell. Biol.* 5:1111-21 (1985); Koutz, P.J, *et al.*, *Yeast* 5:167-77 (1989); Tschopp, J.F., *et al., Nucl. Acids Res.* 15:3859-76 (1987). Thus, a heterologous coding sequence, such as, for example, a polynucleotide of the present invention, under the transcriptional regulation of all or part of the *AOX1* regulatory sequence is expressed at exceptionally high levels in *Pichia* yeast grown in the presence of methanol.

In one example, the plasmid vector pPIC9K is used to express DNA encoding a polypeptide of the invention, as set forth herein, in a *Pichea* yeast system essentially as described in *"Pichia* Protocols: Methods in Molecular Biology," D.R. Higgins and J. Cregg, eds. The Humana Press, Totowa, NJ, 1998. This expression vector allows expression and secretion of a protein of the invention by virtue of the strong *AOX1* promoter linked to the *Pichia pastoris* alkaline phosphatase (PHO) secretory signal peptide (i.e., leader) located upstream of a multiple cloning site.

Many other yeast vectors could be used in place of pPIC9K, such as, pYES2, pYD1, pTEF1/Zeo, pYES2/GS, pPICZ, pGAPZ, pGAPZalpha, pPIC9, pPIC3.5, pHIL-D2, pHIL-S1, pPIC3.5K, and PAO815, as one skilled in the art would readily appreciate, as long as the proposed expression construct provides appropriately located signals for transcription, translation, secretion (if desired), and the like, including an in-frame AUG as required.

In another embodiment, high-level expression of a heterologous coding sequence, such as, for example, a polynucleotide of the present invention, may be achieved by cloning the heterologous polynucleotide of the invention into an expression vector such as, for example, pGAPZ or pGAPZalpha, and growing the yeast culture in the absence of methanol.

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., coding sequence), and/or to include genetic material (e.g., heterologous polynucleotide sequences) that is operably associated with the polynucleotides of the invention, and which activates, alters, and/or amplifies endogenous polynucleotides. For example, techniques known in the art may be used to operably associate heterologous control regions (e.g., promoter and/or, enhancer) and endogenous polynucleotide sequences via homologous recombination, resulting in the formation of a new transcription unit (see, e.g., U.S. Patent No. 5,641,670, issued June 24, 1997; U.S. Patent No. 5,733,761, issued March 31, 1998; International Publication No. WO 96/29411, published September 26, 1996; International Publication No. WO 94/12650, published August 4, 1994; Koller et al., Proc. Natl. Acad. Sci. USA 86:8932-8935 (1989); and Zijlstra et al., Nature 342:435-438 (1989), the disclosures of each of which are incorporated by reference in their entireties).

In addition, polypeptides of the invention can be chemically synthesized using techniques known in the art (e.g., see Creighton, 1983, Proteins: Structures and Molecular Principles, W.H. Freeman & Co., N.Y., and Hunkapiller et al., *Nature,* 310:105-111 (1984)). For example, a polypeptide corresponding to a fragment of a polypeptide sequence of the invention can be synthesized by use of a peptide synthesizer. Furthermore, if desired, nonclassical amino acids or chemical amino acid analogs can be introduced as a substitution or addition into the polypeptide sequence. Non-classical amino acids include, but are not limited to, to the D-isomers of the common amino acids, 2,4-diaminobutyric acid, a-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, b-alanine, fluoro-amino acids, designer amino acids such as b-methyl amino acids, Ca-methyl amino acids, Na-methyl amino acids, and amino acid analogs in general. Furthermore, the amino acid can be D (dextrorotary) or L (levorotary).

The invention encompasses polypeptides which are differentially modified during or after translation, *e.g.*, by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited, to specific chemical cleavage by cyanogen bromide, trypsin, chymotrypsin, papain, V8 protease, NaBH₄; acetylation, formylation, oxidation, reduction; metabolic synthesis in the presence of tunicamycin; etc.

Additional post-translational modifications encompassed by the invention include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The polypeptides may also be modified with a detectable label, such as an enzymatic, fluorescent, isotopic or affinity label to allow for detection and isolation of the protein.

Also provided by the invention are chemically modified derivatives of the polypeptides of the invention which may provide additional advantages such as increased solubility, stability and circulating time of the polypeptide, or decreased immunogenicity (see U.S. Patent NO: 4,179,337). The chemical moieties for derivitization may be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

The polymer may be of any molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 50,000, 55,000, 60,000, 65,000, 70,000, 75,000, 80,000, 85,000, 90,000, 95,000, or 100,000 kDa.

As noted above, the polyethylene glycol may have a branched structure. Branched polyethylene glycols are described, for example, in U.S. Patent No. 5,643,575; *Morpurgo et al., Appl. Biochem. Biotechnol.* 56:59-72 (1996); Vorobjev *et al., Nucleosides Nucleotides* 18:2745-2750 (1999); and Caliceti *et al., Bioconjug. Chem.* 10:638-646 (1999), the disclosures of each of which are incorporated herein by reference.

The polyethylene glycol molecules (or other chemical moieties) should be attached to the protein with consideration of effects on functional or antigenic domains of the protein. There are a number of attachment methods available to those skilled in the art, e.g., EP 0 401 384, herein incorporated by reference (coupling PEG to G-CSF), see also Malik et al., Exp. Hematol. 20:1028-1035 (1992) (reporting . pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group.

As suggested above, polyethylene glycol may be attached to proteins via linkage to any of a number of amino acid residues. For example, polyethylene glycol can be linked to a proteins via covalent bonds to lysine, histidine, aspartic acid, glutamic acid, or cysteine residues. One or more reaction chemistries may be employed to attach polyethylene glycol to specific amino acid residues (e.g., lysine, histidine, aspartic acid, glutamic acid, or cysteine) of the protein or to more than one type of amino acid residue (e.g., lysine, histidine, aspartic acid, glutamic acid, cysteine and combinations thereof) of the protein.

One may specifically desire proteins chemically modified at the N-terminus. Using polyethylene glycol as an illustration of the present composition, one may select from a variety of polyethylene glycol molecules (by molecular weight, branching, etc.), the proportion of polyethylene glycol molecules to protein (polypeptide) molecules in the reaction mix, the type of pegylation reaction to be performed, and the method of obtaining the selected N-terminally pegylated protein. The method of obtaining the N-terminally pegylated preparation (i.e., separating this moiety from other monopegylated moieties if necessary) may be by purification of the N-terminally pegylated material from a population of pegylated protein molecules. Selective proteins chemically modified at the N-terminus modification may be accomplished by reductive alkylation which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved.

As indicated above, pegylation of the proteins of the invention may be accomplished by any number of means. For example, polyethylene glycol may be attached to the protein either directly or by an intervening linker. Linkerless systems for attaching polyethylene glycol to proteins are described in Delgado *et al., Crit. Rev. Thera. Drug Carrier Sys.* 9:249-304 (1992); *Francis et al., Intern. J. of Hematol. 68*:1-18 (1998); U.S. Patent No. 4,002,531; U.S. Patent No. 5,349,052; WO 95/06058; and WO 98/32466, the disclosures of each of which are incorporated herein by reference.

One system for attaching polyethylene glycol directly to amino acid residues of proteins without an intervening linker employs tresylated MPEG, which is produced by the modification of monmethoxy polyethylene glycol (MPEG) using tresylchloride (ClSO₂CH₂CF₃). Upon reaction of protein with tresylated MPEG, polyethylene glycol is directly attached to amine groups of the protein. Thus, the invention includes protein-polyethylene glycol conjugates produced by reacting proteins of the invention with a polyethylene glycol molecule having a 2,2,2-trifluoreothane sulphonyl group.

Polyethylene glycol can also be attached to proteins using a number of different intervening linkers. For example, U.S. Patent No. 5,612,460, the entire disclosure of which is incorporated herein by reference, discloses urethane linkers for connecting polyethylene glycol to proteins. Protein-polyethylene glycol conjugates wherein the polyethylene glycol is attached to the protein by a linker can also be produced by reaction of proteins with compounds such as MPEG-succinimidylsuccinate, MPEG activated with 1,1'-carbonyldiimidazole, MPEG-2,4,5-trichloropenylcarbonate, MPEG-p-nitrophenolcarbonate, and various MPEG-succinate derivatives. A number additional polyethylene glycol derivatives and reaction chemistries for attaching polyethylene glycol to proteins are described in WO 98/32466, the entire disclosure of which is incorporated herein by reference. Pegylated protein products produced using the reaction chemistries set out herein are included within the scope of the invention.

The number of polyethylene glycol moieties attached to each protein of the invention (*i.e.,* the degree of substitution) may also vary. For example, the pegylated proteins of the invention may be linked, on average, to 1, 2, 3, 4, 5, 6, 7, 8, 9, 10, 12, 15, 17, 20, or more polyethylene glycol molecules. Similarly, the average degree of substitution within ranges such as 1-3, 2-4, 3-5, 4-6, 5-7, 6-8, 7-9, 8-10, 9-11, 10-12, 11-13, 12-14, 13-15, 14-16, 15-17, 16-18, 17-19, or 18-20 polyethylene glycol moieties per protein molecule. Methods for determining the degree of substitution are discussed, for example, in Delgado *et al., Crit. Rev. Thera. Drug Carrier Sys. 9*:249-304 (1992).

The polypeptides of the invention may be in monomers or multimers (i.e., dimers, trimers, tetramers and higher multimers). Accordingly, the present invention relates to monomers and multimers of the polypeptides of the invention, their preparation, and compositions (preferably, *Therapeutics*) containing them. In specific embodiments, the polypeptides of the invention are monomers, dimers, trimers or tetramers. In additional embodiments, the multimers of the invention are at least dimers, at least trimers, or at least tetramers.

Multimers encompassed by the invention may be homomers or heteromers. As used herein, the term homomer, refers to a multimer containing only polypeptides corresponding to the amino acid sequence of SEQ ID NO:Y or encoded by the cDNA contained in a deposited clone (including fragments, variants, splice variants, and fusion proteins, corresponding to these polypeptides as described herein). These homomers may contain polypeptides having identical or different amino acid sequences. In a specific embodiment, a homomer of the invention is a multimer containing only polypeptides having an identical amino acid sequence. In another specific embodiment, a homomer of the invention is a multimer containing polypeptides having different amino acid sequences. In specific embodiments, the multimer of the invention is a homodimer (*e.g.*, containing polypeptides having identical or different amino acid sequences) or a homotrimer (*e.g.*, containing polypeptides having identical and/or different amino acid sequences). In additional embodiments, the homomeric multimer of the invention is at least a homodimer, at least a homotrimer, or at least a homotetramer.

As used herein, the term heteromer refers to a multimer containing one or more heterologous polypeptides (*i.e.,* polypeptides of different proteins) in addition to the polypeptides of the invention. In a specific embodiment, the multimer of the invention is a heterodimer, a heterotrimer, or a heterotetramer. In additional embodiments, the heteromeric multimer of the invention is at least a heterodimer, at least a heterotrimer, or at least a heterotetramer.

Multimers of the invention may be the result of hydrophobic, hydrophilic, ionic and/or covalent associations and/or may be indirectly linked, by for example, liposome formation. Thus, in one embodiment, multimers of the invention, such as, for example, homodimers or homotrimers, are formed when polypeptides of the invention contact one another in solution. In another embodiment, heteromultimers of the invention, such as, for example, heterotrimers or heterotetramers, are formed when polypeptides of the invention contact antibodies to the polypeptides of the invention (including antibodies to the heterologous polypeptide sequence in a fusion protein of the invention) in solution. In other embodiments, multimers of the invention are formed by covalent associations with and/or between the polypeptides of the invention. Such covalent associations may involve one or more amino acid residues contained in the polypeptide sequence ( e.g., that recited in the sequence listing, or contained in the polypeptide encoded by a deposited clone). In one instance, the covalent associations are cross-linking between cysteine residues located within the polypeptide sequences which interact in the native (i.e., naturally occurring) polypeptide. In another instance, the covalent associations are the consequence of chemical or recombinant manipulation. Alternatively, such covalent associations may involve one or more amino acid residues contained in the heterologous polypeptide sequence in a fusion protein of the invention.

In one example, covalent associations are between the heterologous sequence contained in a fusion protein of the invention (see, e.g., US Patent Number 5,478,925). In a specific example, the covalent associations are between the heterologous sequence contained in an Fc fusion protein of the invention (as described herein). In another specific example, covalent associations of fusion proteins of the invention are between heterologous polypeptide sequence from another protein that is capable of forming covalently associated multimers, such as for example, oseteoprotegerin (see, e.g., International Publication NO: WO 98/49305, the contents of which are herein incorporated by reference in its entirety). In another embodiment, two or more polypeptides of the invention are joined through peptide linkers. Examples include those peptide linkers described in U.S. Pat. No. 5,073,627 (hereby incorporated by reference). Proteins comprising multiple polypeptides of the invention separated by peptide linkers may be produced using conventional recombinant DNA technology.

Another method for preparing multimer polypeptides of the invention involves use of polypeptides of the invention fused to a leucine zipper or isoleucine zipper polypeptide sequence. Leucine zipper and isoleucine zipper domains are polypeptides that promote multimerization of the proteins in which they are found. Leucine zippers were originally identified in several DNA-binding proteins (Landschulz et al., Science 240:1759, (1988)), and have since been found in a variety of different proteins. Among the known leucine zippers are naturally occurring peptides and derivatives thereof that dimerize or trimerize. Examples of leucine zipper domains suitable for producing soluble multimeric proteins of the invention are those described in PCT application WO 94/10308, hereby incorporated by reference. Recombinant fusion proteins comprising a polypeptide of the invention fused to a polypeptide sequence that dimerizes or trimerizes in solution are expressed in suitable host cells, and the resulting soluble multimeric fusion protein is recovered from the culture supernatant using techniques known in the art.

Trimeric polypeptides of the invention may offer the advantage of enhanced biological activity. Preferred leucine zipper moieties and isoleucine moieties are those that preferentially form trimers. One example is a leucine zipper derived from lung surfactant protein D (SPD), as described in Hoppe et al. (FEBS Letters 344:191, (1994)) and in U.S. patent application Ser. No. 08/446,922, hereby incorporated by reference. Other peptides derived from naturally occurring trimeric proteins may be employed in preparing trimeric polypeptides of the invention.

In another example, proteins of the invention are associated by interactions between Flag® polypeptide sequence contained in fusion proteins of the invention containing Flag® polypeptide seuqence. In a further embodiment, associations proteins of the invention are associated by interactions between heterologous polypeptide sequence contained in Flag® fusion proteins of the invention and anti-Flag® antibody.

The multimers of the invention may be generated using chemical techniques known in the art. For example, polypeptides desired to be contained in the multimers of the invention may be chemically cross-linked using linker molecules and linker molecule length optimization techniques known in the art (see, e.g., US Patent Number 5,478,925, which is herein incorporated by reference in its entirety). Additionally, multimers of the invention may be generated using techniques known in the art to form one or more inter-molecule cross-links between the cysteine residues located within the sequence of the polypeptides desired to be contained in the multimer (see, e.g., US Patent Number 5,478,925, which is herein incorporated by reference in its entirety). Further, polypeptides of the invention may be routinely modified by the addition of cysteine or biotin to the C terminus or N-terminus of the polypeptide and techniques known in the art may be applied to generate multimers containing one or more of these modified polypeptides (see, e.g., US Patent Number 5,478,925, which is herein incorporated by reference in its entirety). Additionally, techniques known in the art may be applied to generate liposomes containing the polypeptide components desired to be contained in the multimer of the invention (see, e.g., US Patent Number 5,478,925, which is herein incorporated by reference in its entirety).

Alternatively, multimers of the invention may be generated using genetic engineering techniques known in the art. In one embodiment, polypeptides contained in multimers of the invention are produced recombinantly using fusion protein technology described herein or otherwise known in the art (see, e.g., US Patent Number 5,478,925, which is herein incorporated by reference in its entirety). In a specific embodiment, polynucleotides coding for a homodimer of the invention are generated by ligating a polynucleotide sequence encoding a polypeptide of the invention to a sequence encoding a linker polypeptide and then further to a synthetic polynucleotide encoding the translated product of the polypeptide in the reverse orientation from the original C-terminus to the N-terminus (lacking the leader sequence) (see, e.g., US Patent Number 5,478,925, which is herein incorporated by reference in its entirety). In another embodiment, recombinant techniques described herein or otherwise known in the art are applied to generate recombinant polypeptides of the invention which contain a transmembrane domain (or hyrophobic or signal peptide) and which can be incorporated by membrane reconstitution techniques into liposomes (see, e.g., US Patent Number 5,478,925, which is herein incorporated by reference in its entirety).

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalizatibn of the polynucleotides can be achieved with panels of specific chromosome fragments. Other gene mapping strategies that can be used include in situ hybridization, prescreening with labeled flow-sorted chromosomes, preselection by hybridization to construct chromosome specific-cDNA libraries and computer mapping techniques (See, e.g., Shuler, Trends Biotechnol 16:456-459 (1998) which is hereby incorporated by reference in its entirety).

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes).

The polynucleotides of the present invention would likewise be useful for radiation hybrid mapping, HAPPY mapping, and long range restriction mapping. For a review of these techniques and others known in the art, see, e.g., Dear, "Genome Mapping: A Practical Approach," IRL Press at Oxford University Press, London (1997); Aydin, J. Mol. Med. 77:691-694 (1999); Hacia et al., Mol. Psychiatry 3:483-492 (1998); Herrick et al., Chromosome Res. 7:409-423 (1999); Hamilton et al., Methods Cell Biol. 62:265-280 (2000); and/or Ott, J. Hered. 90:68-70 (1999) each of which is hereby incorporated by reference in its entirety.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

Thus, the invention also provides a diagnostic method useful during diagnosis of a disorder, involving measuring the expression level of polynucleotides of the present invention in cells or body fluid from an individual and comparing the measured gene expression level with a standard level of polynucleotide expression level, whereby an increase or decrease in the gene expression level compared to the standard is indicative of a disorder.

In still another embodiment, the invention includes a kit for analyzing samples for the presence of proliferative and/or cancerous polynucleotides derived from a test subject. In a general embodiment, the kit includes at least one polynucleotide probe containing a nucleotide sequence that will specifically hybridize with a polynucleotide of the present invention and a suitable container. In a specific embodiment, the kit includes two polynucleotide probes defining an internal region of the polynucleotide of the present invention, where each probe has one strand containing a 31'mer-end internal to the region. In a further embodiment, the probes may be useful as primers for polymerase chain reaction amplification.

Where a diagnosis of a disorder, has already been made according to conventional methods, the present invention is useful as a prognostic indicator, whereby patients exhibiting enhanced or depressed polynucleotide of the present invention expression will experience a worse clinical outcome relative to patients expressing the gene at a level nearer the standard level.

By "measuring the expression level of polynucleotide of the present invention" is intended qualitatively or quantitatively measuring or estimating the level of the polypeptide of the present invention or the level of the mRNA encoding the polypeptide in a first biological sample either directly (e.g., by determining or estimating absolute protein level or mRNA level) or relatively (e.g., by comparing to the polypeptide level or mRNA level in a second biological sample). Preferably, the polypeptide level or mRNA level in the first biological sample is' measured or estimated and compared to a standard polypeptide level or mRNA level, the standard being taken from a second biological sample obtained from an individual not having the disorder or being determined by averaging levels from a population of individuals not having a disorder. As will be appreciated in the art, once a standard polypeptide level or mRNA level is known, it can be used repeatedly as a standard for comparison.

By "biological sample" is intended any biological sample obtained from an individual, body fluid, cell line, tissue culture, or other source which contains the polypeptide of the present invention or mRNA. As indicated, biological samples include body fluids (such as semen, lymph, sera, plasma, urine, synovial fluid and spinal fluid) which contain the polypeptide of the present invention, and other tissue sources found to express the polypeptide of the present invention. Methods for obtaining tissue biopsies and body fluids from mammals are well known in the art. Where the biological sample is to include mRNA, a tissue biopsy is the preferred source.

The method(s) provided above may preferrably be applied in a diagnostic method and/or kits in which polynucleotides and/or polypeptides are attached to a solid support. In one exemplary method, the support may be a "gene chip" or a "biological chip" as described in US Patents 5,837,832, 5,874,219, and 5,856,174. Further, such a gene chip with polynucleotides of the present invention attached may be used to identify polymorphisms between the polynucleotide sequences, with polynucleotides isolated from a test subject. The knowledge of such polymorphisms (i.e. their location, as well as, their existence) would be beneficial in identifying disease loci for many disorders, including cancerous diseases and conditions. Such a method is described in US Patents 5,858,659 and 5,856,104. The US Patents referenced supra are hereby incorporated by reference in their entirety herein.

The present invention encompasses polynucleotides of the present invention that are chemically synthesized, or reproduced as peptide nucleic acids (PNA), or according to other methods known in the art. The use of PNAs would serve as the preferred form if the polynucleotides are incorporated onto a solid support, or gene chip. For the purposes of the present invention, a peptide nucleic acid (PNA) is a polyamide type of DNA analog and the monomeric units for adenine, guanine, thymine and cytosine are available commercially (Perceptive Biosystems). Certain components of DNA, such as phosphorus, phosphorus oxides, or deoxyribose derivatives, are not present in PNAs. As disclosed by P. E. Nielsen, M. Egholm, R. H. Berg and O. Buchardt, Science 254, 1497 (1991); and M. Egholm, O. Buchardt, L.Christensen, C. Behrens, S. M. Freier, D. A. Driver, R. H. Berg, S. K. Kim, B. Norden, and P. E. Nielsen, Nature 365, 666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

The present invention is useful for detecting cancer in mammals. In particular the invention is useful during diagnosis of pathological cell proliferative neoplasias which include, but are not limited to acute myelogenous leukemias including acute monocytic leukemia, acute myeloblastic leukemia, acute promyelocytic leukemia, acute myelomonocytic leukemia, acute erythroleukemia, acute megakaryocytic leukemia, and acute undifferentiated leukemia, etc.; and chronic myelogenous leukemias including chronic myelomonocytic leukemia, chronic granulocytic leukemia, etc. Preferred mammals include monkeys, apes, cats, dogs, cows, pigs, horses, rabbits and humans. Particularly preferred are humans.

Pathological cell proliferative diseases, disorders, and/or conditions are often associated with inappropriate activation of proto-oncogenes. (Gelmann, E. P. et al., "The Etiology of Acute Leukemia: Molecular Genetics and Viral Oncology," in Neoplastic Diseases of the Blood, Vol 1., Wiernik, P. H. et al. eds., 161-182 (1985)). Neoplasias are now believed to result from the qualitative alteration of a normal cellular gene product, or from the quantitative modification of gene expression by insertion into the chromosome of a viral sequence, by chromosomal translocation of a gene to a more actively transcribed region, or by some other mechanism. (Gelmann et al., supra) It is likely that mutated or altered expression of specific genes is involved in the pathogenesis of some leukemias, among other tissues and cell types. (Gelmann et al., supra) Indeed, the human counterparts of the oncogenes involved in some animal neoplasias have been amplified or translocated in some cases of human leukemia and carcinoma. (Gelmann et al., supra)
For example, c-myc expression is highly amplified in the non-lymphocytic leukemia cell line HL-60. When HL-60 cells are chemically induced to stop proliferation, the level of c-myc is found to be downregulated. (International Publication Number WO 91/15580) However, it has been shown that exposure of HL-60 cells to a DNA construct that is complementary to the 5' end of c-myc or c-myb blocks translation of the corresponding mRNAs which downregulates expression of the c-myc or c-myb proteins and causes arrest of cell proliferation and differentiation of the treated cells. (International Publication Number WO 91/15580; Wickstrom et al., Proc. Natl. Acad. Sci. 85:1028 (1988); Anfossi et al., Proc. Natl. Acad. Sci. 86:3379 (1989)). However, the skilled artisan would appreciate the present invention's usefulness would not be limited to treatment of proliferative diseases, disorders, and/or conditions of hematopoietic cells and tissues, in light of the numerous cells and cell types of varying origins which are known to exhibit proliferative phenotypes.

In addition to the foregoing, a polynucleotide can be used to control gene expression through triple helix formation or antisense DNA or RNA. Antisense techniques are discussed, for example, in Okano, J. Neurochem. 56: 560 (1991); "Oligodeoxynucleotides as Antisense Inhibitors of Gene Expression,CRCPress, Boca Raton, FL (1988). Triple helix formation is discussed in, for instance Lee et al., Nucleic Acids Research 6: 3073 (1979); Cooney et al., Science 241: 456 (1988); and Dervan et al., Science 251: 1360 (1991). Both methods rely on binding of the polynucleotide to a complementary DNA or RNA. For these techniques, preferred polynucleotides are usually oligonucleotides 20 to 40 bases in length and complementary to either the region of the gene involved in transcription (triple helix-see Lee et al., Nucl. Acids Res. 6:3073 (1979); Cooney et al., Science 241:456 (1988); and Dervan et al., Science 251:1360 (1991) ) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat or prevent disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification. difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, synovial fluid, amniotic fluid, breast milk, lymph, pulmonary sputum or surfactant,urine,fecal matter, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (1251, 1211), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder. With respect to cancer, the presence of a relatively high amount of transcript in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Moreover, polypeptides of the present invention can be used to treat, prevent, and/or diagnose disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B, SOD, catalase, DNA repair proteins), to inhibit the activity of a polypeptide (e.g., an oncogene or tumor supressor), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth inhibition, enhancement of the immune response to proliferative cells or tissues).

Similarly, antibodies directed to a polypeptide of the present invention can also be used to treat, prevent, and/or diagnose disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Gene Therapy Methods

Another aspect of the present invention is to gene therapy methods for treatingor preventing disorders, diseases and conditions. The gene therapy methods relate to the introduction of nucleic acid (DNA, RNA and antisense DNA or RNA) sequences into an animal to achieve expression of a polypeptide of the present invention. This method requires a polynucleotide which codes for a polypeptide of the invention that operatively linked to a promoter and any other genetic elements necessary for the expression of the polypeptide by the target tissue. Such gene therapy and delivery techniques are known in the art, see, for example, WO90/11092, which is herein incorporated by reference.

Thus, for example, cells from a patient may be engineered with a polynucleotide (DNA or RNA) comprising a promoter operably linked to a polynucleotide of the invention *ex vivo,* with the engineered cells then being provided to a patient to be treated with the polypeptide. Such methods are well-known in the art. For example, see Belldegrun et al., J. Natl. Cancer Inst., 85:207-216 (1993); Ferrantini et al., Cancer Research, 53:107-1112 (1993); Ferrantini et al., J. Immunology 153: 4604-4615 (1994); Kaido, T., et al., Int. J. Cancer 60: 221-229 (1995); Ogura et al., Cancer Research 50: 5102-5106 (1990); Santodonato, et al., Human Gene Therapy 7:1-10 (1996); Santodonato, et al., Gene Therapy 4:1246-1255 (1997); and Zhang, et al., Cancer Gene Therapy 3: 31-38 (1996)), which are herein incorporated by reference. In one embodiment, the cells which are engineered are arterial cells. The arterial cells may be reintroduced into the patient through direct injection to the artery, the tissues surrounding the artery, or through catheter injection.

As discussed in more detail below, the polynucleotide constructs can be delivered by any method that delivers injectable materials to the cells of an animal, such as, injection into the interstitial space of tissues (heart, muscle, skin, lung, liver, and the like). The polynucleotide constructs may be delivered in a pharmaceutically acceptable liquid or aqueous carrier.

In one embodiment, the polynucleotide of the invention is delivered as a naked polynucleotide. The term "naked" polynucleotide, DNA or RNA refers to sequences that are free from any delivery vehicle that acts to assist, promote or facilitate entry into the cell, including viral sequences, viral particles, liposome formulations, lipofectin or precipitating agents and the like. However, the polynucleotides of the invention can also be delivered in liposome formulations and lipofectin formulations and the like can be prepared by methods well known to those skilled in the art. Such methods are described, for example, in U.S. Patent Nos. 5,593,972, 5,589,466, and 5,580,859, which are herein incorporated by reference.

The polynucleotide vector constructs of the invention used in the gene therapy method are preferably constructs that will not integrate into the host genome nor will they contain sequences that allow for replication. Appropriate vectors include pWLNEO, pSV2CAT, pOG44, pXT1 and pSG available from Stratagene; pSVK3, pBPV, pMSG and pSVL available from Pharmacia; and pEF1/V5, pcDNA3.1, and pRc/CMV2 available from Invitrogen. Other suitable vectors will be readily apparent to the skilled artisan.

Any strong promoter known to those skilled in the art can be used for driving the expression of polynucleotide sequence of the invention. Suitable promoters include adenoviral promoters, such as the adenoviral major late promoter; or heterologous promoters, such as the cytomegalovirus (CMV) promoter; the respiratory syncytial virus (RSV) promoter; inducible promoters, such as the MMT promoter, the metallothionein promoter, heat shock promoters; the albumin promoter; the ApoAI promoter; human globin promoters; viral thymidine kinase promoters, such as the Herpes Simplex thymidine kinase promoter; retroviral LTRs; the b-actin promoter; and human growth hormone promoters. The promoter also may be the native promoter for the polynucleotides of the invention.

Unlike other gene therapy techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct of the invention can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular, fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the *nakednucleic,* acid sequence injection, an effective dosage amount of DNA or RNA will be in the range of from about 0.05 mg/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration.

The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked DNA constructs can be delivered to arteries during angioplasty by the catheter used in the procedure.

The naked polynucleotides are delivered by any method known in the art, including, but not limited to, direct needle injection at the delivery site, intravenous injection, topical administration, catheter infusion, and so-called "gene guns". These delivery methods are known in the art.

The constructs may also be delivered with delivery vehicles such as viral sequences, viral particles, liposome formulations, lipofectin, precipitating agents, etc. Such methods of delivery are known in the art.

In certain embodiments, the polynucleotide constructs of the invention are complexed in a liposome preparation. Liposomal preparations for use in the instant invention include cationic (positively charged), anionic (negatively charged) and neutral preparations. However, cationic liposomes are particularly preferred because a tight charge complex can be formed between the cationic liposome and the polyanionic nucleic acid. Cationic liposomes have been shown to mediate intracellular delivery of plasmid DNA (Felgner et al., Proc. Natl. Acad. Sci. USA , 84:7413-7416 (1987), which is herein incorporated by reference); mRNA (Malone et al., Proc. Natl. Acad. Sci. USA , 86:6077-6081 (1989), which is herein incorporated by reference); and purified transcription factors (Debs et al., J. Biol. Chem., 265:10189-10192 (1990), which is herein incorporated by reference), in functional form.

Cationic liposomes are readily available. For example, N[1-2,3-dioleyloxy)propyl]-N,N,N-triethylammonium (DOTMA) liposomes are particularly useful and are available under the trademark Lipofectin, from GIBCO BRL, Grand Island, N.Y. (See, also, Felgner et al., Proc. Natl Acad. Sci. USA, 84:7413-7416 (1987), which is herein incorporated by reference). Other commercially available liposomes include transfectace (DDAB/DOPE) and DOTAP/DOPE (Boehringer).

Other cationic liposomes can be prepared from readily available materials using techniques well known in the art. See, e.g. PCT Publication NO: WO 90/11092 (which is herein incorporated by reference) for a description of the synthesis of DOTAP (1,2-bis(oleoyloxy)-3-(trimethylammonio)propane) liposomes. Preparation of DOTMA liposomes is explained in the literature, see, e.g., Felgner et al., Proc. Natl. Acad. Sci. USA, 84:7413-7417, which is herein incorporated by reference. Similar methods can be used to prepare liposomes from other cationic lipid materials.

Similarly, anionic and neutral liposomes are readily available, such as from Avanti Polar Lipids (Birmingham, Ala.), or can be easily prepared using readily available materials. Such materials include phosphatidyl, choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

For example, commercially dioleoylphosphatidyl choline (DOPC), dioleoylphosphatidyl glycerol (DOPG), and dioleoylphosphatidyl ethanolamine (DOPE) can be used in various combinations to make conventional liposomes, with or without the addition of cholesterol. Thus, for example, DOPG/DOPC vesicles can be prepared by drying 50 mg each of DOPG and DOPC under a stream of nitrogen gas into a sonication vial. The sample is placed under a vacuum pump overnight and is hydrated the following day with deionized water. The sample is then sonicated for 2 hours in a capped vial, using a Heat Systems model 350 sonicator equipped with an inverted cup (bath type) probe at the maximum setting while the bath is circulated at 15EC. Alternatively, negatively charged vesicles can be prepared without sonication to produce multilamellar vesicles or by extrusion through nucleopore membranes to produce unilamellar vesicles of discrete size. Other methods are known and available to those of skill in the art.

The liposomes can comprise multilamellar vesicles (MLVs), small unilamellar vesicles (SUVs), or large unilamellar vesicles (LUVs), with SUVs being preferred. The various liposome-nucleic acid complexes are prepared using methods well known in the art. See, e.g., Straubinger et al., Methods of Immunology , 101:512-527 (1983), which is herein incorporated by reference. For example, MLVs containing nucleic acid can be prepared by depositing a thin film of phospholipid on the walls of a glass tube and subsequently hydrating with a solution of the material to be encapsulated. SUVs are prepared by extended sonication of MLVs to produce a homogeneous population of unilamellar liposomes. The material to be entrapped is added to a suspension of preformed MLVs and then sonicated. When using liposomes containing cationic lipids, the dried lipid film is resuspended in an appropriate solution such as sterile water or an isotonic buffer solution such as 10 mM Tris/Nacl, sonicated, and then the preformed liposomes are mixed directly with the DNA. The liposome and DNA form a very stable complex due to binding of the positively charged liposomes to the cationic DNA. SUVs find use with small nucleic acid fragments. LUVs are prepared by a number of methods, well known in the art. Commonly used methods include Ca²⁺-EDTA chelation (Papahadjopoulos et al., Biochim. Biophys. Acta, 394:483 (1975); Wilson et al., Cell, 17:77 (1979)); ether injection (Deamer et al., Biochim. Biophys. Acta, 443:629 (1976); Ostro et al., Biochem. Biophys. Res. Commun., 76:836 (1977); Fraley et al., Proc. Natl. Acad. Sci. USA, 76:3348 (1979)); detergent dialysis (Enoch et al., Proc. Natl. Acad. Sci. USA , 76:145 (1979)); and reverse-phase evaporation (REV) (Fraley et al., J. Biol. Chem., 255:10431 (1980); Szoka et al., Proc. Natl. Acad. Sci. USA, 75:145 (1978); Schaefer-Ridder et al., Science, 215:166 (1982)), which are herein incorporated by reference.

Generally, the ratio of DNA to liposomes will be from about 10:1 to about 1:10. Preferably, the ration will be from about 5:1 to about 1:5. More preferably, the ration will be about 3:1 to about 1:3. Still more preferably, the ratio will be about 1:1.

U.S. Patent NO: 5,676,954 (which is herein incorporated by reference) reports on the injection of genetic material, complexed with cationic liposomes carriers, into mice. U.S. Patent Nos. 4,897,355, 4,946,787, 5,049,386, 5,459,127, 5,589,466, 5,693,622, 5,580,859, 5,703,055, and international publication NO: WO 94/9469 (which are herein incorporated by reference) provide cationic lipids for use in transfecting DNA into cells and mammals. U.S. Patent Nos. 5,589,466, 5,693,622, 5,580,859, 5,703,055, and international publication NO: WO 94/9469 (which are herein incorporated by reference) provide methods for delivering DNA-cationic lipid complexes to mammals.

In certain embodiments, cells are engineered, *ex vivo* or *in* vivo, using a retroviral particle containing RNA which comprises a sequence encoding polypeptides of the invention. Retroviruses from which the retroviral plasmid vectors may be derived include, but are not limited to, Moloney Murine Leukemia Virus, spleen necrosis virus, Rous sarcoma Virus, Harvey Sarcoma Virus, avian leukosis virus, gibbon ape leukemia virus, human immunodeficiency virus, Myeloproliferative Sarcoma Virus, and mammary tumor virus.

The retroviral plasmid vector is employed to transduce packaging cell lines to form producer cell lines. Examples of packaging cells which may be transfected include, but are not limited to, the PE501, PA317, R-2, R-AM, PA12, T19-14X, VT-19-17-H2, RCRE, RCRIP, GP+E-86, GP+envAm12, and DAN cell lines as described in Miller, Human Gene Therapy, 1:5-14 (1990), which is incorporated herein by reference in its entirety. The vector may transduce the packaging, cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line generates infectious retroviral vector particles which include polynucleotide encoding polypeptides of the invention. Such retroviral vector particles then may be employed, to transduce eukaryotic cells, either *in vitro* or *in vivo.* The transduced eukaryotic cells will express polypeptides of the invention.

In certain other embodiments, cells are engineered, *ex vivo* or in *vivo,* with polynucleotides of the invention contained in an adenovirus vector. Adenovirus can be manipulated such that it encodes and expresses polypeptides of the invention, and at the same time is inactivated in terms of its ability to replicate in a normal lytic viral life cycle. Adenovirus expression is achieved without integration of the viral DNA into the host cell chromosome, thereby alleviating concerns about insertional mutagenesis. Furthermore, adenoviruses have been used as live enteric vaccines for many years with an excellent safety profile (Schwartzet al., Am. Rev. Respir. Dis., 109:233-238 (1974)). Finally, adenovirus mediated gene transfer has been demonstrated in a number of instances including transfer of alpha-1-antitrypsin and CFTR to the lungs of cotton rats (Rosenfeld et al.,Science , 252:431-434 (1991); Rosenfeld et al., Cell, 68:143-155 (1992)). Furthermore, extensive studies to attempt to establish adenovirus as a causative agent in human cancer were uniformly negative (Green et al. Proc. Natl. Acad. Sci. USA , 76:6606 (1979)).

Suitable adenoviral vectors useful in the present invention are described, for example, in Kozarsky and Wilson, Curr. Opin. Genet. Devel., 3:499-503.(1993); Rosenfeld et al., Cell , 68:143-155 (1992); Engelhardt et al., Human Genet. Ther., 4:759-769 (1993); Yang et al., Nature Genet., 7:362-369 (1994); Wilson et al., Nature, 365:691-692 (1993); and U.S. Patent NO: 5,652,224; which are herein incorporated by reference. For example, the adenovirus vector Ad2 is useful and can be grown in human 293 cells. These cells contain the E1 region of adenovirus and constitutively express E1a and E1b, which complement the defective adenoviruses by providing the products of the genes deleted from the vector. In addition to Ad2, other varieties of adenovirus (e.g., Ad3, Ad5, and Ad7) are also useful in the present invention.

Preferably, the adenoviruses used in the present invention are replication. deficient. Replication deficient adenoviruses require the aid of a helper virus and/or packaging cell line to form infectious particles. The resulting virus is capable of infecting cells and can express a polynucleotide of interest which is operably linked to a promoter, but cannot replicate in most cells. Replication deficient adenoviruses-may be deleted in one or more of all or a portion of the following genes: E1a, E1b, E3, E4, E2a, or L1 through L5.

In certain other embodiments, the cells are engineered, *ex vivo* or *in vivo*, using an adeno-associated virus (AAV). AAVs are naturally occurring defective viruses that require helper viruses to produce infectious particles (Muzyczka, Curr. Topics in Microbiol. Immunol., 158:97 (1992)). It is also one of the few viruses that may integrate its DNA into non-dividing cells. Vectors containing as little as 300 base pairs of AAV can be packaged and can integrate, but space for exogenous DNA is limited to about 4.5 kb. Methods for producing and using such AAVs are known in the art. See, for example, U.S. Patent Nos. 5,139,941, 5,173,414, 5,354,678, 5,436,146, 5,474,935, 5,478,745, and 5,589,377.

For example, an appropriate AAV vector for use in the present invention will include all the sequences necessary for DNA replication, encapsidation, and host-cell integration. The polynucleotide construct containing polynucleotides of the invention is inserted into the AAV vector using standard cloning methods, such as those found in Sambrook et al., Molecular Cloning: A Laboratory Manual, Cold Spring Harbor Press (1989). The recombinant AAV vector is then transfected into packaging cells which are infected with a helper virus, using any standard technique, including lipofection, electroporation, calcium phosphate precipitation, etc. Appropriate helper viruses include adenoviruses, cytomegaloviruses, vaccinia viruses, or herpes viruses. Once the packaging cells are transfected and infected, they will produce infectious AAV viral particles which contain the polynucleotide construct of the invention. These viral particles are then used to transduce eukaryotic cells, either *ex vivo* or *in vivo.* The transduced cells will contain the polynucleotide construct integrated into its genome, and will express the desired gene product.

Another method of gene therapy involves operably associating heterologous control regions and endogenous polynucleotide sequences (e.g. encoding the polypeptide sequence of interest) via homologous recombination (see, e.g., U.S. Patent NO: 5,641,670, issued June 24, 1997; International Publication NO: WO 96/29411, published September 26, 1996; International Publication NO: WO 94/12650, published August 4, 1994; Koller et al., Proc. Natl. Acad. Sci. USA, 86:8932-8935 (1989); and Zijlstra et al., Nature, 342:435-438 (1989). This method involves the activation of a gene which is present in the target cells, but which is not normally expressed in the cells, or is expressed at a lower level than desired.

Polynucleotide constructs are made, using standard techniques known in the art, which contain the promoter with targeting sequences flanking the promoter. Suitable promoters are described herein. The targeting sequence is sufficiently complementary to an endogenous sequence to permit homologous recombination of the promoter-targeting sequence with the endogenous sequence. The targeting sequence will be sufficiently near the 5' end of the desired endogenous polynucleotide sequence so the promoter will be operably linked to the endogenous sequence upon homologous recombination.

The promoter and the targeting sequences can be amplified using PCR. Preferably, the amplified promoter contains distinct restriction enzyme sites on the 5' and 3' ends. Preferably, the 3' end of the first targeting sequence contains the same restriction enzyme site as the 5' end of the amplified promoter and the 5' end of the second targeting sequence contains the same restriction site as the 3' end of the amplified promoter. The amplified promoter and targeting sequences are digested and ligated together

The promoter-targeting sequence construct is delivered to the cells, either as naked polynucleotide, or in conjunction with transfection-facilitating agents, such as liposomes, viral sequences, viral particles, whole viruses, lipofection, precipitating agents, etc., described in more detail above. The P promoter-targeting sequence can be delivered by any method, included direct needle injection, intravenous injection, topical administration, catheter infusion, particle accelerators, etc. The methods are described in more detail below.

The promoter-targeting sequence construct is taken up by cells. Homologous recombination between the construct and the endogenous sequence takes place, such that an endogenous sequence is placed under the control of the promoter. The promoter then drives the expression of the endogenous sequence.

The polynucleotides encoding polypeptides of the present invention may be administered along with other polynucleotides encoding other angiongenic proteins. Angiogenic proteins include, but are riot limited to, acidic and basic fibroblast growth factors, VEGF-1, VEGF-2 (VEGF-C), VEGF-3 (VEGF-B), epidermal growth factor alpha and beta, platelet-derived endothelial cell growth factor, platelet-derived growth factor, tumor necrosis factor alpha, hepatocyte growth factor, insulin like growth factor, colony stimulating factor, macrophage colony stimulating factor, granulocyte/macrophage colony stimulating factor, and nitric oxide synthase.

Preferably, the polynucleotide encoding a polypeptide of the invention contains a secretory signal sequence that facilitates secretion of the protein. Typically, the signal sequence is positioned in the coding region of the polynucleotide to be expressed towards or at the 5' end of the coding region. The signal sequence may be homologous or heterologous to the polynucleotide of interest and may be homologous or heterologous to the cells to be transfected. Additionally, the signal sequence may be chemically synthesized using methods known in the art.

Any mode of administration of any of the above-described polynucleotides constructs can be used so long as the mode results in the expression of one or more molecules in an amount sufficient to provide a therapeutic effect. This includes direct needle injection, systemic injection, catheter infusion, biolistic injectors, particle accelerators (i.e., "gene guns"), gelfoam sponge depots, other commercially available depot materials, osmotic pumps (e.g., Alza minipumps), oral or suppositorial solid (tablet or pill) pharmaceutical formulations, and decanting or topical applications during surgery. For example, direct injection of naked calcium phosphate-precipitated plasmid into rat liver and rat spleen or a protein-coated plasmid into the portal vein has resulted in gene expression of the foreign gene in the rat livers. (Kaneda et al., Science, 243:375 (1989)).

A preferred method of local administration is by direct injection. Preferably, a recombinant molecule of the present invention complexed with a delivery vehicle is administered by direct injection into or locally within the area of arteries. Administration of a composition locally within the area of arteries refers to injecting the composition centimeters and preferably, millimeters within arteries.

Another method of local administration is to contact a polynucleotide construct of the present invention in or around a surgical wound. For example, a patient can undergo surgery and the polynucleotide construct can be coated on the surface of tissue inside the wound or the construct can be injected into areas of tissue inside the wound.

Therapeutic compositions useful in systemic administration, include recombinant molecules of the present invention complexed to a targeted delivery vehicle of the present invention. Suitable delivery vehicles for use with systemic administration comprise liposomes comprising ligands for targeting the vehicle to a particular site.

Preferred methods of systemic administration, include intravenous injection, aerosol, oral and percutaneous (topical) delivery. Intravenous injections can be performed using methods standard in the art. Aerosol delivery can also be performed using methods standard in the art (see, for example, Stribling et al., Proc. Natl. Acad. Sci. USA, 189:11277-11281 (1992), which is incorporated herein by reference). Oral delivery can be performed by complexing a polynucleotide construct of the present invention to a carrier capable of withstanding degradation by digestive enzymes in the gut of an animal. Examples of such carriers, include plastic capsules or tablets, such as those known in the art. Topical delivery can be performed by mixing a polynucleotide construct of the present invention with a lipophilic reagent (e.g., DMSO) that is capable of passing into the skin.

Determining an effective amount of substance to be delivered can depend upon a number of factors including, for example, the chemical structure and biological activity of the substance, the age and weight of the animal, the precise condition requiring treatment and its severity, and the route of administration. The frequency of treatments depends upon a number of factors, such as the amount of polynucleotide constructs administered per dose, as well as the health and history of the subject. The precise amount, number of doses, and timing of doses will be determined by the attending physician or veterinarian. Therapeutic compositions of the present invention can be administered to any animal, preferably to mammals and birds. Preferred mammals include humans, dogs, cats, mice, rats, rabbits sheep, cattle, horses and pigs, with humans being particularly

### Biological Activities

The polynucleotides or polypeptides, or agonists or antagonists of the present invention can be used in assays to test for one or more biological activities. If these polynucleotides and polypeptides do exhibit activity in a particular assay, it is likely that these molecules may be involved in the diseases associated with the biological activity. Thus, the polynucleotides or polypeptides, or agonists or antagonists could be used to treat the associated disease.

### Immune Activity

Polynucleotides, polypeptides, antibodies, and/or agonists or antagonists of the present invention may be useful in treating, preventing, and/or diagnosing diseases, disorders, and/or conditions of the immune system, by, for example, activating or inhibiting the proliferation, differentiation, or mobilization (chemotaxis) of immune cells, Immune cells develop through a process called hematopoiesis, producing myeloid (platelets, red blood cells, neutrophils, and macrophages) and lymphoid (B and T lymphocytes) cells from pluripotent stem cells. The etiology of these immune diseases, disorders, and/or conditions may be genetic, somatic, such as cancer and some autoimmune diseases, acquired (e.g., by chemotherapy or toxins), or infectious. Moreover, polynucleotides, polypeptides, antibodies, and/or agonists or antagonists of the present invention can be used as a marker or detector of a particular immune system disease or disorder.

Polynucleotides, polypeptides, antibodies, and/or agonists or antagonists of the present invention may be useful in treating, preventing, and/or diagnosing diseases, disorders, and/or conditions of hematopoietic cells. Polynucleotides, polypeptides, antibodies, and/or agonists or antagonists of the present invention could be used to increase differentiation and proliferation of hematopoietic cells, including the pluripotent stem cells, in an effort to treat or prevent those diseases, disorders, and/or conditions associated with a decrease in certain (or many) types hematopoietic cells. Examples of immunologic deficiency syndromes include, but are not limited to: blood protein diseases, disorders, and/or conditions (e.g., agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, polynucleotides, polypeptides, antibodies, and/or agonists or antagonists of the present invention could also be used to modulate hemostatic (the stopping of bleeding) or thrombolytic activity (clot formation). For example, by increasing hemostatic or thrombolytic activity, polynucleotides or polypeptides, and/or agonists or antagonists of the present invention could be used to treat or prevent blood coagulation diseases, disorders, and/or conditions (e.g., afibrinogenemia, factor deficiencies), blood platelet diseases, disorders, and/or conditions (e.g., thrombocytopenia), or wounds resulting from trauma, surgery, or other causes. Alternatively, polynucleotides, polypeptides, antibodies, and/or agonists or antagonists of the present invention that can decrease hemostatic or thrombolytic activity could be used to inhibit or dissolve clotting. These molecules could be important in the treatment or prevention of heart attacks (infarction), strokes, or scarring.

The polynucleotides, polypeptides, antibodies, and/or agonists or antagonists of the present invention may be useful in treating, preventing, and/or diagnosing autoimmune disorders. Many autoimmune disorders result from inappropriate recognition of self as foreign material by immune cells. This inappropriate recognition results in an immune response leading to the destruction of the host tissue. Therefore, the administration of polynucleotides and polypeptides of the invention that can inhibit an immune response, particularly the proliferation, differentiation, or chemotaxis of T-cells, may be an effective therapy in preventing autoimmune disorders.

Autoimmune diseases or disorders that may be treated, prevented, and/or diagnosed by polynucleotides, polypeptides, antibodies, and/or agonists or antagonists of the present invention include, but are not limited to, one or more of the following: autoimmune hemolytic anemia, autoimmune neonatal thiombocytopenia, idiopathic thrombocytopenia purpura, autoimmunocytopenia, hemolytic anemia, antiphospholipid syndrome, dermatitis, allergic encephalomyelitis, myocarditis, relapsing polychondritis, rheumatic heart disease, glomerulonephritis (e.g, IgA nephropathy), Multiple Sclerosis, Neuritis, Uveitis Ophthalmia, Polyendocrinopathies, Purpura (e.g., Henloch-Scoenlein purpura), Reiter's Disease, Stiff-Man Syndrome, Autoimmune Pulmonary Inflammation, Autism, Guillain-Barre Syndrome, insulin dependent diabetes mellitis, and autoimmune inflammatory eye, autoimmune thyroiditis, hypothyroidism (i.e., Hashimoto's thyroiditis, systemic lupus erhythematosus, Goodpasture's syndrome, Pemphigus, Receptor autoimmunities such as, for example, (a) Graves' Disease, (b) Myasthenia Gravis, and (c) insulin resistance, autoimmune hemolytic anemia, autoimmune thrombocytopenic purpura, rheumatoid arthritis, schleroderma with anti-collagen antibodies, mixed connective tissue disease, polymyositis/dermatomyositis, pernicious anemia, idiopathic Addison's disease, infertility, glomerulonephritis such as primary glomerulonephritis and IgA nephropathy, bullous pemphigoid, Sjogren's syndrome, diabetes millitus, and adrenergic drug resistance (including adrenergic drug resistance with asthma or cystic fibrosis), chronic active hepatitis, primary biliary cirrhosis, other endocrine gland failure, vitiligo, vasculitis, post-MI, cardiotomy syndrome, urticaria, atopic dermatitis, asthma, inflammatory myopathies, and other inflammatory, granulamatous, degenerative, and atrophic disorders:

- Additional autoimmune disorders (that are probable) that may be treated, prevented, and/or diagnosed with the compositions of the invention include, but are not limited to, rheumatoid arthritis (often characterized, e.g., by immune complexes in joints), scleroderma with anti-collagen antibodies (often characterized, e.g., by nucleolar and other nuclear antibodies), mixed connective tissue disease (often characterized, e.g., by antibodies to extractable nuclear antigens (e.g., ribonucleoprotein)), polymyositis (often characterized, e.g., by nonhistone ANA), pernicious anemia (often characterized, e.g., by antiparietal cell, microsomes, and intrinsic factor antibodies), idiopathic Addison's disease (often characterized, e.g., by humoral and cell-mediated adrenal cytotoxicity, infertility (often characterized, e.g., by antispermatozoal antibodies), glomerulonephritis (often characterized, e.g., by glomerular basement membrane antibodies or immune complexes), bullous pemphigoid (often characterized, e.g., by IgG and complement in basement membrane), Sjogren's syndrome (often characterized, e.g., by multiple tissue antibodies, and/or a specific nonhistone ANA (SS-B)), diabetes millitus (often characterized, e.g., by cell-mediated and humoral islet cell antibodies), and adrenergic drug resistance (including adrenergic drug resistance with asthma or cystic fibrosis) (often characterized, e.g., by beta-adrenergic receptor antibodies).

Additional autoimmune disorders (that are possible) that may be treated, prevented, and/or diagnosed with the compositions of the invention include, but are not limited to, chronic active hepatitis (often characterized, e.g., by smooth muscle antibodies), primary biliary cirrhosis (often characterized, e.g., by mitchondrial antibodies), other endocrine gland failure (often characterized, e.g., by specific tissue antibodies in some cases), vitiligo (often characterized, e.g., by melanocyte antibodies), vasculitis (often characterized, e.g., by Ig and complement in vessel walls and/or low serum complement), post-MI (often characterized, e.g., by myocardial antibodies), cardiotomy syndrome (often characterized, e.g., by myocardial antibodies), urticaria (often characterized, e.g., by IgG and IgM antibodies to IgE), atopic dermatitis (often characterized, e.g., by IgG and IgM antibodies to IgE), asthma (often characterized, e.g., by IgG and IgM antibodies to IgE), and many other inflammatory, granulamatous, degenerative, and atrophic disorders.

In a preferred embodiment, the autoimmune diseases and disorders and/or conditions associated with the diseases and disorders recited above are treated, prevented, and/or diagnosed using for example, antagonists or agonists, polypeptides or polynucleotides, or antibodies of the present invention.

In a preferred embodiment polynucleotides, polypeptides, antibodies, and/or agonists or antagonists of the present invention could be used as an agent to boost immunoresponsiveness among B cell and/or T cell immunodeficient individuals.

B cell immunodeficiencies that may be ameliorated or treated by administering the polypeptides or polynucleotides of the invention, and/or agonists thereof, include, but are not limited to, severe combined immunodeficiency (SCID)-X linked, SCID-autosomal, adenosine deaminase deficiency (ADA deficiency), X-linked agammaglobulinemia (XLA), Bruton's disease, congenital agammaglobulinemia, X-linked infantile agammaglobulinemia, acquired agammaglobulinemia, adult onset agammaglobulinemia, late-onset agammaglobulinemia, dysgammaglobulinemia, hypogammaglobulinemia, transient hypogammaglobulinemia of infancy, unspecified hypogammaglobulinemia, agammaglobulinemia, common variable immunodeficiency (CVI) (acquired), Wiskott-Aldrich Syndrome (WAS), X-linked immunodeficiency with hyper IgM, non X-linked immunodeficiency with hyper IgM, selective IgA deficiency, IgG subclass deficiency (with or without IgA deficiency), antibody deficiency with normal or elevated Igs, immunodeficiency with thymoma, Ig heavy chain deletions, kappa chain deficiency, B cell lymphoproliferative disorder (BLPD), selective IgM immunodeficiency, recessive agammaglobulinemia (Swiss type), reticular dysgenesis, neonatal neutropenia, severe congenital leukopenia, thymic alymophoplasia-aplasia or dysplasia with immunodeficiency, ataxia-telangiectasia, short limbed dwarfism, X-linked lymphoproliferative syndrome (XLP), Nezelof syndrome-combined immunodeficiency with Igs, purine nucleoside phosphorylase deficiency (PNP), MHC Class II deficiency (Bare Lymphocyte Syndrome) and severe combined immunodeficiency.

T cell deficiencies that may be ameliorated or treated by administering the polypeptides or polynucleotides of the invention, and/or agonists thereof include, but are not limited to, for example, DiGeorge anomaly, thymic hypoplasia, third and fourth pharyngeal pouch syndrome, 22q11.2 deletion, chronic mucocutaneous candidiasis, natural killer cell deficiency (NK), idiopathic CD4+ T-lymphocytopenia, immunodeficiency with predominant T cell defect (unspecified), and unspecified immunodeficiency of cell mediated immunity. In specific embodiments, DiGeorge anomaly or conditions associated with DiGeorge anomaly are ameliorated or treated by, for example, administering the polypeptides or polynucleotides of the invention, or antagonists or agonists thereof.

Other immunodeficiencies that may be ameliorated or treated by administering polypeptides or polynucleotides of the invention, and/or agonists thereof, include, but are not limited to, severe combined immunodeficiency (SCID; e.g., X-linked SCID, autosomal SCID, and adenosine deaminase deficiency), ataxia-telangiectasia, Wiskott-Aldrich syndrome, short-limber dwarfism, X-linked lymphoproliferative syndrome (XLP), Nezelof syndrome (e.g., purine nucleoside phosphorylase deficiency), MHC Class II deficiency. In specific embodiments, ataxia-telangiectasia or conditions associated with ataxia-telangiectasia are ameliorated or treated by administering the polypeptides or polynucleotides of the invention, and/or agonists thereof.

In a specific preferred embodiment, rheumatoid arthritis is treated, prevented, and/or diagnosed using polynucleotides, polypeptides, antibodies, and/or agonists or antagonists of the present invention. In another specific preferred embodiment, systemic lupus erythemosus is treated, prevented, and/or diagnosed using polynucleotides, polypeptides, antibodies, and/or agonists or antagonists of the present invention. In another specific preferred embodiment, idiopathic thrombocytopenia purpura is treated, prevented, and/or diagnosed using polynucleotides, polypeptides, antibodies, and/or agonists or antagonists of the present invention. In another specific preferred embodiment IgA nephropathy is treated, prevented, and/or diagnosed using polynucleotides, polypeptides, antibodies, and/or agonists or antagonists of the present invention. In a preferred embodiment, the autoimmune diseases and disorders and/or conditions associated with the diseases and disorders recited above are treated, prevented, and/or diagnosed using antibodies against the protein of the invention.

Similarly, allergic reactions and conditions, such as asthma (particularly allergic asthma) or other respiratory problems, may also be treated, prevented, and/or diagnosed using polypeptides, antibodies, or polynucleotides of the invention, and/or agonists or antagonists thereof. Moreover, these molecules can be used to treat, prevent, and/or diagnose anaphylaxis, hypersensitivity to an antigenic molecule, or blood group incompatibility.

Moreover, inflammatory conditions may also be treated, diagnosed, and/or prevented with polynucleotides, polypeptides, antibodies, and/or agonists or antagonists of the present invention. Such inflammatory conditions include, but are not limited to, for example, respiratory disorders (such as, e.g., asthma and allergy); gastrointestinal disorders (such as, e.g., inflammatory bowel disease); cancers (such as, e.g., gastric, ovarian, lung, bladder, liver, and breast); CNS disorders (such as, e.g., multiple sclerosis, blood-brain barrier permeability, ischemic brain injury and/or stroke, traumatic brain injury, neurodegenerative disorders (such as, e.g., Parkinson's disease and "Alzheimer's disease), AIDS-related dementia, and prion disease); cardiovascular disorders' (such as, e.g., atherosclerosis, myocarditis, cardiovascular disease, and cardiopulmonary bypass complications); as well as many additional diseases, conditions, and disorders that are characterized by inflammation (such as, e.g., chronic hepatitis (B and C), rheumatoid arthritis, gout, trauma, septic shock, pancreatitis, sarcoidosis, dermatitis, renal ischemia-reperfusion injury, Grave's disease, systemic lupus erythermatosis, diabetes mellitus (i.e., type 1 diabetes), and allogenic transplant rejection).

In specific embodiments, polypeptides, antibodies, or polynucleotides of the invention, and/or agonists or antagonists thereof, are useful to treat, diagnose, and/or prevent transplantation rejections, graft-versus-host disease, autoimmune and inflammatory diseases (e.g., immune complex-induced vasculitis, glomerulonephritis, hemolytic anemia, myasthenia gravis, type II collagen-induced arthritis, experimental allergic and hyperacute xenograft rejection, rheumatoid arthritis, and systemic lupus erythematosus (SLE). Organ rejection occurs by host immune cell destruction of the transplanted tissue through an immune response. Similarly, an immune response is also involved in GVHD, but, in this case, the foreign transplanted immune cells destroy the host tissues. Polypeptides, antibodies, or polynucleotides of the invention, and/or agonists or antagonists thereof, that inhibit an immune response, particularly the activation, proliferation, differentiation, or chemotaxis of T-cells, may be an effective therapy in preventing organ rejection or GVHD.

Similarly, polynucleotides, polypeptides, antibodies, and/or agonists or antagonists of the present invention may also be used to modulate and/or diagnose inflammation. For example, since polypeptides, antibodies, or polynucleotides of the invention, and/or agonists or antagonists of the invention may inhibit the activation, proliferation and/or differentiation of cells involved in an inflammatory response, these molecules can be used to treat, diagnose, or prognose, inflammatory conditions, both chronic and acute conditions, including, but not limited to, inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, and resulting from over production of cytokines (e.g., TNF or IL-1.).

Polypeptides, antibodies, polynucleotides and/or agonists or antagonists of the invention can be used to treat, detect, and/or prevent infectious agents. For example, by increasing the immune response, particularly increasing the proliferation activation and/or differentiation of B and/or T cells, infectious diseases may be treated, detected, and/or prevented. The immune response may be increased by either enhancing an existing immune response, or by initiating a new immune response. Alternatively, polynucleotides, polypeptides, antibodies, and/or agonists or antagonists of the present invention may also directly inhibit the infectious agent (refer to section of application listing infectious agents, etc), without necessarily eliciting an immune response.

Additional preferred embodiments of the invention include, but are not limited to, the use of polypeptides, antibodies, polynucleotides and/or agonists or antagonists in the following applications:

Administration to an animal (e.g., mouse, rat, rabbit, hamster, guinea pig, pigs, micro-pig, chicken, camel, goat, horse, cow, sheep, dog, cat, non-human primate, and human, most preferably human) to boost the immune system to produce increased quantities of one or more antibodies (e.g., IgG, IgA, IgM, and IgE), to induce higher affinity antibody production (e.g., IgG, IgA, IgM, and IgE), and/or to increase an immune response.

Administration to an animal (including, but not limited to, those listed above, and also including transgenic animals) incapable of producing functional endogenous antibody molecules or having an otherwise compromised endogenous immune system, but which is capable of producing human immunoglobulin molecules by means of a reconstituted or partially reconstituted immune system from another animal (see, e.g., published PCT Application Nos. WO98/24893, WO/9634096, WO/9633735, and WO/9110741.

A vaccine adjuvant that enhances immune responsiveness to specific antigen.

An adjuvant to enhance tumor-specific immune responses.

An adjuvant to enhance anti-viral immune responses. Anti-viral immune responses that may be enhanced using the compositions of the invention as an adjuvant, include virus and virus associated diseases or symptoms described herein or otherwise known in the art. In specific embodiments, the compositions of the invention are used as an adjuvant to enhance an immune response to a virus, disease, or symptom selected from the group consisting of: AIDS, meningitis, Dengue, EBV, and hepatitis (e.g., hepatitis B). In another specific embodiment, the compositions of the invention are used as an adjuvant to enhance an immune response to a virus, disease, or symptom selected from the group consisting of: HIV/AIDS, Respiratory syncytial virus, Dengue, Rotavirus, Japanese B encephalitis, Influenza A and B, Parainfluenza, Measles, Cytomegalovirus, Rabies, Junin, Chikungunya, Rift Valley fever, Herpes simplex, and yellow fever.

An adjuvant to enhance anti-bacterial or anti-fungal immune responses. Antibacterial or anti-fungal immune responses that may be enhanced using the compositions of the invention as an adjuvant, include bacteria or fungus and bacteria or fungus associated diseases or symptoms described herein or otherwise known in the art. In specific embodiments, the compositions of the invention are used as an adjuvant to enhance an immune response to a bacteria or fungus, disease, or symptom selected from the group consisting of: tetanus, Diphtheria, botulism, and meningitis type B. In another specific embodiment, the compositions of the invention are used as an adjuvant to enhance an immune response to a bacteria or fungus, disease, or symptom selected from the group consisting of: *Vibrio cholerae, Mycobacterium leprae, Salmonella typhi, Salmonella paratyphi, Meisseria meningititlis, Streptococcus pneumoniae,* Group B streptococcus, *Shigella spp.,* Enterotoxigenic *Escherichia coli,* Enterohemorrhagic *E. coli, Borrelia burgdorferi,* and Plasmodium (malaria).

An adjuvant to enhance anti-parasitic immune responses. Anti-parasitic immune responses that may be enhanced using the compositions of the invention as an adjuvant, include parasite and parasite associated diseases or symptoms described herein or otherwise known in the art. In specific embodiments, the compositions of the invention are used as an adjuvant to enhance an immune response to a parasite. In another specific embodiment, the compositions of the invention are used as an adjuvant to enhance an immune response to Plasmodium (malaria).

As a stimulator of B cell responsiveness to pathogens.

As an activator of T cells.

As an agent that elevates the immune status of an individual prior to their receipt of immunosuppressive therapies.

As an agent to induce higher affinity antibodies.

As an agent to increase serum immunoglobulin concentrations.

As an agent to accelerate recovery of immunocompromised individuals.

As an agent to boost immunoresponsiveness among aged populations.

As an immune system enhancer prior to, during, or after bone marrow transplant and/or other transplants (e.g., allogeneic or xenogeneic organ transplantation). With respect to transplantation, compositions of the invention may be administered prior to, concomitant with, and/or after transplantation. In a specific embodiment, compositions of the invention are administered after transplantation, prior to the beginning of recovery of T-cell populations. In another specific embodiment, compositions of the invention are first administered after transplantation after the beginning of recovery of T cell populations, but prior to full recovery of B cell populations.

As an agent to boost immunoresponsiveness among individuals having an acquired loss of B cell function. Conditions resulting in an acquired loss of B cell function that may be ameliorated or treated by administering the polypeptides, antibodies, polynucleotides and/or agonists or antagonists thereof, include, but are not limited to, HIV Infection, AIDS, bone marrow transplant, and B cell chronic lymphocytic leukemia (CLL).

As an agent to boost immunoresponsiveness among individuals having a temporary immune deficiency. Conditions resulting in a temporary immune deficiency that may be ameliorated or treated by administering the polypeptides, antibodies, polynucleotides and/or agonists or antagonists thereof, include, but are not limited to, recovery from viral infections (e.g., influenza), conditions associated with malnutrition, recovery from infectious mononucleosis, or conditions associated with stress, recovery from measles, recovery from blood transfusion, recovery from surgery.

As a regulator of antigen presentation by monocytes, dendritic cells, and/or B-cells. In one embodiment, polynucleotides, polypeptides, antibodies, and/or agonists or antagonists of the present invention enhance antigen presentation or antagonizes antigen presentation in vitro or in vivo. Moreover, in related embodiments, said enhancement or antagonization of antigen presentation may be useful as an anti-tumor treatment or to modulate the immune system.

As an agent to direct an individuals immune system towards development of a humoral response (i.e. TH2) as opposed to a TH1 cellular response.

As a means to induce tumor proliferation and thus make it more susceptible to anti-neoplastic agents. For example, multiple myeloma is a slowly dividing disease and is thus refractory to virtually all anti-neoplastic regimens. If these cells were forced to proliferate more rapidly their susceptibility profile would likely change.

As a stimulator of B cell production in pathologies such as AIDS, chronic lymphocyte disorder and/or Common Variable Immunodificiency.

As a therapy for generation and/or regeneration of lymphoid tissues following surgery, trauma or genetic defect.

As a gene-based therapy for genetically inherited disorders resulting in immuno-incompetence such as observed among SCID patients.

As ah antigen for the generation of antibodies to inhibit or enhance immune mediated responses against polypeptides of the invention.

As a means of activating T cells.

As a means of activating monocytes/macrophages to defend against parasitic diseases that effect monocytes such as Leshmania.

As pretreatment of bone marrow samples prior to transplant. Such treatment would increase B cell representation and thus accelerate recover.

As a means of regulating secreted cytokines that are elicited by polypeptides of the invention.

Additionally, polypeptides or polynucleotides of the invention, and/or agonists thereof, may be used to treat or prevent IgE-mediated allergic reactions. Such allergic reactions include, but are not limited to, asthma, rhinitis, and eczema.

All of the above described applications as they may apply to veterinary medicine.

Antagonists of the invention include, for example, binding and/or inhibitory antibodies, antisense nucleic acids, or ribozymes. These would be expected to reverse many of the activities of the ligand described above as well as find clinical or practical application as:

A means of blocking various aspects of immune responses to foreign agents or self. Examples include autoimmune disorders such as lupus, and arthritis, as well as immunoresponsiveness to skin allergies, inflammation, bowel disease, injury and pathogens.

A therapy for preventing the B cell proliferation and Ig secretion associated with autoimmune diseases such as idiopathic thrombocytopenic purpura, systemic lupus erythramatosus and MS.

An inhibitor of B and/or T cell migration in endothelial cells. This activity disrupts tissue architecture or cognate responses and is useful, for example in disrupting immune responses, and blocking sepsis

An inhibitor of graft versus host disease or transplant rejection.

A therapy for B cell and/or T cell malignancies such as ALL, Hodgkins disease, non-Hodgkins lymphoma, Chronic lymphocyte leukemia, plasmacytomas, multiple myeloma, Burkitt's lymphoma, and EBV-transformed diseases.

A therapy for chronic hypergammaglobulinemeia evident in such diseases as monoclonalgammopathy of undetermined significance (MGUS), Waldenstrom's disease, related idiopathic monoclonalgammopathies, and plasmacytomas.

A therapy for decreasing cellular proliferation of Large B-cell Lymphomas.

A means of decreasing the involvement of B cells and Ig associated with Chronic Myelogenous Leukemia.

An immunosuppressive agent(s).

Polynucleotides, polypeptides, antibodies, and/or agonists or antagonists of the present invention may be used to modulate IgE concentrations in vitro or in vivo.

In another embodiment, administration of polypeptides, antibodies, polynucleotides and/or agonists or antagonists of the invention, may be used to treat or prevent IgE-mediated allergic reactions including, but not limited to, asthma, rhinitis, and eczema.

The agonists and antagonists may be employed in a composition with a pharmaceutically acceptable carrier, e.g., as described herein.

The agonists or antagonists may be employed for instance to inhibit polypeptide chemotaxis and activation of macrophages and their precursors, and of neutrophils, basophils, B lymphocytes and some T-cell subsets, e.g., activated and CD8 cytotoxic T cells and natural killer cells, in certain auto-immune and chronic inflammatory and infective diseases. Examples of autoimmune diseases are described herein and include multiple sclerosis, and insulin-dependent diabetes. The antagonists or agonists may also be employed to treat infectious diseases including silicosis, sarcoidosis, idiopathic pulmonary fibrosis by, for example, preventing the recruitment and activation of mononuclear phagocytes. They may also be employed to treat idiopathic hyper-eosinophilic syndrome by, for example, preventing eosinophil production and migration. The antagonists or agonists or may also be employed for treating atherosclerosis, for example, by preventing monocyte infiltration in the artery wall.

Antibodies against polypeptides of the invention may be employed to treat ARDS.

Agonists and/or antagonists of the invention also have uses in stimulating wound and tissue repair, stimulating angiogenesis, stimulating the repair of vascular or lymphatic diseases or disorders. Additionally, agonists and antagonists of the invention may be used to stimulate the regeneration of mucosal surfaces.

In a specific embodiment, polynucleotides or polypeptides, and/or agonists thereof are used to treat or prevent a disorder characterized by primary or acquired immunodeficiency, deficient serum immunoglobulin production, recurrent infections, and/or immune system dysfunction. Moreover, polynucleotides or polypeptides, and/or agonists thereof may be used to treat or prevent infections of the joints, bones, skin, and/or parotid glands, blood-borne infections (e.g., sepsis, meningitis, septic arthritis, and/or osteomyelitis), autoimmune diseases (e.g., those disclosed herein), inflammatory disorders, and malignancies, and/or any disease or disorder or condition associated with these infections, diseases, disorders and/or malignancies) including, but not limited to, CVID, other primary immune deficiencies, HIV disease, CLL, recurrent bronchitis, sinusitis, otitis media, conjunctivitis, pneumonia, hepatitis, meningitis, herpes zoster (e.g., severe herpes zoster), and/or pneumocystis carnii.

In another embodiment, polynucleotides, polypeptides, antibodies, and/or agonists or antagonists of the present invention are used to treat, and/or diagnose an individual having common variable immunodeficiency disease ("CVID"; also known as "acquired agammaglobulinemia" and "acquired hypogammaglobulinemia") or a subset of this disease.

In a specific embodiment, polynucleotides, polypeptides, antibodies, and/or agonists or antagonists of the present invention may be used to treat, diagnose, and/or prevent (1) cancers or neoplasms and (2) autoimmune cell or tissue-related cancers or neoplasms. In a preferred embodiment, polynucleotides, polypeptides, antibodies, and/or agonists or antagonists of the present invention conjugated to a toxin or a radioactive isotope, as described herein, may be used to treat, diagnose, and/or prevent acute myelogeneous leukemia. In a further preferred embodiment, polynucleotides, polypeptides, antibodies, and/or agonists or antagonists of the present invention conjugated to a toxin or a radioactive isotope, as described herein, may be used to treat, diagnose, and/or prevent, chronic myelogeneous leukemia, multiple myeloma, non-Hodgkins lymphoma, and/or Hodgkins disease.

In another specific embodiment, polynucleotides or polypeptides, and/or agonists or antagonists of the invention may be used to treat, diagnose, prognose, and/or prevent selective IgA deficiency, myeloperoxidase deficiency, C2 deficiency; ataxia-telangiectasia, DiGeorge anomaly, common variable immunodeficiency (CVI), X-linked agamrnaglobulinemia, severe combined immunodeficiency (SCID), chronic granulomatous disease (CGD), and Wiskott-Aldrich syndrome.

Examples of autoimmune disorders that can be treated or detected are described above and also include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

In a preferred embodiment, the autoimmune diseases and disorders and/or conditions associated with the diseases and disorders recited above are treated, prognosed, prevented, and/or diagnosed using antibodies against the polypeptide of the invention.

As an agent to boost immunoresponsiveness among B cell immunodeficient individuals, such as, for example, an individual who has undergone a partial or complete splenectomy.

Additionally, polynucleotides, polypeptides, and/or antagonists of the invention may affect apoptosis, and therefore, would be useful in treating a number of diseases associated with increased cell survival or the inhibition of apoptosis. For example, diseases associated with increased cell survival or the inhibition of apoptosis that could be treated or detected by polynucleotides, polypeptides, and/or antagonists of the invention, include cancers (such as follicular lymphomas, carcinomas with p53 mutations, and hormone-dependent tumors, including, but not limited to colon cancer, cardiac tumors, pancreatic cancer, melanoma, retinoblastoma, glioblastoma, lung cancer, intestinal cancer, testicular cancer; stomach cancer, neuroblastoma, myxoma, myoma, lymphoma, endothelioma, osteoblastoma, osteoclastoma, osteosarcoma, chondrosarcoma, adenoma, breast cancer, prostate cancer, Kaposi's sarcoma and ovarian cancer); autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) and viral infections (such as herpes viruses, pox viruses and adenoviruses), inflammation, graft v. host disease, acute graft rejection, and chronic graft rejection. In preferred embodiments, polynucleotides, polypeptides, and/or antagonists of the invention are used to inhibit growth, progression, and/or metastisis of cancers, in particular those listed above.

Additional diseases or conditions associated with increased cell survival that could be treated or detected by polynucleotides, polypeptides, and/or antagonists of the invention, include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

Diseases associated with increased apoptosis that could be treated or detected by polynucleotides, polypeptides, and/or antagonists of the invention, include AIDS; neurodegenerative disorders (such as Alzheimer's disease, Parkinson's disease, Amyotrophic lateral sclerosis, Retinitis pigmentosa, Cerebellar degeneration and brain tumor or prior associated disease); 'autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) myelodysplastic syndromes (such as aplastic anemia), graft v. host disease, ischemic injury (such as that caused by myocardial infarction, stroke and reperfusion injury), liver injury (e.g., hepatitis related liver injury, ischemia/reperfusion injury, cholestosis (bile duct injury) and liver cancer); toxin-induced liver disease (such as that caused by alcohol), septic shock, cachexia and anorexia.

Hyperproliferative diseases and/or disorders that could be detected and/or treated by polynucleotides, polypeptides, and/or antagonists of the invention, include, but are not limited to neoplasms located in the: liver, abdomen, bone, breast, digestive system, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by polynucleotides, polypeptides, and/or antagonists of the invention. Examples of such hyperproliferative disorders include, but are not limited to:
hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Hyperproliferative Disorders

A polynucleotides or polypeptides, or agonists or antagonists of the invention can be used to treat, prevent, and/or diagnose hyperproliferative diseases, disorders, and/or conditions, including neoplasms. A polynucleotides or polypeptides, or agonists or antagonists of the present invention may inhibit the proliferation of the disorder through direct or indirect interactions. Alternatively, a polynucleotides or polypeptides, or agonists or antagonists of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative diseases, disorders, and/or conditions can be treated, prevented, and/or diagnosed. This immune response may be increased by either enhancing an existing immune response, or by initiating a new immune response. Alternatively, decreasing an immune response may also be a method of treating, preventing, and/or diagnosing hyperproliferative diseases, disorders, and/or conditions, such as a chemotherapeutic agent.

Examples of hyperproliferative diseases, disorders, and/or conditions that can be treated, prevented, and/or diagnosed by polynucleotides or polypeptides, or agonists or antagonists of the present invention include, but are not limited to neoplasms located in the: colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative diseases, disorders, and/or conditions can also be treated, prevented, and/or diagnosed by a polynucleotides or polypeptides, or agonists or antagonists of the present invention. Examples of such hyperproliferative diseases, disorders, and/or conditions include, but are not limited to:
hypergammaglobulinemia, lymphoproliferative diseases, disorders, and/or conditions, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

One preferred embodiment utilizes polynucleotides of the present invention to inhibit aberrant cellular division, by gene therapy using the present invention, and/or protein fusions or fragments thereof.

Thus, the present invention provides a method for treating or preventing cell proliferative diseases, disorders, and/or conditions by inserting into an abnormally proliferating cell a polynucleotide of the present invention, wherein said polynucleotide represses said expression.

Another embodiment of the present invention provides a method of treating or preventing cell-proliferative diseases, disorders, and/or conditions in individuals comprising administration of one or more active gene copies of the present invention to an abnormally proliferating cell or cells. In a preferred embodiment, polynucleotides of the present invention is a DNA construct comprising a recombinant expression vector effective in expressing a DNA sequence encoding said polynucleotides. In another preferred embodiment of the present invention, the DNA construct encoding the poynucleotides of the present invention is inserted into cells to be treated utilizing a retrovirus, or more preferrably an adenoviral vector (See G J. Nabel, et. al., PNAS 1999 96: 324-326, which is hereby incorporated by reference). In a most preferred embodiment, the viral vector is defective and will not transform non-proliferating cells, only proliferating cells. Moreover, in a preferred embodiment, the polynucleotides of the present invention inserted into proliferating cells either alone, or in combination with or fused to other polynucleotides, can then be modulated via an external stimulus (i.e. magnetic, specific small molecule, chemical, or drug administration, etc.), which acts upon the promoter upstream of said polynucleotides to induce expression of the encoded protein product. As such the beneficial therapeutic affect of the present invention may be expressly modulated (i.e. to increase, decrease, or inhibit expression of the present invention) based upon said external stimulus.

Polynucleotides of the present invention may be useful in repressing expression of oncogenic genes or antigens. By "repressing expression of the oncogenic genes " is intended the suppression of the transcription of the gene, the degradation of the gene transcript (pre-message RNA), the inhibition of splicing, the destruction of the messenger RNA, the prevention of the post-translational modifications of the protein, the destruction of the protein, or the inhibition of the normal function of the protein.

For local administration to abnormally proliferating cells, polynucleotides of the present invention may be administered by any method known to those of skill in the art including, but not limited to transfection, electroporation, microinjection of cells, or in vehicles such as liposomes, lipofectin, or as naked polynucleotides, or any other method described throughout the specification. The polynucleotide of the present invention may be delivered by known gene delivery systems such as, but not limited to, retroviral vectors (Gilboa, J. Virology 44:845 (1982); Hocke, Nature 320:275 (1986); Wilson, et al., Proc. Natl. Acad. Sci. U.S.A. 85:3014), vaccinia virus system (Chakrabarty et al., Mol. Cell Biol. 5:3403 (1985) or other efficient DNA delivery systems (Yates et al., Nature 313:812 (1985)) known to those skilled in the art. These references are exemplary only and are hereby incorporated by reference. In order to specifically deliver or transfect cells which are abnormally proliferating and spare non-dividing cells, it is preferable to utilize a retrovirus, or adenoviral (as described in the art and elsewhere herein) delivery system known to those of skill in the art. Since host DNA replication is required for retroviral DNA to integrate and the retrovirus will be unable to self replicate due to the lack of the retrovirus genes needed for its life cycle. Utilizing such a retroviral delivery system for polynucleotides of the present invention will target said gene and constructs to abnormally proliferating cells and will spare the non-dividing normal cells.

The polynucleotides of the present invention may be delivered directly to cell proliferative disorder/disease sites in internal organs, body cavities and the like by use of imaging devices used to guide an injecting needle directly to the disease site. The polynucleotides of the present invention may also be administered to disease sites at the time of surgical intervention.

By "cell proliferative disease" is meant any human or animal disease or disorder, affecting any one or any combination of organs, cavities, or body parts, which is characterized by single or multiple local abnormal proliferations of cells, groups of cells, or tissues, whether benign or malignant.

Any amount of the polynucleotides of the present invention may be administered as long as it has a biologically inhibiting effect on the proliferation of the treated cells. Moreover, it is possible to administer more than one of the polyriucleotide of the present invention simultaneously to the same site. By "biologically inhibiting" is meant partial or total growth inhibition as well as decreases in the rate of proliferation or growth of the cells. The biologically inhibitory dose may be determined by assessing the effects of the polynucleotides of the present invention on target malignant or abnormally proliferating cell growth in tissue culture, tumor growth in animals and cell cultures, or any other method known to one of ordinary skill in the art.

The present invention is further directed to antibody-based therapies which involve administering of anti-polypeptides and anti-polynucleotide antibodies to a mammalian, preferably human, patient for treating, preventing, and/or diagnosing one or more of the described diseases, disorders, and/or conditions. Methods for producing anti-polypeptides and anti-polynucleotide antibodies polyclonal and monoclonal antibodies are described in detail elsewhere herein. Such antibodies may be provided in pharmaceutically acceptable compositions as known in the art or as described herein.

A summary of the ways in which the antibodies of the present invention may be used therapeutically includes binding polynucleotides or polypeptides of the present invention locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies of the present invention for diagnostic, monitoring or therapeutic purposes without undue experimentation.

In particular, the antibodies, fragments and derivatives of the present invention are useful for treating, preventing, and/or diagnosing a subject having or developing cell proliferative and/or differentiation diseases, disorders, and/or conditions as described herein. Such treatment comprises administering a single or multiple doses of the antibody, or a fragment, derivative, or a conjugate thereof.

The antibodies of this invention may be advantageously utilized in combination with other monoclonal or chimeric antibodies, or with lymphokines or hematopoietic growth factors, for example, 'which serve to increase the number or activity of effector cells which interact with the antibodies.

It is preferred to use high affinity and/or potent in vivo inhibiting and/or neutralizing antibodies against polypeptides or polynucleotides of the present invention, fragments or regions thereof, for both immunoassays directed to and therapy of diseases, disorders, and/or conditions related to polynucleotides or polypeptides, including fragements thereof, of the present invention. Such antibodies, fragments, or regions, will preferably have an affinity for polynucleotides or polypeptides, including fragements thereof. Preferred binding affinities include those with a dissociation constant or Kd less than 5X10⁻⁶M, 10⁻⁶M, SX10⁻⁷M, 10⁻⁷M, 5X10⁻⁸ M, 10⁻⁸M, 5X10⁻⁹M, 10⁻⁹M, 5X10⁻¹⁰M, 10⁻¹⁰M, 5X10⁻¹¹M, 10⁻¹¹M, 5X10⁻¹²M, 10⁻¹² M, 5X10⁻¹³M, 10⁻¹³M, 5X10⁻¹⁴M, 10⁻¹⁴M, 5X10⁻¹⁵M, and 10⁻¹⁵M.

Moreover, poiypeptides of the present invention are useful in inhibiting the angiogenesis of proliferative cells or tissues, either alone, as a protein fusion, or in combination with other polypeptides directly or indirectly, as described elsewhere herein. In a most preferred embodiment, said anti-angiogenesis effect may be achieved indirectly, for example, through the inhibition of hematopoietic, tumor-specific cells, such as tumor-associated macrophages (See Joseph IB, et al. J Natl Cancer Inst, 90(21):1648-53 (1998), which is hereby incorporated by reference). Antibodies directed to polypeptides or polynucleotides of the present invention may also result in inhibition of angiogenesis directly, or indirectly (See Witte L, et al., Cancer Metastasis Rev. 17(2):155-61 (1998), which is hereby incorporated by reference)).

Polypeptides, including protein fusions, of the present invention, or fragments thereof may be useful in inhibiting proliferative cells or tissues through the induction of apoptosis. Said polypeptides may act either directly, or indirectly to induce apoptosis of proliferative cells and tissues, for example in the activation of a death-domain receptor, such as tumor necrosis factor (TNF) receptor-1, CD95 (Fas/APO-1), TNF-receptor-related apoptosis-mediated protein (TRAMP) and TNF-related apoptosis-inducing ligand (TRAIL) receptor-1 and -2 (See Schulze-Osthoff K, et.al., Eur J Biochem 254(3):439-59 (1998), which is hereby incorporated by reference). Moreover, in another preferred embodiment of the present invention, said polypeptides may induce apoptosis through other mechanisms, such as in the activation of other proteins which will activate apoptosis, or through stimulating the expression of said proteins, either alone or in combination with small molecule drugs or adjuviants, such as apoptonin, galectins, thioredoxins, antiinflammatory proteins (See for example, Mutat Res 400(1-2):447-55 (1998), Med Hypotheses.50(5):423-33 (1998), Chem Biol Interact. Apr 24;111-112:23-34 (1998), J Mol Med.76(6):402-12 (1998), Int J Tissue React;20(1):3-15 (1998), which are all hereby incorporated by reference).

Polypeptides, including protein fusions to, of fragments thereof, of the present invention are useful in inhibiting the metastasis of proliferative cells or tissues. Inhibition may occur as a direct result of administering polypeptides, or antibodies directed to said polypeptides as described elsewere herein, or indirectly, such as activating the expression of proteins known to inhibit metastasis, for example alpha 4 integrins, (See, e.g., Curr Top Microbiol Immunol 1998; 231:125-41, which is hereby incorporated by reference). Such thereapeutic affects of the present invention may be achieved either alone, or in combination with small molecule drugs or adjuvants.

In another embodiment, the invention provides a method of delivering compositions containing the polypeptides of the invention (e.g., compositions containing polypeptides or polypeptide antibodes associated with heterologous polypeptides, heterologous nucleic acids, toxins, or prodrugs) to targeted cells expressing the polypeptide of the present invention. Polypeptides or polypeptide antibodes of the invention may be associated with with heterologous polypeptides, heterologous nucleic acids, toxins, or prodrugs via hydrophobic, hydrophilic, ionic and/or covalent interactions.
Polypeptides, protein fusions to, or fragments thereof, of the present invention are useful in enhancing the immunogenicity and/or antigenicity of proliferating cells or tissues, either directly, such as would occur if the polypeptides of the present invention 'vaccinated' the immune response to respond to proliferative antigens and immunogens, or indirectly, such as in activating the expression of proteins known to enhance the immune response (e.g. chemokines), to said antigens and immunogens.

### Cardiovascular Disorders

Polynucleotides or polypeptides, or agonists or antagonists of the invention may be used to treat, prevent, and/or diagnose cardiovascular diseases, disorders, and/or conditions, including peripheral artery disease, such as limb ischemia.

Cardiovascular diseases, disorders, and/or conditions include cardiovascular abnormalities, such as arterio-arterial fistula, arteriovenous fistula, cerebral arteriovenous malformations, congenital heart defects, pulmonary atresia, and Scimitar Syndrome. Congenital heart defects include aortic coarctation, cor triatriatum, coronary vessel anomalies, crisscross heart, dextrocardia, patent ductus arteriosus, Ebstein's anomaly, Eisenmenger complex, hypoplastic left heart syndrome, levocardia, tetralogy of fallot, transposition of great vessels, double outlet right ventricle, tricuspid atresia, persistent truncus arteriosus, and heart septal defects, such as aortopulmonary septal defect, endocardial cushion defects, Lutembacher's Syndrome, trilogy of Fallot, ventricular heart septal defects.

Cardiovascular diseases, disorders, and/or conditions also include heart disease, such as arrhythmias, carcinoid heart disease, high cardiac output, low cardiac output, cardiac tamponade, endocarditis (including bacterial), heart aneurysm, cardiac arrest, congestive heart failure, congestive cardiomyopathy, paroxysmal dyspnea, cardiac edema, heart hypertrophy, congestive cardiomyopathy, left ventricular hypertrophy, right ventricular hypertrophy, post-infarction heart rupture, ventricular septal rupture, heart valve diseases, myocardial diseases, myocardial ischemia, pericardial effusion, pericarditis (including constrictive and tuberculous), pneumopericardium, postpericardiotomy syndrome, pulmonary heart disease, rheumatic heart disease, ventricular dysfunction, hyperemia, cardiovascular pregnancy complications, Scimitar Syndrome, cardiovascular syphilis, and cardiovascular tuberculosis.

Arrhythmias include sinus arrhythmia, atrial fibrillation, atrial flutter, bradycardia, extrasystole, Adams-Stokes Syndrome, bundle-branch block, sinoatrial block, long QT syndrome, parasystole, Lown-Ganong-Levine Syndrome, Mahaim-type pre-excitation syndrome, Wolff-Parkinson-White syndrome, sick sinus syndrome, tachycardias, and ventricular fibrillation. Tachycardias include paroxysmal tachycardia, supraventricular tachycardia, accelerated idioyentricular rhythm, atrioventricular nodal reentry tachycardia, ectopic atrial tachycardia, ectopic junctional tachycardia, sinoatrial nodal reentry tachycardia, sinus tachycardia, Torsades de Pointes, and ventricular tachycardia.

Heart valve disease include aortic valve insufficiency, aortic valve stenosis, hear murmurs, aortic valve prolapse, mitral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

Myocardial diseases include alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, endomyocardial fibrosis, Kearns Syndrome, myocardial reperfusion injury, and myocarditis.

Myocardial ischemias include coronary disease, such as angina pectoris, coronary aneurysm, coronary arteriosclerosis, coronary thrombosis, coronary vasospasm, myocardial infarction and myocardial stunning.

Cardiovascular diseases also include vascular diseases such as aneurysms, angiodysplasia, angiomatosis, bacillary angiomatosis, Hippel-Lindau Disease, Klippel-Trenaunay-Weber Syndrome, Sturge-Weber Syndrome, angioneurotic edema, aortic diseases, Takayasu's Arteritis, aortitis, Leriche's Syndrome, arterial occlusive diseases, arteritis, enarteritis, polyarteritis nodosa, cerebrovascular diseases, disorders, and/or conditions, diabetic angiopathies, diabetic retinopathy, embolisms, thrombosis, erythromelalgia, hemorrhoids, hepatic veno-occlusive disease, hypertension, hypotension, ischemia, peripheral vascular diseases, phlebitis, pulmonary veno-occlusive disease, Raynaud's disease, CREST syndrome, retinal vein occlusion, Scimitar syndrome, superior vena cava syndrome, telangiectasia, atacia telangiectasia, hereditary hemorrhagic telangiectasia, varicocele, varicose veins, varicose ulcer; vasculitis, and venous insufficiency.

Aneurysms include dissecting aneurysms, false aneurysms, infected aneurysms, ruptured aneurysms, aortic aneurysms, cerebral aneurysms, coronary aneurysms, heart aneurysms, and iliac aneurysms.

Arterial occlusive diseases include arteriosclerosis, intermittent claudication, carotid stenosis, fibromuscular dysplasias, mesenteric vascular occlusion, Moyamoya disease, renal artery obstruction, retinal artery occlusion, and thromboangiitis obliterans.

Cerebrovascular diseases, disorders, and/or conditions include carotid artery diseases, cerebral amyloid angiopathy, cerebral aneurysm, cerebral anoxia, cerebral arteriosclerosis, cerebral arteriovenous malformation, cerebral artery diseases, cerebral embolism and thrombosis, carotid artery thrombosis, sinus thrombosis, Wallenberg's syndrome, cerebral hemorrhage, epidural hematoma, subdural hematoma, subaraxhnoid hemorrhage, cerebral infarction, cerebral ischemia (including transient), subclavian steal syndrome, periventricular leukomalacia, vascular headache, cluster headache, migraine, and vertebrobasilar insufficiency.

Embolisms include air embolisms, amniotic fluid embolisms, cholesterol embolisms, blue toe syndrome, fat embolisms, pulmonary embolisms, and thromoboembolisms. Thrombosis include coronary thrombosis, hepatic vein thrombosis, retinal vein occlusion, carotid artery thrombosis, sinus thrombosis, Wallenberg's syndrome, and thrombophlebitis.

Ischemia includes cerebral ischemia, ischemic colitis, compartment syndromes, anterior compartment syndrome, myocardial ischemia, reperfusion injuries, and peripheral limb ischemia. Vasculitis includes aortitis, arteritis, Behcet's Syndrome, Churg-Strauss Syndrome, mucocutaneous lymph node syndrome, thromboangiitis obliterans, hypersensitivity vasculitis, Schoenlein-Henoch purpura, allergic cutaneous vasculitis, and Wegener's granulomatosis.

Polynucleotides or polypeptides, or agonists or antagonists of the invention, are especially effective for the treatment of critical limb ischemia and coronary disease.

Polypeptides may be administered using any method known in the art, including, but not limited to, direct needle injection at the delivery site, intravenous injection, topical administration, catheter infusion, biolistic injectors, particle accelerators, gelfoam sponge depots, other commercially available depot materials, osmotic pumps, oral or suppositorial solid pharmaceutical formulations, decanting or topical applications during surgery, aerosol delivery. Such methods are known in the art. Polypeptides of the invention may be administered as part of a *Therapeutic,* described in more detail below. Methods of delivering polynucleotides of the invention are described in more detail herein.

### Anti-Angiogenesis Activity

The naturally occurring , balance between endogenous stimulators and inhibitors of angiogenesis is one in which inhibitory influences predominate. Rastinejad *et al., Cell 56*:345-355 (1989). In those rare instances in which neovascularization occurs under normal physiological conditions, such as wound healing, organ regeneration, embryonic development, and female reproductive processes, angiogenesis is stringently regulated and spatially and temporally delimited. Under conditions of pathological angiogenesis such as that characterizing solid tumor growth, these regulatory-controls fail. Unregulated angiogenesis becomes pathologic and sustains progression of many neoplastic and non-neoplastic diseases. A number of serious diseases are dominated by abnormal neovascularization including solid tumor growth and metastases, arthritis, some types of eye diseases, disorders, and/or conditions, and psoriasis. See, e.g., reviews by Moses *et al., Biotech.* 9:630-634 (1991); Folkman *et al., N. Engl. J. Med., 333:*1757-1763 (1995); Auerbach *et al., J. Microvasc. Res. 29*:401-41 (1985); Folkman, Advances in Cancer Research, eds. Klein and Weinhouse, Academic Press, New York, pp. 175-203 (1985); Patz, *Am. J. Opthalmol. 94*:715-743 (1982); and Folkman *et al.,* Science *221*:719-725 (1983). In a number of pathological conditions, the process of angiogenesis contributes to the disease state. For example, significant data have accumulated which suggest that the growth of solid tumors is dependent on angiogenesis. Folkman and Klagsbrun, *Science 235*:442-447 (1987).

The present invention provides for treatment of diseases, disorders, and/or conditions associated with neovascularization by administration of the polynucleotides and/or polypeptides of the invention, as well as agonists or antagonists of the present invention. Malignant and metastatic conditions which can be treated with the polynucleotides and polypeptides, or agonists or antagonists of the invention include, but are not limited to, malignancies, solid tumors, and cancers described herein and otherwise known in the art (for a review of such disorders, see Fishman *et al.,* Medicine, 2d Ed., J. B. Lippincott Co., Philadelphia (1985)).Thus, the present invention provides a method of treating, preventing, and/or diagnosing an angiogenesis-related disease and/or disorder, comprising administering to an individual in need thereof a therapeutically effective amount of a polynucleotide, polypeptide, antagonist and/or agonist of the invention. For example, polynucleotides, polypeptides, antagonists and/or agonists may be utilized in a variety of additional methods in order to therapeutically treator prevent a cancer or tumor. Cancers which may be treated, prevented, and/or diagnosed with polynucleotides, polypeptides, antagonists and/or agonists include, but are not limited to solid tumors, including prostate, lung, breast, ovarian, stomach, pancreas, larynx, esophagus, testes, liver, parotid, biliary tract, colon, rectum, cervix, uterus, endometrium, kidney, bladder, thyroid cancer; primary tumors and metastases; melanomas; glioblastoma; Kaposi's sarcoma; leiomyosarcoma; non- small cell lung cancer; colorectal cancer; advanced malignancies; and blood born tumors such as leukemias. For example, polynucleotides, polypeptides, antagonists and/or agonists may be delivered topically, in order to treat or prevent cancers such as skin cancer, head and neck tumors, breast tumors, and Kaposi's sarcoma.

Within yet other aspects, polynucleotides, polypeptides, antagonists and/or agonists may be utilized to treat superficial forms of bladder cancer by, for example, intravesical administration. Polynucleotides, polypeptides, antagonists and/or agonists may be delivered directly into the tumor, or near the tumor site, via injection or a catheter. Of course, as the artisan of ordinary skill will appreciate, the appropriate mode of administration will vary according to the cancer to be treated. Other modes of delivery are discussed herein.

Polynucleotides, polypeptides, antagonists and/or agonists may be useful in treating, preventing, and/or diagnosing other diseases, disorders, and/or conditions, besides cancers, which involve angiogenesis. These diseases, disorders, and/or conditions include, but are not limited to: benign tumors, for example hemangiomas, acoustic neuromas, neurofibromas, trachomas, and pyogenic granulomas; artheroscleric plaques; ocular angiogenic diseases, for example, diabetic retinopathy, retinopathy of prematurity, macular degeneration, corneal graft rejection, neovascular glaucoma, retrolental fibroplasia, rubeosis, retinoblastoma, uvietis and Pterygia (abnormal blood vessel growth) of the eye; rheumatoid arthritis; psoriasis; delayed wound healing; endometriosis; vasculogenesis; granulations; hypertrophic scars (keloids); nonunion fractures; scleroderma; trachoma; vascular adhesions; myocardial angiogenesis; coronary collaterals; cerebral collaterals; arteriovenous malformations; ischemic limb angiogenesis; Osler-Webber Syndrome; plaque neovascularization; telangiectasia; hemophiliac joints; angiofibroma; fibromuscular dysplasia; wound granulation; Crohn's disease; and atherosclerosis.

For example, within one aspect of the present invention methods are provided for treating, preventing, and/or diagnosing hypertrophic scars and keloids, comprising the step of administering a polynucleotide, polypeptide, antagonist and/or agonist of the invention to a hypertrophic scar or keloid.

Within one embodiment of the present invention polynucleotides, polypeptides, antagonists and/or agonists are directly injected into a hypertrophic scar or keloid, in order to prevent the progression of these lesions. This therapy is of particular value in the prophylactic treatment of conditions which are known to result in the development of hypertrophic scars and keloids (e.g., bums), and is preferably initiated after the proliferative phase has had time to progress (approximately 14 days after the initial injury), but before hypertrophic scar or keloid development. As noted above, the present invention also provides methods for treating, preventing, and/or diagnosing neovascular diseases of the eye, including for example, corneal neovascularization, neovascular glaucoma, proliferative diabetic retinopathy, retrolental fibroplasia and macular degeneration.

Moreover, Ocular diseases, disorders, and/or conditions associated with neovascularization which can be treated, prevented, and/or diagnosed with the polynucleotides and polypeptides of the present invention (including agonists and/or antagonists) include, but are not limited to: neovascular glaucoma, diabetic retinopathy, retinoblastoma, retrolental fibroplasia, uveitis, retinopathy of prematurity macular degeneration, corneal graft neovascularization, as well as other eye inflammatory diseases, ocular tumors and diseases associated with choroidal or iris neovascularization. See, e.g., reviews by Waltman *et al., Am. J. Ophthal. 85*:704-710 (1978) and Gartner *et al., Surv. Ophthal. 22*:291-312 (1978).

Thus, within one aspect of the present invention methods are provided for treating or preventing neovascular diseases of the eye such as corneal neovascularization (including corneal graft neovascularization), comprising the step of administering to a patient a therapeutically effective amount of a compound (as described above) to the cornea, such that the formation of blood vessels is inhibited. Briefly, the cornea is a tissue which normally lacks blood vessels. In 'certain pathological conditions however, capillaries may extend into the cornea from the pericorneal vascular plexus of the limbus. When the cornea becomes vascularized, it also becomes clouded, resulting in a decline in the patient's visual acuity. Visual loss may become complete if the cornea completely opacitates. A wide variety of diseases, disorders, and/or conditions can result in corneal neovascularization, including for example, corneal infections (e.g., trachoma, herpes simplex keratitis, leishmaniasis and onchocerciasis), immunological processes (e.g., graft rejection and Sievens-Johnson's syndrome), alkali burns, trauma, inflammation (of any cause), toxic and nutritional deficiency states, and as a complication of wearing contact lenses

Within particularly preferred embodiments of the invention, may be prepared for topical administration in saline (combined with any of the preservatives and antimicrobial agents commonly used in ocular preparations), and administered in eyedrop form. The solution or suspension may be prepared in its pure form and administered several times daily. Alternatively, anti-angiogenic compositions, prepared as described above, may also be administered directly to the cornea. Within preferred embodiments, the anti-angiogenic composition is prepared with a muco-adhesive polymer which binds to cornea. Within further embodiments, the anti-angiogenic factors or anti-angiogenic compositions may be utilized as an adjunct to conventional steroid therapy. Topical therapy may also be useful prophylactically in corneal lesions which are known to have a high probability of inducing an angiogenic response (such as chemical burns). In these instances the treatment, likely in combination with steroids, may be instituted immediately to help prevent subsequent complications.

Within other embodiments, the compounds described above may be injected directly into the corneal stroma by an ophthalmologist under microscopic guidance. The preferred site of injection may vary with the morphology of the individual lesion, but the goal of the administration would be to place the composition at the advancing front of the vasculature (i.e., interspersed between the blood vessels and the normal cornea). In most cases this would involve perilimbic corneal injection to "protect" the cornea from the advancing blood vessels. This method may also be utilized shortly after a corneal insult in order to prophylactically prevent comeal neovascularization. In this situation the material could be injected in the perilimbic cornea interspersed between the corneal lesion and its undesired potential limbic blood supply. Such methods may also be utilized in a similar fashion to prevent capillary invasion of transplanted corneas. In a sustained-release form injections might only be required 2-3 times per year. A steroid could also be added to the injection solution to reduce inflammation resulting from the injection itself

Within another aspect of the present invention, methods are provided for treating or preventing neovascular glaucoma, comprising the step of administering to a patient a therapeutically effective amount of a polynucleotide, polypeptide, antagonist and/or agonist to the eye, such that the formation of blood vessels is inhibited. In one embodiment, the compound may be administered topically to the eye in order to treat or prevent early forms of neovascular glaucoma. Within other embodiments, the compound may be implanted by injection into the region of the anterior chamber angle. Within other embodiments; the compound may also be placed in any location such that the compound is continuously released into the aqueous humor. Within another aspect of the present invention, methods are provided for treating or preventing proliferative diabetic retinopathy, comprising the step of administering to a patient a therapeutically effective amount of a polynucleotide, polypeptide, antagonist and/or agonist to the eyes, such that the formation of blood vessels is inhibited.

Within particularly preferred embodiments of the invention, proliferative diabetic retinopathy may be treated by injection into the aqueous humor or the vitreous, in order to increase the local concentration of the polynucleotide, polypeptide, antagonist and/or agonist in the retina. Preferably, this treatment should be initiated prior to the acquisition of severe disease requiring photocoagulation.

Within another aspect of the present invention, methods are provided for treating or preventing retrolental fibroplasia, comprising the step of administering to a patient a therapeutically effective amount of a polynucleotide, polypeptide, antagonist and/or agonist to the eye, such that the formation of blood vessels is inhibited. The compound may be administered topically, via intravitreous injection and/or via intraocular implants.

Additionally, diseases, disorders, and/or conditions which can be treated, prevented, and/or diagnosed with the polynucleotides, polypeptides, agonists and/or agonists include, but are not limited to, hemangioma, arthritis, psoriasis, angiofibroma, atherosclerotic plaques, delayed wound healing, granulations, hemophilic joints, hypertrophic scars, nonunion fractures, Osler-Weber syndrome, pyogenic granuloma, scleroderma, trachoma, and vascular adhesions.

Moreover, diseases, disorders, and/or conditions and/or states, which can be treated, prevented, and/or diagnosed with the the polynucleotides, polypeptides, agonists and/or agonists include, but are not limited to, solid tumors, blood born tumors such as leukemias, tumor metastasis, Kaposi's sarcoma, benign tumors, for example hemangiomas, acoustic neuromas, neurofibromas, trachomas, and pyogenic granulomas, rheumatoid arthritis, psoriasis, ocular angiogenic diseases, for example, diabetic retinopathy, retinopathy of prematurity, macular degeneration, corneal graft rejection, neovascular glaucoma, retrolental fibroplasia, rubeosis, retinoblastoma, and uvietis, delayed wound healing, endometriosis, vascluogenesis, granulations, hypertrophic scars (keloids), nonunion fractures, scleroderma, trachoma, vascular adhesions, myocardial angiogenesis, coronary collaterals, cerebral collaterals, arteriovenous malformations, ischemic limb angiogenesis, Osler-Webber Syndrome, plaque neovascularization, telangiectasia, hemophiliac joints, angiofibroma fibromuscular dysplasia, wound granulation, Crohn's disease, atherosclerosis, birth control agent by preventing vascularization required for embryo implantation controlling menstruation, diseases that have angiogenesis as a pathologic consequence such as cat scratch disease (Rochele minalia quintosa), ulcers (Helicobacter pylori), Bartonellosis and bacillary angiomatosis.

In one aspect of the birth control method, an amount of the compound sufficient to block embryo implantation is administered before or after intercourse and fertilization have occurred, thus providing an effective method of birth control, possibly a "morning after" method. Polynucleotides, polypeptides, agonists and/or agonists may also be used in controlling menstruation or administered as either a peritoneal lavage fluid or for peritoneal implantation in the treatment of endometriosis.

Polynucleotides, polypeptides, agonists and/or agonists of the present invention may be incorporated into surgical sutures in order to prevent stitch granulomas.

Polynucleotides, polypeptides, agonists and/or agonists may be utilized in a wide variety of surgical procedures. For example, within one aspect of the present invention a compositions (in the form of, for example, a spray or film) may be utilized to coat or spray an area prior to removal of a tumor, in order to isolate normal surrounding tissues from malignant tissue, and/or to prevent the spread of disease to surrounding tissues. Within other aspects of the present invention, compositions (e.g., in the form of a spray) may be delivered via endoscopic procedures in order to coat tumors, or inhibit angiogenesis in a desired locale. Within yet other aspects of the present invention, surgical meshes which have been coated with anti- angiogenic compositions of the present invention may be utilized in any procedure wherein a surgical mesh might be utilized. For example, within one embodiment of the invention a surgical mesh laden with an anti-angiogenic composition may be utilized during abdominal cancer resection surgery (e.g., subsequent to colon resection) in order to provide support to the structure, and to release an amount of the anti-angiogenic factor.

Within further aspects of the present invention, methods are provided for treating tumor excision sites, comprising administering a polynucleotide, polypeptide, agonist and/or agonist to the resection margins of a tumor subsequent to excision, such that the local recurrence of cancer and the formation of new blood vessels at the site is inhibited. Within one embodiment of the invention, the anti-angiogenic compound is administered directly to the tumor excision site (e.g., applied by swabbing, brushing or otherwise coating the resection margins of the tumor with the anti-angiogenic compound). Alternatively, the anti-angiogenic compounds may be incorporated into known surgical' pastes prior to administration. Within particularly preferred embodiments of the invention, the anti-angiogenic compounds are applied after hepatic resections for malignancy, and after neurosurgical operations.

Within one aspect of the present invention, polynucleotides, polypeptides, agonists and/or agonists may be administered to the resection margin of a wide variety of tumors, including for example, breast, colon, brain and hepatic tumors. For example, within one embodiment of the invention, anti-angiogenic compounds may be administered to the site of a neurological tumor subsequent to excision, such that the formation of new blood vessels at the site are inhibited.

The polynucleotides, polypeptides, agonists and/or agonists of the present invention may also be administered along with other anti-angiogenic factors. Representative examples of other anti-angiogenic factors include: Anti-Invasive Factor, retinoic acid and derivatives thereof, paclitaxel, Suramin, Tissue Inhibitor of Metalloproteinase-1, Tissue Inhibitor of Metalloproteinase-2, Plasminogen Activator Inhibitor-1, Plasminogen Activator Inhibitor-2, and various forms of the lighter "d group" transition metals.

Lighter "d group" transition metals include, for example, vanadium, molybdenum, tungsten, titanium, niobium, and tantalum species. Such transition metal species may form transition metal complexes. Suitable complexes of the above-mentioned transition metal species include oxo transition metal complexes.

Representative examples of vanadium complexes include oxo vanadium complexes such as vanadate and vanadyl complexes. Suitable vanadate complexes include metavanadate and orthovanadate complexes such as, for example, ammonium metavanadate, sodium metavanadate, and sodium orthovanadate. Suitable vanadyl complexes include, for example, vanadyl acetylacetonate and vanadyl sulfate including vanadyl sulfate hydrates such as vanadyl sulfate mono- and trihydrates.

Representative examples of tungsten and molybdenum complexes also include oxo complexes. Suitable oxo tungsten complexes include tungstate and tungsten oxide complexes. Suitable tungstate complexes include ammonium tungstate; calcium tungstate, sodium tungstate dihydrate, and tungstic acid. Suitable tungsten oxides include tungsten (IV) oxide and tungsten (VI) oxide. Suitable oxo molybdenum complexes include molybdate, molybdenum oxide, and molybdenyl complexes. Suitable molybdate complexes include ammonium molybdate and its hydrates, sodium molybdate and its hydrates, and potassium molybdate and its hydrates. Suitable molybdenum oxides include molybdenum (VI) oxide, molybdenum (VI) oxide, and molybdic acid. Suitable molybdenyl complexes include, for example, molybdenyl acetylacetonate. Other suitable tungsten and molybdenum complexes include hydroxo derivatives derived from, for example, glycerol, tartaric acid, and sugars

A wide variety of other anti-angiogenic factors may also be utilized within the context of the present invention. Representative examples include platelet factor 4; protamine sulphate; sulphated chitin derivatives (prepared from queen crab shells), (Murata et al., Cancer Res. 51:22-26, 1991); Sulphated Polysaccharide Peptidoglycan Complex (SP- PG) (the function of this compound may be enhanced by the presence of steroids such as estrogen, arid tamoxifen citrate); Staurosporine; modulators of matrix metabolism, including for example, proline analogs, cishydroxyproline, d,L-3,4-dehydroproline, Thiaproline, alpha, alpha-dipyridyl,aminopropionitrile fumarate; 4-propyl-5-(4-pyridinyl)-2(3H)-oxazolone; Methotrexate; Mitoxantrone; Heparin; Interferons; 2 Macroglobulin-serum; ChIMP-3 (Pavloff et al., J. Bio. Chem. 267:17321-17326, 1992); Chymostatin (Tomkinson et al., Biochem J. 286:475-480, 1992); Cyclodextrin Tetradecasulfate; Eponemycin; Camptothecin; Fumagillin (Ingber et al., Nature 348:555-557, 1990); Gold Sodium Thiomalate ("GST"; Matsubara and Ziff, J. Clin. Invest. 79:1440-1446, 1987); anticollagenase-serum; alpha2-antiplasmin (Holmes et al., J. Biol. Chem. 262(4):1659-1664, 1987); Bisantrene (National Cancer Institute); Lobenzarit disodium (N-(2)-carboxyphenyl-4-chloroanthronilic acid disodium or "CCA"; Takeuchi et al., Agents Actions 36:312-316, 1992); Thalidomide; Angostatic steroid; AGM-1470; carboxynaminolmidazole; and metalloproteinase inhibitors such as BB94.

### Diseases at the Cellular Level

Diseases associated with increased cell survival or the inhibition of apoptosis that could be treated, prevented, and/or diagnosed by the polynucleotides or polypeptides and/or antagonists or agonists of the invention, include cancers (such as follicular lymphomas, carcinomas with p53 mutations, and hormone-dependent tumors, including, but not limited to colon cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostate cancer, Kaposi's sarcoma and ovarian cancer); autoimmune diseases, disorders, and/or conditions (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) and viral infections (such as herpes viruses, pox viruses and adenoviruses), inflammation, graft v. host disease, acute graft rejection, and chronic graft rejection. In preferred embodiments, the polynucleotides or polypeptides, and/or agonists or antagonists of the invention are used to inhibit growth, progression, and/or metasis of cancers, in particular those listed above.

Additional diseases or conditions associated with increased cell survival that could be treated, prevented or diagnosed by the polynucleotides or polypeptides, or agonists or antagonists of the invention, include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

Diseases associated with increased apoptosis that could be treated, prevented, and/or diagnosed by the polynucleotides or polypeptides, and/or agonists or antagonists of the invention, include AIDS; neurodegenerative diseases, disorders, and/or conditions (such as Alzheimer's disease, Parkinson's disease, Amyotrophic lateral sclerosis, Retinitis pigmentosa, Cerebellar degeneration and brain tumor or prior associated disease); autoimmune diseases, disorders, and/or conditions (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) myelodysplastic syndromes (such as aplastic anemia), graft v. host disease, ischemic injury (such as that caused by myocardial infarction, stroke and reperfusion injury), liver injury (e.g., hepatitis related liver injury, ischemia/reperfusion injury, cholestosis (bile duct injury) and liver cancer); toxin-induced liver disease (such as that caused by alcohol), septic shock, cachexia and anorexia.

### Wound Healing and Epithelial Cell Proliferation

In accordance with yet a further aspect of the present invention, there is provided a process for utilizing the polynucleotides or polypeptides, and/or agonists or antagonists of the invention, for therapeutic purposes, for example, to stimulate epithelial cell proliferation and basal keratinocytes for the purpose of wound healing, and' to stimulate hair follicle production and healing of dermal wounds. Polynucleotides or polypeptides, as well as agonists or antagonists of the invention, may be clinically useful in stimulating wound healing including surgical wounds, excisional wounds, deep wounds involving damage of the dermis and epidermis, eye tissue wounds, dental tissue wounds, oral cavity wounds, diabetic ulcers, dermal ulcers, cubitus ulcers, arterial ulcers, venous stasis ulcers, burns resulting from heat exposure or chemicals, and other abnormal wound healing conditions such as uremia; malnutrition, vitamin deficiencies and complications associfed with systemic treatment with steroids, radiation therapy and antineoplastic drugs and antimetabolites. Polynucleotides or polypeptides, and/or agonists or antagonists of the invention, could be used to promote dermal reestablishment subsequent to dermal loss The polynucleotides or polypeptides, and/or agonists or antagonists of the invention, could be used to increase the adherence of skin grafts to a wound bed and to stimulate re-epithelialization from the wound bed. The following are a non-exhaustive list of grafts that polynucleotides or polypeptides, agonists or antagonists of the invention, could be used to increase adherence to a wound bed: auto grafts, artificial skin, allografts, autodermic graft, autoepdermic grafts, avacular grafts, Blair-Brown grafts, bone graft, brephoplastic grafts, cutis graft, delayed graft, dermic graft, epidermic graft, fascia graft, full thickness graft, heterologous graft, xenograft, homologous graft, hyperplastic graft, lamellar graft, mesh graft, mucosal graft, Ollier-Thiersch graft, omenpal graft, patch graft, pedicle graft, penetrating graft, split skin graft, thick split graft. The polynucleotides or polypeptides, and/or agonists or antagonists of the invention, can be used to promote skin strength and to improve the appearance of aged skin.

It is believed that the polynucleotides or polypeptides, and/or agonists or antagonists of the invention, will also produce changes in hepatocyte proliferation, and epithelial cell proliferation in the lung, breast, pancreas, stomach, small intesting, and large intestine. The polynucleotides or polypeptides, and/or agonists or antagonists of the invention, could promote proliferation of epithelial cells such as sebocytes, hair follicles, hepatocytes, type II pneumocytes, mucin-producing goblet cells, and other epithelial cells and their progenitors contained within the skin, lung, liver, and gastrointestinal tract. The polynucleotides or polypeptides, and/or agonists or antagonists of the invention, may promote proliferation of endothelial cells, keratinocytes, and basal keratinocytes.

The polynucleotides or polypeptides, and/or agonists or antagonists of the invention, could also be used to reduce the side effects of gut toxicity that result from radiation, chemotherapy treatments or viral infections. The polynucleotides or polypeptides, and/or agonists or antagonists of the invention, may have a cytoprotective effect on the small intestine mucosa. The polynucleotides or polypeptides, and/or agonists or antagonists of the invention, may also stimulate healing. of mucositis (mouth ulcers) that result from chemotherapy and viral infections.

The polynucleotides or polypeptides, and/or agonists or antagonists of the invention, could further be used in full regeneration of skin in full and partial thickness skin defects, including burns, (i.e., repopulation of hair follicles, sweat glands, and sebaceous glands), treatment of other skin defects such as psoriasis. The polynucleotides or polypeptides, and/or agonists or antagonists of the invention, could be used to treat epidermolysis bullosa, a defect in adherence of the epidermis to the underlying dermis which results in frequent, open and painful blisters by accelerating reepithelialization of these lesions. The polynucleotides or polypeptides, and/or agonists or antagonists of the invention, could also be used to treat gastric and doudenal ulcers and help heal by scar formation of the mucosal lining and regeneration of glandular mucosa and duodenal mucosal lining more rapidly. Inflamamatory bowel diseases, such as Crohn's disease and ulcerative colitis, are diseases which result in destruction of the mucosal surface of the small or large intestine, respectively. Thus, the polynucleotides or polypeptides, and/or agonists or antagonists of the invention, could be used to promote the resurfacing of the mucosal surface to aid more rapid healing and to prevent progression of inflammatory bowel disease. Treatment with the polynucleotides or polypeptides, and/or agonists or antagonists of the invention, is expected to have a significant effect on the production of mucus throughout the gastrointestinal tract and could be used to protect the intestinal mucosa from injurious substances that are ingested or following surgery. The polynucleotides or polypeptides, and/or agonists or antagonists of the invention, could be used to treat diseases associate with the under expression of the polynucleotides of the invention.

Moreover, the polynucleotides or polypeptides, and/or agonists or antagonists of the invention, could be used to prevent and heal damage to the lungs due to various pathological states. A growth factor such as the polynucleotides or polypeptides, and/or agonists or antagonists of the invention, which could stimulate proliferation and differentiation and promote the repair of alveoli and brochiolar epithelium to prevent or treat acute or chronic lung damage. For example, emphysema, which results in the progressive loss of aveoli, and inhalation injuries, i.e., resulting from smoke inhalation and burns, that cause necrosis of the bronchiolar epithelium and alveoli could be effectively treated, prevented, and/or diagnosed using the polynucleotides or polypeptides, and/or agonists or antagonists of the invention. Also, the polynucleotides or polypeptides, and/or agonists or antagonists of the invention, could be used to stimulate the proliferation of and differentiation of type II pneumocytes, which may help treat or prevent disease such as hyaline membrane diseases, such as infant respiratory distress syndrome and bronchopulmonary displasia, in premature infants.

The polynucleotides or polypeptides, and/or agonists or antagonists of the invention, could stimulate the proliferation and differentiation of hepatocytes and, thus, could be used to alleviate or treat liver diseases and pathologies such as fulminant liver failure caused by cirrhosis, liver damage caused by viral hepatitis and toxic substances (i.e., acetaminophen, carbon tetraholoride and other hepatotoxins known in the art).

In addition, the polynucleotides or polypeptides, and/or agonists or antagonists of the invention, could be used treat or prevent the onset of diabetes mellitus. In patients with newly diagnosed Types I and II diabetes, where some islet cell function remains, the polynucleotides or polypeptides, and/or agonists or antagonists of the invention, could be used to maintain the islet function so as to alleviate, delay or prevent permanent manifestation of the disease. Also, the polynucleotides or polypeptides, and/or agonists or antagonists of the invention, could be used as an auxiliary in islet cell transplantation to improve or promote islet cell function.

### Neurological Diseases

Nervous system diseases, disorders, and/or conditions, which can be treated, prevented, and/or diagnosed with the compositions of the invention (e.g:, polypeptides, polynucleotides, and/or agonists or antagonists), include, but are not limited to, nervous system injuries, and diseases, disorders, and/or conditions which result in either a disconnection of axons, a diminution or degeneration of neurons, or demyelination. Nervous system lesions which may be treated, prevented, and/or diagnosed in a patient (including human and non-human mammalian patients) according to the invention, include but are not limited to, the following lesions of either the central (including spinal cord, brain) or peripheral nervous systems: (1) ischemic lesions, in which a lack of oxygen in a portion of the nervous system results in neuronal injury or death, including cerebral infarction or ischemia, or spinal cord infarction or ischemia; (2) traumatic lesions, including lesions caused by physical injury or associated with surgery, for example, lesions which sever a portion of the nervous system, or compression injuries; (3) malignant lesions, in which a portion of the nervous system is destroyed or injured by malignant tissue which is either a nervous system associated malignancy or a malignancy derived from non-nervous system tissue; (4) infectious lesions, in which a portion of the nervous system is destroyed or injured as a result of infection, for example, by an abscess or associated with infection by human immunodeficiency virus, herpes zoster, or herpes simplex virus or with Lyme disease, tuberculosis, syphilis; (5) degenerative lesions, in which a portion of the nervous system is destroyed or injured as a result of a degenerative process including but not limited to degeneration associated with Parkinson's disease, Alzheimer's disease, Huntington's chorea, or amyotrophic lateral sclerosis (ALS); (6) lesions associated with nutritional diseases, disorders, and/or conditions, in which a portion of the nervous system is destroyed or injured by a nutritional disorder or disorder of metabolism including but not limited to, vitamin B12 deficiency, folic acid deficiency, Wernicke disease, tobacco-alcohol amblyopia, Marchiafava-Bignami disease (primary degeneration of the corpus callosum), and alcoholic cerebellar degeneration; (7) neurological lesions associated with systemic diseases including, but not limited to, diabetes (diabetic neuropathy, Bell's palsy), systemic lupus erythematosus, carcinoma, or sarcoidosis; (8) lesions caused by toxic substances including alcohol, lead, or particular neurotoxins; and (9) demyelinated lesions in which a portion of the nervous system is destroyed or injured by a demyelinating disease including, but not limited to, multiple sclerosis, human immunodeficiency virus-associated myelopathy, transverse myelopathy or various etiologies, progressive multifocal leukoencephalopathy, and central pontine myelinolysis.

In a preferred embodiment, the polypeptides, polynucleotides, or agonists or antagonists of the invention are used to protect neural cells from the damaging effects of cerebral hypoxia. According to this embodiment, the compositions of the invention are used to treat, prevent, and/or diagnose neural cell injury associated with cerebral hypoxia. In one aspect of this embodiment, the polypeptides, polynucleotides, or agonists or antagonists of the invention are used to treat, prevent, and/or diagnose neural cell injury associated with cerebral ischemia. In another aspect of this embodiment, the polypeptides, polynucleotides, or agonists or antagonists of the invention are used to treat, prevent, and/or diagnose neural cell injury associated with cerebral infarction. In another aspect of this embodiment, the polypeptides, 'polynucleotides, or agonists or antagonists of the invention are used to treat, prevent, and/or diagnose or prevent neural cell injury associated with a stroke. In a further aspect of this embodiment, the polypeptides, polynucleotides, or agonists or antagonists of the invention are used to treat, prevent, and/or diagnose neural cell injury associated with a heart attack.

The compositions of the invention which are useful for treating or preventing a nervous system disorder may be selected by testing for biological activity in promoting the survival or differentiation of neurons. For example, and not by way of limitation, compositions of the invention which elicit any of the following effects may be useful according to the invention: (1) increased survival time of neurons in culture; (2) increased sprouting of neurons in culture or *in vivo;* (3) increased production of a neuron-associated molecule in culture or *in vivo, e.g.,* choline acetyltransferase or acetylcholinesterase with respect to motor neurons; or (4) decreased symptoms of neuron dysfunction *in vivo.* Such effects may be measured by any method known in the art. In preferred, non-limiting embodiments, increased survival of neurons may routinely be measured using a method set forth herein or otherwise known in the art, such as, for example, the method set forth in Arakawa et al. (J. Neurosci. 10:3507-3515 (1990)); increased sprouting of neurons may be detected by methods known in the art, such as, for example, the methods set forth in Pestronk et al. (Exp. Neurol. 70:65-82 (1980)) or Brown et al. (Ann. Rev. Neurosci. 4:17-42 (1981)); increased production of neuron-associated molecules may be measured by bioassay, enzymatic assay, antibody binding, Northern blot assay, etc., using techniques known in the art and depending on the molecule to be measured; and motor neuron dysfunction may be measured by assessing the physical manifestation of motor neuron disorder, e.g., weakness, motor neuron conduction velocity, or functional disability.

In specific embodiments, motor neuron diseases, disorders, and/or conditions that may be treated, prevented, and/or diagnosed according to the invention include, but are not limited to, diseases, disorders, and/or conditions such as infarction, infection, exposure to toxin, trauma, surgical damage, degenerative disease or malignancy that may affect motor neurons as well as other components of the nervous system, as well as diseases, disorders, and/or conditions that selectively affect neurons such as amyotrophic lateral sclerosis, and including, but not limited to, progressive spinal muscular atrophy, progressive bulbar palsy, primary lateral sclerosis, infantile and juvenile muscular atrophy, progressive, bulbar paralysis of childhood (Fazio-Londe syndrome), poliomyelitis and the post polio syndrome, and Hereditary Motorsensory Neuropathy (Charcot-Marie-Tooth Disease).

Further, polypeptides or polynucleotides of the invention may play a role in neuronal survival; synapse formation; conductance; neural differentiation, etc. Thus, compositions of the invention (including polynucleotides, polypeptides, and agonists or antagonists) may be used to diagnose and/or treat or prevent diseases or disorders associated with these roles, including, but not limited to, learning and/or cognition disorders. The compositions of the invention may also be useful in the treatment or prevention of neurodegenerative disease states and/or behavioural disorders. Such neurodegenerative disease states and/or behavioral disorders include, but are not limited to, Alzheimens Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception. In addition, compositions of the invention may also play a role in the treatment, prevention and/or detection of developmental disorders associated with the developing embryo, or sexually-linked disorders.

Additionally, polypeptides, polynucleotides and/or agonists or antagonists of the invention, may be useful in protecting neural cells from diseases, damage, disorders, or injury, associated with cerebrovascular disorders including, but not limited to, carotid artery diseases (e.g., carotid artery thrombosis, carotid stenosis, or Moyamoya Disease), cerebral amyloid angiopathy, cerebral aneurysm, cerebral anoxia, cerebral arteriosclerosis, cerebral arteriovenous malformations, cerebral artery diseases, cerebral embolism and thrombosis (e.g., carotid artery thrombosis, sinus thrombosis, or Wallenberg's Syndrome), cerebral hemorrhage (e.g., epidural or subdural hematoma, or subarachnoid hemorrhage), cerebral infarction, cerebral ischemia (e.g., transient cerebral ischemia, Subclavian Steal Syndrome, or vertebrobasilar insufficiency), vascular dementia (e.g., multi-infarct), leukomalacia, periventricular, and vascular headache (e.g., cluster headache or migraines).

In accordance with yet a further aspect of the present invention, there is provided a process for utilizing polynucleotides or polypeptides, as well as agonists or antagonists of the present invention, for therapeutic purposes, for example, to stimulate neurological cell proliferation and/or differentiation. Therefore, polynucleotides, polypeptides, agonists and/or antagonists of the invention may be used to treat and/or detect neurologic diseases. Moreover, polynucleotides or polypeptides, or agonists or antagonists of the invention, can be used as a marker or detector of a particular nervous system disease or disorder.

Examples of neurologic diseases which can be treated or detected with polynucleotides, polypeptides, agonists, and/or antagonists of the present invention include brain diseases, such as metabolic brain diseases which includes phenylketonuria such as maternal phenylketonuria, pyruvate carboxylase deficiency, pyruvate dehydrogenase complex deficiency, Wernicke's Encephalopathy, brain edema, brain neoplasms such as cerebellar neoplasms which include infratentorial neoplasms, cerebral ventricle neoplasms such as choroid plexus neoplasms, hypothalamic neoplasms, supratentorial neoplasms, canavan disease, cerebellar diseases such as cerebellar ataxia which include spinocerebellar degeneration such as ataxia telangiectasia, cerebellar dyssynergia, Friederich's Ataxia, Machado-Joseph Disease, olivopontocerebellar atrophy, cerebellar neoplasms such as infratentorial neoplasms, diffuse cerebral sclerosis such as encephalitis periaxialis, globoid cell leukodystrophy, metachromatic leukodystrophy and subacute sclerosing panencephalitis.

Additional neurologic diseases which can be treated or detected with polynucleotides, polypeptides, agonists, and/or antagonists of the present invention include cerebrovascular disorders (such as carotid artery diseases which include carotid artery thrombosis, carotid stenosis and Moyamoya Disease), cerebral amyloid angiopathy, cerebral aneurysm, cerebral anoxia, cerebral arteriosclerosis, cerebral arteriovenous malformations, cerebral artery diseases, cerebral embolism and thrombosis such as carotid artery thrombosis, sinus thrombosis and Wallenberg's. Syndrome, cerebral hemorrhage such as epidural hematoma, subdural hematoma and subarachnoid hemorrhage, cerebral infarction, cerebral ischemia such as transient cerebral ischemia, Subclavian Steal Syndrome and vertebrobasilar insufficiency, vascular dementia such as multi-infarct dementia, periventricular leukomalacia, vascular headache such as cluster headache and migraine.

Additional neurologic diseases which can be treated or detected with polynucleotides, polypeptides, agonists, and/or antagonists of the present invention include dementia such as AIDS Dementia Complex, presenile dementia such as Alzheimer's Disease and Creutzfeldt-Jakob Syndrome, senile dementia such as Alzheimer's Disease and progressive supranuclear palsy, vascular dementia such as multi-infarct dementia, encephalitis which include encephalitis periaxialis, viral encephalitis such as epidemic encephalitis, Japanese Encephalitis, St. Louis Encephalitis, tick-borne encephalitis and West Nile Fever, acute disseminated encephalomyelitis, meningoencephalitis such as uveomeningoencephalitic syndrome, Postencephalitic Parkinson Disease and subacute sclerosing panencephalitis, encephalomalacia such as periventricular leukomalacia, epilepsy such as generalized epilepsy which includes infantile spasms, absence epilepsy, myoclonic epilepsy which includes MERRF Syndrome, tonic-clonic epilepsy, partial epilepsy such as complex partial epilepsy, frontal lobe epilepsy and temporal lobe epilepsy, post-traumatic epilepsy, status epilepticus such as Epilepsia Partialis Continua, and Hallervorden-Spatz Syndrome.

Additional neurologic diseases which can be treated or detected with polynucleotides, polypeptides, agonists, and/or antagonists of the present invention include hydrocephalus such as Dandy-Walker Syndrome and normal pressure hydrocephalus, hypothalamic diseases such as hypothalamic neoplasms, cerebral malaria, narcolepsy which includes cataplexy, bulbar poliomyelitis, cerebri pseudotumor, Rett Syndrome, Reye's Syndrome, thalamic diseases, cerebral toxoplasmosis, intracranial tuberculoma and Zellweger Syndrome, central nervous system infections such as AIDS Dementia Complex, Brain Abscess, subdural empyema; encephalomyelitis such as Equine Encephalomyelitis, Venezuelan Equine Encephalomyelitis, Necrotizing Hemorrhagic Encephalomyelitis, Visna, and cerebral malaria.

Additional neurologic diseases which can be treated or detected with polynucleotides, polypeptides, agonists, and/or antagonists of the present invention include meningitis such as arachnoiditis, aseptic meningtitis such as viral meningtitis which includes lymphocytic choriomeningitis, Bacterial meningtitis which includes Haemophilus Meningtitis, Listeria Meningtitis, Meningococcal Meningtitis such as Waterhouse-Friderichsen Syndrome, Pneumococcal Meningtitis and meningeal tuberculosis, fungal meningitis such as Cryptococcal Meningtitis, subdural effusion, meningoencephalitis such as uvemeningoencephalitic syndrome, myelitis such as transverse myelitis, neurosyphilis such as tabes dorsalis, poliomyelitis which includes bulbar poliomyelitis and postpoliomyelitis syndrome, prion diseases (such as Creutzfeldt-Jakob Syndrome, Bovine Spongiform Encephalopathy, Gerstmann-Straussler Syndrome, Kuru, Scrapie), and cerebral toxoplasmosis.

Additional neurologic diseases which can be treated or detected with polynucleotides, polypeptides, agonists, and/or antagonists of the present invention include central nervous system neoplasms such as brain neoplasms that include cerebellar neoplasms such as infratentorial neoplasms, cerebral ventricle neoplasms such as choroid plexus neoplasms, hypothalamic neoplasms and supratentorial neoplasms, meningeal neoplasms, spinal cord neoplasms which include epidural neoplasms, demyelinating diseases such as Canavan Diseases, diffuse cerebral sceloris which includes adrenoleukodystrophy, encephalitis periaxialis, globoid cell leukodystrophy, diffuse cerebral sclerosis such as metachromatic leukodystrophy, allergic encephalomyelitis, necrotizing hemorrhagic encephalomyelitis, progressive multifocal leukoencephalopathy, multiple sclerosis, central pontine myelinolysis, transverse myelitis, neuromyelitis optica, Scrapie, Swayback, Chronic Fatigue Syndrome, Visna, High Pressure Nervous Syndrome, Meningism, spinal cord diseases such as amyotonia congenita, amyotrophic lateral sclerosis, spinal muscular atrophy such as Werdnig-Hoffmann Disease, spinal cord compression, spinal cord neoplasms such as epidural neoplasms, syringomyelia, Tabes Dorsalis, Stiff-Man Syndrome, mental retardation such as Angelman Syndrome, Cri-du-Chat Syndrome, De Lange's Syndrome, Down Syndrome, Gangliosidoses such as gangliosidoses G(M1), Sandhoff Disease, Tay-Sachs Disease, Hartnup Disease, homocystinuria, Laurence-Moon-Biedl Syndrome, Lesch-Nyhan Syndrome, Maple Syrup Urine Disease, mucolipidosis such as fucosidosis, neuronal ceroid-lipofuscinosis, oculocerebrorenal syndrome, phenylketonuria such as maternal phenylketonuria, Prader-Willi Syndrome, Rett Syndrome, Rubinstein-Taybi Syndrome, Tuberous Sclerosis, WAGR Syndrome, nervous system abnormalities such as holoprosencephaly, neural tube defects such as anencephaly which includes hydrangencephaly, Amold-Chairi Deformity, encephalocele, meningocele, meningomyelocele, spinal dysraphism such as spina bifida cystica and spina bifida occulta.

Additional neurologic diseases which can be treated or detected with polynucleotides, polypeptides, agonists, and/or antagonists of the present invention include hereditary motor and sensory neuropathies which include Charcot-Marie Disease, Hereditary optic atrophy, Refsum's Disease, hereditary spastic paraplegia, Werdnig-Hoffmann Disease, Hereditary Sensory and Autonomic Neuropathies such as Congenital Analgesia and Familial Dysautonomia, Neurologic manifestations (such as agnosia that include Gerstmann's Syndrome, Amnesia such as retrograde amnesia, apraxia, neurogenic bladder, cataplexy, communicative disorders such as hearing disorders that includes deafness, partial hearing loss, loudness recruitment and tinnitus, language disorders such as aphasia which include agraphia, anomia, broca aphasia, and Wernicke Aphasia, Dyslexia such as Acquired Dyslexia, language development disorders, speech disorders such as aphasia which includes anomia, broca aphasia and Wemicke Aphasia, articulation disorders, communicative disorders such as speech disorders which include dysarthria, echolalia, mutism and stuttering, voice disorders such as aphonia and' hoarseness,' decerebrate state, delirium, fasciculation, hallucinations, meningism, movement disorders such as angelman syndrome, ataxia, athetosis, chorea, dystonia, hypokinesia, muscle hypotonia, myoclonus, tic, torticollis and tremor, muscle hypertonia such as muscle rigidity such as stiff-man syndrome, muscle spasticity, paralysis such as facial paralysis which includes Herpes Zoster Oticus, Gastroparesis, Hemiplegia, ophthalmoplegia such as diplopia, Duane's Syndrome, Homer's Syndrome, Chronic progressive external ophthalmoplegia such as Kearns Syndrome, Bulbar Paralysis, Tropical Spastic Paraparesis, Paraplegia such as Brown-Sequard Syndrome, quadriplegia, respiratory paralysis and vocal cord paralysis, paresis, phantom limb, taste disorders such as ageusia and dysgeusia, vision disorders such as amblyopia, blindness, color vision defects, diplopia, hemianopsia, scotoma and subnormal vision, sleep disorders such as hypersomnia which includes Kleine-Levin Syndrome, insomnia, and somnambulism, spasm such as trismus, unconsciousness such as coma, persistent vegetative state and syncope and vertigo, neuromuscular diseases such as amyotonia congenita, amyotrophic lateral sclerosis, Lambert-Eaton Myasthenic Syndrome, motor neuron disease, muscular atrophy such as spinal muscular atrophy, Charcot-Marie Disease and Werdnig-Hoffmann Disease, Postpoliomyelitis Syndrome, Muscular Dystrophy, Myasthenia Gravis, Myotonia Atrophica, Myotonia Confenita, Nemaline Myopathy, Familial Periodic Paralysis, Multiplex Paramyloclonus, Tropical Spastic Paraparesis and Stiff-Man Syndrome, peripheral nervous system diseases such as acrodynia, amyloid neuropathies, autonomic nervous system diseases such as Adie's Syndrome, Barre-Lieou Syndrome, Familial Dysautonomia, Homer's Syndrome, Reflex Sympathetic Dystrophy and Shy-Drager Syndrome, Cranial Nerve Diseases such as Acoustic Nerve Diseases such as Acoustic Neuroma which includes Neurofibromatosis 2, Facial Nerve Diseases such as Facial Neuralgia,Melkersson-Rosenthal Syndrome, ocular motility disorders which includes amblyopia, nystagmus, oculomotor nerve paralysis, ophthalmoplegia such as Duane's Syndrome, Homer's Syndrome, Chronic Progressive External Ophthalmoplegia which includes Kearns Syndrome, Strabismus such as Esotropia and Exotropia, Oculomotor Nerve Paralysis, Optic Nerve Diseases such as Optic Atrophy which includes Hereditary Optic Atrophy, Optic Disk Drusen, Optic Neuritis such as Neuromyelitis Optica, Papilledema, Trigeminal Neuralgia, Vocal Cord Paralysis, Demyelinating Diseases such as Neuromyelitis Optica and Swayback, and Diabetic neuropathies such as diabetic foot.

Additional neurologic diseases which can be treated or detected with polynucleotides, polypeptides, agonists, and/or antagonists of the present invention include nerve compression syndromes such as carpal tunnel syndrome, tarsal tunnel syndrome, thoracic outlet syndrome such as cervical rib syndrome, ulnar nerve compression syndrome, neuralgia such as causalgia, cervico-brachial neuralgia, facial neuralgia and trigeminal neuralgia, neuritis such as experimental allergic neuritis, optic neuritis, polyneuritis, polyradiculoneuritis and radiculities such as polyradiculitis, hereditary motor and sensory neuropathies such as Charcot-Marie Disease, Hereditary Optic Atrophy, Refsum's Disease, Hereditary Spastic Paraplegia and Werdnig-Hoffmann Disease, Hereditary Sensory and Autonomic Neuropathies which include Congenital Analgesia and Familial Dysautonomia, POEMS Syndrome, Sciatica, Gustatory Sweating and Tetany).

### Infectious Disease

A polypeptide or polynucleotide and/or agonist or antagonist of the present invention can be used to treat, prevent, and/or diagnose infectious agents. For example, by increasing the immune response, particularly increasing the proliferation and differentiation of B and/or T cells, infectious diseases may be treated, prevented, and/or diagnosed. The immune response may be increased by either enhancing an existing immune response, or by initiating a new immune response. Alternatively, polypeptide or polynucleotide and/or agonist or antagonist of the present invention may also directly inhibit the infectious agent, without necessarily eliciting an immune response.

Viruses are one example of an infectious agent that can cause disease or symptoms that can be treated, prevented, and/or diagnosed by a polynucleotide or polypeptide and/or agonist or antagonist of the present invention. Examples of viruses, include, but are not limited to Examples of viruses, include, but are not limited to the following DNA and RNA viruses and viral families: Arbovirus; Adenoviridae, Arenaviridae, Arterivirus, Bimaviridae, Bunyaviridae, Caliciviridae, Circoviridae, Coronaviridae, Dengue, EBV, HIV, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza A, Influenza B, and parainfluenza), Papiloma virus, Papovaviridae, Parvoviridae, Picomaviridae, Poxviridae (such as Smallpox or Vaccinia), Reoviridae (e.g., Rotavirus), Retroviridae (HTLV-I; HTLV-II, Lentivirus), and Togaviridae (e.g., Rubivirus). Viruses falling within these families can cause a variety of diseases or symptoms, including, but not limited to: arthritis, bronchiollitis, respiratory syncytial virus, encephalitis, eye infections (e.g., conjunctivitis, keratitis), chronic fatigue syndrome, hepatitis (A,B, C, E, Chronic Active, Delta), Japanese B encephalitis, Junin, Chikungunya, Rift Valley fever, yellow fever, meningitis, opportunistic infections (e.g., AIDS), pneumonia, Burkitt's Lymphoma, chickenpox, hemorrhagic fever, Measles, Mumps, Parainfluenza, Rabies, the common cold, Polio, leukemia, Rubella, sexually transmitted diseases, skin diseases (e.g., Kaposi's, warts), and viremia. polynucleotides or polypeptides, or agonists or antagonists of the invention, can be used to treat, prevent, and/or diagnose any of these symptoms or diseases. In specific embodiments, polynucleotides, polypeptides, or agonists or antagonists of the invention are used to treat, prevent, and/or diagnose: meningitis, Dengue, EBV, and/or hepatitis (e.g., hepatitis B). In an additional specific embodiment polynucleotides, polypeptides, or agonists or antagonists of the invention are used to treat patients nonresponsive to one or more other commercially available hepatitis vaccines. In a further specific embodiment polynucleotides, polypeptides, or agonists or antagonists of the invention are used to treat, prevent, and/or diagnose AIDS.

Similarly, bacterial or fungal agents that can cause disease or symptoms and that can be treated, prevented, and/or diagnosed by a polynucleotide or polypeptide and/or agonist or antagonist of the present invention include, but not limited to, include, but not limited to, the following Gram-Negative and Gram-positive bacteria and bacterial families and fungi: Actinomycetales (e.g., Corynebacterium, Mycobacterium, Norcardia), Cryptococcus neoformans, Aspergillosis, Bacillaceae (e.g., Anthrax, Clostridium), Bacteroidaceae, Blastomycosis, Bordetella, Borrelia (e.g., Borrelia burgdorferi), Brucellosis, Candidiasis, Campylobacter, Coccidioidomycosis, Cryptococcosis, Dermatocycoses, E. coli (e.g., Enterotoxigenic E. coli and Enterohemorrhagic E. coli), Enterobacteriaceae (Klebsiella, Salmonella (e.g., Salmonella typhi, and Salmonella paratyphi), Serratia, Yersinia), Erysipelothrix, Helicobacter, Legionellosis, Leptospirosis, Listeria, Mycoplasmatales, Mycobacterium leprae, Vibrio cholerae, Neisseriaceae (e.g., Acinetobacter, Gonorrhea, Menigococcal), Meisseria meningitidis, Pasteurellacea Infections (e.g., Actinobacillus, Heamophilus (e.g., Heamophilus influenza type B), Pasteurella), Pseudomonas, Rickettsiaceae, Chlamydiaceae, Syphilis, Shigella spp., Staphylococcal, Meningiococcal, Pneumococcal and Streptococcal (e.g., Streptococcus pneumoniae and Group B Streptococcus). These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis (e.g., mengitis types A and B), Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. Polynucleotides or polypeptides, agonists or antagonists of the invention, can be used to treat, prevent, and/or diagnose any of these symptoms or diseases. In specific embodiments, polynucleotides, polypeptides, agonists or antagonists of the invention are used to treat, prevent, and/or diagnose: tetanus, Diptheria, botulism, and/or meningitis type B.

Moreover, parasitic agents causing disease or symptoms that can be treated, prevented, and/or diagnosed by a polynucleotide or polypeptide and/or agonist or antagonist of the present invention include, but not limited to, the following families. or class: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichoinonas and Sporozoans (e.g., Plasmodium virax, Plasmodium falciparium, Plasmodium malariae and Plasmodium ovale). These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), malaria, pregnancy complications, and toxoplasmosis. polynucleotides or polypeptides, or agonists or antagonists of the invention, can be used totreat, prevent, and/or diagnose any of these symptoms or diseases. In specific embodiments, polynucleotides, polypeptides, or agonists or antagonists of the invention are used to treat, prevent, and/or diagnose malaria.

Preferably, treatment or prevention using a polypeptide or polynucleotide and/or agonist or antagonist of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide and/or agonist or antagonist of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, bums, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vasculature (including vascular and lymphatics), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide and/or agonist or antagonist of the present invention may increase regeneration of tissues difficult to heal. For example, increased tendon/ligament regeneration would quicken recovery time after damage. A polynucleotide or polypeptide and/or agonist or antagonist of the present invention could also be used prophylactically in an effort to avoid damage. Specific diseases that could be treated, prevented, and/or diagnosed include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide and/or agonist or antagonist of the present invention to proliferate and differentiate nerve cells. Diseases that could be treated, prevented, and/or diagnosed using this method include central and peripheral nervous system diseases, neuropathies, or mechanical and traumatic diseases, disorders, and/or conditions (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated, prevented, and/or diagnosed using the polynucleotide or polypeptide and/or agonist or antagonist of the present invention.

### Chemotaxis

A polynucleotide or polypeptide and/or agonist or antagonist of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide and/or agonist or antagonist of the present invention may increase chemotaxic activity of particular cells. These chemotactic molecules can then be used to treat, prevent, and/or diagnose inflammation, infection, hyperproliferative diseases, disorders, and/or conditions, or any immune system disorder by increasing the number of cells targeted to a particular location in the body. For example, chemotaxic molecules can be used to treat, prevent, and/or diagnose wounds and other trauma to tissues by attracting immune cells to the injured location. Chemotactic molecules of the present invention can also attract fibroblasts, which can be used to treat, prevent, and/or diagnose wounds.

It is also contemplated that a polynucleotide or polypeptide and/or agonist or antagonist of the present invention may inhibit chemotactic activity. These molecules could also be used totreat, prevent, and/or diagnose diseases, disorders, and/or conditions. Thus, a polynucleotide or polypeptide and/or agonist or antagonist of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E*. *coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

Additionally, the receptor to which a polypeptide of the invention binds can be identified by numerous methods known to those of skill in the art, for example, ligand panning and FACS sorting (Coligan, et al., Current Protocols in Immun., 1(2), Chapter 5, (1991)). For example, expression cloning is employed wherein polyadenylated RNA is prepared from a cell responsive to the polypeptides, for example, NIH3T3 cells which are known to contain multiple receptors for the FGF family proteins, and SC-3 cells, and a cDNA library created from this RNA is divided into pools and used to transfect COS cells or other cells that are not responsive to the polypeptides. Transfected cells which are grown on glass slides are exposed to the polypeptide of the present invention, after they have been labelled. The polypeptides can be labeled by a variety of means including iodination or inclusion of a recognition site for a site-specific protein kinase.

Following fixation and incubation, the slides are subjected to autoradiographic analysis. Positive pools are identified and sub-pools are prepared and re-transfected using an iterative sub-pooling and re-screening process, eventually yielding a single clones that encodes the putative receptor.

As an alternative approach for receptor identification, the labeled polypeptides can be photoaffinity linked with cell membrane or extract preparations that express the receptor molecule. Cross-linked material is resolved by PAGE analysis and exposed to X-ray film. The labeled complex containing the receptors of the polypeptides can be excised, resolved into peptide fragments, and subjected to protein microsequencing. The amino acid sequence obtained from microsequencing would be used to design a set of degenerate oligonucleotide probes to screen a cDNA library to identify the genes encoding the putative receptors.

Moreover, the techniques of gene-shuffling, motif-shuffling, exon-shuffling, and/or codon-shuffling (collectively referred to as "DNA shuffling") may be employed to modulate the activities of polypeptides of the invention thereby effectively generating agonists and antagonists of polypeptides of the invention. See generally, U.S. Patent Nos. 5,605,793, 5,811,238, 5,830,721, 5,834,252, and 5,837,458, and Patten, P. A., et al., Curr. Opinion Biotechnol. 8:724-33 (1997); Harayama, S. Trends Biotechnol. 16(2):76-82 (1998); Hansson, L. O., et al., J. Mol. Biol. 287:265-76 (1999); and Lorenzo, M. M. and Blasco, R. Biotechniques 24(2):308-13 (1998) (each of these patents and publications are hereby incorporated by reference). In one embodiment, alteration of polynucleotides and corresponding polypeptides of the invention may be achieved by DNA shuffling. DNA shuffling involves the assembly of two or more DNA segments into a desired polynucleotide sequence of the invention molecule by homologous, or site-specific, recombination. In another embodiment, polynucleotides and corresponding polypeptides of the invention may be alterred by being subjected to random mutagenesis by error-prone PCR, random nucleotide insertion or other methods prior to recombination. In another embodiment, one or more components, motifs, sections, parts, domains, fragments, etc., of the polypeptides of the invention may be recombined with one or more components, motifs, sections, parts, domains, fragments, etc. of one or more heterologous molecules. In preferred embodiments, the heterologous molecules are family members. In further preferred embodiments, the heterologous molecule is a growth factor such as, for example, platelet-derived growth factor (PDGF), insulin-like growth factor (IGF-I), transforming growth factor (TGF)-alpha, epidermal growth factor (EGF), fibroblast growth factor (FGF), TGF-beta, bone morphogenetic protein (BUT)-2, BMP-4, BMP-5, BMP-6, BMP-7, activins A and B, decapentaplegic(dpp), 60A, OP-2, dorsalin, growth differentiation factors (GDFs), nodal, MIS, inhibin-alpha, TGF-betal, TGF-beta2, TGF-beta3, TGF-beta5, and glial-derived neurotrophic factor (GDNF).

Other preferred fragments are biologically active fragments of the polypeptides of the invention. Biologically active fragments are those exhibiting activity similar, but not necessarily identical, to an activity of the polypeptide. The biological activity of the fragments may include an improved desired activity, or a decreased undesirable activity.

Additionally, this invention provides a method of screening compounds to identify those which modulate the action of the polypeptide of the present invention. An example of such an assay comprises combining a mammalian fibroblast cell, a the polypeptide of the present invention, the compound to be screened and 3[H] thymidine under cell culture conditions where the fibroblast cell would normally proliferate. A control assay may be performed in the absence of the compound to be screened and compared to the amount of fibroblast proliferation in the presence of the compound to determine if the compound stimulates proliferation by determining the uptake of 3[H] thymidine in each case. The amount of fibroblast cell proliferation is measured by liquid scintillation chromatography which measures the incorporation of 3[H] thymidine. Both agonist and antagonist compounds may be identified by this procedure.

In another method, a mammalian cell or membrane preparation expressing a receptor for a polypeptide of the present invention is incubated with a labeled polypeptide of the present invention in the presence of the compound. The ability of the compound to enhance or block this interaction could then be measured. Alternatively, the response of a known second messenger system following interaction of a compound to be screened and the receptor is measured and the ability of the compound to bind to the receptor and elicit a second messenger response is measured to determine if the compound is a potential agonist or antagonist. Such second messenger systems include but are not limited to, cAMP guanylate cyclase, ion channels or phosphoinositide hydrolysis.

All of these above assays can be used as diagnostic or prognostic markers. The molecules discovered using these assays can be used to treat, prevent, and/or diagnose disease or to bring about a particular result in a patient (e.g., blood vessel growth) by activating or inhibiting the polypeptide/molecule. Moreover, the assays can discover agents which may inhibit or enhance the production of the polypeptides of the invention from suitably manipulated cells or tissues. Therefore, the invention includes a method of identifying compounds which bind to the polypeptides of the invention comprising the steps of: (a) incubating a candidate binding compound with the polypeptide; and (b) determining if binding has occurred. Moreover, the invention includes a method of identifying agonists/antagonists comprising the steps of: (a) incubating a candidate compound with the polypeptide, (b) assaying a biological activity, and (b) determining if a biological activity of the polypeptide has been altered.

Also, one could identify molecules bind a polypeptide of the invention experimentally by using the beta-pleated sheet regions contained in the polypeptide sequence of the protein. Accordingly, specific embodiments of the invention are directed to polynucleotides encoding polypeptides which comprise, or alternatively consist of, the amino acid sequence of each beta pleated sheet regions in a disclosed polypeptide sequence. Additional embodiments of the invention are directed to polynucleotides encoding polypeptides which comprise, or alternatively consist of, any combination or all of contained in the polypeptide sequences of the invention. Additional preferred embodiments of the invention are directed to polypeptides which comprise, or alternatively consist of, the amino acid sequence of each of the beta pleated sheet regions in one of the polypeptide sequences of the invention. Additional embodiments of the invention are directed to polypeptides which comprise, or alternatively consist of, any combination or all of the beta pleated sheet regions in one of the polypeptide sequences of the invention.

### Targeted Delivery

In another embodiment, the invention provides a method of delivering compositions to targeted cells expressing a receptor for a polypeptide of the invention, or cells expressing a cell bound form of a polypeptide of the invention.

As discussed herein, polypeptides or antibodies of the invention may be associated with heterologous polypeptides, heterologous nucleic acids, toxins, or prodrugs via hydrophobic, hydrophilic, ionic and/or covalent interactions. In one embodiment, the invention provides a method for the specific delivery of compositions of the invention to cells by administering polypeptides of the invention (including antibodies) that are associated with heterologous polypeptides or nucleic acids. In one example, the invention provides a method for delivering a therapeutic protein into the targeted cell. In another example, the invention provides a method for delivering a single stranded nucleic acid (e.g., antisense or ribozymes) or double stranded nucleic acid (e.g., DNA that can integrate into the cell's genome or replicate episomally and that can be transcribed) into the targeted cell.

In another embodiment, the invention provides a method for the specific destruction of cells (e.g., the destruction of tumor cells) by administering polypeptides of the invention (e.g., polypeptides of the invention or antibodies of the invention) in association with toxins or cytotoxic prodrugs.

By "toxin" is meant compounds that bind and activate endogenous cytotoxic effector systems, radioisotopes, holotoxins, modified toxins, catalytic subunits of toxins, or any molecules or enzymes not normally present in or on the surface of a cell that under defined conditions cause the cell's death. Toxins that may be used according to the methods of the invention include, but are not limited to, radioisotopes known in the art, compounds such as, for example, antibodies (or complement fixing containing portions thereof) that bind an inherent or induced endogenous cytotoxic effector system, thymidine kinase, endonuclease, RNAse, alpha toxin, ricin, abrin, *Pseudomonas* exotoxin A, diphtheria toxin, saporin, momordin, gelonin, pokeweed antiviral protein, alpha-sarcin and cholera toxin. By "cytotoxic prodrug" is meant a non-toxic compound that is converted by an enzyme, normally present in the cell, into a cytotoxic compound. Cytotoxic prodrugs that may be used according to the methods of the invention include, but are not limited to, glutamyl derivatives of benzoic acid mustard alkylating agent, phosphate derivatives of etoposide or mitomycin C, cytosine arabinoside, daunorubisin, and phenoxyacetamide derivatives of doxorubicin.

### Drug Screening

Further contemplated is the use of the polypeptides of the present invention, or the polynucleotides encoding these polypeptides, to screen for molecules which modify the activities of the polypeptides of the present invention. Such a method would include contacting the polypeptide of the present invention with a selected compound(s) suspected of having antagonist or agonist activity, and assaying the activity of these polypeptides following binding.

This invention is particularly useful for screening therapeutic compounds by using the polypeptides of the present invention, or binding fragments thereof, in any of a variety of drug screening techniques. The polypeptide or fragment employed in such a test may be affixed to a solid support, expressed on a cell surface, free in solution, or located intracellularly. One method of drug screening utilizes eukaryotic or prokaryotic host cells which are stably transformed with recombinant nucleic acids expressing the polypeptide or fragment. Drugs are screened against such transformed cells in competitive binding assays. One may measure, for example, the formulation of complexes between the agent being tested and a polypeptide of the present invention.

Thus, the present invention provides methods of screening for drugs or any other agents which affect activities mediated by the polypeptides of the present invention. These methods comprise contacting such an agent with a polypeptide of the present invention or a fragment thereof and assaying for the presence of a complex between the agent and the polypeptide or a fragment thereof, by methods well known in the art. In such a competitive binding assay, the agents to screen are typically labeled. Following incubation, free agent is separated from that present in bound form, and the amount of free or uncomplexed label is a measure of the ability of a particular agent to bind to the polypeptides of the present invention.

Another technique for drug screening provides high throughput screening for compounds having suitable binding affinity to the polypeptides of the present invention, and is described in great detail in European Patent Application 84/03564, published on September 13, 1984, which is incorporated herein by reference herein. Briefly stated, large numbers of different small peptide test compounds are synthesized on a solid substrate, such as plastic pins or some other surface. The peptide test compounds are reacted with polypeptides of the present invention and washed. Bound polypeptides are then detected by methods well known in the art. Purified polypeptides are coated directly onto plates for use in the aforementioned drug screening techniques. In addition, non-neutralizing antibodies may be used to capture the peptide and immobilize it on the solid support.

This invention also contemplates the use of competitive drug screening assays in which neutralizing antibodies capable of binding polypeptides of the present invention specifically compete with a test compound for binding to the polypeptides or fragments thereof. In this manner, the antibodies are used to detect the presence of any peptide which shares one or more antigenic epitopes with a polypeptide of the invention.

### Polypeptides of the Invention Binding Peptides and Other Molecules

The invention also encompasses screening methods for identifying polypeptides and nonpolypeptides that bind polypeptides of the invention, and the polypeptide of the invention binding molecules identified thereby. These binding molecules are useful, for example, as agonists and antagonists of the polypeptides of the invention. Such agonists and antagonists can be used, in accordance with the invention, in the therapeutic embodiments described in detail, below.

This method comprises the steps of:
a. contacting a polypeptide of the invention with a plurality of molecules; and
b. identifying a molecule that binds the polypeptide of the invention.

The step of contacting the polypeptide of the invention with the plurality of molecules may be effected in a number of ways. For example, one may contemplate immobilizing the polypeptide of the invention on a solid support and bringing a solution of the plurality of molecules in contact with the immobilized polypeptide of the invention. Such a procedure would be akin to an affinity chromatographic process, with the affinity matrix being comprised of the immobilized polypeptide of the invention. The molecules having a selective affinity for the polypeptide of the invention can then be purified by affinity selection. The nature of the solid support, process for attachment of the polypeptide of the invention to the solid support, solvent, and conditions of the affinity isolation or selection are largely conventional and well known to those of ordinary skill in the art.

Alternatively, one may also separate a plurality of polypeptides into substantially separate fractions comprising a subset of or individual polypeptides. For instance, one can separate the plurality of polypeptides by gel electrophoresis, column chromatography, or like method known to those of ordinary skill for the separation of polypeptides. The individual polypeptides can also be produced by a transformed host cell in such a way as to be expressed on or about its outer surface (e.g., a recombinant phage). Individual isolates can then be "probed" by the polypeptide of the invention, optionally in the presence of an inducer should one be required for expression, to determine if any selective affinity interaction takes place between the polypeptide of the invention and the individual clone. Prior to contacting the polypeptide of the invention with each fraction comprising individual polypeptides, the polypeptides could first be transferred to a solid support for additional convenience. Such a solid support may simply be a piece of filter membrane, such as one made of nifrocellulose or nylon. In this manner, positive clones could be identified from a collection of transformed host cells of an expression library, which harbor a DNA construct encoding a polypeptide having a selective affinity for a polypeptide of the invention. Furthermore, the amino acid sequence of the polypeptide having a selective affinity for the polypeptide of the invention can be determined directly by conventional means or the coding sequence of the DNA encoding the polypeptide can frequently be determined more conveniently. The primary sequence can then be deduced from the corresponding DNA sequence. If the amino acid sequence is to be determined from the polypeptide itself, one may use microsequencing techniques. The sequencing technique may include mass spectroscopy.

In certain situations, it may be desirable to wash away any unbound polypeptide of the invention, or altemtatively, unbound polypeptides, from a mixture of the polypeptide of the invention and the plurality of polypeptides prior to attempting to determine or to detect the presence of a selective affinity interaction. Such a wash step may be particularly desirable when the polypeptide of the invention or the plurality of polypeptides is bound to a solid support.

The plurality of molecules provided according to this method may be provided by way of diversity libraries, such as random or combinatorial peptide or nonpeptide libraries which can be screened for molecules that specifically bind to a polypeptide of the invention. Many libraries are known in the art that can be used, e.g., chemically synthesized libraries, recombinant (e.g., phage display libraries), and in vitro translation-based libraries. Examples of chemically synthesized libraries are described in Fodor et al., 1991, Science 251:767-773; Houghten et al., 1991, Nature 354:84-86; Lam et al., 1991, Nature 354:82-84; Medynski, 1994, Bio/Technology 12:709-710;Gallop et al., 1994, J. Medicinal Chemistry 37(9):1233-1251; Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; Salmon et al., 1993, Proc. Natl. Acad. Sci. USA 90:11708-11712; PCT Publication No. WO 93/20242; and Brenner and Lerner, 1992, Proc. Natl. Acad. Sci. USA 89:5381-5383.

Examples of phage display libraries are described in Scott and Smith, 1990, Science 249:386-390; Devlin et al., 1990, Science, 249:404-406; Christian, R. B., et al., 1992, J. Mol. Biol. 227:711-718); Lenstra, 1992, J. Immunol. Meth. 152:149-157; Kay et al., 1993, Gene 128:59-65; and PCT Publication No. WO 94/18318 dated Aug. 18,1994.

In vitro translation-based libraries include but are not limited to those described in PCT Publication No. WO 91/05058 dated Apr. 18, 1991; and Mattheakis et al., 1994, Proc. Natl. Acad. Sci. USA 91:9022-9026.

By way of examples of nonpeptide libraries, a benzodiazepine library (see e.g., Bunin et al., 1994, Proc. Natl. Acad. Sci. USA 91:4708-4712) can be adapted for use. Peptoid libraries (Simon et al., 1992, Proc. Natl. Acad. Sci. USA 89:9367-9371) can also be used. Another example of a library that can be used, in which the amide functionalities in peptides have been permethylated to generate a chemically transformed combinatorial library, is described by Ostresh et al. (1994, Proc. Natl. Acad. Sci. USA 91:11138-11142).

The variety of non-peptide libraries that are useful in the present invention is great. For example, Ecker and Crooke, 1995, Bio/Technology 13:351-360 list benzodiazepines, hydantoins, piperazinediones, biphenyls, sugar analogs, beta-mercaptoketones, arylacetic acids, acylpiperidines, benzopyrans, cubanes, xanthines, aminimides, and oxazolones as among the chemical species that form the basis of various libraries.

Non-peptide libraries can be classified broadly into two types: decorated monomers and oligomers. Decorated monomer libraries employ a relatively simple scaffold structure upon which a variety functional groups is added. Often the scaffold will be a molecule with a known useful pharmacological activity. For example, the scaffold might be the benzodiazepine structure.

Non-peptide oligomer libraries utilize-a large number of monomers that are assembled together in ways that create new shapes that depend on the order of the monomers. Among the monomer units that have been used are carbamates, pyrrolinones, and inorpholinos. Peptoids, peptide-like oligomers in which the side chain is attached to the alpha amino group rather than the alpha carbon, form the basis of another version of non-peptide oligomer libraries. The first non-peptide oligomer libraries utilized a single type of monomer and thus contained a repeating backbone. Recent libraries have utilized more than one monomer, giving the libraries added flexibility.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992; BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Pat. No. 5,096,815, U.S. Pat. No. 5,223,409, and U.S. Pat. No. 5,198,346, all to Ladner et al.; Rebar and Pabo, 1993, Science 263:671-673; and CT Publication No. WO 94/18318.

In a specific embodiment, screening to identify a molecule that binds a polypeptide of the invention can be carried out by contacting the library members with a polypeptide of the invention immobilized on a solid phase and harvesting those library members that bind to the polypeptide of the invention. Examples of such screening methods, termed "panning" techniques are described by way of example in Parmley and Smith, 1988, Gene 73:305-318; Fowlkes et al., 1992, BioTechniques 13:422-427; PCT Publication No. WO 94/18318; and in references cited herein.

In another embodiment, the two-hybrid system for selecting interacting proteins in yeast (Fields and Song, 1989, Nature 340:245-246; Chien et al., 1991, Proc. Natl. Acad. Sci. USA 88:9578-9582) can be used to identify molecules that specifically bind to a polypeptide of the invention.

Where the polypeptide of the invention binding molecule is a polypeptide, the polypeptide can be conveniently selected from any peptide library, including random peptide libraries, combinatorial peptide libraries, or biased peptide libraries. The term "biased" is used herein to mean that the method of generating the library is manipulated so as to restrict one or more parameters that govern the diversity of the resulting collection of molecules, in this case peptides.

Thus, a truly random peptide library would generate a collection of peptides in which the probability of finding a particular amino acid at a given position of the peptide is the same for all 20 amino acids. A bias can be introduced into the library, however, by specifying, for example, that a lysine occur every fifth amino acid or that positions 4, 8, and 9 of a decapeptide library be fixed to include only arginine. Clearly, many types of biases can be contemplated, and the present invention is not restricted to any particular bias. Furthermore, the present invention contemplates specific types of peptide libraries, such as phage displayed peptide libraries and those that utilize a DNA construct comprising a lambda phage vector with a DNA insert.

As mentioned above, in the case of a polypeptide of the invention binding molecule that is a polypeptide, the polypeptide may have about 6 to less than about 60 amino acid residues, preferably about 6 to about 10 amino acid residues, and most preferably, about 6 to about 22 amino acids. In another embodiment, a polypeptide of the invention binding polypeptide has in the range of 15-100 amino acids, or 20-50 amino acids.

The selected polypeptide of the invention binding polypeptide can be obtained by chemical synthesis or recombinant expression:

### Antisense And Ribozyme (Antagonists)

In specific embodiments, antagonists according to the present invention are nucleic acids corresponding to the sequences contained in SEQ ID NO:X, or the complementary strand thereof, and/or to nucleotide sequences contained a deposited clone. In one embodiment, antisense sequence is generated internally by the organism, in another embodiment, the antisense sequence is separately administered (see, for example, O'Connor, Neurochem., 56:560 (1991). Oligodeoxynucleotides as Anitsense Inhibitors of Gene Expression, CRC Press, Boca Raton, FL (1988). Antisense technology can be used to control gene expression through antisense DNA or RNA, or through triple-helix formation. Antisense techniques are discussed for example, in Okano, Neurochem., 56:560 (1991); Oligodeoxynucleotides as Antisense Inhibitors of Gene Expression, CRC Press, Boca Raton, FL (1988). Triple helix formation is discussed in, for instance, Lee et al., Nucleic Acids Research, 6:3073 (1979); Cooney et al., Science, 241:456 (1988); and Dervan et al., Science; 251:1300 (1991). The methods are based on binding of a polynucleotide to a complementary DNA or RNA

For example, the use of c-myc and c-myb antisense RNA constructs to inhibit the growth of the non-lymphocytic leukemia cell line HL-60 and other cell lines was previously described. (Wickstrom et al. (1988); Anfossi et al. (1989)). These experiments were performed in vitro by incubating cells with the oligoribonucleotide. A similar procedure for in vivo use is described in WO 91/15580. Briefly, a pair of oligonucleotides for a given antisense RNA is produced as follows: A sequence complimentary to the first 15 bases of the open reading frame is flanked by an EcoR1 site on the 5 end and a HindIII site on the 3 end. Next, the pair of oligonucleotides is heated at 90°C for one minute and then annealed in 2X ligation buffer (20mM TRIS HCl pH 7.5, 10mM MgC12, 10MM dithiothreitol (DTT) and 0.2 mM ATP) and then ligated to the EcoR1/Hind III site of the retroviral vector PMV7 (WO 91/15580).

For example, the 5' coding portion of a polynucleotide that encodes the mature polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of the receptor. The antisense RNA oligonucleotide hybridizes to the mRNA in vivo and blocks translation of the mRNA molecule into receptor polypeptide.

In one embodiment, the antisense nucleic acid of the invention is produced intracellularly by transcription from an exogenous sequence. For example, a vector or a portion thereof, is transcribed, producing an antisense nucleic acid (RNA) of the invention. Such a vector would contain a sequence encoding the antisense nucleic acid of the invention. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the sequence encoding a polypeptide of the invention, or fragments thereof, can be by any promoter known in the art to act in vertebrate, preferably human cells. Such promoters can be inducible or constitutive. Such promoters include, but are not limited to, the SV40 early promoter region (Bemoist and Chambon, Nature, 29:304-310 (1981), the promoter contained in the 3' long terminal repeat of Rous sarcoma virus (Yamamoto et al., Cell, 22:787-797 (1980), the herpes thymidine promoter (Wagner et al., Proc. Natl. Acad. Sci. U.S.A., 78:1441-1445 (1981), the regulatory sequences of the metallothionein gene (Brinster et al., Nature, 296:39-42 (1982)), etc.

The antisense nucleic acids of the invention comprise a sequence complementary to at least a portion of an RNA transcript of a gene of interest. However, absolute complementarity, although preferred, is not required. A sequence "complementary to at least a portion of an RNA," referred to herein, means a sequence having sufficient complementarity to be able to hybridize with the RNA, forming a stable duplex; in the case of double stranded antisense nucleic acids of the invention, a single strand of the duplex DNA may thus be tested, or triplex formation may be assayed. The ability to hybridize will depend on both the degree of complementarity and the length of the antisense nucleic acid Generally, the larger the hybridizing nucleic acid, the more base mismatches with a RNA sequence of the invention it may contain and still form a stable duplex (or triplex as the case may be). One skilled in the art can ascertain a tolerable degree of mismatch by use of standard procedures to determine the melting point of the hybridized complex.

Oligonucleotides that are complementary to the 5' end of the message, e.g., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. See generally, Wagner, R., *Nature,* 372:333-335 (1994). Thus, oligonucleotides complementary to either the 5' - or 3'-non- translated, non-coding regions of a polynucleotide sequence of the invention could be used in an antisense approach to inhibit translation of endogenous mRNA. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5' -, 3' - or coding region of mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

The polynucleotides of the invention can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors in vivo), or agents facilitating transport across the cell membrane (see, e.g., Letsinger et al., Proc. Natl. Acad. Sci. U.S.A. 86:6553-6556 (1989); Lemaitre et al., Proc. Natl. Acad. Sci., 84:648-652 (1987); PCT Publication NO: WO88/09810, published December 15, 1988) or the blood-brain barrier (see, e.g., PCT Publication NO: WO89/10134, published April 25, 1988), hybridization-triggered cleavage agents. (See, e.g., Krol et al., BioTechniques, 6:958-976 (1988)) or intercalating agents. (See, e.g., Zon, Pharm. Res., 5:539-549 (1988)). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including, but not limited to, 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including, but not limited to, arabinose, 2-fluoroarabinose, xylulose, and hexose.

In yet another embodiment, the antisense oligonucleotide comprises at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an a-anomeric oligonucleotide. An a-anomeric oligonucleotide forms specific double-stranded hybrids with complementary RNA in which, contrary to the usual b-units, the strands run parallel to each other (Gautier et al., Nucl. Acids Res., 15:6625-6641 (1987)). The oligonucleotide is a 2-0-methylribonucleotide (Inoue et al., Nucl. Acids Res., 15:6131-6148 (1987)), or a chimeric RNA-DNA analogue (Inoue et al., FEBS Lett. 215:327-330 (1987)).

Polynucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al. (Nucl. Acids Res., 16:3209 (1988)), methylphosphonate oligonucleotides can be prepared by use of controlled pore glass polymer supports (Sarin et al., Proc. Natl. Acad. Sci. U.S.A., 85:7448-7451 (1988)), etc.

While antisense nucleotides complementary to the coding region sequence of the invention could be used, those complementary to the transcribed untranslated region are most preferred.

Potential antagonists according to the invention also include catalytic RNA, or a ribozyme (See, e.g., PCT International Publication WO 90/11364, published October 4, 1990; Sarver et al, Science; 247:1222-1225 (1990). While ribozymes that cleave mRNA at site specific recognition sequences can be used to destroy mRNAs corresponding to the polynucleotides of the invention, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5' -UG-3' . The construction and production of hammerhead ribozymes is well known in the art and is described more fully in Haseloff and Gerlach, Nature, 334:585-591 (1988). There are numerous potential hammerhead ribozyme cleavage sites within each nucleotide sequence disclosed in the sequence listing. Preferably, the ribozyme is engineered so that the cleavage recognition site is located near the 5' end of the mRNA corresponding to the polynucleotides of the invention; i.e., to increase efficiency and minimize the intracellular accumulation of non-functional mRNA transcripts.

As in the antisense approach, the ribozymes of the invention can be composed of modified oligonucleotides (e.g. for improved stability, targeting, etc.) and should be delivered to cells which express the polynucleotides of the invention in vivo. DNA constructs encoding the ribozyme may be introduced into the cell in the same manner as described above for the introduction of antisense encoding DNA. A preferred method of delivery involves using a DNA construct "encoding" the ribozyme under the control of a strong constitutive promoter, such as, for example, pol III or pol II promoter, so that transfected cells will produce sufficient quantities of the ribozyme to destroy endogenous messages and inhibit translation. Since ribozymes unlike antisense molecules, are catalytic, a lower intracellular concentration is required for efficiency.

Antagonist/agonist compounds may be employed to inhibit the cell growth and proliferation effects of the polypeptides of the present invention on neoplastic cells and tissues, i.e. stimulation of angiogenesis of tumors, and, therefore, retard or prevent abnormal cellular growth and proliferation, for example, in tumor formation or growth.

The antagonist/agonist may also be employed to prevent hyper-vascular diseases, and prevent the proliferation of epithelial lens cells after extracapsular cataract surgery. Prevention of the mitogenic activity of the polypeptides of the present invention may also be desirous in cases such as restenosis after balloon angioplasty.

The antagonist/agonist may also be employed to prevent the growth of scar tissue during wound healing.

The antagonist/agonist may also be employed to treat, prevent, and/or diagnose the diseases described herein.

Thus, the invention provides a method of treating or preventing diseases, disorders, and/or conditions, including but not limited to the diseases, disorders, and/or conditions listed throughout this application, associated with overexpression of a polynucleotide of the present invention by administering to a patient (a) an antisense molecule directed to the polynucleotide of the present invention, and/or (b) a ribozyme directed to the polynucleotide of the present invention. invention, and/or (b) a ribozyme directed to the polynucleotide of the present invention

### Other Activities

The polypeptide of the present invention, as a result of the ability to stimulate vascular endothelial cell growth, may be employed in treatment for stimulating re-vascularization of ischemic tissues due to various disease conditions such as thrombosis, arteriosclerosis, and other cardiovascular conditions. These polypeptide may also be employed to stimulate angiogenesis and limb regeneration, as discussed above.

The polypeptide may also be employed for treating wounds due to injuries, burns, post-operative tissue repair, and ulcers since they are mitogenic to various cells of different origins, such as' fibroblast cells and skeletal muscle cells, and therefore, facilitate the repair or replacement of damaged or diseased tissue.

The polypeptide of the present invention may also be employed stimulate neuronal growth and to treat, prevent, and/or diagnose neuronal damage which occurs in certain neuronal disorders or neuro-degenerative conditions such as Alzheimer's disease, Parkinson's disease, and' AIDS-related complex. The polypeptide of the invention may have the ability to stimulate chondrocyte growth, therefore, they may be employed to enhance bone and periodontal regeneration and aid in tissue transplants or bone grafts.

The polypeptide of the present invention may be also be employed to prevent skin aging due to sunburn by stimulating keratinocyte growth.

The polypeptide of the invention may also be employed for preventing hair loss, since FGF family members activate.hair-forming cells and promotes melanocyte growth. Along the same lines, the polypeptides of the present invention may be employed to stimulate growth and differentiation of hematopoietic cells and bone marrow cells when used in combination with other cytokines.

The polypeptide of the invention may also be employed to maintain organs before transplantation or for supporting cell culture of primary tissues.

The polypeptide of the present invention may also be employed for inducing tissue of mesodermal origin to differentiate in early embryos.

The polypeptide or polynucleotides and/or agonist or antagonists of the present invention may also increase or decrease the differentiation or proliferation of embryonic stem cells, besides, as discussed above, hematopoietic lineage.

The polypeptide or polynucleotides and/or agonist or antagonists of the present invention may also be used to modulate mammalian characteristics, such as body height, weight, hair color, eye color, skin, percentage of adipose tissue, pigmentation, size, and shape (e.g., cosmetic surgery). Similarly, polypeptides or polynucleotides and/or agonist or antagonists of the present invention may be used to modulate mammalian metabolism affecting catabolism, anabolism, processing, utilization, and storage of energy.

Polypeptide or polynucleotides and/or agonist or antagonists of the present invention may be used to change a mammal's mental state or physical state by influencing biorhythms, caricadic rhythms, depression (including depressive diseases, disorders, and/or conditions), tendency for violence, tolerance for pain, reproductive capabilities (preferably by Activin or Inhibin-like activity), hormonal or endocrine levels, appetite, libido, memory, stress, or other cognitive qualities.

Polypeptide or polynucleotides and/or agonist or antagonists of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X
wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

The above-recited applications have uses in a wide variety of hosts. Such hosts include, but are not limited to, human, murine, rabbit, goat, guinea pig, carnel, horse, mouse, rat, hamster, pig, micro-pig, chicken, goat, cow, sheep, dog, cat, non-human primate, and human. In specific embodiments, the host is a mouse, rabbit, goat, guinea pig, chicken, rat, hamster, pig, sheep, dog or cat. In preferred embodiments, the host is a mammal. In most preferred embodiments, the host is a human.

In specific embodiments of the invention, for each "Contig ID" listed in the fourth column of Table 5, preferably excluded are one or more polynucleotides comprising, or alternatively consisting of, a nucleotide sequence referenced in the fifth column of Table 5 and described by the general formula of a-b, whereas a and b are uniquely determined for the corresponding SEQ ID NO:X referred to in column 3 of Table 5. Further specific embodiments are directed to polynucleotide sequences excluding one, two, three, four, or more of the specific polynucleotide sequences referred to in the fifth column of Table 5. In no way is this listing meant to encompass all of the sequences which may be excluded by the general formula, it is just a representative example. All references available through these accessions are hereby incorporated by reference in their entirety.

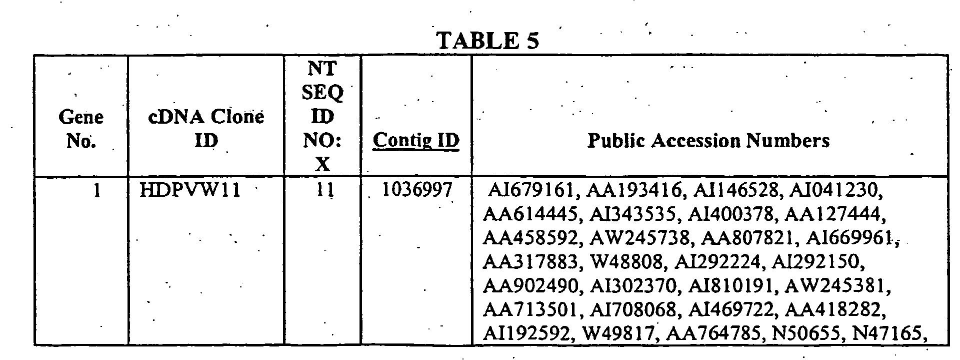

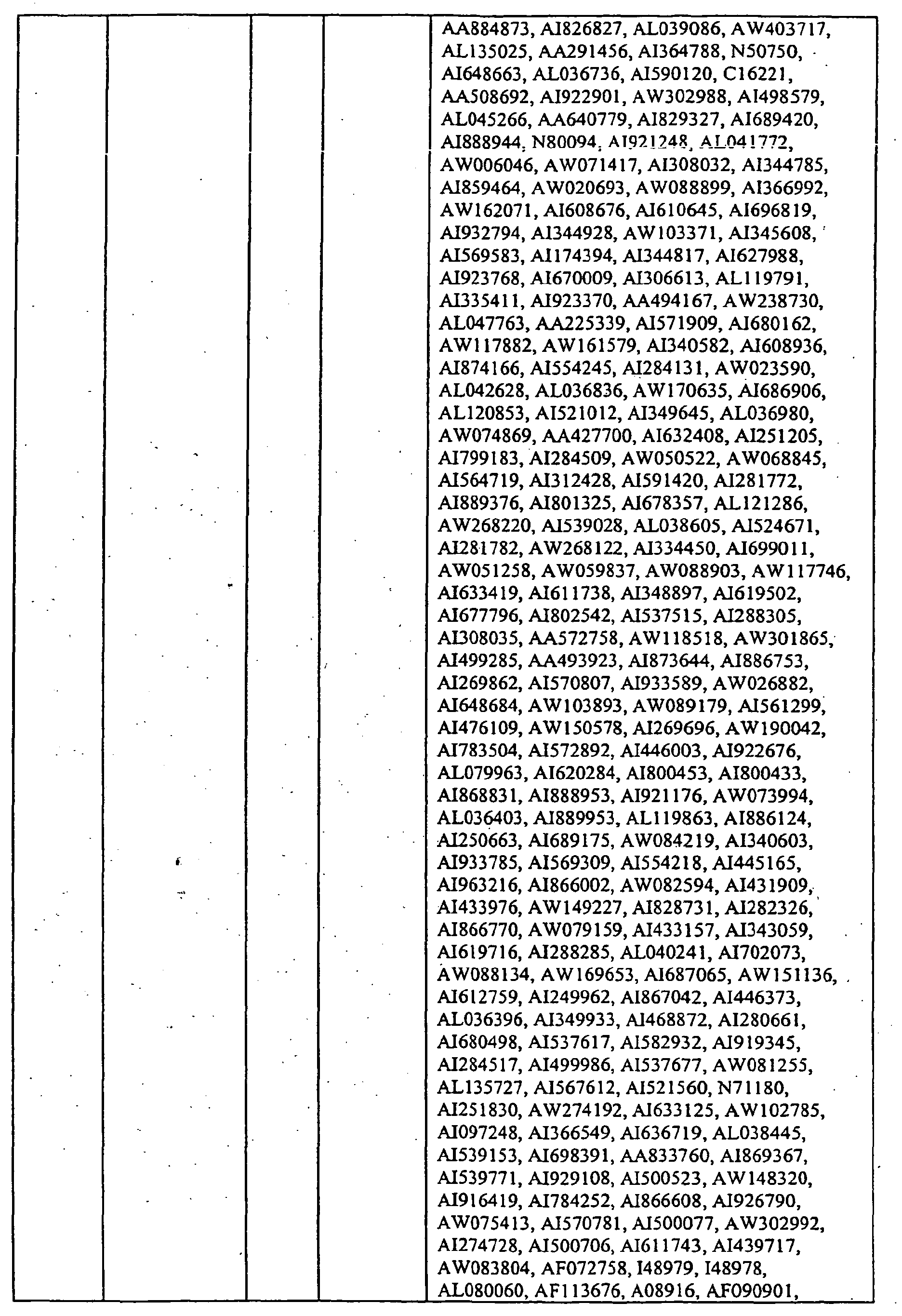

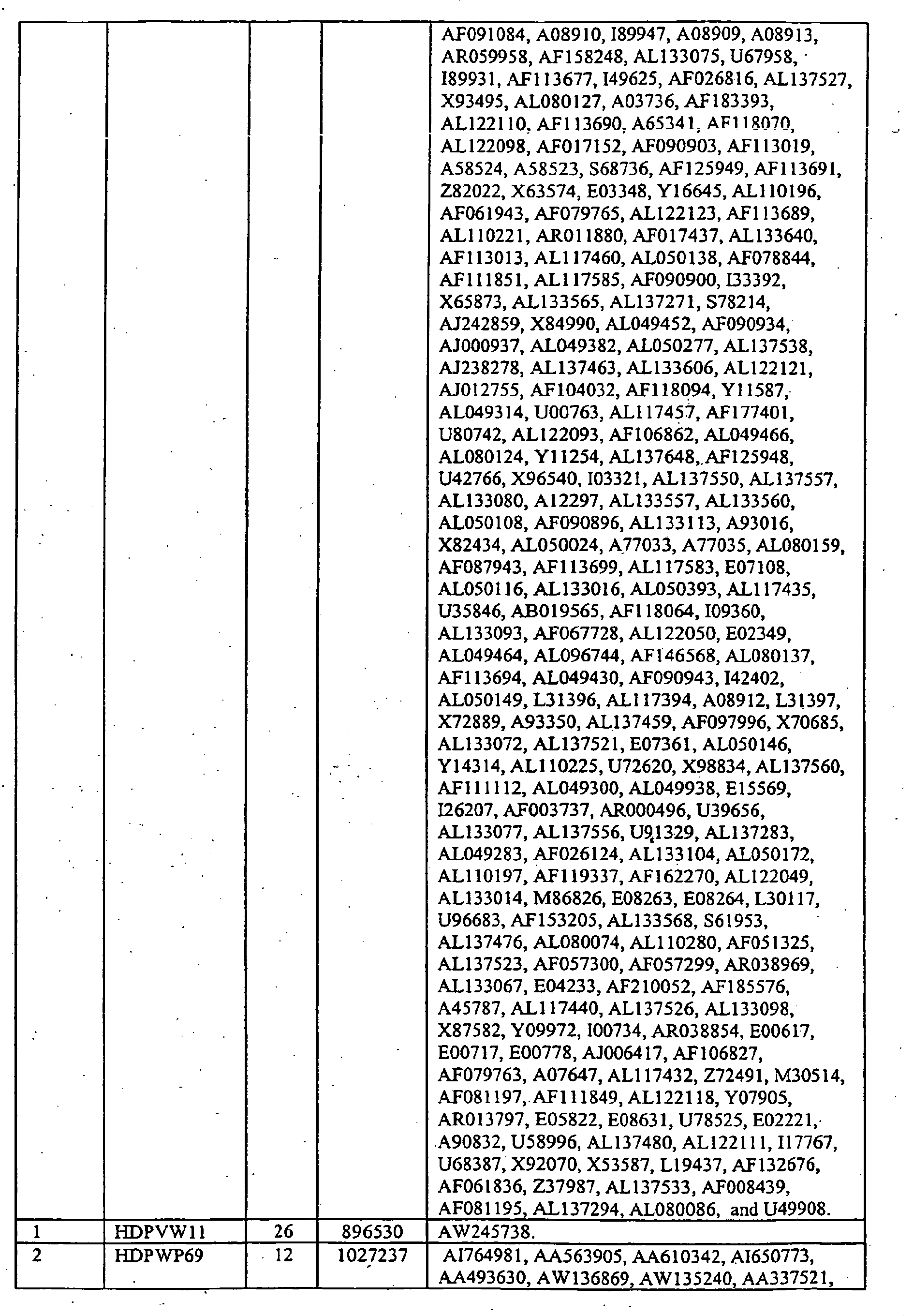

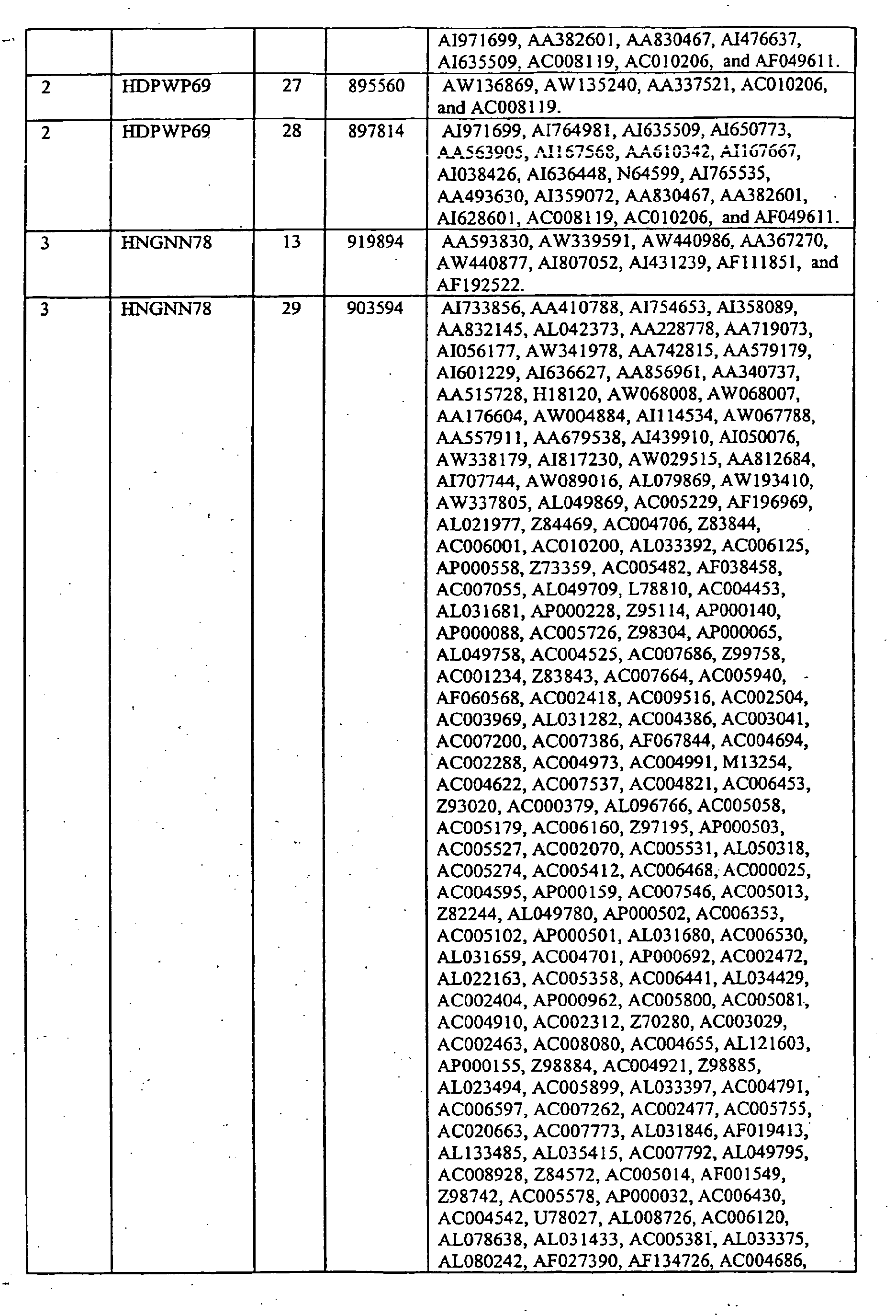

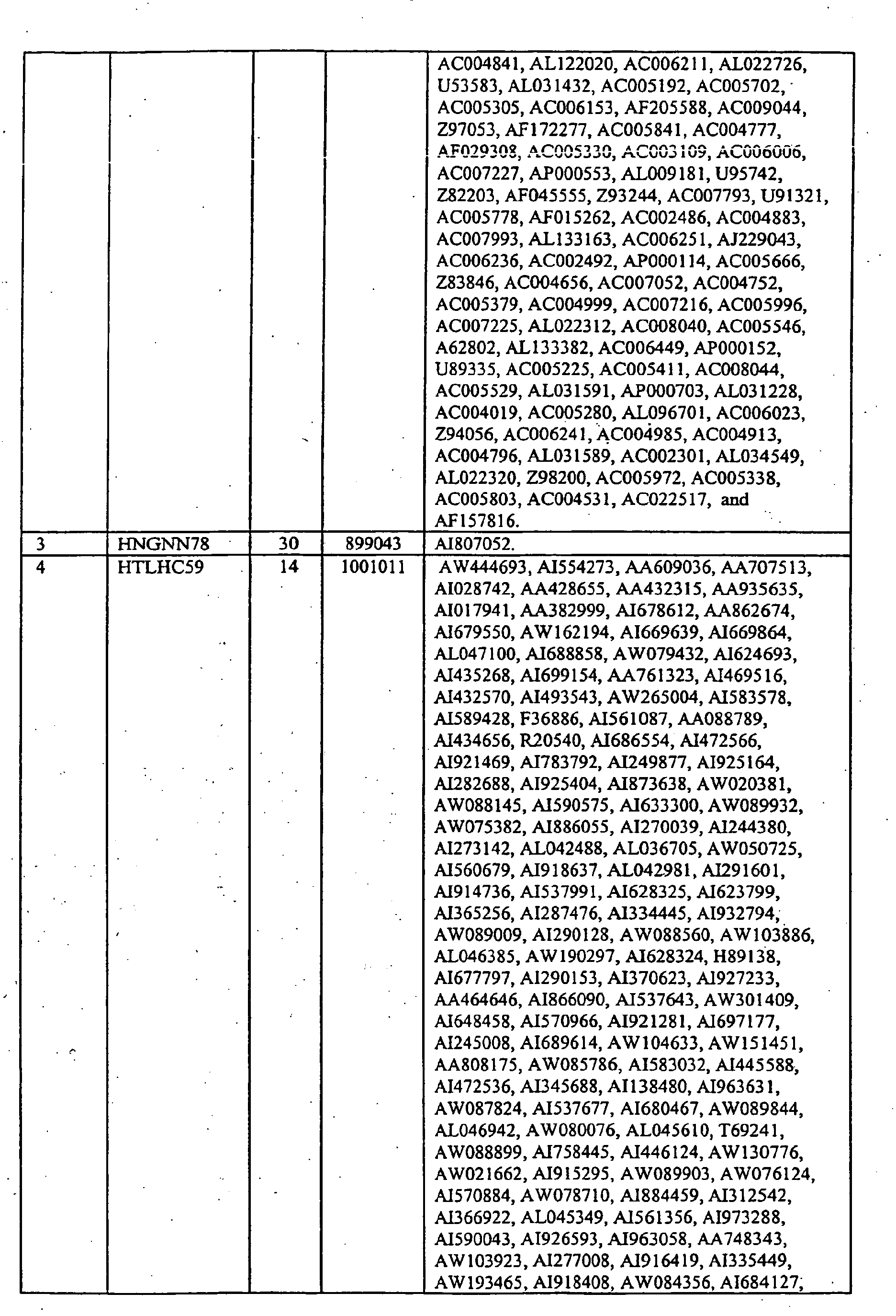

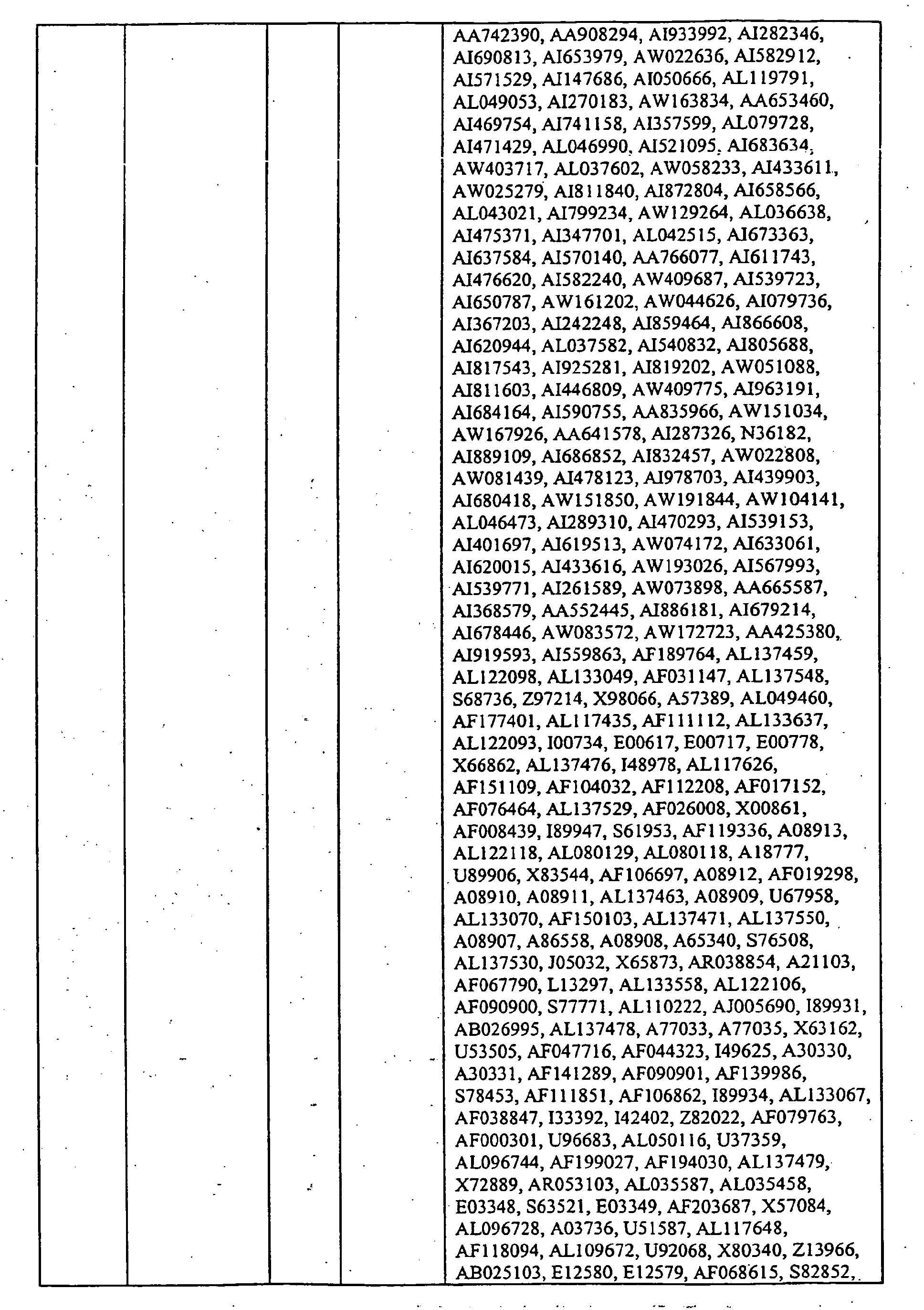

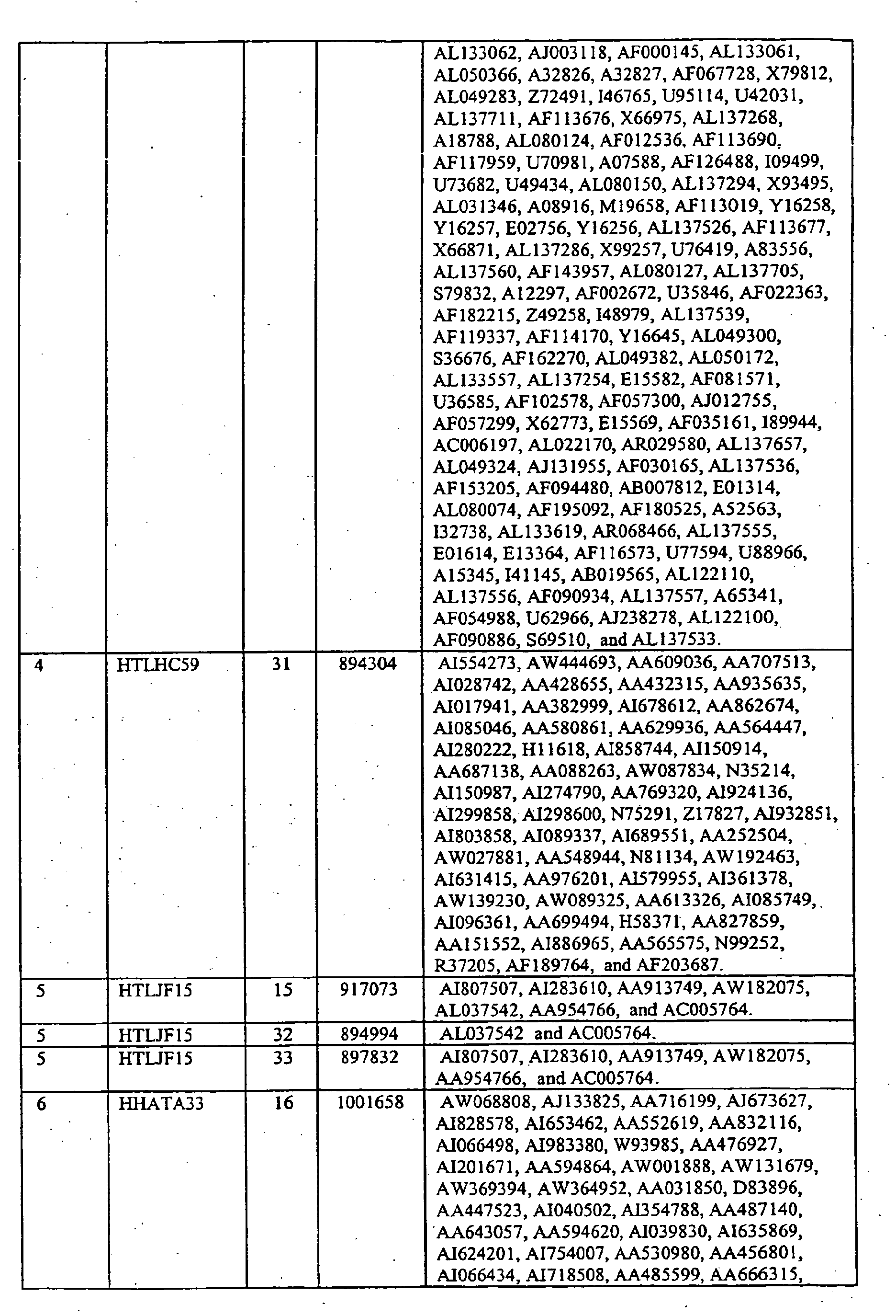

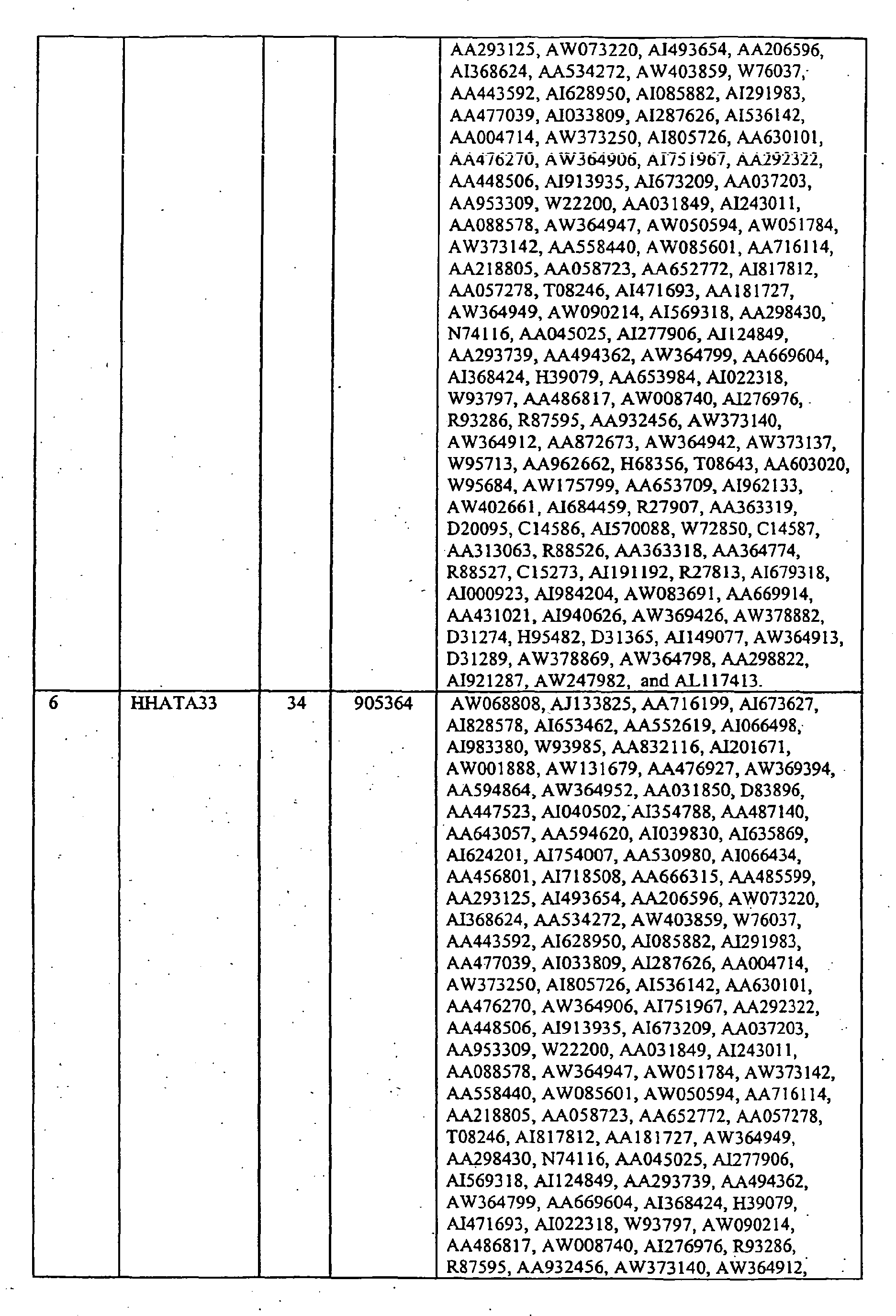

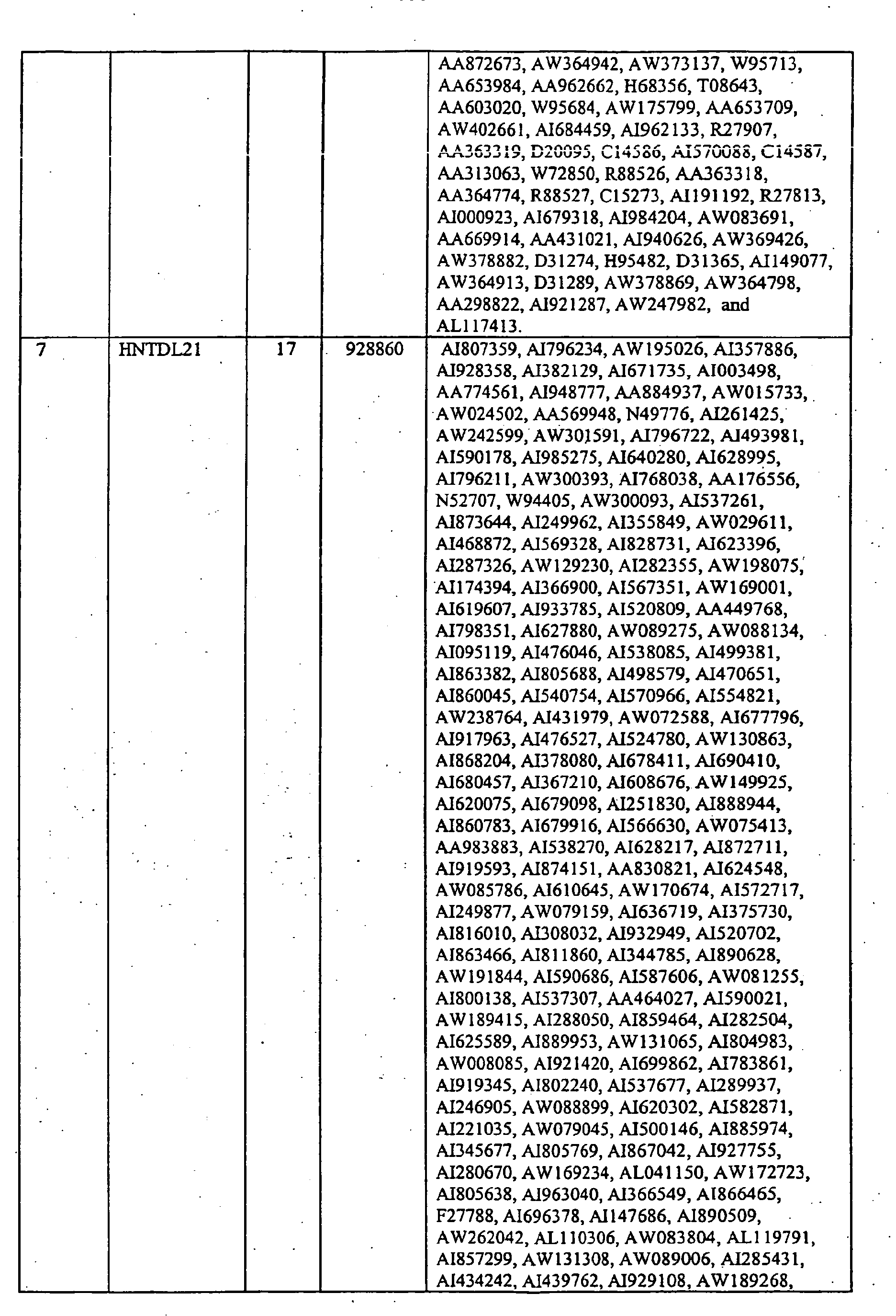

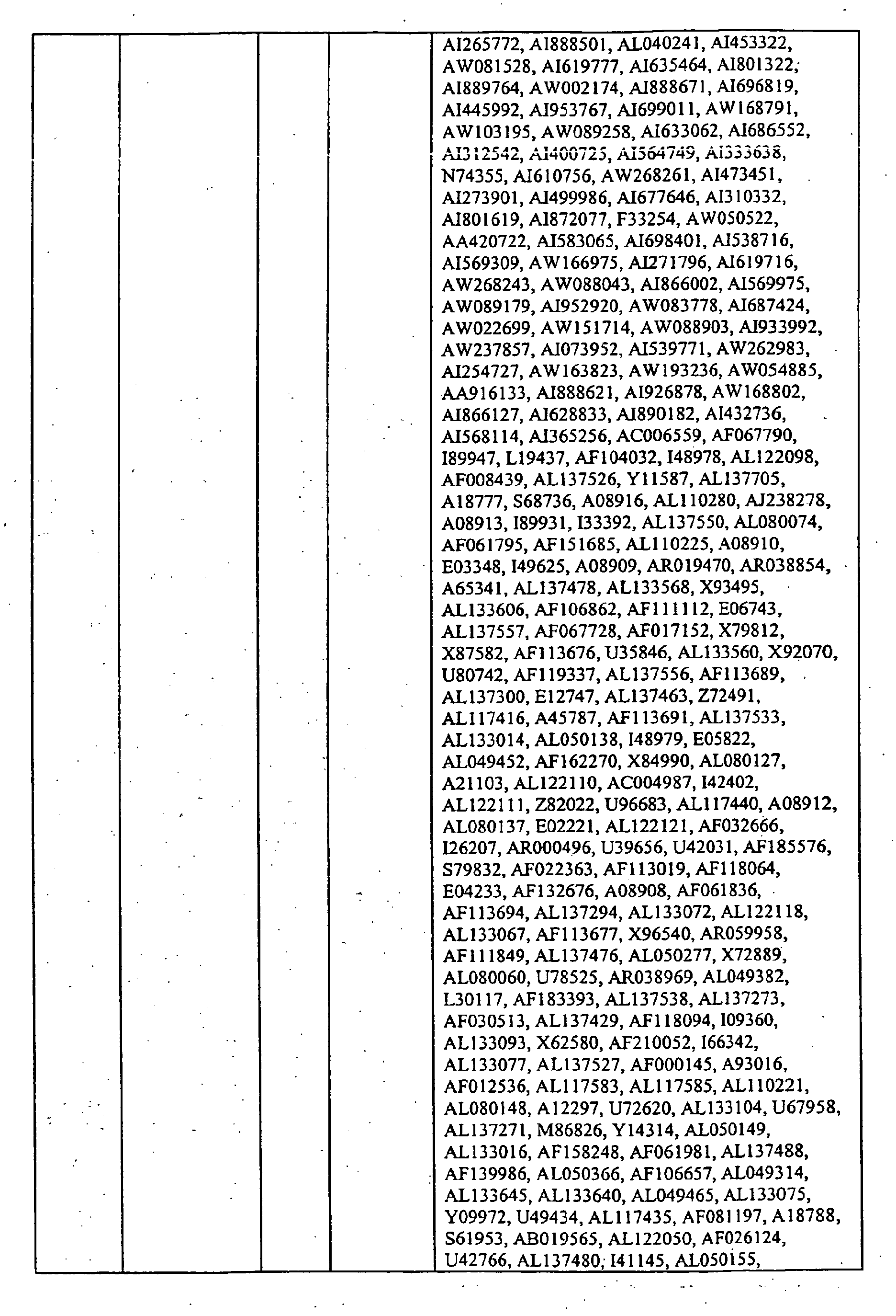

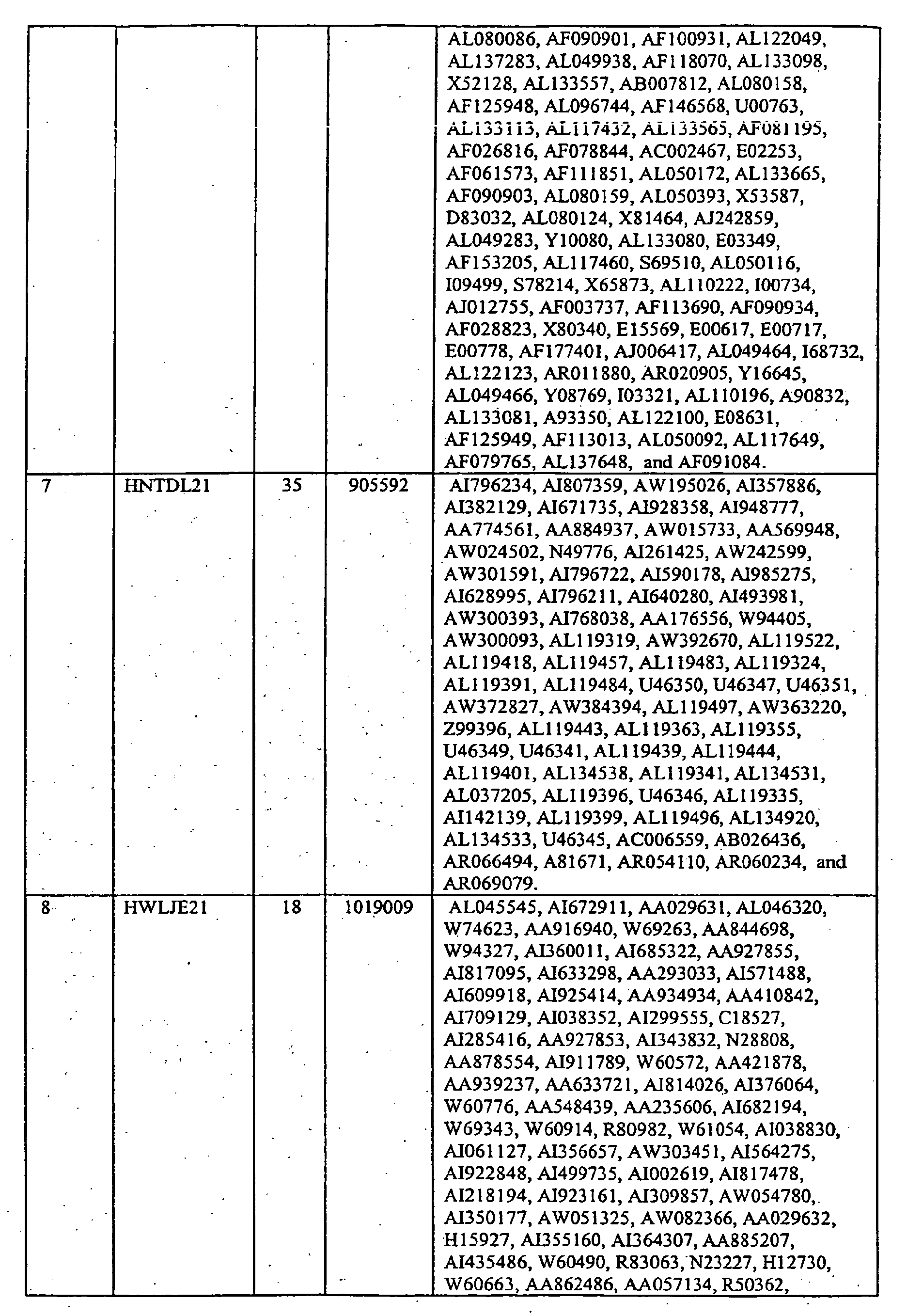

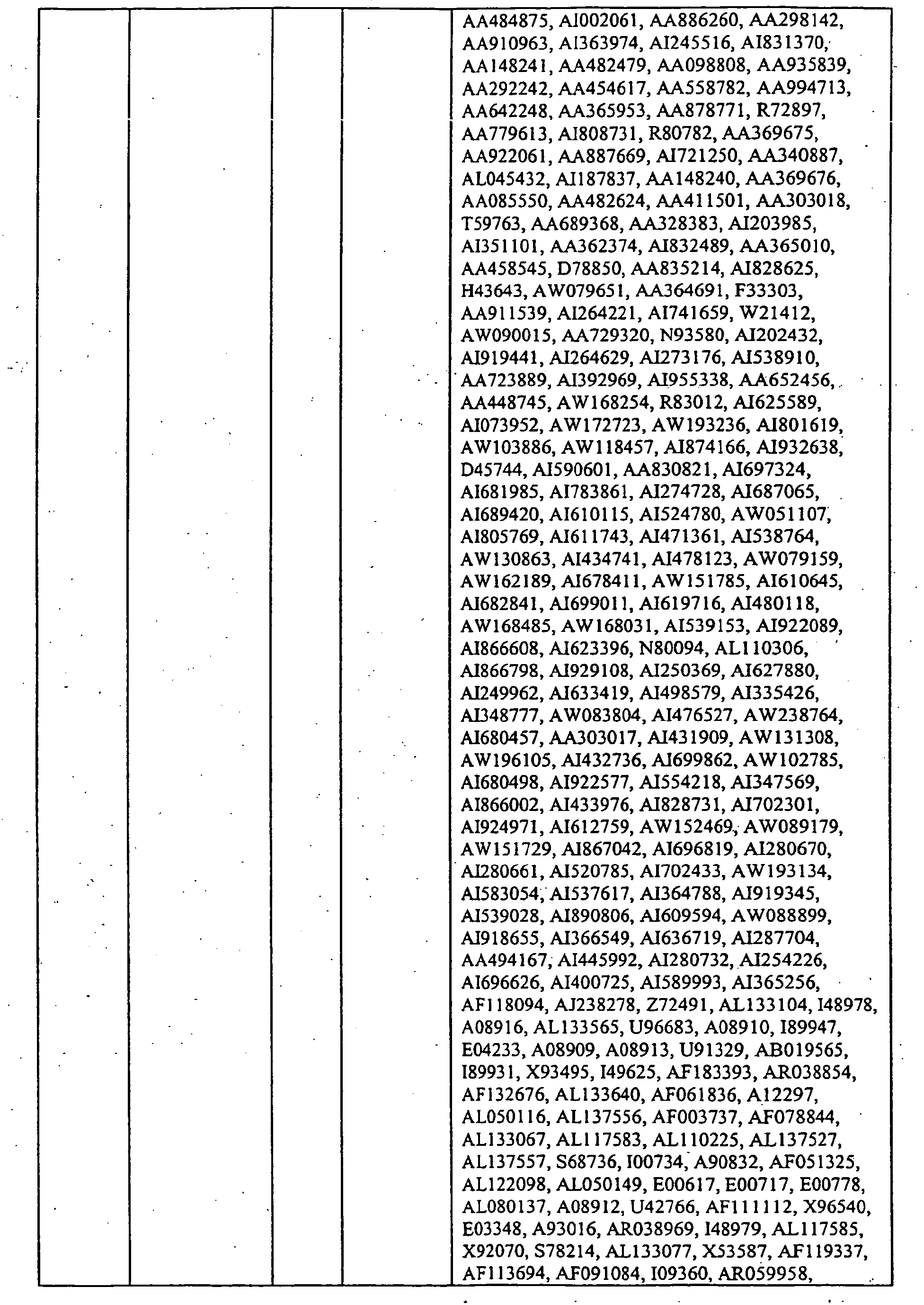

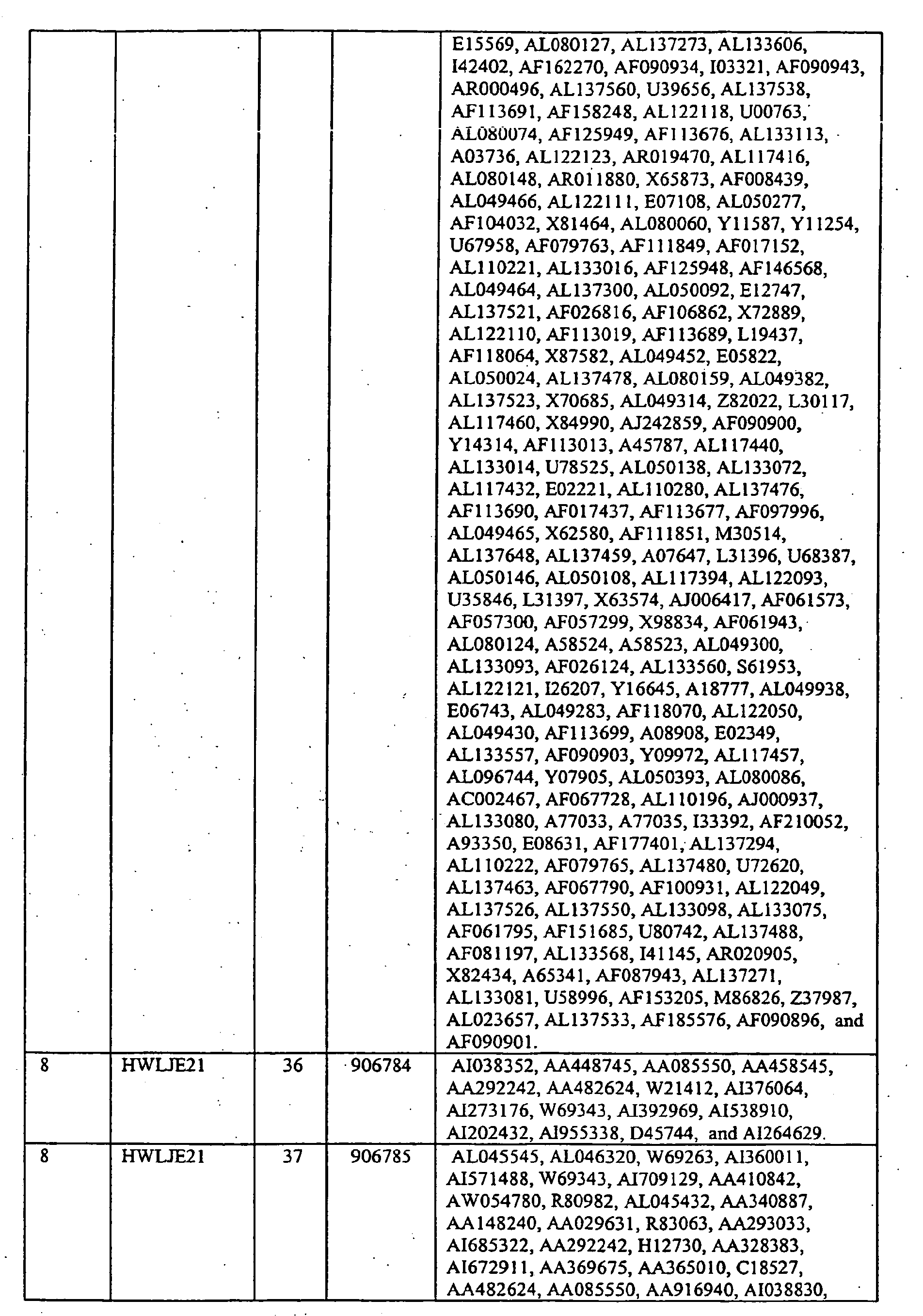

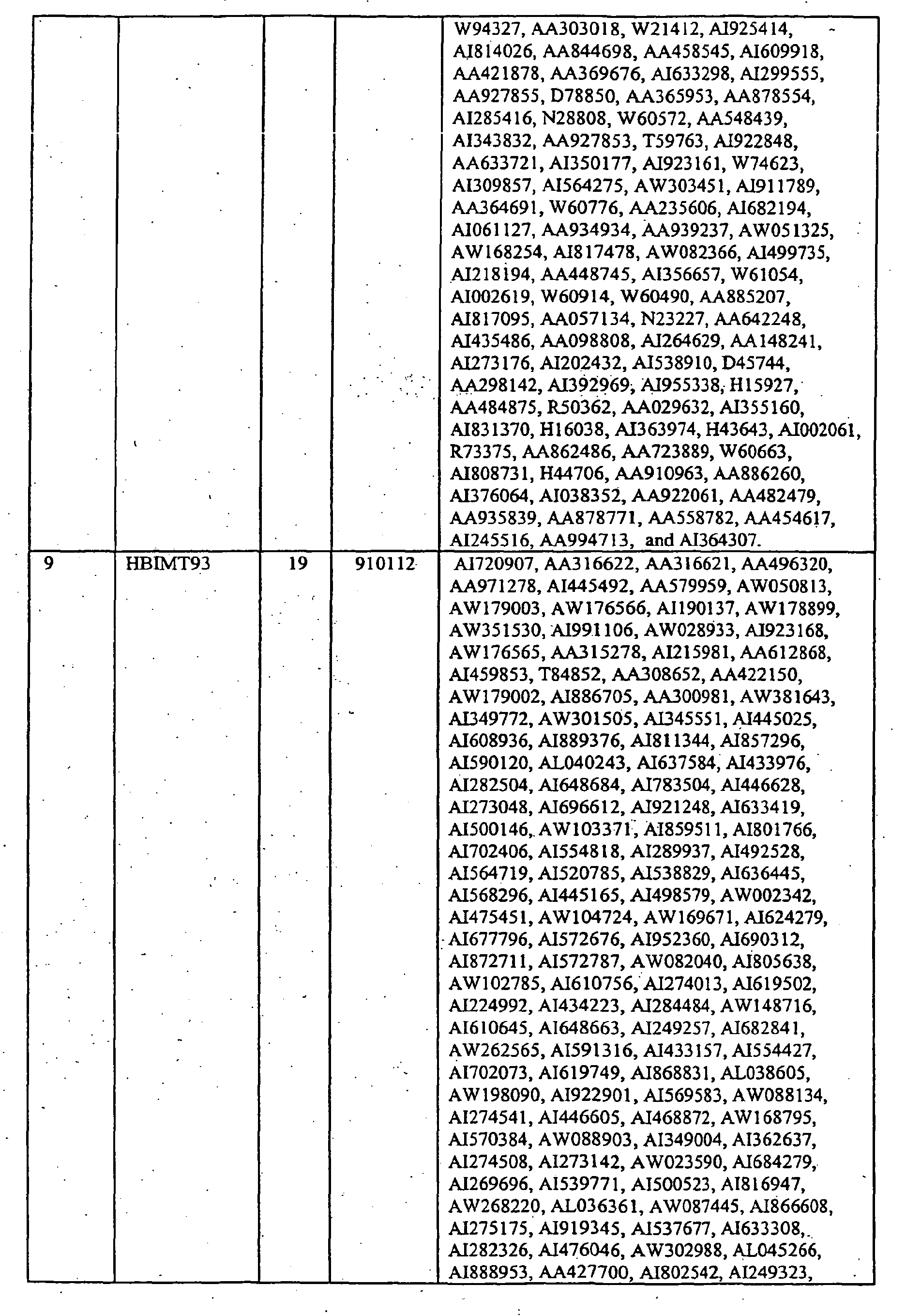

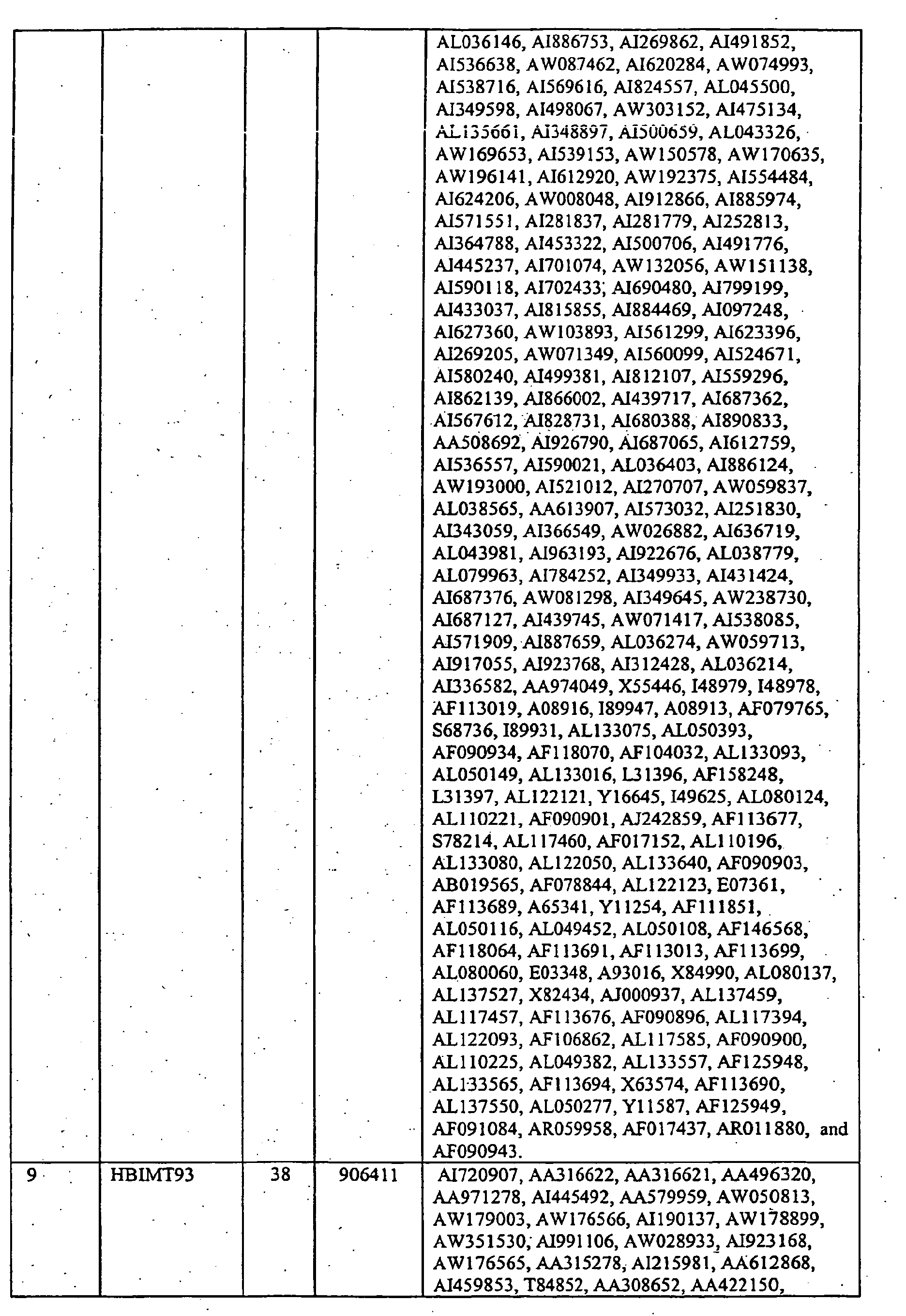

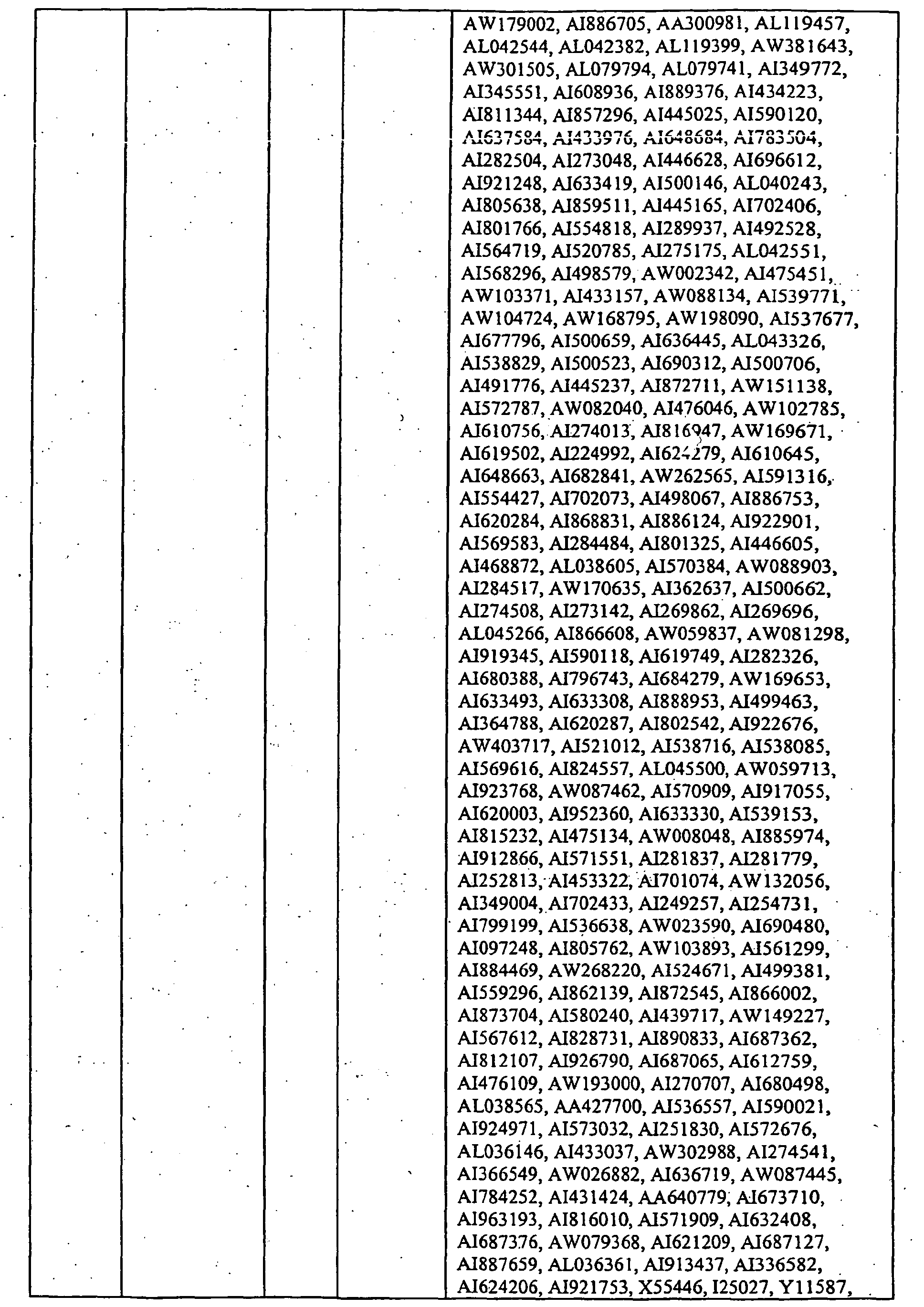

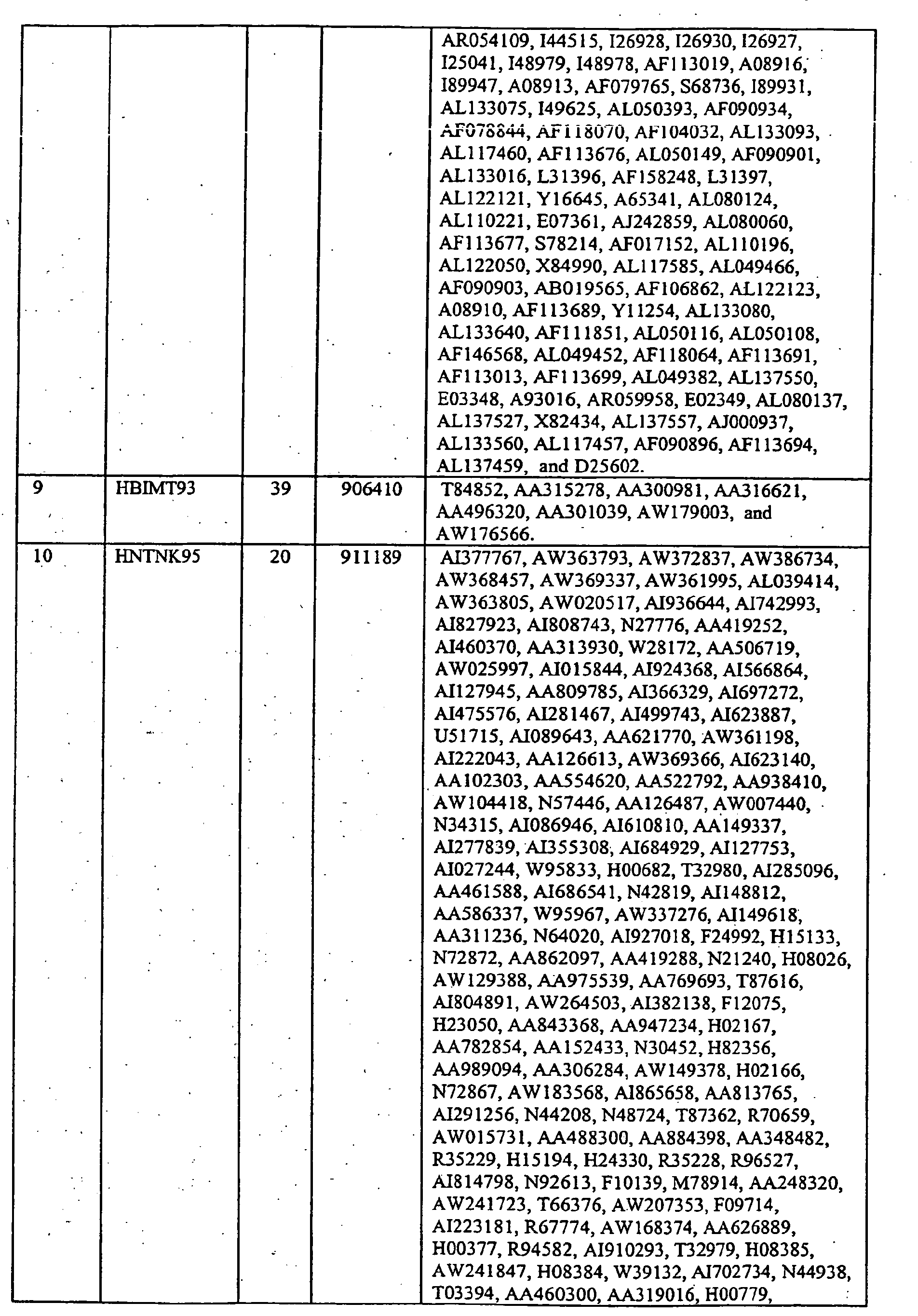

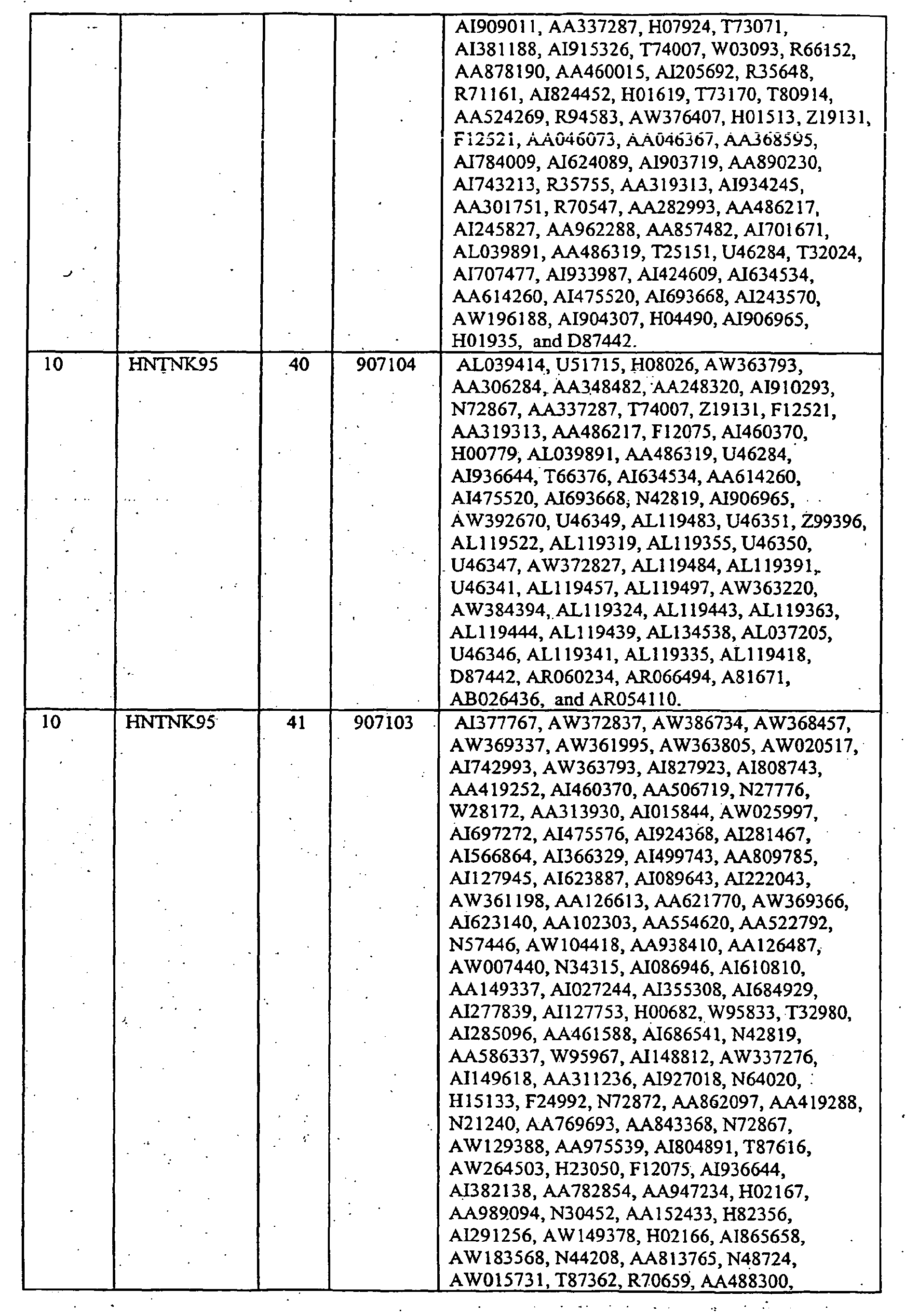

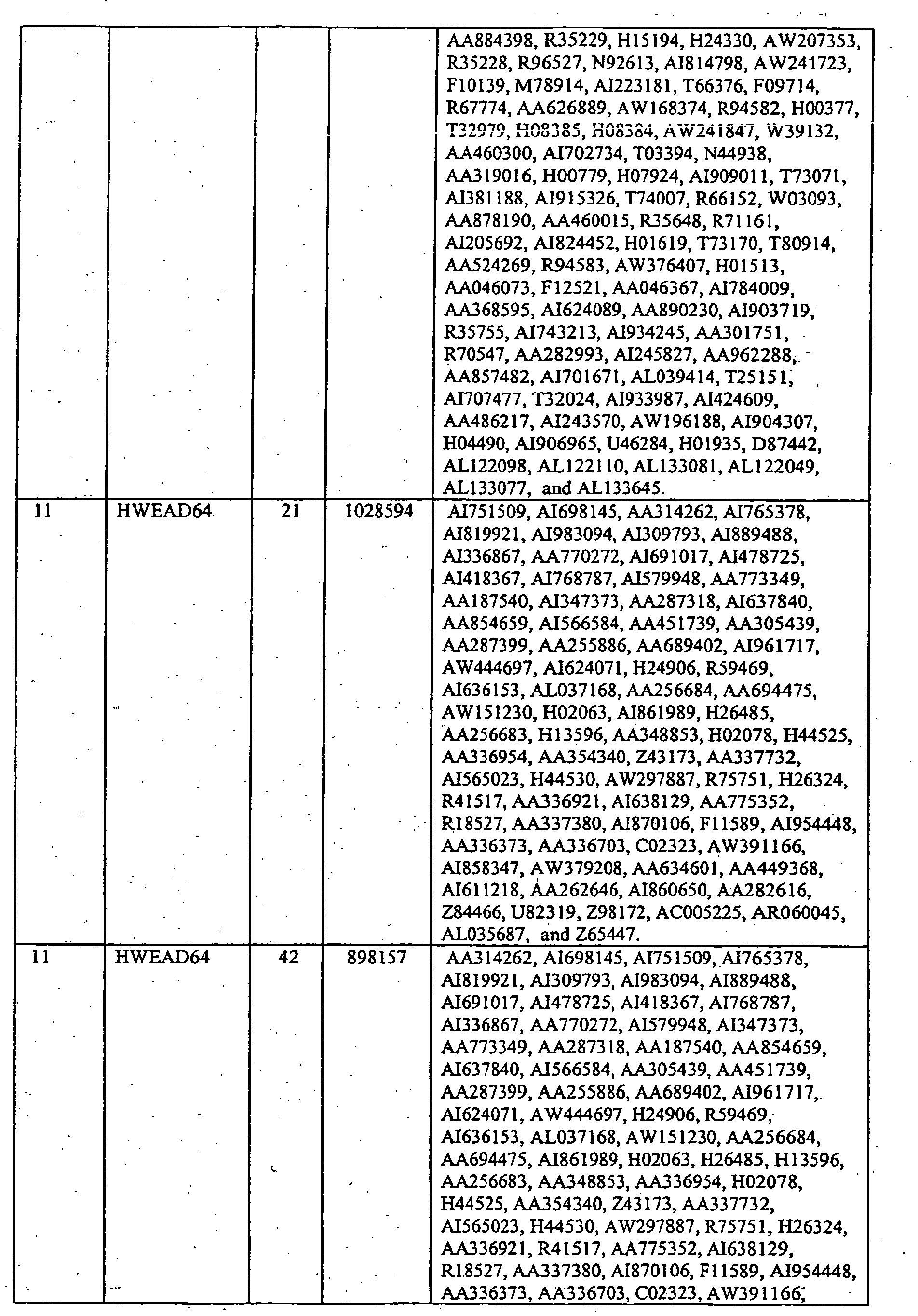

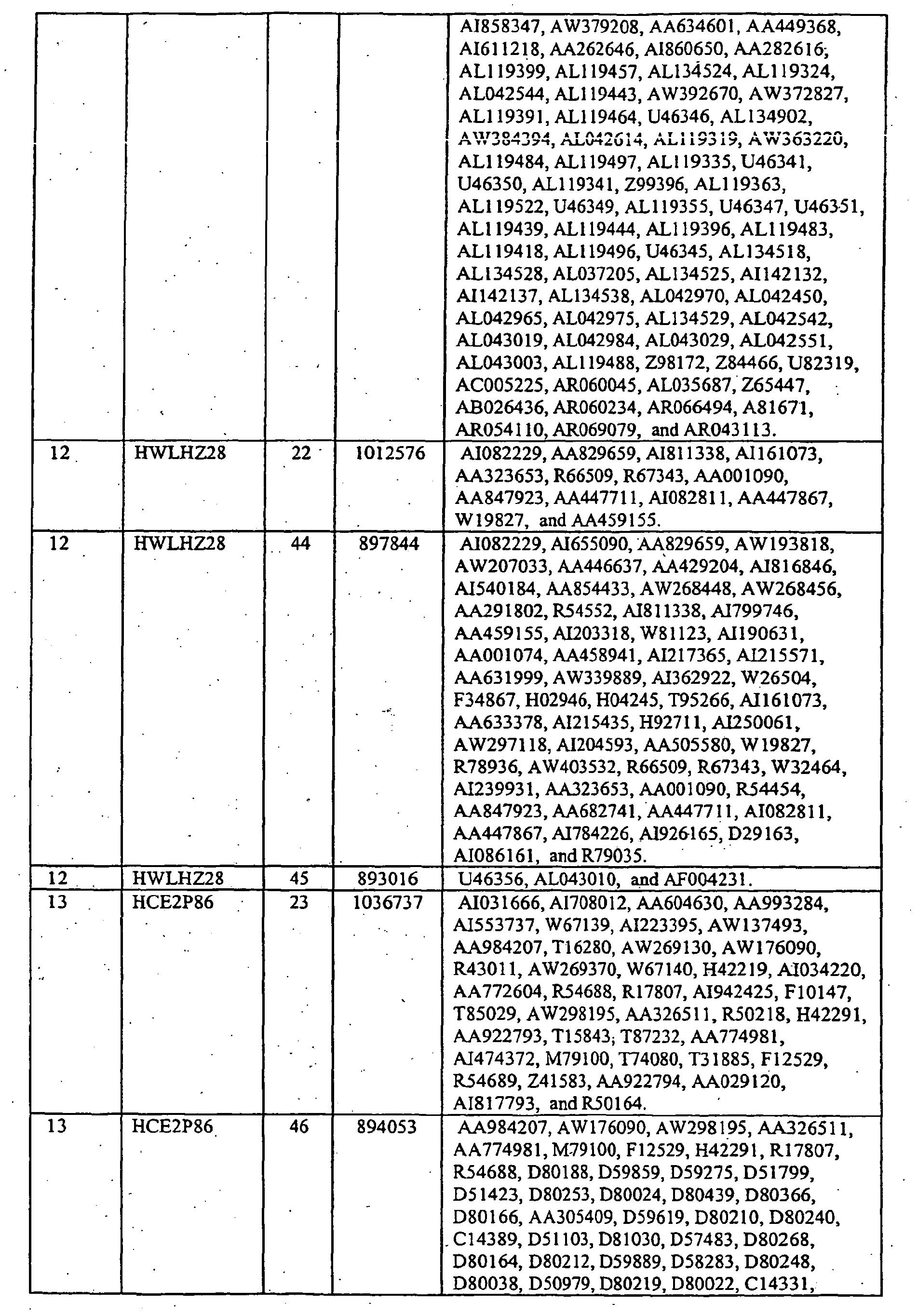

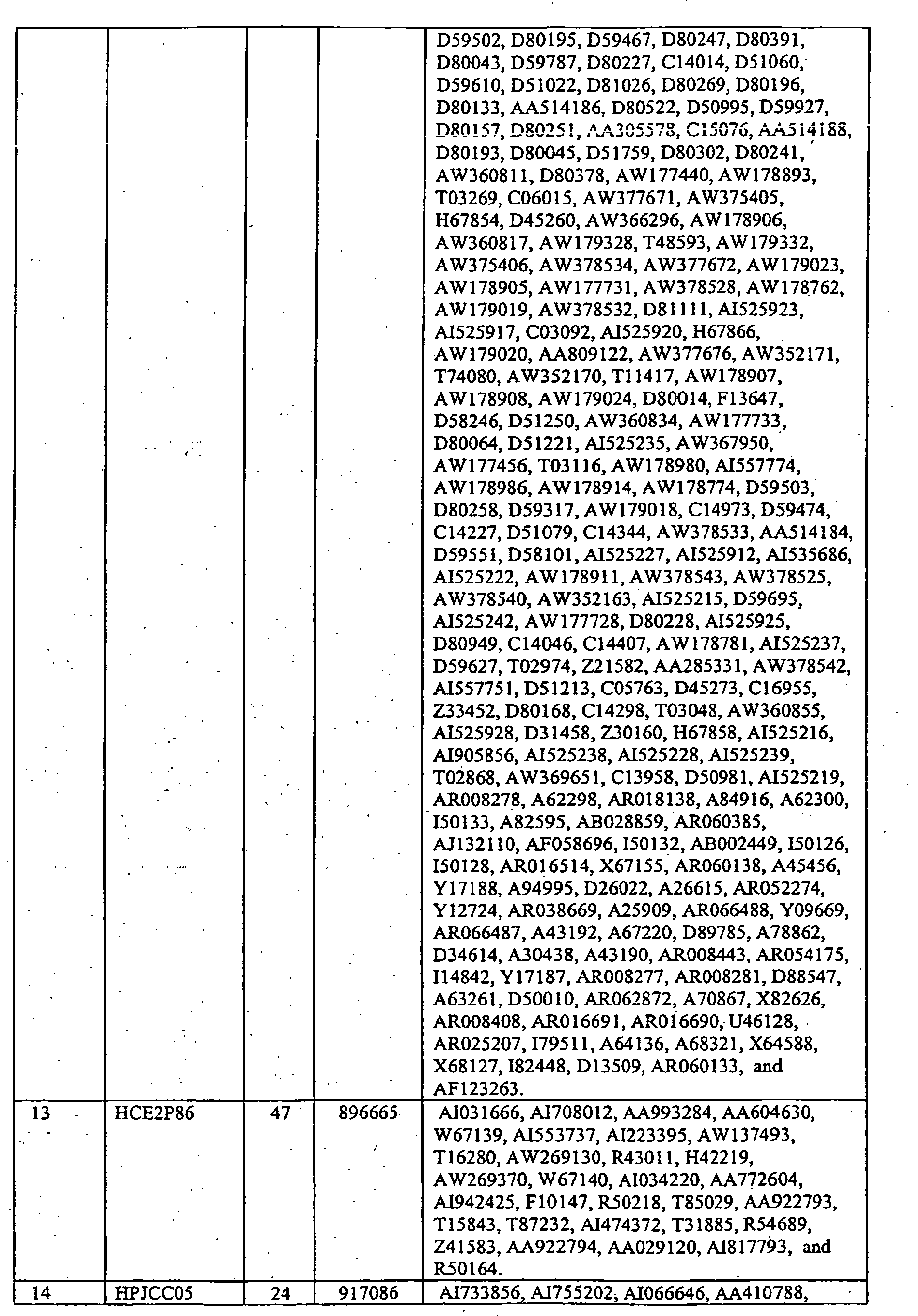

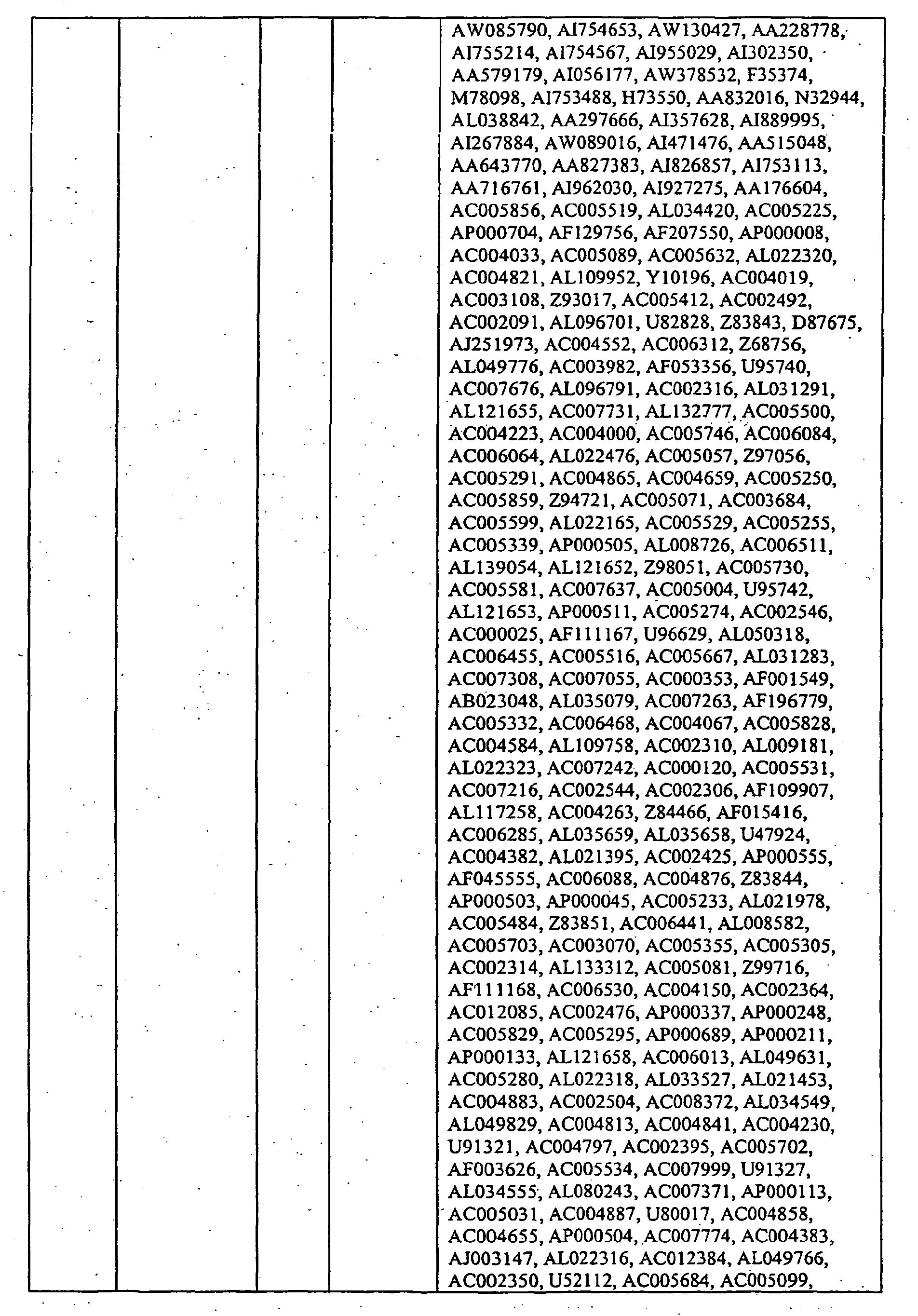

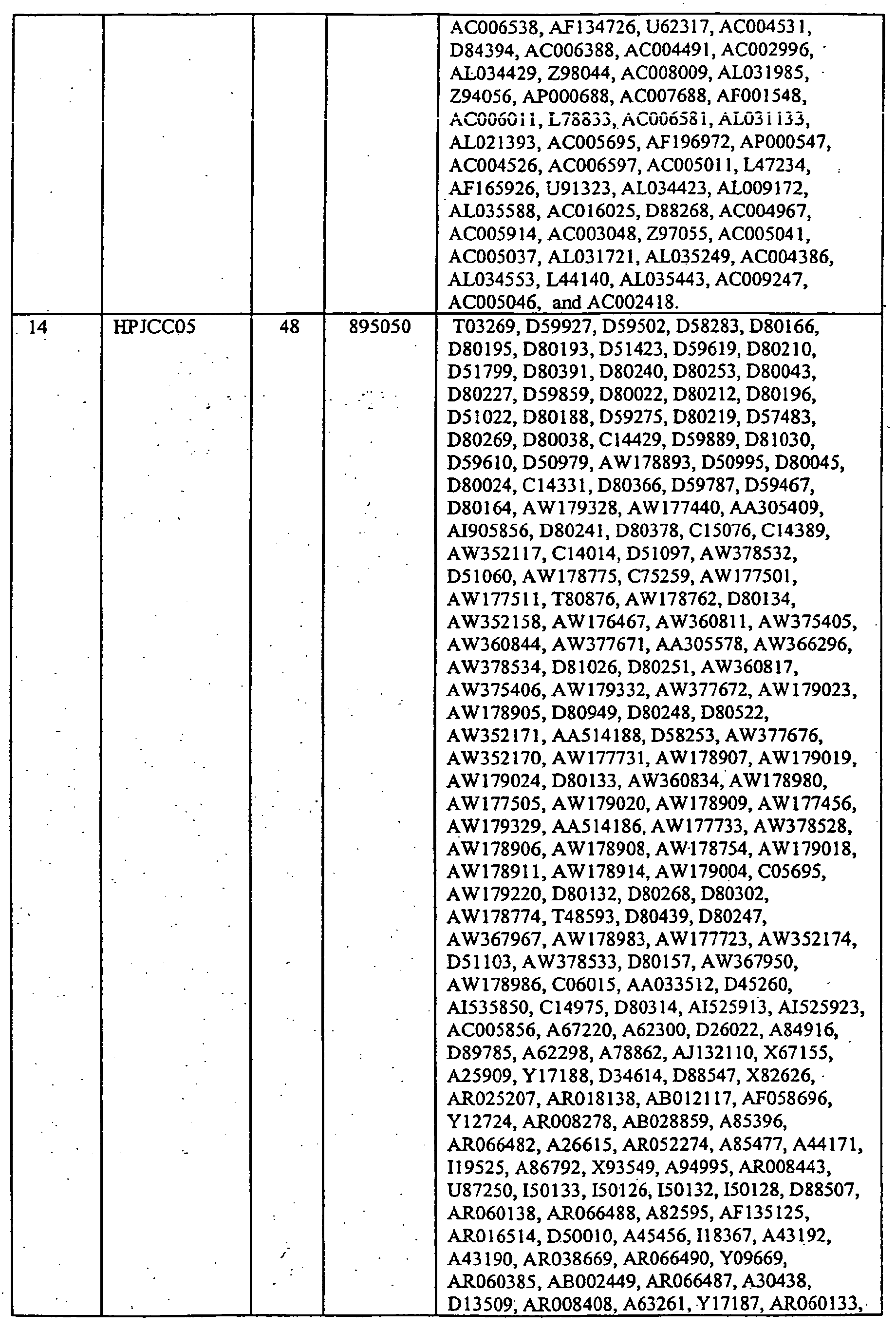

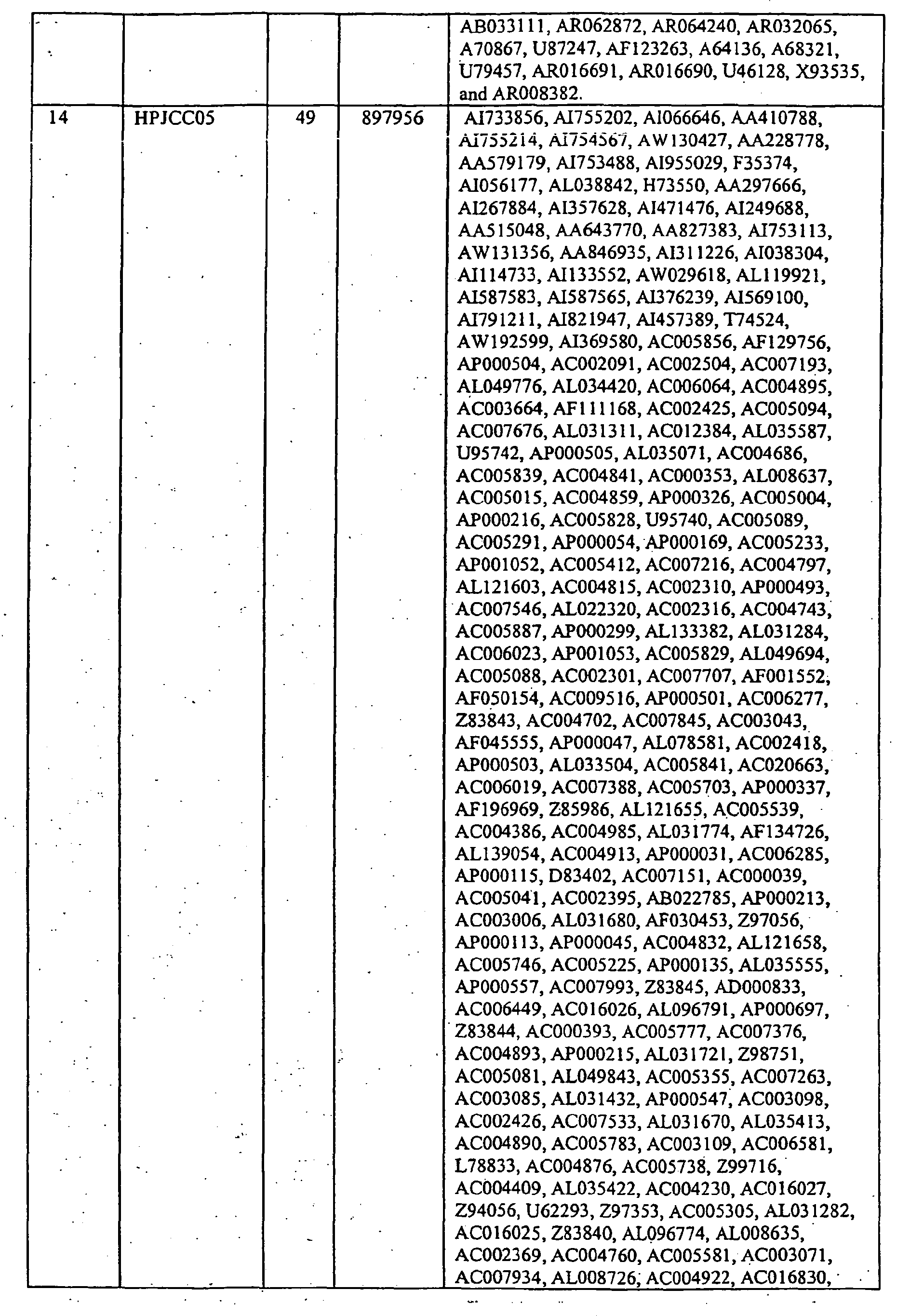

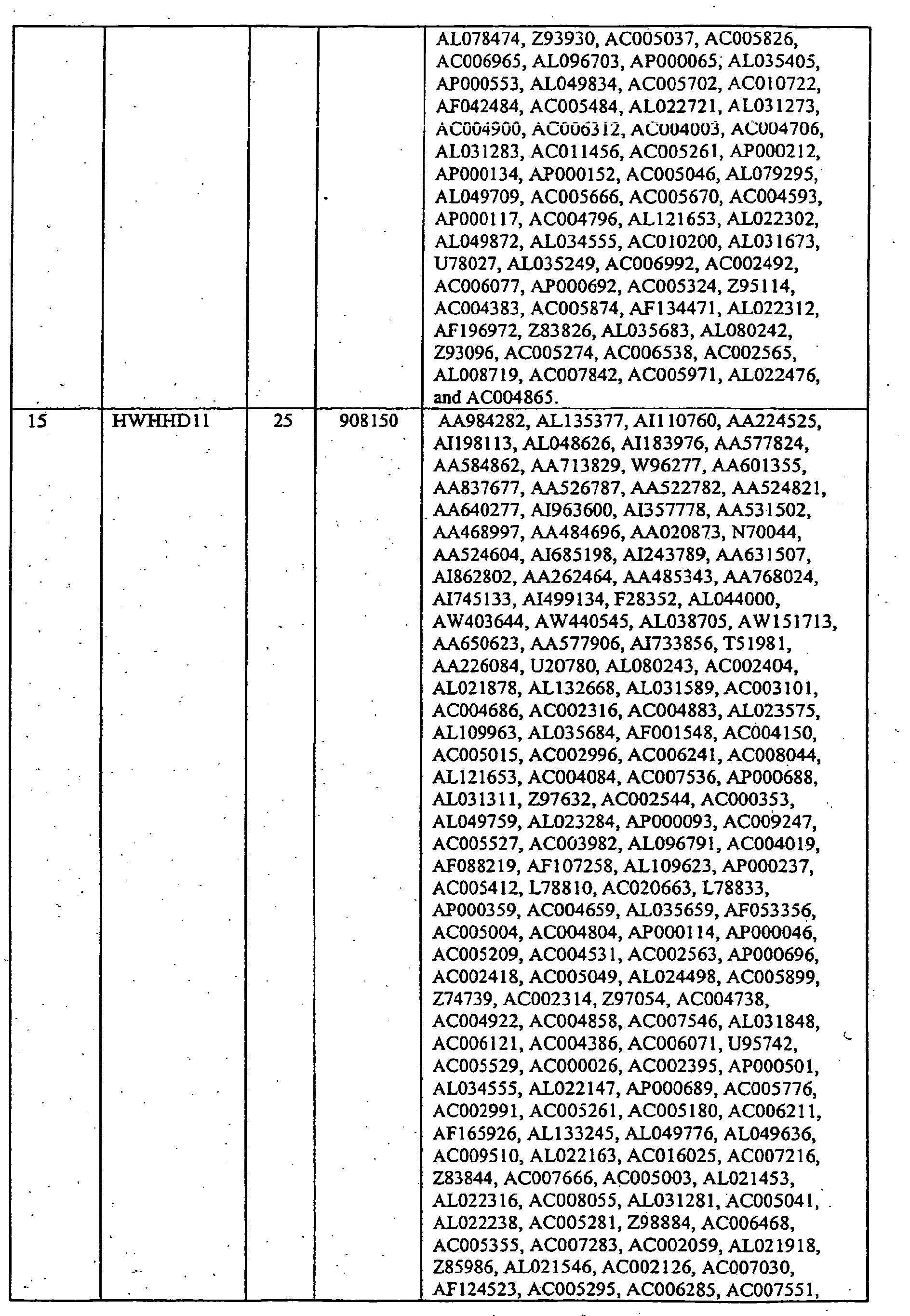

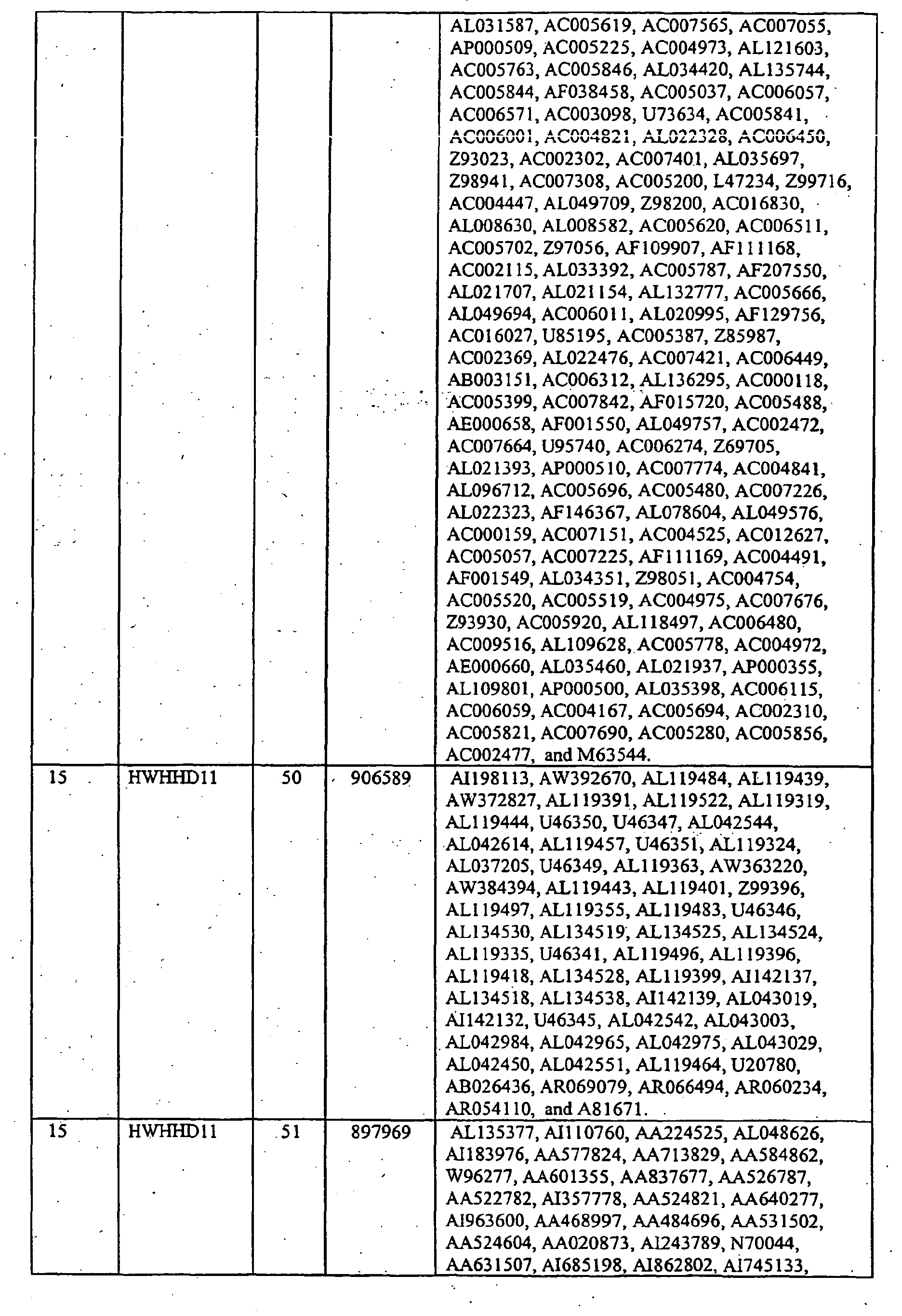

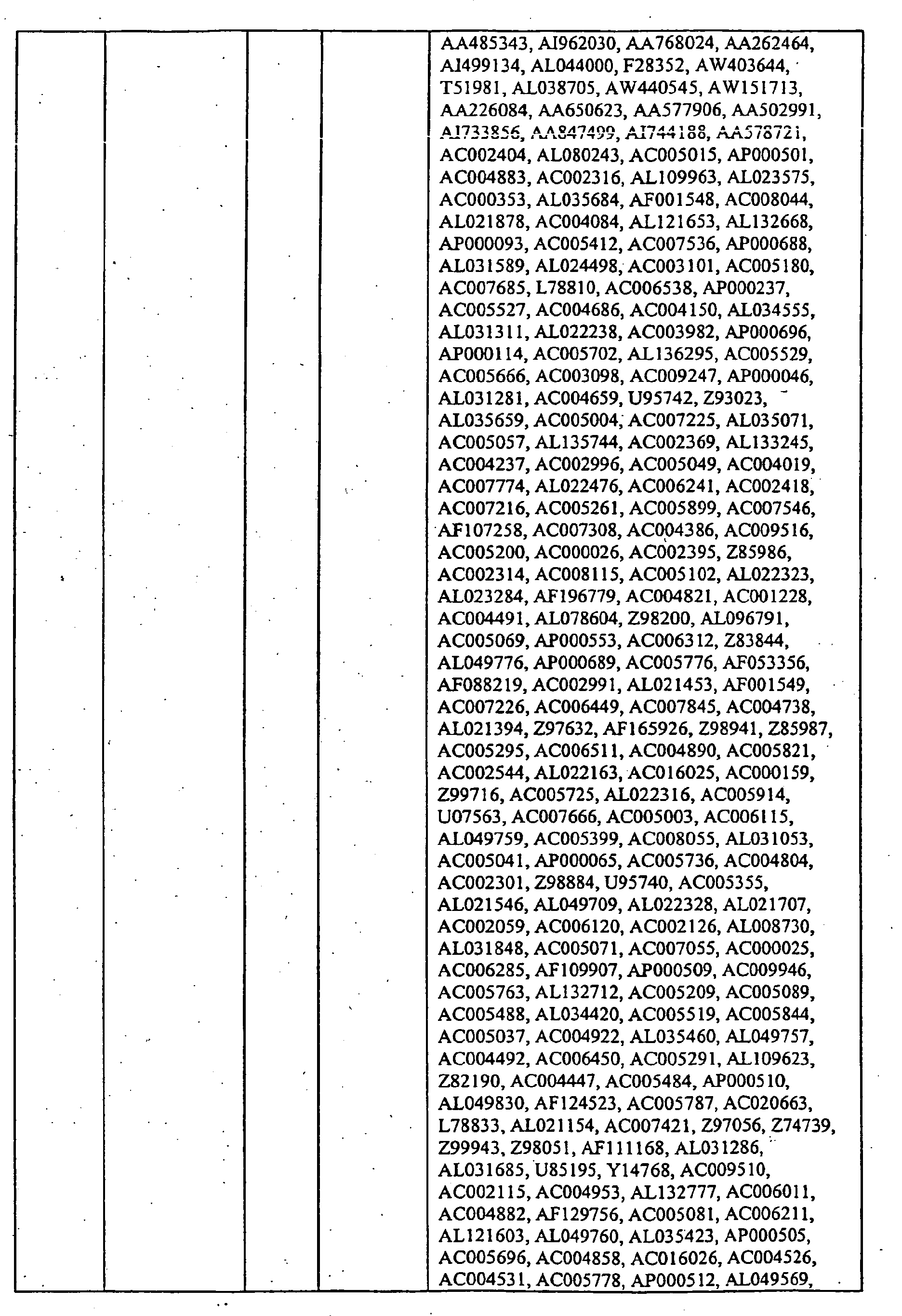

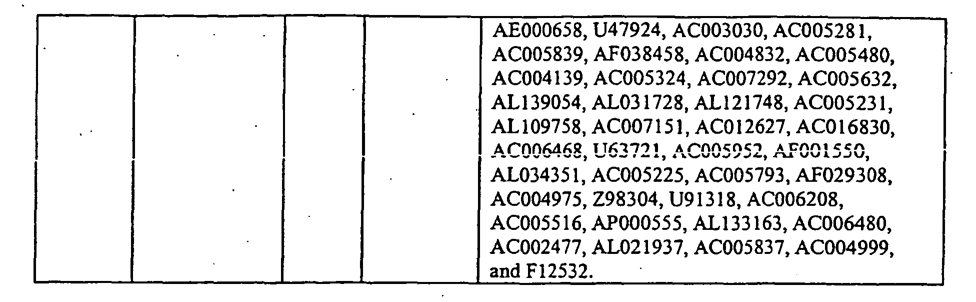

Having generally described the invention, the same will be more readily understood by reference to the following examples, which are provided by way of illustration and are not intended as limiting.

### Examples

### Example 1 : Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited |
|---|---|
| Plasmid | |
| Lambda Zap | pBluescript (pBS) |
| Uni-Zap XR | pBluescript (pBS) |
| Zap Express | pBK |
| lafinid BA | plafmid BA |
| pSport1 | pSport1 |
| pCMVSport 2.0 | pCMVSport 2.0 |
| pCMVSport 3.0 | pCMVSport 3.0 |
| pCR® 2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR® 2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982)) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 ul of reaction mixture with 0.5 ug of the above cDNA template. A convenient reaction mixture is 1.5-5 mM MgCl₂, 0.01 % (w/v) gelatin, 20 uM each of dATP, dCTP, dGTP, dTTP, 25 pmol of each primer and 0.25 Unit of Taq polymerase. Thirty five cycles of PCR (denaturation at 94 degree C for 1 min; annealing at 55 degree C for 1 min; elongation at 72 degree C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5 and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime^{TM} DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb^{TM} hybridization solution (Clontech) according to manufacturer's protocol number PT1190-1. Following hybridization and washing, the blots are mounted and exposed to film at -70 degree C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds,95 degree C; 1 minute, 56 degree C; 1 minute, 70 degree C. This cycle is repeated 32 times followed by one 5 minute cycle at 70 degree C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify tile cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and Xbal and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4 degree C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4 degree C or frozen at -80 degree C.

In addition to the above expression vector, the present invention further includes an expression vector comprising phage operator and promoter elements operatively linked to a polynucleotide of the present invention, called pHE4a. (ATCC Accession Number 209645, deposited on February 25,1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (laclq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an lnclusion Body

The following alternative method can be used to purify a polypeptide expressed in *E coli* when it is present in the form of inclusion bodies. Unless otherwise specified, all of the following steps are conducted at 4-10 degree C.

Upon completion of the production phase of the *E. coli* fermentation, the cell culture is cooled to 4-10 degree C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4 degree C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4 degree C without mixing for 12 hours prior to further purification steps.

To clarify the refolded polypeptide solution, a previously prepared tangential filtration unit equipped with 0.16 um membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (pores CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 ug of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB101 1 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five ug of a plasmid containing the polynucleotide is co-transfected with 1.0 ug of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One ug of BaculoGold™ virus DNA and 5 ug of the plasmid are mixed in a sterile well of a microtiter plate containing 50 ul of serum-free Grace's medium (Life Technologies Inc., Gaithersburg, MD). Afterwards, 10 ul Lipofectin plus 90 ul Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27 degrees C. The transfection solution is then removed from the plate and 1 ml of Grace's insect medium supplemented with 10% fetal calf serum is added. Cultivation is then continued at 27 degrees C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 ul of Grace's medium and the suspension containing the recombinant baculovirus is used to infect Sf9 cells seeded in 35 mm dishes. Four days later the supernatants of these culture dishes are harvested and then they are stored at 4 degree C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 uCi of ³⁵S-methionine and 5 uCi ³⁵ S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening . sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC 1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker. is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markets, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB 101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 a pC4 is cotransfected with 0.5 ug of the plasmid pSVneo using lipofectin (Felgner et al., *supra*). The plasmid pSV2-neo contains a dominant selectable marker, the neo gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 uM, 2 uM, 5 uM, 10 mM, 20 mM). The same procedure is repeated until clones are obtained which grow at a concentration of 100 - 200 uM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

### Human IgG Fc region:

GGGATCCGGAGCCCAAATCTTCTGACAAAACTCACACATGCCCACCGTGC
CCAGCACCTGAATTCGAGGGTGCACCGTCAGTCTTCCTCTTCCCCCCAAAA
CCCAAGGACACCCTCATGATCTCCCGGACTCCTGAGGTCACATGCGTGGT
GGTGGACGTAAGCCACGAAGACCCTGAGGTCAAGTTCAACTGGTACGTGG
ACGGCGTGGAGGTGCATAATGCCAAGACAAAGCCGCGGGAGGAGCAGTA
CAACAGCACGTACCGTGTGGTCAGCGTCCTCACCGTCCTGCACCAGGACT
GGCTGAATGGCAAGGAGTACAAGTGCAAGGTCTCCAACAAAGCCCTCCCA
ACCCCCATCGAGAAAACCATCTCCAAAGCCAAAGGGCAGCCCCGAGAAC
CACAGGTGTACACCCTGCCCCCATCCCGGGATGAGCTGACCAAGAACCAG
GTCAGCCTGACCTGCCTGGTCAAAGGCTTCTATCCAAGCGACATCGCCGT
GGAGTGGGAGAGCAATGGGCAGCCGGAGAACAACTACAAGACCACGCCT
CCCGTGCTGGACTCCGACGGCTCCTTCTTCCTCTACAGCAAGCTCACCGTG

GACAAGAGCAGGTGGCAGCAGGGGAACGTCTTCTCATGCTCCGTGATGCA
TGAGGCTCTGCACAACCACTACACGCAGAAGAGCCTCTCCCTGTCTCCGG
GTAAATGAGTGCGACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) As one example of such methods, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56 degrees C), and supplemented with about 10 g/l of nonessential amino acids, about 1,000 U/ml of penicillin, and about 100 ug/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et all, Nature 314:268 (1985).)

### Example 11: Production Of Secreted Protein For High-Throughput Screening Assays

The following protocol produces a supernatant containing a polypeptide to be tested. This supernatant can then be used in the Screening Assays described herein.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates offPBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37 degrees C for 6 hours.

While cells are incubating, prepare appropriate media, either 1%BSA in DMEM with 1x penstrep, or CHO-5 media (116.6 mg/L of CaCl2 (anhyd); 0:00130 mg/L CuSO₄-SH₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂O; 6.65 mg/ml of L-Aspartic Acid; 29.56 mg/ml of L-Cystine-2HCL-H₂O; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂O; 99.65 mg/ml ofL-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L ofNa Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37 degrees C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proximal region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| EFN-a/B | + | + | - | - | 1,2,3 | ISRE |
| IFN-g | | + | + | - | 1 | GAS (IRF1>Lys6>IFP) |
| I1-10 | + | ? | ? | - | 1,3 | |

| gp130 family | | | | | | |
|---|---|---|---|---|---|---|
| IL-6 (Pleiotrophic) | + | + | + | ? | 1,3 | GAS (IRF1>Lys6>IFP) |
| I1-11 (Pleiotrophic) | ? | + | ? | ? | 1,3 | |
| OnM(Pleiotrophic) | ? | + | + | ? | 1,3 | |
| LIF(Pleiotrophic) | ? | + | + | ? | 1,3 | |
| CNTF(Pleiotrophic) | -/+ | + | + | ? | 1,3 | |
| G-CSF(Pleiotrophic) | ? | + | ? | ? | 1,3 | |
| IL-12(Pleiotrophic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) | - | + | - | + | 6 | GAS (IRF1 = IFP >>Ly6)(IgH) |
| IL-7(lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an Xhol site. The sequence of the 5' primer is:

5':GCGCCTCGAGATTTCCCCGAAATCTAGATTTCCCCGAAATGATTTCCCC
GAAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAA
TGATTTCCCCGAAATATCTGCCATCTCAATTAGTCAGCAACCATAGTCCCG
CCCCTAACTCCGCCCATCCCGCCCCTAACTCCGCCCAGTTCCGCCCATTCT
CCGCCCCATGGCTGACTAATTTTTTTTATTTATGCAGAGGCCGAGGCCGCC
TCGGCCTCTGAGCTATTCCAGAAGTAGTGAGGAGGCTTTTTTGGAGGCCT
AGGCTTTTGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, I1-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, and determining whether supernate containing a polypeptide of the invention proliferates and/or differentiates T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug bfplasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of I x 10⁷ cells in OPTI-MEM to T25 flask and incubate at 37 degrees C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing polypeptides of the invention and/or induced polypeptides of the invention as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20 degrees C until SEAP assays are performed according to Example 17. The plates containing the remaining treated cells are placed at 4 degrees C and serve as a source of material for repeating the assay on a specific well if desired.

As a positive control, 100 Unit/ml interferon gamma can be used which is known to activate Jurkat T cells. Over 30 fold induction is typically observed in the positive control wells.

The above protocol may be used in the generation of both transient, as well as, stable transfected cells, which would be apparent to those of skill in the art.

### Example 14: High-Throughput Screening Assay Identifying Myeloid Activity

The following protocol is used to assess myeloid activity by determining whether polypeptides of the invention proliferates and/or differentiates myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37 degrees C for 45 min.

Wash the cells with RPMI 1640 medium containing 10% FBS and then resuspend in 10 ml complete medium and incubate at 37 degrees C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37 degrees C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC 12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells.with PBS. Scrape off the cells from the plate, suspend the cells well in 2 ml low serum medium. Count the cell number and add more low serum medium to reach final cell density as 5x 10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-KB (Nuclear Factor KB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-KB regulates the expression of genes involved in immune cell activation, control of apoptosis (NF- KB appears to shield cells from apoptosis), B and T-cell development, anti-viral and antimicrobial responses, and multiple stress responses.

In non-stimulated conditions, NF- KB is retained in the cytoplasm with I-KB (Inhibitor KB). However, upon stimulation, I- KB is phosphorylated and degraded, causing NF- KB to shuttle to the nucleus, thereby activating transcription of target genes. Target genes activated by NF- KB include IL-2, IL-6, GM-CSF, ICAM-1 and class 1 MHC.

Due to its central role and ability to respond to a range of stimuli, reporter constructs utilizing the NF-KB promoter element are used to screen the supernatants produced in Example 11. Activators or inhibitors of NF-KB would be useful in treating diseases. For example, inhibitors of NF-KB could be used to treat those diseases related to the acute or chronic activation of NF-KB, such as rheumatoid arthritis.

To construct a vector containing the NF-KB promoter element, a PCR based strategy is employed. The upstream primer contains four tandem copies of the NF-KB binding site (GGGGACTTTCCC) (SEQ ID NO: 8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pb-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGACTTTCCGGGACTTTCC
ATCTGCCATCTCAATTAGTCAGCAACCATAGTCCCGCCCCTAACTCCGCCC
ATCCCGCCCCTAACTCCGCCCAGTTCCGCCCATTCTCCGCCCCATGGCTGA
CTAATTTTTTTTATTTATGCAGAGGCCGAGGCCGCCTCGGCCTCTGAGCTA
TTCCAGAAGTAGTGAGGAGGCTTTTTTGGAGGCCTAGGCTTTTGCAAAAA
GCTT:3' (SEQ ID NO:10)

Next, replace the SV40 minimal promoter element present in the pSEAP2-promoter plasmid (Clontech) with this NF-KB/SV40 fragment using XhoI and HindIII. However, this vector does not contain a neomycin resistance gene, and therefore, is not preferred for mammalian expression systems.

In order to generate stable mammalian cell lines, the NF-KB/SV40/SEAP cassette is removed from the above NF-KB/SEAP vector using restriction enzymes SalI and NotI, and inserted into a vector containing neomycin resistance. Particularly, the NF-KB/SV40/SEAP cassette was inserted into pGFP-1 (Clontech), replacing the GFP gene, after restricting pGFP-1 with SalI and NotI.

Once NF-KB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 ul of 2.5x dilution buffer into Optiplates containing 35 ul of a supernatant. Seal the plates with a plastic sealer and incubate at 65 degree C for 30 min. Separate the Optiplates to avoid uneven heating.

Cool the samples to room temperature for 15 minutes. Empty the dispenser and prime with the Assay Buffer. Add 50 ml Assay Buffer and incubate at room temperature 5 min. Empty the dispenser and prime with the Reaction Buffer (see the table below). Add 50 ul Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| '27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 . | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ('FLIPR") to measure changes in fluorescent molecules (Molecular Probes) that bind small molecules. Clearly, any fluorescent molecule detecting a small molecule can be used instead of the calcium fluorescent molecule, fluo-4 (Molecular Probes, Inc.; catalog no. F-14202), used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-4 is made in 10% pluronic acid DMSO. To load the cells with fluo-4 , 50 ul of 12 ug/ml fluo-4 is added to each well. The plate is incubated at 37 degrees C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml . fluo-4 solution in 10% pluronic acid DMSO is added to each ml of cell suspension. The tube is then placed in a 37 degrees C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-4 . The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4 degree C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48, hr. Falcon plate covers #3071 from. Becton Dickinson (Bedford,MA) are used to cover the Loprodyne Silent Screen Plates. Falcon Microtest III cell culture plates cam also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4 degrees C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4 degrees C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1 mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30 degrees C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37 degrees C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37 degrees C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput-Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (lug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4 degrees C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95 degrees C for 30 seconds; 60-120 seconds at 52-58 degrees C; and 60-120 seconds at 70 degrees C, using buffer solutions described in Sidransky et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton et al., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulation

The invention also provides methods of treatment and/or prevention diseases, disorders, and/or conditions (such as, for example, any one or more of the diseases or disorders disclosed herein) by administration to a subject of an effective amount of a Therapeutic. By therapeutic is meant a polynucleotides or polypeptides of the invention (including fragments and variants), agonists or antagonists thereof, and/or antibodies thereto, in combination with a pharmaceutically acceptable carrier type (e.g., a sterile carrier).

The Therapeutic will be formulated and dosed in a fashion consistent with good medical practice, taking into account the clinical condition of the individual patient (especially the side effects of treatment with the Therapeutic alone), the site of delivery, the method of administration, the scheduling of administration, and other factors known to practitioners. The "effective amount" for purposes herein is thus determined by such considerations.

As a general proposition, the total pharmaceutically effective amount of the Therapeutic administered parenterally per dose will be in the range of about lug/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the Therapeutic is typically administered at a dose rate of about 1 ug/kg/hour to about 50 ug/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Therapeutics can be are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any. The term "parenteral" as used herein refers to modes of administration which include intravenous, intramuscular, intraperitoneal, intrasternal, subcutaneous and intraarticular injection and infusion.

Therapeutics of the invention are also suitably administered by sustained-release systems. Suitable examples of sustained-release Therapeutics are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

Therapeutics of the invention are also suitably administered by sustained-release systems. Suitable examples of sustained-release Therapeutics include suitable polymeric materials (such as, for example, semi-permeable polymer matrices in the form of shaped articles, e.g., films, or mirocapsules), suitable hydrophobic materials (for example as an emulsion in an acceptable oil) or ion exchange resins, and sparingly soluble derivatives (such as, for example, a sparingly soluble salt).

Sustained-release matrices include polylactides (U.S. Pat. No. 3,773,919, EP 58,481), copolymers of L-glutamic acid and gamma-ethyl-L-glutamate (Sidman et al., Biopolymers 22:547-556 (1983)), poly (2- hydroxyethyl methacrylate) (Langer et al., J. Biomed. Mater. Res. 15:167-277 (1981), and Langer, Chem. Tech. 12:98-105 (1982)), ethylene vinyl acetate (Langer et al., Id.) or poly-D- (-)-3-hydroxybutyric acid (EP 133,988).

Sustained-release Therapeutics also include liposomally entrapped Therapeutics of the invention (see generally, Langer, Science 249:1527-1533 (1990); Treat et al., in *Liposomes in the* Therapy *of Infectious Disease and Cancer,* Lopez-Berestein and Fidler (eds.), Liss, New York, pp. 317 -327 and 353-365 (1989)). Liposomes containing the Therapeutic are prepared by methods known per se: DE 3,218,121; Epstein et al., Proc. Natl. Acad. Sci. (USA) 82:3688-3692 (1985); Hwang et al., Proc. Natl. Acad. Sci.(USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal Therapeutic.

In yet an additional embodiment, the Therapeutics of the invention are delivered by way of a pump (*see* Langer, *supra;* Sefton, CRC Crit. Ref. Biomed. Eng. 14:201 (1987); Buchwald et al., Surgery 88:507 (1980); Saudek et al., N. Engl. J. Med. 321:574 (1989)).

Other controlled release systems are discussed in the review by Langer (*Science* 249:1527-1533 (1990)).

For parenteral administration, in one embodiment, the Therapeutic is formulated generally by mixing it at the desired degree of purity, in a unit dosage injectable form (solution, suspension, or emulsion), with a pharmaceutically acceptable carrier, i.e., one that is' non-toxic to recipients at the dosages and concentrations employed and is compatible with other ingredients of the formulation. For example, the formulation preferably does not include oxidizing agents and other compounds that are known to be deleterious to the Therapeutic.

Generally, the formulations are prepared by contacting the Therapeutic uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides; disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The Therapeutic is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any pharmaceutical used for therapeutic administration can be sterile. Sterility is readily accomplished by filtration through sterile filtration membranes (e.g., 0.2 micron membranes). Therapeutics generally are placed into a container having a sterile access port, for example, an intravenous solution bag or vial having a stopper pierceable by a hypodermic injection needle.

Therapeutics ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous Therapeutic solution, and the resulting mixture is lyophilized. The infusion solution is prepared by reconstituting the lyophilized Therapeutic using bacteriostatic Water-for-Injection.

The invention also provides a pharmaceutical pack or kit comprising one or more containers filled with one or more of the ingredients of the Therapeutics of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the Therapeutics may be employed in conjunction with other therapeutic compounds.

The Therapeutics of the invention may be' administered alone or in combination with adjuvants. Adjuvants that may be administered with the Therapeutics. of the invention include, but are not limited to, alum, alum plus deoxycholate (ImmunoAg), MTP-PE (Biocine Corp.), QS21 (Genentech, Inc.), BCG, and MPL. In a specific embodiment, Therapeutics of the invention are administered in combination with alum. In another specific embodiment, Therapeutics of the invention are administered in combination with QS-21: Further adjuvants that may be administered with the Therapeutics of the invention include, but are not limited to, Monophosphoryl lipid immunomodulator, Adju Vax 100a, QS-21, QS-18, CRL1005, Aluminum salts, MF-59, and Virosomal adjuvant technology. Vaccines that may be administered with the Therapeutics of the invention include, but are not limited to, vaccines directed toward protection against MMR (measles, mumps, rubella), polio, varicella, tetanus/diptheria, hepatitis A, hepatitis B, haemophilus influenzae B, whooping cough, pneumonia, influenza, Lyme's Disease, rotavirus, cholera, yellow fever, Japanese encephalitis, poliomyelitis, rabies, typhoid fever, and' pertussis. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

The Therapeutics of the invention may be administered alone or in combination with other therapeutic agents. Therapeutic agents that may be administered in combination with the Therapeutics of the invention, include but not limited to, other members of the TNF family, chemotherapeutic agents, antibiotics, steroidal and non-steroidal anti-inflammatories, conventional immunotherapeutic agents, cytokines and/or growth factors. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.
In one embodiment, the Therapeutics of the invention are administered in combination with members of the TNF family. TNF, TNF-related or TNF-like molecules that may be administered with the Therapeutics of the invention include, but are not limited to, soluble forms of TNF-alpha, lymphotoxin-alpha (LT-alpha, also known as TNF-beta), LT-beta (found in complex heterotrimer LT-alpha2-beta), OPGL, FasL, CD27L, CD30L, CD40L, 4-1BBL, DcR3, OX40L, TNF-gamma (International Publication No. WO 96/14328), AIM-I (International Publication No. WO 97/33899), endokine-alpha (International Publication No. WO 98/07880), TR6 (International Publication No. WO 98/30694), OPG, and neutrokine-alpha (International Publication No. WO 98/18921, OX40, and nerve growth factor (NGF), and soluble forms of Fas, CD30, CD27, CD40 and 4-IBB, TR2 (International Publication No. WO 96/34095), DR3 (International Publication No. WO 97/33904), DR4 (International Publication No. WO 98/32856), TR5 (International Publication-No. WO 98/30693), TR6 (International Publication No. WO 98/30694), TR7 (International Publication No. WO 98/41629), TRANK, TR9 (International Publication No. WO 98/56892),TR10 (International Publication No. WO 98/54202), 312C2 (International Publication No. WO 98/06842), and TR12, and soluble forms CD154, CD70, and CD153.

In certain embodiments, Therapeutics of the invention are administered in combination with antiretroviral agents, nucleoside reverse transcriptase inhibitors, non-nucleoside reverse transcriptase inhibitors, and/or protease inhibitors. Nucleoside reverse transcriptase inhibitors that may be administered in combination with the Therapeutics of the invention, include, but are not limited to, RETROVIR™ (zidovudine/AZT), VIDEX™ (didanosine/ddI), HIVID™ (zalcitabine/ddC), ZERIT™ (stavudine/d4T), EPIVIR^{TM} (lamivudine/3TC), and COMBIVIR^{TM} (zidovudine/lamivudine). Non-nucleoside reverse transcriptase inhibitors that may be administered in combination with the Therapeutics of the invention, include, but are not limited to, VIRAMUNE™ (nevirapine), RESCRIPTOR^{TM} (delavirdine), and SUSTIVA^{TM} (efavirenz). Protease inhibitors that may be administered in combination with the Therapeutics of the invention, include, but are not limited to, CRIXIVAN™ (indinavir), NORVIR™ (ritonavir), INVIRASE™ (saquinavir), and VIRACEPT™ (nelfinavir). In a specific embodiment, antiretroviral agents, nucleoside reverse transcriptase inhibitors, non-nucleoside reverse trariscriptase inhibitors, and/or protease inhibitors may be used in any combination with Therapeutics of the invention to treat AIDS and/or to prevent or treat HIV infection.

In other embodiments, Therapeutics of the invention may be administered in combination with anti-opportunistic infection agents. Anti-opportunistic agents that may be administered in combination with the Therapeutics of the invention, include, but are not limited to, TRIMETHOPRIM-SULFAMETHOXAZOLE™, DAPSONE™, PENTAMIDINE™, ATOVAQUONE™, ISONIAZID™, RIFAMPIN™, PYRAZINAMIDE™, ETHAMBUTOL™, RIFABUTIN™, CLARITHROMYCIN™, AZITHROMYCIN™, GANCICLOVIR™, FOSCARNET™, CIDOFOVIR™, FLUCONAZOLE™, ITRACONAZOLE™, KETOCONAZOLE™, ACYCLOVIR™, FAMCICOLVIR™, PYRIMETHAMINE™, LEUCOVORIN™, NEUPOGEN™ (filgrastim/G-CSF), and LEUKINE™ (sargramostim/GM-CSF). In a specific embodiment, Therapeutics of the invention are used in any combination with TRIMETHOPRIM-SULFAMETHOXAZOLE™, DAPSONE™, PENTAMIDINE™, and/or ATOVAQUONE™ to prophylactically treat or prevent an opportunistic *Pneumocystis carinii* pneumonia. infection. In another specific embodiment, Therapeutics of the invention are used in any combination with ISONIAZID^{TM}, RIFAMPIN^{TM}, PYRAZINAMIDE^{TM}, and/or ETHAMBUTOL^{TM} to prophylactically treat or prevent an opportunistic *Mycobacterium avium.* complex infection. In another specific embodiment, Therapeutics of the invention are used in any combination with RIFABUTIN™, CLARITHROMYCIN™, and/or AZITHROMYCIN^{TM} to prophylactically treat or prevent an opportunistic *Mycobacterium tuberculosis* infection. In another specific embodiment, Therapeutics of the invention are used in any combination with GANCICLOVIR™, FOSCARNET™, and/or CIDOFOVIR™ to prophylactically treat or prevent an opportunistic cytomegalovirus infection. In another specific embodiment, Therapeutics of the invention are used in any combination with FLUCONAZOLE™; ITRACONAZOLE™, and/or KETOCONAZOLE™ to prophylactically treat or prevent an opportunistic fungal infection. In another specific embodiment, Therapeutics of the invention are used in any combination with ACYCLOVIR^{TM} and/or FAMCICOLVIR™ to prophylactically treat or prevent an opportunistic herpes simplex virus type I and/or type II infection. In another specific embodiment, Therapeutics of the invention are used in any combination with PYRIMETHAMINE^{TM} and/or LEUCOVORIN^{TM} to prophylactically treat or prevent an opportunistic *Toxoplasma gondii* infection. In another specific embodiment, Therapeutics of the invention are used in any combination with LEUCOVORIN™ and/or NEUPOGEN^{TM} to prophylactically treat or prevent an opportunistic bacterial infection.

In a further embodiment, the Therapeutics of the invention are administered in combination with an antiviral agent. Antiviral agents that may be administered with the Therapeutics of the invention include, but are not limited to, acyclovir, ribavirin, amantadine, and remantidine.

In a further embodiment, the Therapeutics of the invention are administered in combination with an antibiotic agent. Antibiotic agents that may be administered with the Therapeutics of the invention include, but are not limited to, amoxicillin, beta-lactamases, aminoglycosides, beta-lactam. (glycopeptide), beta-lactamases, Clindamycin, chloramphenicol, cephalosporins, ciprofloxacin, ciprofloxacin, erythromycin, flubroquinolones, macrolides, metronidazole, penicillins, quinolones, rifampin, streptomycin, sulfonamide, tetracyclines, trimethoprim, trimethoprimsulfamthoxazole, and vancomycin.

Conventional nonspecific immunosuppressive. agents, that may be administered in combination with the Therapeutics of the invention include, but are not limited to, steroids, cyclosporine, cyclosporine analogs, cyclophosphamide methylprednisone, prednisone, azathioprine, FK-506, 15-deoxyspergualin, and other immunosuppressive agents that act by suppressing the function of responding T cells.

In specific embodiments, Therapeutics of the invention are administered in combination with immunosuppressants. Immunosuppressants preparations that may be administered with the Therapeutics of the invention include, but are not limited to, ORTHOCLONE™ (OKT3), SANDBEWUNE™/NEORAL™/SANGDYA™ (cyclosporin), PROGRAF™ (tacrolimus), CELLCEPT™ (mycophenolate); Azathioprine, glucorticosteroids, and RAPAMUNE™ (sirolimus). In a specific embodiment, immunosuppressants may be used to prevent rejection of organ or bone marrow transplantation.

In an additional embodiment, Therapeutics of the invention are administered alone or in combination with one or more intravenous immune globulin preparations. Intravenous immune globulin preparations that may be administered with the Therapeutics of the invention include, but not limited to, GAMMAR™, IVEEGAM^{TM}, SANDOGLOBULIN™, GAMMAGARD S/D^{TM}, and GAMIMUNE^{TM}. In a specific embodiment, Therapeutics of the invention are administered in combination with intravenous immune globulin preparations in transplantation therapy (e.g., bone marrow transplant).

In an additional embodiment, the Therapeutics of the invention are administered alone or in combination with an anti-inflammatory agent. Antiinflammatory agents that may be administered with the Therapeutics of the invention include, but are not limited to, glucocorticoids and the nonsteroidal anti-inflammatories, aminoarylcarboxylic acid derivatives; arylacetic acid derivatives, arylbutyric acid derivatives, arylcarboxylic acids, arylpropionic acid derivatives, pyrazoles, pyrazolones, salicylic acid derivatives, thiazinecarboxamides, e-acetamidocaproic acid, S-adenosylmethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazoie, and tenidap.

In another embodiment, compostions of the invention are administered in combination with a chemotherapeutic agent. Chemotherapeutic agents that may be administered with the Therapeutics of the invention include, but are not limited to, antibiotic derivatives (e.g., doxorubicin, bleomycin, daunorubicin, and dactinomycin); antiestrogens (e.g., tamoxifen); antimetabolites (e.g., fluorouracil, 5-FU, methotrexate, floxuridine, interferon alpha-2b, glutamic acid, plicamycin, mercaptopurine, and 6-thioguanine); cytotoxic agents (e.g., carmustine, BCNU, lomustine, CCNU, cytosine arabinoside, cyclophosphamide, estramustine, hydroxyurea, procarbazine, mitomycin, busulfan, cis-platin, and vincristine sulfate); hormones (e.g., medroxyprogesterone, estramustine phosphate sodium, ethinyl estradiol, estradiol, megestrol acetate, methyltestosterone, diethylstilbestrol diphosphate, chlorotrianisene, and testolactone); nitrogen mustard derivatives (eg., mephalen, chorambucil, mechlorethamine (nitrogen mustard) and thiotepa); steroids and combinations (e.g., bethamethasone sodium phosphate); and others (e.g., dicarbazine, asparaginase, mitotane, vincristine sulfate, vinblastine sulfate, and etoposide).

In a specific embodiment, Therapeutics of the invention are administered in combination with CHOP (cyclophosphamide, doxorubicin, vincristine, and prednisone) or any combination of the components of CHOP. In another embodiment, Therapeutics of the invention are administered in combination with Rituximab. In a further embodiment, Therapeutics of the invention are administered with Rituxmab and CHOP, or Rituxmab and any combination of the components of CHOP.

In an additional embodiment, the Therapeutics of the invention are administered in combination with cytokines. Cytokines that may be administered with the Therapeutics of the invention include, but are not limited to, EL2, IL3, 1L4, IL5, IL6, IL7, IL10, IL12, IL13, IL15, anti-CD40, CD40L, IFN-gamma and TNF-alpha. In another embodiment, Therapeutics of the invention may be administered with any interleukin, including, but not limited to, IL-lalpha, EL-1beta, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, and IL-21.

In an additional embodiment, the Therapeutics of the invention are administered in combination with angiogenic proteins. Angiogenic proteins that may be administered with the Therapeutics of the invention include, but are not limited to, Glioma Derived Growth Factor (GDGF), as disclosed in European Patent Number EP-399816; Platelet Derived Growth Factor-A (PDGF-A), as disclosed in European Patent Number EP-682110; Platelet Derived Growth Factor-B (PDGF-B), as disclosed in European Patent Number EP-282317; Placental Growth Factor (PIGF), as disclosed in International Publication Number WO 92/06194; Placental Growth Factor-2 (P1GF-2), as disclosed in Hauser et al., Gorwth Factors, 4:259-268 (1993); Vascular Endothelial Growth Factor (VEGF), as disclosed in International Publication Number WO 90/13649; Vascular Endothelial Growth Factor-A (VEGF-A), as disclosed in European Patent Number EP-506477; Vascular Endothelial Growth Factor-2 (VEGF-2), as disclosed in International Publication Number WO 96/39515; Vascular Endothelial Growth Factor B (VEGF-3); Vascular Endothelial Growth Factor B-186 (VEGF-B186), as disclosed in International Publication Number WO 96/26736; Vascular Endothelial Growth Factor-D (VEGF-D), as disclosed in International Publication Number WO 98/02543; Vascular Endothelial Growth Factor-D (VEGF-D), as disclosed in International Publication Number WO 98/07832; and Vascular Endothelial Growth Factor-E (VEGF-E), as disclosed in German Patent Number DE19639601. The above mentioned references are incorporated herein by reference herein

In an additional embodiment, the Therapeutics of the invention are administered in combination with hematopoietic growth factors. Hematopoietic growth factors that may be administered with the Therapeutics of the invention include, but are not limited to, LEUKINE™ (SARGRAMOSTIM™) and NEUPOGEN™ (FILGRASTIM™).

In an additional embodiment, the Therapeutics of the invention are administered in combination with Fibroblast Growth Factors. Fibroblast Growth Factors that may be administered with the Therapeutics of the invention include, but are not limited to, FGF-1, FGF-2, FGF-3, FGF-4, FGF-5, FGF-6, FGF-7, FGF-8, FGF-9, FGF-10, FGF-11, FGF-12, FGF-13, FGF-14, and FGF-15.

In additional embodiments, the Therapeutics of the invention are administered in combination with other therapeutic or prophylactic regimens, such as, for example, radiation therapy.

### Example 24: Method of Treating Decreased Levels of the Polypeptide

The present invention relates to a method for treating an individual in need of an increased level of a polypeptide of the invention in the body comprising administering to such an individual a composition comprising a therapeutically effective amount of an agonist of the invention (including polypeptides of the invention). Moreover, it will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a Therapeutic comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

The present invention also relates to a method of treating an individual in need of a decreased level of a polypeptide of the invention in the body comprising administering to such an individual a composition comprising a therapeutically effective amount of an antagonist of the invention (including polypeptides and antibodies of the invention).

In one example, antisense technology is used to inhibit production of a polypeptide of the present invention. This technology is one example of a method of decreasing levels of a polypeptide, preferably a secreted form, due to a variety of etiologies, such as cancer. For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy-Ex Vivo

One method of gene therapy transplants fibroblasts, which are capable of expressing a polypeptide, onto a patient. Generally, fibroblasts are obtained from a subject by skin biopsy: The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37 degree C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond. to the 5' and 3' end sequences respectively as set forth in Example 1 using primers and having appropriate restriction sites and initiation/stop codons, if necessary. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

### Example 27: Gene Therapy Using Endogenous Genes Corresponding To Polynucleotides of the Invention

Another method of gene therapy according to the present invention involves operably associating the endogenous polynucleotide sequence of the invention with a promoter via homologous recombination as described, for example, in U.S. Patent NO: 5,641,670, issued June 24, 1997; International Publication NO: WO 96/29411, published September 26, 1996; International Publication NO: WO 94/12650, published August 4, 1994; Koller et al., *Proc. Natl. Acad. Sci. USA,* 86:8932-8935 (1989); and Zijlstra et al., *Nature,* 342:435-438 (1989). This method involves the activation of a gene which is present in the target cells, but which is not expressed in the cells, or is expressed at a lower level than desired.

Polynucleotide constructs are made which contain a promoter and targeting sequences, which are homologous to the 5' non-coding sequence of endogenous polynucleotide sequence, flanking the promoter. The targeting sequence will be sufficiently near the 5' end of the polynucleotide sequence so the promoter will be operably linked to the endogenous sequence upon homologous recombination. The promoter and the targeting sequences can be amplified using PCR. Preferably, the amplified promoter contains distinct restriction enzyme sites on the 5' and 3' ends. Preferably, the 3' end of the first targeting sequence contains the same restriction enzyme site as the 5' end of the amplified promoter and the 5' end of the second targeting sequence contains the same restriction site as the 3' end of the amplified promoter.

The amplified promoter and the amplified targeting sequences are digested with the appropriate restriction enzymes and subsequently treated with calf intestinal phosphatase. The digested promoter and digested targeting sequences are added together in the presence of T4 DNA ligase. The resulting mixture is maintained under conditions appropriate for ligation of the two fragments. The construct is size fractionated on an agarose gel then purified by phenol extraction and ethanol precipitation.

In this Example, the polynucleotide constructs are administered as naked polynucleotides via electroporation. However, the polynucleotide constructs may also be administered with transfection-facilitating agents, such as liposomes, viral sequences, viral particles, precipitating agents, etc. Such methods of delivery are known in the art.

Once the cells are transfected, homologous recombination will take place which results in the promoter being operably linked to the endogenous polynucleotide sequence. This results in the expression of polynucleotide corresponding to the polynucleotide in the cell. Expression may be detected by immunological staining, or any other method known in the art.

Fibroblasts are obtained from a subject by skin biopsy. The resulting tissue is placed in DMEM + 10% fetal calf serum. Exponentially growing or early stationary phase fibroblasts are trypsinized and rinsed from the plastic surface with nutrient medium. An aliquot of the cell suspension is removed for counting, and the remaining cells are subjected to centrifugation. The supernatant is aspirated and the pellet is resuspended in 5 ml of electroporation buffer (20 mM HEPES pH 7.3, 137 mM NaCl, 5 mM KCl, 0.7 mM Na₂ HPO₄, 6 mM dextrose). The cells are recentrifuged, the supernatant aspirated, and the cells resuspended in electroporation buffer containing 1 mg/ml acetylated bovine serum albumin. The final cell suspension contains approximately 3X10⁶ cells/ml. Electroporation should be performed immediately following resuspension.

Plasmid DNA is prepared according to standard techniques. For example, to construct a plasmid for targeting to the locus corresponding to the polynucleotide of the invention, plasmid pUC18 (MBI Fermentas, Amherst, NY) is digested with HindIII. The CMV promoter is amplified by PCR with an Xbal site on the 5' end and a BamHI site on the 3'end. Two non-coding sequences are amplified via PCR: one non-coding sequence (fragment 1) is amplified with a HindIII site at the 5' end and an Xba site at the 3'end; the other non-coding sequence (fragment 2) is amplified with a BamHI site at the 5'end and a HindIII site at the 3'end. The CMV promoter and the fragments (1 and 2) are digested with the appropriate enzymes (CMV promoter - XbaI and BamHI; fragment 1 - XbaI; fragment 2 - BamHI) and ligated together. The resulting ligation product is digested with HindIII, and ligated with the HindIII-digested pUC18 plasmid.

Plasmid DNA is added to a sterile cuvette with a 0.4 cm electrode gap (Bio-Rad). The final DNA concentration is generally at least 120 µg/ml. 0.5 ml of the cell suspension (containing approximately 1.5.X10⁶ cells) is then added to the cuvette, and the cell suspension and DNA solutions are gently mixed. Electroporation is performed with a Gene-Pulser apparatus (Bio-Rad). Capacitance and voltage are set at 960 µF and 250-300 V, respectively. As voltage increases, cell survival decreases, but the percentage of surviving cells that stably incorporate the introduced DNA into their genome increases dramatically. Given these parameters, a pulse time of approximately 14-20 mSec should be observed.

Electroporated cells are maintained at room temperature for approximately 5 min, and the contents of the cuvette are then gently removed with a sterile transfer pipette. The cells are added directly to 10 ml of prewarmed nutrient media (DMEM with 15% calf serum) in a 10 cm dish and incubated at 37 degree C. The following day, the media is aspirated and replaced with 10 ml of fresh media and incubated for a further 16-24 hours.

The engineered fibroblasts are then injected into the host, either alone or after having been grown to confluence on cytodex 3 microcarrier beads. The fibroblasts now produce the protein product. The fibroblasts can then be introduced into a patient as described above.

### Example 28: Method of Treatment Using Gene Therapy - In Vivo

Another aspect of the present invention is using *in vivo* gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide. The polynucleotide of the present invention may be operatively linked to a promoter or any other genetic elements necessary for the expression of the polypeptide by the target tissue. Such gene therapy and delivery techniques and methods are known in the art, see, for example, WO90/11092, WO98/11779 U.S. Patent NO. 5693622, 5705151, 5580859; Tabata et al., Cardiovasc. Res. 35(3):470-479 (1997); Chao et al., Pharmacol. Res. 35(6):517-522 (1997); Wolff, Neuromuscul. Disord. 7(5):314-318 (1997); Schwartz et al., Gene Ther. 3(5):405-411 (1996); Tsurumi et al., Circulation 94(12):3281-3290 (1996) (incorporated herein by reference).

The polynucleotide constructs may be delivered by any method that delivers injectable materials to the cells of an animal, such as, injection into the interstitial space of tissues (heart, muscle, skin, lung, liver, intestine and the like). The polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides of the present invention may also be delivered in liposome formulations (such as those taught in Felgner P.L. et al. (1995) Ann. NY Acad. Sci. 772:126-139 and Abdallah B. et al. (1995) Biol. Cell 85(1):1-7) which can be prepared by methods well known to those skilled in the art.

The polynucleotide vector constructs used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely 'differentiated cells, such as, for example, stem cells of blood or skin fibroblasts. In vivo muscle cells are particularly competent in their ability to take up and express polynucleotides.

For the naked polynucleotide injection; an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in muscle *in vivo* is determined as follows. Suitable template DNA for production of mRNA coding for polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supematants from injected and control mice. The results of the above experimentation in mice can be use to extrapolate proper dosages and other treatment parameters in humans and other animals using naked DNA.

### Example 29: Transgenic Animals.

The polypeptides of the invention can also be expressed in transgenic animals. Animals of any species, including, but not limited to, mice, rats, rabbits, hamsters, guinea pigs, pigs, micro-pigs, goats, sheep, cows and non-human primates, *e.g.*, baboons, monkeys, and chimpanzees may be used to generate transgenic animals. In a specific embodiment, techniques described herein or otherwise known in the art, are used to express polypeptides of the invention in humans, as part of a gene therapy protocol.

Any technique known in the art may be used to introduce the transgene (i.e., polynucleotides of the invention) into animals to produce the founder lines of transgenic animals. Such techniques include, but are not limited to, pronuclear microinjection (Paterson et al., Appl. Microbiol. Biotechnol. 40:691-698 (1994); Carver et al., Biotechnology (NY) 11:1263-1270 (1993); Wright et al., Biotechnology (NY) 9:830-834 (1991); and Hoppe et al., U.S. Pat. No. 4,873,191 (1989)); retrovirus mediated gene transfer into germ lines (Van der Putten et al., Proc. Natl. Acad. Sci., USA 82:6148-6152 (1985)), blastocysts or embryos; gene targeting in embryonic stem cells (Thompson et al., Cell 56:313-321 (1989)); electroporation of cells or embryos (Lo, 1983, Mol Cell. Biol. 3:1803-1814 (1983)); introduction of the polynucleotides of the invention using a gene gun (see, e.g., Ulmer et al., Science 259:1745 (1993); introducing nucleic acid constructs into embryonic pleuripotent stem cells and transferring the stem cells back into the blastocyst; and sperm-mediated gene transfer (Lavitrano et al., Cell 57:717-723 (1989); etc. For a review of such techniques, see Gordon, "Transgenic Animals," Int1. Rev. Cytol. 115:171-229 (1989), which is incorporated by reference herein in its entirety.

Any technique known in the art may be used to produce transgenic clones containing polynucleotides of the invention, for example, nuclear transfer into enucleated oocytes of nuclei from cultured embryonic, fetal, or adult cells induced to quiescence (Campell et al., Nature 380:64-66 (1996); Wilmut et al., Nature 385:810-813 (1997)).

The present invention provides for transgenic animals that carry the transgene in all their cells, as well as animals which carry the transgene in some, but not all their cells, *i.e.,* mosaic animals or chimeric. The transgene may he integrated as a single transgene or as multiple copies such as in concatamers, e.g., head-to-head tandems or head-to-tail tandems. The transgene may also be selectively introduced into and activated in a particular cell type by following, for example, the teaching of Lasko et al. (Lasko et al., Proc. Natl. Acad. Sci. USA 89:6232-6236 (1992)). The regulatory sequences required for such a cell-type specific activation will depend upon the particular cell type of interest, and will be apparent to those of skill in the art. When it is desired that the polynucleotide transgene be integrated into the chromosomal site of the endogenous gene, gene targeting is preferred. Briefly, when such a technique is to be utilized, vectors containing some nucleotide sequences homologous to the endogenous gene are designed for the purpose of integrating, via homologous recombination with chromosomal sequences, into and disrupting the function of the nucleotide sequence of the endogenous gene. The transgene may also be selectively introduced into a particular cell type, thus inactivating the endogenous gene in only that cell type, by following, for example, the teaching of Gu et al. (Gu et al., Science 265:103-106 (1994)). The regulatory sequences required for such a cell-type specific inactivation will depend upon the particular cell type of interest, and will be apparent to those of skill in the art.

Once transgenic animals have been generated, the expression of the recombinant gene may be assayed utilizing standard techniques: Initial screening may be accomplished by Southern blot analysis or PCR techniques to analyze animal tissues to verify that integration of the transgene has taken place. The level of mRNA expression of the transgene in the tissues of the transgenic animals may also be assessed using techniques which include, but are not limited to, Northern blot analysis of tissue samples obtained from the animal, *in situ* hybridization analysis, and reverse transcriptase-PCR (rt-PCR). Samples of transgenic gene-expressing tissue may also be evaluated immunocytochemically or immunohistochemically using antibodies specific for the transgene product.

Once the founder animals are produced, they may be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic animals to produce animals homozygous for a given integration site in order to both augment expression and eliminate the need for screening of animals by DNA analysis; crossing of separate homozygous lines to produce compound heterozygous or homozygous lines; and breeding to place the transgene on a distinct background that is appropriate for an experimental model of interest.

Transgenic animals of the invention have uses which include, but are not limited to, animal model systems useful in elaborating the biological function of polypeptides of the present invention, studying diseases, disorders, and/or conditions associated with aberrant expression, and in screening for compounds effective in ameliorating such diseases, disorders, and/or conditions.

### Example 30: Knock-Out Animals.

Endogenous gene expression can also be reduced by inactivating or "knocking out" the gene and/or its promoter using targeted homologous recombination. (*E.g*., see Smithies et al., Nature 317:230-234 (1985); Thomas & Capecchi, Cell 51:503-512 (1987); Thompson et al., Cell 5:313-321 (1989); each of which is incorporated by reference herein in its entirety). For example, a mutant, non-functional polynucleotide of the invention (or a completely unrelated DNA sequence) flanked by DNA homologous to the endogenous polynucleotide sequence (either the coding regions or regulatory regions of the gene) can be used, with or without a selectable marker and/or a negative selectable marker, to transfect cells that express polypeptides of the invention *in vivo.* In another embodiment, techniques known in the art are used to generate knockouts in cells that contain, but do not express the gene of interest. Insertion of the DNA construct, via targeted homologous recombination, results in inactivation of the targeted gene. Such approaches are particularly suited in research and agricultural fields where modifications to embryonic stem cells can be used to generate animal offspring with an inactive targeted gene (*e.g.,* see Thomas & Capecchi 1987 and Thompson 1989, *supra*). However this approach can be routinely adapted for use in humans provided the recombinant DNA constructs are directly administered or targeted to the required site *in vivo* using appropriate viral vectors that will be apparent to those of skill in the art.

In further embodiments of the invention, cells that are genetically engineered to express the polypeptides of the invention, or alternatively, that are genetically engineered not to express the polypeptides of the invention (e.g., knockouts) are administered to a patient *in vivo.* Such cells. may be obtained from the patient (i.e., animal, including human) or an MHC compatible donor and can include, but are not limited to fibroblasts, bone marrow cells, blood cells (e.g., lymphocytes), adipocytes, muscle cells, endothelial cells etc. The cells are genetically engineered *in vitro* using recombinant DNA techniques to introduce the coding sequence of polypeptides of the invention into the cells, or alternatively, to disrupt the coding sequence and/or endogenous regulatory sequence associated with the polypeptides of the invention, e.g., by transduction (using viral vectors, and preferably vectors that integrate the transgene into the cell genome) or transfection procedures, including, but not limited to, the use of plasmids, cosmids, YACs, naked DNA, electroporation, liposomes, etc. The coding sequence of the polypeptides of the invention can be placed under the control of a strong constitutive or inducible promoter or promoter/enhancer to achieve expression, and preferably secretion, of the polypeptides of the invention. The engineered cells which express and preferably secrete the polypeptides of the invention can be introduced into the patient systemically, e.g., in the circulation, or intraperitoneally.

Alternatively, the cells can be incorporated into a matrix and implanted in the body, e.g., genetically engineered fibroblasts can be implanted as part of a skin graft; genetically engineered endothelial cells can be implanted as part of a lymphatic or vascular graft. (See, for example, Anderson et al. U.S. Patent No. 5,399,349; and Mulligan & Wilson, U.S. Patent No. 5,460,959 each of which is incorporated by reference herein in its entirety).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

Transgenic and "knock-out" animals of the invention have uses which include, but are not limited to, animal model systems useful in elaborating the biological function of polypeptides of the present invention, studying diseases, disorders, and/or conditions associated with aberrant expression, and in screening for compounds effective in ameliorating such diseases, disorders, and/or conditions.

### Example 31: Production of an Antibody

### a) Hybridoma Technology

The antibodies of the present invention can be prepared by a variety of methods. (See, Current Protocols, Chapter 2.) As one example of such methods, cells expressing polypeptide(s) of the invention are administered to an animal to induce the production of sera containing polyclonal antibodies. In a preferred method, a preparation of polypeptide(s) of the invention is prepared and purified to render it substantially free of natural contaminants. Such a preparation is then introduced into an animal in order to produce polyclonal antisera of greater specific activity.

Monoclonal antibodies specific for polypeptide(s) of the invention are prepared using hybridoma technology. (Kohler et al., Nature 256:495 (1975); Kohler et al., Eur. J. Immunol. 6:511 (1976); Kohler et al., Eur. J. Immunol. 6:292 (1976); Hammerling et al., in: Monoclonal Antibodies and T-Cell Hybridomas, Elsevier, N.Y., pp. 563-681 (1981)). In general, an animal (preferably a mouse) is immunized with polypeptide(s) of the invention, or, more preferably, with a secreted polypeptide-expressing cell. Such polypeptide-expressing cells are cultured in any suitable tissue culture medium, preferably in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981)). The hybridoma cells obtained through such a selection are then assayed to identify clones which secrete antibodies capable of binding the polypeptide(s) of the invention.

Alternatively, additional antibodies capable of binding polypeptide(s) of the invention can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the polypeptide(s) of the invention protein-specific antibody can be blocked by polypeptide(s) of the invention. Such antibodies comprise anti-idiotypic antibodies to the polypeptide(s) of the invention protein-specific antibody and are used to immunize an animal to induce formation of further polypeptide(s) of the invention protein-specific antibodies.

For in vivo use of antibodies in humans, an antibody is "humanized". Such antibodies can be produced using genetic constructs derived from hybridoma cells producing the monoclonal antibodies described above. Methods for producing chimeric and humanized antibodies are known in the art and are discussed herein. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### b). Isolation Of Antibody Fragments Directed polypeptide(s) of the invention From A Library OfscFvs

Naturally occurring V-genes isolated from human PBLs are constructed into a library of antibody fragments which contain reactivities against polypeptide(s) of the invention to which the donor may or may not have been exposed (see e.g., U.S. Patent 5,885,793 incorporated herein by reference in its entirety).

Rescue of the Library. A library of scFvs is constructed from the RNA of human PBLs as described in PCT publication WO 92/01047. To rescue phage displaying antibody fragments, approximately 109 E. coli harboring the phagemid are used to inoculate 50 ml of 2xTY containing 1% glucose and 100 µg/ml of ampicillin (2xTY-AMP-GLU) and grown to an O.D. of 0.8 with shaking. Five ml of this culture is used to innoculate 50 ml of 2xTY-AMP-GLU, 2 x 108 TU of delta gene 3 helper (M13 delta gene III, see PCT publication WO 92/01047) are added and the culture incubated at 37°C for 45 minutes without shaking and then at 37°C for 45 minutes with shaking. The culture is centrifuged at 4000 r.p.m. for 10 min. and the pellet resuspended in 2 liters of 2xTY containing 100 µg/ml ampicillin and 50 ug/ml kanamycin and grown overnight. Phage are prepared as described in PCT publication WO 92/01047.

M13 delta gene III is prepared as follows: M13 delta gene III helper phage does not encode gene III protein, hence the phage(mid) displaying antibody fragments have a greater avidity of binding to antigen. Infectious M13 delta gene III particles are made by growing the helper phage in cells harboring a pUC19 derivative supplying the wild type gene III protein during phage morphogenesis. The culture is incubated for 1 hour at 37° C without shaking and then for a further hour at 37°C with shaking. Cells are spun down (IEC-Centra 8,400 r.p.m. for 10 min), resuspended in 300 ml 2xTY broth containing 100 µg ampicillin/ml and 25 µg kanamycin/ml (2xTY-AMP-KAN) and grown overnight, shaking at 37°C. Phage particles are purified and concentrated from the culture medium by two PEG-precipitations (Sambrook et al., 1990), resuspended in 2 ml PBS and passed through a 0.45 µm filter (Minisart NML; Sartorius) to give a final concentration of approximately 1013 transducing units/ml (ampicillin-resistant clones).

Panning of the Library. Immunotubes (Nuric) are coated overnight in PBS with 4 ml of either 100 µg/ml or 10 µg/ml of a polypeptide of the present invention. Tubes are blocked with 2% Marvel-PBS for 2 hours at 37°C and then washed 3 times in PBS. Approximately 1013 TU of phage is applied to the tube and incubated for 30 minutes at room temperature tumbling on an over and under turntable and then left to stand for another 1.5 hours. Tubes are washed 10 times with PBS 0.1 % Tween-20, and 10 times with PBS. Phage are eluted by adding 1 ml of 100 mM triethylamine and rotating 15 minutes on an under and over turntable after which the solution is immediately neutralized with 0.5 ml of 1.0M Tris-HCl, pH 7.4. Phage are then used to infect 10 ml of mid-log E. coli TG1 by incubating eluted phage with bacteria for 30 minutes at 37°C. The E. coli are then plated on TYE plates containing 1% glucose and 100 µg/ml ampicillin. The resulting bacterial library is then rescued with delta gene 3 helper phage as described above to prepare phage for a subsequent round of selection. This process is then repeated for a total of 4 rounds of affinity purification with tube-washing increased to 20 times with PBS, 0.1% Tween-20 and 20 times with PBS for rounds 3 and 4.

Characterization of Binders. Eluted phage from the 3rd and 4th rounds of selection are used to infect E. coli HB 2151 and soluble scFv is produced (Marks, et al., 1991) from single colonies for assay. ELISAs are performed with microtitre plates coated with either 10 pg/ml of the polypeptide of the present invention in 50 mM bicarbonate pH 9.6. Clones positive in ELISA are further characterized by PCR fingerprinting (see, e.g., PCT publication WO 92/01047) and then by sequencing. These ELISA positive clones may also be further characterized by techniques known in the art, such as, for example, epitope mapping, binding affinity, receptor signal transduction, ability to block or competitively inhibit antibody/antigen binding, and competitive agonistic or antagonistic activity.

### Example 32: Assays Detecting Stimulation or Inhibition of B cell Proliferation and Differentiation

Generation of functional humoral immune responses requires both soluble and cognate signaling between B-lineage cells and their microenvironment. Signals may impart a positive stimulus that allows a B-lineage cell to continue its programmed development, or a negative stimulus that instructs the cell to arrest its current developmental pathway. To date, numerous stimulatory and inhibitory signals have been found to influence B cell responsiveness including IL-2, IL-4, IL-5, IL-6, IL-7, IL10, IL-13, IL-14 and IL-15. Interestingly, these signals are by themselves weak effectors but can, in combination with various co-stimulatory proteins, induce activation, proliferation differentiation, homing, tolerance and death among B cell populations.

One of the best studied classes of B-cell co-stimulatory proteins is the TNF superfamily. Within this family CD40, CD27, and CD30 along with their respective ligands CD154, CD70, and CD153 have been found to regulate a variety of immune responses. Assays which allow for the detection and/or observation of the proliferation and differentiation of these B-cell populations and their precursors are valuable tools in determining the effects various proteins may have on these B-cell populations in terms of proliferation and differentiation. Listed below are two assays designed to allow for the detection of the differentiation, proliferation, or inhibition of B-cell populations and their precursors.

In Vitro Assay- Purified polypeptides of the invention, or truncated forms thereof, is assessed for its ability to induce activation, proliferation, differentiation or inhibition and/or death in B-cell populations and their precursors, The activity of the polypeptides of the invention on purified human tonsillar B cells, measured qualitatively over the dose range from 0.1 to 10,000 ng/mL, is assessed in a standard B-lymphocyte co-stimulation assay in which purified tonsillar B cells are cultured in the presence of either formalin-fixed Staphylococcus aureus Cowan I (SAC) or immobilized anti-human IgM antibody as the priming; agent. Second signals such as IL-2 and IL-15 synergize with SAC and IgM crosslinking to elicit B cell proliferation as measured by tritiated-thymidine incorporation. Novel synergizing agents can be readily identified using this assay. The assay involves isolating human tonsillar B cells by magnetic bead (MACS) depletion of CD3-positive cells. The resulting cell population is greater than 95% B cells as assessed by expression of CD45R(B220).

Various dilutions of each sample are placed into individual wells of a 96-well plate to which are added 10⁵ B-cells suspended in culture medium (RPMI 1640 containing 10% FBS, 5 X 10⁻⁵M 2MB, 100U/ml penicillin, 10ug/ml streptomycin, and 10⁻⁵ dilution of SAC) in a total volume of 150ul. Proliferation or inhibition is quantitated by a 20h pulse (luCi/well) with 3H-thymidine (6.7 Ci/mM) beginning. 72h post factor addition. The positive and negative controls are IL2 and medium respectively:

In Vivo Assay- BALB/c mice are injected (i.p.) twice per day with buffer only, or 2 mg/Kg of a polypeptide of the invention, or truncated forms thereof. Mice receive this treatment for 4 consecutive days, at which time they are sacrificed and various tissues and serum collected for analyses. Comparison of H&E sections from normal spleens and spleens treated with polypeptides of the invention identify the results of the activity of the polypeptides on spleen cells, such as the diffusion of periarterial lymphatic sheaths, and/or significant increases in the nucleated cellularity of the red pulp regions, which may indicate the activation of the differentiation and proliferation of B-cell populations. Immunohistochemical studies using a B cell marker, anti-CD45R(B220), are used to determine whether any physiological changes to splenic cells, such as splenic disorganization, are due to increased B-cell representation within loosely defined B-cell zones that infiltrate established T-cell regions.

Flow cytometric analyses of the spleens from mice treated with polypeptide is used to indicate whether the polypeptide specifically increases the proportion of ThB+, CD45R(B220)dull B cells over that which is observed in control mice.

Likewise, a predicted consequence of increased mature B-cell representation in vivo is a relative increase in serum Ig titers. Accordingly, serum IgM and IgA levels are compared between buffer and polypeptide-treated mice.

The studies described in this example tested activity of a polypeptide of the invention. However, one skilled in the art could easily modify the exemplified studies to test the activity of polynucleotides of the invention (e.g., gene therapy), agonists, and/or antagonists ofpolynucleotides or polypeptides of the invention.

### Example 33: T Cell Proliferation Assay

### Proliferation assay for Resting PBLs.

A CD3-induced proliferation assay is performed on PBMCs and is measured by the uptake of ³H-thymidine. The assay is performed as follows. Ninety-six well plates are coa with 100 microliters per well of mAb to CD3 (HIT3a, Pharmingen) or isotype-matched cor mAb (B33.1) overnight at 4 C (1 microgram/ml in .05M bicarbonate buffer, pH 9.5), then three times with PBS. PBMC are isolated by F/H gradient centrifugation from human peripheric blood and added to quadruplicate wells (5 x 10⁴/Well) of mAb coated plates in RPMI containing 10% FCS and P/S in the presence of varying concentrations of TNF Delta and/or TNF Epsilon protein (total volume 200 microliters). Relevant protein buffer and medium alone are controls. After 48 hr. culture at 37 C, plates are spun for 2 min. at 1000 rpm and 100 microliters of supernatant is removed and stored -20 C for measurement of IL-2 (or other cytokines) if effect on proliferation is observed. Wells are supplemented with 100 microliters of medium containing 0.5 microcuries of ³H-thymidine and cultured at 37 C for 18-24 hr. Wells are harvested and incorporation of ³H-thymidine used as a measure of proliferation. Anti-CD3 alone is the positive control for proliferation. IL-2 (100 U/ml) is also used as a control which enhances proliferation. Control antibody which does not induce proliferation of T cells is used as the negative controls for the effects of TNF Delta and/or TNF Epsilon proteins.

Alternatively, a proliferation assay on resting PBL (peripheral blood lymphocytes) is measured by the up-take of ³H-thymidine. The assay is performed as follows. PBMC are isolated by Ficoll (LSM, ICN Biotechnologies, Aurora, Ohio) gradient centrifugation from human peripheral blood, and are cultured overnight in 10% (Fetal Calf Serum, Biofluids, Rockville, MD)/RPMI (Gibco BRL, Gaithersburg, MD). This overnight incubation period allows the adherent cells to attach to the plastic, which results in a lower background in the assay as there are fewer cells that can act as antigen presenting cells or that might be producing growth factors. The following day the non-adherent cells are collected, washed and used in the proliferation assay. The assay is performed in a 96 well plate using 2×10⁴ cells/well in a final volume of 200 microliters. The supernatants (e.g., CHO or 293T supernatants) expressing the protein of interest are tested at a 30% final dilution, therefore 60ul are added to 140ul of 10% FCS/RPMI containing the cells. Control supernatants are used at the same final dilution and express the following proteins: vector (negative control), IL-2 (*), IFNγ, TNFα, IL- 10 and TR2. In addition to the control supernatants, recombinant human IL-2 (R & D Systems, Minneapolois, MN) at a final concentration of 100ng/ml is also used. After 24 hours of culture, each well is pulsed with 1uCi of ³H-thymidine (Nen, Boston, MA). Cells are then harvested 20 hours following pulsing and incorporation of ³H-thymidine is used as a measure of proliferation. Results are expressed as an average of triplicate samples plus or minus standard error.
(*) The amount of the control cytokines IL-2, IFNγ, TNFα and IL-10 produced in each transfection varies between 300pg to 5ng/ml.

### Costimulation assay.

A costimulation assay on resting PBL (peripheral blood lymphocytes) is performed in the presence of immobilized antibodies to CD3 and CD28. The use of antibodies specific for the invariant regions of CD3 mimic the induction of T cell activation that would occur through stimulation of the T cell receptor by an antigen. Cross-linking of the TCR (first signal) in the absence of a costimulatory signal (second signal) causes very low induction of proliferation and will eventually result in a state of "anergy", which is characterized by the absence of growth and inability to produce cytokines. The addition of a costimulatory signal such as an antibody to CD28, which mimics the action of the costimulatory molecule. B7-1 expressed on activated APCs, results in enhancement of T cell responses including cell survival and production of IL-2. Therefore this type of assay allows to detect both positive and negative effects caused by addition of supematants expressing the proteins of interest on T cell proliferation.

The assay is performed as follows. Ninety-six well plates are coated with 100ng/ml anti-CD3 and 5ug/ml anti-GD28 (Phanningen, San Diego, CA) in a final volume of 100ul and incubated overnight at 4C. Plates are washed twice with PBS before use. PBMC are isolated by Ficoll (LSM, ICN Biotechnologies, Aurora, Ohio) gradient centrifugation from human peripheral blood, and are cultured overnight in 10% FCS(Fetal Calf Serum, Biofluids, Rockville, MD)/RPMI (Gibco BRL, Gaithersburg, MD). This overnight incubation period allows the adherent cells to attach to the plastic, which results in a lower background in the assay as there are fewer. cells that can act as antigen presenting cells or that might be producing growth factors. The following day the non adherent cells are collected, washed and used in the proliferation assay. The assay is performed in a 96 well plate using 2 x10⁴ cells/well in a final volume of 200ul. The supernatants (e.g., CHO or 293T supernatants) expressing the protein of interest are tested at a 30% final dilution, therefore 60ul are added to 140ul of 10% FCS/RPMI containing the cells. Control supernatants are used at the same final dilution and express the following proteins: vector only (negative control), IL-2, IFNγ, TNFα, IL-10 and TR2. In addition to the control supernatants recombinant human IL-2 (R & D Systems, Minneapolis, MN) at a final concentration of 10ng/ml is also used. After 24 hours of culture, each well is pulsed with 1uCi of ³H-thymidine (Nen, Boston, MA). Cells are then harvested 20 hours following pulsing and incorporation of ³H-thymidine is used as a measure of proliferation. Results are expressed as an average of triplicate samples plus or minus standard error.

### Costimulation assay: IFN γ and IL-2 ELISA

The assay is performed as follows. Twenty-four well plates are coated with either 300ng/ml or 600ng/ml anti-CD3 and 5ug/ml anti-CD28 (Pharmingen, San Diego, CA) in a final volume of 500ul and incubated overnight at 4C. Plates are washed twice with PBS before use. PBMC are isolated by Ficoll (LSM, ICN Biotechnologies, Aurora, Ohio) gradient centrifugation from human peripheral blood, and are cultured overnight in 10% FCS(Fetal Calf Serum, Biofluids, Rockville, MD)/RPMI (Gibco BRL, Gaithersburg, MD). This overnight incubation period allows the adherent cells to attach to the plastic, which results in a lower background in the assay as there are fewer cells that can act as antigen presenting cells or that might be producing growth factors. The following day the non adherent cells are collected, washed and used in the costimulation assay. The assay is performed in the pre-coated twenty-four well plate using 1 x 10⁵ cells/well in a final volume of 900ul. The supernatants (293T supernatants) expressing the protein of interest are tested at a 30% final dilution, therefore 300ul are added to 600ul of 10% FCS/RPMI containing the cells. Control supernatants are used at the same final dilution and express the following proteins: vector only(negative control), IL-2, IFNγ, IL-12 and IL-18. In addition to the control supernatants recombinant human IL-2 (all cytokines were purchased from R & D Systems, Minneapolis, MN) at a final concentration of 10ng/ml, IL-12 at a final concentration of 1ng/ml and IL-18 at a final concentration of 50ng/ml are also used. Controls and unknown samples are tested in duplicate. Supernatant samples (250ul) are collected 2 days and 5 days after the beginning of the assay. ELISAs to test for IFNγ arid IL-2 secretion are performed using kits purchased from R & D Systems, (Minneapolis, MN). Results are expressed as an average of duplicate samples plus or minus standard error.

### Proliferation assay for preactivated-resting T cells.

A proliferation assay on preactivated-resting T cells is performed on cells that are previously activated with the lectin phytohemagglutinin (PHA). Lectins are polymeric plant proteins that can bind to residues on T cell surface glycoproteins including the TCR and act as polyclonal activators. PBLs treated with PHA and then cultured in the presence of low doses of IL-2 resemble effector T cells. These cells are generally more sensitive to further activation induced by growth factors such as IL-2. This is due to the expression of high affinity IL-2 receptors that allows this population to respond to amounts of IL-2 that are 100 fold lower than what would have an effect on a naive T cell. Therefore the use of this type of cells might enable to detect the effect of very low doses of an unknown growth factor, that would not be sufficient to induce proliferation on resting (naïve ) T cells.

The assay is performed as follows. PBMC are isolated by F/H gradient centrifugation from human peripheral blood, and are cultured in10% FCS(Fetal Calf Serum, Biofluids, Rockville, MD)/RPMI (Gibco BRL, Gaithersburg, MD) in the presence of 2ug/ml PHA (Sigma, Saint Louis, MO) for three days. The cells are then washed in PBS and cultured in 10% FCS/RPMI in the presence of 5ng/ml of human recombinant IL-2 (R & D Systems, Minneapolis, MN) for 3 days. The cells are washed and rested in starvation medium (1%FCS/RPMI) for 16 hours prior to the beginning of the proliferation assay. An aliquot of the cells is analyzed by FACS to determine the percentage of T cells (CD3 positive cells) present; this usually ranges between 93-97% depending on the donor: The assay is performed in a 96 well plate using 2 x10⁴ cells/well in a final volume of 200ul. The supernatants (e.g., CHO or 293T supernatants) expressing the protein of interest are tested at a 30% final dilution, therefore 60ul are added to 140ul of in10% FCS/RPMI containing the cells. Control supernatants are used at the same final dilution and express the following proteins: vector (negative control), IL-2, IFNγ, TNFα, IL-10 and TR2. In addition to the control supernatants recombinant human IL-2 at a final concentration of 10ng/ml is also used. After 24 hours of culture, each well is pulsed with 1uCi of ³H-thymidine(Nen, Boston, MA). Cells are then harvested 20 hours following pulsing and incorporation of ³H-thymidine is used as a measure of proliferation. Results are expressed as an average of triplicate samples plus or minus standard error.

The studies described in this example test activity of polypeptides of the invention. However, one skilled in the art could easily modify the exemplified studies to test the activity of polynucleotides of the invention (e.g., gene therapy), agonists, and/or antagonists of polynucleotides or polypypeptides of the invention.

### Example 34: Effect of Polypeptides of the Invention on the Expression of MHC Class II, Costimulatory and Adhesion Molecules and Cell Differentiation of Monocytes and Monocyte-Derived Human Dendritic Cells

Dendritic cells are generated by the expansion of proliferating precursors found in the peripheral blood: adherent PBMC or elutriated monocytic fractions are cultured for 7-10 days with GM-CSF (50 ng/ml) and IL-4 (20 ng/ml). These dendritic cells have the characteristic phenotype of immature cells (expression of CD1, CD80, CD86, CD40 and MHC class II antigens). Treatment with activating factors, such as TNF-α, causes a rapid change in surface phenotype (increased expression of MHC class I and II; costimulatory and adhesion molecules, downregulation of FCγRII, upregulation of CD83). These changes correlate with increased antigen-presenting capacity and with functional maturation of the dendritic cells.

FACS analysis of surface antigens is performed as follows. Cells are treated 1-3 days with increasing concentrations of polypeptides of the invention or LPS (positive control), washed with PBS containing 1% BSA and 0.02 mM sodium azide, and then incubated with 1:20 dilution of appropriate FITC- or PE-labeled monoclonal antibodies for 30 minutes at 4 degrees C. After an additional wash, the labeled cells are analyzed by flow cytometry on a FACScan (Becton Dickinson).

Effect on the production of cytokines. Cytokines generated by dendritic cells, in particular IL-12, are important in the initiation of T-cell dependent immune responses. IL-12 strongly influences the development of Th1 helper T-cell immune response, and induces cytotoxic T and NK cell function. An ELISA is used to measure the IL-12 release as follows. Dendritic cells (10⁶/ml) are treated with increasing concentrations of polypeptides of the invention for 24 hours. LPS (100 ng/ml) is added to the cell culture as positive control. Supernatants from the cell cultures are then collected and analyzed for IL-12 content using commercial ELISA kit (e..g, R & D Systems (Minneapolis, The standard protocols provided with the kits are used.

Effect on the expression of MHC Class II, costimulatory and adhesion molecules. Three major families of cell surface antigens can be identified on monocytes: adhesion molecules, molecules involved in antigen presentation, and Fc receptor. Modulation of the expression of MHC class II antigens and other costimulatory molecules, such as B7 and ICAM-1, may result in changes in the antigen presenting capacity of monocytes and ability to induce T cell activation. Increase expression of Fc receptors may correlate with improved monocyte cytotoxic activity, cytokine release and phagocytosis.

FACS analysis is used to examine the surface antigens as follows. Monocytes are treated 1-5 days with increasing concentrations of polypeptides of the invention or LPS (positive control), washed with PBS containing 1% BSA and 0.02 mM sodium azide, and then incubated with 1:20 dilution of appropriate FITC- or PE-labeled monoclonal antibodies for 30 minutes at 4 degreesC. After an additional wash, the labeled cells are analyzed by flow cytometry on a FACScan (Becton Dickinson).

Monocyte activation and/or increased survival. Assays for molecules that activate (or alternatively, inactivate) monocytes and/or increase monocyte survival (or alternatively, decrease monocyte survival) are known in the art and may routinely be applied to determine whether a molecule of the invention functions as an inhibitor or activator of monocytes. Polypeptides, agonists, or antagonists of the invention can be screened using the three assays described below. For each of these assays, Peripheral blood mononuclear cells (PBMC) are purified from single donor leukopacks (American Red Cross, Baltimore, MD) by centrifugation through a Histopaque gradient (Sigma). Monocytes are isolated from PBMC by counterflow centrifugal elutriation.

Monocyte Survival Assay. Human peripheral blood monocytes progressively lose viability when cultured in absence of serum or other stimuli. Their death results from internally regulated process (apoptosis). Addition to the culture of activating factors, such as TNF-alpha dramatically improves cell survival and prevents DNA fragmentation. Propidium iodide (PI) staining is used to measure apoptosis as follows. Monocytes are cultured for 48 hours in polypropylene tubes in serum-free medium (positive control), in the presence of 100 ng/ml TNF-alpha (negative control), and in the presence of varying concentrations of the compound to be tested. Cells are suspended at a concentration of 2 x 10⁶/ml in PBS containing PI at a final concentration of 5 µg/ml, and then incubaed at room temperature for 5 minutes before FACScan analysis. PI uptake has been demonstrated to correlate with DNA fragmentation in this experimental paradigm.

Effect on cytokine release. An important function of monocytes/macrophages is their regulatory activity on other cellular populations of the immune system through the release of cytokines after stimulation. An ELISA to measure cytokine release is performed as follows. Human monocytes are incubated at a density of 5×10⁵ cells/ml with increasing concentrations of the a polypeptide of the invention and under the same conditions, but in the absence of the polypeptide. For IL-12 production, the cells are primed overnight with IFN (100 U/ml) in presence of a polypeptide of the invention. LPS (10 ng/ml) is then added. Conditioned media are collected after 24h and kept frozen until use. Measurement of TNF-alpha, IL-10, MCP-1 and IL-8 is then performed using a commercially available ELISA kit (e..g, R & D Systems (Minneapolis, MN)) and applying the standard protocols provided with the kit.

Oxidative burst. Purified monocytes are plated in 96-w plate at 2-1x10⁵ cell/well. Increasing concentrations of polypeptides of the invention are added to the wells in a total volume of 0.2 ml culture medium (RPMI 1640 + 10% FCS, glutamine and antibiotics). After 3 days incubation, the plates are centrifuged and the medium is removed from the wells. To the macrophage monolayers, 0.2 ml per well of phenol red solution (140 mM NaCl, 10 mM potassium phosphate buffer pH 7.0, 5.5 mM dextrose, 0.56 mM phenol red and 19 U/ml of HRPO) is added, together with the stimulant (200 nM PMA). The plates are incubated at 37°C for 2 hours and the reaction is stopped by adding 20 µl 1N NaOH per well. The absorbance is read at 610 nm. To calculate the amount of H₂O₂ produced by the macrophages, a standard curve of a H₂O₂ solution of known molarity is performed for each experiment.

The studies described in this example tested activity of a polypeptide of the invention. However, one skilled in the art could easily modify the exemplified studies to test the activity of polypeptides, polynucleotides (e.g., gene therapy), agonists, and/or antagonists of the invention.

### Example 35: Biological Effects of Polypeptides of the Invention

### Astrocyte and Neuronal Assays·

Recombinant polypeptides of the invention, expressed in *Escherichia coli* and purified as described above, can be tested for activity in promoting the survival, neurite outgrowth, or phenotypic differentiation of cortical neuronal cells and for inducing the proliferation of glial fibrillary acidic protein immunopositive cells, astrocytes. The selection of cortical cells for the bioassay is based on the prevalent expression of FGF-1 and FGF-2 in cortical structures and on the previously reported enhancement of cortical neuronal survival resulting from FGF-2 treatment. A thymidine incorporation assay, for example, can be used to elucidate a polypeptide of the invention's activity on these cells.

Moreover, previous reports describing the biological effects of FGF-2 (basic FGF) on cortical or hippocampal neurons *in vitro* have demonstrated increases in both neuron survival and neurite outgrowth (Walicke et al., "Fibroblast growth factor promotes survival of dissociated hippocampal neurons and enhances neurite extension." *Proc. Natl. Acad. Sci. USA 83*:3012-3016. (1986), assay herein incorporated by reference in its entirety). However, reports from experiments done on PC-12 cells suggest that these two responses are not necessarily synonymous and may depend on not only which FGF is being tested but also on which receptor(s) are expressed on the target cells. Using the primary cortical neuronal culture paradigm, the ability of a polypeptide of the invention to induce neurite outgrowth can be compared to the response achieved with FGF-2 using, for example, a thymidine incorporation assay.

### Fibroblast and endothelial cell assays·

Human lung fibroblasts are obtained from Clonetics (San Diego, CA) and maintained in growth media from Clonetics. Dermal microvascular endothelial cells are obtained from Cell Applications (San Diego, CA). For proliferation assays, the human lung fibroblasts and dermal microvascular endothelial cells can be cultured at 5,000 cells/well in a 96-well plate for one day in growth medium. The cells are then incubated for one day in 0.1 % BSA basal medium. After replacing the medium with fresh 0.1% BSA medium, the cells are incubated with the test proteins for 3 days. Alamar Blue (Alamar Biosciences, Sacramento, CA) is added to each well to a final concentration of 10%. The cells are incubated for 4 hr. Cell viability is measured by reading in a CytoFluor fluorescence reader. For the PGE₂ assays, the human lung fibroblasts are cultured at 5,000 cells/well in a 96-well plate for one day. After a medium change to 0.1% BSA basal medium, the cells are incubated with FGF-2 or polypeptides of the invention with or without IL-1α for 24 hours. The supernatants are collected and assayed for PGE₂ by EIA kit (Cayman, Ann Arbor, MI), For the IL-6 assays, the human lung fibroblasts are cultured at 5,000 cells/well in a 96-well plate for one day. After a medium change to 0.1% BSA basal medium, the cells are incubated with FGF-2 or with or without polypeptides of the invention IL-1α for 24 hours: The supernatants are collected and assayed for IL-6 by ELISA kit (Endogen, Cambridge, MA).

Human lung fibroblasts are cultured with FGF-2 or polypeptides of the invention for 3 days in basal medium before the addition of Alamar Blue to assess effects on growth of the fibroblasts. FGF-2 should show a stimulation at 10 - 2500 ng/ml which can be used to compare stimulation with polypeptides of the invention.

### Parkinson Models.

The loss of motor function in Parkinson's disease is attributed to a deficiency of striatal dopamine resulting from the degeneration of the nigrostriatal dopaminergic projection neurons. An animal model for Parkinson's 'that has been extensively characterized involves the systemic administration of 1-methyl-4 phenyl 1,2,3,6-tetrahydropyridine (MPTP). In the CNS, MPTP is taken-up by astrocytes and catabolized by monoamine oxidase B to 1-methyl-4-phenyl pyridine (MPP⁺) and released. Subsequently, MPP⁺ is actively accumulated in dopaminergic neurons by the high-affinity reuptake transporter for dopamine. MPP⁺ is then concentrated in mitochondria by the electrochemical gradient and selectively inhibits nicotidamide adenine disphosphate: ubiquinone oxidoreductionase (complex 1), thereby interfering with electron transport and eventually generating oxygen radicals.

It has been demonstrated in tissue culture paradigms that FGF-2 (basic FGF) has trophic activity towards nigral dopaminergic neurons (Ferrari et al., Dev. Biol. 1989). Recently, Dr. Unsicker's group has demonstrated that administering FGF-2 in gel foam implants in the striatum results in the near complete protection of nigral dopaminergic neurons from the toxicity associated with MPTP exposure (Otto and Unsicker, J. Neuroscience, 1990).

Based on the data with FGF-2, polypeptides of the invention can be evaluated to determine whether it has an action similar to that of FGF-2 in enhancing dopaminergic neuronal survival *in vitro* and it can also be tested *in vivo* for protection of dopaminergic neurons in the striatum from the damage associated with MPTP treatment. The potential effect of a polypeptide of the invention is first examined in vitro in a dopaminergic neuronal cell culture paradigm. The cultures are prepared by dissecting the midbrain floor plate from gestation day 14 Wistar rat embryos. The tissue is dissociated with trypsin and seeded at a density of 200,000 cells/cm² on polyorthinine-laminin coated glass coverslips. The cells are maintained in Dulbecco's Modified Eagle's medium and F12 medium containing hormonal supplements (N1). The cultures are fixed with paraformaldehyde after 8 days in vitro and are processed for tyrosine hydroxylase, a specific marker for dopminergic neurons, immunohistochemical staining. Dissociated cell cultures are prepared from embryonic rats. The culture medium is changed every third day and the factors are also added at that time.

Since the dopaminergic neurons are isolated from animals at gestation day 14, a developmental time which is past the stage when the dopaminergic precursor cells are proliferating, an increase in the number of tyrosine hydroxylase immunopositive neurons would represent an increase in the number of dopaminergic neurons surviving *in vitro.* Therefore, if a polypeptide of the invention acts to prolong the survival of dopaminergic neurons, it would suggest that the polypeptide may be involved in Parkinson's Disease.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 36: The Effect of Polypeptides of the Invention on the Growth of Vascular Endothelial Cells

On day 1, human umbilical vein endothelial cells (HUVEC) are seeded at 2-5x10⁴ cells/35 mm dish density in M199 medium containing 4% fetal bovine serum (FBS), 16 units/ml heparin, and 50 units/ml endothelial cell growth supplements (ECGS, Biotechnique, Inc.). On day 2, the medium is replaced with M199-containing 10% FBS, 8 units/ml heparin. A polypeptide having the amino acid sequence of SEQ ID NO:Y, and positive controls, such as VEGF and basic FGF (bFGF) are added, at varying concentrations. On days 4 and 6, the medium is replaced. On day 8, cell number is determined with a Coulter Counter.

An increase in the number of HUVEC cells indicates that the polypeptide of the invention may proliferate vascular endothelial cells.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 37: Stimulatory Effect of Polypeptides of the Invention on the Proliferation of Vascular Endothelial Cells

For evaluation of mitogenic activity of growth factors, the colorimetric MTS (3-(4,5-dimethylthiazol-2-yl)-5-(3-carboxymethoxyphenyl)-2-(4-sulfophenyl)2H-tetrazolium) assay with the electron coupling reagent PMS (phenazine methosulfate) was performed (CellTiter 96 AQ, Promega). Cells are seeded in a 96-well plate (5,000 cells/well) in 0.1 mL serum-supplemented medium and are allowed to attach overnight. After serum-starvation for 12 hours in 0.5% FBS, conditions (bFGF, VEGF₁₆₅ or a polypeptide of the invention in 0.5% FBS) with or without Heparin (8 U/ml) are added to wells for 48 hours. 20 mg of MTS/PMS mixture (1:0.05) are added per well and allowed to incubate for 1 hour at 37°C before measuring the absorbance at 490 nm in an ELISA plate reader. Background absorbance from control wells (some media, no cells) is subtracted, and seven wells are performed in parallel for each condition. See, Leak *et al. In Vitro Cell. Dev. Biol. 30A:*512-518 (1994).

The studies described in this example tested activity of a polypeptide of the invention. However, one skilled in the art could easily modify the exemplified studies to test the activity of polynucleotides (e.g., gene therapy), agonists, and/or antagonists of the invention.

### Example 38: Inhibition of PDGF-induced Vascular Smooth Muscle Cell Proliferation Stimulatory Effect

HAoSMC proliferation can be measured, for example, by BrdUrd incorporation. Briefly, subconfluent, quiescent cells grown on the 4-chamber slides are transfected with CRP or FITC-labeled AT2-3LP. Then, the cells are pulsed with 10% calf serum and 6 mg/ml BrdUrd. After 24 h, immunocytochemistry is performed by using BrdUrd Staining Kit (Zymed Laboratories). In brief, the cells are incubated with the biotinylated mouse anti-BrdUrd antibody at 4 degrees C for 2 h after being exposed to denaturing solution and then incubated with the streptavidin-peroxidase and diaminobenzidine. After counterstaining with hematoxylin, the cells are mounted for microscopic examination, and the BrdUrd-positive cells are counted. The BrdUrd index is calculated as a percent of the BrdUrd-positive cells to the total cell number. In addition, the simultaneous detection of the BrdUrd staining (nucleus) and the FITC uptake (cytoplasm) is performed for individual cells by the concomitant use of bright field illumination and dark field-UV fluorescent illumination. See, Hayashida et al., J. Biol. Chem. 6:271(36):21985-21992 (1996).

The studies described in this example tested activity of a polypeptide of the invention. However, one skilled in the art could easily modify the exemplified studies to test the activity of polynucleotides (e.g., gene therapy), agonists, and/or antagonists of the invention.

### Example 39: Stimulation of Endothelial Migration

This example will be used to explore the possibility that a polypeptide of the invention may stimulate lymphatic endothelial cell migration.

Endothelial cell migration assays are performed using a 48 well microchemotaxis chamber (Neuroprobe Inc., Cabin John, MD; Falk, W., et al., J. Immunological Methods 1980;33:239-247). Polyvinylpyrrolidone-free polycarbonate filters with a pore size of 8 um (Nucleopore Corp. Cambridge, MA) are coated with 0.1% gelatin for at least 6 hours at room temperature and dried under sterile air. Test substances are diluted to appropriate concentrations in M199 supplemented with 0.25% bovine serum albumin (BSA), and 25 ul of the final dilution is placed in the lower chamber of the modified Boyden apparatus. Subconfluent, early passage (2-6) HUVEC or BMEC cultures are washed and trypsinized for the minimum time required to achieve cell detachment. After placing the filter between lower and upper chamber, 2.5 x 10⁵ cells suspended in 50 ul M199 containing 1% FBS are seeded in the upper compartment. The apparatus is then incubated for 5 hours at 37°C in a humidified chamber with 5% CO2 to allow cell migration. After the incubation period, the filter is removed and the upper side of the filter with the non-migrated cells is scraped with a rubber policeman. The filters are fixed with methanol and stained with a Giemsa solution (Diff-Quick, Baxter, McGraw Park; IL). Migration is quantified by counting cells of three random high-power fields (40x)in each well, and all groups are performed in quadruplicate.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 40: Stimulation of Nitric Oxide Production by Endothelial Cells

Nitric oxide released by the vascular endothelium is believed to be a mediator of vascular endothelium relaxation. Thus, activity of a polypeptide of the invention can be assayed by determining nitric oxide production by endothelial cells in response to the polypeptide.

Nitric oxide is measured in 96-well plates of confluent microvascular endothelial cells after 24 hours starvation and a subsequent 4 hr exposure to various levels of a positive control (such as VEGF-1) and the polypeptide of the invention. Nitric oxide in the medium is determined by use of the Griess reagent to measure total nitrite after reduction of nitric oxide-derived nitrate by nitrate reductase. The effect of the polypeptide of the invention on nitric oxide release is examined on HUVEC.

Briefly, NO release from cultured HUVEC monolayer is measured with a NO-specific polarographic electrode connected to a NO meter (Iso-NO, World Precision Instruments Inc.) (1049). Calibration of the NO elements is performed according to the following equation:

2KNO₂+2KI+2H₂SO₄62NO+I₂+2H₂O+2K₂SO₄

The standard calibration curve is obtained by adding graded concentrations of KNO₂ (0, 5, 10, 25, 50, 100, 250, and 500 nmol/L) into the calibration solution containing KI and H₂SO₄. The specificity of the Iso-NO electrode to NO is previously determined by measurement of NO from authentic NO gas (1050). The culture medium is removed and HUVECs are washed twice with Dulbecco's phosphate buffered saline. The cells are then bathed in 5 ml of filtered Krebs-Henseleit solution in 6-well plates, and the cell plates are kept on a slide warmer (Lab Line Instruments Inc.) To maintain the temperature at 37°C. The NO sensor probe is inserted vertically into the wells, keeping the tip of the electrode 2 mm under the surface of the solution, before addition of the different conditions. S-nitroso acetyl penicillamin (SNAP) is used as a positive control. The amount of released NO is expressed as picomoles per 1x10⁶ endothelial cells. All values reported arc means of four to six measurements in each group (number of cell culture wells). See, Leak *et al. Biochem. and Biophys. Res. Comm. 217*:96-105 (1995).

The studies described in this example tested activity of polypeptides of the invention. However, one skilled in the art could easily modify the exemplified studies to test the activity of polynucleotides (e.g., gene therapy), agonists, and/or antagonists of the invention.

### Example 41: Effect of Polypepides of the Invention on Cord Formation in Angiogenesis

Another step in angiogenesis is cord formation, marked by differentiation of endothelial cells. This bioassay measures the ability of microvascular endothelial cells to form capillary-like structures (hollow structures) when cultured *in vitro.*

CADMEC (microvascular endothelial cells) are purchased from Cell Applications, Inc. as proliferating (passage 2) cells and are cultured in Cell Applications' CADMEC Growth Medium and used at passage 5. For the *in vitro* angiogenesis assay, the wells of a 48-well cell culture plate-are coated with Cell Applications' Attachment Factor Medium (200 ml/well) for 30 min. at 37°C. CADMEC are seeded onto the coated wells at 7,500 cells/well and cultured overnight in Growth Medium. The Growth Medium is then replaced with 300 mg Cell Applications' Chord Formation Medium containing control buffer or a polypeptide of the invention (0.1 to 100 ng/ml) and the cells are cultured for an additional 48 hr. The numbers and lengths of the capillary-like chords are quantitated through use of the Boeckeler VIA-170 video image analyzer. All assays are done in triplicate.

Commercial (R&D) VEGF (50 ng/ml) is used as a positive control, b-esteradiol (1 ng/ml) is used as a negative control. The appropriate buffer (without protein) is also utilized as a control.

The studies described in this example tested activity of a polypeptide of the invention. However,.one skilled in the art could easily modify the exemplified studies to test the activity of polynucleotides (e.g., gene therapy), agonists, and/or antagonists of the invention.

### Example 42: Angiogenic Effect on Chick Chorioallantoic Membrane

Chick chorioallantoic membrane (CAM) is a well-established system to examine angiogenesis. Blood vessel formation on CAM is easily visible and quantifiable. The ability of polypeptides of the invention to stimulate angiogenesis in CAM can be examined.

Fertilized eggs of the White Leghorn chick (*Gallus gallus*) and the Japanese qual (*Coturnix coturnix*) are incubated at 37.8°C and 80% humidity. Differentiated CAM of 16-day-old chick and 13-day-old qual embryos is studied with the following methods.

On Day 4 of development, a window is made into the egg shell of chick eggs. The embryos are checked for normal development and the eggs sealed with cellotape. They are further incubated until Day 13. Thermanox coverslips (Nunc, Naperville, IL) are cut into disks of about 5 mm in diameter. Sterile and salt-free growth factors are dissolved in distilled water and about 3.3 mg/ 5 ml are pipetted on the disks. After air-drying, the inverted disks are applied on CAM. After 3 days, the specimens are fixed in 3% glutaraldehyde and 2% formaldehyde and rinsed in 0.12 M sodium cacodylate buffer. They are photographed with a stereo microscope [Wild M8] and embedded for semi- and ultrathin sectioning as described above. Controls are performed with carrier disks alone.'

The studies described in this example tested activity of a polypeptide of the invention. However, one skilled in the art could easily modify the exemplified studies to test the activity of polynucleotides (e.g., gene therapy), agonists, and/or antagonists of the invention.

### Example 43: Angiogenesis Assay Using a Matrigel Implant in Mouse

*In vivo* angiogenesis assay of a polypeptide of the invention measures the ability of an existing capillary network to form new vessels in an implanted capsule of murine extracellular matrix material (Matrigel). The protein is mixed with the liquid Matrigel at 4 degree C and the mixture is then injected subcutaneously in mice where it solidifies. After 7 days, the solid "plug" of Matrigel is removed and examined for the presence of new blood vessels. Matrigel is purchased from Becton Dickinson Labware/Collaborative Biomedical Products.

When thawed at 4 degree C the Matrigel material is a liquid. The Matrigel is mixed with a polypeptide of the invention at 150 ng/ml at 4 degrees C and drawn into cold 3 ml syringes. Female C57B1/6 mice approximately 8 weeks old are injected with the mixture of Matrigel and experimental protein at 2 sites at the midventral aspect of the abdomen (0.5 ml/site). After 7 days, the mice are sacrificed by cervical dislocation, the Matrigel plugs are removed and cleaned (i.e., all clinging membranes and fibrous tissue is removed). Replicate whole plugs are fixed in neutral buffered 10% formaldehyde, embedded in paraffin and used to produce sections for histological examination after staining with Masson's Trichrome. Cross sections from 3 different regions of each plug are processed. Selected sections are stained for the presence of vWF. The positive control for this assay is bovine basic FGF (150 ng/ml). Matrigel alone is used to determine basal levels of angiogenesis.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 44: Rescue of Ischemia in Rabbit Lower Limb Model

To study the in vivo effects of polynucleotides and polypeptides of the invention on ischemia, a rabbit hindlimb ischemia model is created by surgical removal of one femoral arteries as described previously (Takeshita *et al., Am J. Pathol 147*:1649-1660 (1995)). The excision of the femoral artery results in retrograde propagation of thrombus and occlusion of the external iliac artery. Consequently, blood flow to the ischemic limb is dependent upon collateral vessels originating from the internal iliac artery (Takeshitaet *al. Am J. Pathol 147*:1649-1660 (1995)). An interval of 10 days is allowed for post-operative recovery of rabbits and development of endogenous collateral vessels. At 10 day post-operatively (day 0), after performing a baseline angiogram, the internal iliac artery of the ischemic limb is transfected with 500 mg naked expression plasmid containing a polynucleotide of the invention by arterial gene transfer technology using a hydrogel-coated balloon catheter as described (Riessen *et al. Hum Gene Ther. 4*:749-758 (1993); Leclerc *et al. J. Clin. Invest. 90*: 936-944 (1992)). When a polypeptide of the invention is used in the treatment, a single bolus of 500 mg polypeptide of the invention or control is delivered into the internal iliac artery of the ischemic limb over a period of 1 min. through an infusion catheter. On day 30, various parameters are measured in these rabbits: (a) BP ratio - The blood pressure ratio of systolic pressure of the ischemic limb to that of normal limb; (b) Blood Flow and Flow Reserve - Resting FL: the blood flow during undilated condition and Max FL: the blood flow during fully dilated condition (also an indirect measure of the blood vessel amount) and Flow Reserve is reflected by the ratio of max FL: resting FL; (c) Angiographic Score - This is measured by the angiogram of collateral vessels. A score is determined by the percentage of circles in an overlaying grid that with crossing opacified arteries divided by the total number m the rabbit thigh; (d) Capillary density - The number of collateral capillaries determined in light microscopic sections taken from hindlimbs.

The studies described in this example tested activity of polynucleotides and polypeptides of the invention. However, one skilled in the art could easily modify the exemplified studies to test the agonists, and/or antagonists of the invention.

### Example 45: Effect of Polypeptides of the Invention on Vasodilation

Since dilation of vascular endothelium is important in reducing blood pressure, the ability of polypeptides of the invention to affect the blood pressure in spontaneously hypertensive rats (SHR) is examined. Increasing doses (0, 10, 30, 100, 300, and 900 mg/kg) of the polypeptides of the invention are administered to 13-14 week old spontaneously hypertensive rats (SHR). Data are expressed as the mean +/- SEM. Statistical analysis are performed with a paired t-test and statistical significance is defined as p<0.05 vs. the response to buffer alone.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 46: Rat Ischemic Skin Flap Model

The evaluation parameters include skin blood flow, skin temperature, and factor VIII immunohistochemistry or endothelial alkaline phosphatase reaction. Expression of polypeptides of the invention, during the skin ischemia, is studied using in situ hybridization.

The study in this model is divided into three parts as follows:
a) Ischemic skin
b) Ischemic skin wounds
c) Normal wounds

The experimental protocol includes:
a) Raising a 3x4 cm, single pedicle full-thickness random skin flap (myocutaneous flap over the lower back of the animal).
b) An excisional wounding (4-6 mm in diameter) in the ischemic skin (skin-flap).
c) Topical treatment with a polypeptide of the invention of the excisional wounds (day 0, 1, 2, 3, 4 post-wounding) at the following various dosage ranges: 1mg to 100 mg.
d) Harvesting the wound tissues at day 3, 5, 7, 10, 14 and 21 post-wounding for histological, immunohistochemical, and in situ studies.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 47: Peripheral Arterial Disease Model

Angiogenic therapy using a polypeptide of the invention is a novel therapeutic strategy to obtain restoration of blood flow around the ischemia in case of peripheral arterial diseases. The experimental protocol includes:
a) One side of the femoral artery is ligated to create ischemic muscle of the hindlimb, the other side of hindlimb serves as a control.
b) a polypeptide of the invention, in a dosage range of 20 mg - 500 mg, is delivered intravenously and/or intramuscularly 3 times (perhaps more) per week for 2-3 weeks.
c) The ischemic muscle tissue is collected after ligation of the femoral artery at 1, 2, and 3 weeks for the analysis of expression of a polypeptide of the invention and histology. Biopsy is also performed on the other side of normal muscle of the contralateral hindlimb.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 48: Ischemic Myocardial Disease Model

A polypeptide of the invention is evaluated as a potent mitogen capable of stimulating the development of collateral vessels, and restructuring new vessels after coronary artery occlusion. Alteration of expression of the polypeptide is investigated in situ. The experimental protocol includes:
a) The heart is exposed through a left-side thoracotomy in the rat. Immediately, the left coronary artery is occluded with a thin suture (6-0) and the thorax is closed.
b) a polypeptide of the invention, in a dosage range of 20 mg - 500 mg, is delivered intravenously and/or intramuscularly 3 times (perhaps, more) per week for 2-4 weeks.
c) Thirty days after the surgery, the heart is removed and cross-sectioned for morphometric and in situ analyzes.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 49: Rat Corneal Wound Healing Model

This animal model shows the effect of a polypeptide of the invention on neovascularization. The experimental protocol includes:
a) Making a 1-1.5 mm long incision from the center of cornea into the stromal layer.
b) Inserting a spatula below the lip of the incision facing the outer comer of the eye.
c) Making a pocket (its base is 1-1.5 mm form the edge of the eye).
d) Positioning a pellet, containing 50ng- 5ug of a polypeptide of the invention, within the pocket.
e) Treatment with a polypeptide of the invention can also be applied topically to the corneal wounds in a dosage range of 20mg - 500mg (daily treatment for five days).

The studies described in this example tested activity of a polypeptide of the invention. However, one skilled in the art could easily modify the exemplified studies to test the activity of polynucleotides (e.g., gene therapy), agonists, and/or antagonists of the invention.

### Example 50: Diabetic Mouse and Glucocorticoid-Impaired Wound Healing Models

### A. Diabetic db+/db+ Mouse ModeL

To demonstrate that a polypeptide of the invention accelerates the healing process, the genetically diabetic mouse model of wound healing is used. The full thickness wound healing model in the db+/db+ mouse is a well characterized, clinically relevant and reproducible model of impaired wound healing. Healing of the diabetic wound is dependent on formation of granulation tissue and re-epithelialization rather than contraction (Gartner, M.H. *et al., J. Surg. Res. 52*:389 (1992); Greenhalgh, D.G. *et al.*, *Am. J. Pathol. 136*:1235 (1990)).

The diabetic animals have many of the characteristic features observed in Type II diabetes mellitus. Homozygous (db+/db+) mice are obese in comparison to their normal heterozygous (db+/+m) littermates. Mutant diabetic (db+/db+) mice have a single autosomal recessive mutation on chromosome 4 (db+) (Coleman *et. al. Proc. Natl. Acad. Sci. USA 77*:283-293 (1982)). Animals show polyphagia, polydipsia and polyuria. Mutant diabetic mice (db+/db+) have elevated blood glucose, increased or normal insulin levels, and suppressed cell-mediated immunity (Mandel *et al., J. Immunol. 120*:1375 (1978); Debray-Sachs, M. *et al., Clin. Exp. Immunol. 51 (1)*:1-7 (1983); Leiter *et al., Am. J. of Pathol. 114*:46-55 (1985)). Peripheral neuropathy, myocardial complications, and microvascular lesions, basement membrane thickening and glomerular filtration abnormalities have been described in these animals (Norido, F. *et al., Exp. Neurol. 83(2)*:221-232 (1984); Robertson *et al., Diabetes 29(1)*:60-67 (1980); Giacomelli *et al., Lab Invest. 40(4)*:460-473 (1979); Coleman, D.L., *Diabetes 31.(Suppl)*:1-6 (1982)). These homozygous diabetic mice develop hyperglycemia that is resistant to insulin analogous to human type II diabetes (Mandel *et al., J. Immunol. 120*:1375-1377 (1978)).

The characteristics observed in these animals suggests that healing in this model may be similar to the healing observed in human diabetes (Greenhalgh, *et al., Am. J. of Pathol. 136*:1235-1246 (1990)).

Genetically diabetic female C57BL/KsJ (db+/db+) mice and their non-diabetic (db+/+m) heterozygous littermates are used in this study (Jackson Laboratories). The animals are purchased at 6 weeks of age and are 8 weeks old at the beginning of the study. Animals are individually housed and received food and water ad libitum. All manipulations are performed using aseptic techniques. The experiments are conducted according to the rules and guidelines of Human Genome Sciences, Inc. Institutional Animal Care and Use Committee and the Guidelines for the Care and Use of Laboratory Animals.

Wounding protocol is performed according to previously reported methods (Tsuboi, R and Rifkin, D.B., *J. Exp. Med. 172*:245-251 (1990)). Briefly, on the day of wounding, animals are anesthetized with an intraperitoneal injection of Avertin (0.01 mg/mL), 2,2,2-tribromoethanol and 2-methyl-2-butanol dissolved in deionized water. The dorsal region of the animal is shaved and the skin washed with 70% ethanol solution and iodine. The surgical area is dried with sterile gauze prior to wounding. An 8 mm full-thickness wound is then created using a Keyes tissue punch. Immediately following wounding, the surrounding skin is gently stretched to eliminate wound expansion. The wounds are left open for the duration of the experiment. Application of the treatment is given topically for 5 consecutive days commencing on the day of wounding. Prior to treatment, wounds are gently cleansed with sterile saline and gauze sponges.

Wounds are visually examined and photographed at a fixed distance at the day of surgery and at two day intervals thereafter. Wound closure is determined by daily measurement on days 1-5 and on day 8. Wounds are measured horizontally and vertically using a calibrated Jameson caliper. Wounds are considered healed if granulation tissue is no longer visible and the wound is covered by a continuous epithelium.

A polypeptide of the invention is administered using at a range different doses, from 4mg to 500mg per wound per day for 8 days in vehicle. Vehicle control groups received 50mL of vehicle solution.

Animals are euthanized on day 8 with an intraperitoneal injection of sodium pentobarbital (300mg/kg). The wounds and surrounding skin are then harvested for histology and immunohistochemistry. Tissue specimens are placed in 10% neutral buffered formalin in tissue cassettes between biopsy sponges for further processing.

Three groups of 10 animals each (5 diabetic and 5 non-diabetic controls) are evaluated: 1) Vehicle placebo control, 2) untreated group, and 3) treated group.

Wound closure is analyzed by measuring the area in the vertical and horizontal axis and obtaining the total square area of the wound. Contraction is then estimated by establishing the differences between the initial wound area (day 0) and that of post treatment (day 8). The wound area on day 1 is 64mm², the corresponding size of the dermal punch. Calculations are made using the following formula:$\text{[Open area on day 8] - [Open area on day 1] / [Open area on day 1]}$

Specimens are fixed in 10% buffered formalin and paraffin embedded blocks are sectioned perpendicular to the wound surface (5mm) and cut using a Reichert-Jung microtome. Routine hematoxylin-eosin (H&E) staining is performed on cross-sections of bisected wounds. Histologic examination of the wounds are used to assess whether the healing process and the morphologic appearance of the repaired skin is altered by treatment with a polypeptide of the invention. This assessment included verification of the presence of cell accumulation, inflammatory cells, capillaries, fibroblasts, re-epithelialization and epidermal maturity (Greehhalgh, D.G. *et al., Am. J. Pathol. 136:*1235 (1990)). A calibrated lens micrometer is used by a blinded observer.

Tissue sections are also stained immunohistochemically with a polyclonal rabbit anti-human keratin antibody using ABC Elite detection system. Human skin is used as a positive tissue control while non-immune IgG is used as a negative control. Keratinocyte growth is determined by evaluating the extent of reepithelialization of the wound using a calibrated lens micrometer.

Proliferating cell nuclear antigen/cyclin (PCNA) in skin specimens is demonstrated by using anti-PCNA antibody (1:50) with an ABC Elite detection system. Human colon cancer can serve as a positive tissue control and human brain tissue can be used as a negative tissue control. Each specimen includes a section with omission of the primary antibody and substitution with non-immune mouse IgG. Ranking of these sections is based on the extent of proliferation on a scale of 0-8, the lower side of the scale reflecting slight proliferation to the higher side reflecting intense proliferation.

Experimental data are analyzed using an unpaired t test. A p value of < 0.05 is considered significant.

### B. Steroid Impaired Rat Model

The inhibition of wound healing by steroids has been well documented in various *in vitro* and *in vivo* systems (Wahl, Glucocorticoids and Wound healing. In: AntiInflammatory Steroid Action: Basic and Clinical Aspects. 280-302 (1989); Wahlet *al., J. Immunol. 115:* 476-481 (1975); Werb *et al., J. Exp. Med. 147*:1684-1694 (1978)). Glucocorticoids retard wound healing by inhibiting angiogenesis, decreasing vascular permeability (Ebert *et al., An. Intern. Med. 37*:701-705 (1952)), fibroblast proliferation, and collagen synthesis (Beck *et al., Growth Factors. 5:* 295-304 (1991); Haynes *et al., J. Clin. Invest. 61:* 703-797 (1978)) and producing a transient reduction of circulating monocytes (Haynes et al., *J. Clin. Invest. 61:* 703-797 (1978); Wahl, "Glucocorticoids and wound healing", *In:* Antiinflammatory Steroid Action: Basic and Clinical Aspects, Academic Press, New York, pp. 280-302 (1989)). The systemic administration of steroids to impaired wound healing is a well establish phenomenon in rats (Beck *et al., Growth Factors. 5:* 295-304 (1991); Haynes *et al., J. Clin. Invest*. *61:* 703-797 (1978); Wahl, "Glucocorticoids and wound healing", *In:* Antiinflammatory Steroid Action: Basic and Clinical Aspects, Academic Press, New York, pp. 280-302 (1989); Pierce *et al., Proc. Natl. Acad. Sci. USA 86*: 2229-2233 (1989)).

To demonstrate that a polypeptide of the invention can accelerate the healing process, the effects of multiple topical applications of the polypeptide on full thickness excisional skin wounds in rats in which healing has been impaired by the systemic administration of methylprednisolone is assessed.

Young adult male Sprague Dawley rats weighing 250-300 g (Charles River Laboratories) are used in this example. The animals are purchased at 8 weeks of age and are 9 weeks old at the beginning of the study. The healing response of rats is impaired by the systemic administration of methylprednisolone (17mg/kg/rat intramuscularly) at the time of wounding. Animals are individually housed and received food and water ad *libitum.* All manipulations are performed using aseptic techniques. This study is conducted according to the rules and guidelines of Human Genome Sciences; Inc. Institutional Animal Care and Use Committee and the Guidelines for the Care and Use of Laboratory Animals.

The wounding protocol is followed according to section A, above. On the day of wounding, animals are anesthetized with an intramuscular injection of ketamine (50 mg/kg) and xylazine (5 mg/kg). The dorsal region of the animal is shaved and the skin washed with 70% ethanol and iodine solutions. The surgical area is dried with sterile gauze prior to wounding. An 8 mm full-thickness wound is created using a Keyes tissue punch. The wounds are left open for the duration of the experiment. Applications of the testing materials are given topically once a day for 7 consecutive days commencing on the day of wounding and subsequent to methylprednisolone administration. Prior to treatment, wounds are gently cleansed with sterile saline and gauze sponges.

Wounds are visually examined and photographed at a fixed distance at the day of wounding and at the end of treatment. Wound closure is determined by daily measurement on days 1-5 and on day 8. Wounds are measured horizontally and vertically using a calibrated Jameson caliper. Wounds are considered healed if granulation tissue is no longer visible and the wound is covered by a continuous epithelium.

The polypeptide of the invention is administered using at a range different doses, from 4mg to 500mg per wound per day for 8 days in vehicle. Vehicle control groups received 50mL of vehicle solution.

Animals are euthanized on day 8 with an intraperitoneal injection of sodium pentobarbital (300mg/kg). The wounds and surrounding skin are then harvested for histology. Tissue specimens are placed in 10% neutral buffered formalin in tissue cassettes between biopsy sponges for further processing.

Four groups of 10 animals each (5 with methylprednisolone and' 5 without glucocorticoid) are evaluated: 1) Untreated group 2) Vehicle placebo control 3) treated groups.

Wound closure is analyzed by measuring the area in the vertical and horizontal axis and obtaining the total area of the wound. Closure is then estimated by establishing the differences between the initial wound area (day 0) and that of post treatment (day 8). The wound area on day 1 is 64mm², the corresponding size of the dermal punch. Calculations are made using the following formula:$\text{[Open area on day 8] - [Open area on day 1] / [Open area on day 1]}$

Specimens are fixed in 10% buffered formalin and paraffin embedded blocks are sectioned perpendicular to the wound surface (5mm) and cut using an Olympus microtome. Routine hematoxylin-eosin (H&E) staining is performed on cross-sections of bisected wounds. Histologic examination of the wounds allows assessment of whether the healing process and the morphologic appearance of the repaired skin is improved by treatment with a polypeptide of the invention. A calibrated lens micrometer is used by a blinded observer to determine the distance of the wound gap.

Experimental data are analyzed using an unpaired t test. A p value of < 0.0.5 is considered significant.

The studies described in this example tested activity of a polypeptide of the invention. However, one skilled in the art could easily modify the exemplified studies to test. the activity of polynucleotides (e.g., gene therapy), agonists, and/or antagonists of the. invention.

### Example 51: Lymphadema Animal Model

**or** The purpose of this experimental approach is to create an appropriate and consistent lymphedema model for testing the therapeutic effects of a polypeptide of the invention in lymphangiogenesis and re-establishment of the lymphatic circulatory system in the rat hind limb. Effectiveness is measured by swelling volume of the affected limb, quantification of the amount of lymphatic vasculature, total blood plasma protein, and histopathology. Acute lymphedema is observed for 7-10 days. Perhaps more importantly, the chronic progress of the edema is followed for up to 3-4 weeks.

Prior to beginning surgery, blood sample is drawn for protein concentration analysis. Male rats weighing approximately ∼350g are dosed with Pentobarbital. Subsequently, the right legs are shaved from knee to hip. The shaved area is swabbed with gauze soaked in 70% EtOH. Blood is drawn for serum total protein testing. Circumference and volumetric measurements are made prior to injecting dye into paws after marking 2 measurement levels (0.5 cm above heel, at mid-pt of dorsal paw). The intradermal dorsum of both right and left paws are injected with 0.05 ml of 1% Evan's Blue. Circumference and volumetric measurements are then made following injection of dye into paws.

Using the knee joint as a landmark, a mid-leg inguinal incision is made circumferentially allowing the femoral vessels to be located. Forceps and hemostats are used to dissect and separate the skin flaps. After locating the femoral vessels, the lymphatic vessel that runs along side and underneath the vessel(s) is located. The main lymphatic vessels in this area are then electrically coagulated suture ligated.

Using a microscope, muscles in back of the leg (near the semitendinosis and adductors) are bluntly dissected. The popliteal lymph node is then located. The 2 proximal and 2 distal lymphatic vessels and distal blood supply of the popliteal node are then and ligated by suturing. The popliteal lymph node, and any accompanying adipose tissue, is then removed by cutting connective tissues.

Care is taken to control any mild bleeding resulting from this procedure. After lymphatics are occluded, the skin flaps are sealed by using liquid skin (Vetbond) (AJ Buck). The separated skin edges are sealed to the underlying muscle tissue while leaving a gap of ∼0.5 cm around the leg. Skin also may be anchored by suturing to underlying muscle when necessary.

To avoid infection, animals are housed individually with mesh (no bedding). Recovering animals are checked daily through the optimal edematous peak, which typically occurred by day 5-7. The plateau edematous peak are then observed. To evaluate the intensity of the lymphedema, the circumference and volumes of 2 designated places on each paw before operation and daily for 7 days are measured. The effect plasma proteins on lymphedema is determined and whether protein analysis is a useful testing perimeter is also investigated. The weights of both control and edematous limbs are evaluated at 2 places. Analysis is performed in a blind manner.

Circumference Measurements: Under brief gas anesthetic to prevent limb movement, a cloth tape is used to measure limb circumference. Measurements are done at the ankle bone and dorsal paw by 2 different people then those 2 readings are averaged. Readings are taken from both control and edematous limbs.

Volumetric Measurements: On the day of surgery, animals are anesthetized with Pentobarbital and are tested prior to surgery. For daily volumetrics animals are under brief halothane anesthetic (rapid immobilization and quick recovery), both legs are shaved and equally marked using waterproof marker on legs. Legs are first dipped in water, then dipped into instrument to each marked level then measured by Buxco edema soflware(Chen/Victor). Data is recorded by one person, while the other is dipping the limb to marked area.

Blood-plasma protein measurements: Blood is drawn, spun, and serum separated prior to surgery and then at conclusion for total protein and Ca2+ comparison.

Limb Weight Comparison: After drawing blood, the animal is prepared for tissue collection. The limbs are amputated using a quillitine, then both experimental and control legs are cut at the ligature and weighed. A second weighing is done as the tibio-cacaneal joint is disarticulated and the foot is weighed.

Histological Preparations: The transverse muscle located behind the knee (popliteal) area is dissected and arranged in a metal mold, filled with freezeGel, dipped into cold methylbutane, placed into labeled sample bags at - 80EC until sectioning. Upon sectioning, the muscle is observed under fluorescent microscopy for lymphatics.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 52: Suppression of TNF alpha-induced adhesion molecule expression by a Polypeptide of the Invention

The recruitment of lymphocytes to areas of inflammation and angiogenesis involves specific receptor-ligand interactions between cell surface adhesion molecules (CAMs) on lymphocytes and the vascular endothelium. The adhesion process, in both normal and pathological settings, follows a multi-step cascade that involves intercellular adhesion molecule-1 (ICAM-1), vascular cell adhesion molecule-1 (VCAM-1), and endothelial leukocyte adhesion molecule-1 (E-selectin) expression on endothelial cells (EC). The expression of these molecules and others on the vascular endothelium determines the efficiency with which leukocytes may adhere to the local vasculature and extravasate into the local tissue during the development of an inflammatory response. The local concentration of cytokines and growth factor participate in the modulation of the expression of these CAMs.

Tumor necrosis factor alpha (TNF-a), a potent proinflammatory cytokine, is a stimulator of all three CAMs on endothelial cells and may be involved in a wide variety of inflammatory responses, often resulting in a pathological outcome.

The potential of a polypeptide of the invention to mediate a suppression ofTNF-a induced CAM expression can be examined. A modified ELISA assay which uses ECs as a solid phase absorbent is employed to measure the amount of CAM expression on TNF-a treated ECs when co-stimulated with a member of the FGF family of proteins.

To perform the experiment, human umbilical vein endothelial cell (HUVEC) cultures are obtained from pooled cord harvests and maintained in growth medium (EGM-2; Clonetics, San Diego, CA) supplemented with 10% FCS and 1% penicillin/streptomycin in a 37 degree C humidified incubator containing 5% CO₂. HUVECs are seeded in 96-well plates at concentrations of 1 x 10⁴ cells/well in EGM medium at 37 degree C for 18-24 hrs or until confluent. The monolayers are subsequently washed 3 times with a serum-free solution of RPMI-1640 supplemented with 100 U/ml penicillin and 100 mg/ml streptomycin, and treated with a given cytokine and/or growth factor(s) for 24 h at 37 degree C. Following incubation, the cells are then evaluated for CAM expression.

Human Umbilical Vein Endothelial cells (HUVECs) are grown in a standard 96 well plate to confluence. Growth medium is removed from the cells and replaced with 90 ul of 199 Medium (10% FBS). Samples for testing and positive or negative controls are added to the plate in triplicate (in 10 ul volumes). Plates are incubated at 37 degree C for either 5 h (selectin and integrin expression) or 24 h (integrin expression only). Plates are aspirated to remove medium and 100 µl of 0.1% parafonnaldehyde-PBS(with Ca++ and Mg++) is added to each well. Plates are held at 4°C for 30 min.

Fixative is then removed from the wells and wells are washed 1X with PBS(+Ca,Mg)+0.5% BSA and drained. Do not allow the wells to dry. Add 10 µl of diluted primary antibody to the test and control wells. Anti-ICAM-1-Biotin, Anti-VCAM-1-Biotin and Anti-E-selectin-Biotin are used at a concentration of 10 µg/ml (1:10 dilution of 0.1 mg/ml stock antibody). Cells are incubated at 37°C for 30 min. in a humidified environment. Wells are washed X3 with PBS(+Ca,Mg)+0.5% BSA.

Then add 20 µl of diluted ExtrAvidin-Alkaline Phosphotase (1:5,000 dilution) to each well and incubated at 37°C for 30 min. Wells are washed X3 with PBS(+Ca,Mg)+0.5% BSA. 1 tablet of p-Nitrophenol Phosphate pNPP is dissolved in 5 ml of glycine buffer (pH 10.4). 100 µl of pNPP substrate in glycine buffer is added to each test well. Standard wells in triplicate are prepared from the working dilution of the ExtrAvidin-Alkaline Phosphotase in glycine buffer: 1:5,000 (10°) > 10^{-0.5} > 10⁻¹ > 10^{-1.5}. 5 µl of each dilution is added to triplicate wells and the resulting AP content in each well is 5.50 ng, 1.74 ng, 0.55 ng, 0.18 ng. 100 µl of pNNP reagent must then be added to each of the standard wells. The plate must be incubated at 37°C for 4h. A volume of 50 µl of 3M NaOH is added to all wells. The results are quantified on a plate reader at 405 nm. The background subtraction option is used on blank wells filled with glycine buffer only. The template is set up to indicate the concentration of AP-conjugate in each standard well [ 5.50 ng; 1.74 ng; 0.55 ng; 0.18 ng]. Results are indicated as amount of bound AP-conjugate in each sample.

The studies described in this example tested activity of a polypeptide of the invention. However, one skilled in the art could easily modify the exemplified studies to test the activity of polynucleotides (e.g., gene therapy), agonists, and/or antagonists of the invention.

### Example 53: Assay for the Stimulation of Bone Marrow CD34+ Cell Proliferation

This assay is based on the ability of human CD34+ to proliferate in the presence of hematopoietic growth factors and evaluates the ability of isolated polypeptides expressed in mammalian cells to stimulate proliferation of CD34+ cells.

It has been previously shown that most mature precursors will respond to only a single signal. More immature precursors require at least two signals to respond. Therefore, to test the effect of polypeptides on hematopoietic activity of a wide range of progenitor cells, the assay contains a given polypeptide in the presence or absence of other hematopoietic growth factors. Isolated cells are cultured for 5 days in the presence of Stem Cell Factor (SCF) in combination with tested sample. SCF alone has a very limited effect on the proliferation of bone marrow (BM) cells, acting in such conditions only as a "survival" factor. However, combined with any factor exhibiting stimulatory effect on these cells (e.g., IL-3), SCF will cause a synergistic effect. Therefore, if the tested polypeptide has a stimulatory effect on a hematopoietic progenitors, such activity can be easily detected. Since normal BM cells have a low level of cycling cells, it is likely that any inhibitory effect of a given polypeptide, or agonists or antagonists thereof, might not be detected. Accordingly, assays for an inhibitory effect on progenitors is preferably tested in cells that are first subjected to *in vitro* stimulation with SCF+IL+3, and then contacted with the compound that is being evaluated for inhibition of such induced proliferation.

Briefly, CD34+ cells are isolated using methods known in the art. The cells are thawed and resuspended in medium (QBSF 60 serum-free medium with 1% L-glutamine (500ml) Quality Biological, Inc., Gaithersburg, MD Cat# 160-204-101). After several gentle centrifugation steps at 200 x g, cells are allowed to rest for one hour. The cell count is adjusted to 2.5 x 10⁵ cells/ml. During this time, 100 µl of sterile water is added to the peripheral wells of a 96-well plate. The cytokines that can be tested with a given polypeptide in this assay is rhSCF (R&D Systems, Minneapolis, MN, Cat# 255-SC) at 50 ng/ml alone and in combination with rhSCF and rhIL-3 (R&D Systems, Minneapolis, MN, Cat# 203-ML) at 30 ng/ml. After one hour, 10 µl of prepared cytokines, 50 µl SID (supernatants at 1:2 dilution = 50 µl) and 20 µl of diluted cells are added to the media which is already present in the wells to allow for a final total volume of 100 µl. The plates are then placed in a 37°C/5% CO₂ incubator for five days.

Eighteen hours before the assay is harvested, 0.5 µCi/well of [3H] Thymidine is added in a 10 µl volume to each well to determine the proliferation rate. The experiment is terminated by harvesting the cells from each 96-well plate to a filtermat using the Tomtec Harvester 96. After harvesting, the filtermats are dried, trimmed and placed into OmniFiltet assemblies consisting of one OmniFilter plate and one OmniFilter Tray. 60 µl Microscint is added to each well and the plate sealed with TopSeal-A press-on sealing film A bar code 15 sticker is affixed to the first plate for counting. The sealed plates is then loaded and the level of radioactivity determined via the Packard Top Count and the printed data collected for analysis. The level of radioactivity reflects the amount of cell proliferation.

The studies described in this example test the activity of a given polypeptide to stimulate bone marrow CD34+ cell proliferation. One skilled in the art could easily modify the exemplified studies to test the activity ofpolynucleotides (e.g., gene therapy), antibodies, agonists, and/or antagonists and fragments and variants thereof. As a nonlimiting example, potential antagonists tested in this assay would be expected to inhibit cell proliferation in the presence of cytokines and/or to increase the inhibition of cell proliferation in the presence of cytokines and a given polypeptide. In contrast, potential agonists tested in this assay would be expected to enhance cell proliferation and/or to decrease the inhibition of cell proliferation in the presence of cytokines and a given polypeptide.

The ability of a gene to stimulate the proliferation of bone marrow CD34+ cells indicates that polynucleotides and polypeptides corresponding to the gene are useful for the diagnosis and treatment of disorders affecting the immune system and hematopoiesis. Representative uses are described in the "Immune Activity" and "Infectious Disease" sections above, and elsewhere herein.

### Example 54: Assay for Extracellular Matrix Enhanced Cell Response (EMECR)

The objective of the Extracellular Matrix Enhanced Cell Response (EMECR) assay is to identify gene products (e.g., isolated polypeptides) that act on the hematopoietic stem cells in the context of the extracellular matrix (ECM) induced signal.

Cells respond to the regulatory factors in the context of signal(s) received from the surrounding microenvironment. For exainple, fibroblasts, and endothelial and epithelial stem cells fail to replicate in the absence of signals from the ECM. Hematopoietic stem cells can undergo self-renewal in the bone marrow, but not in *in vitro* suspension culture. The ability of stem cells to undergo self-renewal *in vitro* is dependent upon their interaction with the stromal cells and the ECM protein fibronectin (fn). Adhesion of cells to fn is mediated by the α₅.β₁ and α₄.β₁ integrin receptors, which are expressed by human and mouse hematopoietic stem cells. The factor(s) which integrate with the ECM environment and responsible for stimulating stem cell self-renewal has not yet been identified. Discovery of such factors should be of great interest in gene therapy and bone marrow transplant applications

Briefly, polystyrene, non tissue culture treated, 96-well plates are coated with fn fragment at a coating concentration of 0.2 µg/ cm². Mouse bone marrow cells are plated (1,000 cells/well ) in 0.2 ml of serum-free medium. Cells cultured in the presence of IL-3 ( 5 ng/ml ) + SCF ( 50 ng/ml ) would serve as the positive control, conditions under which little self-renewal but pronounced differentiation of the stem cells is to be expected. Gene products are tested with appropriate negative controls in the presence and absence of SCF(5.0 ng/ml), where test factor supernates represent 10% of the total assay volume. The plated cells are then allowed to grow by incubating in a low oxygen environment ( 5% CO₂, 7% O₂, and 88% N₂ ) tissue culture incubator for 7 days. The number of proliferating cells within the wells is then quantitated by measuring thymidine incorporation into cellular DNA. Verification of the positive hits in the assay will require phenotypic characterization of the cells, which can be accomplished by scaling up of the culture system and using appropriate antibody reagents against cell surface antigens and FACScan.

One skilled in the art could easily modify the exemplified studies to test the activity of polynucleotides (e.g., gene therapy), antibodies, agonists, and/or antagonists and fragments and variants thereof.

If a particular gene product is found to be a stimulator of hematopoietic progenitors, polynucleotides and polypeptides corresponding to the gene may be useful for the diagnosis and treatment of disorders affecting the immune system and hematopoiesis. Representative uses are described in the "Immune Activity" and "Infectious Disease'' sections above, and elsewhere herein. The gene product may also be useful in the expansion of stem cells and committed progenitors of various blood lineages, and in the differentiation and/or proliferation of various cell types.

Additionally, the polynucleotides and/or polypeptides of the gene of interest and/or agonists and/or antagonists thereof, may also be employed to inhibit the proliferation and differentiation of hematopoietic cells and therefore may be employed to protect bone marrow stem cells from chemotherapeutic agents during chemotherapy. This antiproliferative effect may allow administration of higher doses of chemotherapeutic agents and, therefore, more effective chemotherapeutic treatment.

Moreover, polynucleotides and polypeptides corresponding to the gene of interest may also be useful for the treatment and diagnosis of hematopoietic related disorders such as, for example, anemia, pancytopenia, leukopenia, thrombocytopenia or leukemia since stromal cells are important in the production of cells of hematopoietic lineages. The uses include bone marrow cell ex-vivo culture, bone marrow transplantation, bone marrow reconstitution, radiotherapy or chemotherapy of neoplasia.

### Example 55: Human Dermal Fibroblast and Aortic Smooth Muscle Cell Proliferation

The polypeptide of interest is added to cultures of normal human dermal fibroblasts (NHDF) and human aortic smooth muscle cells (AoSMC) and two co-assays are performed with each sample. The first assay examines the effect of the polypeptide of interest on the proliferation of normal human dermal fibroblasts (NHDF) or aortic smooth muscle cells (AoSMC). Aberrant growth of fibroblasts or smooth muscle cells is a part of several pathological processes, including fibrosis, and restenosis. The second assay examines IL6 production by both NHDF and SMC. IL6 production is an indication of functional activation. Activated cells will have increased production of a number of cytokines and other factors, which can result in a proinflammatory or immunomodulatory outcome. Assays are run with and without co-TNFa stimulation, in order to check for costimulatory or inhibitory activity.

Briefly, on day 1, 96-well black plates are set up with 1000 cells/well (NHDF) or 2000 cells/well (AoSMC) in 100 µl culture media. NHDF culture media contains: Clonetics FB basal media, 1mg/ml hFGF, 5mg/ml insulin, 50mg/ml gentamycin, 2%FBS, while AoSMC culture media contains.Clonetics SM basal media, 0.5 µg/ml hEGF, 5mg/ml insulin, 1µg/ml hFGF, 50mg/ml gentamycin, 50 µg/ml Amphotericin B, 5%FBS. After incubation @ 37°C for at least 4-5 hours culture media is aspirated and replaced with growth arrest media. Growth arrest media for NHDF contains fibroblast basal media, 50mg/ml gentamycin, 2% FBS, while growth arrest media for AoSMC contains SM basal media, 50mg/ml gentamycin, 50µg/ml Amphotericin B, 0.4% FBS. Incubate at 37C until day 2

On day 2, serial dilutions and templates of the polypeptide of interest are designed which should always include media controls and known-protein controls. For both stimulation and inhibition experiments, proteins are diluted in growth arrest media. For inhibition experiments, TNFa is added to a final concentration of 2ng/ml (NHDF) or 5ng/ml (AoSMC). Then add 1/3 vol media containing controls or supernatants and incubate at 37C/5% CO₂ until day 5.

Transfer 60µl from each well to another labeled 96-well plate, cover with a plate-sealer, and store at 4C until Day 6 (for IL6 ELISA). To the remaining 100 µl in the cell culture plate, aseptically add Alamar Blue in an amount equal to 10% of the culture volume (10µl). Return plates to incubator for 3 to 4 hours. Then measure fluorescence with excitation at 530nm and emission at 590nm using the CytoFluor. This yields the growth stimulation/inhibition data.

On day 5, the IL6 ELISA is performed by coating a 96 well plate with 50-100 ul/well of Anti-Human IL6 Monoclonal antibody diluted in PBS, pH 7.4, incubate ON at room temperature.

On day 6, empty the plates into the sink and blot on paper towels. Prepare Assay Buffer containing PBS with 4% BSA. Block the plates with 200 µl/well of Pierce Super Block blocking buffer in PBS for 1-2 hr and then wash plates with wash buffer (PBS, 0:05% Tween-20). Blot plates on paper towels. Then add 50 µl/well of diluted Anti-Human IL-6 Monoclonal, Biotin-labeled antibody at 0.50 mg/ml. Make dilutions of IL-6 stock in media (30, 10, 3, 1, 0.3, 0 ng/ml). Add duplicate samples to top row of plate. Cover the plates and incubate for 2 hours at RT on shaker.

Wash plates with wash buffer and blot on paper towels. Dilute EU-labeled Streptavidin 1:1000 in Assay buffer, and add 100 µl/well. Cover the plate and incubate 1 h at RT. Wash plates with wash buffer. Blot on paper towels.

Add 100 µl/well of Enhancement Solution. Shake for 5 minutes. Read the plate on the Wallac DELFIA Fluorometer. Readings from triplicate samples in each assay were tabulated and averaged.

A positive result in this assay suggests AoSMC cell proliferation and that the gene product of interest may be involved in dermal fibroblast proliferation and/or smooth muscle cell proliferation. A positive result also suggests many potential uses of polypeptides, polynucleotides, agonists and/or antagonists of the gene/gene product of interest. For example, inflammation and immune responses, wound healing, and angiogenesis, as detailed throughout this specification. Particularly, polypeptides of the gene product and polynucleotides of the gene may be used in wound healing and dermal regeneration, as well as the promotion of vasculargenesis, both of the blood vessels and lymphatics. The growth of vessels can be used in the treatment of, for example, cardiovascular diseases. Additionally, antagonists of polypeptides of the gene product and polynucleotides of the gene may be useful in treating diseases, disorders, and/or conditions which involve angiogenesis by acting as an anti-vascular (e.g., anti-angiogenesis). These diseases, disorders, and/or conditions are known in the art and/or are described herein, such as, for example, malignancies, solid tumors, benign tumors, for example hemangiomas, acoustic neuromas, neurofibromas, trachomas, and pyogenic granulomas; artheroscleric plaques; ocular angiogenic diseases, for example, diabetic retinopathy, retinopathy of prematurity, macular degeneration, corneal graft rejection, neovascular glaucoma, retrolental fibroplasia, rubeosis, retinoblastoma, uvietis and Pterygia (abnormal blood vessel growth) of the eye; rheumatoid arthritis; psoriasis; delayed wound healing; endometriosis; vasculogenesis; granulations; hypertrophic scars (keloids); nonunion fractures; scleroderma; trachoma; vascular adhesions; myocardial angiogenesis; coronary collaterals; cerebral collaterals; arteriovenous malformations; ischemic limb angiogenesis; Osler-Webber Syndrome; plaque neovascularization; telangiectasia; hemophiliac joints; angiofibroma; fibromuscular dysplasia; wound granulation; Crohn's disease; and atherosclerosis. Moreover, antagonists of polypeptides of the gene product and polynucleotides of the gene may be useful in treating anti-hyperproliferative diseases and/or anti-inflammatory known in the art and/or described herein.

One skilled in the art could easily modify the exemplified studies to test the activity of polynucleotides (e.g., gene therapy), antibodies, agonists, and/or antagonists and fragments and variants thereof.

### Example 56: Cellular Adhesion Molecule (CAM) Expression on Endothelial Cells

The recruitment of lymphocytes to areas of inflammation and angiogenesis involves specific receptor-ligand interactions between cell surface adhesion molecules (CAMs) on lymphocytes and the vascular endothelium. The adhesion process, in both normal and pathological settings, follows a multi-step cascade that involves intercellular adhesion molecule-1 (ICAM-1), vascular cell adhesion molecule-1 (VCAM-1), and endothelial leukocyte adhesion molecule-1 (E-selectin) expression on endothelial cells (EC). The expression of these molecules and others on the vascular endothelium determines the efficiency with which leukocytes may adhere to the local vasculature and extravasate into the local tissue during the development of an inflammatory response. The local concentration of cytokines and growth factor participate in the modulation of the expression of these CAMs.

Briefly, endothelial cells (e.g., Human Umbilical Vein Endothelial cells HUVECs)) are grown in a standard 96 well plate to confluence, growth medium is removed from the cells and replaced with 100 µl of 199 Medium (10% fetal bovine serum (FBS)). Samples for testing and positive or negative controls are added to the. plate in triplicate (in 10 µl volumes). Plates are then incubated at 37°C for either 5 h (selectin and integrin expression) or 24 h (integrin expression only). Plates are aspirated to remove medium and 100 µl of 0.1% paraformaldehyde-PBS(with Ca++ and Mg++) is added to each well. Plates are held at 4°C for 30 min. Fixative is removed from the wells and wells are washed 1X with PBS(+Ca,Mg) + 0.5% BSA and drained. 10 µl of diluted primary antibody is added to the test and control wells. Anti-ICAM-1-Biotin, Anti-VCAM-1-Biotin and Anti-E-selectin-Biotin are used at a concentration of 10 µg/ml (1:10 dilution of 0.1 mg/ml stock antibody). Cells are incubated at 37°C for 30 min. in a humidified environment. Wells are washed three times with PBS(+Ca,Mg) + 0.5% BSA. 20 µl of diluted ExtrAvidin-Alkaline Phosphotase (1:5,000 dilution, refered to herein as the working dilution) are added to each well and incubated at 37°C for 30 min. Wells are washed three times with PBS(+Ca,Mg)+0.5% BSA. Dissolve 1 tablet of p-Nitrophenol Phosphate pNPP per 5 ml of glycine buffer (pH 10.4). 100 µl of pNPP substrate in glycine buffer is added to each test well. Standard wells in triplicate are prepared from the working dilution of the ExtrAvidin-Alkaline Phosphotase in glycine buffer: 1:5,000 (10⁰) > 10^{-0.5} > 10⁻¹ > 10^{-1.5}. 5 µl of each dilution is added to triplicate wells and the resulting AP content in each well is 5.50 ng, 1.74 ng, 0.55 ng, 0.18 ng. 100 µl of pNNP reagent is then added to each of the standard wells. The plate is incubated at 37°C for 4h. A volume of 50 µl of 3M NaOH is added to all wells. The plate is read on a plate reader at 405 nm using the background subtraction option on blank wells filled with glycine buffer only. Additionally, the template is set up to indicate the concentration of AP-conjugate in each standard well [5.50 ng; 1.74 ng; 0.55 ng; 0.18 ng]. Results are indicated as amount of bound AP-conjugate in each sample.

### Example 57: Alamar Blue Endothelial Cells Proliferation Assay

This assay may be used to quantitatively determine protein mediated inhibition of bFGF-induced proliferation of Bovine Lymphatic Endothelial Cells (LECs), Bovine Aortic Endothelial Cells (BAECs) or Human Microvascular Uterine Myometrial Cells (UTMECs). This assay incorporates a fluorometric growth indicator based on detection of metabolic activity. A standard Alamar Blue Proliferation Assay is prepared in EGM-2MV with 10 ng /ml of bFGF added as a source of endothelial cell stimulation. This assay may be used with a variety of endothelial cells with slight changes in growth medium and cell concentration. Dilutions of the protein batches to be tested are diluted as appropriate. Serum-free medium (GIBCO SFM) without bFGF is used as a non-stimulated control and Angiostatin or TSP-1 are included as a known inhibitory controls.

Briefly, LEC, BAECs or UTMECs are seeded in growth media at a density of 5000 to 2000 cells/well in a 96 well plate and placed at 37-C overnight. After the overnight incubation of the cells, the growth media is removed and replaced with GIBCO EC-SFM. The cells are treated with the appropriate dilutions of the protein of interest or control protein sample(s) (prepared in SFM ) in triplicate wells with additional bFGF to a concentration of 10 ng/ ml. Once the cells have been treated with the samples, the plate(s) is/are placed back in the 37° C incubator for three days. After three days 10 ml of stock alamar blue (Biosource Cat# DAL1100) is added to each well and the plate(s) is/are placed back in the 37°C incubator for four hours. The plate(s) are then read at 530nm excitation and 590nm emission using the CytoFluor fluorescence reader. Direct output is recorded in relative fluorescence units.

Alamar blue is an oxidation-reduction indicator that both fluoresces and changes color in response to chemical reduction of growth medium resulting from cell growth. As cells grow in culture, innate metabolic activity results in a chemical reduction of the immediate surrounding environment. Reduction related to growth causes the indicator to change from oxidized (non-fluorescent blue) form to reduced (fluorescent red) form. i.e. stimulated proliferation will produce a stronger signal and inhibited proliferation will produce a weaker signal and the total signal is proportional to the total number of cells as well as their metabolic activity. The background level of activity is observed with the starvation medium alone. This is compared to the output observed from the positive control samples (bFGF in growth medium) and protein dilutions.

### Example 58: Detection of Inhibition of a Mixed Lymphocyte Reaction

This assay can be used to detect and evaluate inhibition of a Mixed Lymphocyte Reaction (MLR) by gene products (e.g., isolated polypeptides). Inhibition of a MLR may be due to a direct effect on cell proliferation and viability, modulation of costimulatory molecules on interacting cells, modulation of adhesiveness between lymphocytes and accessory cells, or modulation of cytokine production by accessory cells. Multiple cells may be targeted by these polypeptides since the peripheral blood mononuclear fraction used in this assay includes T, B and natural killer lymphocytes, as well as monocytes and dendritic cells.

Polypeptides of interest found to inhibit the MLR may find application in diseases associated with lymphocyte and monocyte activation or proliferation. These include, but are not limited to, diseases such as asthma, arthritis, diabetes, inflammatory skin conditions, psoriasis, eczema, systemic lupus erythematosus, multiple sclerosis, glomerulonephritis, inflammatory bowel disease, crohn's disease, ulcerative colitis, arteriosclerosis, cirrhosis, graft vs. host disease, host vs. graft disease, hepatitis, leukemia and lymphoma.

Briefly, PBMCs from human donors are purified by density gradient centrifugation using Lymphocyte Separation Medium (LSM®, density 1.0770 g/ml, Organon Teknika Corporation, West Chester, PA). PBMCs from two donors are adjusted to 2 x 10⁶ cells/ml in RPMI-1640 (Life Technologies, Grand Island, NY) supplemented with 10% FCS and 2 mM glutamine. PBMCs from a third donor is adjusted to 2 x 10⁵ cells/ml. Fifty microliters of PBMCs from each donor is added to wells of a 96-well round bottom microtiter plate. Dilutions of test materials (50 µl) is added in triplicate to microtiter wells. Test samples (of the protein of interest) are added for final dilution of 1:4; rhuIL-2 (R&D Systems, Minneapolis, MN, catalog number 202-IL) is added to a final concentration of 1 µg/ml; anti-CD4 mAb (R&D Systems, clone 34930.11, catalog number MAB379) is added to a final concentration of 10 µg/ml. Cells are cultured for 7-8 days at 37°C in 5% CO₂, and 1 µC of [³H] thymidine is added to wells for the last 16 hrs of culture. Cells are harvested and thymidine incorporation determined using a Packard TopCount. Data is expressed as the mean and standard deviation of triplicate determinations.

Samples of the protein of interest are screened in separate experiments and compared to the negative control treatment, anti-CD4 mAb, which inhibits proliferation of lymphocytes and the positive control treatment, IL-2 (either as recombinant material or supernatant), which enhances proliferation of lymphocytes.

One skilled in the art could easily modify the exemplified studies to test the activity of polynucleotides (e.g., gene therapy), antibodies, agonists, and/or antagonists and fragments and variants thereof.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference. Further, the hard copy of the sequence listing submitted herewith and the corresponding computer readable form are both incorporated herein by reference in their entireties. Additionally, the contents of U.S. Provisional Applications Serial No. 60/164,834 and 60/224,007 are all hereby incorporated by reference in their entirety.

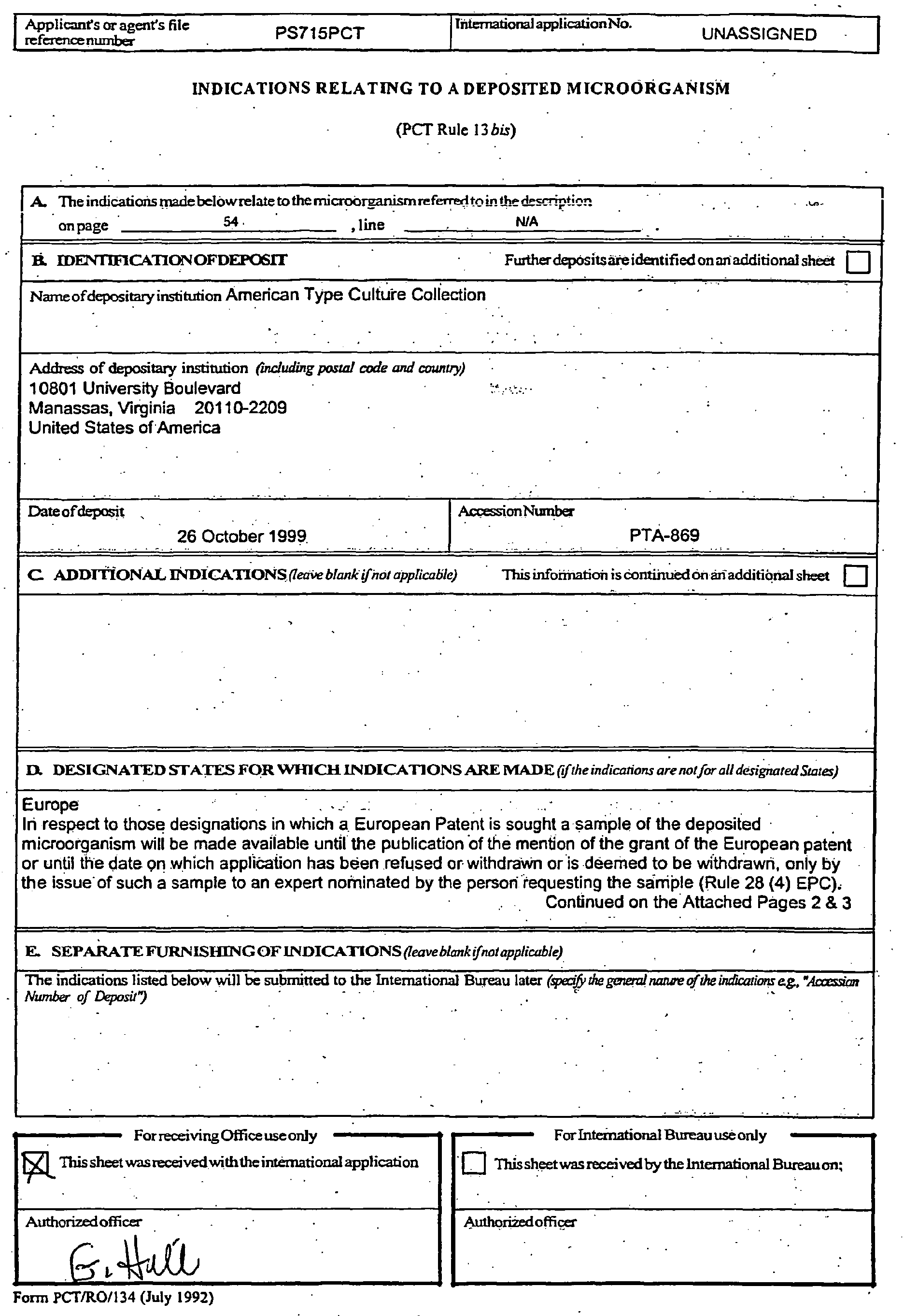
Applicant's or agent's file reference number | PS715PCT | International application No. | UNASSIGNED

INDICATIONS RELATING TO A DEPOSITED MICROORGANISM

(PCT Rule 13bis)

A. The indications made below relate to the microorganism referred to in the description
on page 54 , line N/A .

B. IDENTIFICATION OF DEPOSIT | Further deposits are identified on an additional sheet [ ]

Name of depositary institution American Type Culture Collection

Address of depositary institution (including postal code and country)
10801 University Boulevard
Manassas, Virginia 20110-2209
United States of America

Date of deposit | Accession Number
26 October 1999 | PTA-869

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

Europe
In respect to those designations in which a European Patent is sought a sample of the deposited
microorganism will be made available until the publication of the mention of the grant of the European patent
or until the date on which application has been refused or withdrawn or is deemed to be withdrawn, only by
the issue of such a sample to an expert nominated by the person requesting the sample (Rule 28 (4) EPC).
Continued on the Attached Pages 2 & 3

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:
Authorized officer | Authorized officer
 |

Form PCT/RO/134 (July 1992)

### CANADA

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

### NORWAY

The applicant hereby requests that the application has been laid open to public inspection (by the Norwegian Patent Office), or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

### AUSTRALIA

The applicant hereby gives notice that the furnishing of a sample of a microorganism shall only be effected prior to the grant of a patent, or prior to the lapsing, refusal or withdrawal of the application, to a person who is a skilled addressee'without an interest in the invention (Regulation 3.25(3) of the Australian Patents Regulations).

### FINLAND

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

### UNITED KINGDOM

The applicant hereby requests that the furnishing of a sample of a microorganism shall only be made available to an expert. The request to this effect must be filed by the applicant with the International Bureau before the completion of the technical preparations for the international publication of the application.

### DENMARK

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

### SWEDEN

The applicant hereby requests that, until the application has been laid open to public inspection (by the Swedish Patent Office), or has been finally decided upon by the Swedish Patent Office without having been laid open to public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

### NETHERLANDS

The applicant hereby requests that until the date of a grant of a Netherlands patent or until the date on which the application is refused or withdrawn or lapsed, the microorganism shall be made available as provided in the 31 F(1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

## Claims

1. A nucleic acid molecule comprising a polynucleotide having a nucleotide sequence selected from the group consisting of:
(a) a polynucleotide having the entire nucleotide sequence of SEQ lD NO: 15;
(b) a polynucleotide having the entire cDNA sequence contained in the clone included in ATCC Deposit No. PTA-869, which is hybridizable to SEQ lD NO: 15;
(c) a polynucleotide fragment of SEQ ID NO: 15;
(d) a polynucleotide fragment of the cDNA sequence contained in the clone included in ATCC Deposit No. PTA-869, which is hybridizable to SEQ lD NO: 15;
(e) a polynucleotide encoding a polypeptide fragment of SEQ ID NO: 56;
(f) a polynucleotide encoding a polypeptide fragment encoded by the cDNA sequence contained in the clone included in ATCC Deposit No. PTA-869, which is hybridizable to SEQ ID NO: 15;
(g) a polynucleotide encoding a polypeptide fragment of SEQ ID NO: 56 or a polypeptide fragment encoded by the cDNA sequence included in ATCC Deposit No. PTA-869, having biological activity;
(h) a polynucleotide encoding a polypeptide fragment of SEQ ID NO: 56 or a polypeptide fragment encoded by the cDNA sequence contained in the clone included in ATCC Deposit No. PTA-869, which is secreted;
(i) a polynucleotide encoding a full length polypeptide of SEQ ID NO: 56 or being encoded by the cDNA sequence contained in the clone included in ATCC Deposit No. PTA-869;
(j) a polynucleotide encoding a polypeptide domain of SEQ ID NO: 56;
(k) a polynucleotide encoding a polypeptide domain encoded by the cDNA sequence contained in the clone included in ATCC Deposit No. PTA-869, which is hybridizable to SEQ ID NO: 15;
(l) a polynucleotide encoding a polypeptide epitope of SEQ ID NO:56;
(m) a polynucleotide encoding a polypeptide epitope encoded by the cDNA sequence contained in the clone included in ATCC Deposit No. PTA-869, which is hybridizable to SEQ ID NO: 15;
(n) a polynucleotide which is at least 95% identical to a polynucleotide as defined in any one of (a) to (m);
(o) a polynucleotide encoding a polypeptide having an amino acid sequence at least 95% identical to the amino acid sequence of a polypeptide encoded by a polynucleotide of (a) to (n);
(p) the polynucleotide of any one of (a) to (o), wherein the polynucleotide fragment comprises a nucleotide sequence encoding a secreted protein; and
(q) a polynucleotide capable of hybridizing under stringent conditions to any one of the polynucleotides specified in (a) to (p), wherein said polynucleotide does not hybridize under stringent conditions to a nucleic acid molecule having a nucleotide sequence of only A residues or of only T residues;
or the complementary strand of polynucleotide (a) to (q).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

4. A gene corresponding to the cDNA sequence of SEQ ID NO: 15 or encoding the polypeptide having the amino acid sequence of SEQ ID NO: 56.

5. A vector comprising the nucleic acid molecule of any one of claims 1 to 3 or the gene of claim 4.

6. A method of making a recombinant host cell comprising introducing the nucleic acid molecule of any one of claims 1 to 3, the gene of claim 4 or the vector of claim 5.

7. A recombinant host cell containing the nucleic acid molecule of claims 1 to 3, the gene of claim 4 or the vector of claim 5 or produced by the method of claim 6.

8. The recombinant host cell of claim 7 which expresses a polypeptide encoded by the nucleic acid molecule of any one of claims 1 to 3 or the gene of claim 4.

9. A method of making a polypeptide encoded by the nucleic acid molecule of any one of claims 1 to 3 or the gene of claim 4 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3, the gene of claim 4 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the ceii membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3 or the gene of claim 4 or the vector of claim 5 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the gene of claim 4, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, the gene of claim 4, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3, the gene of claim 4 or the antibody of claim 11.

23. A method for the production of a pharmaceutical composition comprising the steps of the method of any one of claim 14, 17 or 18 and the further step of formulating the compound identified in a pharmaceutically acceptable form.
